# EUROPEAN PATENT SPECIFICATION

(11) **EP 2 155 722 B1**
(45) Date of publication and mention of the grant of the patent: **14.08.2013**
(21) Application number: 08768067.4
(22) Date of filing: 04.06.2008
(51) Int. Cl.: C07D 401/14, C07D 403/14, C07D 407/14, C07D 409/14, C07D 413/14, C07D 417/14, C07D 491/056, A61K 31/4245, A61K 31/444, A61K 31/506, A61P 35/00

(54) **POLYCYCLIC INDAZOLE DERIVATIVES AND THEIR USE AS ERK INHIBITORS FOR THE TREATMENT OF CANCER**
POLYCYCLISCHE INDAZOLDERIVATE UND DEREN VERWENDUNG ALS ERK-INHIBITOREN ZUR BEHANDLUNG VON KREBS
DÉRIVÉS D'INDAZOLE POLYCYCLIQUE ET LEUR UTILISATION EN TANT QU'INHIBITEURS ERK POUR LE TRAITEMENT DU CANCER

(30) Priority: 05.06.2007 US 810282
(43) Date of publication of application: 24.02.2010
(73) Proprietor: Merck Sharp & Dohme Corp., Rahway, NJ 07065-0907 (US)
(72) Inventor: COOPER, Alan, West Caldwell, New Jersey 07006 (US); DENG, Yongqi, Newton, Massachusetts 02459 (US); SHIPPS, Gerald, W., Jr., Stoneham, Massachusetts 02180 (US); SHIH, Neng-Yang, Lexington, Massachusetts 02420 (US); ZHU, Hugh, Warren, New Jersey 07059 (US); KELLY, Joseph, Parlin, New Jersey 08859 (US); DOLL, Ronald, Convent Station, New Jersey 07960 (US); NAN, Yang, Malden, Massachusetts 02148 (US); DESAI, Jagdish, Monroe Township, New Jersey 08831 (US); WANG, James, Westfield, New Jersey 07090 (US); PALIWAL, Sunil, Monroe Township, New Jersey 08831 (US); TSUI, Hon-Chung, East Brunswick, New Jersey 08816 (US); BOGA, Sobhana, Babu, Scotch Plains, New Jersey 07076 (US); ALHASSAN, Abdul-Basit, Scotch Plains, New Jersey 07076 (US); GAO, Xiaolei, Bridgewater, New Jersey 08807 (US); ZHU, Liang, Waltham, Massachusetts 02473 (US); PATEL, Mehul, Waltham, Massachusetts 02453 (US)
(74) Representative: Horgan, James Michael Frederic
(86) International application number: PCT/US2008/006979
(87) International publication number: WO 2008/153858

(56) References cited:
- WO-A-02/064586
- WO-A-02/088090
- WO-A-2005/113541
- WO-A-2007/070398

## Description

### BACKGROUND

The processes involved in tumor growth, progression, and metastasis are mediated by signaling pathways that are activated in cancer cells. The ERK pathway plays a central role in regulating mammalian cell growth by relaying extracellular signals from ligand-bound cell surface tyrosine kinase receptors such as erbB family, PDGF, FGF, and VEGF receptor tyrosine kinase. Activation of the ERK pathway is via a cascade of phosphorylation events that begins with activation of Ras. Activation of Ras leads to the recruitment and activation of Raf, a serine-threonine kinase. Activated Raf then phosphorylates and activates MEK1/2, which then phosphorylates and activates ERK1/2. When activated, ERK1/2 phosphorylates several downstream targets involved in a multitude of cellular events including cytoskeletal changes and transcriptional activation. The ERK/MAPK pathway is one of the most important for cell proliferation, and it is believed that the ERK/MAPK pathway is frequently activated in many tumors. Ras genes, which are upstream of ERK1/2, are mutated in several cancers including colorectal, melanoma, breast and pancreatic tumors. The high Ras activity is accompanied by elevated ERK activity in many human tumors. In addition, mutations of BRAF, a serine-threonine kinase of the Raf family, are associated with increased kinase activity. Mutations in BRAF have been identified in melanomas (60%), thyroid cancers (greater than 40%) and colorectal cancers. These observations indicate that the ERK1/2 signalling pathway is an attractive pathway for anticancer therapies in a broad spectrum of human tumours.
WO 2005 113 541 discloses pyrrole derivatives 5 and their use as inhibitors of ERK protein kinase.
WO 02064 586 discloses pyridine derivatives and their use as inhibitors of ERK protein kinase.
WO 02088 090 discloses pyrazole derivatives and their use as inhibitors of ERK protein kinase

Therefore, a welcome contribution to the art would be small-molecules (i.e., compounds) that inhibit ERK activity (i.e., ERK1 and ERK2 activity), which small-molecules would be useful for treating a broad spectrum of cancers, such as, for example, melanoma, pancreatic cancer, thryroid cancer, colorectal cancer, lung cancer, breast cancer, and ovarian cancer. Such a contribution is provided by this invention.

### SUMMARY OF THE INVENTION

This invention provides compounds that inhibit the activity of ERK1 and/or the activity of ERK2.

The compounds of this invention also inhibit the phosphorylation of ERK1 and ERK2.

Thus, this invention provides compounds that are ERK inhibitors (i.e., ERK1 inhibitors and/or ERK2 inhibitors), said compounds having the following structures:

| Example No. | COMPOUND |
|---|---|
| 612 | 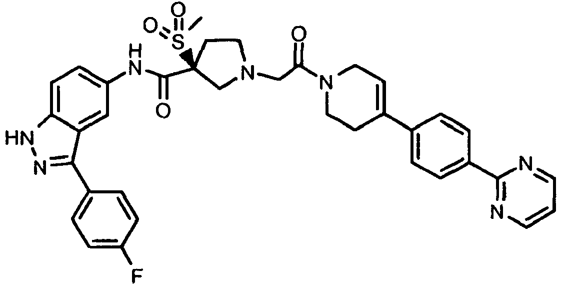 |
| 613 | 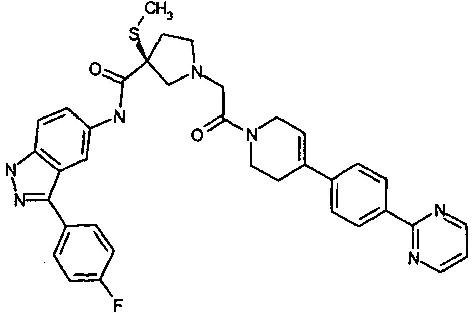 |
| 614 | 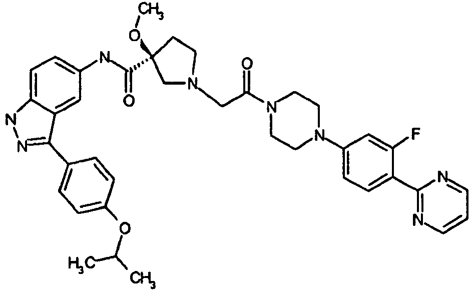 |
| 615 | 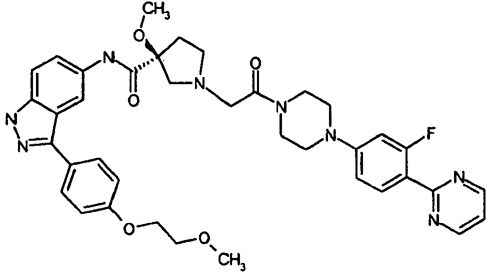 |
| 616 | 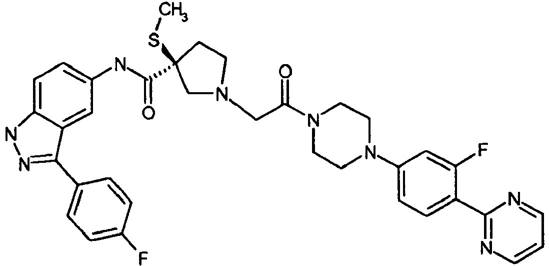 |
| 617 | 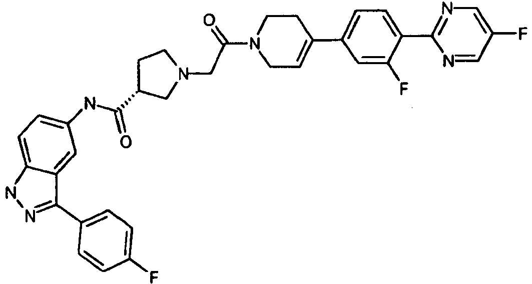 |
| 619 | 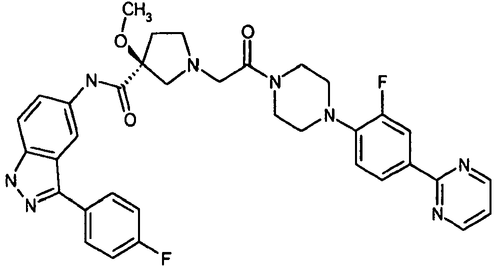 |
| 620 | 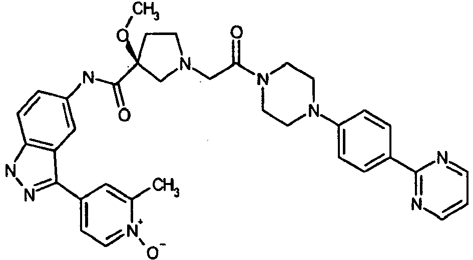 |
| 621 | 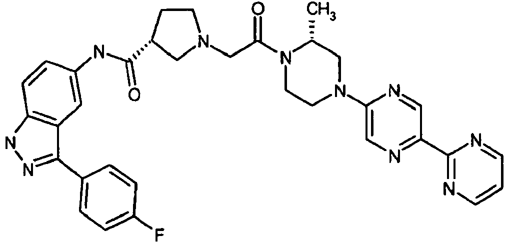 |
| 622 | 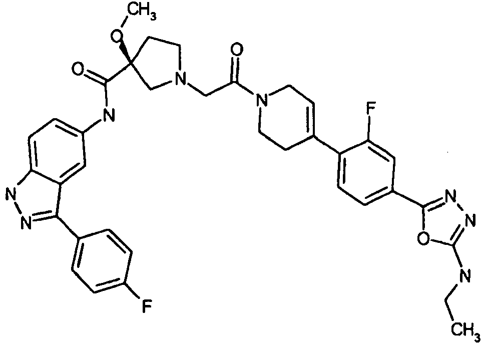 |
| 623 | 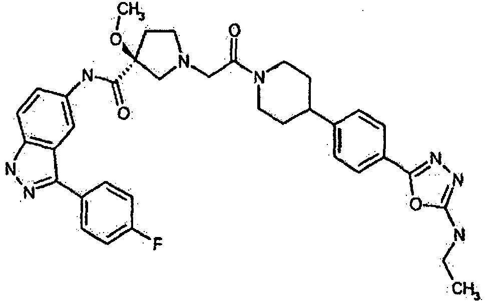 |
| 624 | 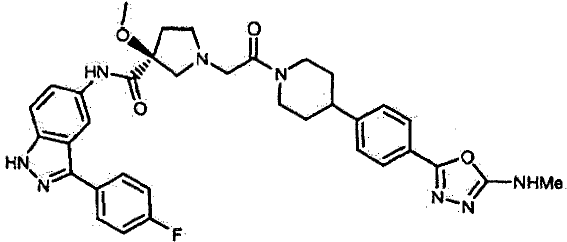 |
| | |
| 625 | 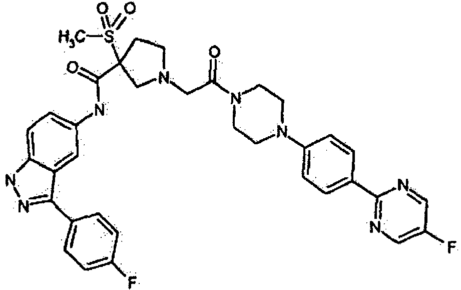 |
| 626 | 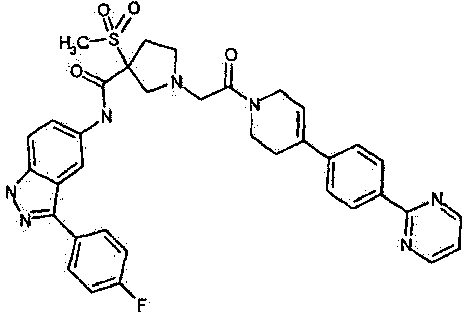 |
| 627 | 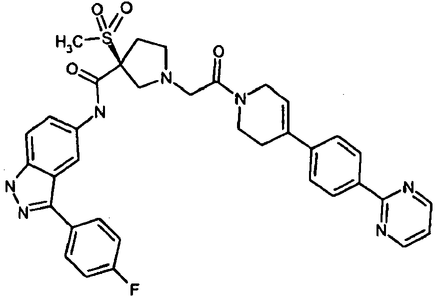 |
| 628 | 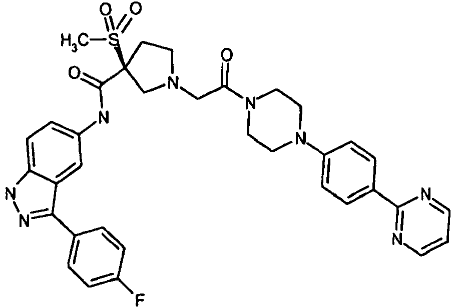 |
| 629 | 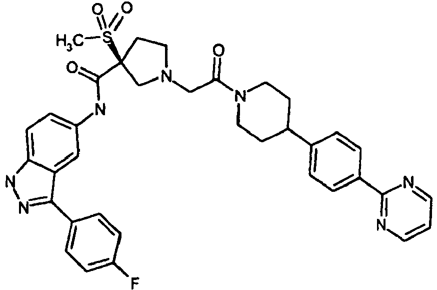 |
| 630 | 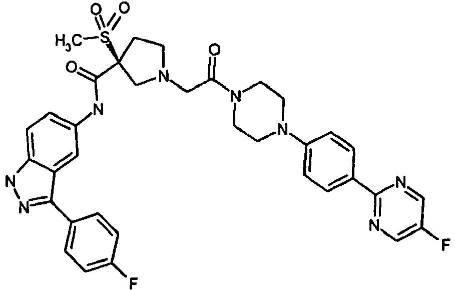 |
| 631 | 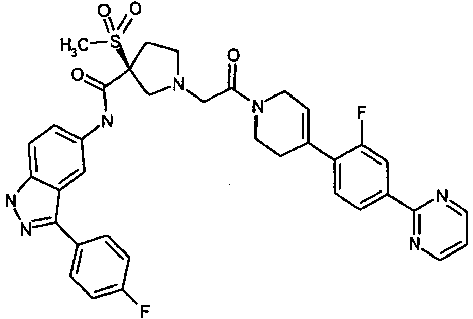 |
| 632 | 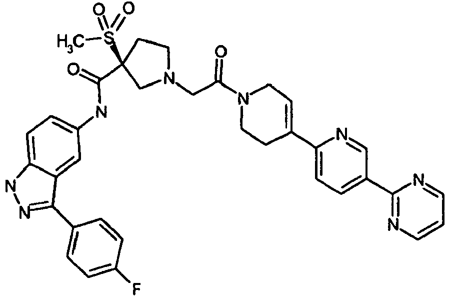 |
| 633 | 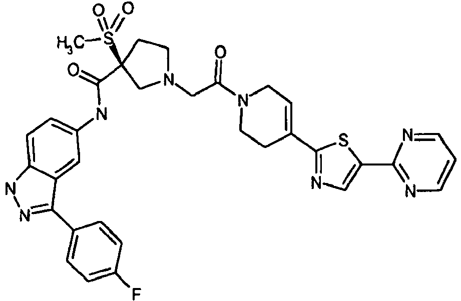 |
| 634 | 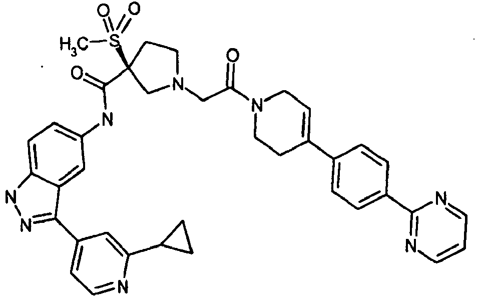 |
| 635 | 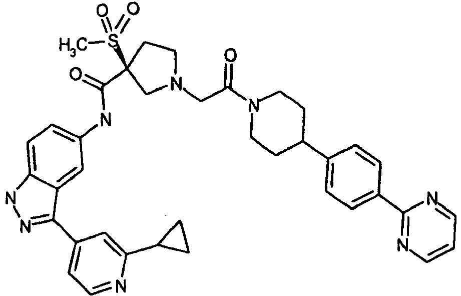 |
| 636 | 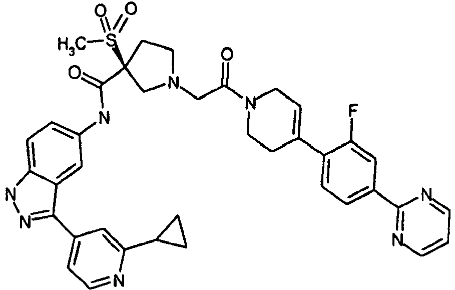 |
| 637 | 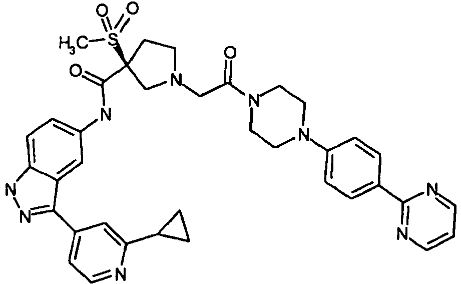 |
| 638 | 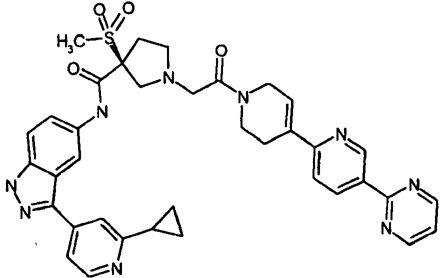 |
| 639 | 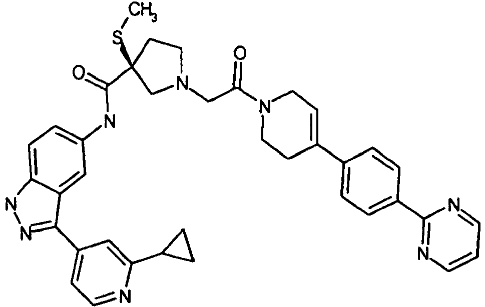 |
| 640 | 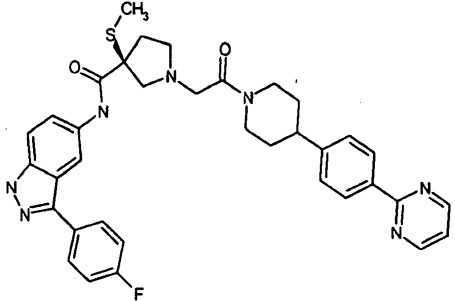 |
| 641 | 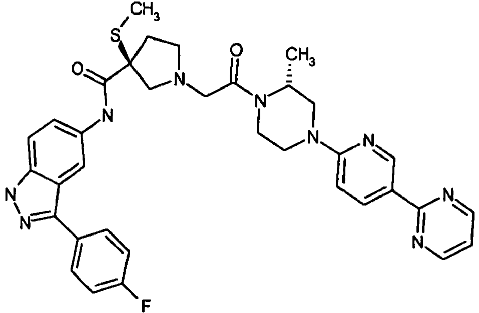 |
| 642 | 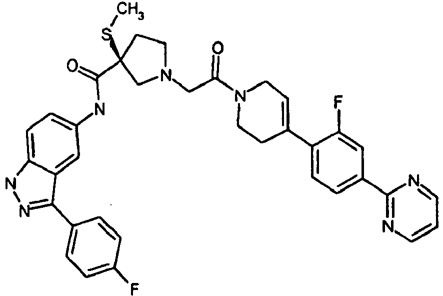 |
| 643 | 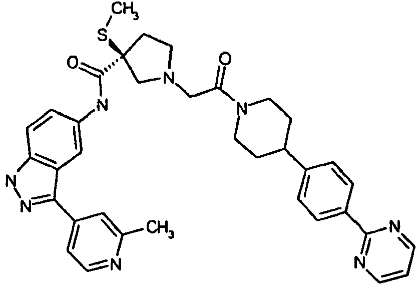 |
| 644 | 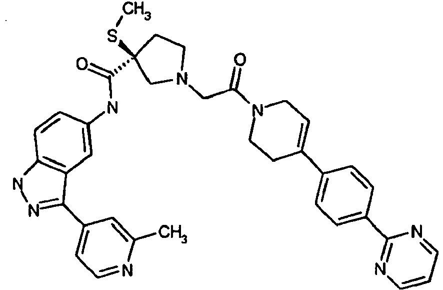 |
| 645 | 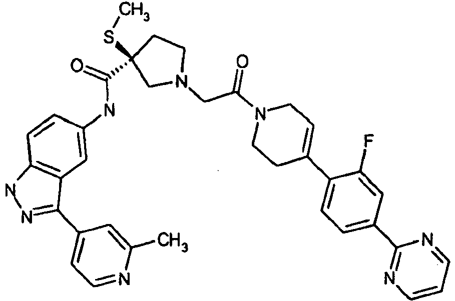 |
| 646 | 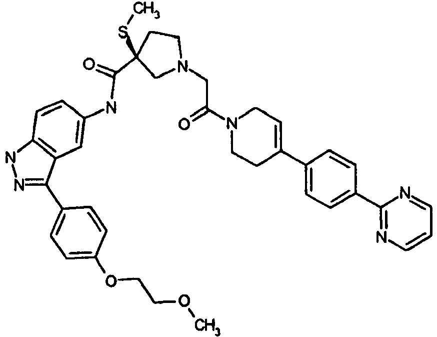 |
| 647 | 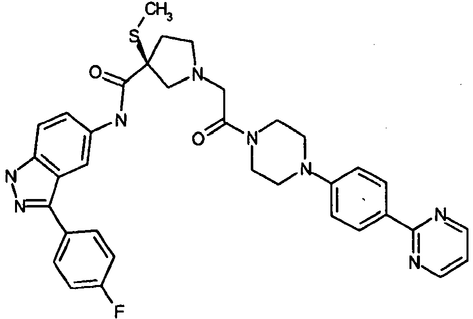 |
| 648 | 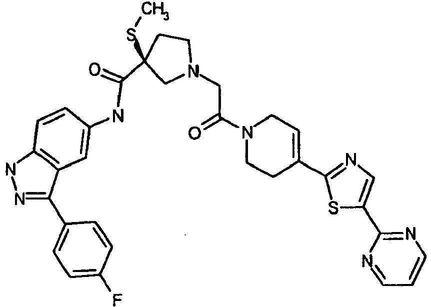 |
| 649 | 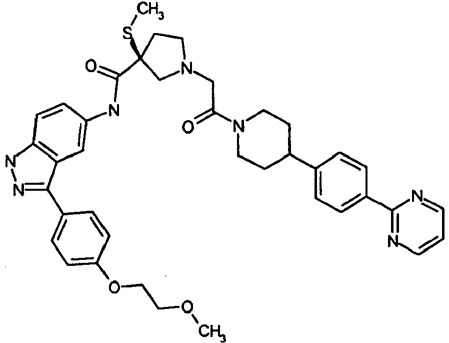 |
| 650 | 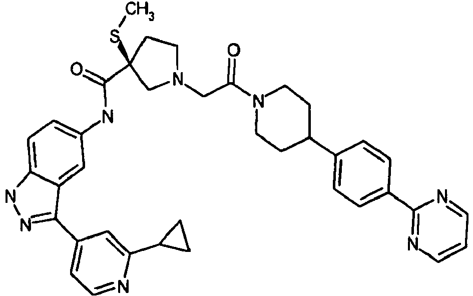 |
| 651 | 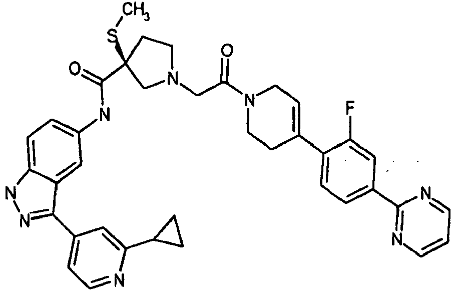 |
| 652 | 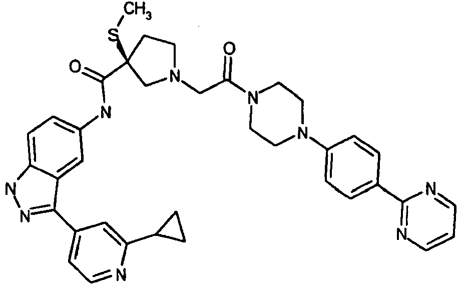 |
| 653 | 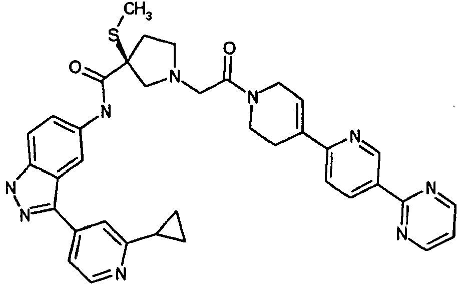 |
| 654 | 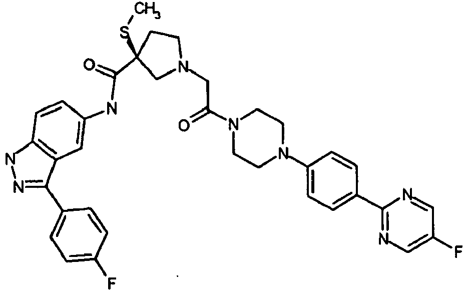 |
| 655 | 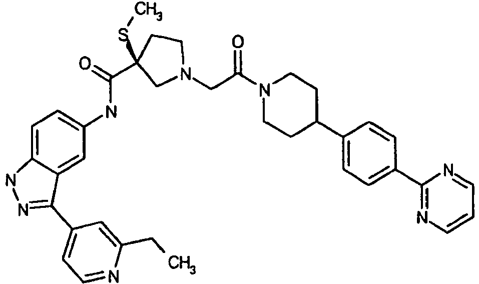 |
| 656 | 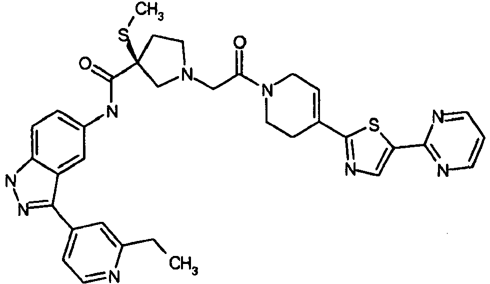 |
| 657 | 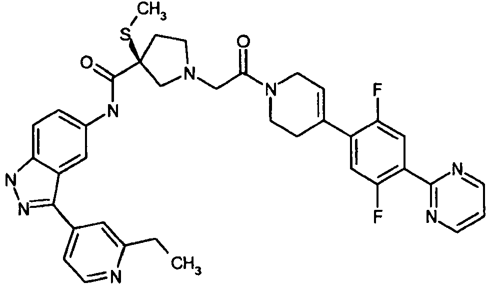 |
| 658 | 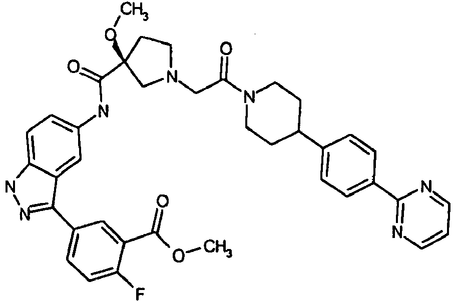 |
| 659 | 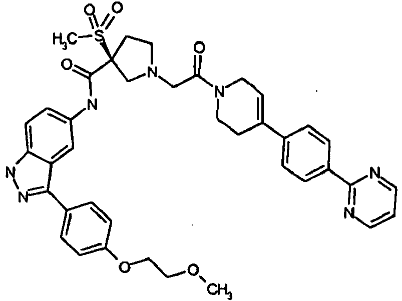 |
| 660 | 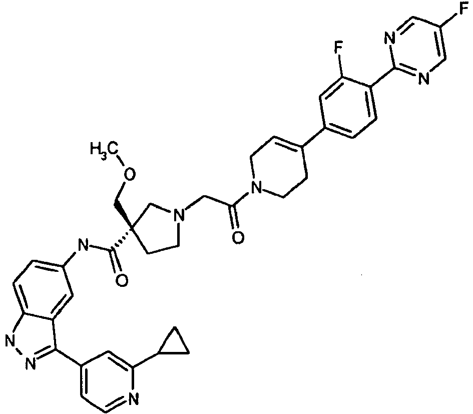 |
| 661 | 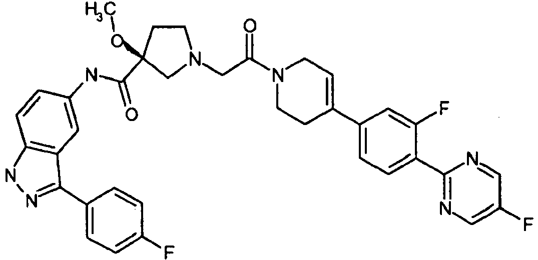 |
| 662 | 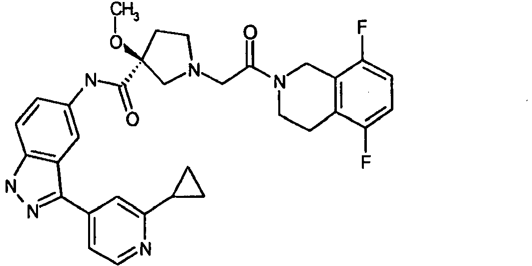 |
| 663 | 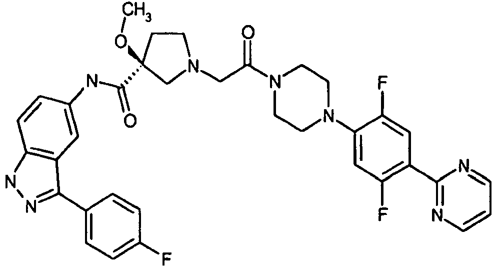 |
| 664 | 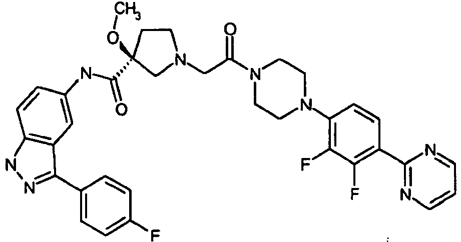 |
| 665 | 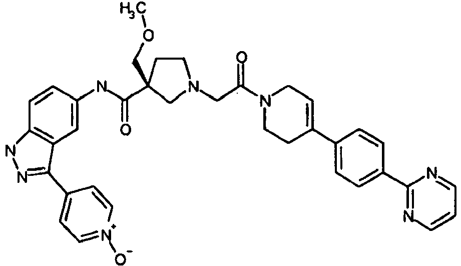 |
| 666 | 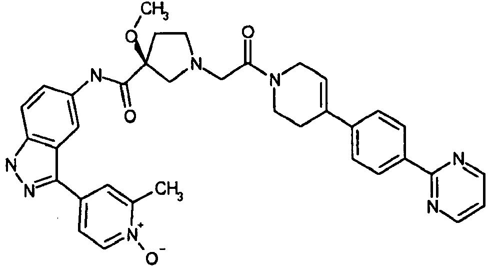 |
| 667 | 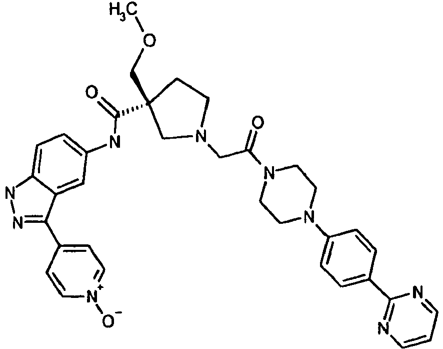 |
| 668 | 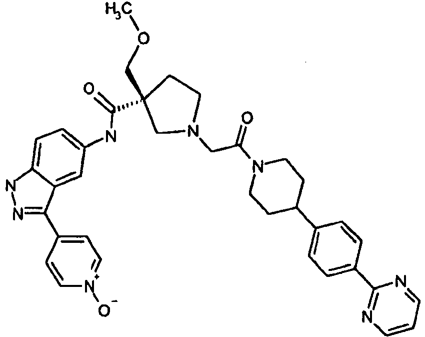 |
| 669 | 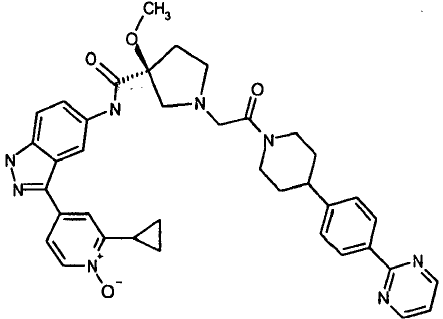 |
| 670 | 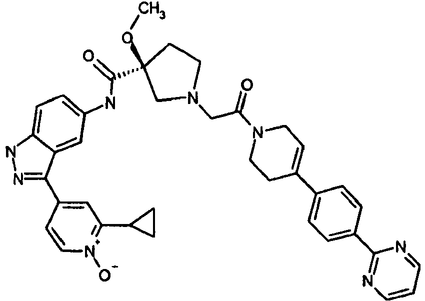 |
| 671 | 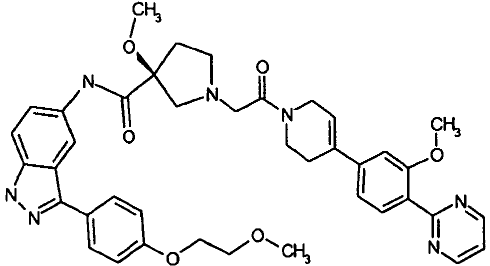 |
| 672 | 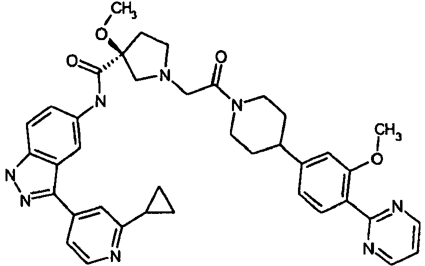 |
| 673 | 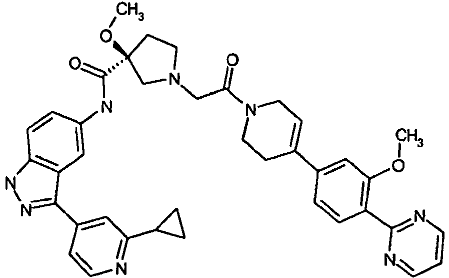 |
| 674 | 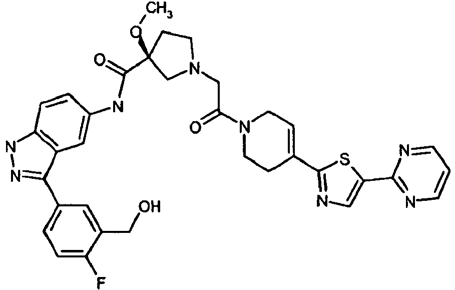 |
| 675 | 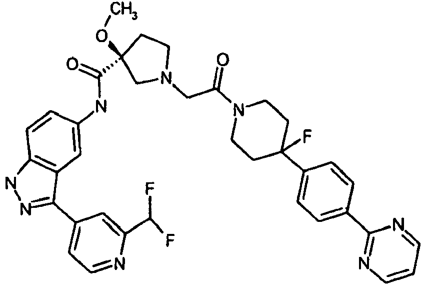 |
| 676 | 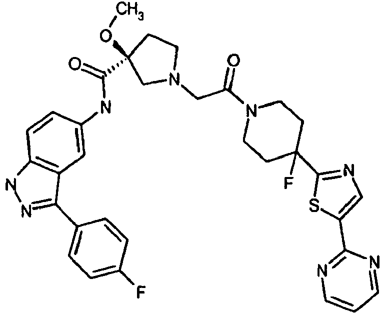 |
| 677 | 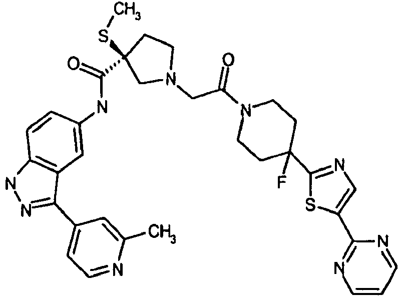 |
| 678 | 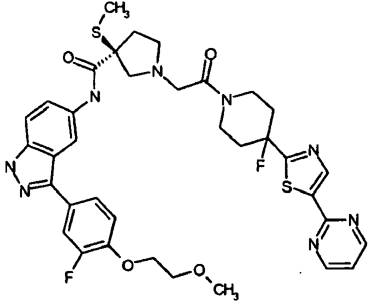 |
| 679 | 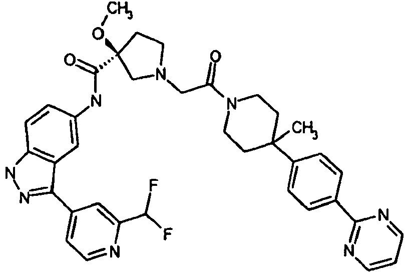 |
| 680 | 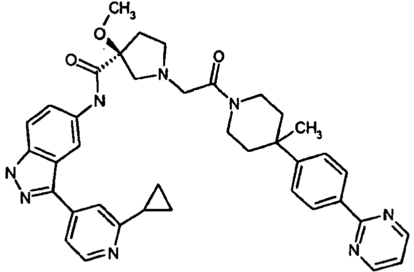 |
| 681 | 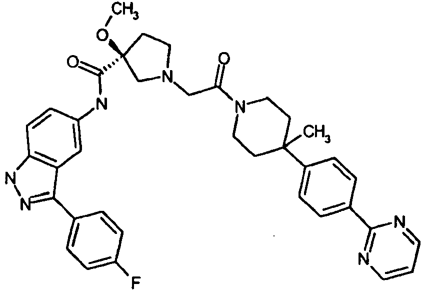 |
| 682 | 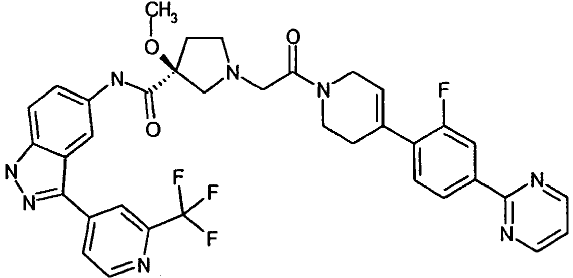 |
| 683 | 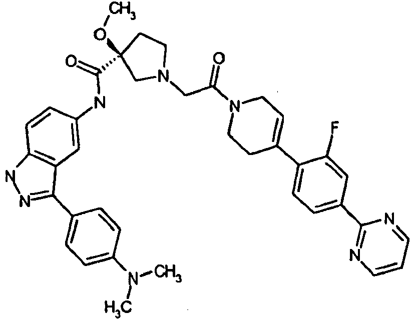 |
| 684 | 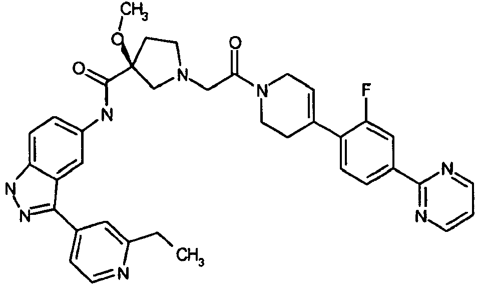 |
| 685 | 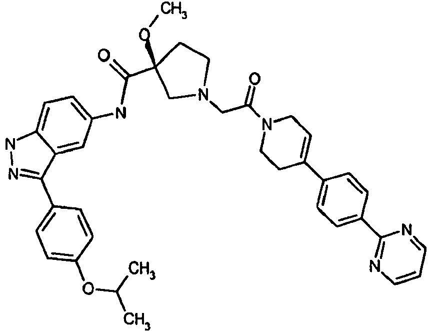 |
| 686 | 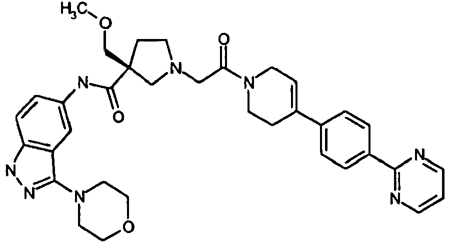 |
| 687 | 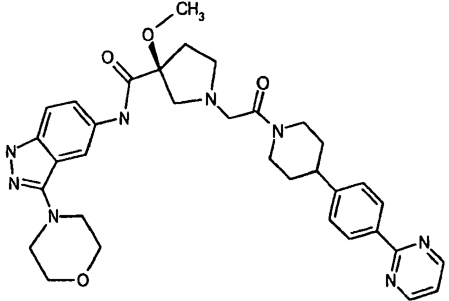 |
| 688 | 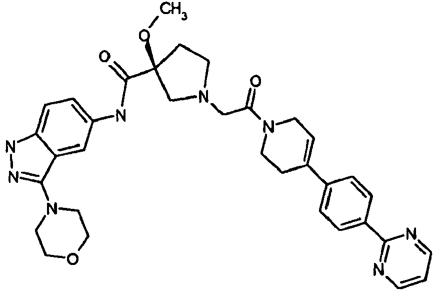 |
| 689 | 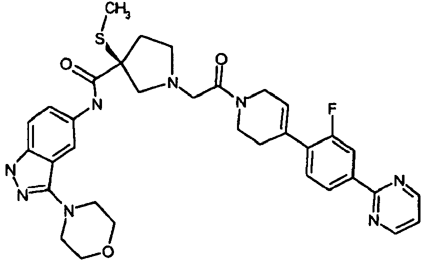 |
| 690 | 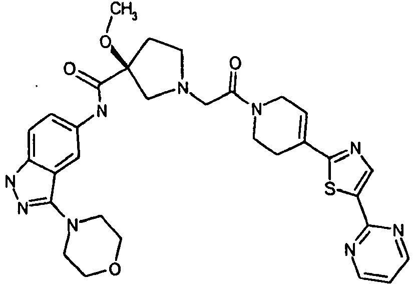 |
| 691 | 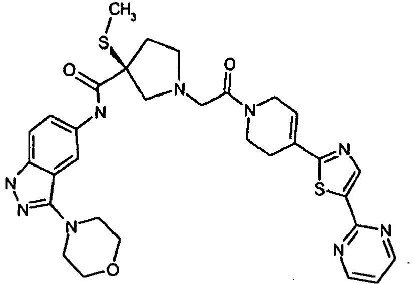 |
| 692 | 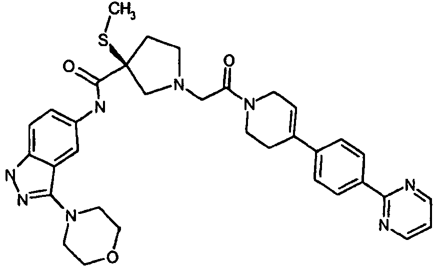 |
| 693 | 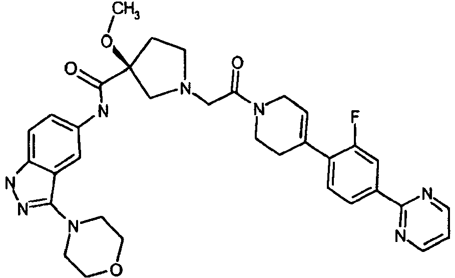 |
| 694 | 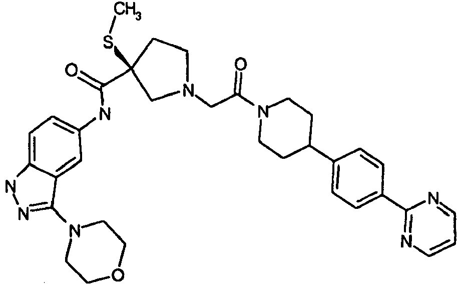 |
| 695 | 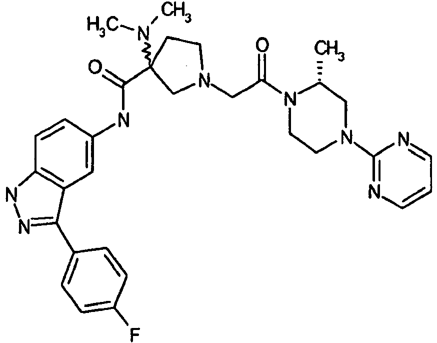 |
| 696 | 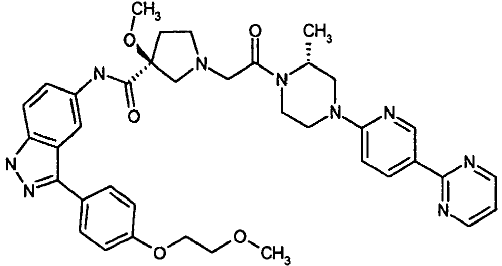 |
| 697 | 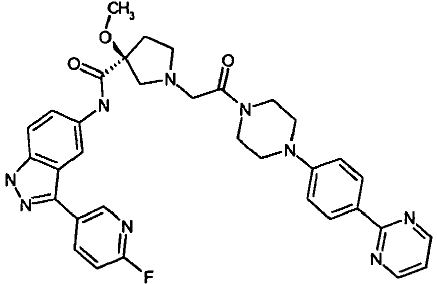 |
| 698 | 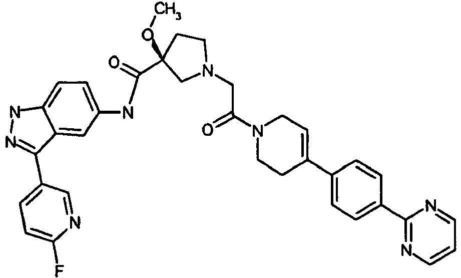 |
| 699 | 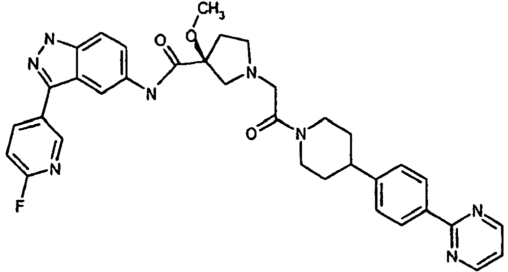 |
| 700 | 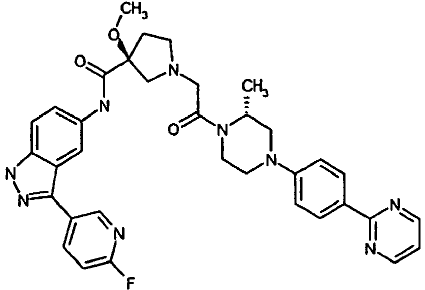 |
| 701 | 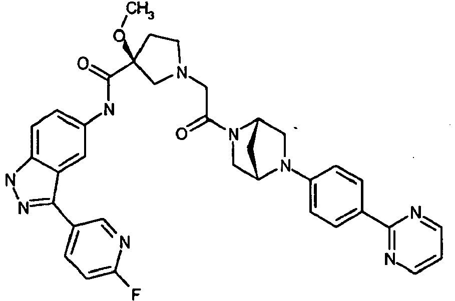 |
| 702 | 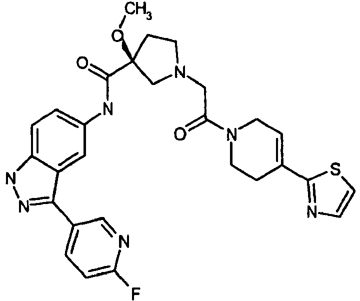 |
| 703 | 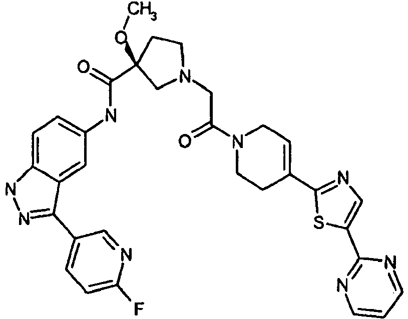 |
| 704 | 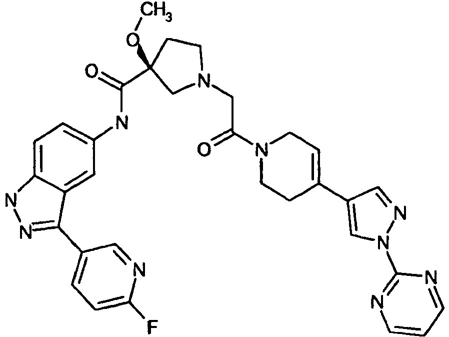 |
| 705 | 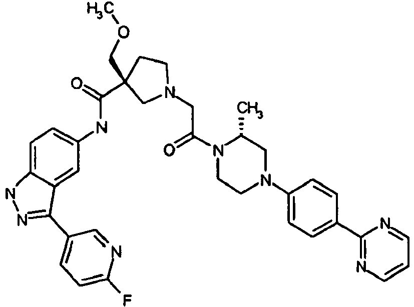 |
| 706 | 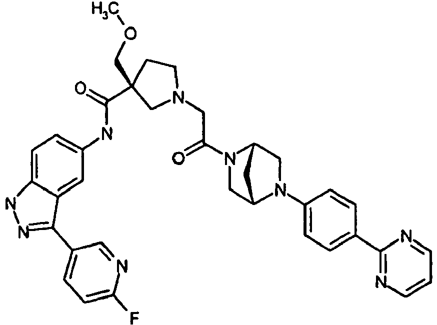 |
| 707 | 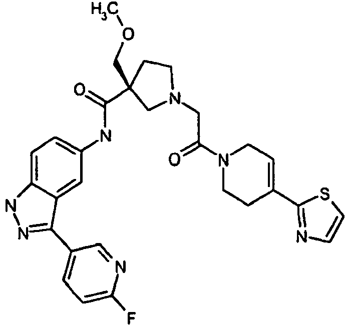 |
| 708 | 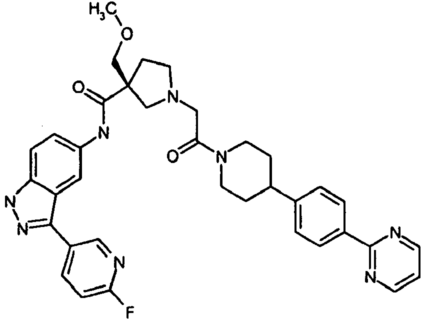 |
| 709 | 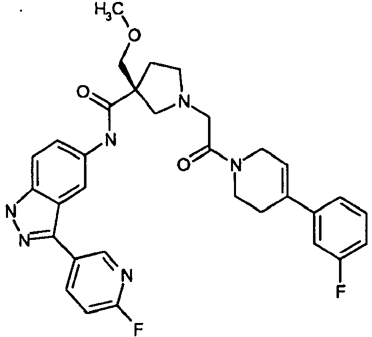 |
| 710 | 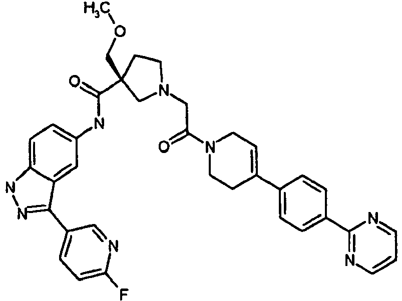 |
| 711 | 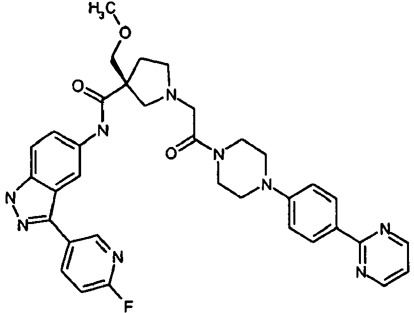 |
| 712 | 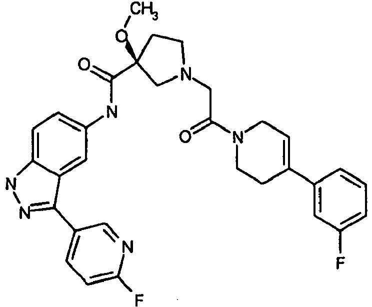 |
| 713 | 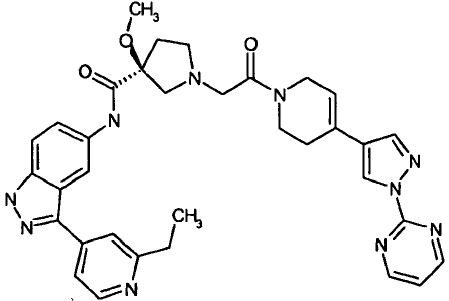 |
| 714 | 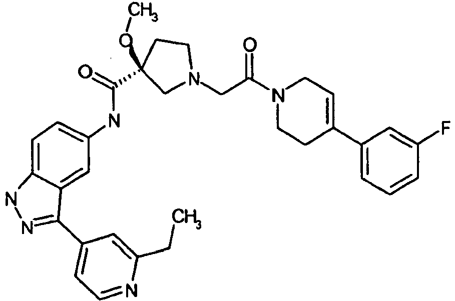 |
| 715 | 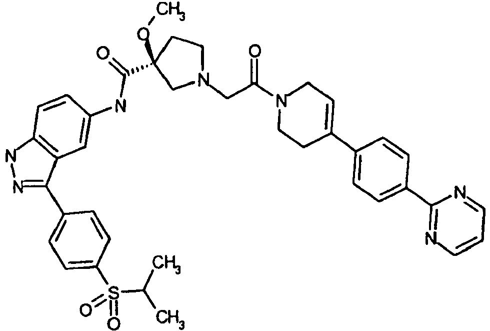 |
| 716 | 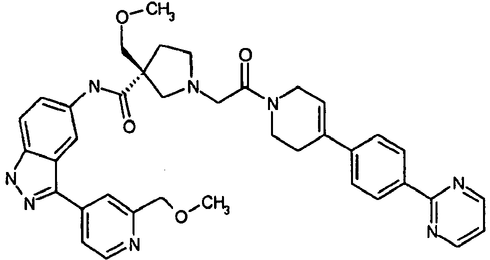 |
| 717 | 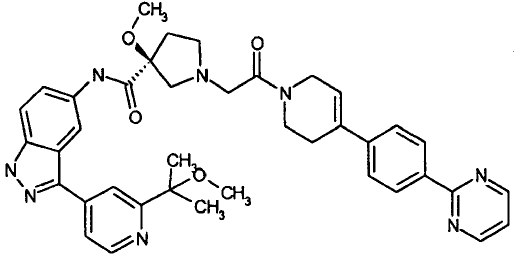 |
| 718 | 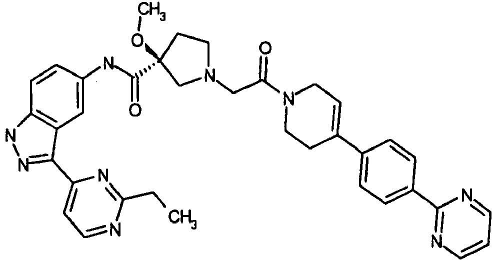 |
| 719 | 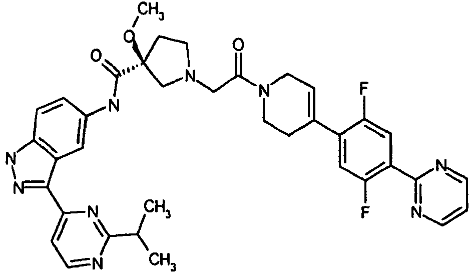 |
| 720 | 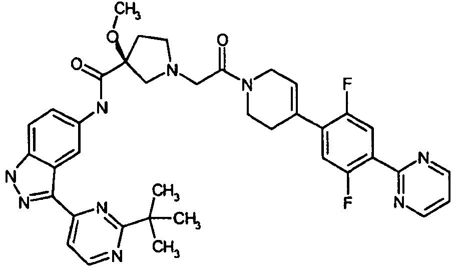 |
| 721 | 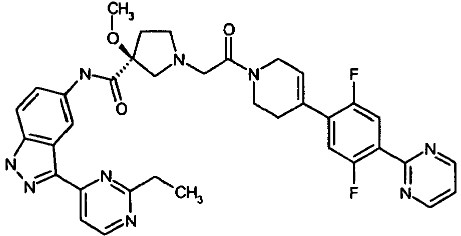 |
| 722 | 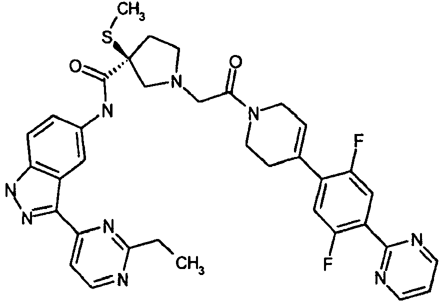 |
| 723 | 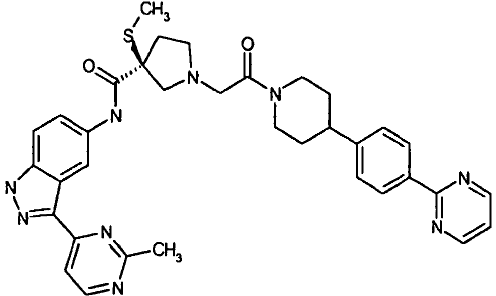 |
| 724 | 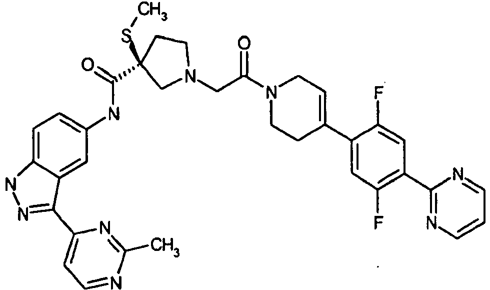 |
| 725 | 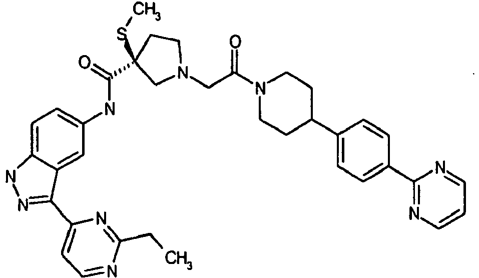 |
| 726 | 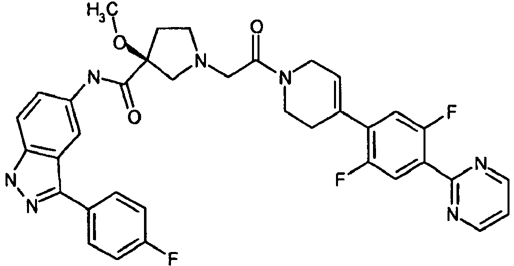 |
| 727 | 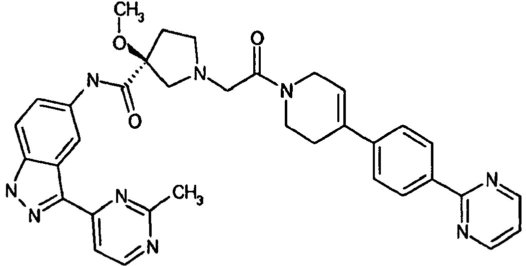 |
| 728 | 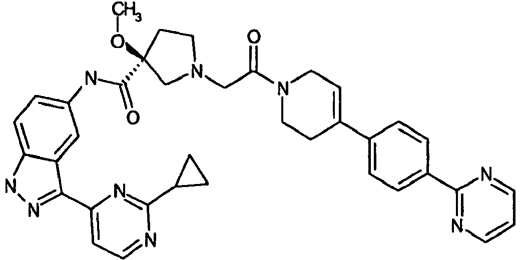 |
| 729 | 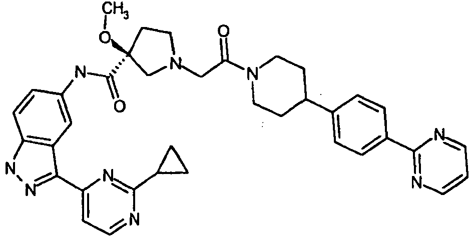 |
| 730 | 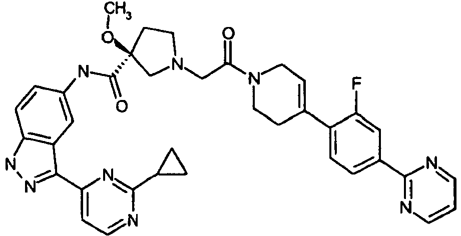 |
| 731 | 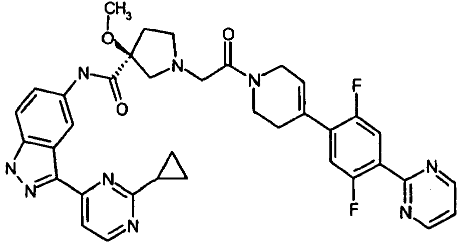 |
| 732 | 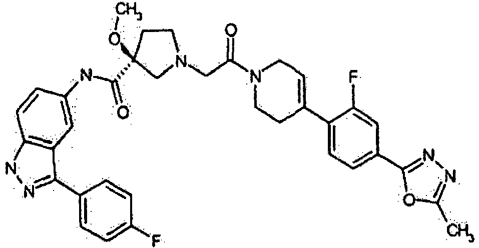 |
| 733 | 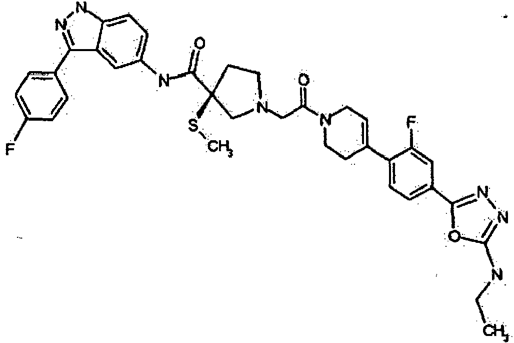 |
| 734 | 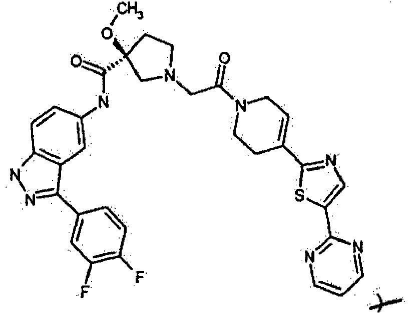 |
| 735 | 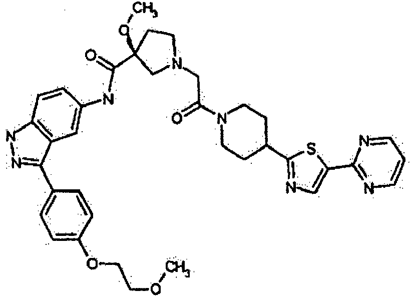 |
| 736 | 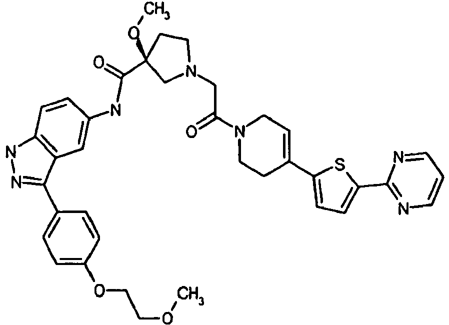 |
| 737 | 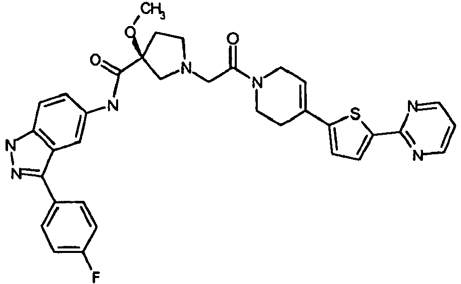 |
| 738 | 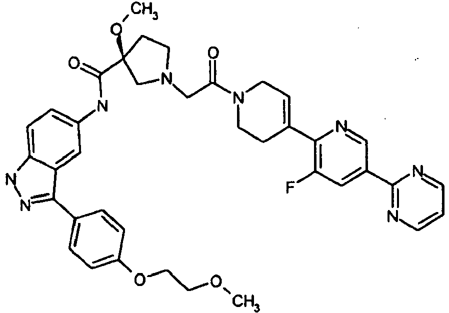 |
| 739 | 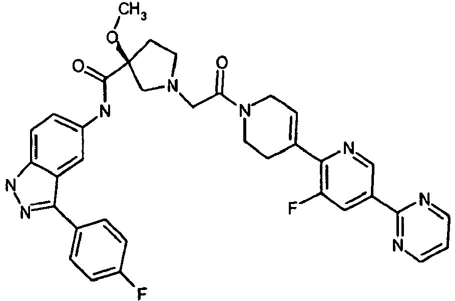 |
| 740 | 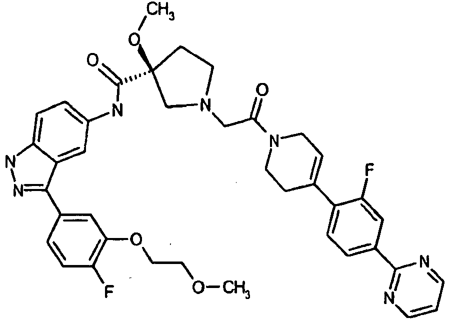 |
| 741 | 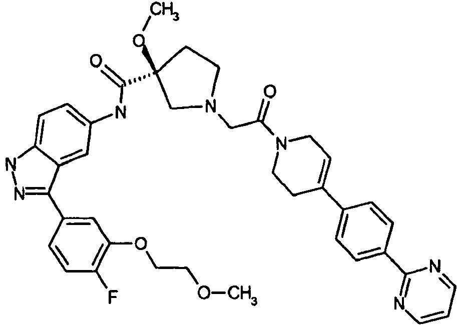 |
| 742 | 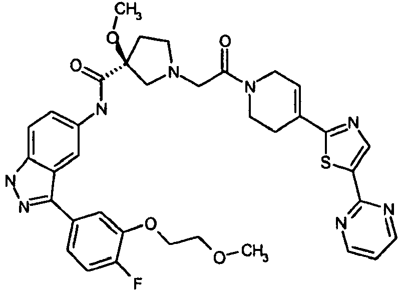 |
| 743 | 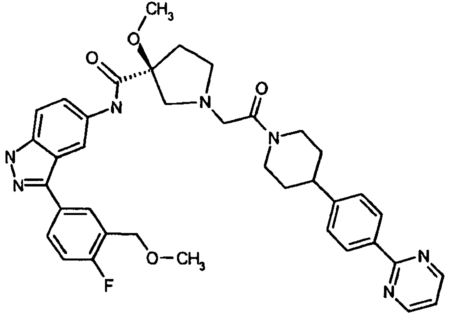 |
| 744 | 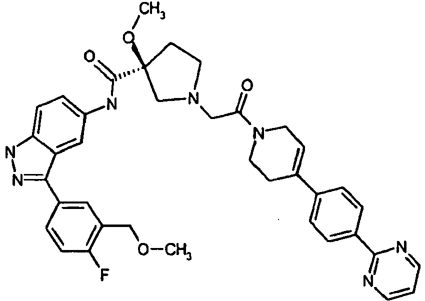 |
| 745 | 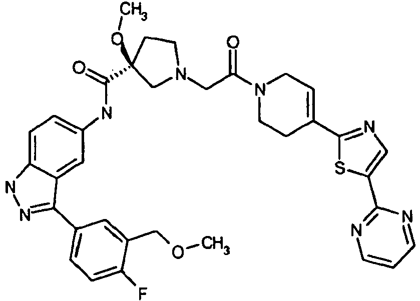 |
| 746 | 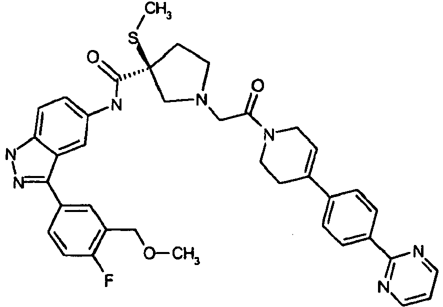 |
| 747 | 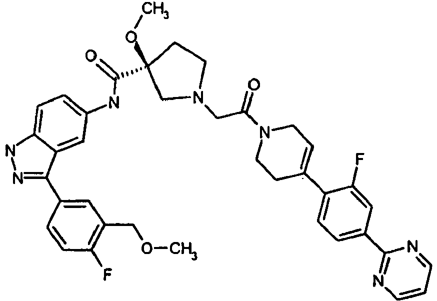 |
| 748 | 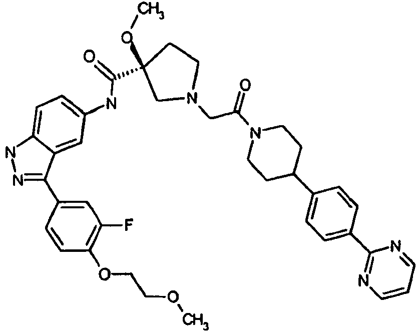 |
| 749 | 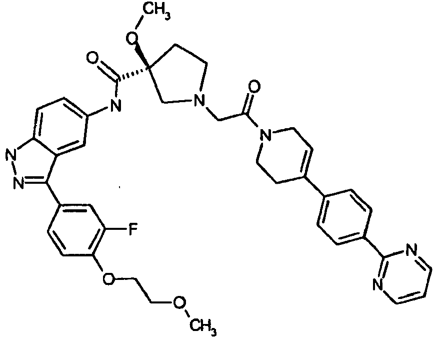 |
| 750 | 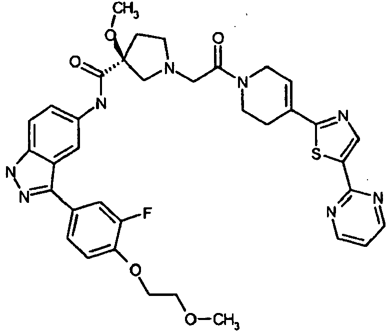 |
| 751 | 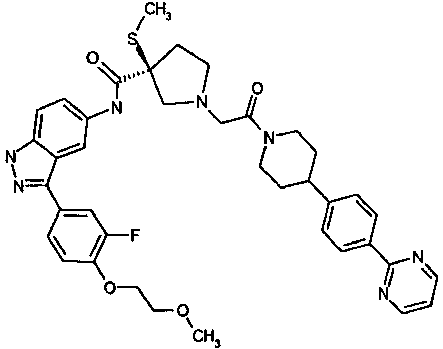 |
| 752 | 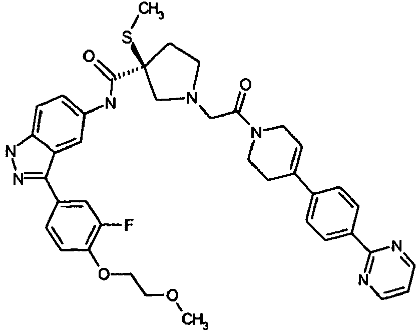 |
| 753 | 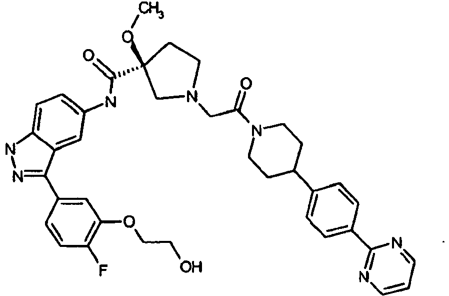 |
| 754 | 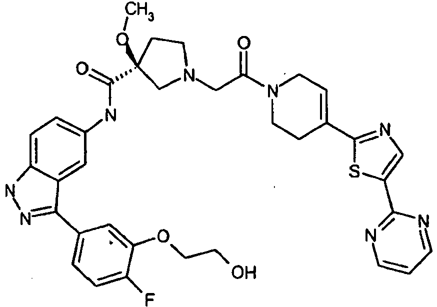 |
| 755 | 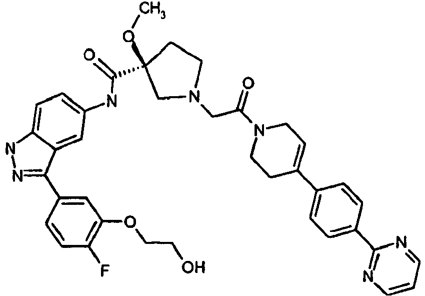 |
| 756 | 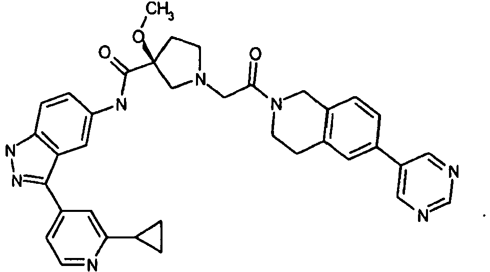 |
| 757 | 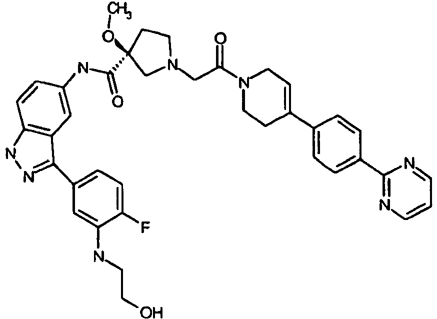 |
| 758 | 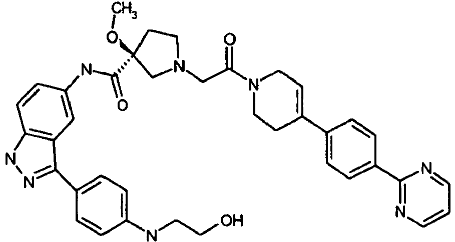 |
| 759 | 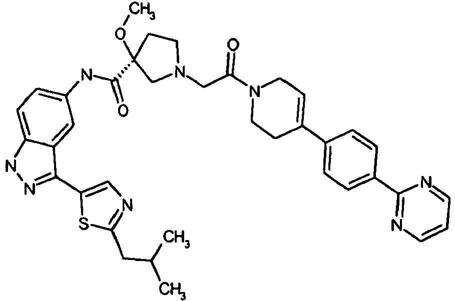 |
| 760 | 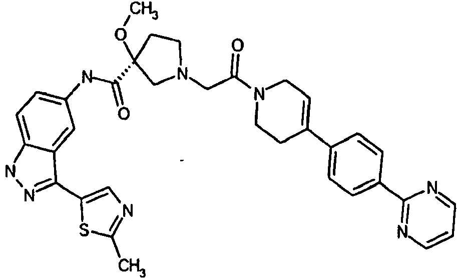 |
| 761 | 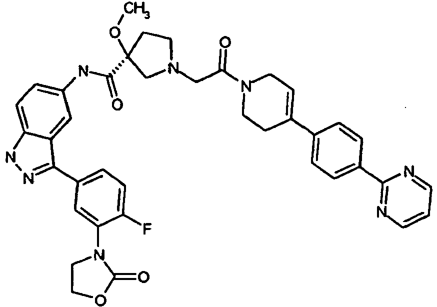 |
| 762 | 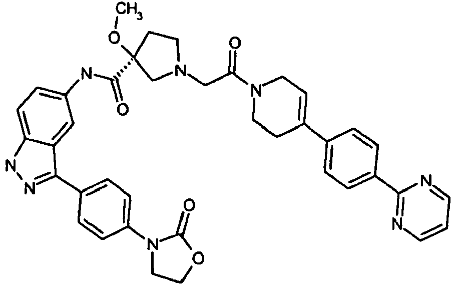 |
| 763 | 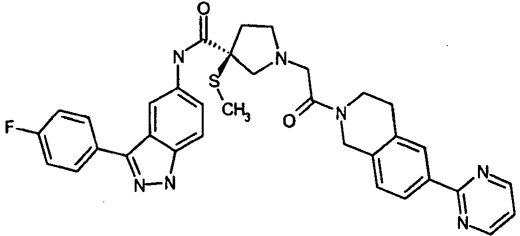 |
| 764 | 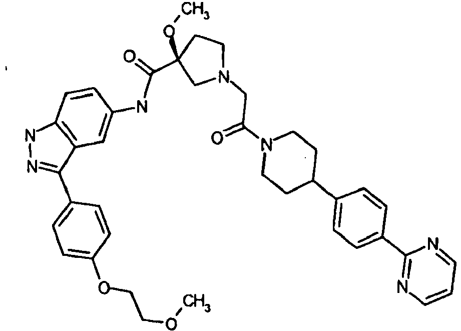 |
| 765 | 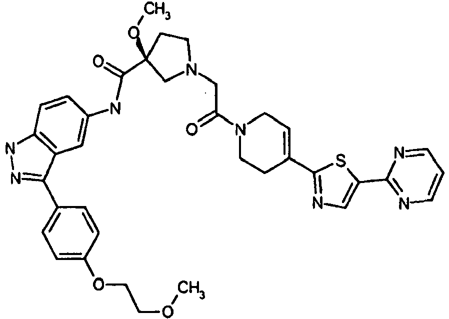 |
| 766 | 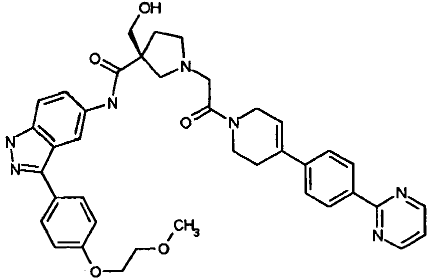 |
| 767 | 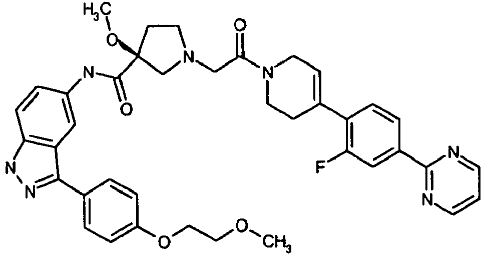 |
| 768 | 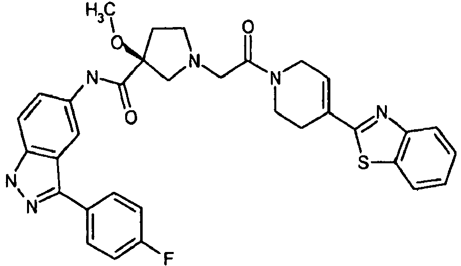 |
| 769 | 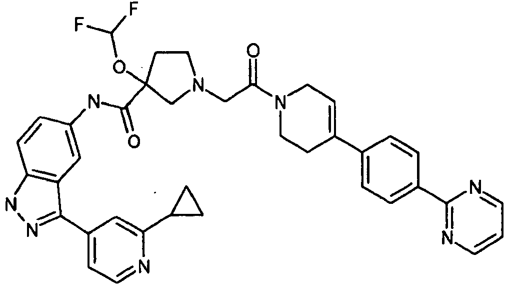 |
| 770 | 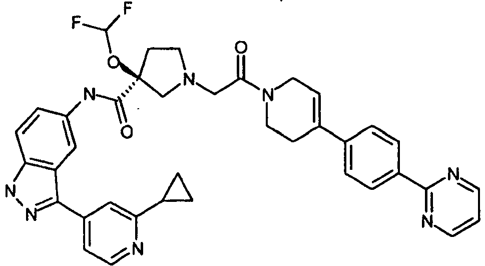 |
| 771 | 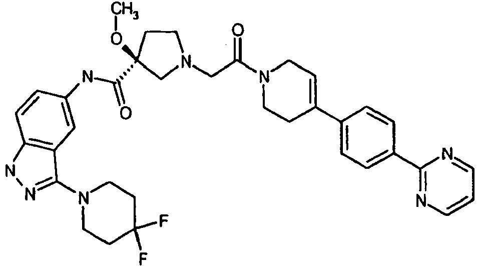 |
| 772 | 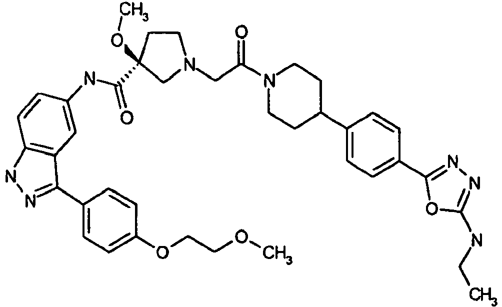 |
| 773 | 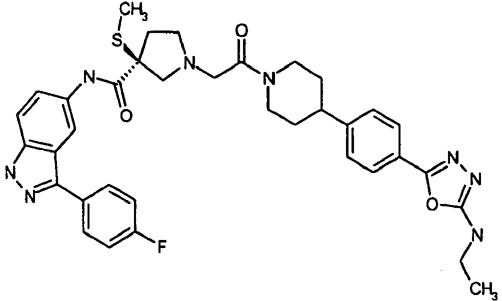 |
| 774 | 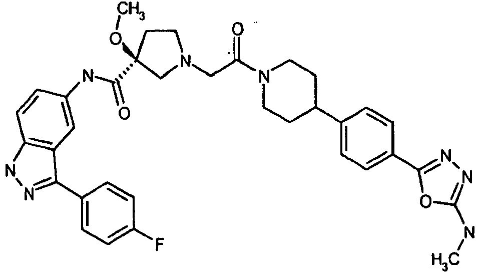 |
| 775 | 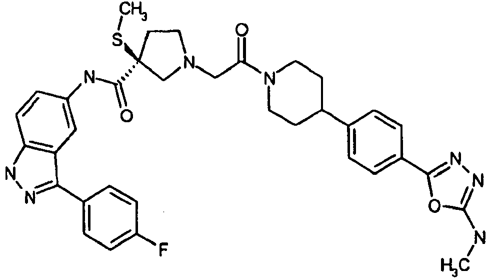 |
| 776 | 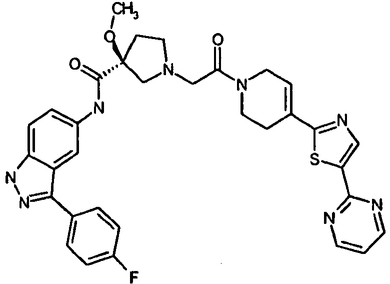 |
| 777 | 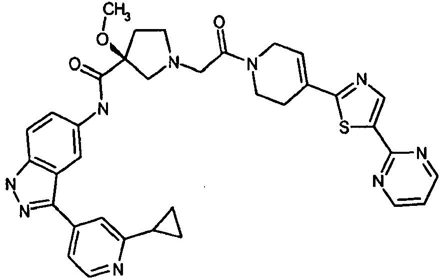 |
| 778 | 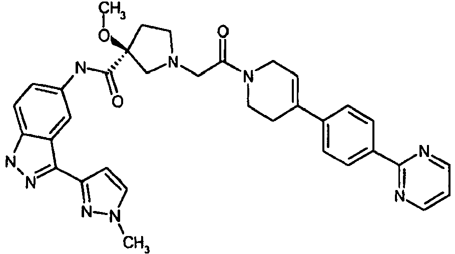 |
| 779 | 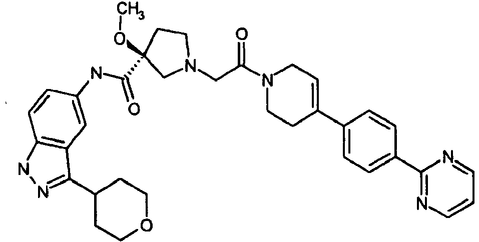 |
| 780 | 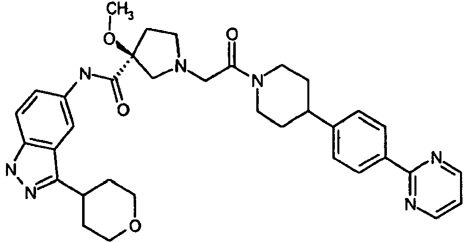 |
| 781 | 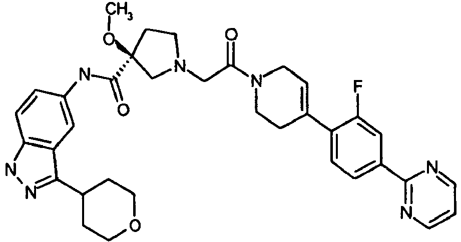 |
| 782 | 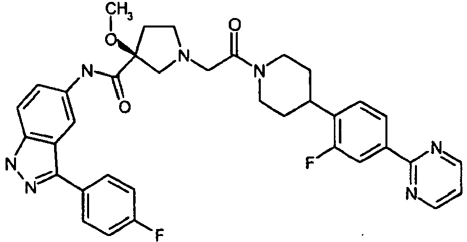 |
| 783 | 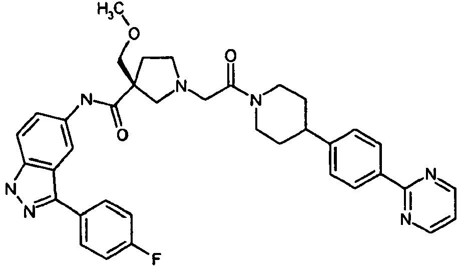 |
| 784 | 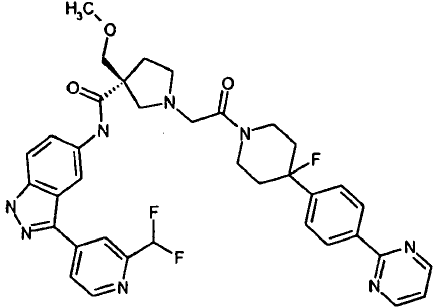 |
| 785 | 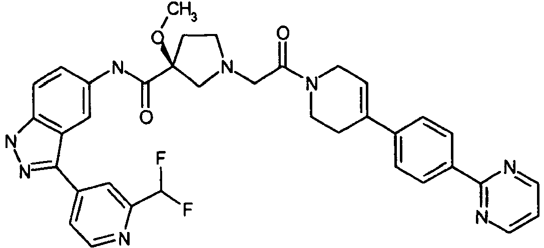 |
| 786 | 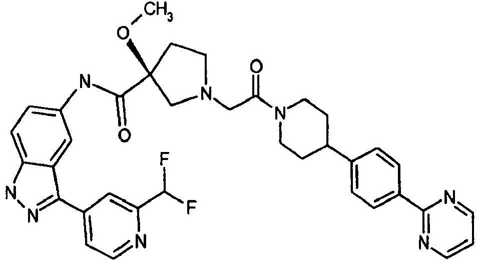 |
| 787 | 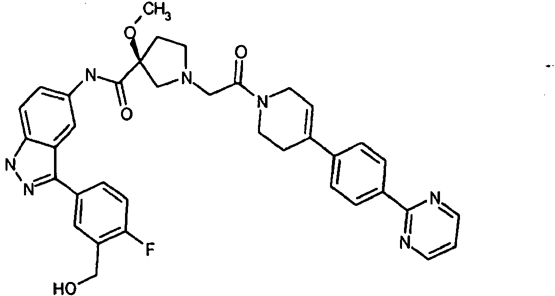 |
| 788 | 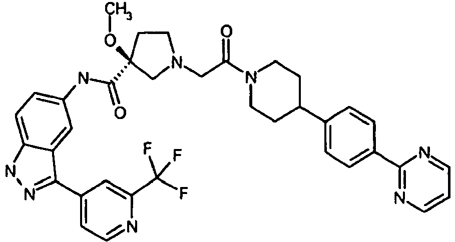 |
| 789 | 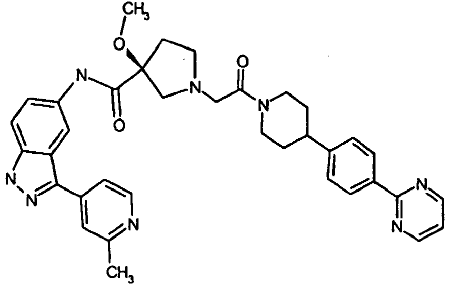 |
| 790 | 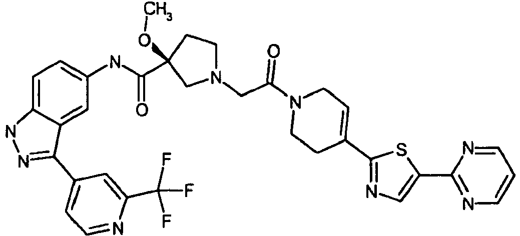 |
| 791 | 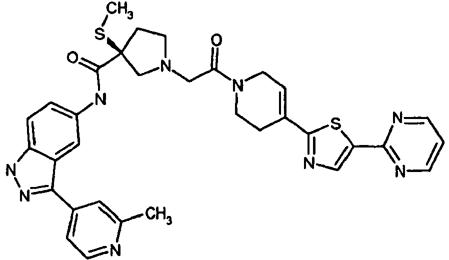 |
| 792 | 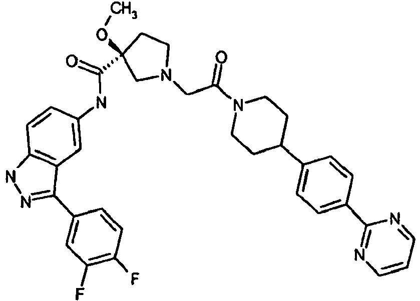 |
| 793 | 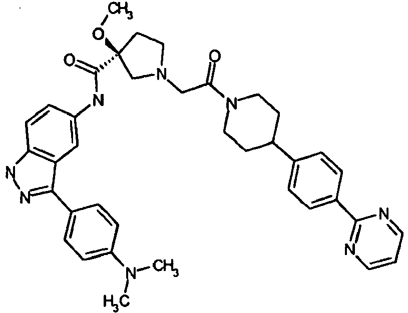 |
| 794 | 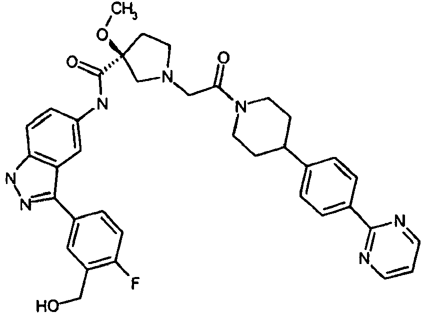 |
| 795 | 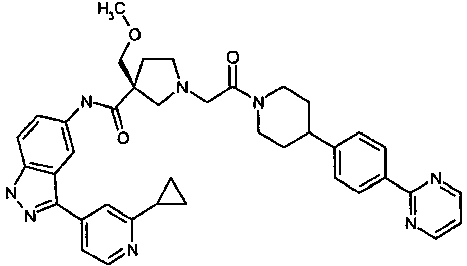 |
| 796 | 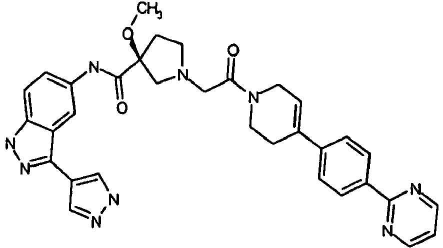 |
| 797 | 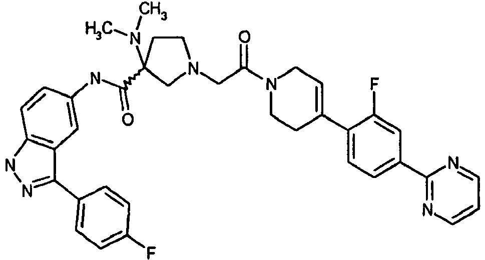 |
| 798 | 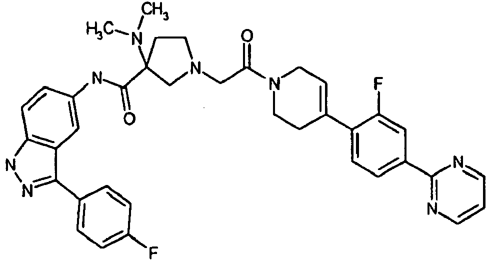 |
| 799 | 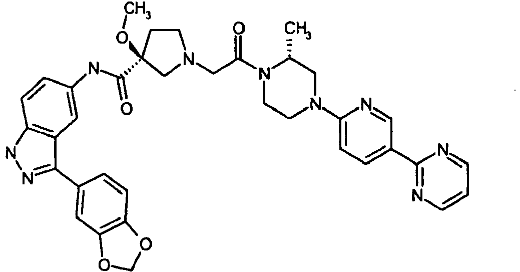 |
| 800 | 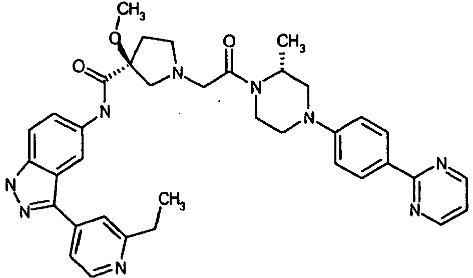 |
| 801 | 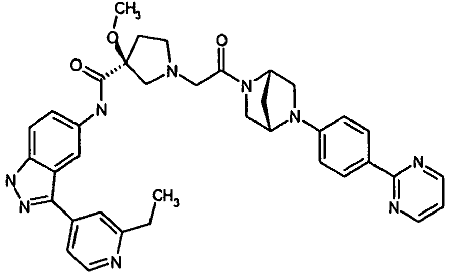 |
| 802 | 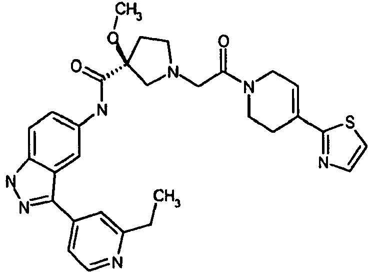 |
| 803 | 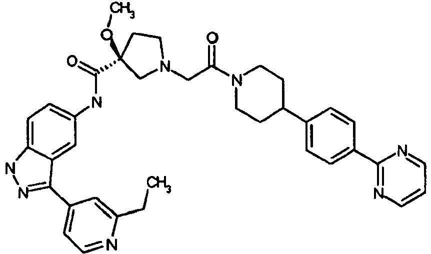 |
| 804 | 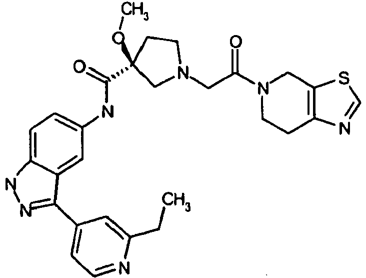 |
| 805 | 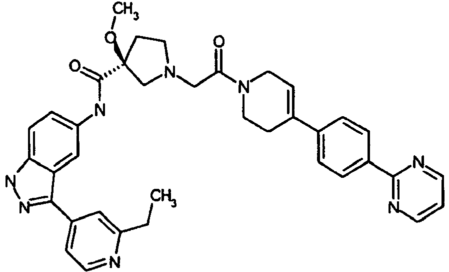 |
| 806 | 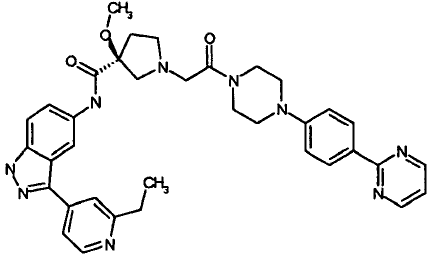 |
| 807 | 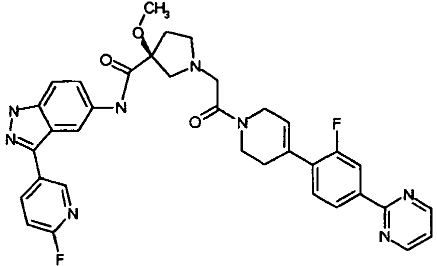 |
| 808 | 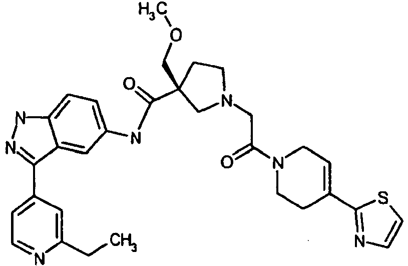 |
| 809 | 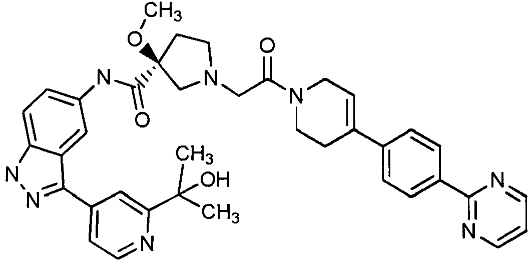 |
| 810 | 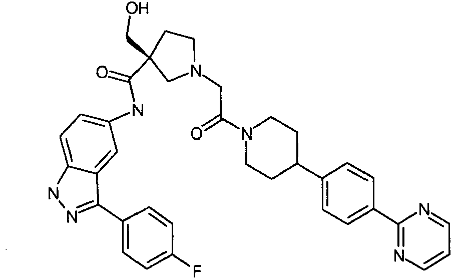 |
| 811 | 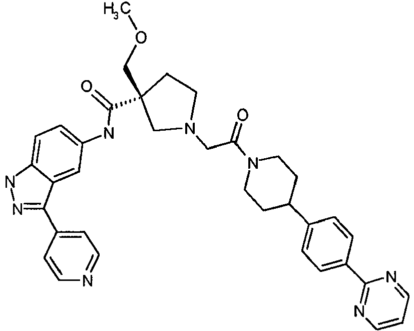 |
| 812 | 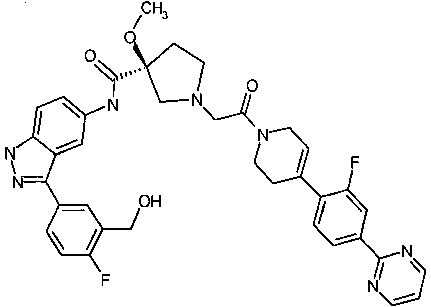 |
| 813 | 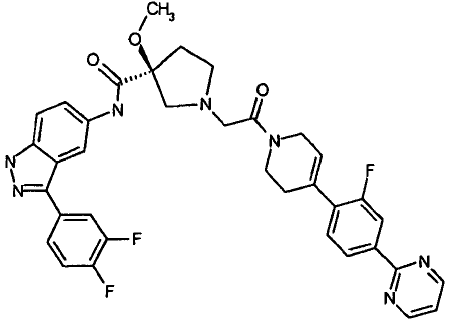 |
| 814 | 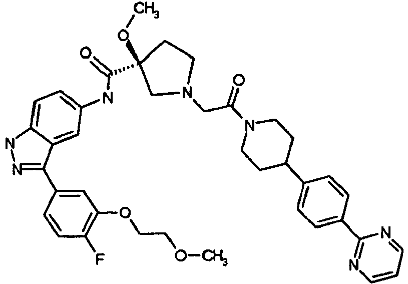 |
| 815 | 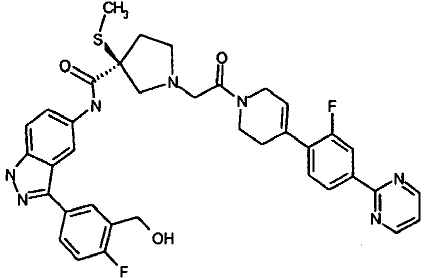 |
| 816 | 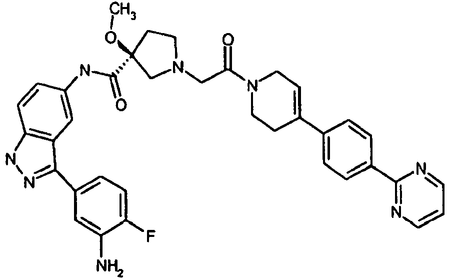 |
| 817 | 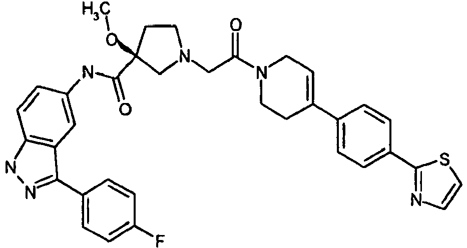 |
| 818 | 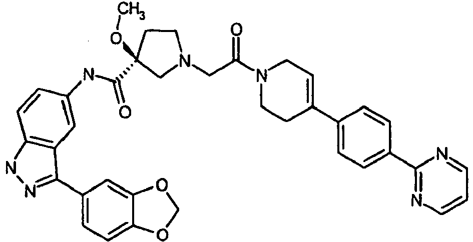 |
| 819 | 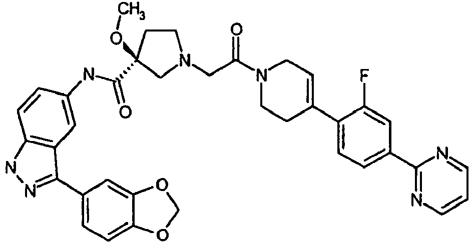 |
| 820 | 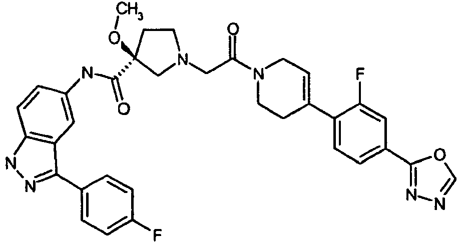 |
| 821 | 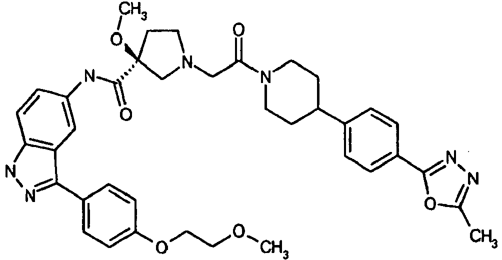 |
| 822 | 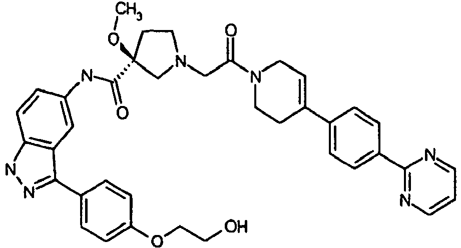 |
| 823 | 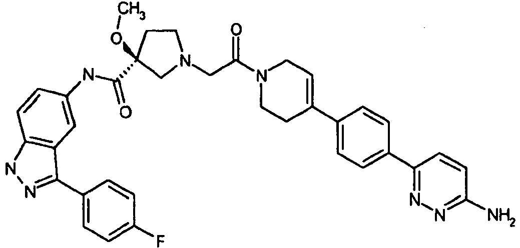 |
| 824 | 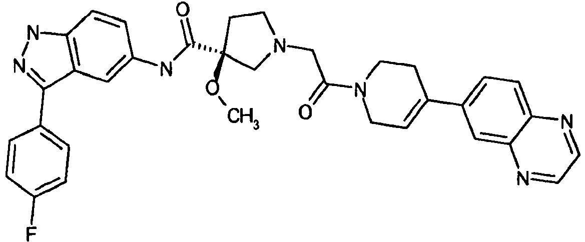 |
| 826 | 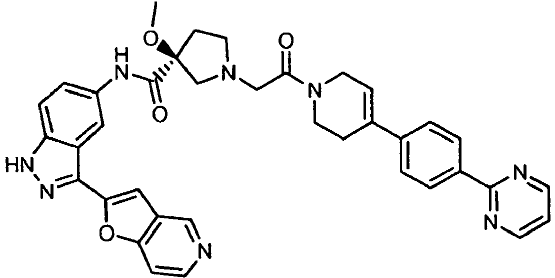 |
| 827 | 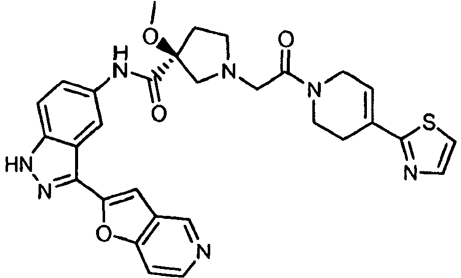 |

or a pharmaceutically acceptable salt thereof.

This invention provides the claimed compounds in pure or isolated form.

This invention provides the claimed compounds in pure form.

This invention provides the claimed compounds in isolated form.

This invention provides pharmaceutically acceptable salts of the claimed compounds.

This invention provides pharmaceutically acceptable esters of the claimed compounds.

This invention provides solvates of the claimed compounds.

This invention provides the final compounds of Examples 612 to 824, 826 and 827.

This invention also provides a pharmaceutical composition comprising an effective amount of at least one (e.g., 1, 2 or 3, 1 or 2, and usually 1) claimed compound and a pharmaceutically acceptable carrier.

This invention also provides a pharmaceutical composition comprising an effective amount of at least one (e.g., 1, 2 or 3, 1 or 2, and usually 1) claimed compound and an effective amount of at least one other (e.g., 1, 2 or 3, 1 or 2, and usually 1) pharmaceutically active ingredient (such as, for example, a chemotherapeutic agent), and a pharmaceutically acceptable carrier.

Disclosed is a method of inhibiting ERK (i.e., inhibiting the activity of ERK) in a patient in need of such treatment comprising administering to said patient an effective amount of at least one (e.g., 1, 2 or 3, 1 or 2, and usually 1) claimed compound.

Also disclosed is a method of inhibiting ERK1 (i.e., inhibiting the activity of ERK1) in a patient in need of such treatment comprising administering to said patient an effective amount of at least one (e.g., 1, 2 or 3, 1 or 2, and usually 1) claimed compound.

Further disclosed is a method of inhibiting ERK2 (i.e., inhibiting the activity of ERK2) in a patient in need of such treatment comprising administering to said patient an effective amount of at least one (e.g., 1, 2 or 3, 1 or 2, and usually 1) claimed compound.

Also disclosed is a method of inhibiting ERK1 and ERK2 (i.e., inhibiting the activity of ERK1 and ERK2) in a patient in need of such treatment comprising administering to said patient an effective amount of at least one (e.g., 1, 2 or 3, 1 or 2, and usually 1) claimed compound.

Further disclosed is a method for treating cancer in a patient in need of such treatment, said method comprising administering to said patient an effective amount of at least one (e.g., 1, 2 or 3, 1 or 2, and usually 1) claimed compound.

Also disclosed is a method for treating cancer in a patient in need of such treatment, said method comprising administering to said patient an effective amount of a pharmaceutical composition comprising an effective amount of at least one (e.g., 1, 2 or 3, 1 or 2, and usually 1) claimed compound.

Further disclosed is a method for treating cancer in a patient in need of such treatment, said method comprising administering to said patient an effective amount of at least one (e.g., 1, 2 or 3, 1 or 2, and usually 1) claimed compound in combination with an effective amount of at least one (e.g., 1, 2 or 3, 1 or 2, or 1) chemotherapeutic agent.

Also disclosed is a method for treating cancer in a patient in need of such treatment, said method comprising administering to said patient an effective amount of a pharmaceutical composition comprising an effective amount of at least one (e.g., 1, 2 or 3, 1 or 2, and usually 1) claimed compound in combination with an effective amount of at least one (e.g., 1, 2 or 3, 1 or 2, or 1) chemotherapeutic agent.

Further disclosed is a method of treating cancer in a patient in need of such treatment, said method comprising administering to said patient an effective amount of at least one (e.g., 1, 2 or 3, 1 or 2, and usually 1) claimed compound in combination with at least one (e.g., 1, 2 or 3, 1 or 2, and usually 1) signal transduction inhibitor.

Also disclosed is a method of treating cancer in a patient in need of such treatment, said method comprising administering to said patient an effective amount of a pharmaceutical composition comprising an effective amount of at least one (e.g., 1, 2 or 3, 1 or 2, and usually 1) claimed compound in combination with at least one (e.g., 1, 2 or 3, 1 or 2, and usually 1) signal transduction inhibitor.

Further disclosed is a method for treating lung cancer, pancreatic cancer, colon cancer (e.g., colorectal cancer), myeloid leukemias (e.g., AML, CML, and CMML), thyroid cancer, myelodysplastic syndrome (MDS), bladder carcinoma, epidermal carcinoma, melanoma, breast cancer, prostate cancer, head and neck cancers (e.g., squamous cell cancer of the head and neck), ovarian cancer, brain cancers (e.g., gliomas, such as glioma blastoma multiforme), cancers of mesenchymal origin (e.g., fibrosarcomas and rhabdomyosarcomas), sarcomas, tetracarcinomas, nuroblastomas, kidney carcinomas, hepatomas, non-Hodgkin's lymphoma, multiple myeloma, or anaplastic thyroid carcinoma, in a patient in need of such treatment, said method comprising administering to said patient an effective amount of at least one (e.g., 1, 2 or 3, 1 or 2, and usually 1) claimed compound.

Also disclosed is a method for treating lung cancer, pancreatic cancer, colon cancer (e.g., colorectal cancer), myeloid leukemias (e.g., AML, CML, and CMML), thyroid cancer, myelodysplastic syndrome (MDS), bladder carcinoma, epidermal carcinoma, melanoma, breast cancer, prostate cancer, head and neck cancers (e.g., squamous cell cancer of the head and neck), ovarian cancer, brain cancers (e.g., gliomas, such as glioma blastoma multiforme), cancers of mesenchymal origin (e.g., fibrosarcomas and rhabdomyosarcomas), sarcomas, tetracarcinomas, nuroblastomas, kidney carcinomas, hepatomas, non-Hodgkin's lymphoma, multiple myeloma, or anaplastic thyroid carcinoma in a patient in need of such treatment, said method comprising administering to said patient an effective amount of at least one (e.g., 1, 2 or 3, 1 or 2, and usually 1) claimed compound in combination with an effective amount of at least one (e.g., 1, 2 or 3, 1 or 2, or 1) chemotherapeutic agent.

Further disclosed is a method for treating lung cancer, pancreatic cancer, colon cancer (e.g., colorectal cancer), myeloid leukemias (e.g., AML, CML, and CMML), thyroid cancer, myelodysplastic syndrome (MDS), bladder carcinoma, epidermal carcinoma, melanoma, breast cancer, prostate cancer, head and neck cancers (e.g., squamous cell cancer of the head and neck), ovarian cancer, brain cancers (e.g., gliomas, such as glioma blastoma multiforme), cancers of mesenchymal origin (e.g., fibrosarcomas and rhabdomyosarcomas), sarcomas, tetracarcinomas, nuroblastomas, kidney carcinomas, hepatomas, non-Hodgkin's lymphoma, multiple myeloma, or anaplastic thyroid carcinoma in a patient in need of such treatment, said method comprising administering to said patient an effective amount of a pharmaceutical composition comprising an effective amount of at least one (e.g., 1, 2 or 3, 1 or 2, and usually 1) claimed compound.

Also disclosed is a method for treating lung cancer, pancreatic cancer, colon cancer (e.g., colorectal cancer), myeloid leukemias (e.g., AML, CML, and CMML), thyroid cancer, myelodysplastic syndrome (MDS), bladder carcinoma, epidermal carcinoma, melanoma, breast cancer, prostate cancer, head and neck cancers (e.g., squamous cell cancer of the head and neck), ovarian cancer, brain cancers (e.g., gliomas, such as glioma blastoma multiforme), cancers of mesenchymal origin (e.g., fibrosarcomas and rhabdomyosarcomas), sarcomas, tetracarcinomas, nuroblastomas, kidney carcinomas, hepatomas, non-Hodgkin's lymphoma, multiple myeloma, or anaplastic thyroid carcinoma in a patient in need of such treatment, said method comprising administering to said patient an effective amount of a pharmaceutical composition comprising an effective amount of at least one (e.g., 1, 2 or 3, 1 or 2, and usually 1) claimed compound, in combination with an effective amount of at least one (e.g., 1, 2 or 3, 1 or 2, or 1) chemotherapeutic agent.

Further disclosed is a method for treating cancer in a patient in need of such treatment, said method comprising administering to said patient an effective amount of at least one (e.g., 1, 2 or 3, 1 or 2, and usually 1) claimed compound, wherein said cancer is selected from the group consisting of: melanoma, pancreatic cancer, thryroid cancer, colorectal cancer, lung cancer, breast cancer, and ovarian cancer.

Also disclosed is a method for treating cancer in a patient in need of such treatment, said method comprising administering to said patient an effective amount of at least one (e.g., 1, 2 or 3, 1 or 2, and usually 1) claimed compound, in combination with an effective amount of at least one (e.g., 1, 2 or 3, 1 or 2, or 1) chemotherapeutic agent wherein said cancer is selected from the group consisting of: melanoma, pancreatic cancer, thryroid cancer, colorectal cancer, lung cancer, breast cancer, and ovarian cancer.

Further disclosed is a method for treating cancer in a patient in need of such treatment, said method comprising administering to said patient an effective amount of a pharmaceutical composition comprising an effective amount of at least one (e.g., 1, 2 or 3, 1 or 2, and usually 1) claimed compound, wherein said cancer is selected from the group consisting of: melanoma, pancreatic cancer, thryroid cancer, colorectal cancer, lung cancer, breast cancer, and ovarian cancer.

Also disclosed is a method for treating cancer in a patient in need of such treatment, said method comprising administering to said patient an effective amount of a pharmaceutical composition comprising an effective amount of at least one (e.g., 1, 2 or 3, 1 or 2, and usually 1) claimed compound, in combination with an effective amount of at least one (e.g., 1, 2 or 3, 1 or 2, or 1) chemotherapeutic agent wherein said cancer is selected from the group consisting of: melanoma, pancreatic cancer, thryroid cancer, colorectal cancer, lung cancer, breast cancer, and ovarian cancer.

Further disclosed is a method for treating melanoma in a patient in need of such treatment, said method comprising administering to said patient an effective amount of at least one (e.g., 1, 2 or 3, 1 or 2, and usually 1) claimed compound.

Also disclosed is a method for treating melanoma in a patient in need of such treatment, said method comprising administering to said patient an effective amount of at least one (e.g., 1, 2 or 3, 1 or 2, and usually 1) claimed compound, in combination with an effective amount of at least one (e.g., 1, 2 or 3, 1 or 2, or 1) chemotherapeutic agent.

Further disclosed is a method for treating melanoma in a patient in need of such treatment, said method comprising administering to said patient an effective amount of a pharmaceutical composition comprising an effective amount of at least one (e.g., 1, 2 or 3, 1 or 2, and usually 1) claimed compound.

Also disclosed is a method for treating melanoma in a patient in need of such treatment, said method comprising administering to said patient an effective amount of a pharmaceutical composition comprising an effective amount of at least one (e.g., 1, 2 or 3, 1 or 2, and usually 1) claimed compound, in combination with an effective amount of at least one (e.g., 1, 2 or 3, 1 or 2, or 1) chemotherapeutic agent.

Further disclosed is a method for treating pancreatic cancer in a patient in need of such treatment, said method comprising administering to said patient an effective amount of at least one (e.g., 1, 2 or 3, 1 or 2, and usually 1) claimed compound.

Also disclosed is a method for treating pancreatic cancer in a patient in need of such treatment, said method comprising administering to said patient an effective amount of at least one (e.g., 1, 2 or 3, 1 or 2, and usually 1) claimed compound, in combination with an effective amount of at least one (e.g., 1, 2 or 3, 1 or 2, or 1) chemotherapeutic agent.

Further disclosed is a method for treating pancreatic cancer in a patient in need of such treatment, said method comprising administering to said patient an effective amount of a pharmaceutical composition comprising an effective amount of at least one (e.g., 1, 2 or 3, 1 or 2, and usually 1) claimed compound.

Also disclosed is a method for treating pancreatic cancer in a patient in need of such treatment, said method comprising administering to said patient an effective amount of a pharmaceutical composition comprising an effective amount of at least one (e.g., 1, 2 or 3, 1 or 2, and usually 1) claimed compound, in combination with an effective amount of at least one (e.g., 1, 2 or 3, 1 or 2, or 1) chemotherapeutic agent.

Further disclosed is a method for treating thyroid cancer in a patient in need of such treatment, said method comprising administering to said patient an effective amount of at least one (e.g., 1, 2 or 3, 1 or 2, and usually 1) claimed compound.

Also disclosed is a method for treating thyroid cancer in a patient in need of such treatment, said method comprising administering to said patient an effective amount of at least one (e.g., 1, 2 or 3, 1 or 2, and usually 1) claimed compound, in combination with an effective amount of at least one (e.g., 1, 2 or 3, 1 or 2, or 1) chemotherapeutic agent.

Further disclosed is a method for treating thyroid cancer in a patient in need of such treatment, said method comprising administering to said patient an effective amount of a pharmaceutical composition comprising an effective amount of at least one (e.g., 1, 2 or 3, 1 or 2, and usually 1) claimed compound.

Also disclosed is a method for treating thyroid cancer in a patient in need of such treatment, said method comprising administering to said patient an effective amount of a pharmaceutical composition comprising an effective amount of at least one (e.g., 1, 2 or 3, 1 or 2, and usually 1) claimed compound, in combination with an effective amount of at least one (e.g., 1, 2 or 3, 1 or 2, or 1) chemotherapeutic agent.

Further disclosed is a method for treating colorectal cancer in a patient in need of such treatment, said method comprising administering to said patient an effective amount of at least one (e.g., 1, 2 or 3, 1 or 2, and usually 1) claimed compound.

Also disclosed is a method for treating colorectal cancer in a patient in need of such treatment, said method comprising administering to said patient an effective amount of at least one (e.g., 1, 2 or 3, 1 or 2, and usually 1) claimed compound, in combination with an effective amount of at least one (e.g., 1, 2 or 3, 1 or 2, or 1) chemotherapeutic agent.

Further disclosed is a method for treating colorectal cancer in a patient in need of such treatment, said method comprising administering to said patient an effective amount of a pharmaceutical composition comprising an effective amount of at least one (e.g., 1, 2 or 3, 1 or 2, and usually 1) claimed compound.

Also disclosed is a method for treating colorectal cancer in a patient in need of such treatment, said method comprising administering to said patient an effective amount of a pharmaceutical composition comprising an effective amount of at least one (e.g., 1, 2 or 3, 1 or 2, and usually 1) claimed compound, in combination with an effective amount of at least one (e.g., 1, 2 or 3, 1 or 2, or 1) chemotherapeutic agent.

Further disclosed is a method for treating lung cancer in a patient in need of such treatment, said method comprising administering to said patient an effective amount of at least one (e.g., 1, 2 or 3, 1 or 2, and usually 1) claimed compound.

Also enclosed is a method for treating lung cancer in a patient in need of such treatment, said method comprising administering to said patient an effective amount of at least one (e.g., 1, 2 or 3, 1 or 2, and usually 1) claimed compound, in combination with an effective amount of at least one (e.g., 1, 2 or 3, 1 or 2, or 1) chemotherapeutic agent.

Further disclosed is a method for treating lung cancer in a patient in need of such treatment, said method comprising administering to said patient an effective amount of a pharmaceutical composition comprising an effective amount of at least one (e.g., 1, 2 or 3, 1 or 2, and usually 1) claimed compound.

Also disclosed is a method for treating lung cancer in a patient in need of such treatment, said method comprising administering to said patient an effective amount of a pharmaceutical composition comprising an effective amount of at least one (e.g., 1, 2 or 3, 1 or 2, and usually 1) claimed compound, in combination with an effective amount of at least one (e.g., 1, 2 or 3, 1 or 2, or 1) chemotherapeutic agent.

Further disclosed is a method for treating breast cancer in a patient in need of such treatment, said method comprising administering to said patient an effective amount of at least one (e.g., 1, 2 or 3, 1 or 2, and usually 1) claimed compound.

Also disclosed is a method for treating breast cancer in a patient in need of such treatment, said method comprising administering to said patient an effective amount of at least one (e.g., 1, 2 or 3, 1 or 2, and usually 1) claimed compound, in combination with an effective amount of at least one (e.g., 1, 2 or 3, 1 or 2, or 1) chemotherapeutic agent.

Further disclosed is a method for treating breast cancer in a patient in need of such treatment, said method comprising administering to said patient an effective amount of a pharmaceutical composition comprising an effective amount of at least one (e.g., 1, 2 or 3, 1 or 2, and usually 1) claimed compound.

Also enclosed is a method for treating breast cancer in a patient in need of such treatment, said method comprising administering to said patient an effective amount of a pharmaceutical composition comprising an effective amount of at least one (e.g., 1, 2 or 3, 1 or 2, and usually 1) claimed compound, in combination with an effective amount of at least one (e.g., 1, 2 or 3, 1 or 2, or 1) chemotherapeutic agent.

Further disclosed is a method for treating ovarian cancer in a patient in need of such treatment, said method comprising administering to said patient an effective amount of at least one (e.g., 1, 2 or 3, 1 or 2, and usually 1) claimed compound.

Also disclosed is a method for treating ovarian cancer in a patient in need of such treatment, said method comprising administering to said patient an effective amount of at least one (e.g., 1, 2 or 3, 1 or 2, and usually 1) claimed compound, in combination with an effective amount of at least one (e.g., 1, 2 or 3, 1 or 2, or 1) chemotherapeutic agent.

Further disclosed is a method for treating ovarian cancer in a patient in need of such treatment, said method comprising administering to said patient an effective amount of a pharmaceutical composition comprising an effective amount of at least one (e.g., 1, 2 or 3, 1 or 2, and usually 1) claimed compound.

Also disclosed is a method for treating ovarian cancer in a patient in need of such treatment, said method comprising administering to said patient an effective amount of a pharmaceutical composition comprising an effective amount of at least one (e.g., 1, 2 or 3, 1 or 2, and usually 1) claimed compound, in combination with an effective amount of at least one (e.g., 1, 2 or 3, 1 or 2, or 1) chemotherapeutic agent.

Further disclosed are methods of treating breast cancer (i.e., post-menopausal and premenopausal breast cancer, e.g., hormone-dependent breast cancer) in a patient in need of such treatment, said treatment comprising the administration of an effective amount of at least one (e.g., 1, 2 or 3, 1 or 2, and usually 1) claimed compound in combination with hormonal therapies (i.e., antihormonal agents).

Also disclosed are methods of treating breast cancer (i.e., post-menopausal and premenopausal breast cancer, e.g., hormone-dependent breast cancer) in a patient in need of such treatment, said treatment comprising the administration of an effective amount of a pharmaceutical composition comprising an effective amount of at least one (e.g., 1, 2 or 3, 1 or 2, and usually 1) claimed compound in combination with hormonal therapies (i.e., antihormonal agents).

Further disclosed are methods of treating breast cancer (i.e., post-menopausal and premenopausal breast cancer, e.g., hormone-dependent breast cancer) in a patient in need of such treatment, said treatment comprising the administration of an effective amount of at least one (e.g., 1, 2 or 3, 1 or 2, and usually 1) claimed compound in combination with hormonal therapies (i.e., antihormonal agents), and in combination with an effective amount of at least one (e.g., 1, 2 or 3, 1 or 2, or 1) chemotherapeutic agent.

Also disclosed are methods of treating breast cancer (i.e., post-menopausal and premenopausal breast cancer, e.g., hormone-dependent breast cancer) in a patient in need of such treatment, said treatment comprising the administration of an effective amount of a pharmaceutical composition comprising an effective amount of at least one (e.g., 1, 2 or 3, 1 or 2, and usually 1) claimed compound in combination with hormonal therapies (i.e., antihormonal agents), and in combination with an effective amount of at least one (e.g., 1, 2 or 3, 1 or 2, or 1) chemotherapeutic agent.

The methods of treating breast cancer described herein include the treatment of hormone-dependent metastatic and advanced breast cancer, adjuvant therapy for hormone-dependent primary and early breast cancer, the treatment of ductal carcinoma in situ, and the treatment of inflammatory breast cancer in situ.

The methods of treating hormone-dependent breast cancer can also be used to prevent breast cancer in patients having a high risk of developing breast cancer.

Further disclosed are methods of preventing breast cancer (i.e., post-menopausal and premenopausal breast cancer, e.g., hormone-dependent breast cancer) in a patient in need of such treatment, said treatment comprising the administration of an effective amount of at least one (e.g., 1, 2 or 3, 1 or 2, and usually 1) claimed compound in combination with hormonal therapies (i.e., antihormonal agents).

Also disclosed are methods of preventing breast cancer (i.e., post-menopausal and premenopausal breast cancer, e.g., hormone-dependent breast cancer) in a patient in need of such treatment, said treatment comprising the administration of an effective amount of a pharmaceutical composition comprising an effective amount of at least one (e.g., 1, 2 or 3, 1 or 2, and usually 1) claimed compound in combination with hormonal therapies (i.e., antihormonal agents).

Further disclosed are methods of preventing breast cancer (i.e., post-menopausal and premenopausal breast cancer, e.g., hormone-dependent breast cancer) in a patient in need of such treatment, said treatment comprising the administration of an effective amount of at least one (e.g., 1, 2 or 3, 1 or 2, and usually 1) claimed compound in combination with hormonal therapies (i.e., antihormonal agents), and in combination with an effective amount of at least one (e.g., 1, 2 or 3, 1 or 2, or 1) chemotherapeutic agent.

Also disclosed are methods of preventing breast cancer (i.e., post-menopausal and premenopausal breast cancer, e.g., hormone-dependent breast cancer) in a patient in need of such treatment, said treatment comprising the administration of an effective amount of a pharmaceutical composition comprising an effective amount of at least one (e.g., 1, 2 or 3, 1 or 2, and usually 1) claimed compound in combination with hormonal therapies (i.e., antihormonal agents), and in combination with an effective amount of at least one (e.g., 1, 2 or 3, 1 or 2, or 1) chemotherapeutic agent.

Further disclosed is a method for treating brain cancer (e.g., glioma, such as glioma blastoma multiforme) in a patient in need of such treatment, said method comprising administering to said patient an effective amount of at least one (e.g., 1, 2 or 3, 1 or 2, and usually 1) claimed compound.

Also disclosed is a method for treating brain cancer (e.g., glioma, such as glioma blastoma multiforme) in a patient in need of such treatment, said method comprising administering to said patient an effective amount of at least one (e.g., 1, 2 or 3, 1 or 2, and usually 1) claimed compound, in combination with an effective amount of at least one (e.g., 1, 2 or 3, 1 or 2, or 1) chemotherapeutic agent.

Further disclosed is a method for treating brain cancer (e.g., glioma, such as glioma blastoma multiforme) a in a patient in need of such treatment, said method comprising administering to said patient an effective amount of a pharmaceutical composition comprising an effective amount of at least one (e.g., 1, 2 or 3, 1 or 2, and usually 1) claimed compound.

Also disclosed is a method for treating brain cancer (e.g., glioma, such as glioma blastoma multiforme) in a patient in need of such treatment, said method comprising administering to said patient an effective amount of a pharmaceutical composition comprising an effective amount of at least one (e.g., 1, 2 or 3, 1 or 2, and usually 1) claimed compound, in combination with an effective amount of at least one (e.g., 1, 2 or 3, 1 or 2, or 1) chemotherapeutic agent.

Further disclosed is a method for treating brain cancer (e.g., glioma, such as glioma blastoma multiforme) in a patient in need of such treatment, said method comprising administering to said patient an effective amount of at least one (e.g., 1, 2 or 3, 1 or 2, and usually 1) claimed compound, in combination with an effective amount of a chemotherapeutic agent wherein said chemotherapeutic agent is temozolomide.

Also disclosed is a method for treating brain cancer (e.g., glioma, such as glioma blastoma multiforme) in a patient in need of such treatment, said method comprising administering to said patient an effective amount of a pharmaceutical composition comprising an effective amount of at least one (e.g., 1, 2 or 3, 1 or 2, and usually 1) claimed compound, in combination with an effective amount of a chemotherapeutic agent, wherein said chemotherapeutic agent is temozolomide.

Further disclosed is a method for treating prostate cancer in a patient in need of such treatment, said method comprising administering to said patient an effective amount of at least one (e.g., 1, 2 or 3, 1 or 2, and usually 1) claimed compound.

Also disclosed is a method for treating prostate cancer in a patient in need of such treatment, said method comprising administering to said patient an effective amount of at least one (e.g., 1, 2 or 3, 1 or 2, and usually 1) claimed compound, in combination with an effective amount of at least one (e.g., 1, 2 or 3, 1 or 2, or 1) chemotherapeutic agent.

Further disclosed is a method for treating prostate cancer in a patient in need of such treatment, said method comprising administering to said patient an effective amount of a pharmaceutical composition comprising an effective amount of at least one (e.g., 1, 2 or 3, 1 or 2, and usually 1) claimed compound.

Also disclosed is a method for treating prostate cancer in a patient in need of such treatment, said method comprising administering to said patient an effective amount of a pharmaceutical composition comprising an effective amount of at least one (e.g., 1, 2 or 3, 1 or 2, and usually 1) claimed compound, in combination with an effective amount of at least one (e.g., 1, 2 or 3, 1 or 2, or 1) chemotherapeutic agent.

Further disclosed is a method for treating myelodysplastic syndrome in a patient in need of such treatment, said method comprising administering to said patient an effective amount of at least one (e.g., 1, 2 or 3, 1 or 2, and usually 1) claimed compound.

Also disclosed is a method for treating myelodysplastic syndrome in a patient in need of such treatment, said method comprising administering to said patient an effective amount of at least one (e.g., 1, 2 or 3, 1 or 2, and usually 1) claimed compound, in combination with an effective amount of at least one (e.g., 1, 2 or 3, 1 or 2, or 1) chemotherapeutic agent.

Further disclosed is a method for treating myelodysplastic syndrome in a patient in need of such treatment, said method comprising administering to said patient an effective amount of a pharmaceutical composition comprising an effective amount of at least one (e.g., 1, 2 or 3, 1 or 2, and usually 1) claimed compound.

Also disclosed is a method for treating myelodysplastic syndrome in a patient in need of
such treatment, said method comprising administering to said patient an effective amount of a pharmaceutical composition comprising an effective amount of at least one (e.g., 1, 2 or 3, 1 or 2, and usually 1) claimed compound, in combination with an effective amount of at least one (e.g., 1, 2 or 3, 1 or 2, or 1) chemotherapeutic agent.

Further disclosed is a method for treating myeloid leukemias in a patient in need of such treatment, said method comprising administering to said patient an effective amount of at least one (e.g., 1, 2 or 3, 1 or 2, and usually 1) claimed compound.

Also disclosed is a method for treating myeloid leukemias in a patient in need of such treatment, said method comprising administering to said patient an effective amount of at least one (e.g., 1, 2 or 3, 1 or 2, and usually 1) claimed compound, in combination with an effective amount of at least one (e.g., 1, 2 or 3, 1 or 2, or 1) chemotherapeutic agent.

Further disclosed is a method for treating myeloid leukemias in a patient in need of such treatment, said method comprising administering to said patient an effective amount of a pharmaceutical composition comprising an effective amount of at least one (e.g., 1, 2 or 3, 1 or 2, and usually 1) claimed compound.

Also disclosed is a method for treating myeloid leukemias in a patient in need of such treatment, said method comprising administering to said patient an effective amount of a pharmaceutical composition comprising an effective amount of at least one (e.g., 1, 2 or 3, 1 or 2, and usually 1) claimed compound, in combination with an effective amount of at least one (e.g., 1, 2 or 3, 1 or 2, or 1) chemotherapeutic agent.

Further disclosed is a method for treating acute myelogenous leukemia (AML) in a patient in need of such treatment, said method comprising administering to said patient an effective amount of at least one (e.g., 1, 2 or 3, 1 or 2, and usually 1) claimed compound.

Also disclosed is a method for treating acute myelogenous leukemia (AML) in a patient in need of such treatment, said method comprising administering to said patient an effective amount of at least one (e.g., 1, 2 or 3, 1 or 2, and usually 1) claimed compound, in combination with an effective amount of at least one (e.g., 1, 2 or 3, 1 or 2, or 1) chemotherapeutic agent.

Further disclosed is a method for treating acute myelogenous leukemia (AML)in a patient in need of such treatment, said method comprising administering to said patient an effective amount of a pharmaceutical composition comprising an effective amount of at least one (e.g., 1, 2 or 3, 1 or 2, and usually 1) claimed compound.

Also disclosed is a method for treating acute myelogenous leukemia (AML)in a patient in need of such treatment, said method comprising administering to said patient an effective amount of a pharmaceutical composition comprising an effective amount of at least one (e.g., 1, 2 or 3, 1 or 2, and usually 1) claimed compound, in combination with an effective amount of at least one (e.g., 1, 2 or 3, 1 or 2, or 1) chemotherapeutic agent.

Further disclosed is a method for treating chronic myelomonocytic leukemia (CMML) in a patient in need of such treatment, said method comprising administering to said patient an effective amount of at least one (e.g., 1, 2 or 3, 1 or 2, and usually 1) claimed compound.

Also disclosed is a method for treating chronic myelomonocytic leukemia (CMML) in a patient in need of such treatment, said method comprising administering to said patient an effective amount of at least one (e.g., 1, 2 or 3, 1 or 2, and usually 1) claimed compound, in combination with an effective amount of at least one (e.g., 1, 2 or 3, 1 or 2, or 1) chemotherapeutic agent.

Further disclosed is a method for treating chronic myelomonocytic leukemia (CMML) in a patient in need of such treatment, said method comprising administering to said patient an effective amount of a pharmaceutical composition comprising an effective amount of at least one (e.g., 1, 2 or 3, 1 or 2, and usually 1) claimed compound.

Also disclosed is a method for treating chronic myelomonocytic leukemia (CMML) in a patient in need of such treatment, said method comprising administering to said patient an effective amount of a pharmaceutical composition comprising an effective amount of at least one (e.g., 1, 2 or 3, 1 or 2, and usually 1) claimed compound, in combination with an effective amount of at least one (e.g., 1, 2 or 3, 1 or 2, or 1) chemotherapeutic agent.

Further disclosed is a method for treating chronic myelogenous leukemia (chronic myeloid leukemia, CML) in a patient in need of such treatment, said method comprising administering to said patient an effective amount of at least one (e.g., 1, 2 or 3, 1 or 2, and usually 1) claimed compound.

Also disclosed is a method for treating chronic myelogenous leukemia (chronic myeloid leukemia, CML) in a patient in need of such treatment, said method comprising administering to said patient an effective amount of at least one (e.g., 1, 2 or 3, 1 or 2, and usually 1) claimed compound, in combination with an effective amount of at least one (e.g., 1, 2 or 3, 1 or 2, or 1) chemotherapeutic agent.

Further disclosed is a method for treating chronic myelogenous leukemia (chronic myeloid leukemia, CML) in a patient in need of such treatment, said method comprising administering to said patient an effective amount of a pharmaceutical composition comprising an effective amount of at least one (e.g., 1, 2 or 3, 1 or 2, and usually 1) claimed compound.

Also disclosed is a method for treating chronic myelogenous leukemia (chronic myeloid leukemia, CML) in a patient in need of such treatment, said method comprising administering to said patient an effective amount of a pharmaceutical composition comprising an effective amount of at least one (e.g., 1, 2 or 3, 1 or 2, and usually 1) claimed compound, in combination with an effective amount of at least one (e.g., 1, 2 or 3, 1 or 2, or 1) chemotherapeutic agent.

Further disclosed is a method for treating myeloid leukemias in a patient in need of such treatment, said method comprising administering to said patient an effective amount of at least one (e.g., 1, 2 or 3, 1 or 2, and usually 1) claimed compound.

Also disclosed is a method for treating myeloid leukemias in a patient in need of such treatment, said method comprising administering to said patient an effective amount of at least one (e.g., 1, 2 or 3, 1 or 2, and usually 1) claimed compound, in combination with an effective amount of at least one (e.g., 1, 2 or 3, 1 or 2, or 1) chemotherapeutic agent.

Further disclosed is a method for treating myeloid leukemias in a patient in need of such treatment, said method comprising administering to said patient an effective amount of a pharmaceutical composition comprising an effective amount of at least one (e.g., 1, 2 or 3, 1 or 2, and usually 1) claimed compound.

Also disclosed is a method for treating myeloid leukemias in a patient in need of such treatment, said method comprising administering to said patient an effective amount of a pharmaceutical composition comprising an effective amount of at least one (e.g., l, 2 or 3, 1 or 2, and usually 1) claimed compound, in combination with an effective amount of at least one (e.g., 1, 2 or 3, 1 or 2, or 1) chemotherapeutic agent.

Further disclosed is a method for treating bladder cancer in a patient in need of such treatment, said method comprising administering to said patient an effective amount of at least one (e.g., 1, 2 or 3, 1 or 2, and usually 1) claimed compound.

Also disclosed is a method for treating bladder cancer in a patient in need of such treatment, said method comprising administering to said patient an effective amount of at least one (e.g., 1, 2 or 3, 1 or 2, and usually 1) claimed compound, in combination with an effective amount of at least one (e.g., 1, 2 or 3, 1 or 2, or 1) chemotherapeutic agent.

Further disclosed is a method for treating bladder cancer in a patient in need of such treatment, said method comprising administering to said patient an effective amount of a pharmaceutical composition comprising an effective amount of at least one (e.g., 1, 2 or 3, 1 or 2, and usually 1) claimed compound.

Also disclosed is a method for treating bladder cancer in a patient in need of such treatment, said method comprising administering to said patient an effective amount of a pharmaceutical composition comprising an effective amount of at least one (e.g., 1, 2 or 3, 1 or 2, and usually 1) claimed compound, in combination with an effective amount of at least one (e.g., 1, 2 or 3, 1 or 2, or 1) chemotherapeutic agent.

Further disclosed is a method for treating non-Hodgkin's lymphoma in a patient in need of such treatment, said method comprising administering to said patient an effective amount of at least one (e.g., 1, 2 or 3, 1 or 2, and usually 1) claimed compound.

Also disclosed is a method for treating non-Hodgkin's lymphoma in a patient in need of such treatment, said method comprising administering to said patient an effective amount of at least one (e.g., 1, 2 or 3, 1 or 2, and usually 1) claimed compound, in combination with an effective amount of at least one (e.g., 1, 2 or 3, 1 or 2, or 1) chemotherapeutic agent.

Further disclosed is a method for treating non-Hodgkin's lymphoma in a patient in need of such treatment, said method comprising administering to said patient an effective amount of a pharmaceutical composition comprising an effective amount of at least one (e.g., 1, 2 or 3, 1 or 2, and usually 1) claimed compound.

Also disclosed is a method for treating non-Hodgkin's lymphoma in a patient in need of such treatment, said method comprising administering to said patient an effective amount of a pharmaceutical composition comprising an effective amount of at least one (e.g., 1, 2 or 3, 1 or 2, and usually 1) claimed compound, in combination with an effective amount of at least one (e.g., 1, 2 or 3, 1 or 2, or 1) chemotherapeutic agent.

Further disclosed is a method for treating multiple myeloma in a patient in need of such treatment, said method comprising administering to said patient an effective amount of at least one (e.g., 1, 2 or 3, 1 or 2, and usually 1) claimed compound.

Also disclosed is a method for treating multiple myeloma in a patient in need of such treatment, said method comprising administering to said patient an effective amount of at least one (e.g., 1, 2 or 3, 1 or 2, and usually 1) claimed compound, in combination with an effective amount of at least one (e.g., 1, 2 or 3, 1 or 2, or 1) chemotherapeutic agent.

Further disclosed is a method for treating multiple myeloma in a patient in need of such treatment, said method comprising administering to said patient an effective amount of a pharmaceutical composition comprising an effective amount of at least one (e.g., 1, 2 or 3, 1 or 2, and usually 1) claimed compound.

Also disclosed is a method for treating multiple myeloma in a patient in need of such treatment, said method comprising administering to said patient an effective amount of a pharmaceutical composition comprising an effective amount of at least one (e.g., 1, 2 or 3, 1 or 2, and usually 1) claimed compound, in combination with an effective amount of at least one (e.g., 1, 2 or 3, 1 or 2, or 1) chemotherapeutic agent.

In the methods of this invention the compounds of this invention can be administered concurrently or sequentially (i.e., consecutively) with the chemotherapeutic agents or the signal transduction inhibitor.

The methods of treating cancers described herein can optionally include the administration of an effective amount of radiation (i.e., the methods of treating cancers described herein optionally include the administration of radiation therapy).

### DETAILED DESCRIPTION OF THE INVENTION

As described herein, unless otherwise indicated, the use of a drug or compound in a specified period is per treatment cycle. For example, once a day means once per day of each day of the treatment cycle. Twice a day means twice per day each day of the treatment cycle. Once a week means one time per week during the treatment cycle. Once every three weeks means once per three weeks during the treatment cycle.

The following abbreviations have the following meanings unless defined otherwise:

| | |
|---|---|
| ACN | Acetonitrile |
| AcOH | Acetic acid |
| DAST | (diethylamino)sulfur trifluoride |
| DCC | Dicyclohexylcarbodiimide |
| DCU | Dicyclohexylurea |
| DCM | Dichloromethane |
| DI | Deionized water |
| DIAD | Diisopropylazodicarboxylate |
| DIEA | Diisopropylethylamine |
| DMAP | 4-Dimethylaminopyridine |
| DME | Dimethoxyethane |
| DMF | Dimethylformamide |
| DMFDMA | N,N-Dimethylformamide dimethylacetal |
| DMSO | Dimethyl sulfoxide |
| DTT | Dithiothreitol |
| EDCI | 1-(3-dimethylamino-propyl)-3-ethylcarbodiimide hydrochloride |
| EtOAc | Ethyl acetate |
| EtOH | Ethanol |
| HATU | N,N,N',N'-Tetramethyl-O-(7-Azabenzotriazol-1-yl)Uronium |
| | hexafluorophosphate |
| Hex | hexanes |
| HOBt | 1-Hydroxylbenzotriazole |
| HPLC | High pressure liquid chromatography |
| LCMS | Liquid chromatography mass spectrometry |
| LDA | Lithium diisopropylamide |
| mCPBA | *m*eta-Chloroperoxybenzoic acid |
| MeOH | Methanol |
| MTT | (3-[4,5-dimethyl-thiazol-2-yl]-2,5-diphenyltetrazolium bromide, Thiazolyl blue) |
| NMR | Nuclear magnetic resonance |
| PFP | Pentafluorophenol |
| PMB | p-methoxybenzyl |
| Pyr | Pyridine |
| Rb | Round bottom flask |
| Rbt | Round bottom flask |
| RT | Room temperature |
| SEMCI | 2-(Trimethylsily)ethoxy methyl chloride |
| TEA | Triethylamine |
| Tr | Triphenyl methane |
| Trt | Triphenyl methane |
| TrCl | Triphenyl methane chloride |
| TFA | Trifluoroacetic acid |
| THF | Tetrahydrofuran |
| TLC | Thin layer chromatography |
| TMS | Trimethylsilyl |

As used herein, unless otherwise specified, the following terms have the following meanings:
"anti-cancer agent" means a drug (medicament or pharmaceutically active ingredient) for treating cancer;
"antineoplastic agent" means a drug (medicament or pharmaceutically active ingredient) for treating cancer (i.e., a chemotherapeutic agent);
"at least one", as used in reference to the number of compounds of this invention means for example 1-6, generally 1-4, more generally 1, 2 or 3, and usually one or two, and more usually one;
"at least one", as used in reference to the number of chemotherapeutic agents used, means for example 1-6, generally 1-4, more generally 1, 2 or 3, and usually one or two, or one;
"chemotherapeutic agent" means a drug (medicament or pharmaceutically active ingredient) for treating cancer (i.e., and antineeoplastic agent);
"compound" with reference to the antineoplastic agents, includes the agents that are antibodies;
"concurrently" means (1) simultaneously in time (e.g., at the same time); or (2) at different times during the course of a common treatment schedule;
"consecutively" means one following the other;
"different" as used in the phrase "different antineoplastic agents" means that the agents are not the same compound or structure; preferably, "different" as used in the phrase "different antineoplastic agents" means not from the same class of antineoplastic agents; for example, one antineoplastic agent is a taxane, and another antineoplastic agent is a platinum coordinator compound;
"effective amount" or "therapeutically effective amount" is meant to describe an amount of compound or a composition of the present invention, or an amount of radiation, effective in treating or inhibiting the diseases or conditions described herein, and thus producing the desired therapeutic, ameliorative, inhibitory or preventative effect; thus, for example, in the methods of treating cancer described herein "effective amount" (or "therapeutically effective amount") means, for example, the amount of the compound (or drug), or radiation, that results in: (a) the reduction, alleviation or disappearance of one or more symptoms caused by the cancer, (b) the reduction of tumor size, (c) the elimination of the tumor, and/or (d) long-term disease stabilization (growth arrest) of the tumor; for example, in the treatment of lung cancer (e.g., non small cell lung cancer) a therapeutically effective amount is that amount that alleviates or eliminates cough, shortness of breath and/or pain; also, for example, an effective amount, or a therapeutically effective amount of the ERK inhibitor (i.e., a compound of this invention) is that amount which results in the reduction in ERK (ERK1 and/or ERK2) activity and phosphorylation; the reduction in ERK activity may be determined by the analysis of pharmacodynamic markers such as phosphorylated RSK1,2 and phosphorylated ERK1,2, using techniques well known in the art;
"Ex" in the tables represents "Example";
"one or more" has the same meaning as "at least one";
"patient" means an animal, such as a mammal (e.g., a human being, and preferably a human being);
"prodrug" means compounds that are rapidly transformed, for example, by hydrolysis in blood, in vivo to the parent compound, i.e., to the compounds of formula 1.0 or to a salt and/or to a solvate thereof; a thorough discussion is provided in T. Higuchi and V. Stella, Pro-drugs as Novel Delivery Systems, Vol. 14 of the A.C.S. Symposium Series, and in Edward B. Roche, ed., Bioreversible Carriers in Drug Design, American Pharmaceutical Association and Pergamon Press, 1987, both of which are incorporated herein by reference; the scope of this invention includes Prodrugs of the novel compounds of this invention;
sequentially-represents (1) administration of one component of the method ((a) compound of the invention, or (b) chemotherapeutic agent, signal transduction inhibitor and/or radiation therapy) followed by administration of the other component or components; after adminsitration of one component, the next component can be administered substantially immediately after the first component, or the next component can be administered after an effective time period after the first component; the effective time period is the amount of time given for realization of maximum benefit from the administration of the first component; and
"solvate" means a physical association of a compound of this invention with one or more solvent molecules; this physical association involves varying degrees of ionic and covalent bonding, including hydrogen bonding; in certain instances the solvate will be capable of isolation, for example when one or more solvent molecules are incorporated in the crystal lattice of the crystalline solid; "solvate" encompasses both solution-phase and isolatable solvates; non-limiting examples of suitable solvates include ethanolates, methanolates, and the like; "hydrate" is a solvate wherein the solvent molecule is H₂O.

Any carbon or heteroatom with unsatisfied valences in the text, schemes, examples, structural formulae, and any Tables herein is assumed to have the hydrogen atom or atoms to satisfy the valences.

One or more compounds of the invention may also exist as, or optionally converted to, a solvate. Preparation of solvates is generally known. Thus, for example, M. Caira et al, J. Pharmaceutical Sci., 93(3), 601-611 (2004) describe the preparation of the solvates of the antifungal fluconazole in ethyl acetate as well as from water. Similar preparations of solvates, hemisolvate, hydrates and the like are described by E. C. van Tonder et al, AAPS PharmSciTech., 5(1), article 12 (2004); and A. L. Bingham et al, Chem. Commun., 603-604 (2001). A typical, non-limiting, process involves dissolving the inventive compound in desired amounts of the desired solvent (organic or water or mixtures thereof) at a higher than ambient temperature, and cooling the solution at a rate sufficient to form crystals which are then isolated by standard methods. Analytical techniques such as, for example I. R. spectroscopy, show the presence of the solvent (or water) in the crystals as a solvate (or hydrate).

The term "pharmaceutical composition" is also intended to encompass both the bulk composition and individual dosage units comprised of more than one (e.g., two) pharmaceutically active agents such as, for example, a compound of the present invention and an additional agent selected from the lists of the additional agents described herein, along with any pharmaceutically inactive excipients. The bulk composition and each individual dosage unit can contain fixed amounts of the afore-said "more than one pharmaceutically active agents". The bulk composition is material that has not yet been formed into individual dosage units. An illustrative dosage unit is an oral dosage unit such as tablets, capsules, pills and the like. Similarly, the herein-described methods of treating a patient by administering a pharmaceutical composition of the present invention is also intended to encompass the administration of the afore-said bulk composition and individual dosage units.

This invention also includes the compounds of this invention in isolated and purified form.

Polymorphic forms of the claimed compounds and of the salts and solvates of the claimed compounds, are intended to be included in the present invention.

Certain compounds of the invention may exist in different isomeric (e.g., enantiomers, diastereoisomers, atropisomers) forms. The invention contemplates all such isomers both in pure form and in admixture, including racemic mixtures. Enol forms are also included.

All stereoisomers (for example, geometric isomers, optical isomers and the like) of the present compounds (including those of the salts, solvates and prodrugs of the compounds as well as the salts and solvates of the prodrugs), such as those which may exist due to asymmetric carbons on various substituents, including enantiomeric forms (which may exist even in the absence of asymmetric carbons), rotameric forms, atropisomers, and diastereomeric forms, are contemplated within the scope of this invention. Individual stereoisomers of the compounds of the invention may, for example, be substantially free of other isomers, or may be admixed, for example, as racemates or with all other, or other selected, stereoisomers. The chiral centers of the present invention can have the S or R configuration as defined by the *IUPAC* 1974 Recommendations. The use of the terms "salt", "solvate" "prodrug" and the like, is intended to equally apply to the salt, solvate and prodrug of enantiomers, stereoisomers, rotamers, tautomers, racemates or prodrugs of the inventive compounds.

Diasteromeric mixtures can be separated into their individual diastereomers on the basis of their physical chemical differences by methods well known to those skilled in the art, such as, for example, by chromatography and/or fractional crystallization. Enantiomers can be separated by converting the enantiomeric mixture into a diasteromeric mixture by reaction with an appropriate optically active compound (e.g., chiral auxiliary such as a chiral alcohol or Mosher's acid chloride), separating the diastereomers and converting (e.g., hydrolyzing) the individual diastereomers to the corresponding pure enantiomers. Enantiomers can also be separated by use of chiral HPLC column.

The claimed compounds form salts that are also within the scope of this invention. Reference to a claimed compound herein is understood to include reference to salts thereof, unless otherwise indicated. The term "salt(s)", as employed herein, denotes acidic salts formed with inorganic and/or organic acids, as well as basic salts formed with inorganic and/or organic bases. In addition, when a claimed compound contains both a basic moiety, such as, but not limited to a pyridine or imidazole, and an acidic moiety, such as, but not limited to a carboxylic acid, zwitterions ("inner salts") may be formed and are included within the term "salt(s)" as used herein. Pharmaceutically acceptable (i.e., non-toxic, physiologically acceptable salts) are preferred. Salts of the claimed compounds may be formed, for example, by reacting a claimed compound with an amount of acid or base, such as an equivalent amount, in a medium such as one in which the salt precipitates or in an aqueous medium followed by *lyophilization.* Acids (and bases) which are generally considered suitable for the formation of pharmaceutically useful salts from basic (or acidic) pharmaceutical compounds are discussed, for example, by S. Berge et al, Journal of Pharmaceutical Sciences (1977) 66(1) 1-19; P. Gould, International J. of Pharmaceutics (1986) 33 201-217; Anderson et al, The Practice of Medicinal Chemistry (1996), Academic Press, New York; in The Orange Book (Food & Drug Administration, Washington, D.C. on their website); and P. Heinrich Stahl, Camille G. Wermuth (Eds.), Handbook of Pharmaceutical Salts: Properties, Selection, and Use, (2002) Int'l. Union of Pure and Applied Chemistry, pp. 330-331.

Exemplary acid addition salts include acetates, adipates, alginates, ascorbates, aspartates, benzoates, benzenesulfonates, bisulfates, borates, butyrates, citrates, camphorates, camphorsulfonates, cyclopentanepropionates, digluconates, dodecylsulfates, ethanesulfonates, fumarates, glucoheptanoates, glycerophosphates, hemisulfates, heptanoates, hexanoates, hydrochlorides, hydrobromides, hydroiodides, 2-hydroxyethanesulfonates, lactates, maleates, methanesulfonates, methyl sulfates, 2-naphthalenesulfonates, nicotinates, nitrates, oxalates, pamoates, pectinates, persulfates, 3-phenylpropionates, phosphates, picrates, pivalates, propionates, salicylates, succinates, sulfates, sulfonates (such as those mentioned herein), tartarates, thiocyanates, toluenesulfonates (also known as tosylates,) undecanoates, and the like.

Exemplary basic salts include ammonium salts, alkali metal salts such as sodium, lithium, and potassium salts, alkaline earth metal salts such as calcium and magnesium salts, aluminum salts, zinc salts, salts with organic bases (for example, organic amines) such as benzathines, diethylamine, dicyclohexylamines, hydrabamines (formed with N,N-bis(dehydroabietyl)ethylenediamine), N-methyl-D-glucamines, N-methyl-D-glucamides, t-butyl amines, piperazine, phenylcyclohexyl-amine, choline, tromethamine, and salts with amino acids such as arginine, lysine and the like. Basic nitrogen-containing groups may be quarternized with agents such as lower alkyl halides (e.g. methyl, ethyl, propyl, and butyl chlorides, bromides and iodides), dialkyl sulfates (e.g. dimethyl, diethyl, dibutyl, and diamyl sulfates), long chain halides (e.g. decyl, lauryl, myristyl and stearyl chlorides, bromides and iodides), aralkyl halides (e.g. benzyl and phenethyl bromides), and others.

All such acid and base salts are intended to be pharmaceutically acceptable salts within the scope of the invention and all acid and base salts are considered equivalent to the free forms of the corresponding compounds for purposes of the invention.

Claimed compounds and salts and solvates thereof, may exist in their tautomeric form (for example, as an amide or imino ether). All such tautomeric forms are contemplated herein as part of the present invention.

The compounds of this invention inhibit the activity of ERK1 and ERK2 Thus, disclosed is a method of inhibiting ERK in mammals, especially humans, by the administration of an effective amount (e.g., a therapeutically effective amount) of one or more (e.g., one) compounds of this invention. The administration of the compounds of this invention to patients, to inhibit ERK1 and/or ERK2, is useful in the treatment of cancer.

In any of the methods of treating cancer described herein, unless stated otherwise, the methods can optionally include the administration of an effective amount of one or more (e.g., 1, 2 or 3, or 1 or 2, or 1) chemotherapeutic agents. The chemotherapeutic agents can be administered currently or sequentially with the compounds of this invention.

The methods of treating cancer described herein include methods wherein a combination of drugs (i.e., compounds, or pharmaceutically active ingredients, or pharmaceutical compositions) are used (i.e., the methods of treating cancer of this invention include combination therapies). Those skilled in the art will appreciate that the drugs are generally administered individually as a pharmaceutical composition. The use of a pharmaceutical composition comprising more than one drug is within the scope of this invention.

In any of the methods of treating cancer described herein, unless stated otherwise, the methods can optionally include the administration of an effective amount of radiation therapy. For radiation therapy, γ-radiation is preferred.

Examples of cancers which may be treated by the methods of this invention include, but are not limited to: (A) lung cancer (e.g., lung adenocarcinoma and non small cell lung cancer), (B) pancreatic cancers (e.g., pancreatic carcinoma such as, for example, exocrine pancreatic carcinoma), (C) colon cancers (e.g., colorectal carcinomas, such as, for example, colon adenocarcinoma and colon adenoma), (D) myeloid leukemias (for example, acute myelogenous leukemia (AML), CML, and CMML), (E) thyroid cancer, (F) myelodysplastic syndrome (MDS), (G) bladder carcinoma, (H) epidermal carcinoma, (I) melanoma, (J) breast cancer, (K) prostate cancer, (L) head and neck cancers (e.g., squamous cell cancer of the head and neck), (M) ovarian cancer, (N) brain cancers (e.g., gliomas, such as glioma blastoma multiforme), (O) cancers of mesenchymal origin (e.g., fibrosarcomas and rhabdomyosarcomas), (P) sarcomas, (Q) tetracarcinomas, (R) nuroblastomas, (S) kidney carcinomas, (T) hepatomas, (U) non-Hodgkin's lymphoma, (V) multiple myeloma, and (W) anaplastic thyroid carcinoma.

Chemotherapeutic agents (antineoplastic agent) include but are not limited to: microtubule affecting agents, alkylating agents, antimetabolites, natural products and their derivatives, hormones and steroids (including synthetic analogs), and synthetics.

Examples of natural products and their derivatives (including vinca alkaloids, antitumor antibiotics, enzymes, lymphokines and epipodophyllotoxins) include: Vinblastine, Vincristine, Vindesine, Bleomycin, Dactinomycin, Daunorubicin, Doxorubicin, Epirubicin, Idarubicin, Paclitaxel (paclitaxel is a microtubule affecting agent and is commercially available as Taxol^{®}), Paclitaxel derivatives (e.g. taxotere), Mithramycin, Deoxyco-formycin, Mitomycin-C, L-Asparaginase, Interferons (especially IFN-a), Etoposide, and Teniposide.

Examples of hormones and steroids (including synthetic analogs) include: 17α-Ethinylestradiol, Diethylstilbestrol, Testosterone, Prednisone, Fluoxymesterone, Dromostanolone propionate, Testolactone, Megestrolacetate, Tamoxifen, Methylprednisolone, Methyltestosterone, Prednisolone, Triamcinolone, Chlorotrianisene, Hydroxyprogesterone, Aminoglutethimide, Estramustine, Medroxyprogesteroneacetate, Leuprolide, Flutamide, Toremifene, and Zoladex.

Examples of synthetics (including inorganic complexes such as platinum coordination complexes): Cisplatin, Carboplatin, Hydroxyurea, Amsacrine, Procarbazine, Mitotane, Mitoxantrone, Levamisole, and Hexamethylmelamine.

Examples of other chemotherapeutics include: Navelbene, CPT-11, Anastrazole, Letrazole, Capecitabinbe, Reloxafine, and Droloxafme.

A microtubule affecting agent (e.g., paclitaxel, a paclitaxel derivative or a paclitaxel-like compound), as used herein, is a compound that interferes with cellular mitosis, i.e., having an anti-mitotic effect, by affecting microtubule formation and/or action. Such agents can be, for instance, microtubule stabilizing agents or agents which disrupt microtubule formation.

Microtubule affecting agents include, but are not limited to: Allocolchicine (NSC 406042), Halichondrin B (NSC 609395), Colchicine (NSC 757), Colchicine derivatives (e.g., NSC 33410), Dolastatin 10 (NSC 376128), Maytansine (NSC 153858), Rhizoxin (NSC 332598), Paclitaxel (Taxol^{®}, NSC 125973), Paclitaxel derivatives (e.g., Taxotere, NSC 608832), Thiocolchicine (NSC 361792), Trityl Cysteine (NSC 83265), Vinblastine Sulfate (NSC 49842), Vincristine Sulfate (NSC 67574), Epothilone A, Epothilone, Discodermolide (see Service, (1996) Science, 274:2009), Estramustine, Nocodazole, MAP4, and the like. Examples of such agents are described in, for example, Bulinski (1997) J. Cell Sci. 110:3055-3064, Panda (1997) Proc. Natl. Acad. Sci. USA 94:10560-10564, Muhlradt (1997) Cancer Res. 57:3344-3346, Nicolaou (1997) Nature 387:268-272, Vasquez (1997) Mol. Biol. Cell. 8:973-985, and Panda (1996) J. Biol. Chem. 271:29807-29812.

Chemotherapeutic agents with paclitaxel-like activity include, but are not limited to, paclitaxel and paclitaxel derivatives (paclitaxel-like compounds) and analogues. Paclitaxel and its derivatives (e.g. Taxol and Taxotere) are available commercially. In addition, methods of making paclitaxel and paclitaxel derivatives and analogues are well known to those of skill in the art (see, e.g., U.S. Patent Nos: 5,569,729; 5,565,478; 5,530,020; 5,527,924; 5,508,447; 5,489,589; 5,488,116; 5,484,809; 5,478,854; 5,478,736; 5,475,120; 5,468,769; 5,461,169; 5,440,057; 5,422,364; 5,411,984; 5,405,972; and 5,296,506).

More specifically, the term "paclitaxel" as used herein refers to the drug commercially available as Taxol^{®} (NSC number: 125973). Taxol^{®} inhibits eukaryotic cell replication by enhancing polymerization of tubulin moieties into stabilized microtubule bundles that are unable to reorganize into the proper structures for mitosis. Of the many available chemotherapeutic drugs, paclitaxel has generated interest because of its efficacy in clinical trials against drug-refractory tumors, including ovarian and mammary gland tumors (Hawkins (1992) Oncology, 6: 17-23, Horwitz (1992) Trends Pharmacol. Sci. 13: 134-146, Rowinsky (1990) J. Natl. Canc. Inst. 82: 1247-1259).

Additional microtubule affecting agents can be assessed using one of many such assays known in the art, e.g., a semiautomated assay which measures the tubulin-polymerizing activity of paclitaxel analogs in combination with a cellular assay to measure the potential of these compounds to block cells in mitosis (see Lopes (1997) Cancer Chemother. Pharmacol. 41:37-47).

Generally, activity of a test compound is determined by contacting a cell with that compound and determining whether or not the cell cycle is disrupted, in particular, through the inhibition of a mitotic event. Such inhibition may be mediated by disruption of the mitotic apparatus, e.g., disruption of normal spindle formation. Cells in which mitosis is interrupted may be characterized by altered morphology (e.g., microtubule compaction, increased chromosome number, etc.).

Compounds with possible tubulin polymerization activity can be screened in vitro. For example, the compounds are screened against cultured WR21 cells (derived from line 69-2 wapras mice) for inhibition of proliferation and/or for altered cellular morphology, in particular for microtubule compaction. In vivo screening of positive-testing compounds can then be performed using nude mice bearing the WR21 tumor cells. Detailed protocols for this screening method are described by Porter (1995) Lab. Anim. Sci., 45(2):145-150.

Other methods of screening compounds for desired activity are well known to those of skill in the art. Typically such assays involve assays for inhibition of microtubule assembly and/or disassembly. Assays for microtubule assembly are described, for example, by Gaskin et al. (1974) J. Molec. Biol., 89: 737-758. U.S. Patent No. 5,569,720 also provides in vitro and in vivo assays for compounds with paclitaxel-like activity.

Thus, in the disclosed methods, wherein at least one chemotherapeutic agent is used, examples of said chemotherapeutic agents include those selected from the group consisting of: microtubule affecting agents, alkylating agents, antimetabolites, natural products and their derivatives, hormones and steroids (including synthetic analogs), and synthetics.

In the disclosed methods wherein at least one chemotherapeutic agent is used, examples of said chemotherapeutic agents also include: (1) taxanes, (2) platinum coordinator compounds, (3) epidermal growth factor (EGF) inhibitors that are antibodies, (4) EGF inhibitors that are small molecules, (5) vascular endolithial growth factor (VEGF) inhibitors that are antibodies, (6) VEGF kinase inhibitors that are small molecules, (7) estrogen receptor antagonists or selective estrogen receptor modulators (SERMs), (8) anti-tumor nucleoside derivatives, (9) epothilones, (10) topoisomerase inhibitors, (11) vinca alkaloids, (12) antibodies that are inhibitors of V 3 integrins, (13) folate antagonists, (14) ribonucleotide reductase inhibitors, (15) anthracyclines, (16) biologics; (17) inhibitors of angiogenesis and/or suppressors of tumor necrosis factor alpha (TNF-alpha) such as thalidomide (or related imid), (18) Bcr/abl kinase inhibitors, (19) MEK1 and/or MEK 2 inhibitors that are small molecules, (20) IGF-1 and IGF-2 inhibitors that are small molecules, (21) small molecule inhibitors of RAF and BRAF kinases, (22) small molecule inhibitors of cell cycle dependent kinases such as CDK1, CDK2, CDK4 and CDK6, (23) alkylating agents, and (24) farnesyl protein transferase inhibitors (also know as FPT inhibitors or FTI (i.e., farnesyl transfer inhibitors)).

In the disclosed methods, wherein at least one chemotherapeutic agent is used, examples of such chemotherapeutic agents include:
(1) taxanes such as paclitaxel (TAXOL^{®}) and/or docetaxel (Taxotere^{®});
(2) platinum coordinator compounds, such as, for example, carboplatin, cisplatin and oxaliplatin (e.g. Eloxatin);
(3) EGF inhibitors that are antibodies, such as: HER2 antibodies (such as, for example trastuzumab (Herceptin, Genentech, Inc.), Cetuximab (Erbitux, IMC-C225, ImClone Systems), EMD 72000 (Merck KGaA), anti-EFGR monoclonal antibody ABX (Abgenix), TheraCIM-h-R3 (Center of Molecular Immunology), monoclonal antibody 425 (Merck KGaA), monoclonal antibody ICR-62 (ICR, Sutton, England); Herzyme (Elan Pharmaceutical Technologies and Ribozyme Pharmaceuticals), PKI 143 (Novartis), EKB 569 (Wyeth-Ayerst), GW 572016 (GlaxoSmithKline), CI 1033 (Pfizer Global Research and Development), trastuzmabmaytansinoid conjugate (Genentech, Inc.), mitumomab (Imclone Systems and Merck KGaA) and Melvax II (Imclone Systems and Merck KgaA);
(4) EGF inhibitors that are small molecules, such as, Tarceva (TM) (OSI-774, OSI Pharmaceuticals, Inc.), and Iressa (ZD 1839, Astra Zeneca);
(5) VEGF inhibitors that are antibodies such as: bevacizumab (Genentech, Inc.), and IMC-1C11 (ImClone Systems), DC 101 (a KDR VEGF Receptor 2 from ImClone Systems);
(6) VEGF kinase inhibitors that are small molecules such as SU 5416 (from Sugen, Inc), SU 6688 (from Sugen, Inc.), Bay 43-9006 (a dual VEGF and bRAF inhibitor from Bayer Pharmaceuticals and Onyx Pharmaceuticals);
(7) estrogen receptor antagonists or selective estrogen receptor modulators (SERMs), such as tamoxifen, idoxifene, raloxifene, trans-2,3-dihydroraloxifene, levormeloxifene, droloxifene, MDL 103,323, and acolbifene (Schering Corp.);
(8) anti-tumor nucleoside derivatives such as 5-fluorouracil, gemcitabine, capecitabine, cytarabine (Ara-C), fludarabine (F-Ara-A), decitabine, and chlorodeoxyadenosine (Cda, 2-Cda);
(9) epothilones such as BMS-247550 (Bristol-Myers Squibb), and EPO906 (Novartis Pharmaceuticals);
(10) topoisomerase inhibitors such as topotecan (Glaxo SmithKline), and Camptosar (Pharmacia);
(11) vinca alkaloids, such as, navelbine (Anvar and Fabre, France), vincristine and vinblastine;
(12) antibodies that are inhibitors of αVβ3 integrins, such as, LM-609 (see, Clinical Cancer Research, Vol. 6, page 3056-3061, August 2000, the disclosure of which is incorporated herein by reference thereto);
(13) folate antagonists, such as Methotrexate (MTX), and Premetrexed (Alimta);
(14) ribonucleotide reductase inhibitors, such as Hydroxyurea (HU);
(15) anthracyclines, such as Daunorubicin, Doxorubicin (Adriamycin), and Idarubicin;
(16) biologics, such as interferon (e.g., Intron-A and Roferon), pegylated interferon (e.g., Peg-Intron and Pegasys), and Rituximab (Rituxan, antibody used for the treatment of non-Hodgkin's lymphoma);
(17) thalidomide (or related imid);
(18) Bcr/abl kinase inhibitors, such as, for example Gleevec (STI-571), AMN-17, ONO12380 SU11248 (Sunitinib) and BMS-354825
(19) MEK1 and/or MEK2 inhibitors, such as PD0325901 and Arry-142886 (AZD6244);
(20) IGF-1 and IGF-2 inhibitors that are small molecules, such as, for example, NVP-AEW541;
(21) small molecule inhibitors of RAF and BRAF kinases, such as, for example, BAY 43-9006 (Sorafenib);
(22) small molecule inhibitors of cell cycle dependent kinases such as CDK1, CDK2, CDK4 and CDK6, such as, for example, CYC202, BMS387032, and Flavopiridol;
(23) alkylating agents, such as, for example, Temodar® brand of temozolomide;
(24) farnesyl protein transferase inhibitors, such as, for example:
   (a) Sarasar® brand of lonifarnib (i.e., 4-[2-[4-(3,10-dibromo-8-chloro-6,11-dihydro-5H-benzo[5,6]cyclohepta[1,2-*b*]byridin-11-yl)-1-piperidinyl)-2-oxoethyl]-1-piperidinecarboxamide, see for example, U.S. 5,874,442 issued February 23, 1999, and U.S. 6,632,455 issued October 14, 2003),
   (b) Zarnestra® brand of tipifarnib (i.e., (R)-6-amino[(4-chlorophenyl)(1-methyl-1H-imidazol-5-yl)methyl]-4-(3-chlorophenyl)-1-methyl-2(1H)-quinolinone, see for example, WO 97/16443 published May 9, 1997 and U.S. 5,968,952 issued October 19, 1999, and
   (c) Bristol-Myers Squibb 214662: 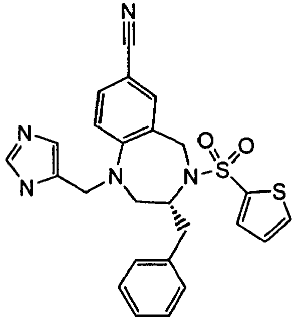
(see WO97/30992 published August 28, 1997, U.S. 6,011,029 issued January 4, 2000, and U.S. 6,455,523).

The Bcr/abl kinase inhibitors, EGF receptor inhibitors, and HER-2 antibodies (EGF receptor inhibitors that are antibodies) described above are also known as signal transduction inhibitors. Therefore, chemotherapeutic agents, as used herein, include signal transduction inhibitors.

Typical signal transduction inhibitors, that are chemotherapeutic agents, include but are not limited to: (i) Bcr/abl kinase inhibitors such as, for example, STI 571 (Gleevec), (ii) Epidermal growth factor (EGF) receptor inhibitor such as, for example, Kinase inhibitors (Iressa, OSI-774) and antibodies (Imclone: C225 [Goldstein et al. (1995), Clin Cancer Res. 1:1311-1318], and Abgenix: ABX-EGF) and (iii) HER-2/neu receptor inhibitors such as, for example, Herceptin® (trastuzumab).

Methods for the safe and effective administration of most of these chemotherapeutic agents are known to those skilled in the art. In addition, their administration is described in the standard literature. For example, the administration of many of the chemotherapeutic agents is described in the "Physicians' Desk Reference" (PDR), e.g., 1996 edition (Medical Economics Company, Montvale, NJ 07645-1742, USA), the Physician's Desk Reference, 56^{th} Edition, 2002 (published by Medical Economics company, Inc. Montvale, NJ 07645-1742), and the Physician's Desk Reference, 57^{th} Edition, 2003 (published by Thompson PDR, Montvale, NJ 07645-1742).

For example, the claimed compounds (e.g., a pharmaceutical composition comprising the claimed compounds); can be administered orally (e.g., as a capsule), and the chemotherapeutic agents can be administered intravenously, usually as an IV solution. The use of a pharmaceutical composition comprising more than one drug is also disclosed.

The claimed compound and the chemotherapeutic agents are administered in therapeutically effective dosages to obtain clinically acceptable results, e.g., reduction or elimination of symptoms or of the tumor. Thus, the claimed compound and chemotherapeutic agents can be administered concurrently or consecutively in a treatment protocol. The administration of the chemotherapeutic agents can be made according to treatment protocols already known in the art.

In general when more than one chemotherapeutic agent is used in the methods disclosed, the chemotherapeutic agents are administered on the same day either concurrently or consecutively in their standard dosage form. For example, the chemotherapeutic agents are usually administered intravenously, preferably by an IV drip using IV solutions well known in the art (e.g., isotonic saline (0.9% NaCl) or dextrose solution (e.g., 5% dextrose)).

When two or more chemotherapeutic agents are used, the chemotherapeutic agents are generally administered on the same day; however, those skilled in the art will appreciate that the chemotherapeutic agents can be administered on different days and in different weeks. The skilled clinician can administer the chemotherapeutic agents according to their recommended dosage schedule from the manufacturer of the agent and can adjust the schedule according to the needs of the patient, e.g., based on the patient's response to the treatment. For example, when gemcitabine is used in combination with a platinum coordinator compound, such as, for example, cisplatin, to treat lung cancer, both the gemcitabine and the cisplatin are given on the same day on day one of the treatment cycle, and then gemcitabine is given alone on day 8 and given alone again on day 15

The compounds of this invention and chemotherapeutic agents can be administered in a treatment protocol that usually lasts one to seven weeks, and is repeated typically from 6 to 12 times. Generally the treatment protocol can last one to four weeks. Treatment protocols of one to three weeks can also be used. A treatment protocol of one to two weeks can also be used. During this treatment protocol or cycle the compounds of this invention can be administered daily while the chemotherapeutic agents can be administered one or more times a week. Generally, a compound of this invention can be administered daily (i.e., once per day), and in one embodiment twice per day, and the chemotherapeutic agent is administered once a week or once every three weeks. For example, the taxanes (e.g., Paclitaxel (e.g., Taxol^{®}) or Docetaxel (e.g.,Taxotere^{®})) can be administered once a week or once every three weeks.

However, those skilled in the art will appreciate that treatment protocols can be varied according to the needs of the patient. Thus, the combination of compounds (drugs) used in the methods of this invention can be administered in variations of the protocols described above. For example, the compounds of this invention can be administered discontinuously rather than continuously during the treatment cycle. Thus, for example, during the treatment cycle the compounds of this invention can be administered daily for a week and then discontinued for a week, with this administration repeating during the treatment cycle. Or the compounds of this invention can be administered daily for two weeks and discontinued for a week, with this administration repeating during the treatment cycle. Thus, the compounds of this invention can be administered daily for one or more weeks during the cycle and discontinued for one or more weeks during the cycle, with this pattern of administration repeating during the treatment cycle. This discontinuous treatment can also be based upon numbers of days rather than a full week. For example, daily dosing for 1 to 6 days, no dosing for 1 to 6 days with this pattern repeating during the treatment protocol. The number of days (or weeks) wherein the compounds of this invention are not dosed do not have to equal the number of days (or weeks) wherein the compounds of this invention are dosed. Usually, if a discontinuous dosing protocol is used, the number of days or weeks that the compounds of this invention are dosed is at least equal or greater than the number of days or weeks that the compounds of this invention are not dosed.

The chemotherapeutic agent could be given by bolus or continuous infusion. The chemotherapeutic agent could be given daily to once every week, or once every two weeks, or once every three weeks, or once every four weeks during the treatment cycle. If administered daily during a treatment cycle, this daily dosing can be discontinuous over the number of weeks of the treatment cycle. For example, dosed for a week (or a number of days), no dosing for a week (or a number of days, with the pattern repeating during the treatment cycle.

The compounds of this invention can be administered orally, preferably as a solid dosage form, and in one embodiment as a capsule, and while the total therapeutically effective daily dose can be administered in one to four, or one to two divided doses per day, generally, the therapeutically effective dose is given once or twice a day, and in one embodiment twice a day. The compounds of this invention can be administered in an amount of about 50 to about 400 mg once per day, and can be administered in an amount of about 50 to about 300 mg once per day. The compounds of this invention are generally administered in an amount of about 50 to about 350 mg twice a day, usually 50 mg to about 200 mg twice a day, and in one embodiment about 75 mg to about 125 mg administered twice a day, and in another embodiment about 100 mg administered twice a day.

If the patient is responding, or is stable, after completion of the therapy cycle, the therapy cycle can be repeated according to the judgment of the skilled clinician. Upon completion of the therapy cycles, the patient can be continued on the compounds of this invention at the same dose that was administered in the treatment protocol, or, if the dose was less than 200mg twice a day, the dose can be raised to 200 mg twice a day. This maintenance dose can be continued until the patient progresses or can no longer tolerate the dose (in which case the dose can be reduced and the patient can be continued on the reduced dose).

The chemotherapeutic agents, used with the compounds of this invention, are administered in their normally prescribed dosages during the treatment cycle (i.e., the chemotherapeutic agents are administered according to the standard of practice for the administration of these drugs). For example: (a) about 30 to about 300 mg/m² for the taxanes; (b) about 30 to about 100 mg/m² for Cisplatin; (c) AUC of about 2 to about 8 for Carboplatin; (d) about 2 to about 4 mg/m² for EGF inhibitors that are antibodies; (e) about 50 to about 500 mg/m² for EGF inhibitors that are small molecules; (f) about 1 to about 10 mg/m² for VEGF kinase inhibitors that are antibodies; (g) about 50 to about 2400 mg/m² for VEGF inhibitors that are small molecules; (h) about 1 to about 20 mg for SERMs; (i) about 500 to about 1250 mg/m² for the anti-tumor nucleosides 5-Fluorouracil, Gemcitabine and Capecitabine; (j) for the anti-tumor nucleoside Cytarabine (Ara-C) 100-200mg/m²/day for 7 to 10 days every 3 to 4 weeks, and high doses for refractory leukemia and lymphoma, i.e., 1 to 3 gm/m² for one hour every 12 hours for 4-8 doses every 3 to four weeks; (k) for the anti-tumor nucleoside Fludarabine (F-ara-A) 10-25mg/m²/day every 3 to 4 weeks; (I) for the anti-tumor nucleoside Decitabine 30 to 75 mg/m² for three days every 6 weeks for a maximum of 8 cycles; (m) for the anti-tumor nucleoside Chlorodeoxyadenosine (CdA, 2-CdA) 0.05-0.1 mg/kg/day as continuous infusion for up to 7 days every 3 to 4 weeks; (n) about 1 to about 100 mg/m² for epothilones; (o) about 1 to about 350 mg/m² for topoisomerase inhibitors; (p) about 1 to about 50 mg/m² for vinca alkaloids; (q) for the folate antagonist Methotrexate (MTX) 20-60 mg/m² by oral, IV or IM every 3 to 4 weeks, the intermediate dose regimen is 80-250 mg/m² IV over 60 minutes every 3 to 4 weeks, and the high dose regimen is 250-1000mg/m² IV given with leucovorin every 3 to 4 weeks; (r) for the folate antagonist Premetrexed (Alimta) 300-600 mg/m² (10 minutes IV infusion day 1) every 3 weeks; (s) for the ribonucleotide reductase inhibitor Hydroxyurea (HU) 20-50 mg/kg/day (as needed to bring blood cell counts down); (t) the platinum coordinator compound Oxaliplatin (Eloxatin) 50-100 mg/m² every 3 to 4 weeks (preferably used for solid tumors such as non-small cell lung cancer, colorectal cancer and ovarian cancer); (u) for the anthracycline daunorubicin 10-50 mg/m²/day IV for 3-5 days every 3 to 4 weeks; (v) for the anthracycline Doxorubicin (Adriamycin) 50-100 mg/m² IV continuous infusion over 1-4 days every 3 to 4 weeks, or 10-40 mg/m² IV weekly; (w) for the anthracycline Idarubicin 10-30 mg/m² daily for 1-3 days as a slow IV infusion over 10-20 minutes every 3 to 4 weeks; (x) for the biologic interferon (Intron-A, Roferon) 5 to 20 million IU three times per week; (y) for the biologic pegylated interferon (Peg-intron, Pegasys) 3 to 4 micrograms/kg/day chronic sub cutaneous (until relapse or loss of activity); (z) for the biologic Rituximab (Rituxan) (antibody used for non-Hodgkin's lymphoma) 200-400mg/m² IV weekly over 4-8 weeks for 6 months; (aa) for the alkylating agent temozolomide 75 mg/m² to 250mg/m², for example, 150 mg/m², or for example, 200 mg/m², such as 200mg/m² for 5 days; and (bb) for the MEK1 and/or MEK2 inhibitor PD0325901, 15 mg to 30 mg, for example, 15 mg daily for 21 days every 4 weeks.

Gleevec can be used orally in an amount of about 200 to about 800 mg/day.

Thalidomide (and related imids) can be used orally in amounts of about 200 to about 800 mg/day, and can be contiuously dosed or used until releapse or toxicity. See for example Mitsiades et al., "Apoptotic signaling induced by immunomodulatory thalidomide analogs in human multiple myeloma cells; therapeutic implications", Blood, 99(12):4525-30, June 15, 2002.

The FPT inhibitor Sarasar® (brand of lonifarnib) can be administered orally (e.g., capsule) in amounts of about 50 to about 200 mg given twice a day, or in amounts of about 75 to about 125 mg given twice a day, or in amounts of about 100 to about 200 mg given twice a day, or in an amount of about 100 mg given twice a day.

Paclitaxel (e.g., Taxol^{®}, for example, can be administered once per week in an amount of about 50 to about 100 mg/m² and in another example about 60 to about 80 mg/m². In another example Paclitaxel (e.g., Taxol^{®}, can be administered once every three weeks in an amount of about 150 to about 250 mg/m² and in another example about 175 to about 225 mg/m².

In another example, Docetaxel (e.g., Taxotere^{®}) can be administered once per week in an amount of about 10 to about 45 mg/m². In another example Docetaxel (e.g., Taxotere^{®}) can be administered once every three weeks in an amount of about 50 to about 100 mg/m².

In another example Cisplatin can be administered once per week in an amount of about 20 to about 40 mg/m². In another example Cisplatin can be administered once every three weeks in an amount of about 60 to about 100 mg/m².

In another example Carboplatin can be administered once per week in an amount to provide an AUC of about 2 to about 3. In another example Carboplatin can be administered once every three weeks in an amount to provide an AUC of about 5 to about 8.

Other embodiments of this invention are described below. The embodiments have been numbered for the purpose of making it easier to refer to the embodiments. The term "in any one of Embodiment Nos." or the term "of any of Embodiment Nos.",

Thus one embodiment is directed to the final compound of Example 612. Another embodiment is directed to the final compound of Example 613. Another embodiment is directed to the final compound of Example 614. Another embodiment is directed to the final compound of Example 615. Another embodiment is directed to the final compound of Example 616. Another embodiment is directed to the final compound of Example 617. Another embodiment is directed to the final compound of Example 618. Another embodiment is directed to the final compound of Example 619. Another embodiment is directed to the final compound of Example 620. Another embodiment is directed to the final compound of Example 621. Another embodiment is directed to the final compound of Example 622. Another embodiment is directed to the final compound of Example 623. Another embodiment is directed to the final compound of Example 624. Another embodiment is directed to the final compound of Example 625. Another embodiment is directed to the final compound of Example 626. Another embodiment is directed to the final compound of Example 627. Another embodiment is directed to the final compound of Example 628. Another embodiment is directed to the final compound of Example 629. Another embodiment is directed to the final compound of Example 630. Another embodiment is directed to the final compound of Example 631. Another embodiment is directed to the final compound of Example 632. Another embodiment is directed to the final compound of Example 633. Another embodiment is directed to the final compound of Example 634. Another embodiment is directed to the final compound of Example 635. Another embodiment is directed to the final compound of Example 636. Another embodiment is directed to the final compound of Example 637. Another embodiment is directed to the final compound of Example 638. Another embodiment is directed to the final compound of Example 639. Another embodiment is directed to the final compound of Example 640. Another embodiment is directed to the final compound of Example 641. Another embodiment is directed to the final compound of Example 642. Another embodiment is directed to the final compound of Example 643. Another embodiment is directed to the final compound of Example 644. Another embodiment is directed to the final compound of Example 645. Another embodiment is directed to the final compound of Example 646. Another embodiment is directed to the final compound of Example 647. Another embodiment is directed to the final compound of Example 648. Another embodiment is directed to the final compound of Example 649. Another embodiment is directed to the final compound of Example 650. Another embodiment is directed to the final compound of Example 651. Another embodiment is directed to the final compound of Example 652. Another embodiment is directed to the final compound of Example 653. Another embodiment is directed to the final compound of Example 654. Another embodiment is directed to the final compound of Example 655. Another embodiment is directed to the final compound of Example 656. Another embodiment is directed to the final compound of Example 657. Another embodiment is directed to the final compound of Example 658. Another embodiment is directed to the final compound of Example 659. Another embodiment is directed to the final compound of Example 660. Another embodiment is directed to the final compound of Example 661. Another embodiment is directed to the final compound of Example 662. Another embodiment is directed to the final compound of Example 663. Another embodiment is directed to the final compound of Example 664. Another embodiment is directed to the final compound of Example 665. Another embodiment is directed to the final compound of Example 666. Another embodiment is directed to the final compound of Example 667. Another embodiment is directed to the final compound of Example 668. Another embodiment is directed to the final compound of Example 669. Another embodiment is directed to the final compound of Example 670. Another embodiment is directed to the final compound of Example 671. Another embodiment is directed to the final compound of Example 672. Another embodiment is directed to the final compound of Example 673. Another embodiment is directed to the final compound of Example 674. Another embodiment is directed to the final compound of Example 675. Another embodiment is directed to the final compound of Example 676. Another embodiment is directed to the final compound of Example 677. Another embodiment is directed to the final compound of Example 678. Another embodiment is directed to the final compound of Example 679. Another embodiment is directed to the final compound of Example 680. Another embodiment is directed to the final compound of Example 681. Another embodiment is directed to the final compound of Example 682. Another embodiment is directed to the final compound of Example 683. Another embodiment is directed to the final compound of Example 684. Another embodiment is directed to the final compound of Example 685. Another embodiment is directed to the final compound of Example 686. Another embodiment is directed to the final compound of Example 687. Another embodiment is directed to the final compound of Example 688. Another embodiment is directed to the final compound of Example 689. Another embodiment is directed to the final compound of Example 690. Another embodiment is directed to the final compound of Example 691. Another embodiment is directed to the final compound of Example 692. Another embodiment is directed to the final compound of Example 693. Another embodiment is directed to the final compound of Example 694. Another embodiment is directed to the final compound of Example 695. Another embodiment is directed to the final compound of Example 696. Another embodiment is directed to the final compound of Example 697. Another embodiment is directed to the final compound of Example 698. Another embodiment is directed to the final compound of Example 699. Another embodiment is directed to the final compound of Example 700. Another embodiment is directed to the final compound of Example 701. Another embodiment is directed to the final compound of Example 702. Another embodiment is directed to the final compound of Example 703. Another embodiment is directed to the final compound of Example 704. Another embodiment is directed to the final compound of Example 705. Another embodiment is directed to the final compound of Example 706. Another embodiment is directed to the final compound of Example 707. Another embodiment is directed to the final compound of Example 708. Another embodiment is directed to the final compound of Example 709. Another embodiment is directed to the final compound of Example 710. Another embodiment is directed to the final compound of Example 711. Another embodiment is directed to the final compound of Example 712. Another embodiment is directed to the final compound of Example 713. Another embodiment is directed to the final compound of Example 714. Another embodiment is directed to the final compound of Example 715. Another embodiment is directed to the final compound of Example 716. Another embodiment is directed to the final compound of Example 717. Another embodiment is directed to the final compound of Example 718. Another embodiment is directed to the final compound of Example 719. Another embodiment is directed to the final compound of Example 720. Another embodiment is directed to the final compound of Example 721. Another embodiment is directed to the final compound of Example 722. Another embodiment is directed to the final compound of Example 723. Another embodiment is directed to the final compound of Example 724. Another embodiment is directed to the final compound of Example 725. Another embodiment is directed to the final compound of Example 726. Another embodiment is directed to the final compound of Example 727. Another embodiment is directed to the final compound of Example 728. Another embodiment is directed to the final compound of Example 729. Another embodiment is directed to the final compound of Example 730. Another embodiment is directed to the final compound of Example 731. Another embodiment is directed to the final compound of Example 732. Another embodiment is directed to the final compound of Example 733. Another embodiment is directed to the final compound of Example 734. Another embodiment is directed to the final compound of Example 735. Another embodiment is directed to the final compound of Example 736. Another embodiment is directed to the final compound of Example 737. Another embodiment is directed to the final compound of Example 738. Another embodiment is directed to the final compound of Example 739. Another embodiment is directed to the final compound of Example 740. Another embodiment is directed to the final compound of Example 741. Another embodiment is directed to the final compound of Example 742. Another embodiment is directed to the final compound of Example 743. Another embodiment is directed to the final compound of Example 744. Another embodiment is directed to the final compound of Example 745. Another embodiment is directed to the final compound of Example 746. Another embodiment is directed to the final compound of Example 747. Another embodiment is directed to the final compound of Example 748. Another embodiment is directed to the final compound of Example 749. Another embodiment is directed to the final compound of Example 750. Another embodiment is directed to the final compound of Example 751. Another embodiment is directed to the final compound of Example 752. Another embodiment is directed to the final compound of Example 753. Another embodiment is directed to the final compound of Example 754. Another embodiment is directed to the final compound of Example 755. Another embodiment is directed to the final compound of Example 756. Another embodiment is directed to the final compound of Example 757. Another embodiment is directed to the final compound of Example 758. Another embodiment is directed to the final compound of Example 759. Another embodiment is directed to the final compound of Example 760. Another embodiment is directed to the final compound of Example 761. Another embodiment is directed to the final compound of Example 762. Another embodiment is directed to the final compound of Example 763. Another embodiment is directed to the final compound of Example 764. Another embodiment is directed to the final compound of Example 765. Another embodiment is directed to the final compound of Example 766. Another embodiment is directed to the final compound of Example 767. Another embodiment is directed to the final compound of Example 768. Another embodiment is directed to the final compound of Example 769. Another embodiment is directed to the final compound of Example 770. Another embodiment is directed to the final compound of Example 671. Another embodiment is directed to the final compound of Example 772. Another embodiment is directed to the final compound of Example 773. Another embodiment is directed to the final compound of Example 774. Another embodiment is directed to the final compound of Example 775. Another embodiment is directed to the final compound of Example 776. Another embodiment is directed to the final compound of Example 777. Another embodiment is directed to the final compound of Example 778. Another embodiment is directed to the final compound of Example 779. Another embodiment is directed to the final compound of Example 780. Another embodiment is directed to the final compound of Example 781. Another embodiment is directed to the final compound of Example 782. Another embodiment is directed to the final compound of Example 783. Another embodiment is directed to the final compound of Example 784. Another embodiment is directed to the final compound of Example 785. Another embodiment is directed to the final compound of Example 786. Another embodiment is directed to the final compound of Example 787. Another embodiment is directed to the final compound of Example 788. Another embodiment is directed to the final compound of Example 789. Another embodiment is directed to the final compound of Example 790. Another embodiment is directed to the final compound of Example 791. Another embodiment is directed to the final compound of Example 792. Another embodiment is directed to the final compound of Example 793. Another embodiment is directed to the final compound of Example 794. Another embodiment is directed to the final compound of Example 795. Another embodiment is directed to the final compound of Example 796. Another embodiment is directed to the final compound of Example 797. Another embodiment is directed to the final compound of Example 798. Another embodiment is directed to the final compound of Example 799. Another embodiment is directed to the final compound of Example 800. Another embodiment is directed to the final compound of Example 801. Another embodiment is directed to the final compound of Example 802. Another embodiment is directed to the final compound of Example 803. Another embodiment is directed to the final compound of Example 804. Another embodiment is directed to the final compound of Example 805. Another embodiment is directed to the final compound of Example 806. Another embodiment is directed to the final compound of Example 807. Another embodiment is directed to the final compound of Example 808. Another embodiment is directed to the final compound of Example 809. Another embodiment is directed to the final compound of Example 810. Another embodiment is directed to the final compound of Example 811. Another embodiment is directed to the final compound of Example 812. Another embodiment is directed to the final compound of Example 813. Another embodiment is directed to the final compound of Example 814. Another embodiment is directed to the final compound of Example 815. Another embodiment is directed to the final compound of Example 816. Another embodiment is directed to the final compound of Example 817. Another embodiment is directed to the final compound of Example 818. Another embodiment is directed to the final compound of Example 819. Another embodiment is directed to the final compound of Example 820. Another embodiment is directed to the final compound of Example 821. Another embodiment is directed to the final compound of Example 822. Another embodiment is directed to the final compound of Example 823. Another embodiment is directed to the final compound of Example 824. Another embodiment is directed to the final compound of Example 826. Another embodiment is directed to the final compound of Example 827.

Embodiment No. 2 is directed to the final compound of Example 622.

Embodiment No. 3 is directed to the final compound of Example 613.

Embodiment No. 4 is directed to a compound of any one of Embodiment Nos. 1 to 3 in pure and isolated form.

Embodiment No. 5 is directed to a pharmaceutical composition comprising an effective amount of at least one compound (e.g., 1, 2 or 3, or 1 or 2, or 1, and usually 1) claimed compound and a pharmaceutically acceptable carrier.

Embodiment No. 6 is directed to a pharmaceutical composition of any one of Embodiment Nos. 1 to 4 further comprising an effective amount of at least one (e.g., 1, 2 or 3, or 1 or 2, or 1, and usually 1) other active pharmaceutically active ingredient.

Embodiment No. 7 is directed to a pharmaceutical composition of any one of Embodiment Nos. 1 to 4 further comprising an effective amount of another (i.e., one other) pharmaceutically active ingredient.

Embodiment No. 8 is directed to a pharmaceutical composition of any one of Embodiment Nos. 1 to 4 further comprising an effective amount of at least one (e.g., 1, 2 or 3, or 1 or 2, or 1, and usually 1) chemotherapeutic agent.

Embodiment No. 9 is directed to a pharmaceutical composition of any one of Embodiment Nos. 1 to 4 further comprising an effective amount of a chemotherapeutic agent.

Disclosed is a method of treating cancer in a patient in need of such treatment, said method comprising administering to said patient an effective amount of at least one (1, 2 or 3, or 1 or 2, or 1, and usually 1) claimed compound.

Also disclosed is a method of treating cancer in a patient in need of such treatment, said method comprising administering to said patient an effective amount of one claimed compound.

Further disclosed is a method of treating cancer further comprising the administration of an effective amount of at least one (1, 2 or 3, or 1 or 2, or 1, and usually 1) chemotherapeutic agent.

Also disclosed is a method of treating cancer wherein the further chemotherapeutic agent is selected from the group consisting of: paclitaxel, docetaxel, carboplatin, cisplatin, gemcitabine, tamoxifen, Herceptin, Cetuximab, Tarceva, Iressa, bevacizumab, navelbine, IMC-1C11, SU5416 and SU6688.

Further disclosed is a method of treating cancer wherein the further chemotherapeutic agent is selected from the group consisting of: paclitaxel, docetaxel, carboplatin, cisplatin, navelbine, gemcitabine, and Herceptin.

Also disclosed is a method of treating cancer wherein the further chemotherapeutic agent is selected from the group consisting of: Cyclophasphamide, 5-Fluorouracil, Temozolomide, Vincristine, Cisplatin, Carboplatin, and Gemcitabine.

Further disclosed is a method of treating cancer wherein the further chemotherapeutic agent is selected from the group consisting of: Gemcitabine, Cisplatin and Carboplatin.

Disclosed is a method of treating cancer in a patient in need of such treatment, said treatment comprising administering to said patient a therapeutically effective amount at least one (e.g., 1, 2 or 3, or 1 or 2, or 1, and usually 1) claimed compound and therapeutically effective amounts of at least one (e.g., 1, 2 or 3, or 1 or 2, or 2, or 1) chemotherapeutic agent selected from the group consisting of: (1) taxanes, (2) platinum coordinator compounds, (3) epidermal growth factor (EGF) inhibitors that are antibodies, (4) EGF inhibitors that are small molecules, (5) vascular endolithial growth factor (VEGF) inhibitors that are antibodies, (6) VEGF kinase inhibitors that are small molecules, (7) estrogen receptor antagonists or selective estrogen receptor modulators (SERMs), (8) anti-tumor nucleoside derivatives, (9) epothilones, (10) topoisomerase inhibitors, (11) vinca alkaloids, (12) antibodies that are inhibitors of αVβ3 integrins, (13) folate antagonists, (14) ribonucleotide reductase inhibitors, (15) anthracyclines, (16) biologics; (17) inhibitors of angiogenesis and/or suppressors of tumor necrosis factor alpha (TNF-alpha) such as thalidomide (or related imid), (18) Bcr/abl kinase inhibitors, (19) MEK1 and/or MEK 2 inhibitors that are small molecules, (20) IGF-1 and IGF-2 inhibitors that are small molecules, (21) small molecule inhibitors of RAF and BRAF kinases, (22) small molecule inhibitors of cell cycle dependent kinases such as CDK1, CDK2, CDK4 and CDK6, (23) alkylating agents, and (24) farnesyl protein transferase inhibitors (also know as FPT inhibitors or FTI (i.e., farnesyl transfer inhibitors)).

Also disclosed is a method of treating cancer in a patient in need of such treatment, said treatment comprising administering to said patient a therapeutically effective amount at least one (e.g., 1, 2 or 3, or 1 or 2, or 1, and usually 1) claimed compound, and therapeutically effective amounts of at least two (e.g., 2 or 3, or 2, and usually 2) different antineoplastic agents selected from the group consisting of: (1) taxanes, (2) platinum coordinator compounds, (3) epidermal growth factor (EGF) inhibitors that are antibodies, (4) EGF inhibitors that are small molecules, (5) vascular endolithial growth factor (VEGF) inhibitors that are antibodies, (6) VEGF kinase inhibitors that are small molecules, (7) estrogen receptor antagonists or selective estrogen receptor modulators (SERMs), (8) anti-tumor nucleoside derivatives, (9) epothilones, (10) topoisomerase inhibitors, (11) vinca alkaloids, (12) antibodies that are inhibitors of αVβ3 integrins, (13) folate antagonists, (14) ribonucleotide reductase inhibitors, (15) anthracyclines, (16) biologics; (17) inhibitors of angiogenesis and/or suppressors of tumor necrosis factor alpha (TNF-alpha) such as thalidomide (or related imid), (18) Bcr/abl kinase inhibitors, (19) MEK1 and/or MEK 2 inhibitors that are small molecules, (20) IGF-1 and IGF-2 inhibitors that are small molecules, (21) small molecule inhibitors of RAF and BRAF kinases, (22) small molecule inhibitors of cell cycle dependent kinases such as CDK1, CDK2, CDK4 and CDK6, (23) alkylating agents, and (24) farnesyl protein transferase inhibitors (also know as FPT inhibitors or FTI (i.e., farnesyl transfer inhibitors)).

Further disclosed is a method of treating cancer in a patient in need of such treatment, said method comprising administering to said patient therapeutically effective amounts at least one (e.g., 1, 2 or 3, or 1 or 2, or 1, and usually 1) claimed compound and an antineoplastic agent selected from the group consisting of: (1) EGF inhibitors that are antibodies, (2) EGF inhibitors that are small molecules, (3) VEGF inhibitors that are antibodies, and (4) VEGF inhibitors that are small molecules. Radiation therapy can also be used in conjunction with this above combination therapy, i.e., the above method using a combination of compounds of the invention and antineoplastic agent can also comprise the administration of a therapeutically effect amount of radiation.

Also disclosed is a method of treating leukemias (e.g., acute myeloid leukemia (AML), and chronic myeloid leukemia (CML)) in a patient in need of such treatment, said method comprising administering to said patient therapeutically effective amounts at least one (e.g., 1, 2 or 3, or 1 or 2, or 1, and usually 1) claimed compound and: (1) Gleevec and interferon to treat CML; (2) Gleevec and pegylated interferon to treat CML; (3) Gleevec to treat CML; (4) an anti-tumor nucleoside derivative (e.g., Ara-C) to treat AML; or (5) an anti-tumor nucleoside derivative (e.g., Ara-C) in combination with an anthracycline to treat AML.

Further disclosed is a method of treating non-Hodgkin's lymphoma in a patient in need of such treatment, said method comprising administering therapeutically effective amounts at least one (e.g., 1, 2 or 3, or 1 or 2, or 1, and usually 1) claimed compound and: (1) a biologic (e.g., Rituxan); (2) a biologic (e.g., Rituxan) and an anti-tumor nucleoside derivative (e.g., Fludarabine); or (3) Genasense (antisense to BCL-2).

Also disclosed is a method of treating multiple myeloma in a patient in need of such treatment, said method comprising administering to said patient therapeutically effective amounts of at least one (e.g., 1, 2 or 3, or 1 or 2, or 1, and usually 1) claimed compound and: (1) a proteosome inhibitor (e.g., PS-341 from Millenium); or (2) Thalidomide (or related imid).

Further disclosed is a method of treating cancer in a patient in need of such treatment, said method comprising administering to said patient therapeutically effective amounts of: (a) at least one (e.g., 1, 2 or 3, or 1 or 2, or 1, and usually 1) claimed compound and (b) at least one (e.g., 1, 2 or 3, or 1 or 2, or 2, or 1) antineoplastic agent selected from the group consisting of: (1) taxanes, (2) platinum coordinator compounds, (3) EGF inhibitors that are antibodies, (4) EGF inhibitors that are small molecules, (5) VEGF inhibitors that are antibodies, (6) VEGF kinase inhibitors that are small molecules, (7) estrogen receptor antagonists or selective estrogen receptor modulators, (8) anti-tumor nucleoside derivatives, (9) epothilones, (10) topoisomerase inhibitors, (11) vinca alkaloids, and (12) antibodies that are inhibitors of αVβ3 integrins.

Also disclosed is a method of treating non small cell lung cancer in a patient in need of such treatment, said method comprising administering to said patient therapeutically effective amounts of: (a) at least one (e.g., 1, 2 or 3, or 1 or 2, or 1, and usually 1) claimed compound and (b) at least one (e.g., 1, 2 or 3, or 1 or 2, or 2, or 1) antineoplastic agent selected from the group consisting of: (1) taxanes, (2) platinum coordinator compounds, (3) EGF inhibitors that are antibodies, (4) EGF inhibitors that are small molecules, (5) VEGF inhibitors that are antibodies, (6) VEGF kinase inhibitors that are small molecules, (7) estrogen receptor antagonists or selective estrogen receptor modulators, (8) anti-tumor nucleoside derivatives, (9) epothilones, (10) topoisomerase inhibitors, (11) vinca alkaloids, and (12) antibodies that are inhibitors of αVβ3 integrins.

Further disclosed is a method of treating non small cell lung cancer in a patient in need of such treatment, said method comprising administering to said patient therapeutically effective amounts of: (a) at least one (e.g., 1, 2 or 3, or 1 or 2, or 1, and usually 1) claimed compound and (b) at least one (e.g., 1, 2 or 3, or 1 or 2, or 2, or 1) antineoplastic agent selected from the group consisting of: (1) taxanes, (2) platinum coordinator compounds, (3) anti-tumor nucleoside derivatives, (4) topoisomerase inhibitors, and (5) vinca alkaloids.

Also disclosed is a method of treating non small cell lung cancer in a patient in need of such treatment, said method comprising administering therapeutically effective amounts of: (a) at least one (e.g., 1, 2 or 3, or 1 or 2, or 1, and usually 1) claimed compound (b) carboplatin, and (c) paclitaxel.

Further disclosed is a method of treating non small cell lung cancer in a patient in need of such treatment, said method comprising administering to said patient therapeutically effective amounts of: (a) at least one (e.g., 1, 2 or 3, or 1 or 2, or 1, and usually 1) claimed compound (b) cisplatin, and (c) gemcitabine.

Also disclosed is a method of treating non small cell lung cancer in a patient in need of such treatment, said method comprising administering therapeutically effective amounts of: (a) at least one (e.g., 1, 2 or 3, or 1 or 2, or 1, and usually 1) claimed compound, (b) carboplatin, and (c) gemcitabine.

Further disclosed is a method of treating non small cell lung cancer in a patient in need of such treatment, said method comprising administering therapeutically effective amounts of: (a) at least one (e.g., 1, 2 or 3, or 1 or 2, or 1, and usually 1) claimed compound, (b) Carboplatin, and (c) Docetaxel.

Also disclosed is a method of treating cancer in a patient in need of such treatment, said method comprising administering therapeutically effective amounts of: (a) at least one (e.g., 1, 2 or 3, or 1 or 2, or 1, and usually 1) claimed compound, and (b) an antineoplastic agent selected from the group consisting of: (1) EGF inhibitors that are antibodies, (2) EGF inhibitors that are small molecules, (3) VEGF inhibitors that are antibodies, (4) VEGF kinase inhibitors that are small molecules.

Further disclosed is a method of treating squamous cell cancer of the head and neck, in a patient in need of such treatment, said method comprising administering to said patient therapeutically effective amounts of: (a) at least one (e.g., 1, 2 or 3, or 1 or 2, or 1, and usually 1) claimed compound, and (b) at least one (e.g., 1, 2 or 3, or 1 or 2, or 2, or 1) antineoplastic agent selected from the group consisting of: (1) taxanes, and (2) platinum coordinator compounds.

Also disclosed is a method of treating squamous cell cancer of the head and neck, in a patient in need of such treatment, said method comprising administering to said patient therapeutically effective amounts of: (a) at least one (e.g., 1, 2 or 3, or 1 or 2, or I, and usually 1) claimed compound, and (b) at least one (e.g., 1, 2 or 3, or 1 or 2, or 2, or 1) antineoplastic agent selected from the group consisting of: (1) taxanes, (2) platinum coordinator compounds, and (3) anti-tumor nucleoside derivatives (e.g., 5-Fluorouracil).

Further disclosed is a method of treating CML in a patient in need of such treatment, said method comprising administering therapeutically effective amounts of: (a) at least one (e.g., 1, 2 or 3, or 1 or 2, or 1, and usually 1) claimed compound, (b) Gleevec, and (c) interferon (e.g., Intron-A).

Also disclosed is a method of treating CML in a patient in need of such treatment comprising administering therapeutically effective amounts of: (a) at least one (e.g., 1, 2 or 3, or 1 or 2, or 1, and usually 1) claimed compound, (b) Gleevec; and (c) pegylated interferon (e.g., Peg-Intron, and Pegasys).

Further disclosed is a method of treating CML in a patient in need of such treatment comprising administering therapeutically effective amounts of: (a) at least one (e.g., 1, 2 or 3, or 1 or 2, or 1, and usually 1) claimed compound and (b) Gleevec.

Also disclosed is a method of treating CMML in a patient in need of such treatment, said method comprising administering to said patient therapeutically effective amounts of at least one (e.g., 1, 2 or 3, or 1 or 2, or 1, and usually 1) claimed compound.

Further disclosed is a method of treating AML in a patient in need of such treatment, said method comprising administering to said patient therapeutically effective amounts of: (a) at least one (e.g., 1, 2 or 3, or 1 or 2, or 1, and usually 1) claimed compound, and (b) an anti-tumor nucleoside derivative (e.g., Cytarabine (i.e., Ara-C)).

Also disclosed is a method of treating AML in a patient in need of such treatment, said method comprising administering to said patient therapeutically effective amounts of: (a) at least one (e.g., 1, 2 or 3, or 1 or 2, or 1, and usually 1) claimed compound, (b) an anti-tumor nucleoside derivative (e.g., Cytarabine (i.e., Ara-C)), and (c) an anthracycline.

Further disclosed is a method of treating non-Hodgkin's lymphoma in a patient in need of such treatment, said method comprising administering to said patient therapeutically effective amounts of: (a) at least one (e.g., 1, 2 or 3, or 1 or 2, or 1, and usually 1) claimed compound, and (b) Rituximab (Rituxan).

Also disclosed is a method of treating non-Hodgkin's lymphoma in a patient in need of such treatment, said method comprising administering to said patient therapeutically effective amounts of: (a) at least one (e.g., 1, 2 or 3, or 1 or 2, or 1, and usually 1) claimed compound, (b) Rituximab (Rituxan), and (c) an anti-tumor nucleoside derivative (e.g., Fludarabine (i.e., F-ara-A).

Further disclosed is a method of treating non-Hodgkin's lymphoma in a patient in need of such treatment, said method comprising administering to said patient therapeutically effective amounts of: (a) at least one (e.g., 1, 2 or 3, or 1 or 2, or 1, and usually 1) claimed compound, and (b) Genasense (antisense to BCL-2).

Also disclosed is a method of treating multiple myeloma in a patient in need of such treatment, said method comprising administering therapeutically effective amounts of: (a) at least one (e.g., 1, 2 or 3, or 1 or 2, or 1, and usually 1) claimed compound, and (b) a proteosome inhibitor (e.g., PS-341 (Millenium)).

Further disclosed is a method of treating multiple myeloma in a patient in need of such treatment, said method comprising administering to said patient therapeutically effective amounts of: (a) at least one (e.g., 1, 2 or 3, or 1 or 2, or 1, and usually 1) claimed compound, and (b) Thalidomide or related imid.

Also disclosed is a method of treating multiple myeloma in a patient in need of such treatment, said method comprising administering therapeutically effective amounts of: (a) at least one (e.g., 1, 2 or 3, or 1 or 2, or 1, and usually 1) claimed compound, and (b) Thalidomide.

Further disclosed are methods of treating cancer described herein, particularly those described above, wherein in addition to the administration of the claimed compound and antineoplastic agents, radiation therapy is also administered prior to, during, or after the treatment cycle.

Also disclosed is a method for treating cancer (e.g., lung cancer, prostate cancer and myeloid leukemias) in a patient in need of such treatment, said method comprising administering to said patient (1) an effective amount of at least one (e.g., 1, 2 or 3, or 1 or 2, or 1, and usually 1) claimed compound, in combination with (2) at least one (e.g., 1, 2 or 3, or 1 or 2, or 2, or 1) antineoplastic agent, microtubule affecting agent and/or radiation therapy.

Further disclosed a method of treating cancer in a patient in need of such treatment, said method comprising administering to said patient an effective amount of at least one (e.g., 1, 2 or 3, or 1 or 2, or 1, and usually 1) claimed compound in combination with an effective amount of at least one (e.g., 1, 2 or 3, or 1 or 2, or 1, and usually 1) signal transduction inhibitor.

Thus, in one example (e.g., treating non small cell lung cancer): (1) the claimed compound is administered in an amount of about 50 mg to about 200 mg twice a day, and in another example about 75 mg to about 125 mg administered twice a day, and in yet another example about 100 mg administered twice a day, (2) Paclitaxel (e.g., Taxol® is administered once per week in an amount of about 50 to about 100 mg/m², and in another example about 60 to about 80 mg/m², and (3) Carboplatin is administered once per week in an amount to provide an AUC of about 2 to about 3.

In another example (e.g., treating non small cell lung cancer): (1) the claimed compound is administered in an amount of about 50 mg to about 200 mg twice a day, and in another example about 75 mg to about 125 mg administered twice a day, and yet in another example about 100 mg administered twice a day, (2) Paclitaxel (e.g., Taxol® is administered once per week in an amount of about 50 to about 100 mg/m², and in another example about 60 to about 80 mg/m², and (3) Cisplatin is administered once per week in an amount of about 20 to about 40 mg/m².

In another example (e.g., treating non small cell lung cancer): (1) the claimed compound is administered in an amount of about 50 mg to about 200 mg twice a day, and in another example about 75 mg to about 125 mg administered twice a day, and in yet another example about 100 mg administered twice a day, (2) Docetaxel (e.g., Taxotere®) is administered once per week in an amount of about 10 to about 45 mg/m², and (3) Carboplatin is administered once per week in an amount to provide an AUC of about 2 to about 3.

In another example (e.g., treating non small cell lung cancer): (1) the claimed compound is administered in an amount of about 50 mg to about 200 mg twice a day, and in another example about 75 mg to about 125 mg administered twice a day, and in yet another example about 100 mg administered twice a day, (2) Docetaxel (e.g., Taxotere®) is administered once per week in an amount of about 10 to about 45 mg/m², and (3) Cisplatin is administered once per week in an amount of about 20 to about 40 mg/m².

In another example (e.g., treating non small cell lung cancer): (1) the claimed compound is administered in an amount of about 50 mg to about 200 mg twice a day, and in another example about 75 mg to about 125 mg administered twice a day, and in yet another example about 100 mg administered twice a day, (2) Paclitaxel (e.g., Taxol® is administered once every three weeks in an amount of about 150 to about 250 mg/m², and in another example about 175 to about 225 mg/m², and in yet another example 175 mg/m², and (3) Carboplatin is administered once every three weeks in an amount to provide an AUC of about 5 to about 8, and in another example 6.

In another example of treating non small cell lung cancer: (1) the claimed compound is administered in an amount of 100 mg administered twice a day, (2) Paclitaxel (e.g., Taxol® is administered once every three weeks in an amount of 175 mg/m², and (3) Carboplatin is administered once every three weeks in an amount to provide an AUC of 6.

In another example (e.g., treating non small cell lung cancer): (1) the claimed compound is administered in an amount of about 50 mg to about 200 mg twice a day, and in another example about 75 mg to about 125 mg administered twice a day, and in yet another example about 100 mg administered twice a day, (2) Paclitaxel (e.g., Taxol® is administered once every three weeks in an amount of about 150 to about 250 mg/m², and in another example about 175 to about 225 mg/m², and (3) Cisplatin is administered once every three weeks in an amount of about 60 to about 100 mg/m².

In another example (e.g., treating non small cell lung cancer): (1) the claimed compound is administered in an amount of about 50 mg to about 200 mg twice a day, and in another example about 75 mg to about 125 mg administered twice a day, and in yet another example about 100 mg administered twice a day, (2) Docetaxel (e.g., Taxotere® is administered once every three weeks in an amount of about 50 to about 100 mg/m², and (3) Carboplatin is administered once every three weeks in an amount to provide an AUC of about 5 to about 8.

In another example (e.g., treating non small cell lung cancer): (1) the claimed compound is administered in an amount of about 50 mg to about 200 mg twice a day, in another example about 75 mg to about 125 mg administered twice a day, and in yet another example about 100 mg administered twice a day, (2) Docetaxel (e.g., Taxotere® is administered once every three weeks in an amount of about 50 to about 100 mg/m², and (3) Cisplatin is administered once every three weeks in an amount of about 60 to about 100 mg/m².

In another example for treating non small cell lung cancer using the claimed compounds Docetaxel and Carboplatin: (1) the claimed compound is administered in an amount of about 50 mg to about 200 mg twice a day, and in another example about 75 mg to about 125 mg administered twice a day, and in yet another example about 100 mg administered twice a day, (2) Docetaxel (e.g., Taxotere® is administered once every three weeks in an amount of about 75 mg/m², and (3) Carboplatin is administered once every three weeks in an amount to provide an AUC of about 6.

In another example of the treatments of non-small cell lung cancer described above the Docetaxel (e.g., Taxotere® and Cisplatin, the Docetaxel (e.g., Taxotere® and Carboplatin, the Paclitaxel (e.g., Taxol® and Carboplatin, or the Paclitaxel (e.g., Taxol® and Cisplatin are administered on the same day.

In another example (e.g., CML): (1) the claimed compound is administered in an amount of about 100 mg to about 200 mg administered twice a day, (2) Gleevec is administered in an amount of about 400 to about 800 mg/day orally, and (3) interferon (Intron-A) is administered in an amount of about 5 to about 20 million IU three times per week.

In another example (e.g., CML): (1) the claimed compound is administered in an amount of about 100 mg to about 200 mg administered twice a day, (2) Gleevec is administered in an amount of about 400 to about 800 mg/day orally, and (3) pegylated interferon (Peg-Intron or Pegasys) is administered in an amount of about 3 to about 6 micrograms/kg/day.

In another example (e.g., non-Hodgkin's lymphoma): (1) the claimed compound is administered in an amount of about 50 mg to about 200 mg twice a day, and in another example about 75 mg to about 125 mg administered twice a day, and in yet another example about 100 mg administered twice a day, and (2) Genasense (antisense to BCL-2) is administered as a continuous IV infusion at a dose of about 2 to about 5 mg/kg/day (e.g., 3 mg/kg/day) for 5 to 7 days every 3 to 4 weeks.

In another example (e.g., multiple myeloma): (1) the claimed compound is administered in an amount of about 50 mg to about 200 mg twice a day, and in another example about 75 mg to about 125 mg administered twice a day, and in yet another example about 100 mg administered twice a day, and (2) the proteosome inhibitor (e.g., PS-341 - Millenium) is administered in an amount of about 1.5mg/m² twice weekly for two consecutive weeks with a one week rest period.

In another example (e.g., multiple myeloma): (1) the claimed compound is administered in an amount of about 50 mg to about 200 mg twice a day, and in another example about 75 mg to about 125 mg administered twice a day, and in yet another example about 100 mg administered twice a day, and (2) the Thalidomide (or related imid) is administered orally in an amount of about 200 to about 800 mg/day, with dosing being continuous until relapse or toxicity.

In one embodiment of the disclosed methods of treating cancer the chemotherapeutic agents are selected from the group consisting of: paclitaxel, docetaxel, carboplatin, cisplatin, gemcitabine, tamoxifen, Herceptin, Cetuximab, Tarceva, Iressa, bevacizumab, navelbine, IMC-1C11, SU5416 and SU6688.

In another embodiment of the disclosed methods, the chemotherapeutic agents are selected from the group consisting of: paclitaxel, docetaxel, carboplatin, cisplatin, navelbine, gemcitabine, and Herceptin.

Thus, described is a method of treating cancer comprising administering to a patient in need of such treatment therapeutically effective amounts of the claimed compound, a taxane, and a platinum coordination compound.

Also disclosed is a method of treating cancer comprising administering to a patient in need of such treatment therapeutically effective amounts of the claimed compound, a taxane, and a platinum coordination compound, wherein said claimed compound is administered every day, said taxane is administered once per week per cycle, and said platinum coordinator compound is administered once per week per cycle. In another embodiment the treatment is for one to four weeks per cycle.

Further disclosed is a method of treating cancer comprising administering to a patient in need of such treatment therapeutically effective amounts of the claimed compound, a taxane, and a platinum coordination compound, wherein said claimed compound is administered every day, said taxane is administered once every three weeks per cycle, and said platinum coordinator compound is administered once every three weeks per cycle. In another embodiment the treatment is for one to three weeks per cycle.

Also disclosed is a method of treating cancer comprising administering to a patient in need of such treatment therapeutically effective amounts of the claimed compound, paclitaxel, and carboplatin. In another embodiment, said claimed compound is administered every day, said paclitaxel is administered once per week per cycle, and said carboplatin is administered once per week per cycle. In another embodiment the treatment is for one to four weeks per cycle.

Further disclosed is a method of treating cancer comprising administering to a patient in need of such treatment therapeutically effective amounts of the claimed compound, paclitaxel, and carboplatin. In another embodiment, said claimed compound is administered every day, said paclitaxel is administered once every three weeks per cycle, and said carboplatin is administered once every three weeks per cycle. In another embodiment the treatment is for one to three weeks per cycle.

Also disclosed is a method for treating non small cell lung cancer in a patient in need of such treatment comprising administering daily a therapeutically effective amount of the claimed compound, administering a therapeutically effective amount of carboplatin once a week per cycle, and administering a therapeutically effective amount of paclitaxel once a week per cycle, wherein the treatment is given for one to four weeks per cycle. In another embodiment said claimed compound is administered twice per day. In another embodiment said carboplatin and said paclitaxel are administered on the same day, and in another embodiment said carboplatin and said paclitaxel are administered consecutively, and in another embodiment said carboplatin is administered after said paclitaxel.

Further disclosed is a method for treating non small cell lung cancer in a patient in need of such treatment comprising administering daily a therapeutically effective amount of a claimed compound, administering a therapeutically effective amount of carboplatin once every three weeks per cycle, and administering a therapeutically effective amount of paclitaxel once every three weeks per cycle, wherein the treatment is given for one to three weeks. In another embodiment said claimed compound is administered twice per day. In another embodiment said carboplatin and said paclitaxel are administered on the same day, and in another embodiment said carboplatin and said paclitaxel are administered consecutively, and in another embodiment said carboplatin is administered after said paclitaxel.

Also disclosed is a method for treating non small cell lung cancer in a patient in need of such treatment comprising administering about 50 to about 200 mg of a claimed compound twice a day, administering carboplatin once per week per cycle in an amount to provide an AUC of about 2 to about 8 (and in another embodiment about 2 to about 3), and administering once per week per cycle about 60 to about 300 mg/m² (and in another embodiment about 50 to 100mg/m², and in yet another embodiment about 60 to about 80 mg/m²) of paclitaxel, wherein the treatment is given for one to four weeks per cycle. In another embodiment said claimed compound is administered in amount of about 75 to about 125 mg twice a day, and in another embodiment about 100 mg twice a day. In another embodiment said carboplatin and said paclitaxel are administered on the same day, and in another embodiment said carboplatin and said paclitaxel are administered consecutively, and in another embodiment said carboplatin is administered after said paclitaxel.

Further disclosed is a method for treating non small cell lung cancer in a patient in need of such treatment comprising administering about 50 to about 200 mg of a claimed compound twice a day, administering carboplatin once every three weeks per cycle in an amount to provide an AUC of about 2 to about 8 (in another embodiment about 5 to about 8, and in another embodiment 6), and administering once every three weeks per cycle about 150 to about 250 mg/m² (and in another embodiment about 175 to about 225 mg/m², and in another embodiment 175 mg/m²) of paclitaxel, wherein the treatment is given for one to three weeks. In another embodiment said claimed compound is administered in an amount of about 75 to about 125 mg twice a day, and in another embodiment about 100 mg twice a day. In another embodiment said carboplatin and said paclitaxel are administered on the same day, and in another embodiment said carboplatin and said paclitaxel are administered consecutively, and in another embodiment said carboplatin is administered after said paclitaxel.

Also disclosed are methods of treating cancer as described in the above embodiments (i.e., the embodiments directed to treating cancer and to treating non small cell lung cancer with a taxane and platinum coordinator compound) except that in place of paclitaxel and carboplatin the taxanes and platinum coordinator compounds used together in the methods are: (1) docetaxel (Taxotere®) and cisplatin; (2) paclitaxel and cisplatin; and (3) docetaxel and carboplatin. In another embodiment of the methods of this invention cisplatin is used in amounts of about 30 to about 100 mg/m². In another embodiment of the disclosed methods docetaxel is used in amounts of about 30 to about 100 mg/m².

Further disclosed is a method of treating cancer comprising administering to a patient in need of such treatment therapeutically effective amounts of a claimed compound, a taxane, and an EGF inhibitor that is an antibody. In another embodiment the taxane used is paclitaxel, and the EGF inhibitor is a HER2 antibody (in one embodiment Herceptin) or Cetuximab, and in another embodiment Herceptin is used. The length of treatment, and the amounts and administration of said claimed compound and the taxane are as described in the embodiments above. The EGF inhibitor that is an antibody is administered once a week per cycle, and in another embodiment is administered on the same day as the taxane, and in another embodiment is administered consecutively with the taxane. For example, Herceptin is administered in a loading dose of about 3 to about 5 mg/m² (in another embodiment about 4 mg/m²), and then is administered in a maintenance dose of about 2 mg/m² once per week per cycle for the remainder of the treatment cycle (usually the cycle is 1 to 4 weeks). In one embodiment the cancer treated is breast cancer.

Also disclosed is a method of treating cancer comprising administering to a patient in need of such treatment therapeutically effective amounts of: (1) a claimed compound, (2) a taxane, and (3) an antineoplastic agent selected from the group consisting of: (a) an EGF inhibitor that is a small molecule, (b) a VEGF inhibitor that is an antibody, and (c) a VEGF kinase inhibitor that is a small molecule. In another embodiment, the taxane paclitaxel or docetaxel is used. In another embodiment the antineoplastic agent is selected from the group consisting of: tarceva, Iressa, bevacizumab, SU5416, SU6688 and BAY 43-9006. The length of treatment, and the amounts and administration of said claimed compound and the taxane are as described in the embodiments above. The VEGF kinase inhibitor that is an antibody is usually given once per week per cycle. The EGF and VEGF inhibitors that are small molecules are usually given daily per cycle. In another embodiment, the VEGF inhibitor that is an antibody is given on the same day as the taxane, and in another embodiment is administered concurrently with the taxane. In another embodiment, when the EGF inhibitor that is a small molecule or the VEGF inhibitor that is a small molecule is administered on the same day as the taxane, the administration is concurrently with the taxane. The EGF or VEGF kinase inhibitor is generally administered in an amount of about 10 to about 500 mg/m².

Further disclosed is a method of treating cancer comprising administering to a patient in need of such treatment therapeutically effective amounts of a claimed compound, an anti-tumor nucleoside derivative, and a platinum coordination compound.

Also disclosed is a method of treating cancer comprising administering to a patient in need of such treatment therapeutically effective amounts of a claimed compound, an anti-tumor nucleoside derivative, and a platinum coordination compound, wherein said claimed compound is administered every day, said anti-tumor nucleoside derivative is administered once per week per cycle, and said platinum coordinator compound is administered once per week per cycle. Although the treatment can be for one to four weeks per cycle, in one embodiment the treatment is for one to seven weeks per cycle.

Further disclosed is a method of treating cancer comprising administering to a patient in need of such treatment therapeutically effective amounts of a claimed compound, an anti-tumor nucleoside derivative, and a platinum coordination compound, wherein said claimed compound is administered every day, said an anti-tumor nucleoside derivative is administered once per week per cycle, and said platinum coordinator compound is administered once every three weeks per cycle. Although the treatment can be for one to four weeks per cycle, in one embodiment the treatment is for one to seven weeks per cycle.

Also disclosed is a method of treating cancer comprising administering to a patient in need of such treatment therapeutically effective amounts of a claimed compound, gemcitabine, and cisplatin. In another embodiment, said claimed compound is administered every day, said gemcitabine is administered once per week per cycle, and said cisplatin is administered once per week per cycle. In one embodiment the treatment is for one to seven weeks per cycle.

Further disclosed is a method of treating cancer comprising administering to a patient in need of such treatment therapeutically effective amounts of a claimed compound, gemcitabine, and cisplatin. In another embodiment, said claimed compound is administered every day, said gemcitabine is administered once per week per cycle, and said cisplatin is administered once every three weeks per cycle. In another embodiment the treatment is for one to seven weeks.

Also disclosed is a method of treating cancer comprising administering to a patient in need of such treatment therapeutically effective amounts of a claimed compound, gemcitabine, and carboplatin. In another embodiment said claimed compound is administered every day, said gemcitabine is administered once per week per cycle, and said carboplatin is administered once per week per cycle. In another embodiment the treatment is for one to seven weeks per cycle.

Further disclosed is a method of treating cancer comprising administering to a patient in need of such treatment therapeutically effective amounts of a claimed compound, gemcitabine, and carboplatin. In another embodiment said claimed compound is administered every day, said gemcitabine is administered once per week per cycle, and said carboplatin is administered once every three weeks per cycle. In another embodiment the treatment is for one to seven weeks per cycle.

In the above embodiments using gemcitabine, the claimed compound and the platinum coordinator compound are administered as described above for the embodiments using taxanes. Gemcitabine is administered in an amount of about 500 to about 1250 mg/m². In one embodiment the gemcitabine is administered on the same day as the platinum coordinator compound, and in another embodiment consecutively with the platinum coordinator compound, and in another embodiment the gemcitabine is administered after the platinum coordinator compound.

Also disclosed is a method of treating cancer in a patient in need of such treatment comprising administering to said patient a claimed compound and an antineoplastic agent selected from: (1) EGF inhibitors that are antibodies, (2) EGF inhibitors that are small molecules, (3) VEGF inhibitors that are antibodies, and (4) VEGF kinase inhibitors that are small molecules all as described above. The treatment is for one to seven weeks per cycle, and generally for one to four weeks per cycle. The claimed compound is administered in the same manner as described above for the other embodiments of this invention. The small molecule antineoplastic agents are usually administered daily, and the antibody antineoplastic agents are usually administered once per week per cycle. In one embodiment the antineoplastic agents are selected from the group consisting of: Herceptin, Cetuximab, Tarceva, Iressa, bevacizumab, IMC-1C11, SU5416, SU6688 and BAY 43-9006.

In the embodiments wherein a platinum coordinator compound is used as well as at least one other antineoplastic agent, and these drugs are administered consecutively, the platinum coordinator compound is generally administered after the other antineoplastic agents have been administered.

Other embodiments include the administration of a therapeutically effective amount of radiation to the patient in addition to the administration of a claimed compound and antineoplastic agents in the embodiments described above. Radiation is administered according to techniques and protocols well know to those skilled in the art.

Another embodiment of this invention is directed to a pharmaceutical composition comprising a claimed compound and at least two different antineoplastic agents and a pharmaceutically acceptable carrier for intravenous administration. Preferably the pharmaceutically acceptable carrier is an isotonic saline solution (0.9% NaCl) or a dextrose solution (e.g., 5% dextrose).

Another embodiment of this invention is directed to a pharmaceutical composition comprising a claimed compound and at least one antineoplastic agent and a pharmaceutically acceptable carrier for intravenous administration. Preferably the pharmaceutically acceptable carrier is an isotonic saline solution (0.9% NaCl) or a dextrose solution (e.g., 5% dextrose).

Thus disclosed is a method of treating (or preventing) breast cancer (i.e., postmenopausal and premenopausal breast cancer, e.g., hormone-dependent breast cancer) in a patient in need of such treatment comprising administering to said patient a therapeutically effective amount of at least one (e.g., one) claimed compound and a therapeutically effective amount of at least one antihormonal agent selected from the group consisting of: (a) aromatase inhibitors, (b) antiestrogens, and (c) LHRH analogues; and said treatment optionally including the administration of at least one chemotherapeutic agent.

The claimed compound is preferably administered orally, and in one embodiment is administered in capsule form.

Examples of aromatase inhibitors include but are not limited to: Anastrozole (e.g., Arimidex), Letrozole (e.g., Femara), Exemestane (Aromasin), Fadrozole and Formestane (e.g., Lentaron).

Examples of antiestrogens include but are not limited to: Tamoxifen (e.g., Nolvadex), Fulvestrant (e.g., Faslodex), Raloxifene (e.g., Evista), and Acolbifene.

Examples of LHRH analogues include but are not limited to: Goserelin (e.g., Zoladex) and Leuprolide (e.g., Leuprolide Acetate, such as Lupron or Lupron Depot).

Examples of chemotherapeutic agents include but are not limited to: Trastuzumab (e.g., Herceptin), Gefitinib (e.g., Iressa), Erlotinib (e.g., Erlotinib HCl, such as Tarceva), Bevacizumab (e.g., Avastin), Cetuximab (e.g., Erbitux), and Bortezomib (e.g., Velcade).

Preferably, when more than one antihormonal agent is used, each agent is selected from a different category of agent. For example, one agent is an aromatase inhibitor (e.g., Anastrozole, Letrozole, or Exemestane) and one agent is an antiestrogen (e.g., Tamoxifen or Fulvestrant).

Also disclosed is a method of treating or preventing breast cancer in a patient in need of such treatment wherein said treatment comprises administering a therapeutically effective amount of at least one (e.g., one) claimed compound and at least one antihormonal agent selected from the group consisting of: (a) aromatase inhibitors, (b) antiestrogens, and (c) LHRH analogues; and administering an effective amount of at least one chemotherapeutic agent.

Further disclosed is a method of treating or preventing breast cancer in a patient in need of such treatment wherein said treatment comprises administering a therapeutically effective amount of at least one (e.g., one) claimed compound and at least one antihormonal agent selected from the group consisting of: (a) aromatase inhibitors, (b) antiestrogens, and (c) LHRH analogues.

Also disclosed is a method of treating or preventing breast cancer in a patient in need of such treatment wherein said treatment comprises administering a therapeutically effective amount of at least one (e.g., one) claimed compound and at least one antihormonal agent selected from the group consisting of: (a) aromatase inhibitors, and (b) antiestrogens.

Further disclosed is a method of treating or preventing breast cancer in a patient in need of such treatment wherein said treatment comprises administering a therapeutically effective amount of at least one (e.g., one) claimed compound, at least one antihormonal agent selected from the group consisting of: (a) aromatase inhibitors and (b) antiestrogens; and at least one chemotherapeutic agent.

Also disclosed is a method of treating or preventing breast cancer in a patient in need of such treatment wherein said treatment comprises administering a therapeutically effective amount of at least one (e.g., one) claimed compound and at least one aromatase inhibitor.

Further disclosed is a method of treating or preventing breast cancer in a patient in need of such treatment wherein said treatment comprises administering a therapeutically effective amount of at least one (e.g., one) claimed compound, at least one aromatase inhibitor, and at least one chemotherapeutic agent.

Also disclosed is a method of treating or preventing breast cancer in a patient in need of such treatment wherein said treatment comprises administering a therapeutically effective amount of: (1) at least one (e.g., one) claimed compound; and (2) at least one antihormonal agent selected from the group consisting of: (a) aromatase inhibitors that are selected from the group consisting of Anastrozole, Letrozole, Exemestane, Fadrozole and Formestane, (b) antiestrogens that are selected from the group consisting of: Tamoxifen, Fulvestrant, Raloxifene, and Acolbifene, and (c) LHRH analogues that are selected from the group consisting of: Goserelin and Leuprolide; and administering an effective amount of at least one chemotherapeutic agent selected from the group consisting of: Trastuzumab, Gefitinib, Erlotinib, Bevacizumab, Cetuximab, and Bortezomib.

Further disclosed is a method of treating or preventing breast cancer in a patient in need of such treatment wherein said treatment comprises administering a therapeutically effective amount of: (1) at least one (e.g., one) claimed compound; and (2) at least one antihormonal agent selected from the group consisting of: (a) aromatase inhibitors that are selected from the group consisting of Anastrozole, Letrozole, Exemestane, Fadrozole and Formestane, (b) antiestrogens that are selected from the group consisting of: Tamoxifen, Fulvestrant, Raloxifene, and Acolbifene, and (c) LHRH analogues that are selected from the group consisting of: Goserelin and Leuprolide.

Also disclosed is a method of treating or preventing breast cancer in a patient in need of such treatment wherein said treatment comprises administering a therapeutically effective amount of: (1) at least one (e.g., one) claimed compound; and (2) at least one antihormonal agent selected from the group consisting of: (a) aromatase inhibitors that are selected from the group consisting of Anastrozole, Letrozole, Exemestane, Fadrozole and Formestane, and (b) antiestrogens that are selected from the group consisting of: Tamoxifen, Fulvestrant, Raloxifene, and Acolbifene.

Further disclosed is a method of treating or preventing breast cancer in a patient in need of such treatment wherein said treatment comprises administering a therapeutically effective amount of: (1) at least one (e.g., one) claimed compound; and (2) at least one antihormonal agent selected from the group consisting of: (a) aromatase inhibitors that are selected from the group consisting of Anastrozole, Letrozole, Exemestane, Fadrozole and Formestane, (b) antiestrogens that are selected from the group consisting of: Tamoxifen, Fulvestrant, Raloxifene, and Acolbifene; and administering an effective amount of at least one chemotherapeutic agents are selected from the group consisting of: Trastuzumab, Gefitinib, Erlotinib, Bevacizumab, Cetuximab, and Bortezomib.

Also disclosed is a method of treating or preventing breast cancer in a patient in need of such treatment wherein said treatment comprises administering a therapeutically effective amount of: (1) at least one (e.g., one) claimed compound; and (2) at least one aromatase inhibitor selected from the group consisting of Anastrozole, Letrozole, Exemestane, Fadrozole and Formestane.

Further disclosed is a method of treating or preventing breast cancer in a patient in need of such treatment wherein said treatment comprises administering a therapeutically effective amount of: (1) at least one (e.g., one) claimed compound; (2) at least one aromatase inhibitor that is selected from the group consisting of Anastrozole, Letrozole, Exemestane, Fadrozole and Formestane; and (3) administering an effective amount of at least one chemotherapeutic agent selected from the group consisting of: Trastuzumab, Gefitinib, Erlotinib, Bevacizumab, Cetuximab, and Bortezomib.

Also disclosed is a method of treating or preventing breast cancer in a patient in need of such treatment wherein said treatment comprises administering a therapeutically effective amount of: (1) at least one (e.g., one) claimed compound; (2) at least one aromatase inhibitor; and (3) at least one LHRH analogue.

Further disclosed is a method of treating or preventing breast cancer in a patient in need of such treatment wherein said treatment comprises administering a therapeutically effective amount of:(1) at least one (e.g., one) claimed compound; (2) at least one antiestrogen ; and (3) at least one LHRH analogue.

Also disclosed is a method of treating or preventing breast cancer in a patient in need of such treatment wherein said treatment comprises administering a therapeutically effective amount of: (1) at least one (e.g., one) claimed compound; (2) at least one aromatase inhibitor that is selected from the group consisting of Anastrozole, Letrozole, Exemestane, Fadrozole and Formestane; and (3) at least one LHRH analogue that is selected from the group consisting of: Goserelin and Leuprolide.

Further disclosed is a method of treating or preventing breast cancer in a patient in need of such treatment wherein said treatment comprises administering a therapeutically effective amount of: (1) at least one (e.g., one) claimed compound; (2) at least one antiestrogen that is selected from the group consisting of: Tamoxifen, Fulvestrant, Raloxifene, and Acolbifene; and (3) at least one LHRH analogue that is selected from the group consisting of: Goserelin and Leuprolide.

Also disclosed is a method of treating or preventing breast cancer in a patient in need of such treatment wherein said treatment comprises administering a therapeutically effective amount of at least one (e.g., one) claimed compound and Anastrozole.

Further disclosed is a method of treating or preventing breast cancer in a patient in need of such treatment wherein said treatment comprises administering a therapeutically effective amount of at least one (e.g., one) claimed compound and Letrazole.

Also disclosed is a method of treating or preventing breast cancer in a patient in need of such treatment wherein said treatment comprises administering a therapeutically effective amount of at least one (e.g., one) claimed compound and Exemestane.

Further disclosed is a method of treating or preventing breast cancer in a patient in need of such treatment wherein said treatment comprises administering a therapeutically effective amount of at least one (e.g., one) claimed compound and and Fadrozole.

Also disclosed is a method of treating or preventing breast cancer in a patient in need of such treatment wherein said treatment comprises administering a therapeutically effective amount of at least one (e.g., one) claimed compound and Formestane.

Further disclosed is a method of treating or preventing breast cancer in a patient in need of such treatment wherein said treatment comprises administering a therapeutically effective amount of at least one (e.g., one) claimed compound and Tamoxifen.

Also disclosed is a method of treating or preventing breast cancer in a patient in need of such treatment wherein said treatment comprises administering a therapeutically effective amount of at least one (e.g., one) claimed compound Fulvestrant.

Further disclosed is a method of treating or preventing breast cancer in a patient in need of such treatment wherein said treatment comprises administering a therapeutically effective amount of at least one (e.g., one) claimed compound and Raloxifene.

Also disclosed is a method of treating or preventing breast cancer in a patient in need of such treatment wherein said treatment comprises administering a therapeutically effective amount of at least one (e.g., one) claimed and Acolbifene.

Further disclosed is a method of treating or preventing breast cancer in a patient in need of such treatment wherein said treatment comprises administering a therapeutically effective amount of at least one (e.g., one) claimed compound and Goserelin.

Also disclosed is a method of treating or preventing breast cancer in a patient in need of such treatment wherein said treatment comprises administering a therapeutically effective amount of at least one (e.g., one) claimed compound and Leuprolide.

Further disclosed is a method of treating or preventing breast cancer in a patient in need of such treatment wherein said treatment comprises administering a therapeutically effective amount of at least one (e.g., one) claimed compound, Anastrozole, and an antiestrogen selected from the group consisting of: Tamoxifen, Fulvestrant, Raloxifene, and Acolbifene.

Also disclosed is a method of treating or preventing breast cancer in a patient in need of such treatment wherein said treatment comprises administering a therapeutically effective amount of at least one (e.g., one) claimed compound, Letrozole, and an antiestrogen selected from the group consisting of: Tamoxifen, Fulvestrant, Raloxifene, and Acolbifene.

Further disclosed is a method of treating or preventing breast cancer in a patient in need of such treatment wherein said treatment comprises administering a therapeutically effective amount of at least one (e.g., one) claimed compound, Exemestane, and an antiestrogen selected from the group consisting of: Tamoxifen, Fulvestrant, Raloxifene, and Acolbifene.

Also disclosed is a method of treating or preventing breast cancer in a patient in need of such treatment wherein said treatment comprises administering a therapeutically effective amount of at least one (e.g., one) claimed compound, Fadrozole, and an antiestrogen selected from the group consisting of: Tamoxifen, Fulvestrant, Raloxifene, and Acolbifene.

Further disclosed is a method of treating or preventing breast cancer in a patient in need of such treatment wherein said treatment comprises administering a therapeutically effective amount of at least one (e.g., one) claimed compound, Formestane, and an antiestrogen selected from the group consisting of: Tamoxifen, Fulvestrant, Raloxifene, and Acolbifene.

Also enclosed is a method of treating or preventing breast cancer in a patient in need of such treatment wherein said treatment comprises administering a therapeutically effective amount of at least one (e.g., one) claimed compound, Anastrozole, and Tamoxifen.

Further disclosed is a method of treating or preventing breast cancer in a patient in need of such treatment wherein said treatment comprises administering a therapeutically effective amount of at least one (e.g., one) claimed compound, Letrozole, and Tamoxifen.

Also disclosed is a method of treating or preventing breast cancer in a patient in need of such treatment wherein said treatment comprises administering a therapeutically effective amount of at least one (e.g., one) claimed compound, Exemestane, and Tamoxifen.

Further disclosed is a method of treating or preventing breast cancer in a patient in need of such treatment wherein said treatment comprises administering a therapeutically effective amount of at least one (e.g., one) claimed compound, Fadrozole, and Tamoxifen.

Also disclosed is a method of treating or preventing breast cancer in a patient in need of such treatment wherein said treatment comprises administering a therapeutically effective amount of at least one (e.g., one) claimed compound, Formestane, and Tamoxifen.

Further disclosed is a method of treating or preventing breast cancer in a patient in need of such treatment wherein said treatment comprises administering a therapeutically effective amount of at least one (e.g., one) claimed compound, Anastrozole, and Fulvestrant.

Also disclosed is a method of treating or preventing breast cancer in a patient in need of such treatment wherein said treatment comprises administering a therapeutically effective amount of at least one (e.g., one) claimed compound, Letrozole, and Fulvestrant.

Further disclosed is a method of treating or preventing breast cancer in a patient in need of such treatment wherein said treatment comprises administering a therapeutically effective amount of at least one (e.g., one) claimed compound, Exemestane, and Fulvestrant.

Also disclosed is a method of treating or preventing breast cancer in a patient in need of such treatment wherein said treatment comprises administering a therapeutically effective amount of at least one (e.g., one) claimed compound, Fadrozole, and Fulvestrant.

Further disclosed is a method of treating or preventing breast cancer in a patient in need of such treatment wherein said treatment comprises administering a therapeutically effective amount of at least one (e.g., one) claimed compound, Formestane, and Fulvestrant.

Also disclosed is a method of treating or preventing breast cancer in a patient in need of such treatment wherein said treatment comprises administering a therapeutically effective amount of at least one (e.g., one) claimed compound, Anastrozole, and a chemotherapeutic agent selected from the group consisting of: Trastuzumab, Gefitinib, Erlotinib, Bevacizumab, Cetuximab, and Bortezomib.

Further disclosed is a method of treating or preventing breast cancer in a patient in need of such treatment wherein said treatment comprises administering a therapeutically effective amount of at least one (e.g., one) claimed compound, Letrozole, and a chemotherapeutic agent selected from the group consisting of: Trastuzumab, Gefitinib, Erlotinib, Bevacizumab, Cetuximab, and Bortezomib.

Also disclosed is a method of treating or preventing breast cancer in a patient in need of such treatment wherein said treatment comprises administering a therapeutically effective amount of at least one (e.g., one) claimed compound, Exemestane, and a chemotherapeutic agent selected from the group consisting of: Trastuzumab, Gefitinib, Erlotinib, Bevacizumab, Cetuximab, and Bortezomib.

Further disclosed is a method of treating or preventing breast cancer in a patient in need of such treatment wherein said treatment comprises administering a therapeutically effective amount of at least one (e.g., one) claimed compound, Fadrozole, and a chemotherapeutic agent selected from the group consisting of: Trastuzumab, Gefitinib, Erlotinib, Bevacizumab, Cetuximab, and Bortezomib.

Also disclosed is a method of treating or preventing breast cancer in a patient in need of such treatment wherein said treatment comprises administering a therapeutically effective amount of at least one (e.g., one) claimed compound, Formestane, and a chemotherapeutic agent selected from the group consisting of: Trastuzumab, Gefitinib, Erlotinib, Bevacizumab, Cetuximab, and Bortezomib.

Further disclosed is a method of treating or preventing breast cancer in a patient in need of such treatment wherein said treatment comprises administering a therapeutically effective amount of at least one (e.g., one) claimed compound, Tamoxifen, and a chemotherapeutic agent selected from the group consisting of: Trastuzumab, Gefitinib, Erlotinib, Bevacizumab, Cetuximab, and Bortezomib.

Also disclosed is a method of treating or preventing breast cancer in a patient in need of such treatment wherein said treatment comprises administering a therapeutically effective amount of at least one (e.g., one) claimed compound, Fulvestrant, and a chemotherapeutic agent selected from the group consisting of: Trastuzumab, Gefitinib, Erlotinib, Bevacizumab, Cetuximab, and Bortezomib.

Further disclosed is a method of treating or preventing breast cancer in a patient in need of such treatment wherein said treatment comprises administering a therapeutically effective amount of at least one (e.g., one) claimed compound, Raloxifene, and a chemotherapeutic agent selected from the group consisting of: Trastuzumab, Gefitinib, Erlotinib, Bevacizumab, Cetuximab, and Bortezomib.

Also disclosed is a method of treating or preventing breast cancer in a patient in need of such treatment wherein said treatment comprises administering a therapeutically effective amount of at least one (e.g., one) claimed compound, Acolbifene, and a chemotherapeutic agent selected from the group consisting of: Trastuzumab, Gefitinib, Erlotinib, Bevacizumab, Cetuximab, and Bortezomib.

Further disclosed is a method of treating or preventing breast cancer in a patient in need of such treatment wherein said treatment comprises administering a therapeutically effective amount of at least one (e.g., one) claimed compound, Goserelin, and a chemotherapeutic agent selected from the group consisting of: Trastuzumab, Gefitinib, Erlotinib, Bevacizumab, Cetuximab, and Bortezomib.

Also disclosed is a method of treating or preventing breast cancer in a patient in need of such treatment wherein said treatment comprises administering a therapeutically effective amount of at least one (e.g., one) claimed compound, Leuprolein, and a chemotherapeutic agent selected from the group consisting of: Trastuzumab, Gefitinib, Erlotinib, Bevacizumab, Cetuximab, and Bortezomib.

Further disclosed is a method of treating or preventing breast cancer in a patient in need of such treatment wherein said treatment comprises administering a therapeutically effective amount of at least one (e.g., one) claimed compound, Anastrozole, an antiestrogen selected from the group consisting of: Tamoxifen, Fulvestrant, Raloxifene, and Acolbifene, and a chemotherapeutic agent selected from the group consisting of: Trastuzumab, Gefitinib, Erlotinib, Bevacizumab, Cetuximab, and Bortezomib.

Also disclosed is a method of treating or preventing breast cancer in a patient in need of such treatment wherein said treatment comprises administering a therapeutically effective amount of at least one (e.g., one) claimed compound, Letrozole, an antiestrogen selected from the group consisting of: Tamoxifen, Fulvestrant, Raloxifene, and Acolbifene, and a chemotherapeutic agent selected from the group consisting of: Trastuzumab, Gefitinib, Erlotinib, Bevacizumab, Cetuximab, and Bortezomib.

Further disclosed is a method of treating or preventing breast cancer in a patient in need of such treatment wherein said treatment comprises administering a therapeutically effective amount of at least one (e.g., one) claimed compound, Exemestane, an antiestrogen selected from the group consisting of: Tamoxifen, Fulvestrant, Raloxifene, and Acolbifene, and a chemotherapeutic agent selected from the group consisting of: Trastuzumab, Gefitinib, Erlotinib, Bevacizumab, Cetuximab, and Bortezomib.

Further disclosed is a method of treating or preventing breast cancer in a patient in need of such treatment wherein said treatment comprises administering a therapeutically effective amount of at least one (e.g., one) claimed compound, Fadrozole, an antiestrogen selected from the group consisting of: Tamoxifen, Fulvestrant, Raloxifene, and Acolbifene, and a chemotherapeutic agent selected from the group consisting of: Trastuzumab, Gefitinib, Erlotinib, Bevacizumab, Cetuximab, and Bortezomib.

Also disclosed is a method of treating or preventing breast cancer in a patient in need of such treatment wherein said treatment comprises administering a therapeutically effective amount of at least one (e.g., one) claimed compound, Formestane, an antiestrogen selected from the group consisting of: Tamoxifen, Fulvestrant, Raloxifene, and Acolbifene, and a chemotherapeutic agent selected from the group consisting of: Trastuzumab, Gefitinib, Erlotinib, Bevacizumab, Cetuximab, and Bortezomib.

Further disclosed is a method of treating or preventing breast cancer in a patient in need of such treatment wherein said treatment comprises administering a therapeutically effective amount of at least one (e.g., one) claimed compound, Anastrozole, Tamoxifen, and a chemotherapeutic agent selected from the group consisting of: Trastuzumab, Gefitinib, Erlotinib, Bevacizumab, Cetuximab, and Bortezomib.

Also disclosed is a method of treating or preventing breast cancer in a patient in need of such treatment wherein said treatment comprises administering a therapeutically effective amount of at least one (e.g., one) claimed compound, Letrozole, Tamoxifen, and a chemotherapeutic agent selected from the group consisting of: Trastuzumab, Gefitinib, Erlotinib, Bevacizumab, Cetuximab, and Bortezomib.

Also disclosed is a method of treating or preventing breast cancer in a patient in need of such treatment wherein said treatment comprises administering a therapeutically effective amount of at least one (e.g., one) claimed compound, Exemestane, Tamoxifen, and a chemotherapeutic agent selected from the group consisting of: Trastuzumab, Gefitinib, Erlotinib, Bevacizumab, Cetuximab, and Bortezomib.

Further disclosed is a method of treating or preventing breast cancer in a patient in need of such treatment wherein said treatment comprises administering a therapeutically effective amount of at least one (e.g., one) claimed compound, Fadrozole, Tamoxifen, and a chemotherapeutic agent selected from the group consisting of: Trastuzumab, Gefitinib, Erlotinib, Bevacizumab, Cetuximab, and Bortezomib.

Also disclosed is a method of treating or preventing breast cancer in a patient in need of such treatment wherein said treatment comprises administering a therapeutically effective amount of at least one (e.g., one) claimed compound, Formestane, Tamoxifen, and a chemotherapeutic agent selected from the group consisting of: Trastuzumab, Gefitinib, Erlotinib, Bevacizumab, Cetuximab, and Bortezomib.

Further disclosed is a method of treating or preventing breast cancer in a patient in need of such treatment wherein said treatment comprises administering a therapeutically effective amount of at least one (e.g., one) claimed compound, Anastrozole, Fulvestrant, and a chemotherapeutic agent selected from the group consisting of: Trastuzumab, Gefitinib, Erlotinib, Bevacizumab, Cetuximab, and Bortezomib.

Also disclosed is a method of treating or preventing breast cancer in a patient in need of such treatment wherein said treatment comprises administering a therapeutically effective amount of at least one (e.g., one) claimed compound, Letrozole, Fulvestrant, and a chemotherapeutic agent selected from the group consisting of: Trastuzumab, Gefitinib, Erlotinib, Bevacizumab, Cetuximab, and Bortezomib.

Further disclosed is a method of treating or preventing breast cancer in a patient in need of such treatment wherein said treatment comprises administering a therapeutically effective amount of at least one (e.g., one) claimed compound, Exemestane, Fulvestrant, and a chemotherapeutic agent selected from the group consisting of: Trastuzumab, Gefitinib, Erlotinib, Bevacizumab, Cetuximab, and Bortezomib.

Also disclosed is a method of treating or preventing breast cancer in a patient in need of such treatment wherein said treatment comprises administering a therapeutically effective amount of at least one (e.g., one) claimed compound, Fadrozole, Fulvestrant, and a chemotherapeutic agent selected from the group consisting of: Trastuzumab, Gefitinib, Erlotinib, Bevacizumab, Cetuximab, and Bortezomib.

Further disclosed is a method of treating or preventing breast cancer in a patient in need of such treatment wherein said treatment comprises administering a therapeutically effective amount of at least one (e.g., one) claimed compound, Formestane, Fulvestrant, and a chemotherapeutic agent selected from the group consisting of: Trastuzumab, Gefitinib, Erlotinib, Bevacizumab, Cetuximab, and Bortezomib.

Also disclosed is a method of treating or preventing breast cancer in a patient in need of such treatment wherein said treatment comprises administering a therapeutically effective amount of at least one (e.g., one) claimed compound, Goserelin and Tamoxifen.

Further disclosed is a method of treating or preventing breast cancer in a patient in need of such treatment wherein said treatment comprises administering a therapeutically effective amount of at least one (e.g., one) claimed compound, Goserelin, and Fulvestrant.

Also disclosed is a method of treating or preventing breast cancer in a patient in need of such treatment wherein said treatment comprises administering a therapeutically effective amount of at least one (e.g., one) claimed compound, Goserelin, and Raloxifene.

Further disclosed is a method of treating or preventing breast cancer in a patient in need ofsuch treatment wherein said treatment comprises administering a therapeutically effective amount of at least one (e.g., one) claimed compound, Goserelin and Acolbifene.

Also disclosed is a method of treating or preventing breast cancer in a patient in need of such treatment wherein said treatment comprises administering a therapeutically effective amount of at least one (e.g., one) claimed compound, Leuprolide, and Tamoxifen.

Further disclosed is a method of treating or preventing breast cancer in a patient in need of such treatment wherein said treatment comprises administering a therapeutically effective amount of at least one (e.g., one) claimed compound, Leuprolide, and Fulvestrant.

Also enclosed is a method of treating or preventing breast cancer in a patient in need of such treatment wherein said treatment comprises administering a therapeutically effective amount of at least one (e.g., one) claimed compound, Leuprolide, and Raloxifene.

Further disclosed is a method of treating or preventing breast cancer in a patient in need of such treatment wherein said treatment comprises administering a therapeutically effective amount of at least one (e.g., one) claimed compound, Leuprolide and Acolbifene.

Also disclosed is a method of treating or preventing breast cancer in a patient in need of such treatment wherein said treatment comprises administering a therapeutically effective amount of at least one (e.g., one) claimed compound, Goserelin and Anastrozole.

Further disclosed is a method of treating or preventing breast cancer in a patient in need of such treatment wherein said treatment comprises administering a therapeutically effective amount of at least one (e.g., one) claimed compound, Goserelin and Letrozole.

Also disclosed is a method of treating or preventing breast cancer in a patient in need of such treatment wherein said treatment comprises administering a therapeutically effective amount of at least one (e.g., one) claimed compound, Goserelin and Exemestane.

Further disclosed is a method of treating or preventing breast cancer in a patient in need of such treatment wherein said treatment comprises administering a therapeutically effective amount of at least one (e.g., one) claimed compound, Goserelin and Fadrozole.

Also disclosed is a method of treating or preventing breast cancer in a patient in need of such treatment wherein said treatment comprises administering a therapeutically effective amount of at least one (e.g., one) claimed compound, Goserelin and Formestane.

Further disclosed is a method of treating or preventing breast cancer in a patient in need of such treatment wherein said treatment comprises administering a therapeutically effective amount of at least one (e.g., one) claimed compound, Leuprolide and Anastrozole.

Also disclosed is a method of treating or preventing breast cancer in a patient in need of such treatment wherein said treatment comprises administering a therapeutically effective amount of at least one (e.g., one) claimed compound, Leuprolide and Letrozole.

Further disclosed is a method of treating or preventing breast cancer in a patient in need of such treatment wherein said treatment comprises administering a therapeutically effective amount of at least one (e.g., one) claimed compound, Leuprolide and Exemestane.

Also disclosed is a method of treating or preventing breast cancer in a patient in need of such treatment wherein said treatment comprises administering a therapeutically effective amount of at least one (e.g., one) claimed compound, Leuprolide and Fadrozole.

Further disclosed is a method of treating or preventing breast cancer in a patient in need of such treatment wherein said treatment comprises administering a therapeutically effective amount of at least one (e.g., one) claimed compound, Leuprolide and Formestane.

Also disclosed is the treatment or prevention of breast cancer in a patient in need of such treatment, said treatment comprising the administration of a therapeutically effective amount of at least one (e.g., one) claimed compound and Anastrozole.

Further disclosed is the treatment or prevention of breast cancer in a patient in need of such treatment, said treatment comprising the administration of a therapeutically effective amount of at least one (e.g., one) claimed compound and Letrozole.

Also disclosed is the treatment or prevention of breast cancer in a patient in need of such treatment, said treatment comprising the administration of a therapeutically effective amount of at least one (e.g., one) claimed compound and Exemestane.

Further disclosed is the treatment or prevention of breast cancer in a patient in need of such treatment, said treatment comprising the administration of a therapeutically effective amount of at least one (e.g., one) claimed compound and Tamoxifen.

Also disclosed is the treatment or prevention of breast cancer in a patient in need of such treatment, said treatment comprising the administration of a therapeutically effective amount of at least one (e.g., one) claimed compound and Fulvestrant.

Further disclosed is the treatment or prevention of breast cancer in a patient in need of such treatment, said treatment comprising the administration of a therapeutically effective amount of at least one (e.g., one) claimed compound, Anastrozole, and Fulvestrant.

Also disclosed is the treatment or prevention of breast cancer in a patient in need of such treatment, said treatment comprising the administration of a therapeutically effective amount of at least one claimed compound (e.g., one), Letrozole, and Fulvestrant.

Further disclosed is the treatment or prevention of breast cancer in a patient in need of such treatment, said treatment comprising the administration of a therapeutically effective amount of at least one (e.g., one) claimed compound, Exemestane, and Fulvestrant.

Also disclosed is the treatment or prevention of breast cancer in a patient in need of such treatment, said treatment comprising the administration of a therapeutically effective amount of at least one (e.g., one) claimed compound, Anastrozole, and Tamoxifen.

Further disclosed is the treatment or prevention of breast cancer in a patient in need of such treatment, said treatment comprising the administration of a therapeutically effective amount of at least one (e.g., one) claimed compound, Letrozole, and Tamoxifen.

Also disclosed is the treatment or prevention of breast cancer in a patient in need of such treatment, said treatment comprising the administration of a therapeutically effective amount of at least one (e.g., one) claimed, Exemestane, and Tamoxifen.

Other embodiments are directed to any of the above described embodiments for the treatment of Breast Cancer wherein the chemotherapeutic agent is Trastuzumab.

Other embodiments are directed to any of the above described embodiments for the treatment or prevention of Breast Cancer wherein the method is directed to the treatment of breast cancer.

The claimed compound, antihormonal agents and chemotherapeutic agents can be administered concurrently or sequentially.

The antihormonal agents and optional chemotherapeutic agents are administered according to their protocols, dosage amounts, and dosage forms that are well know to those skilled in the art (e.g., the Physician's Desk Reference or published literature). For example, for Tamoxifen, Fulvestrant, Raloxifene, Anastrozole, Letrozole, Exemestane, Leuprolide and Goserelin, see the Physician's Desk Reference, 57th Edition, 2003, published by Thomas PDR at Montvale, N.J. 07645-1742.

In general, in the embodiments directed to the methods of treating Breast Cancer: (1) the claimed compound can be administered daily (e.g., once per day, and in one embodiment twice a day), (2) the aromatase inhibitors can be administered in accordance with the known protocol for the aromatase inhibitor used (e.g., once per day), (3) the antiestrogens can be administered in accordance with the known protocol for the antiestrogen used (e.g., from once a day to once a month), (4) the LHRH analogue can be administered in accordance with the known protocol for the LHRH analogue used (e.g., once a month to once every three months), and (5) the chemotherapeutic agent can be administered in accordance with the known protocol for the chemotherapeutic agent used (e.g., from once a day to once a week).

Radiation therapy, if administered in the above treatments for breast cancer, is generally administered according to known protocols before administration of the claimed compound, antihormonal agents and optional chemotherapeutic agents.

Treatment according to the methods of treating breast cancer is continuous (i.e., a continuous dosing schedule is followed). The treatment is continued until there is a complete response, or until the skilled clinician determines that the patient is not benefiting from the treatment (for example, when there is disease progression).

The continuous treatment protocol for breast cancer can be changed to a discontinuous treatment schedule if, in the judgment of the skilled clinician, the patient would benefit from a discontinuous treatment schedule with one or more of the administered drugs. For example, the claimed compound can be given using a discontinous treatment schedule while the remaining drugs used in the treatment are given as described herein. An example of a discontinuous treatment protocol for the claimed compound is a repeating cycle of three weeks with the claimed compound followed by one week without the claimed compound.

After a complete response is achieved with the breast cancer treatment, maintenance therapy with the claimed compound can be continued using the dosing described in the methods of this invention. Maintenance therapy can also include administration of the antihormonal agents using the dosing described in the methods of this invention. Maintenance therapy can just be with the antihormonal agents. For example, after a complete response is achieved, an aromatase inhibitor (e.g., Anastrozole, Letrozole or Exemestane) can be continued for up to five years. Or, for example, an antiestrogen, e.g., Tamoxifen, may be used for up to five years after a complete response is achieved. Or, for example, an antiestrogen (e.g., Tamoxifen) can be used for up to five years after a complete response is achieved followed by the use of an aromatase inhibitor (e.g., Anastrozole, Letrozole or Exemestane) for up to five years.

In the embodiments directed to the treatment of breast cancer described above, the claimed compound is administered continuously in a total daily dose of about 100 mg to about 600 mg. Usually this amount is administered in divided doses, and in one embodiment this amount is administered twice a day. In one embodiment the claimed compound is dosed twice a day in an amount of about 50 mg to about 300 mg per dose. In another embodiment the claimed compound is dosed twice a day in an amount of about 100 mg to about 200 mg per dose. Examples include the claimed compound being dosed twice a day at 100 mg per dose. Examples also include the claimed compound being dosed twice a day at 200 mg per dose.

Anastrozole is administered p.o. and is dosed once a day in amounts of about 0.5 to about 10 mg per dose, and in one embodiment in an amount of about 1.0 mg per dose.

Letrozole is administered p.o. and is dosed once a day in amounts of about 1.0 to about 10 mg per dose, and in one embodiment in an amount of about 2.5 mg per dose.

Exemestane is administered p.o. and is dosed once a day in amounts of about 10 to about 50 mg per dose, and in one embodiment in an amount of about 25 mg per dose.

Fadrozole is administered p.o. and is dosed twice a day in amounts of about 0.5 to about 10 mg per dose, and in one embodiment in an amount of about 2.0 mg per dose.

Formestane is administered i.m. and is dosed once every two weeks in amounts of about 100 to about 500 mg per dose, and in one embodiment in an amount of about 250 mg per dose.

Tamoxifen is administered p.o. and is dosed once a day in amounts of about 10 to about 100 mg per dose, and in one embodiment in an amount of about 20 mg per dose.

Fulvestrant is administered i.m. and is dosed once a month in amounts of about 100 to about 1000 mg per dose, and in one embodiment in an amount of about 250 mg per dose.

Raloxifene is administered p.o. and is dosed once a day in amounts of about 10 to about 120 mg per dose, and in one embodiment in an amount of about 60 mg per dose.

Acolbifene is administered p.o. and is dosed once a day in amounts of about 5 to about 20 mg per dose, and in one embodiment in an amount of about 20 mg per dose.

Goserelin is administered s.c. and is dosed once a month, or once every three months, in amounts of about 2 to about 20 mg per dose, and in one embodiment in an amount of about 3.6 mg per dose when administered once a month, and in another embodiment in an amount of about 10.8 mg per dose when administered once every three months.

Leuprolide is administered s.c. and is dosed once a month, or once every three months, in amounts of about 2 to about 20 mg per dose, and in one embodiment in an amount of about 3.75 mg per dose when administered once a month, and in another embodiment in an amount of about 11.25 mg per dose when administered once every three months.

Trastuzumab is administered by i.v. and is dosed once a week in amounts of about 2 to about 20 mpk per dose, and in one embodiment in an amount of about 2 mpk per dose. Trastuzumab is generally initially administered in a loading dose that is generally twice the dose of the weekly dose. Thus, for example, a 4 mpk loading dose is administered and then dosing is 2 mpk per dose per week.

Gefitinib is administered p.o. and is dosed once a day in amounts of about 100 to about 1000 mg per dose, and in one embodiment in an amount of about 250 mg per dose.

Erlotinib is administered p.o. and is dosed once a day in amounts of about 100 to about 500 mg per dose, and in one embodiment in an amount of about 150 mg per dose.

Bevacizumab is administered i.v. and is dosed once every two weeks in amounts of about 2.5 to about 15 mg per kilogram of body weight per dose, and in one embodiment in an amount of about 10 mg per kilogram per dose.

Cetuximab is administered i.v. and is dosed once a week in amounts of about 200 to about 500 mg per meter squared dose, and in one embodiment in an amount of about 250 mg per meter squared per dose.

Bortezomib is administered i.v. and is dosed twice a week for 2 weeks followed by a 10 day rest period (21 day treatment cycle) for a maximum of 8 treatment cycles in amounts of about 1.0 to about 2.5 mg per meter squared per dose, and in one embodiment in an amount of about 1.3 mg per meter squared per dose.

Thus breast cancer can be treated (or prevented) in a patient in need of such treatment wherein said treatment comprises administering to said patient: (1) the claimed compound orally in an amount of about 50 mg to about 300 mg per dose wherein each dose is administered twice a day, and (2) Anastrozole p.o. in an amount of about 0.5 to about 10 mg per dose wherein each dose is given once a day.

Breast cancer can be treated (or prevented) in a patient in need of such treatment wherein said treatment comprises administering to said patient: (1) the claimed compound orally in an amount of about 100 to 200 mg per dose, wherein each dose is administered twice a day, and (2) Anastrozole in an amount of about 1.0 mg per dose wherein each dose is given once a day.

Breast cancer can be treated (or prevented) in a patient in need of such treatment wherein said treatment comprises administering to said patient: (1) the claimed compound orally in an amount of about 50 mg to about 300 mg per dose wherein each dose is administered twice a day, and (2) Letrozole p.o. in an amount of about 1.0 to about 10 mg per dose wherein each dose is given once a day.

Breast cancer can be treated (or prevented) in a patient in need of such treatment wherein said treatment comprises administering to said patient: (1) the claimed compound orally in an amount of about 100 to 200 mg per dose, wherein each dose is administered twice a day, and (2) Letrozole p.o. in an amount of about 2.5 mg per dose wherein each dose is given once a day.

Breast cancer can be treated (or prevented) in a patient in need of such treatment wherein said treatment comprises administering to said patient: (1) the claimed compound orally in an amount of about 50 mg to about 300 mg per dose wherein each dose is administered twice a day, and (2) Exemestane p.o. in an amount of about 10 to about 50 mg per dose wherein each dose is given once a day.

Breast cancer can be treated (or prevented) in a patient in need of such treatment wherein said treatment comprises administering to said patient: (1) the claimed compound orally in an amount of about 100 to 200 mg per dose, wherein each dose is administered twice a day, and (2) Exemestane in an amount of about 25 mg per dose wherein each dose is given once a day.

Breast cancer can be treated (or prevented) in a patient in need of such treatment wherein said treatment comprises administering to said patient: (1) the claimed compound orally in an amount of about 50 mg to about 300 mg per dose wherein each dose is administered twice a day, and (2) Fulvestrant i.m. in an amount of about 100 to about 1000 mg per dose wherein each dose is given once a month.

Breast cancer can be treated (or prevented) in a patient in need of such treatment wherein said treatment comprises administering to said patient: (1) the claimed compound orally in an amount of about 100 to 200 mg per dose, wherein each dose is administered twice a day, and (2) Fulvestrant i.m. in an amount of about 250 mg per dose wherein each dose is given once a month.

Breast cancer can be treated (or prevented) in a patient in need of such treatment wherein said treatment comprises administering to said patient: (1) the claimed compound p.o. in an amount of about 50 mg to about 300 mg per dose wherein each dose is administered twice a day, and (2) Tamoxifen p.o. in an amount of about 10 to about 100 mg per dose wherein each dose is given once a day.

Breast cancer can be treated (or prevented) in a patient in need of such treatment wherein said treatment comprises administering to said patient: (1) the claimed compound p.o. in an amount of about 100 to 200 mg per dose, wherein each dose is administered twice a day, and (2) Tamoxifen p.o. in an amount of about 20 mg per dose wherein each dose is given once a day.

Breast cancer can be treated in a patient in need of such treatment wherein said treatment comprises the administration of the claimed compound, one of the aromatase inhibitors (e.g., Anastrozole, Letrozole, or Exemestane, and in one embodiment Anastrozole), and one of the antiestrogens (e.g., Fulvestrant or Tamoxifen), wherein the claimed compound, aromatase inhibitor and antiestrogen are administered in the dosages described above.

Breast cancer can be treated (or prevented) in a patient in need of such treatment wherein said treatment comprises administering to said patient: (1) the claimed compound p.o. in an amount of about 50 mg to about 300 mg per dose wherein each dose is administered twice a day, (2) Anastrozole p.o. in an amount of about 0.5 to about 10 mg per dose wherein each dose is given once a day, and (3) Fulvestrant i.m. in an amount of about 100 to about 1000 mg per dose wherein each dose is given once a month.

Breast cancer can be treated (or prevented) in a patient in need of such treatment wherein said treatment comprises administering to said patient: (1) the claimed compound p.o in an amount of about 100 to 200 mg per dose, wherein each dose is administered twice a day, (2) Anastrozole p.o. in an amount of about 1.0 mg per dose wherein each dose is given once a day, and (3) Fulvestrant i.m. in an amount of about 250 mg per dose wherein each dose is given once a month.

Breast cancer can be treated (or prevented) in a patient in need of such treatment wherein said treatment comprises administering to said patient: (1) the claimed compound p.o. in an amount of about 50 mg to about 300 mg per dose wherein each dose is administered twice a day, (2) Letrozole p.o in an amount of about 1.0 to about 10 mg per dose wherein each dose is given once a day, and (3) Fulvestrant in an amount of about 100 to about 1000 mg per dose wherein each dose is given once a month.

Breast cancer can be treated (or prevented) in a patient in need of such treatment wherein said treatment comprises administering to said patient: (1) the claimed compound p.o. in an amount of about 100 to 200 mg per dose, wherein each dose is administered twice a day, (2) Letrozole p.o. in an amount of about 2.5 mg per dose wherein each dose is given once a day, and (3) Fulvestrant i.m. in an amount of about 250 mg per dose wherein each dose is given once a month.

Breast cancer can be treated (or prevented) in a patient in need of such treatment wherein said treatment comprises administering to said patient: (1) the claimed compound p.o. in an amount of about 50 mg to about 300 mg per dose wherein each dose is administered twice a day, (2) Exemestane p.o. in an amount of about 10 to about 50 mg per dose wherein each dose is given once a day, and (3) Fulvestrant i.m. in an amount of about 100 to about 1000 mg per dose wherein each dose is given once a month.

Breast cancer can be treated (or prevented) in a patient in need of such treatment wherein said treatment comprises administering to said patient: (1) the claimed compound p.o. in an amount of about 100 to 200 mg per dose, wherein each dose is administered twice a day, (2) Exemestane p.o. in an amount of about 25 mg per dose wherein each dose is given once a day, and (3) Fulvestrant i.m. in an amount of about 250 mg per dose wherein each dose is given once a month.

Breast cancer can be treated (or prevented) in a patient in need of such treatment wherein said treatment comprises administering to said patient: (1) the claimed compound p.o. in an amount of about 50 mg to about 300 mg per dose wherein each dose is administered twice a day, (2) Anastrozole p.o. in an amount of about 0.5 to about 10 mg per dose wherein each dose is given once a day, and (3) Tamoxifen p.o.in an amount of about 10 to about 100 mg per dose wherein each dose is given once a day.

Breast cancer can be treated (or prevented) in a patient in need of such treatment wherein said treatment comprises administering to said patient: (1) the claimed compound p.o. in an amount of about 100 to 200 mg per dose, wherein each dose is administered twice a day, (2) Anastrozole p.o. in an amount of about 1.0 mg per dose wherein each dose is given once a day, and (3) Tamoxifen p.o. in an amount of about 20 mg per dose wherein each dose is given once a day.

Breast cancer can be treated (or prevented) in a patient in need of such treatment wherein said treatment comprises administering to said patient: (1) the claimed compound p.o. in an amount of about 50 mg to about 300 mg per dose wherein each dose is administered twice a day, (2) Letrozole p.o. in an amount of about 1.0 to about 10 mg per dose wherein each dose is given once a day, and (3) Tamoxifen p.o. in an amount of about 10 to about 100 mg per dose wherein each dose is given once a day.

Breast cancer can be treated (or prevented) in a patient in need of such treatment wherein said treatment comprises administering to said patient: (1) the claimed compound p.o. in an amount of about 100 to 200 mg per dose, wherein each dose is administered twice a day, (2) Letrozole p.o. in an amount of about 2.5 mg per dose wherein each dose is given once a day, and (3) Tamoxifen p.o. in an amount of about 20 mg per dose wherein each dose is given once a day.

Breast cancer can be treated (or prevented) in a patient in need of such treatment wherein said treatment comprises administering to said patient: (1) the claimed compound p.o. in an amount of about 50 mg to about 300 mg per dose wherein each dose is administered twice a day, (2) Exemestane p.o. in an amount of about 10 to about 50 mg per dose wherein each dose is given once a day, and (3) Tamoxifen p.o. in an amount of about 10 to about 100 mg per dose wherein each dose is given once a day.

Breast cancer can be treated (or prevented) in a patient in need of such treatment wherein said treatment comprises administering to said patient: (1) the claimed compound p.o. in an amount of about 100 to 200 mg per dose, wherein each dose is administered twice a day, (2) Exemestane p.o. in an amount of about 25 mg per dose wherein each dose is given once a day, and (3) Tamoxifen p.o. in an amount of about 20 mg per dose wherein each dose is given once a day.

Those skilled in the art will appreciate that when other combinations of antihormonal agents are used, the individual antihormonal agent is used in the amounts specified above for that individual antihormonal agent.

Other embodiments of the treatment of Breast Cancer are directed to the methods of treating Breast Cancer described above wherein the claimed compound is dosed twice a day in an amount of about 100 mg per dose.

Other embodiments of the treatment of Breast Cancer are directed to the methods of treating Breast Cancer described above wherein the claimed compound is dosed twice a day in an amount of about 200 mg per dose.

Other embodiments of the treatment of Breast Cancer are directed to the methods of treating Breast Cancer described above wherein a chemotherapeutic agent is administered in addition to the claimed compound and antihormonal agent (or antihormonal agents). In these embodiments the dosage ranges of the claimed compound and antihormonal agents are as those described above in the combination therapies, or those described above for the individual compound of the invention and antihormonal agents, and the dosages of the chemotherapeutic agents are those described above for the individual chemotherapeutic agent. The dosages for the chemotherapeutic agents are well known in the art.

Other embodiments of this invention are directed to pharmaceutical compositions comprising the claimed compound and at least one antihormonal agent and a pharmaceutically acceptable carrier.

Other embodiments of this invention are directed to pharmaceutical compositions comprising the claimed compound, at least one antihormonal agent, at least one chemotherapeutic agent, and a pharmaceutically acceptable carrier.

Other embodiments of this invention are directed to pharmaceutical compositions comprising the claimed compound, at least one chemotherapeutic agent, and a pharmaceutically acceptable carrier.

Those skilled in the art will appreciate that the compounds (drugs) used in the methods of this invention are available to the skilled clinician in pharmaceutical compositions (dosage forms) from the manufacturer and are used in those compositions. So, the recitation of the compound or class of compounds in the above described methods can be replaced with a recitation of a pharmaceutical composition comprising the particular compound or class of compounds. For example, the embodiment directed to a method of treating cancer comprising administering to a patient in need of such treatment therapeutically effective amounts of the claimed compound, a taxane, and a platinum coordination compound, includes within its scope a method of treating cancer comprising administering to a patient in need of such treatment therapeutically effective amounts of a pharmaceutical composition comprising the claimed compound, a pharmaceutical composition comprising a taxane, and a pharmaceutical composition comprising a platinum coordination compound.

Those skilled in the art will recognize that the actual dosages and protocols for administration employed in the methods of this invention may be varied according to the judgment of the skilled clinician. The actual dosage employed may be varied depending upon the requirements of the patient and the severity of the condition being treated. Determination of the proper dosage for a particular situation is within the skill of the art. A determination to vary the dosages and protocols for administration may be made after the skilled clinician takes into account such factors as the patient's age, condition and size, as well as the severity of the cancer being treated and the response of the patient to the treatment.

The amount and frequency of administration of the claimed compound and the chemotherapeutic agents will be regulated according to the judgment of the attending clinician (physician) considering such factors as age, condition and size of the patient as well as severity of the cancer being treated.

The chemotherapeutic agent can be administered according to therapeutic protocols well known in the art. It will be apparent to those skilled in the art that the administration of the chemotherapeutic agent can be varied depending on the cancer being treated and the known effects of the chemotherapeutic agent on that disease. Also, in accordance with the knowledge of the skilled clinician, the therapeutic protocols (e.g., dosage amounts and times of administration) can be varied in view of the observed effects of the administered therapeutic agents on the patient, and in view of the observed responses of the cancer to the administered therapeutic agents.

The initial administration can be made according to established protocols known in the art, and then, based upon the observed effects, the dosage, modes of administration and times of administration can be modified by the skilled clinician.

The particular choice of chemotherapeutic agent will depend upon the diagnosis of the attending physicians and their judgement of the condition of the patient and the appropriate treatment protocol.

The determination of the order of administration, and the number of repetitions of administration of the chemotherapeutic agent during a treatment protocol, is well within the knowledge of the skilled physician after evaluation of the cancer being treated and the condition of the patient.

Thus, in accordance with experience and knowledge, the practicing physician can modify each protocol for the administration of an chemotherapeutic agent according to the individual patient's needs, as the treatment proceeds. All such modifications are within the scope of the present invention.

The particular choice of antihormonal agents, optional chemotherapeutic agents and optional radiation will depend upon the diagnosis of the attending physicians and their judgment of the condition of the patient and the appropriate treatment protocol.

The determination of the order of administration, and the number of repetitions of administration of the antihormonal agents, optional chemotherapeutic agents and optional radiation during a treatment protocol, is well within the knowledge of the skilled physician after evaluation of the breast cancer being treated and the condition of the patient.

Thus, in accordance with experience and knowledge, the practicing physician can modify each protocol for the administration of antihormonal agents, optional chemotherapeutic agents and optional radiation according to the individual patient's needs, as the treatment proceeds. All such modifications are within the scope of the present invention.

The attending clinician, in judging whether treatment is effective at the dosage administered, will consider the general well-being of the patient as well as more definite signs such as relief of cancer-related symptoms (e.g., pain, cough (for lung cancer), and shortness of breath (for lung cancer)), inhibition of tumor growth, actual - shrinkage of the tumor, or inhibition of metastasis. Size of the tumor can be measured by standard methods such as radiological studies, e.g., CAT or MRI scan, and successive measurements can be used to judge whether or not growth of the tumor has been retarded or even reversed. Relief of disease-related symptoms such as pain, and improvement in overall condition can also be used to help judge effectiveness of treatment.

The compounds of this invention can be made according to the processes described in WO2007/070398 published on June 21, 2007, and the compounds of the invention can be made according to the processes described below.

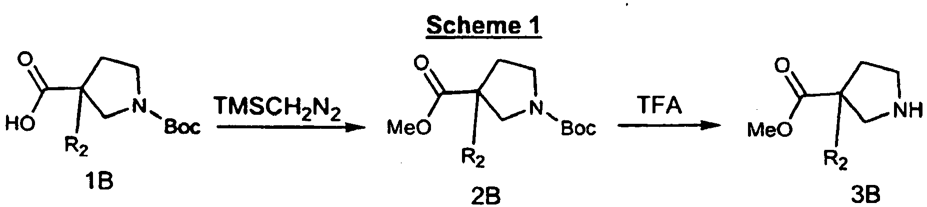

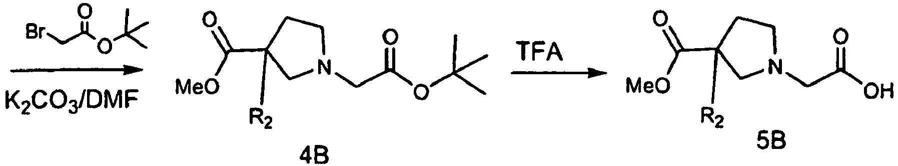

The appropriately substituted pyrrolidine is prepared as follows. The methyl ester of pyrrolidine 1 B is prepared by reaction with TMSdiazomethane. After removing the BOC protecting group, compound 3B is reacted with bromo-tert.butylacetate to obtain 4B. The tert.butyl ester is then removed to obtain intermediate 5B.

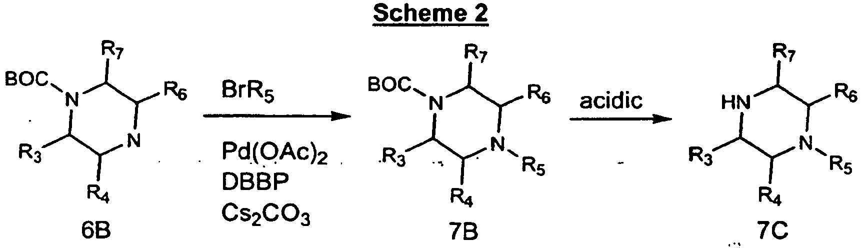

The R⁵ substituted piperazine is prepared by Buchwald type coupling of the piperazine 6B with an aryl bromide in the presence of palladium to obtain the piperazine 7B. The BOC group is removed using acidic conditions (e.g., TFA) to give piperazine 7C.

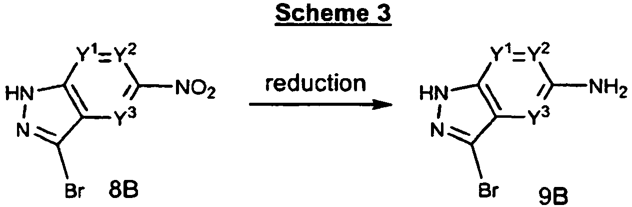

The indazole bromide 9B is prepared by reduction of the indazole nitro compound 8B using reducing conditions such as palladium on carbon in the presence of hydrogen atmosphere.

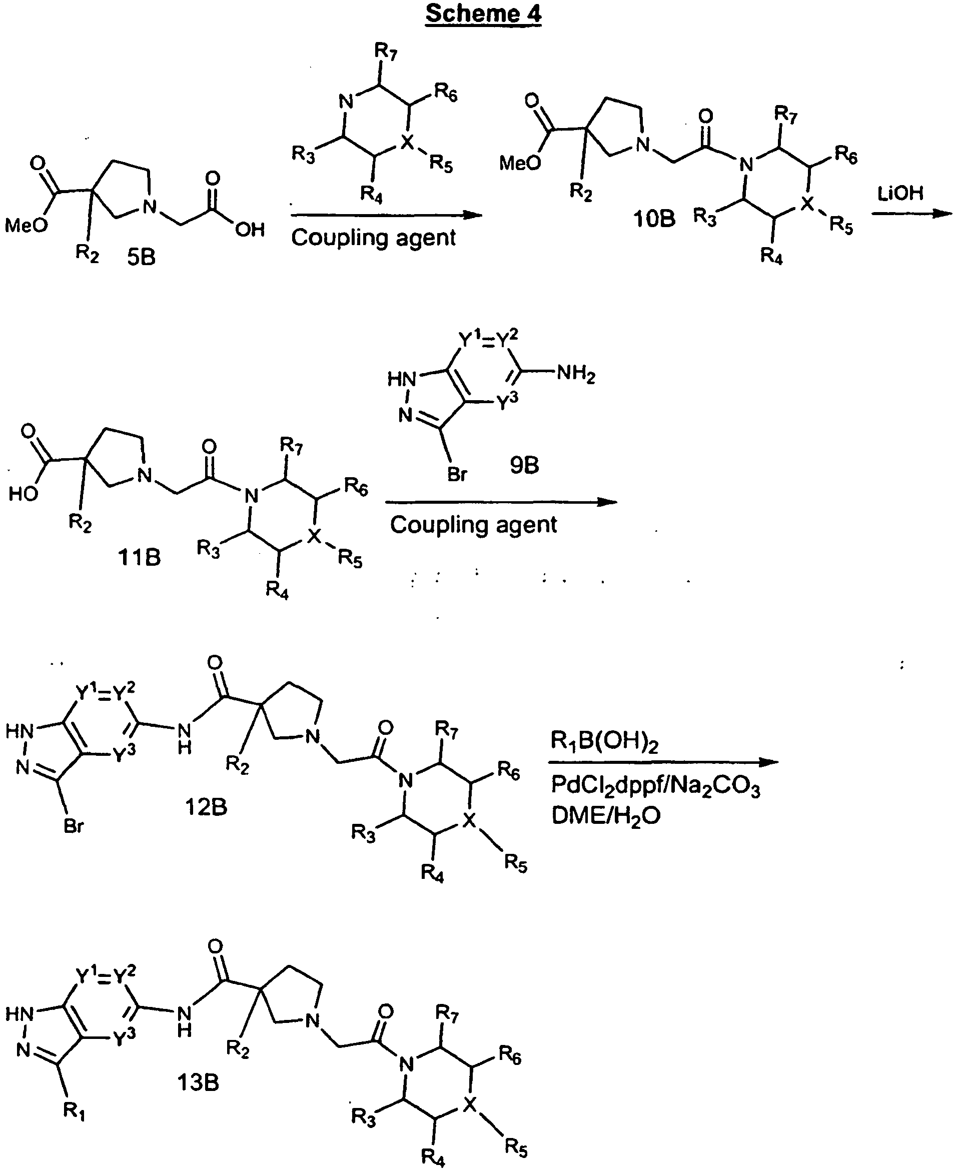

The final molecule is assembled by coupling the pyrrolidine 5B with the piperazine (X is N, see compound 7C from Scheme 2), or with the piperidine (X is C, prepared in Schemes 11 or 12, or prepared according to the Examples described herein, or prepared according to methods well known to those skilled in the art), using standard coupling conditions such as HATU in DMF to obtain 10B. After hydrolysis of the methyl ester to obtain 11 B, the indazole intermediate 9B is then coupled using standard coupling conditions such as HATU in DMF to obtain 12B. The indazole is then derivatized at the 3 position by suzuki coupling with an a boronic acid to obtain the final product 13B.

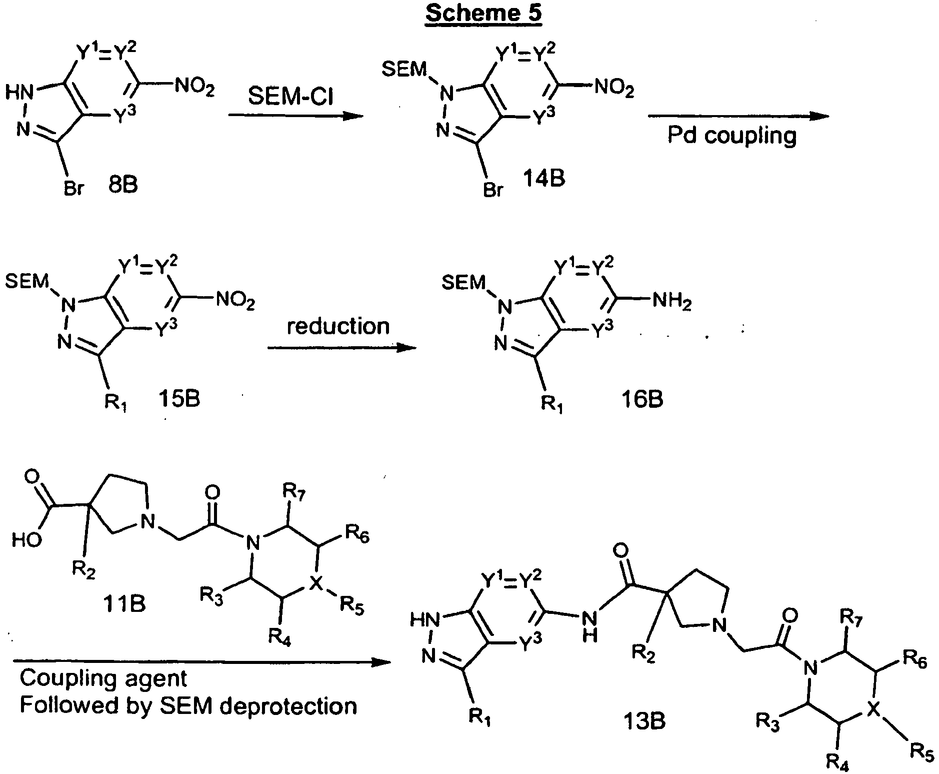

Alternatively, the final product 13B can be prepared by protecting the indazole 8B as the SEM (or trityl) derivative followed by Suzuki coupling to give the appropriately substituted indazole intermediate 15B. After reduction using reducing conditions such as palladium on carbon in the presence of hydrogen atmosphere to obtain 16B, the amine 16B can be coupled to 11 B using standard coupling conditions such as HATU in DMF to obtain 13B.

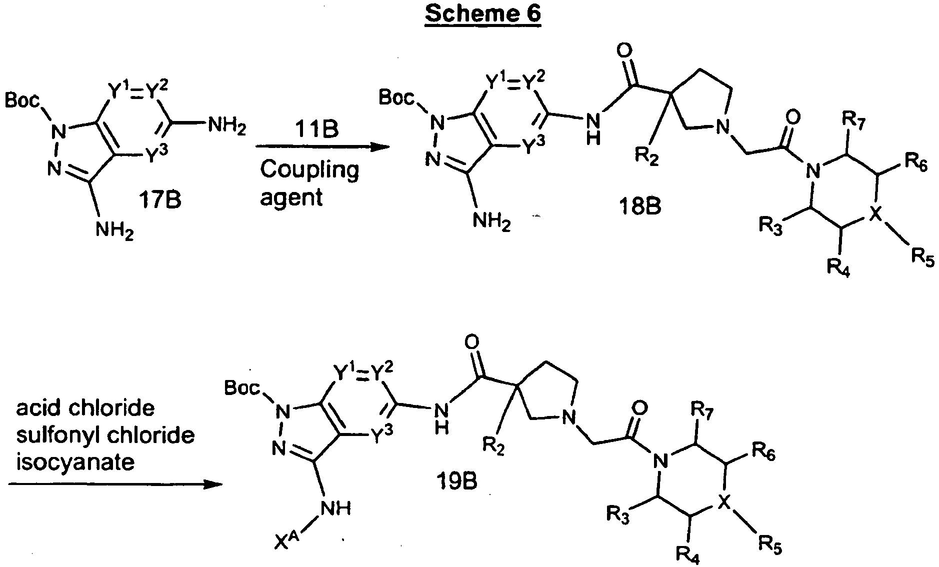

3-Amino substituted indazole derivatives can be prepared according to general Scheme 6 by coupling the Boc protected 3,5-diamino indazole 17B with 11 B under standard coupling conditions such as HATU in DMF to obtain 18B. The 3-amino group can then be reacted with acid chlorides, sulfonyl chlorides and isocyanates to prepare the corresponding amides, sulfonamides and ureas 19B respectively.

Those skilled in the art will appreciate that the moiety -NHX^{A} represents the amino substituted R¹ groups, such as, -N(R¹⁰)₂, -NR³²-C(O)-R¹⁴, -N(R¹⁰)C(O)N(R¹⁰)₂, and -N(R¹⁰)S(O)ₜR¹⁰.

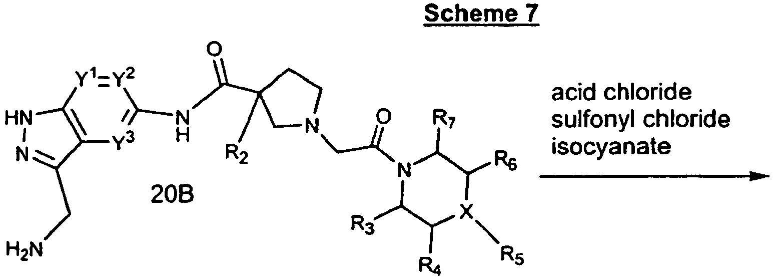

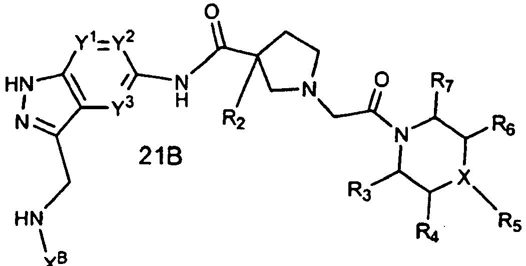

The amino methyl indazole derivative 20B can be prepared according to Example 76 step 1 through 4 by substituting the appropriately substituted rings. The 3-aminomethyl group can then be reacted with acid chlorides, sulfonyl chlorides and isocyanates to prepare the corresponding amides, sulfonamides and ureas 21 B respectively.

Those skilled in the art will appreciate that the moiety -CH₂NHX^{B} represents the amino substituted methyl R¹ groups, such as, -(C(R³⁰)₂)ₙ-NR³²-C(O)-R¹⁰ (wherein n is 1 and each R³⁰ is H), -(C(R³⁰)₂)ₙ-NR³²-S(O)ₜR¹⁰ (wherein n is 1 and each R³⁰ is H), -(C(R³⁰)₂)ₙ-NR³²-C(O)-N(R³²)-R¹⁰ (wherein n is 1 and each R³⁰ is H), -(C(R³⁰)₂)ₙR¹³ (wherein n is 1, each R³⁰ is H and, R¹³ is -N(R¹⁰)₂),

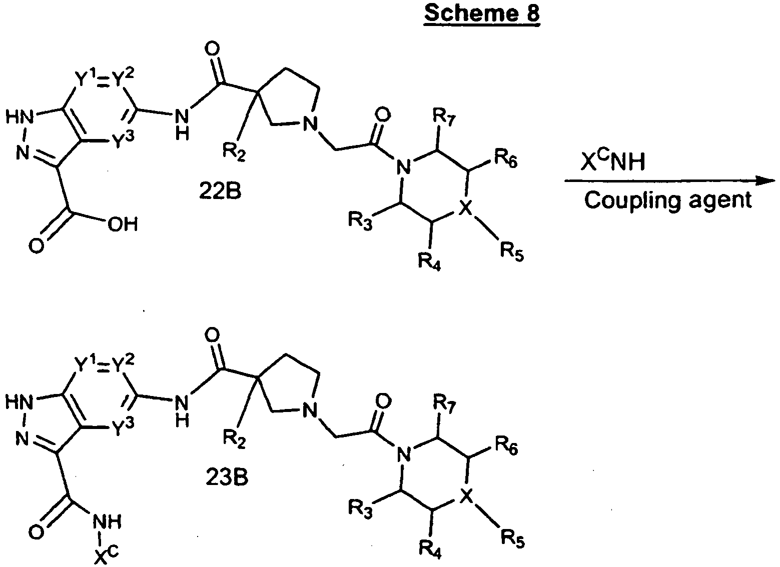

The amide compound 23B can be prepared by following Example 87 substituting the appropriate compound 11 B (General Scheme 4). The resulting acid 22B is then coupled with the appropriate amine under standard coupling conditions such as in the presence of EDC/HOBt to obtain 23B.

Those skilled in the art will appreciate that the moiety -C(O)NHX^{c} represents the amide R¹ groups, such as, -C(O)N(R¹⁰)₂ and -C(O)-NR³²-C(R¹⁸)₃

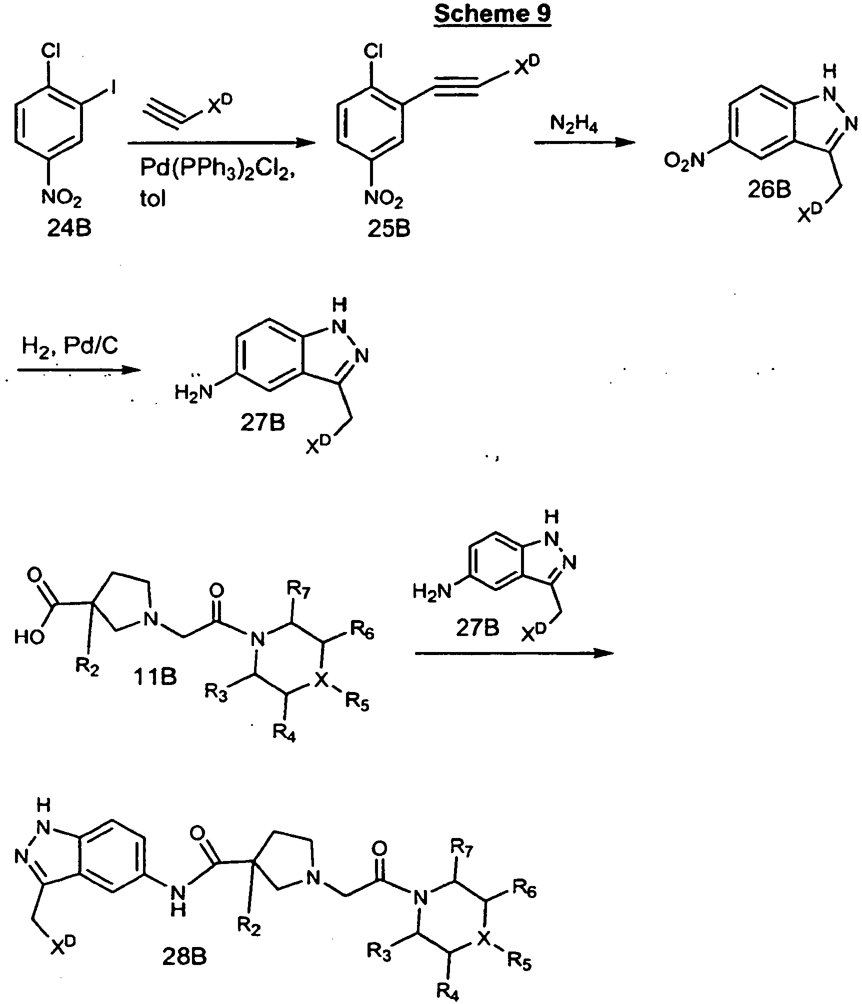

Indazoles of type 27B can be prepared by reacting 24B with the appropriately substituted acetylene in the presence of Pd(PPh₃)₂Cl₂ in toluene to obtain 25B. After heating with hydrazine to obtain 26B, 27B is obtained after hydrogenation. Coupling of 27B with 11 B under standard coupling conditions such as HATU or EDC/HOBt gives 28B.

The moiety -CH₂X^{D} represents R¹ groups that are an R¹⁰ substituent, such as, alkyl, heteroarylalkyl, and arylalkyl.

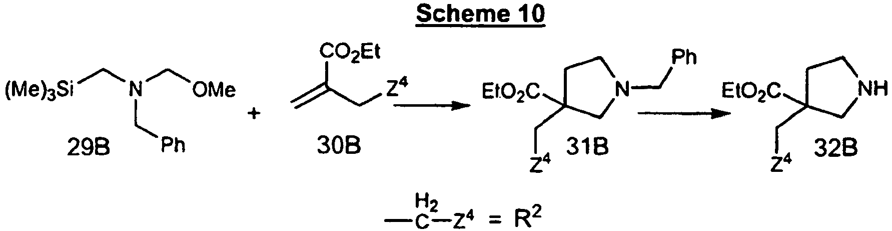

The appropriately substituted pyrrolidine 32B can be obtained by reacting 29B with the appropriately substituted compound 30B in the presence of trifluoroacetic acid to obtain 31 B. Compound 31 B can then be deprotection under hydrogenation conditions (Pd/C, H₂ to obtain 32B).

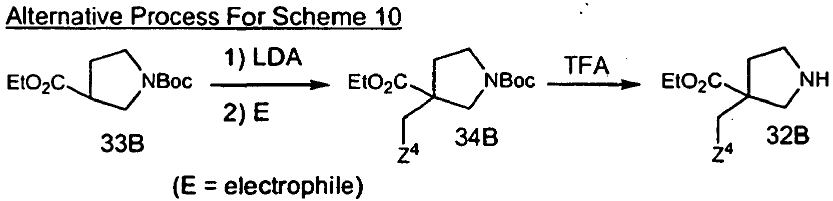

Alternatively, 32B can be obtain by reaction 33B with LDA followed by the addition of a suitable electrophile such as allylbromide, as in example 127, to obtain 34B. Treatment of 34B with trifluoroacetic acid yields 32B.

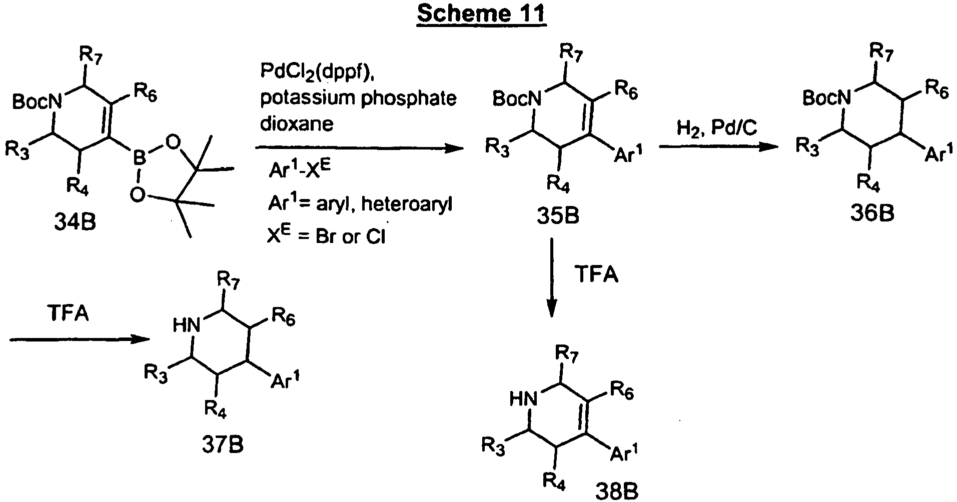

Aryl or heteroaryl substituted piperidines can be prepared by Suzuki coupling of an aryl or heteroaryl halide with the pinicolboronate 34B to obtain 35B. The ring double bond can then be hydrogenated to obtain 36B followed by removal of the Boc protecting group under trifluoroacetic acid conditions. Alternatively the double bond can be retained and the Boc group removed to give 38B.

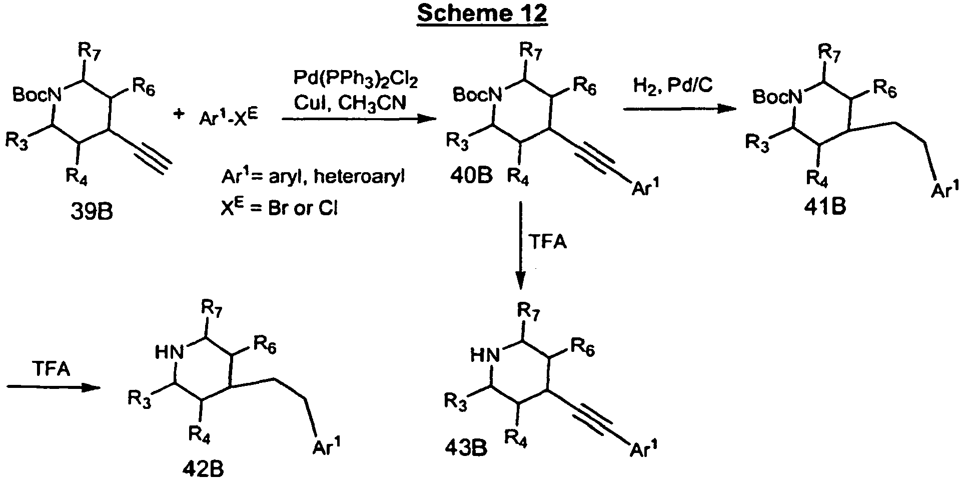

Similarly aryl or heteroaryl substituted piperizines with a 2 carbon spacer can be prepared as shown in Scheme 12 by coupling an aryl or heteroaryl halide with an acetylene derivative 39B that can be prepared according to procedures known in the art to obtain 40B. 40B can then be reduced to 41 B followed by by removal of the Boc protecting group under trifluoroacetic acid conditions. Alternatively the Boc protecting group from 40B can be removed under trifluoroacetic acid conditions to give 43B.

Compounds of this invention are exemplified in the following examples, which should not be construed as limiting the scope of the disclosure. Alternative mechanistic pathways and analogous structures within the scope of the invention may be apparent to those skilled in the art.

The LCMS conditions are: (1) column: C-18 reverse phase, 5um, 4.6 x 50 mm, (2) MS:PE Sciex API-150EX, and (3) HPLC: Shimadzu LC-10 ADvp, 1 ml/min, linerar gradient 10% acetonitirle in water to 95% acetonitrile in water, both contain 0.05% TFA

Examples 1 to 611 are disclosed in WO2007/070398 (published on June 21, 2007) on page 188 at about line 15 to page 453 at about line 10, which pages are copied herein in their entirety and should be considered as part of the present disclosure, even though the said published pages are not reproduced here for the sake of brevity.

### EXAMPLE 612

### Step 1

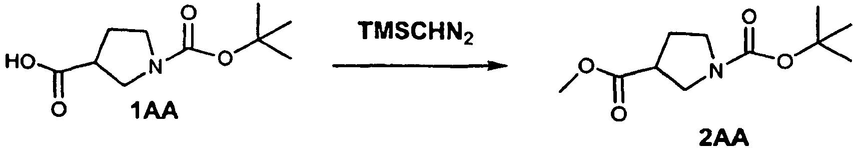

Pyrrolidine-1,3-dicarboxylic acid 1-tert-butyl ester **1AA**(4.3 gm, 20 mmol) was dissolved in 28 mL of toluene and 3.5 ml of methanol. Trimethylsilyldiazomethane 2N solution in hexanes(13 ml, 26 mmol) was added dropwise at 0 C and the reaction mixture stirred for 10 min at ambient temperature. The mixture was evaporated to obtain 4.3 gm of oil.

### Step 2

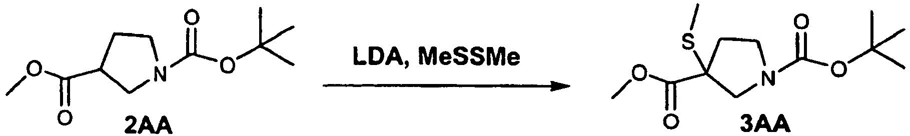

To the oil 2 (0.5 gm, 2.1 mmol) dissolved in tetrahydrofuran (15 ml) 1.2 ml of lithium diisopropylamide 2N solution in hexanes was added dropwise and the reaction mixture stirred for 1 hr at -78 C. Dimethyldisulfide (0.48 mL, 5.4 mmol) was added slowly and let warm to ambient temperature gradually. The reaction mixture was stirred for 18 hrs. A saturated solution of Ammonium chloride (25 ml) was added and the reaction mixture stirred for 5 min. The reaction mixture was extracted with ethyl acetate three times (3x25 ml), dried over magnesium sulfate, filtered and evaporated to give 0.386g of title product **3AA** after column chromatography.

### Step 3

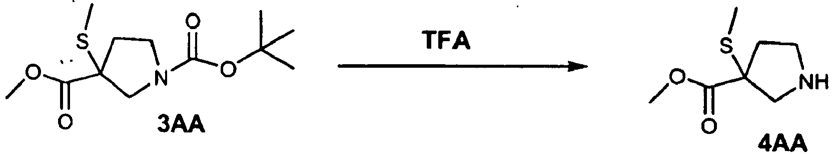

3-Methylsulfanyl-pyrrolidine-1,3-dicarboxylic acid 1-tert-butyl ester 3-methyl ester **3AA** (2.15 gm, 8.8 mmol) was dissolved in 20 ml of 50% trifluoroacetic acid/dichloromethane and stirred for 2 hrs. The reaction mixture was evaporated to an oil and exchanged with hydrochloric acid by dissolving in 20 ml of dichloromethane and adding 10 ml of 1 N HCl in ether to obtain 3.35 gm **4AA** gummy solid.

### Step 4

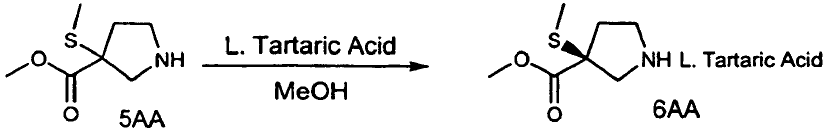

Compound A (42.9g, 244.8 mmol) and 1 eq. of L-tartaric acid were placed in a 1 L rbf (round bottom flask) and dissolved with 250 mL of MeOH. The flask was then attached to a rotavapor at 75°C. The mixture was allowed to gently spin at this temperature for about 20 mins. to ensure complete dissolution. After the formation of a clear solution, about 10mg of pure crystals were added (to aid the crystal formation) was allowed to settle gently settle down for crystal formation. After 3 days, 19.4g of crystals were recovered which was then washed with cold MeOH to give 18.2g of crystals.

The chiral purity of the crystals was determined by derivatizing with 4-nitrobenzyl chloroformate and subjecting it to HPLC under the conditions of chiral AD column, 20% isopropanol/hexane solvent system and a flow rate of 1 mUmin. The purity was found to be - 99.9% with a retention time of 16.58 min.

### Step 5

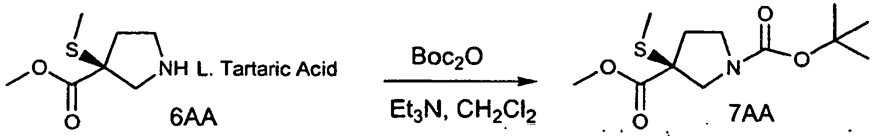

Compound 6AA (3.0g, 9.2 mmol) was dissolved in 100mL of CH₂Cl₂ at rt. 2.5 eq. of Et₃N was added followed by 1.2 eq. of Boc₂O. The reaction mixture was allowed to stir for 1 day after it was diluted with CH₂Cl₂ and washed with sat. NaHCO₃ followed by sat. NaCl. The organic layer was dried over anhydrous MgSO4 and evaporated to dryness. The crude product was column purified (20% EtAOc/Hex) and evaporated to give 2.43g of pure product in the yield of ∼ 96%.

### Step 5

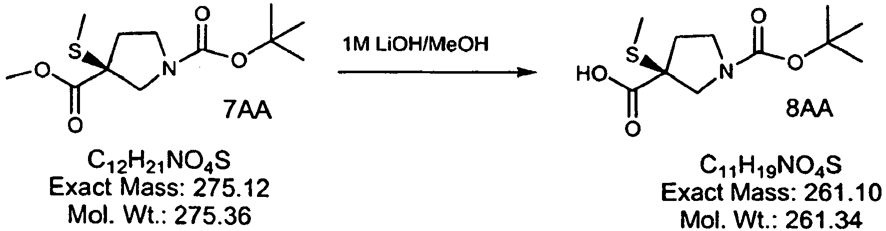

Compound 7AA (0.75g, 2.73 mmol) was dissolved in 20 mL of MeOH. 6 mL of 1 M LiOH was added to the reaction flask and the resulting solution was allowed to stir at rt for 1 overnight. Upon completion of the reaction (determined by TLC), 6 mL of 1 N HCl was added to neutralize the LiOH present in the reaction mixture. The mixture was then evaporated to dryness and azeotroped with dioxane to give 1.6g of 8AA as a white solid.

### Step 7

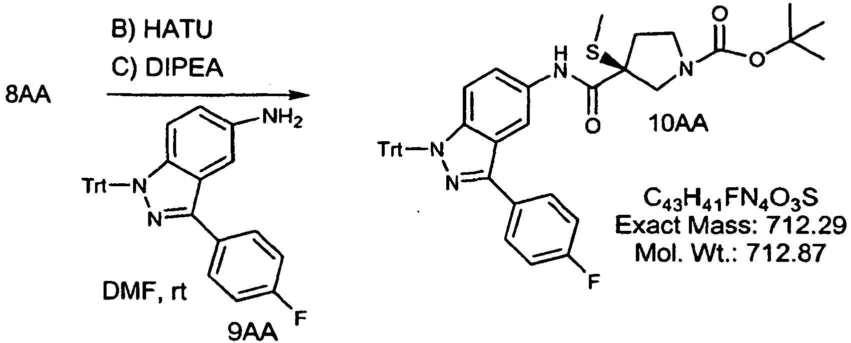

In a rbf equipped with septum inlet was placed 0.26g of 8AA (0.994 mMol) and 0.467g of 9AA (0.994 mmol). These 2 components were dissolved in DMF. DIPEA was added to the reaction mixture followed by HATU. The mixture was stirred rt under a stream of nitrogen for 2 days. The crude was then quenched with 50 mL of sat. NaHCO₃ and extracted (3x50 mL) with EtOAc. The combined organic extracts was washed with brine and dried over Na₂SO₄. It was then filtered and evaporated to dryness to give a crude product which was column purified (30% E/H) to give 0.605 of product.

### Step 8

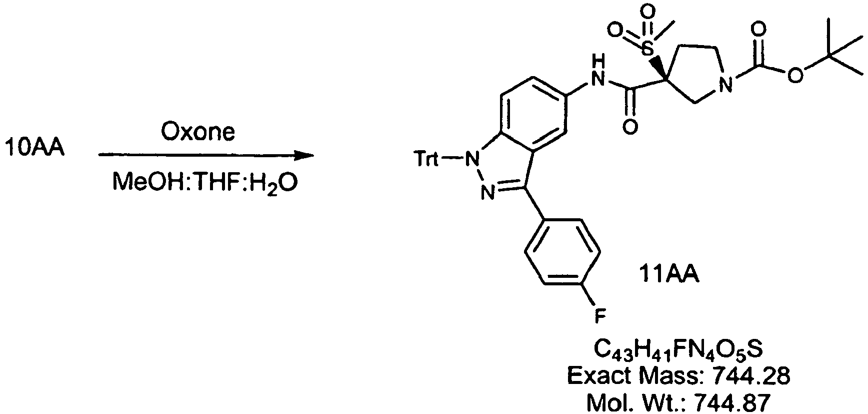

Compound 10AA (0.9g, 1.26 mMol) was dissolved in 6 mL each of MeOH:THF:H₂O. Oxone was added and the resulting mixture was allowed to stir for an overnight. Upon completion, the mixture was filtered through a glass fritz and washed with MeOH. The filtrate was then concentrated to give a crude product which was then subjected to the next step.

### Step 9

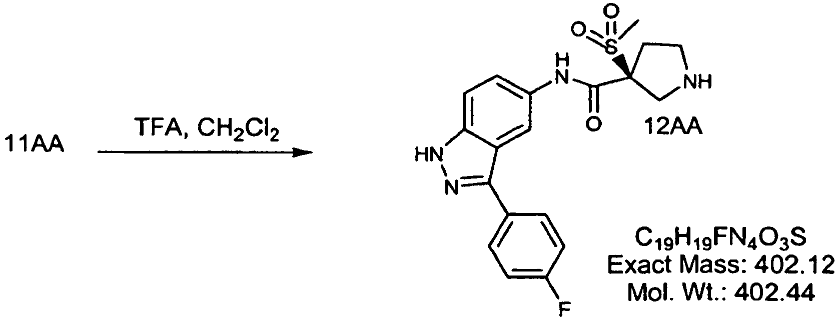

The starting material 11AA (0.64g, 0.858 mmol) was dissolved in 20 mL of anhydrous CH₂Cl₂. 2.5 mL of TFA was added slowly and was allowed to stir at rt under a stream of nitrogen for 1 overnight. The mixture was evaporated to dryness and column purified (7% 2N NH₃, MeOH/CH₂Cl₂) to give 0.31g of product in the yield of 91 %.

### Step 10

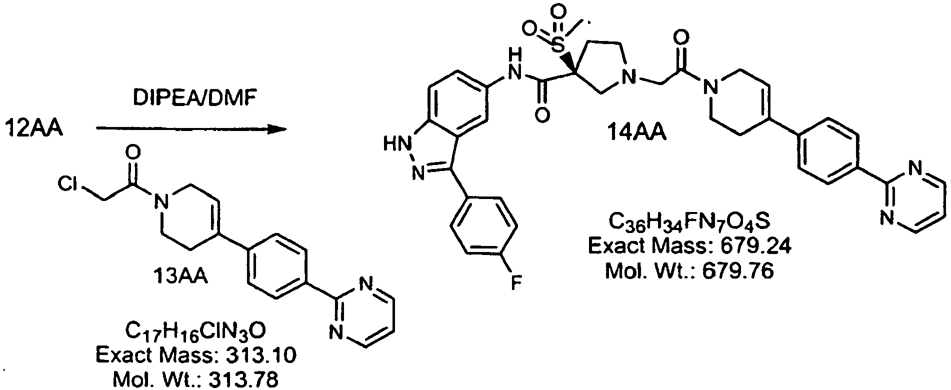

Compound 12AA (0.105g, 0.261 mmol) was dissolved in 5 mL of DMF. 3 eq. of DIPEA was added followed by 1.1 eq. of 13AA. The reaction mixture was stirred for 3 days at rt under a stream of nitrogen. Upon completion, 25 mL of brine was added to the reaction mixture which was then extracted 3 times with EtOAc. The combined organic extracts was dried over anhydrous MgSO₄, filtered and evaporated to obtain crude title product. The crude product was purified by PTLC (6% 2N NH₃, MeOH/CH₂Cl₂) to give 132 mg of pure product.

### EXAMPLE 613

### Step 1

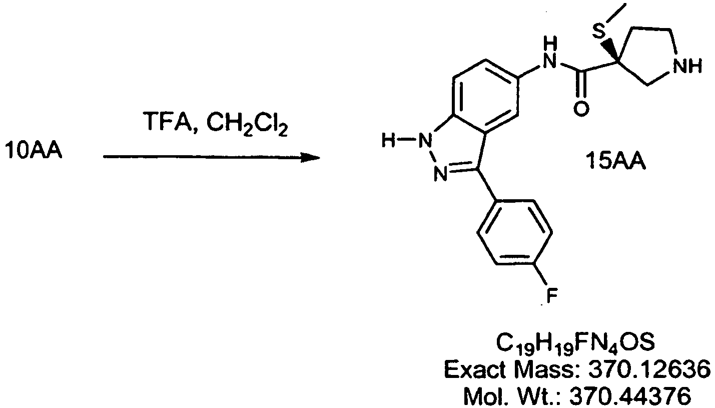

The starting material 10AA (see Example 612, Step 8) (0.712g, 1.0 mmol) was. dissolved in 20 mL of anhydrous CH₂Cl₂. 2 mL of TFA was added slowly and was allowed to stir at rt under a stream of nitrogen for 1 overnight. The mixture was evaporated to dryness and treated with 30 mL of 2% 2N NH₃, MeOH to give 0.612g of product.

### Step 2

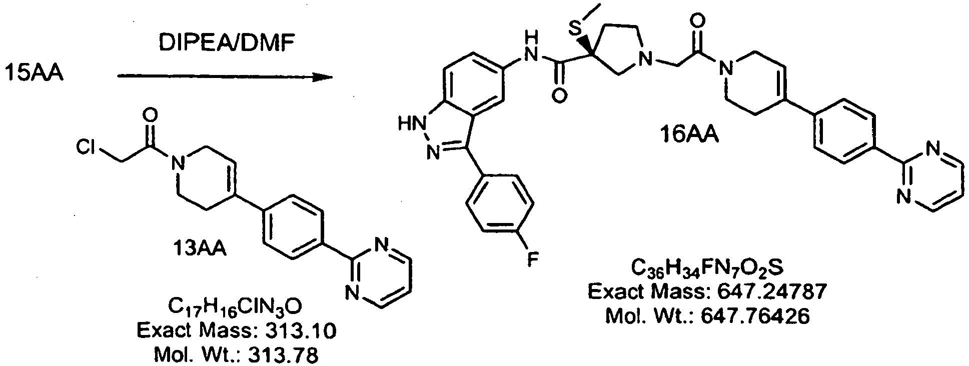

Compound 15AA (0.300g, 0.81 mmol) was dissolved in 5 mL of DMF. 3 eq. of DIPEA was added followed by 1.1 eq. of 13AA. The reaction mixture was stirred for 2 days at rt under a stream of nitrogen. Upon completion, 25 mL of brine was added to the reaction mixture which was then extracted 3 times with EtOAc. The combined organic extracts was dried over anhydrous MgSO₄, filtered and evaporated to obtain crude title product. The crude product was purified by PTLC (6% 2N NH₃, MeOH/CH₂Cl₂) to give 431 mg of pure product.

### Preparation 46

### Step 1

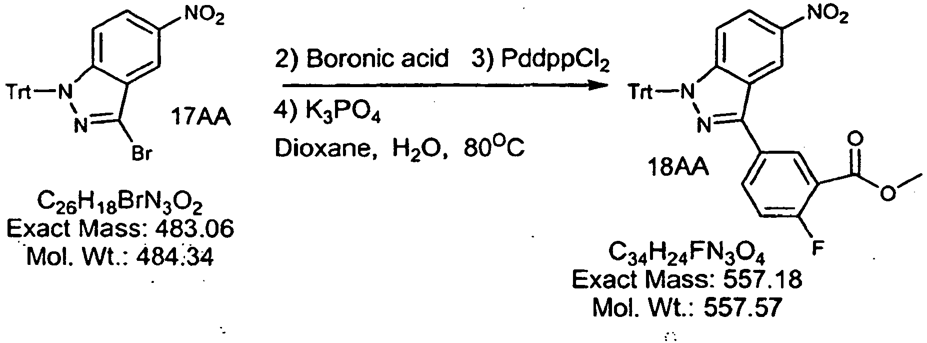

In a flame dried sealed tube was placed 3.0g of compound 17AA (6.19 mMol). 1.5 eq. of 2, 0.1 eq. of 3 and 2.5 eq. of 4 were also added. 65 mL of dioxane and 30 mL of H₂O were added to the mixture. The mixture was degassed under nitrogen and then heated to 80°C for 1 day. After completion, mixture was diluted with EtOAc and filtered through celite. The ethyl acetate layer was washed with water, dried over anhydrous Mg SO₄ and evaporated to dryness. The crude product was column purified (5%E/H) to give 1.1g of product.

### Step 2

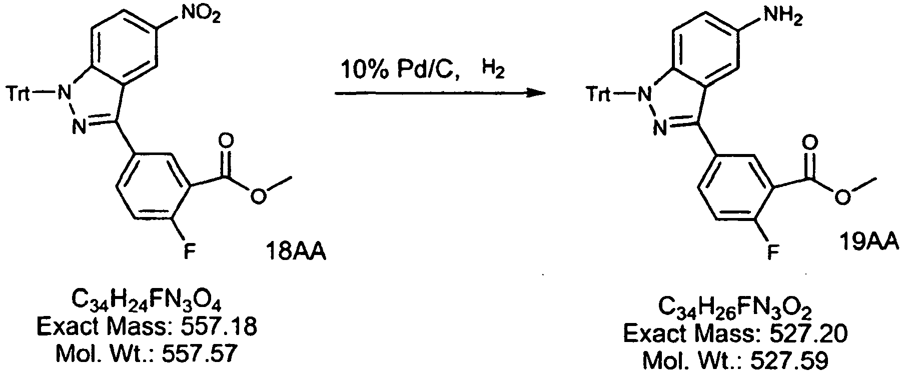

Compound 18AA (1.2g, 2.15 mmol) was dissolved in 20 mL each of MeOH:Toluene. 20% of 10% Pd/C catalyst was added and the mixture was subjected to H₂ in the form of ballon. After degassing for a number of times, mixture was allowed to stir for 1 day. After completion, the mixture was diluted with CH₂Cl₂ and filtered through celite. It was then washed with 50% E/H. The filtrate was evaporated to dryness to give 1.18g of title product.

### EXAMPLE 614

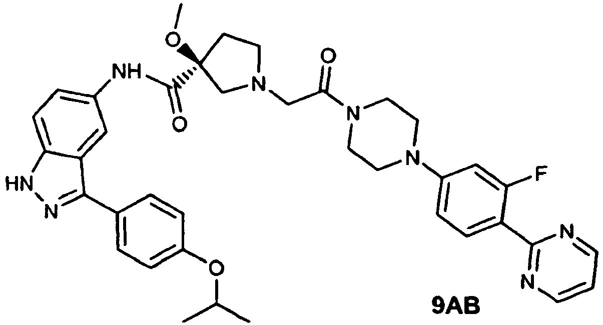

### Step 1: Synthesis of 2-(4-Bromo-2-fluoro-phenyl)-pyrimidine (3AB)

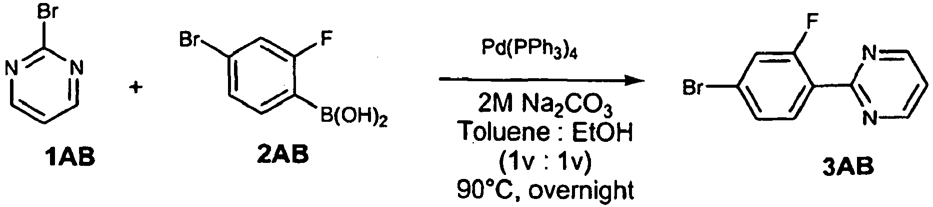

4-Bromo-2-fluorophenyl boronic acid **(2AB)** (3.0g, 13.71 mmol, 1 equiv), 2-bromopyrimidine **(1AB)** (6.54 g, 41.13 mmol, 3 equiv), and 2M sodium carbonate (34 mL) were added in a pressure vessel (350 mL) and a (1v : 1v) mixture of toluene and ethanol (45 mL : 45 mL) was added. The mixture was then bubbled with nitrogen gas for about 10 minutes. Tetrakistriphenylphosphine palladium (0) (793mg, 0.686 mmol, 0.05 equiv) was added to the mixture. The reaction vessel was tightly capped, placed in an oil bath at 90°C and stirred overnight.

The reaction mixture was cooled down to room temperature and the content was filtered into a flask and the solvent mixture was evaporated off on the rotovap. The residue was then taken up in one to one mixture of toluene and ethyl acetate and washed with (3v : 1 v) mixture of brine and DI water twice. The organic layer was separated and combined and dried over magnesium sulfate. The crude product was then filtered into a flask and the solvent was removed on rotovap. The residue was taken up in as little dichloromethane as possible and purified by column chromatography using Analogix purification system with the following conditions: Solvent A: Hexanes; Solvent B: Ethylacetate. Flow Rate: 65 mL/min. Gradient: 0% Solvent B to 50% Solvent B in 60 minutes.
Yield= 2.79 g (80.4%)

### Step 2: Synthesis of 2-(2-Fluoro-4-piperazin-1-yl-phenyl)-pyrimidine (5AB)

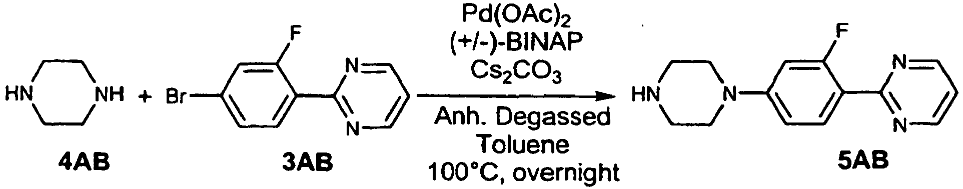

2-(4-Bromo-2-fluoro-phenyl)-pyrimidine **(3AB)** (2.0 g, 7.9 mmol, 1 equiv), piperazine **(4AB)** (2.72 g, 31.6 mmol, 4 equiv), cesium carbonate (20.6 g, 63.2 mmol, 8 equiv), racemic (+/-) BINAP (492 mg, 0.79 mmol, 0.1 equiv), and palladium (II) acetate (89 mg, 0.395 mmol, 0.05 equiv) were all weighed out in a flamed dried pressure vessel and the vessel was sealed with a rubber septa and the content of the reaction vessel was kept under vacuum for 2 hours. Anhydrous degassed toluene (100 mL) was added to the reaction vessel using a cannula. The rubber septa was replaced with a Teflon cap and the vessel was tightly sealed and placed in an oil bath at 100°C to stir the content overnight.

The reaction vessel was cooled down to room temperature and the content was transferred into a flask. Some water was added to solubilize the excess inorganic base along with some ethyl acetate. The organic layer was then washed with water and brine twice, and separated and dried over magnesium sulfate. The crude product was then filtered into a flask and the solvent was removed on rotovap. The residue was taken up in as little dichloromethane as possible and purified by column chromatography using Analogix purification system with the following conditions: Solvent A: Dichloromethane; Solvent B: 40% 7N NH₃ in Methanol. Flow Rate: 65 mL/min. Gradient: 0% Solvent B to 30% Solvent B in 52 minutes and stayed at 30% Solvent B for 10 minutes.
Yield= 889 mg (44%).

### Step 3: Synthesis of 2-Chloro-1-[4-(3-fluoro-4-pyrimidin-2-yl-phenyl)-piperazin-1-yl]-ethanone (7AB)

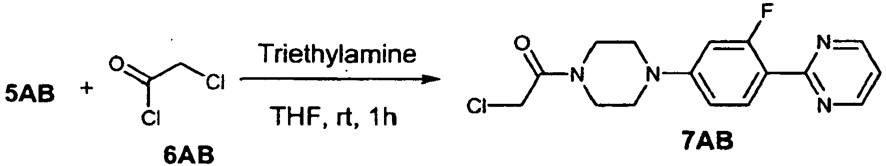

2-(2-Fluoro-4-piperazin-1-yl-phenyl)-pyrimidine **(5AB)** (889 mg, 3.442 mmol, 1 equiv) was dissolved in anhydrous tetrahydrofuran (5 mL) and triethylamine (697 mg, 959 uL, 6.884 mmol, 2 equiv) was added, followed by slow addition of a solution of chloroacetyl chloride **(6AB)** (466.5 mg, 330 uL, 4.13 mmol, 1.2 equiv) in tetrahydrofuran at room temperature. The mixture was then stirred for about 1 hour at room temperature.

Upon the completion of the reaction, the solvent was removed on rotovap and the residue was taken up in dichloromethane and washed with a (1v : 1v) mixture of brine and water in a seperatory funnel. The organic layer was separated, concentrated down, and dried on pump. The crude residue was then taken up in dichloromethane and purified by column chromatography using Analogix purification system with the following conditions: Solvent A: Dichloromethane; Solvent B: 40% 7N NH₃ in Methanol. Flow Rate: 65 mL/min. Gradient: 0% Solvent B to 30% Solvent B in 52 minutes and stayed at 30% Solvent B for 10 minutes.
Yield= 1.05 g (91.1 %)

### Step 4: Synthesis of 1-{2-[4-(3-Fluoro-4-pyrimidin-2-yl-phenyl)-piperazin-1-yl]-2-oxo-ethyl}-3-methoxy-pyrrolidine-3-carboxylic acid [3-(4-isopropoxy-phenyl)-1H-indazol-5-yl]-amide (9AB)

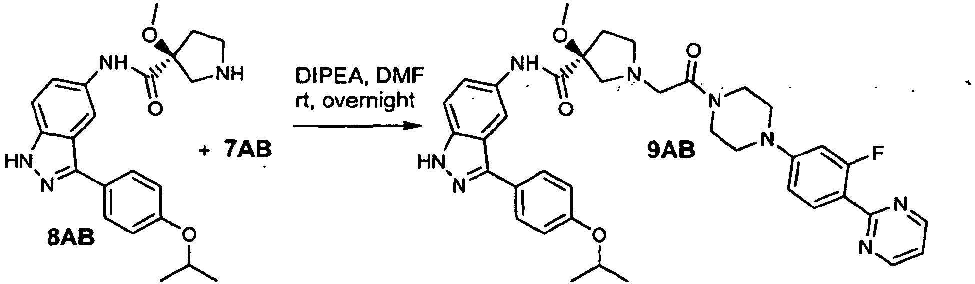

3-Methoxy-pyrrolidine-3-carboxylic acid [3-(4-isopropoxy-phenyl)-1H-indazol-5-yl]-amide **(8AB)** (40 mg, 0.086 mmol, 1 equiv) and 2-Chloro-1-[4-(3-fluoro-4-pyrimidin-2-yl-phenyl)-piperazin-1-yl]-ethanone **(7AB)** (35 mg, 0.103 mmol, 1.2 equiv) were weighed out in a flask, and to it was added N,N-dimethylformamide (3 mL) and diisopropylethylamine (22 mg, 30 uL, 0.172 mmol, 2 equiv). The reaction content was stirred at room temperature, overnight.

The crude reaction mixture was diluted out with enough amount of ethylacetate and the organic phase was washed with a (1v : 1v) mixture of brine and water twice. The organic layer was separated and concentrated down on rotovap to dryness, and further dried on a high vacuum pump. The residue was taken up in as little dichloromethane as possible and purified by column chromatography using Analogix purification system with the following conditions: Solvent A: Dichloromethane; Solvent B: 40% 7N NH₃ in Methanol. Flow Rate: 30 mL/min. Gradient: 0% Solvent B to 20% Solvent B in 30 minutes. The clean fractions were combined in a flask and concentrated down on rotovap. The residue was then dissolved in methanol and acidified with 4N HCl in 1,4-dioxane (80 uL) to make it into HCl salt form before submitting it to biological assay.
Yield= 46.4 mg, LCMS [M+H⁺] = 693.4.

### EXAMPLE 615

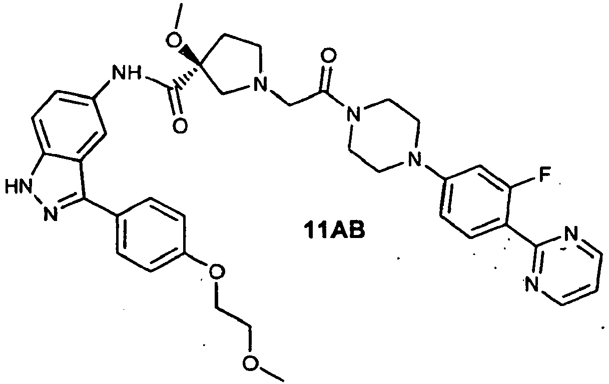

### Steps 1 to 3

Steps 1 to 3 are similar to Steps 1 to 3 of Example 614..

### Step 4: Synthesis of 1-{2-[4-(3-Fluoro-4-pyrimidin-2-yl-phenyl)-piperazin-1-yl]-2-oxo-ethyl}-3-methoxy-pyrrolidine-3-carboxylic acid {3-[4-(2-methoxy-ethoxy)-phenyl]-1H-indazol-5-yl}-amide (11AB)

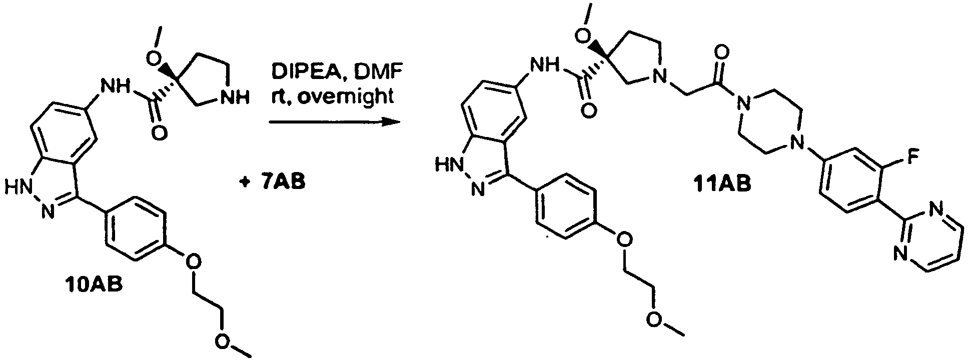

3-Methoxy-pyrrolidine-3-carboxylic acid {3-[4-(2-methoxy-ethoxy)₋phenyl]-1H-indazol-5-yl)-amide **(10AB)** (50 mg, 0.103 mmol, 1 equiv) and 2-Chloro-1-[4-(3-fluoro-4-pyrimidin-2-yl-phenyl)-piperazin-1-yl]-ethanone **(7AB)** (42 mg, 0.124 mmol, 1.2 equiv) were weighed out in a flask and to it was added N,N-dimethylformamide (3 mL) and diisopropylethylamine (26.6 mg, 36 uL, 0.206 mmol, 2 equiv). The reaction content was stirred at room temperature, overnight.

The crude reaction mixture was diluted out with enough amount of ethylacetate and the organic phase was washed with a (1v : 1v) mixture of brine and water twice. The organic layer was separated and concentrated down on rotovap to dryness, and further dried on a high vacuum pump. The residue was taken up in as little dichloromethane as possible and purified by column chromatography using Analogix purification system with the following conditions: Solvent A: Dichloromethane; Solvent B: 40% 7N NH₃ in Methanol. Flow Rate: 30 mL/min. Gradient: 0% Solvent B to 20% Solvent B in 30 minutes. The clean fractions were combined in a flask and concentrated down on rotovap. The residue was then dissolved in methanol and acidified with 4N HCl in 1,4-dioxane (100 uL) to make it into HCl salt form before submitting it to biological assay.
Yield= 46.4 mg, LCMS [M+H⁺] = 709.4.

### EXAMPLE 616

### Steps 1 to 3

Steps 1 to 3 are similar to Steps 1 to 3 of Example 614..

### Step 4: Synthesis of 1-{2-[4-(3-Fluoro-4-pyrimidin-2-yl-phenyl)-piperazin-1-yl]-2-oxo-ethyl}-3-methylsulfanyl-pyrrolidine-3-carboxylic acid [3-(4-ftuoro-phenyl)-1H-indazol-5-yl]-amide (13AB)

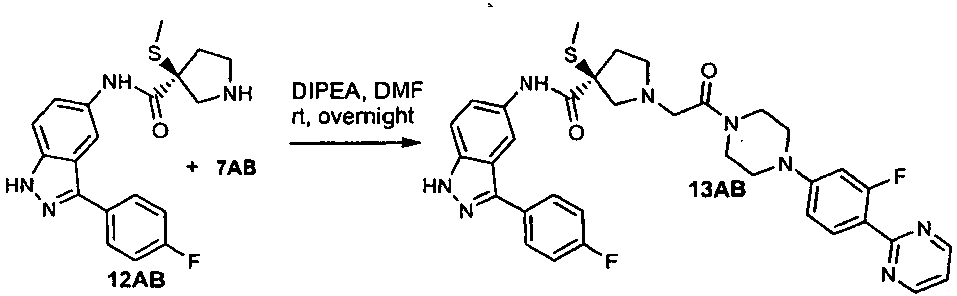

3-Methylsulfanyl-pyrrolidine-3-carboxylic acid [3-(4-fluoro-phenyl)-1H-indazol-5-yl]-amide **(12AB)** (40 mg, 0.09 mmol, 1 equiv) and 2-Chloro-1-[4-(3-fluoro-4-pyrimidin-2-yl-phenyl)-piperazin-1-yl]-ethanone **(7AB)** (37 mg, 0.11 mmol, 1.2 equiv) were weighed out in a flask, and to it was added N,N-dimethylformamide (3 mL) and diisopropylethylamine (23 mg, 32 uL, 0.18 mmol, 2 equiv). The reaction content was stirred at room temperature overnight.

The crude reaction mixture was diluted out with enough amount of ethylacetate and the organic phase was washed with a (1v : 1v) mixture of brine and water twice. The organic layer was separated and concentrated down on rotovap to dryness, and further dried on a high vacuum pump. The residue was taken up in as little dichloromethane as possible and purified by column chromatography using Analogix purification system with the following conditions: Solvent A: Dichloromethane; Solvent B: 40% 7N NH₃ in Methanol. Flow Rate: 30 mL/min. Gradient: 0% Solvent B to 20% Solvent B in 30 minutes. The clean fractions were combined in a flask and concentrated down on rotovap. The residue was then dissolved in methanol and acidified with 4N HCl in 1,4-dioxane (100 uL) to make it into HCl salt form before submitting it to biological assay.
Yield= 58 mg, LCMS [M+H⁺] = 669.4.

### EXAMPLE 617

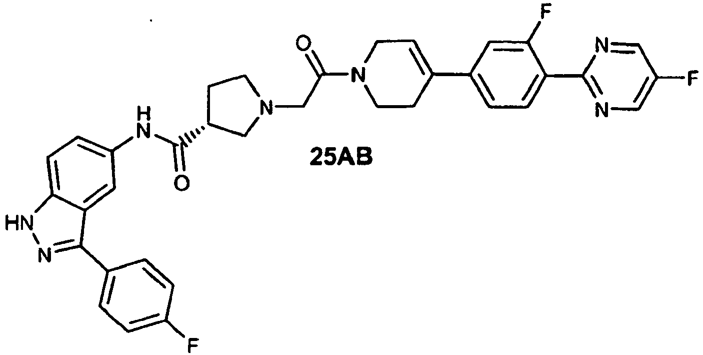

### Step 1: Synthesis of 4-(4-Bromo-3-fluoro-phenyl)-3,6-dihydro-2H-pyridine-1-carboxylic acid tert-butyl ester (16AB)

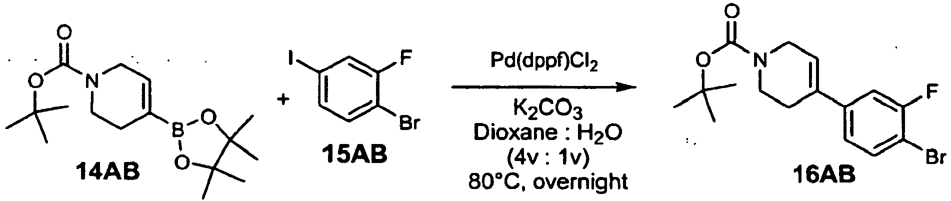

4-(4,4,5,5-Tetramethyl-[1,3,2]dioxaborolan-2-yl)-3,6-dihydro-2H-pyridine-1-carboxylic acid tert-butyl ester **(14AB)** (4.0g, 12.9 mmol, 1 equiv), 1-Bromo-2-fluoro-4-iodo-benzene **(15AB)** (5.84 g, 19.4 mmol, 1.5 equiv), potassium carbonate (5.4 g, 38.8 mmol, 3 equiv), and a (4v : 1 v) mixture of 1,4-dioxane and water (120 mL : 30 mL) were all added in a pressure vessel (350 mL) and the mixture was bubbled with nitrogen gas for about 10 minutes. To this mixture was added dichloro[1, 1'-bis(diphenylphosphino)ferrocene] palladium (II)/dichloromethane adduct (1.05 g, 1.29 mmol, 0.1 equiv), and the reaction vessel was tightly capped, placed in an oil bath at 80°C, and stirred overnight.

The reaction mixture was cooled down to room temperature and the content was transferred into a flask and concentrated down on rotovap. The residue was then taken up in ethyl acetate and, in a seperatory funnel; the crude mixture was washed with water, 10% sodium carbonate and brine. The organic layer was dried on magnesium sulfate and passed through a Celite plug. The filtrate was then treated with activated carbon at 65°C in an Erlenmeyer in a water bath for about 10 minutes to decolorize the solution. The charcoal was separated by a Celite plug. The solvent was removed on rotovap and the residue was dried on pump overnight. The residue was taken up in as little dichloromethane as possible and purified by column chromatography using Analogix purification system with the following conditions: Solvent A: Hexanes; Solvent B: Ethylacetate. Flow Rate: 65 mL/min. Gradient: 0% Solvent B to 50% Solvent B in 60 minutes.
Yield= 3.12 g (68%)

### Step 2: Synthesis of 4-[4-(5,5-Dimethyl-[1,3,2]dioxaborinan-2-yl)-3-fluoro-phenyl]-3,6-dihydro-2H-pyridine-1-carboxylic acid tert-butyl ester (18AB):

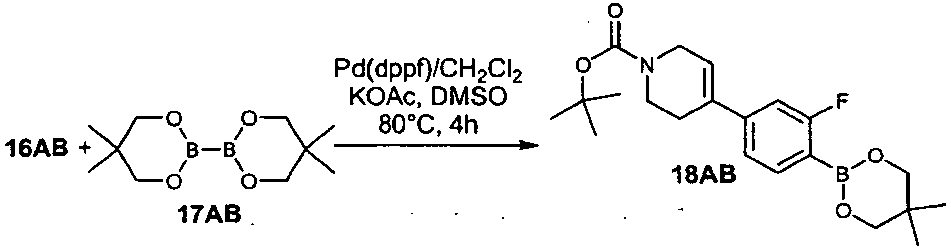

4-(4-Bromo-3-fluoro-phenyl)-3,6-dihydro-2H-pyridine-1-carboxylic acid tert-butyl ester (**16AB**)(2.3 g, 6.59 mmol, 1 equiv), bis(neopentylglycolato)diboron **(17AB)**(1.79 g, 7.91 mmol, 1.2 equiv), and potassium acetate (1.94 g, 19.77 mmol, 3 equiv) were all weighed out in a dry pressure vessel and dissolved in dimethylsulfoxide (50 mL). The mixture was bubbled with nitrogen gas for 10 minutes. Dichloro[1, 1'-bis(diphenylphosphino) ferrocene]palladium (II)/dichloromethane adduct (540 mg, 0.66 mmol, 0.1 equiv) was added and the reaction vessel was sealed tightly with a cap and placed in on oil bath at 80°C for 4 hours.

Upon the completion of 4 hours, the reaction vessel was cooled down to room temperature and the content was transferred into a flask. Some water was added to solubilize the excess inorganic base along with some ethyl acetate. The organic layer was then washed with water and brine twice, and separated and dried over magnesium sulfate. The organic layer was concentrated down on rotovap and taken up with dichloromethane. In an Erlenmeyer the crude compound was treated with activated carbon at 65°C in a water bath for about 10 minutes to decolorize the solution. The charcoal was separated by a Celite plug. The solvent was removed on rotovap and the residue was dried on pump overnight. The residue was taken up in as little dichloromethane as possible and purified by column chromatography using Analogix purification system with the following conditions: Solvent A: Hexanes; Solvent B: Ethylacetate. Flow Rate: 65 mL/min. Gradient: 0% Solvent B to 50% Solvent B in 60 minutes. Relatively clean fractions were combined and the solvent was concentrated down. The product spot on TLC was streaking; that is probably because during the purification of this compound on a silica gel column some of the boronic acid ester was getting hydrolyzed to boronic acid. Therefore, even though the separation was not as desirable, the compound was used as-is in the next reaction after the purification step.

### Step 3: Synthesis 4-[3-Fluoro-4-(5-fluoro-pyrimidin-2-yl)-phenyl]-3,6-dihydro-2H-pyridine-1-carboxylic acid tert-butyl ester (21AB)

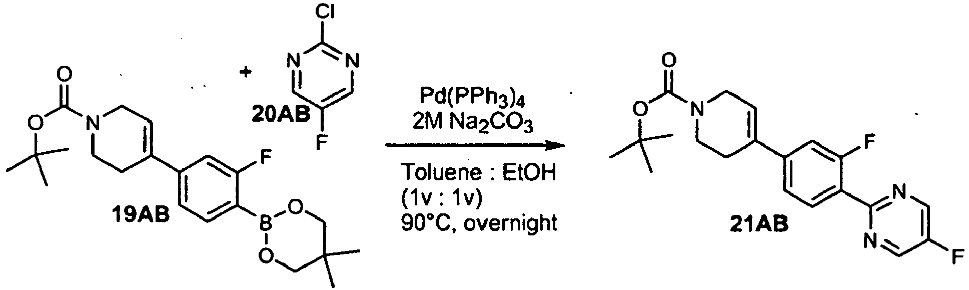

4-[4-(5,5-Dimethyl-[1,3,2]dioxaborinan-2-yl)-3-fluoro-phenyl]-3,6-dihydro-2H-pyridine-1-carboxylic acid tert-butyl ester (**19AB**)(1.55g, 3.98 mmol, 1 equiv), 2-Chloro-5-fluoro-pyrimidine **(20AB)**(634 mg, 591 uL, 4.78 mmol, 1.2 equiv), and 2M sodium carbonate (9.95 mL) were added in a pressure vessel (350 mL) and a (1v : 1 v) mixture of toluene and ethanol (25 mL : 25 mL) was added. The mixture was then bubbled with nitrogen gas for about 10 minutes. Tetrakis(triphenylphosphine) palladium (0) (462 mg, 0.4 mmol, 0.1 equiv) was added to the mixture. The reaction vessel was tightly capped, placed in an oil bath at 90°C, and stirred overnight.

The reaction mixture was cooled down to room temperature and diluted with ethyl acetate. The crude mixture was transferred into a seperatory funnel and washed with a (1v : 1 v) brine and water mixture. The organic layer was separated and combined and dried over magnesium sulfate. The crude product was then filtered into a flask and the solvent was removed on rotovap. The residue was taken up in as little dichloromethane as possible and purified by column chromatography using Analogix purification system with the following conditions: Solvent A: Dichloromethane; Solvent B: Methanol. Flow Rate: 45 mL/min. Gradient: 0% Solvent B to 10% Solvent B in 60 minutes.
Yield= 677 mg (46%)

### Step 4: Synthesis of 5-Fluoro-2-[2-fluoro-4-(1,2,3,6-tetrahydro-pyridin-4-yl)-phenyl]-pyrimidine (22AB)

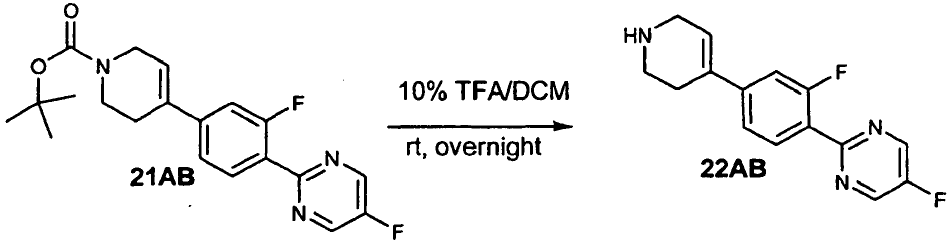

4-[3-Fluoro-4-(5-fluoro-pyrimidin-2-yl)-phenyl]-3,6-dihydro-2H-pyridine-1-carboxylic acid tert-butyl ester **(21AB)** (717 mg, 1.92 mmol, 1 equiv) was treated with 10% solution of trifluoroacetic acid in dichloromethane at room temperature overnight.

The solvent was concentrated down and the residue was taken up in ethyl acetate and washed with 10% aqueous sodium carbonate twice. Water layers were combined and saturated with sodium chloride and the remaining product in water layer thus extracted with ethyl acetate. The organic layers were combined and evaporated to dryness on a rotovap. The residue was taken up in as little dichloromethane as possible and purified by column chromatography using Analogix purification system with the following conditions: Solvent A: Dichloromethane; Solvent B: 40% 7N NH₃ in Methanol. Flow Rate: 65 mL/min. Gradient: 0% Solvent B to 30% Solvent B in 60 minutes and stayed at 30% Solvent B for 10 minutes. LCMS [M+H⁺] = 274.2.

### STEP 5: Synthesis of 2-Chloro-1-{4-[3-fluoro-4-(5-fluoro-pyrimidin-2-yl)-phenyl]-3,6-dihydro-2H-pyridin-1-yl}-ethanone (23)

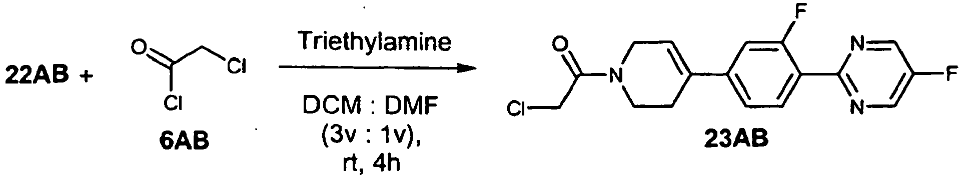

5-Fluoro-2-[2-fluoro-4-(1,2,3,6-tetrahydro-pyridin-4-yl)-phenyl]-pyrimidine **(22AB)** (1.94 g, 7.1 mmol, 1 equiv) was dissolved in a (3v : 1v) mixture of dichloromethane (30 mL) and N,N-dimethyl formamide (10 mL) and triethylamine (862 mg, 1.19 mL, 8.52 mmol, 1.2 equiv) was added, followed by slow addition of chloroacetyl chloride (962 mg, 678 uL, 8.52 mmol, 1.2 equiv) at room temperature. The mixture was then stirred for about 4 hours at room temperature.

Upon the completion of the reaction, the solvent mixture was removed on rotovap and the residue was taken up in dichloromethane and washed with saturated solution of sodium bicarbonate and a (1v : 1v) mixture of brine and water in a seperatory funnel. The organic layer was separated, concentrated down, and dried on pump. The crude residue was then taken up in dichloromethane and purified by column chromatography using Analogix purification system with the following conditions: Solvent A: Dichloromethane; Solvent B: 40% 7N NH₃ in Methanol. Flow Rate: 65 mL/min. Gradient: 0% Solvent B to 30% Solvent B in 52 minutes and stayed at 30% Solvent B for 10 minutes.
Yield= 851 mg (34%)

### Step 6: Synthesis of 1-(2-{4-[3-Fluoro-4-(5-fluoro-pyrimidin-2-yl)-phenyl]-3,6-dihydro-2H-pyridin-1-yl}-2-oxo-ethyl)-pyrrolidine-3-carboxylic acid [3-(4-fluoro-phenyl)-1H-indazol-5-yl]-amide (25AB)

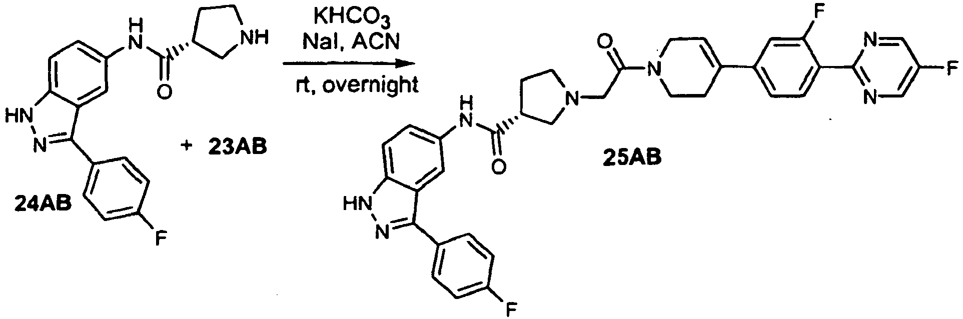

Pyrrolidine-3-carboxylic acid [3-(4-fluoro-phenyl)-1H-indazol-5-yl]-amide **(24AB)** (30 mg, 0.093 mmol, 1 equiv), 2-Chloro-1-{4-[3-fluoro-4-(5-fluoro-pyrimidin-2-yl)-phenyl]-3,6-dihydro-2H-pyridin-1-yl}-ethanone **(23AB)** (36 mg, 0.102 mmol, 1.1 equiv), potassium bicarbonate (47 mg, 0.465 mmol, 5 equiv), and sodium iodide (3 mg, 0.019 mmol, 0.2 equiv) were all weighed out in a flask and to it was added acetonitrile (3 mL). The reaction content was stirred at room temperature overnight.

The crude reaction mixture was concentrated down on rotovap and the residue was taken up in ethyl acetate. In a seperatory funnel, the organic phase was washed with a (1v : 1v) mixture of brine and water twice. The organic layer was separated and concentrated down on rotovap to dryness, and further dried on a high vacuum pump. The residue was taken up in as little dichloromethane as possible and purified by column chromatography using Analogix purification system with the following conditions: Solvent A: Dichloromethane; Solvent B: 40% 7N NH₃ in Methanol. Flow Rate: 30 mL/min. Gradient: 0% Solvent B to 20% Solvent B in 30 minutes. The clean fractions were combined in a flask and concentrated down on rotovap. The residue was then dissolved in methanol and acidified with 4N HCl in 1,4-dioxane (100 uL) to make it into HCl salt form before submitting it to biological assay.
Yield= 16.9 mg, LCMS [M+H⁺] = 638.4.

### Example 618

### Synthesis of 1-(2-{4-[3-Fluoro-4-(5-fluoro-pyrimidin-2-yl)-phenyl]-3,6-dihydro-2H-pyridin-1-yl}-2-oxo-ethyl)-3-methoxymethyl-pyrrolidine-3-carboxylic acid [3-(2-cyclopropyl-pyridin-4-yl)-1H-indazol-5-yl]-amide (27AB)

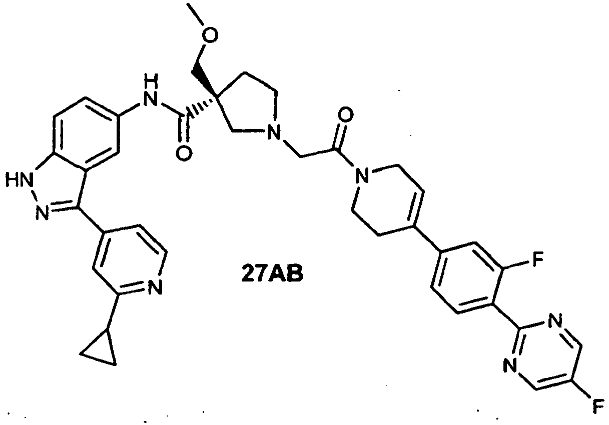

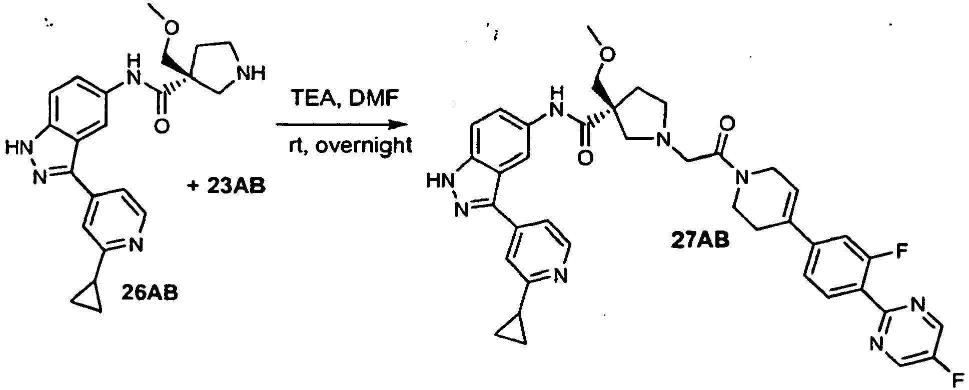

3-Methoxymethyl-pyrrolidine-3-carboxylic acid [3-(2-cyclopropyl-pyridin-4-yl)-1 H-indazol-5-yl]-amide **(26AB)** (85 mg, 0.217 mmol, 1 equiv) and 2-Chloro-1-{4-[3-fluoro-4-(5-fluoro-pyrimidin-2-yl)phenyl]-3,6-dihydro-2H-pyridin-1-yl}-ethanone **(23AB)** (84 mg, 0.239 mmol, 1.1 equiv)were weighed out in a flask and dissolved in anhydrous N,N-dimethylformamide (5 mL). Triethyl amine (33 mg, 46 uL, 0.326 mmol, 1.5 equiv) was added to the reaction mixture and the reaction content was stirred at room temperature overnight.

The crude reaction mixture was diluted out with enough amount of ethylacetate and the organic phase was washed with a saturated solution of sodium bicarbonate and a (1v : 1v) mixture of (brine : water) twice. The organic layer was separated and concentrated down on rotovap to dryness, and further dried on a high vacuum pump. The residue was taken up in as little dichloromethane as possible and purified by column chromatography using Analogix purification system with the following conditions: Solvent A: Dichloromethane; Solvent B: 40% 7N NH₃ in Methanol. Flow Rate: 30 mL/min. Gradient: 0% Solvent B to 20% Solvent B in 30 minutes. The clean fractions were combined in a flask and concentrated down on rotovap. The residue was then dissolved in methanol and acidified with 4N HCl in 1,4-dioxane (100 uL) to make it into HCl salt form before submitting it to biological assay.
Yield= 75 mg (49%), LCMS [M+H⁺] = 705.4.

### Example 619

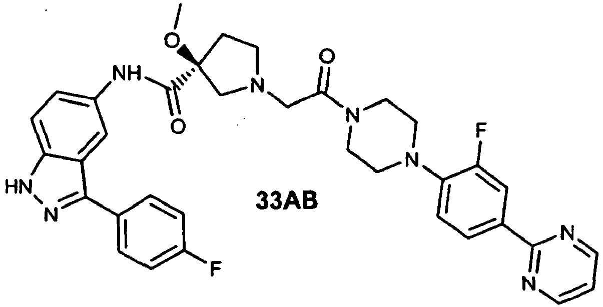

### Step 1: Synthesis of 2-(4-Bromo-3-fluoro-phenyl)-pyrimidine (29AB)

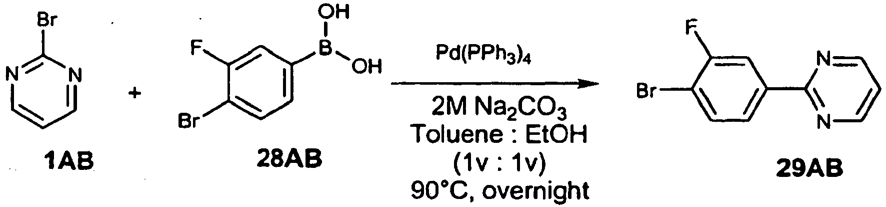

4-Bromo-3-fluorophenyl boronic acid **(28AB)** (1.0g, 4.57 mmol, 1 equiv), 2-bromopyrimidine **(1AB)** (2.18 g, 13.7 mmol, 3 equiv), and 2M sodium carbonate (12 mL) were added in a pressure vessel (150 mL) and a (1v: 1v) mixture of toluene and ethanol (25 mL : 25 mL) was added. The mixture was then bubbled with nitrogen gas for about 10 minutes. Tetrakistriphenylphosphine palladium (0) (266 mg, 0.23 mmol, 0.05 equiv) was added to the mixture. The reaction vessel was tightly capped, placed in an oil bath at 90°C, and stirred overnight.

The reaction mixture was cooled down to room temperature and the content was filtered into a flask and the solvent mixture was evaporated off on the rotovap. The residue was then taken up in one to one mixture of toluene and ethyl acetate and washed with (3v : 1 v) mixture of brine : DI water twice. The organic layer was separated and combined and dried over magnesium sulfate. The crude product was then filtered into a flask and the solvent was removed on rotovap. The residue was taken up in as little dichloromethane as possible and purified by column chromatography using Analogix purification system with the following conditions: Solvent A: Hexanes; Solvent B: Ethylacetate. Flow Rate: 65 mL/min. Gradient: 0% Solvent B to 50% Solvent B in 60 minutes.
Yield= 1.08 g (94%)

### Step 2: Synthesis of 2-(3-Fluoro-4-piperazin-1-yl-phenyl)-pyrimidine (30AB)

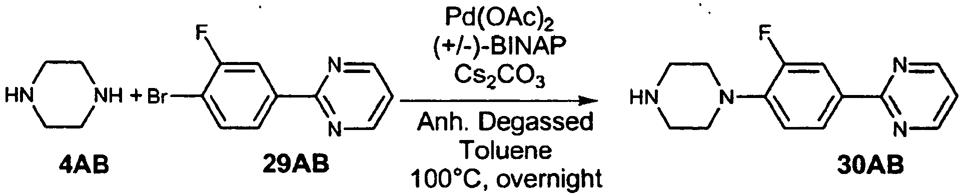

2-(4-Bromo-3-fluoro-phenyl)-pyrimidine **(29AB)** (874 mg, 3.45 mmol, 1 equiv), piperazine (1.19 g, 13.8 mmol, 4 equiv), cesium carbonate (9.0 g, 27.6 mmol, 8 equiv), racemic (+/-) BINAP (215 mg, 0.345 mmol, 0.1 equiv), and palladium (II) acetate (38.8 mg, 0.173 mmol, 0.05 equiv) were all weighed out in a flamed dried pressure vessel and the vessel was sealed with a rubber septa and the all-solid mixture was kept under vacuum for 2 hours. Anhydrous degassed toluene (30 mL) was added to the reaction vessel using a cannula. The rubber septa was replaced with a Teflon cap and the vessel was tightly sealed and placed in an oil bath at 100°C to stir the content overnight.

The reaction vessel was cooled down to room temperature and the content was transferred into a flask. Some water was added to solubilize the excess inorganic base along with some ethyl acetate. The organic layer was then washed with water and brine twice, and separated and dried over magnesium sulfate. The crude product was then filtered into a flask and the solvent was removed on rotovap. The residue was taken up in as little dichloromethane as possible and purified by column chromatography using Analogix purification system with the following conditions: Solvent A: Dichloromethane; Solvent B: 40% 7N NH₃ in Methanol. Flow Rate: 40 mL/min. Gradient: 0% Solvent B to 30% Solvent B in 52 minutes and stayed at 30% Solvent B for 10 minutes.
Yield= 675 mg (76%)

### Step 3: Synthesis of 2-Chloro-1-[4-(2-fluoro-4-pyrimidin-2-yl-phenyl)-piperazin-1-yl]-ethanone (31AB)

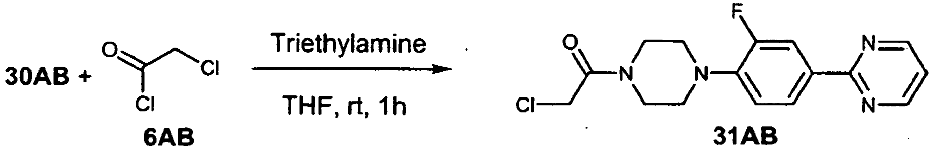

2-(3-Fluoro-4-piperazin-1-yl-phenyl)-pyrimidine **(29AB)** (675 mg, 2.61 mmol, 1 equiv) was dissolved in anhydrous tetrahydrofuran (5 mL), and triethylamine (1.32 mg, 1.82 mL, 13.05 mmol, 5 equiv) was added, followed by slow addition of a solution of chloroacetyl chloride (591 mg, 417 uL, 5.23 mmol, 2 equiv) in tetrahydrofuran at room temperature. The mixture was then stirred for about 1 hour at room temperature.

Upon the completion of the reaction, the solvent was removed on rotovap and the residue was taken up in dichloromethane and washed with a (1v : 1 v) mixture of brine and water in a seperatory funnel. The organic layer was separated, concentrated down, and dried on pump. The crude residue was then taken up in dichloromethane and purified by column chromatography using Analogix purification system with the following conditions: Solvent A: Dichloromethane; Solvent B: 40% 7N NH₃ in Methanol. Flow Rate: 40 mL/min. Gradient: 0% Solvent B to 30% Solvent B in 52 minutes and stayed at 30% Solvent B for 10 minutes.
Yield= 821 mg (94%)

### Step 4: Synthesis of 1-{2-[4-(2-Fluoro-4-pyrimidin-2-yl-phenyl)-piperazin-1-yl]-2-oxo-ethyl}-3-methoxy-pyrrrolidine-3-carboxylic acid [3-(4-fluoro-phenyl)-1H-indazol-5-yl]-amide (33AB)

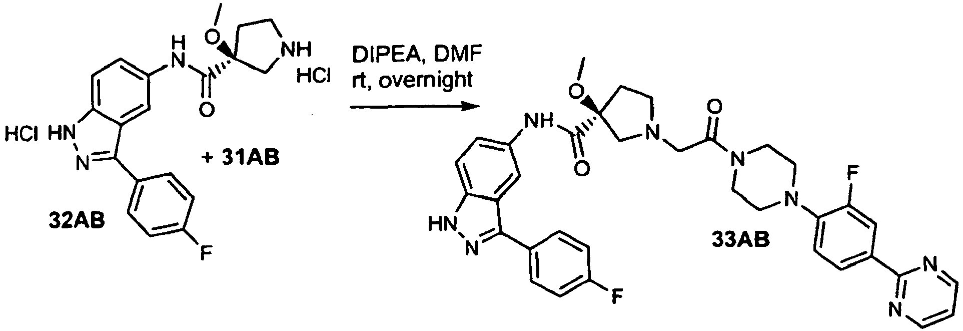

3-Methoxy-pyrrolidine-3-carboxylic acid [3-(4-fluoro-phenyl)-1H-indazol-5-yl]-amide 2.HCl Salt **(32AB)** (30 mg, 0.07 mmol, 1 equiv) and 2-Chloro-1-[4-(2-fluoro-4-pyrimidin-2-yl-phenyl)-piperazin-1-yl]-ethanone **(31AB)** (28 mg, 0.08 mmol), 1.2 equiv) were weighed out in a flask and to it was added N,N-dimethylformamide (3 mL) and diisopropylethylamine (54.3 mg, 73 uL, 0.42 mmol, 6 equiv). The reaction content was stirred at room temperature overnight.

The crude reaction mixture was diluted out with enough amount of ethylacetate and the organic phase was washed with a (1v : 1v) mixture of brine and water twice. The organic layer was separated and concentrated down on rotovap to dryness, and further dried on a high vacuum pump. The residue was taken up in as little dichloromethane as possible and purified by column chromatography using Analogix purification system with the following conditions: Solvent A: Dichloromethane; Solvent B: 40% 7N NH₃ in Methanol. Flow Rate: 30 mL/min. Gradient: 0% Solvent B to 15% Solvent B in 30 minutes. The clean fractions were combined in a flask and concentrated down on rotovap. The residue was then dissolved in methanol and acidified with 4N HCl in 1,4-dioxane (100 uL) to make it into HCl salt form before submitting it to biological assay.
Yield= 41 mg (90%), LCMS [M+H⁺] = 653.4.

### Preparation 47

### Preparation of 2-Chloro-1-(5,8-dichloro-3,4-dihydro-1H-isoquinolin-2-yl)-ethanone

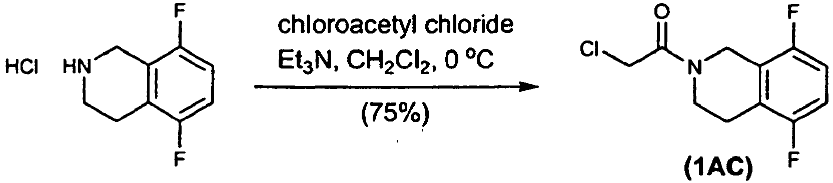

To a stirred suspension of 5, 8-di-fluoro-1,2,3,4-tetrahydroisoquinoline hydrochloride (0. 69g, 3.36 mmol) in dichloromethane (20 ml) at 0 °C under nitrogen, diisopropylethylamine (1.40 ml, 8.05 mmol) followed by chloroacetyl chloride (0.32 ml, 4.03 mmol) were added. The mixture was stirred at 0 °C for 2 hr. After being quenched with saturated sodium carbonate solution, water and dichloromethane were added. Layers were separated and the separated aqueous layer was extracted with dichloromethane. The combined organic layers were dried (MgSO₄), filtered and solvents were removed in vacuum. Column purification [Hexanes - Ethyl acetate, 4:1 (v/v)] gave chloride **1AC** (619 mg, 75%) as colourless oil.

### Preparation 48

### STEP 1: Preparation of 2-(4-Bromo-2,3-difluoro-phenyl)-pyrimidine

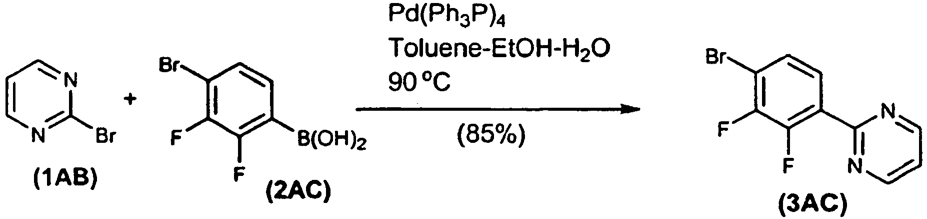

A mixture of 2-bromopyrimidine (2.0 g, 12.7 mmol), 4-bromo-2,3-difluorobenzeneboronic acid (1.0g, 4.22 mmol), potassium carbonate (2.93 g, 21.1 mmol) in a mixture of toluene (30 ml) /ethanol (30 ml) /water (15 ml) were purged with nitrogen for 15 min. Tetrakis(triphenylphosphine)palladium(0) (488 mg, 0.42 mmol) was added and the mixture was stirred at 90 °C in a sealed-tube for overnight. The mixture was cooled to r.t. and was diluted with water and ethyl acetate. Layers were separated. The separated organic layer was dried (MgSO₄), filtered and solvents were removed in vacuum. Column purification [Hexanes - Ethyl acetate, 2:1 (v/v)] gave bromide **3AC** (0.97 g, 85%) as white solid.

### Step 2: Preparation of 2-(2,3-Difluoro-4-piperazin-1-yl-phenyl)-pyrimidine

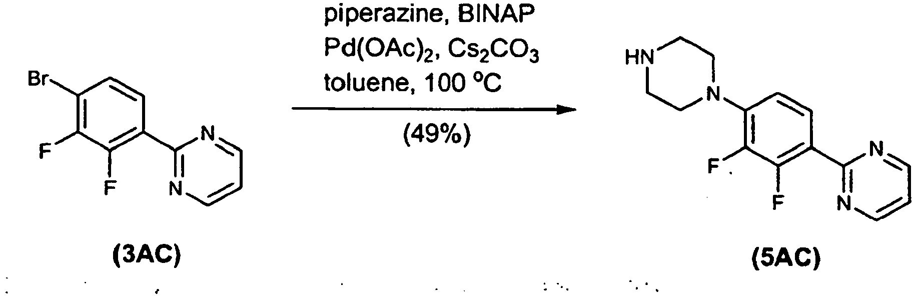

A mixture of bromide **3AC** (300 mg, 1.11 mmol), piperazine (286 mg, 3.32 mmol), BINAP (69 mg, 0.11 mmol), cesium carbonate (721 mg, 2.21 mmol) in toluene (10 ml) were purged with nitrogen for 15 min. Palladium (II) acetate (13 mg, 0.055 mmol) was added and the mixture was stirred at 100 °C in a sealed-tube for overnight. The mixture was cooled to r.t. and was diluted with water and ethyl acetate. Layers were separated. The separated organic layer was dried (MgSO₄), filtered and solvents were removed in vacuum. Column purification [Methanol - Ethyl acetate, 1:1 (v/v)] gave piperazine **SAC** (150 mg, 49%) as white solid.

### Step 3: Preparation of 2-Chloro-1-[4-(2,3-Difluoro-4-pyrimidin-2-yl-phenyl)-piperazin-1-yl]-ethanone

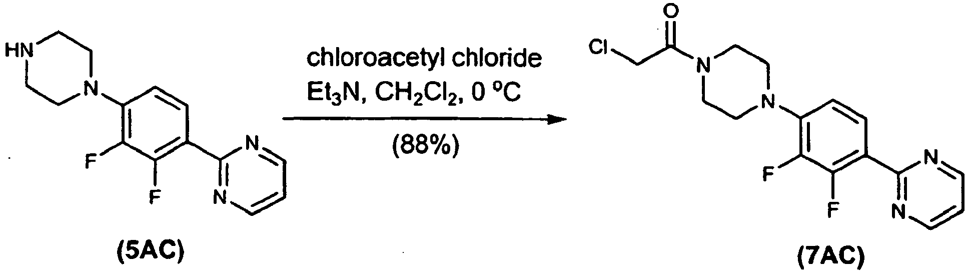

To a stirred solution of piperazine 5AC (150 mg, 0.54 mmol) in dichloromethane (5 ml) at 0 °C under nitrogen, triethylamine (0.076 ml, 0.54 mmol) followed by chloroacetyl chloride (0.043 ml, 0.54 mmol) were added. The mixture was stirred at 0 °C for 2 hr. After being quenched with saturated sodium carbonate solution, water and dichloromethane were added. Layers were separated and the separated aqueous layer was extracted with dichloromethane. The combined organic layers were dried (MSO₄), filtered and solvents were removed in vacuum. Column purification [Hexanes - Ethyl acetate, 1:1 (v/v)] gave chloride **7AC** (169 mg, 88%) white solid.

### Preparation 49

### Step 1: Preparation of 2-(4-Bromo-2,5-difluoro-phenyl)-pyrimidine

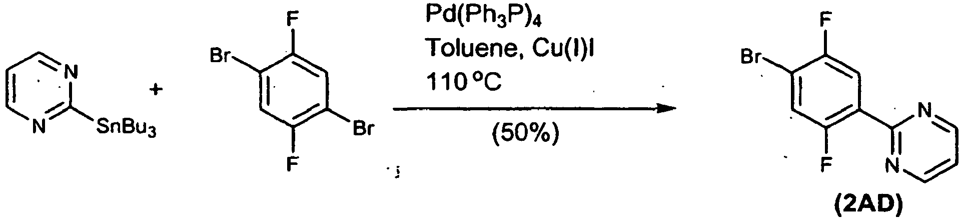

A mixture of 1, 4-dibromobenzene (4.4 g, 16.3 mmol), 2-(tributylstannyl)pyrimidine (3.0 g, 8.13 mmol), copper (I) iodide (154 mg, 0.81 mmol) in toluene (50 ml) were purged with nitrogen for 15 min. Tetrakis(triphenylphosphine)palladium(0) (939 mg, 0.81 mmol) was added and the mixture was stirred at 110 °C in a sealed-tube for 1 day. The mixture was cooled to r.t. and was diluted with water and ethyl acetate. Layers were separated. The separated organic layer was dried (MgSO₄), filtered and solvents were removed in vacuum. Column purification [Hexanes - Ethyl acetate, 2:1 (v/v)] gave bromide **2AD** (1.09 g. 50%) as white solid.

### Step 2: Preparation of 2-(2,5-Difluoro-4-piperazin-1-yl-phenyl)-pyrimidine

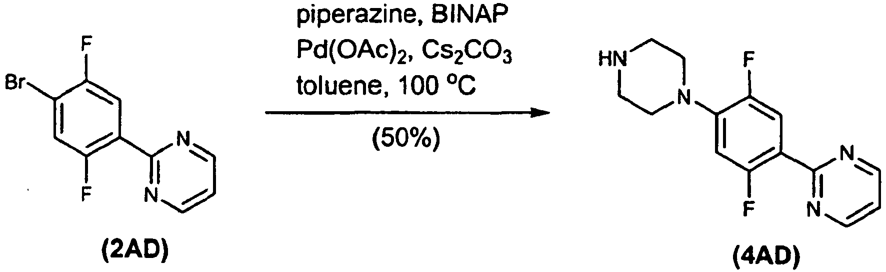

A mixture of bromide **2AD** (850 mg, 3.14 mmol), piperazine (810 mg, 9.41 mmol), BINAP (196 mg, 0.31 mmol), cesium carbonate (2.0 g, 6.27 mmol) in toluene (30 ml) were purged with nitrogen for 15 min. Palladium (II) acetate (35 mg, 0.16 mmol) was added and the mixture was stirred at 100 °C in a sealed-tube for overnight. The mixture was cooled to r.t. and was diluted with water and ethyl acetate. Layers were separated. The separated organic layer was dried (MgSO₄), filtered and solvents were removed in vacuum. Column purification [Methanol - Ethyl acetate, 1:1 (v/v)] gave piperazine **4AD** (393 mg, 43%) as white solid.

### Step 3: Preparation of 2-Chloro-1-[4-(2,5-Difluoro-4-pyrimidin-2-yl-phenyl)-piperazin-1-yl]-ethanone

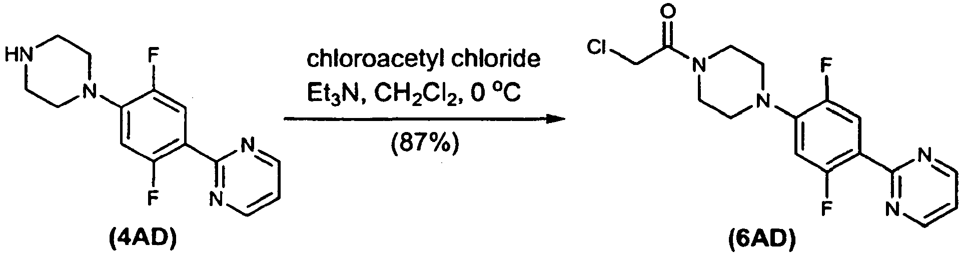

To a stirred solution of piperazine **4AD** (255 mg, 0.92 mmol) in dichloromethane (5 ml) at 0 °C under nitrogen, triethylamine (0.13 ml, 0.92 mmol) followed by chloroacetyl chloride (0.074 ml, 0.92 mmol) were added. The mixture was stirred at 0 °C for 2 hr. After being quenched with saturated sodium carbonate solution, water and dichloromethane were added. Layers were separated and the separated aqueous layer was extracted with dichloromethane. The combined organic layers were dried (MgSO₄), filtered and solvents were removed in vacuum. Column purification [Hexanes - Ethyl acetate, 1:1 (v/v)] gave chloride **6AD** (283 mg, 87%) white solid.

### Example 620

### STEP 1: Preparation of 3-Methoxy-3-[3-(2-methyl-1-oxy-pyridin-4-yl)-1-trityl-1H-indazol-5-ylcarbamoyl]-pyrrolidine-1-carboxylic acid tert-butyl ester

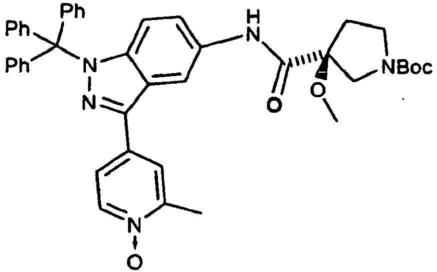

3-Methoxy-3-[3-(2-methyl-pyridin-4-yl)-1-trityl-1H-indazol-5-ylcarbamoyl]-pyrrolidine-1-carboxylic acid tert-butyl ester (2.9 g, 4.2 mmol) was dissolved in CH₂Cl₂ (80 mL) at rt followed by addition of mCPBA (2.0 g, max. 77%). After stirring for 24 hrs, the reaction solution was conc. to a small volume *in vacuo* and purified using silica gel column eluting with 2%, 5% and 10% methanol in CH₂Cl₂ to yield a brown solid (1.94 g).

### STEP 2: Preparation of 3-Methoxy-1-{2-oxo-2-[4-(4-pyrimidin-2-yl-phenyl)-piperazin-1-yl]-ethyl}-pyrrolidine-3-carboxylic acid [3-(2-methyl-1-oxy-pyridin-4-yl)-1H-indazol-5-yl]-amide

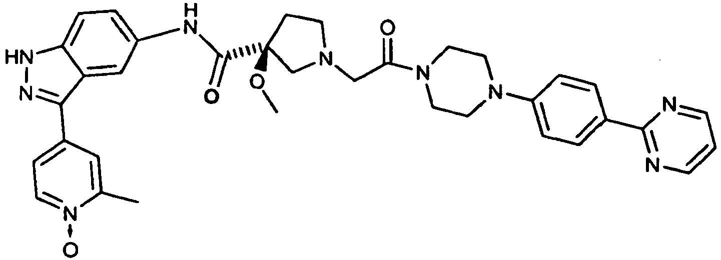

3-Methoxy-3-[3-(2-methyl-1-oxy-pyridin-4-yl)-1-trityl-1H-indazol-5-ylcarbamoyl]-pyrrolidine-1-carboxylic acid tert-butyl ester (170 mg) and triethylsilane (0.1 mL) were dissolved in CH₂Cl₂ (5 mL) at rt followed by addition of TFA (1 mL). After 2 hrs, the reaction mixture was conc. to dryness *in vacuo.* To this, DMF (5 mL), triethyl amine (0.1 mL) and 2-chloro-1-[4-(4-pyrimidin-2-yl-phenyl)-piperazin-1-yl]-ethanone (170 mg) were added. The resulting mixture was heated at 70 °C for 16 hrs. After cooling to rt, the reaction mixture was conc. to dryness. The resulting crude was purified via reverse phase HPLC (ACN/H2O/0.1% HCOOH) to yield a yellow solid (70.0 mg).
LCMS (M+1) 648.4 (rt 2.12)

### Preparation 50

### Preparation of 4-(3-Methoxy-4-pyrimidin-2-yl-phenyl)-3,6-dihydro-2H-pyridine-1-carboxylic acid tert-butyl ester

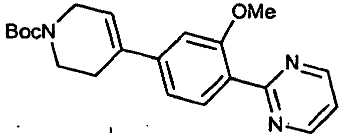

4-(3-Methoxy-4-pyrimidin-2-yl-phenyl)-3,6-dihydro-2H-pyridine-1-carboxylic acid tert-butyl ester was prepared using essentially the same scheme for Preparation 38 starting from 1-bromo-4-iodo-2-methoxy-benzene.

### Preparation 51

### Preparation of [5-(5-Amino-1H-indazol-3-yl)-2-fluoro-phenyl]-methanol

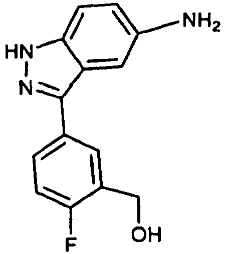

[5-(5-Amino-1H-indazol-3-yl)-2-fluoro-phenyl]-methanol was prepared using essentially the same scheme for preparing 10H (see Preparation 17 Step 3). The boronic acid for the first step was 4-fluoro-3-hydroxylmethylphenylboronic acid.

### Preparation 52

### Preparation of 4-Fluoro-4-(4-pyrimidin-2-yl-phenyl)-piperidine-1-carboxylic acid tert-butyl ester

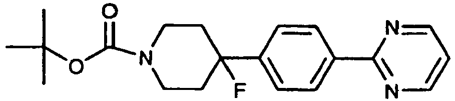

The above compound was prepared using a procedure similar to that of Preparation 54 Step 4 by using 4-(4-Bromo-phenyl)-4-fluoro-piperidine-1-carboxylic acid tert-butyl ester in place of **3AE.**

### Preparation 53

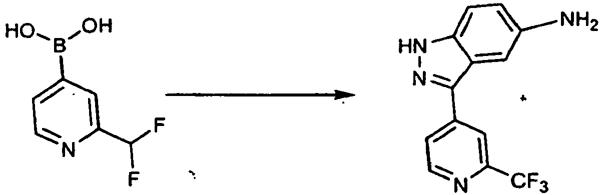

3-(2-Trifluoromethyl-pyridin-4-yl)-1H-indazol-5-ylamine was prepared by a procedure similar to that of Preparation 25 by substituting CF₃CO₂Et with CF₂CO₂Et in Preparation 25 (Chem. Het. Cpds, 1997, p995) and utilizing the procedure similar to that of Example 98 Steps 2 & 3,

### Preparation 54

### Step 1

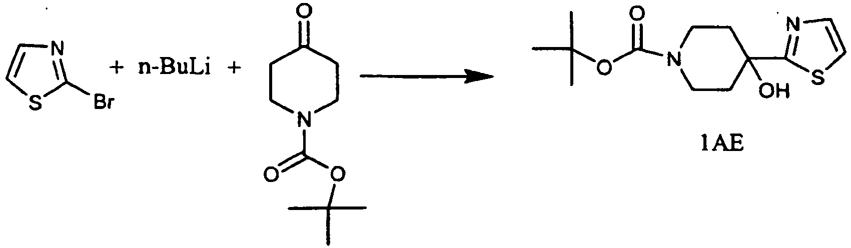

Compound 1AE was prepared following a procedure similar to that of Example 132.

### Step 2

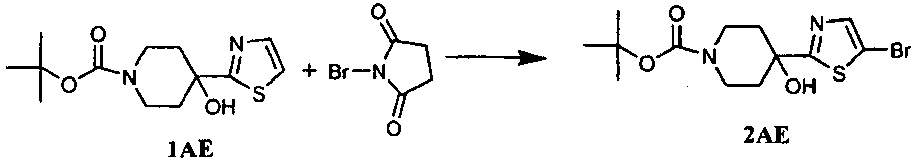

4.03 g (14.18 m.mole) **1AE** was dissolved in 50 ml anhy. CH₃CN at r.t. under dry N2 gas this 2.524 g (14.18 m.mole) of N-Bromosuccinimde was added at r.t. and mixture was heated a 50°C under dry N2 gas for 3 hrs.. The mixture was evaporated to dryness. The residue was partitioned between 100 ml EtOAc and 100 ml saturated NaHCO₃ solution. The organic phase dried over MgSO₄ and evaporated to dryness. The resulting brown gum was purified on silica g (Hexane-30% EtOAc/ Hexane) gave 1.3 g (25%) yellow solid.

### Step 3

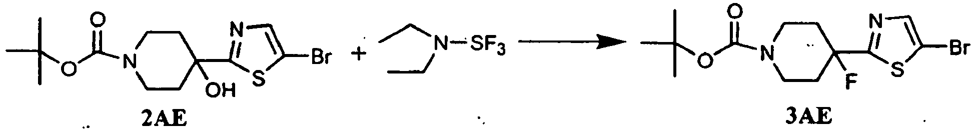

2.38 g (6.55 m.mole) of 3 was dissolved in 30 ml anhy. Dichloromethane at r.t. under dry N₂ gas. The mixture was cooled to 0°C in ice-bath and 2.112g (13.1 m. mole) of DAST was added dropwise at 0°C under dry N₂. The mixture was stirred at 0° C for 1 hr. The reaction mixture was cautiously (CO₂ gas evolution) basified with saturated NaHCO₃ solution at 0°C. The mixture was tranfered to separatory funnel and shaken well. The organic phase was removed and aqueous phase was extracted with 2X50 ml Dichloromethane. The combine organic phases were dried over MgSO₄ and evaporated to dryness, gave 2.368 (99%) off-white low melting solid.

### Step 4

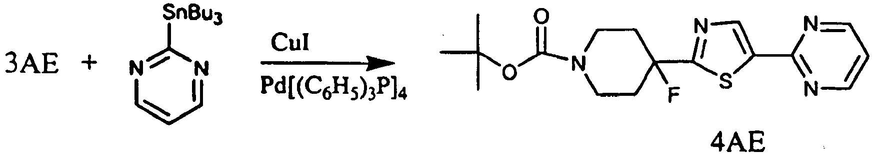

1.7g (4.65 m. mole) of **4AE** was dissolved in 15 ml anhy DMF at r.t. under dry N₂ gas. To this mixture Cul 0.93g (4.88 m.mole), Tetrakis(triphenylphosphine) palladium 0.537g (0.465 m.mole) and 2-Tributylstannylpyrimidine 1.762g (5.2 m.mole) were added and the mixture was stirred at 60°C under dry N₂ gas for 24 hrs. The mixture was concentrated to small volume, diluted with 50ml EtOAc and filtered through pad of celite. The filtrate was washed with brine and dried over MgSO₄. The evaporation of the solvent gave dark brown gum which was purified on silica gel (Hexane-25% EtOAc/ Hexane) gave 0.300g (17%) of brown solid.

### Preparation 55

### Step 1

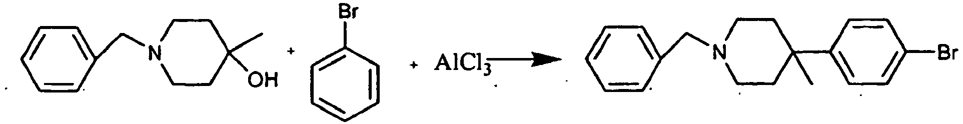

1-Benzyl-4-hydroxy-4-methyl piperidine (4.927g, 24m.mole) was dissolved in Bromobenzene(12ml, 114m.mole) at r.t.under dry N₂ gas. AlCl₃ (4.81g,36 m.mole) as solid was added to the above mixture at r.t. under dry N₂ gas. There was slightly exothermic reaction. The resulting dark brown solution was heated at 100°C over the week-end. The reaction was allowed to cool to r.t and was poured into ice-water. Saturated aqueous NaHCO₃ was added till pH 7. The mixture was extracted with 3X100 ml EtOAc. The combined organic extract dried over MgSO₄ and evaporated to dryness. The resulting dark brown gum was purified on silica and was eluted with (Hexane-25%EtOAc/Hexane), gave 4.43g (53%) as violet clear thick oil.

### Step 2

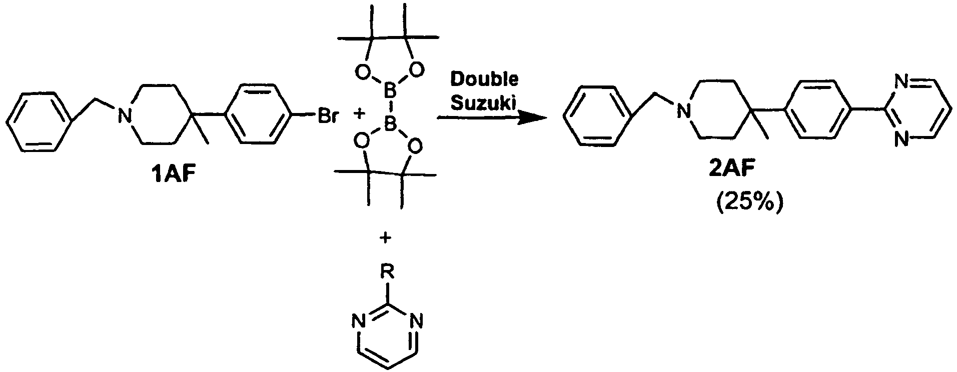

To a solution of 4.43 g (12.87 m.mole) of **1AF** in 100 ml anhy. DMSO were added 4.903 g (19.31 m.mole) of Bis(pinacolato)diboron, 3.784 g (38.61 m.mole) of Potassium acetate and 1.051 g (1.287 m.mole) of Pd(dppf)Cl₂ at r.t. under dry N₂ gas. The contents were degassed couple of times with N₂ gas and stirred at 100°C for 2 hrs. The mixture was allowed to cool to r.t. and 50 ml of water was added followed by 2.455 g (15.44 m.mole) of 2-Bromopyrimidine, 8.894 g (64.35 m.mole) of Potassium carbonate and 1.49 g (1.29 m.mole) of Tetrakis(triphenylphosphine)palladium. The contents were degassed couple of times with N2 gas and stirred at 100 C for 2 hrs. The mixture was allowed to cool to r.t.. 100 ml of water and 100 ml of EtOAc were added to the reaction mixture and filtered through pad of celite and washed with EtOAc. The contents were transfered to separatory funnel and the organic phase was separated and the aqueous phase was extracted with EtOAC . The organic phases were combined and washed with water and dried over MgSO4. The solvent was evaporated to dryness and dark brown gum was purified on silica gel (Hexane-25% EtOAC/Hexane), gave 1.00 off white solid.

### Step 3

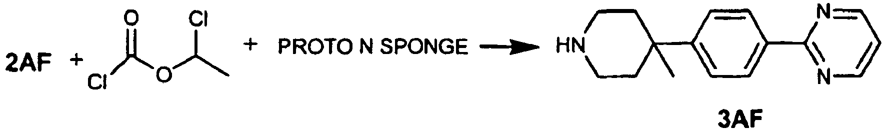

1.00 g(2.9 m.mole) **of 2AF** was in 20 ml anhy. dichloromethane at r.t. under dry N₂ gas. To this solution 0.178g (0.83 m.mole) Proton Sponge was added at r.t. followed by dropwise addition of 0.713g (4.99 m.mole) of 2-Chloroethyl chloroformate. The reaction mixture was stirred at r.t. under dry N₂ gas for 4 hrs. The mixture was evaporated to dryness and dried under high vacuum for 15 minutes. The resulting residue was dissolved in 20 ml anhy. MeOH under dry N₂ gas and was stirred under reflux under dry N₂ gas for 4 hrs. The mixture was allowed to cool to r.t. and evaporated to dryness. The crude was purified on silica gel (CH₂Cl₂-25%MeOH/CH₂Cl₂) and gave 0.606g (82%) white solid.

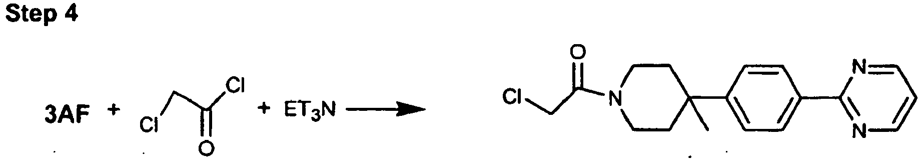

To a stirred solution of 0.60 g (2.37 m.mole) of **3AF** in 15 ml anhy. Dichloromethane 1.20 (11.85 m.mole) of Triethylamine was added at r.t. under dry N₂ gas. The mixture was cooled to ice- water bath and 0.321 g (2.84 m.mole) of Chloroacetylchloride was added dropwise at 0°C dry N₂ gas the mixture was stirred at 0°C for half an hr. 25 ml of CH₂Cl₂ and aqueous saturated NaHCO₃ solution were added at 0°C. The contents were transferred to separatory funnel and s well. The oranic phase was separated, dried over MgSO₄ and evaporated to dryness, gave bro solid. This solid was used without purification for subsequent reaction.

### Preparation 56

### Preparation of 3-(4-isopropoxyphenyl)-1-trityl-1H-indazol-5-amine

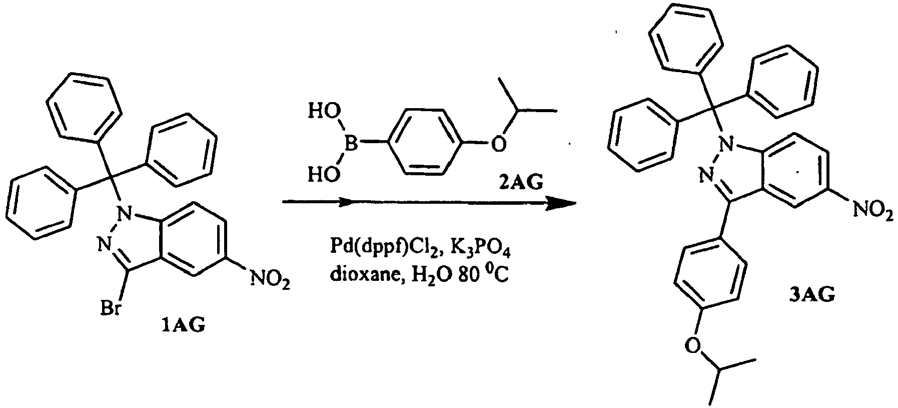

In a Sealed tube containing **1AG** (1.21 g, 2.50 mmol), **2AG** (0.67 g, 3.75 mmol), Pd(dppf)Cl₂ (0.21g, 0.25 mmol) and K₃PO₄ (1.31 g, 6.25 mmol) was added dioxane (25 mL) and water (12 mL) and degassed under nitrogen atmosphere three times. This reaction mixture was stirred at 80 °C for 18 hours. The reaction mixture was diluted with water and EtOAc, then filtered through Celite. The organic layer was washed with water, dried over MgSO₄, filtered and rotovap to dryness. The crude was chromat. (Biotage, 40L, 5%EtOAc/hexane) to give 3AG (0.68 g, 50.4%), PMR (CDCl₃)

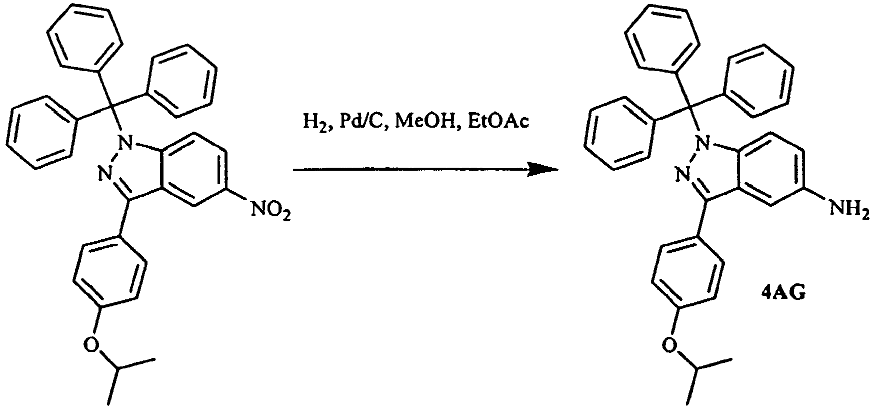

In a RB compound **3AG** (0.6 g, 1.11 mmol) stirred in a mixture of MeOH and EtOAc (10 mL each) was added 0.2 g of Pd/C (10%, 50% water). The reaction mixture was degassed under hydrogen atmosphere (balloon) and stirred overnight. MS of the reaction mixture showed (M+H) at 510. The reaction mixture was filtered (Celite) and rotovap to dryness to give a crude **4AG,** used as is for next reaction.

### Example 621

### 1-[2-(3-Methy)-5'-pyrimidm-2-yl-2,3,5,6.tetrahydro-[1,2']bipyrazinyl-4-yl)-2-oxo-ethyl]-pyrrolidine-3-carboxylic acid [3-(4-fluoro-phenyl)-1H-indazol-5-yl]-amide

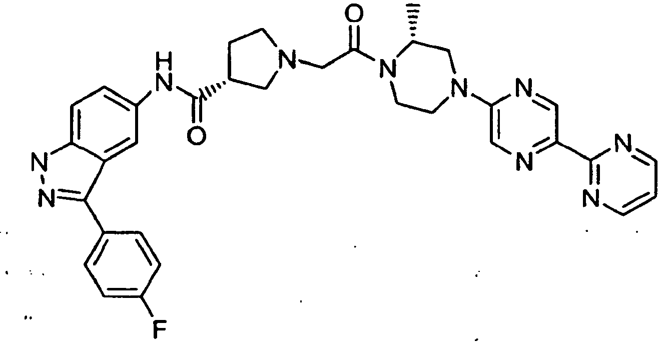

### Step 1: 5'-Iodo-3-methyl-3,4,5,6-tetrahydro-2H-[1,2']bipyrazinyl (1)

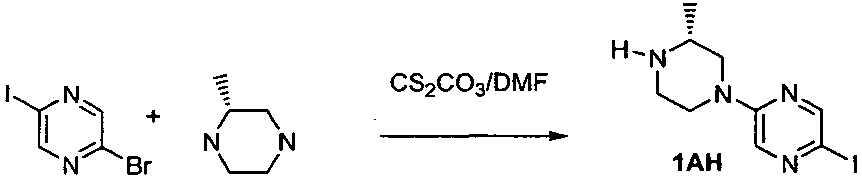

A mixture of 2-bromo-5-iodopyrazine (200mg, 0.704mmol), cesium carbonate (400mg, 1.23mmol) and 2R methyl piperazine (85mg, 0.85mmol) in DMF (10ml) was stirred at 100°C overnight. The reaction was cooled and solvent evaporated.Water (100ml) was added and insoluble solid was filtered, then dissolved in MeCl₂ (100ml)), dried over Na₂SO₄, filtered and solvent evaporated yielding product (205mg, 95%) Mass Spec (MH, 305)

### Step 2: 3-Methyl-5'-pyrimidin-2-yl-3,4,5,6-tetrahydro-2H-[1,2']bipyrazinyl (2)

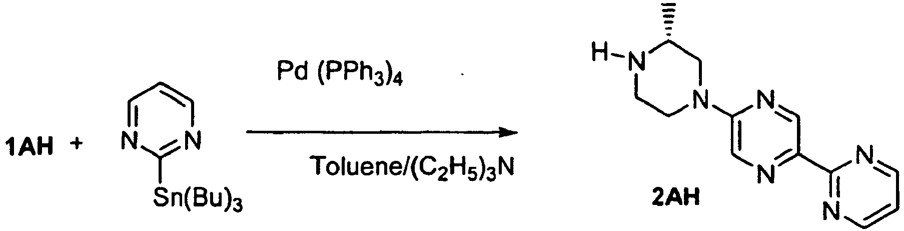

Added Pd(PPh₃)₄ (30mg,0.025mmol) to a mixture of **5'-Iodo-3-methyl-3,4,5,6-tetrahydro-2H-[1,2']bipyrazinyl (1AH)** (50mg, 0.164mmol), 2-tributyl stannyl pyrimidine (0.2ml), triethylamine (0.2ml, 1.43mmol) in toluene (3ml) at room temperature then stirred at 100°C for 5 hours. The reaction was cooled, diluted with EtOAc (50ml) and water (20ml). The organic layer was separated, dried (Na₂SO₄) filtered and solvent evaporated. The residue was purified on Prep TLC eluting with 10% MeOH:MeCl₂:NH₄OH yielding product (10mg,24%) Mass Spec MH 256

### Step 3: 1-[2-(3-Methyl-5'-pyrimidin-2-yl-2,3,5,6-tetrahydro-[1,2']bipyrazinyl-4-yl)-2-oxo-ethyl]-pyrrolidine-3-carboxylic acid [3-(4-fluoro-phenyl)-1H-indazol-5-yl]-amide

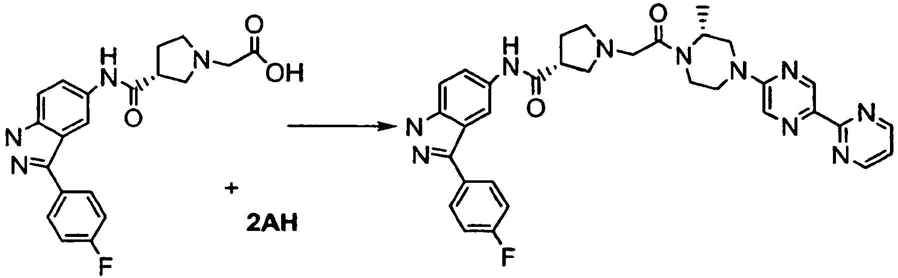

A mixture of the acid (20mg, 0.044mmol); **3-Methyl-5'-pyrimidin-2-yl-3,4,5,6-tetrahydro-2H-[1,2']bipyrazinyl (2AH)** (10mg,0.039mmol) EDCI (10mg, 0.052mmol);HOBT (7mg, 0.052mmol) and NMM (0.1 ml) was stirred in DMF (3ml) at room temperature overnight. The reaction was diluted with EtOAc (20ml) and water (10ml) and organic layer separated. The organics were washed with brine (10ml) dried over Na₂SO₄ filtered and solvent evaporated yielding a residue which purified on silica gel eluting with 10% MeOH; MeCl₂;NH₄OH yielding title product as a solid (10mg,31%) Mass Spec (MH, 621)

### Preparation 57

### 2-[6-(3-R-Methyl-piperazin-1-yl)-pyridin-3-yl]-pyrimidine

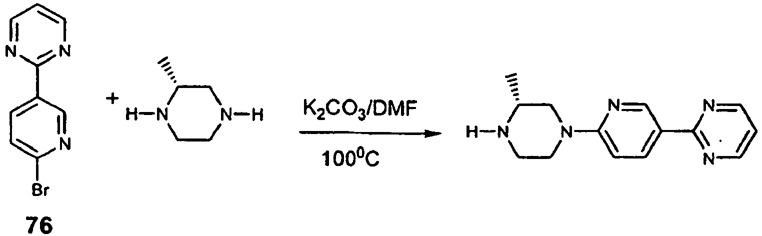

Following the procedure described in Example 98 Step 7, but substituting an equivalent quantity of 2-R-Methyl piperazine for piperazine, the title compound is obtained as a white solid (ESMS MH,256) 95% Yield.

### Preparation 58

### Step 1: Preparation of 1-N-Trityl-3-(6-Fluoro-pyridin-3-yl)-1-methyl-5-nitro-1H-indazole

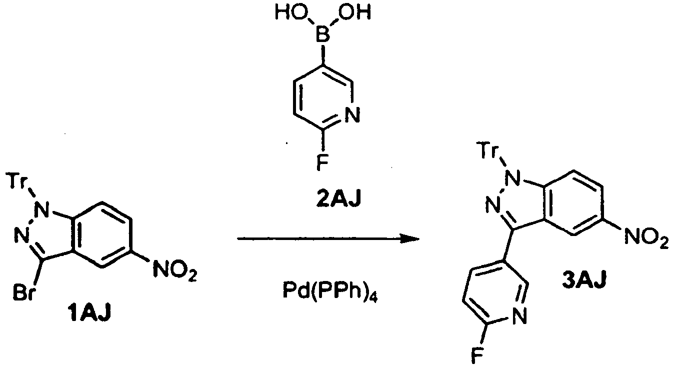

A mixture of compound **1AJ** (8 g, 16.53 mmol), compound **2AJ** (2.33 g, 16.53 mmol), Pd(PPh₃)₄ (1.9 g, 1.653 mmol) and 1:1 dioxane/Na₂CO₃ (2M) (60 ml) was degassed for 15 min. Then it was heated at 120 °C for overnight. Cooled down to room temperature and diluted with DCM (300 ml). The organic layer was washed with water (200 ml), dried over Na₂SO₄, filtered and concentrated. The residue was purified by Biotage to give the desired product **3AJ** (6 g, 58%).

### Step 2: Preparation of 1-N-Trityl-3-(6-Fluoro-pyridin-3-yl)-1-methyl-5-amino-1H-indazole

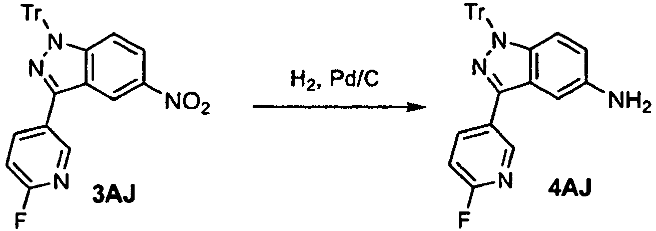

A mixture of compound **3AJ** (6 g) and Pd/C (10%, 200 mg) in EtOAc (200 ml) was stirred for overnight. Filtered and concentrated. The residue was purified by Biotage to give the desired product **4AJ** (5 g, 83%).

### Preparation 59

### Step 1: 2-Pyrazol-1-yl-pyrimidine

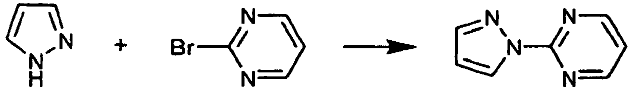

A reaction mixture containing pyrazole (2 g, 29 mmol), 2-bromopyrimidine (3.8 g, 24 mmol), copper (I) iodide (0.91 g, 4.8 mmol) and 1,10-phenanthroline (1.7 g, 9.6 mmol) in DMA was heated at 140 °C in a sealed tube for 6 hours. After the reaction, ethyl acetate (30 mL) was added, followed by water. The aqueous layer was extract three times (20 mL) and the organic layer was collected, dry over sodium sulfate. After concentration under vacuum, the crude product was purified using column chromatography (10% ethyl acetate in dichloromethane) to give 0.55 g of pure product. 15 % yield. MS (ESMS, M+H 146).

### Step 2: 2-(4-Bromo-pyrazol-1-yl)-pyrimidine

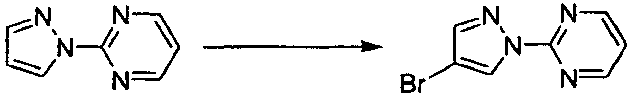

To a solution of 2-pyrazol-1-yl-pyrimidine (0.55 g, 3.7 mmol) in acetic acid (5 mL) was added bromine (1.2 g, 7.5 mmol) in acetic acid (3 mL) dropwisely. After addition, the reaction mixture was stirred at room temperature overnight. After removed the acetic acid, the crude product was purified using column chromatography (2% methanol in dichloromethane) to give 0.7 g of pure product in 85 % yield. MS (ESMS, M+H 225).

### Step 3: 2-[4-(1,2,3,6-Tetrahydro-pyridin-4-yl)-pyrazol-1-yl]-pyrimidine

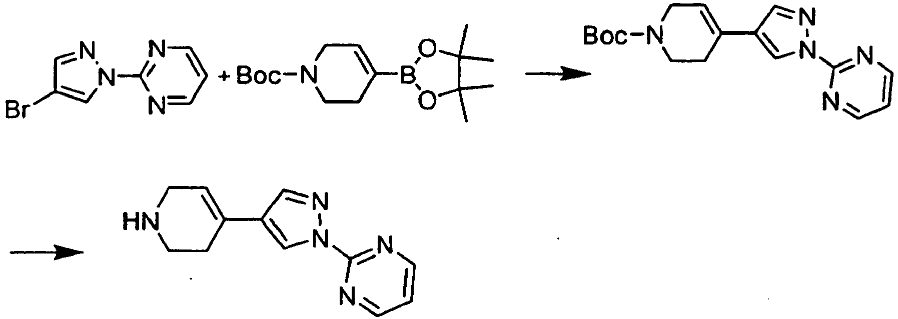

A solution containing 2-(4-bromo-pyrazol-1-yl)-pyrimidine (300 mg, 1.34 mmol), 4-(4,4,5,5-tetramethyl-[1,3,2]dioxaborolan-2-yl)-3,6-dihydro-2H-pyridine-1-carboxylic acid tert-butyl ester (280 mg, 1.34 mmol), PdCl₂(dppf) (95 mg, 0.13 mmol) and potassium phosphate (800 mg, 4 mmol) in dioxane was heated at 80 °C under argon for overnight. After removed the solvent, ethylacetate was added and the mixture was filtered, washed with water. After concentration under vacuum, it was found the product was hard to separated from impurity and the crude product was treated with 90% of TFA for 20 min and TFA was removed under vacuum. The crude product was then purified using prep HPLC to give desired product as TFA salt (120 mg, 0.37 mmol) in 27 % overall yield. MS (ESMS, M+H 228)

### Preparation 60

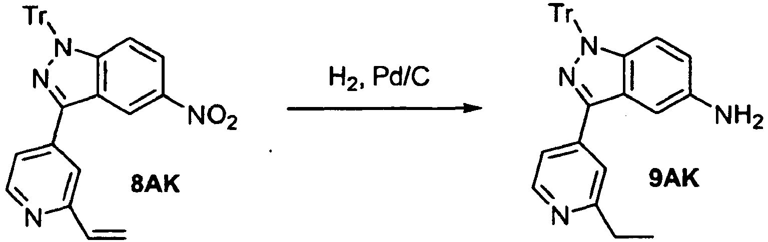

A mixture of compound **8AK** (5 g) and Pd/C (10%, 200 mg) in EtOAc (200 ml) was stirred for overnight. Filtered and concentrated. The residue was purified by Biotage to give the desired product **9AK** (4.6 g, 92%).

### Preparation 61

### Step 1

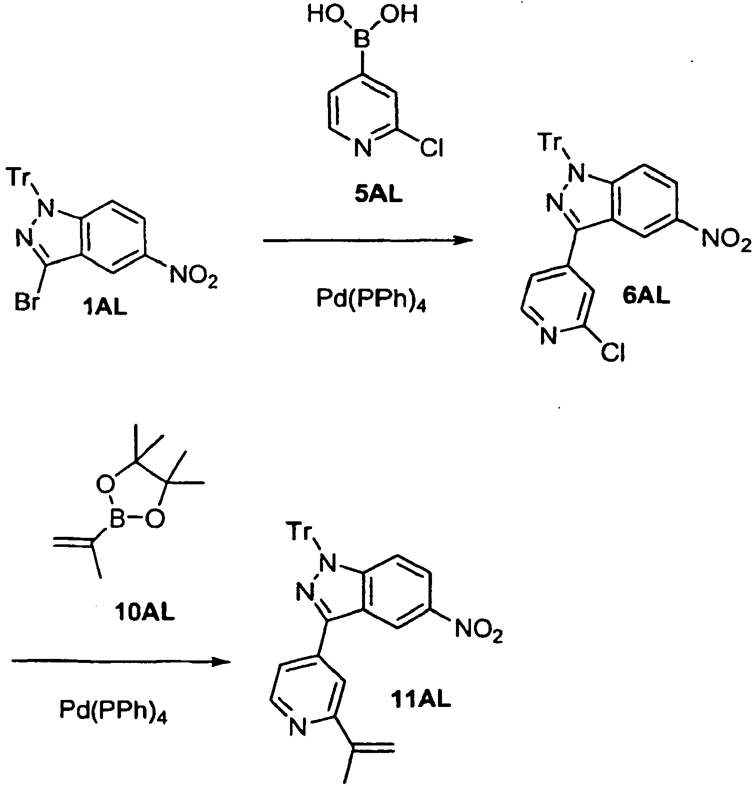

A mixture of compound **1AL** (10 g, 20.66 mmol), compound **5AL** (3.23 g, 20.66 mmol), Pd(PPh₃)₄ (2.3 g, 2 mmol) and 1:1 dioxane/Na₂CO₃ (2M) (70 ml) was degassed for 15 min. Then it was heated at 120 °C for overnight. Cooled down to room temperature and added compound **10AL** (3.47 g, 20.66 mmol), then heated at 120 °C for overnight. Cooled down to room temperature and diluted with DCM (400 ml). The organic layer was washed with water (250 ml), dried over Na₂SO₄, filtered and concentrated. The residue was purified by Biotage to give the desired product **11AL** (6 g, 50%).

### Step 2

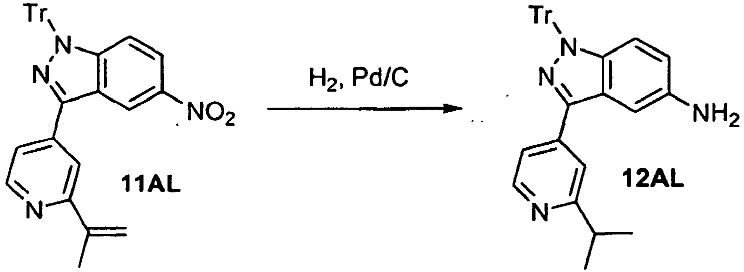

A mixture of compound **11AL** (6 g) and Pd/C (10%, 200 mg) in EtOAc (200 ml) was stirred for overnight. Filtered and concentrated. The residue was purified by Biotage to give the desired product **12AL** (5 g, 83%).

### Preparation 62

### Step 1

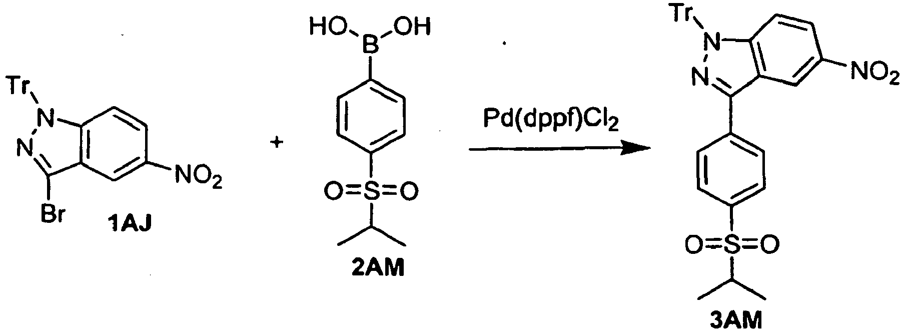

A mixture of 3-bromo-5-nitro-1-trityl-1*H*-indazole **1AJ** (0.43g, 0.9mmol), 4-(propane-2-sulfonyl)-phenyl boronic acid **2AM** (0.3 g, 1.35mmol), potassium carbonate (0.38g, 2.7mmol), Pd(dppf)Cl₂ (0.07g, 0.09mmol) and 4/1 /dioxane/water (5ml) was degassed for 5 minutes. Then it was heated at 80°C for overnight. Cooled to room temperature and diluted with EtOAc (60ml). The organic layer was washed with water (20ml), dried over MgSO₄, filtered and concentrated. The residue was purified on silica gel eluting with 1/2 EtOAc/Hexane to give the desired product **3AM** (0.4g, 76%).

### Step 2

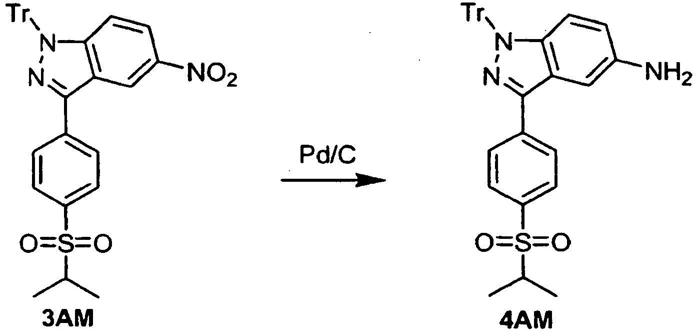

A solution of 5-nitro-3-[4-(propane -2-sulfonyl)-phenyl]-1-trityl-1*H*-indazole **3AM** (0.4g, 0.68mmol) in EtOAc (18ml) and MeOH (9ml) was degassed for 5 minutes. To this solution 10% Pd/C (38mg) was added. After stirring for overnight at 40°C, it was cooled and filtered on a celite pad. The filtrate was concentrated to give the desired product **4AM** (0.36g, 99%).

### Preparation 63

### Step 1

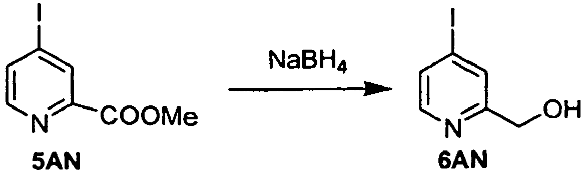

To a solution of methyl 4-iodopicolinate **5AN** (2.83g, 10.78mmol) in MeOH (30ml) was added NaBH₄ (1.59g, 43.12mmol) portion wise at 0°C. Reaction mixture was allowed to warm up to r.t. After stirring for 3 days at r.t, it was quenched with 14ml of 1 N NaOH. Reaction mixture was diluted with DCM (200ml). The organic layer was washed with water (2 x 30ml), dried over MgSO₄, filtered and concentrated. The residue was purified on silica gel eluting with 3/1 EtOAc/Hexane to give the desired product (4-iodo-pyridin-2-yl)-methanol **6AN** (2.27g, 90%).

### Step 2

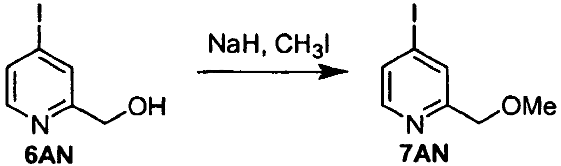

A solution of (4-iodo-pyridin-2-yl)-methanol **6AN** (0.51g, 2.16mmol) in THF (7.5ml) was added in a dry NaH (0.17g, 4.32mmol). Reaction mixture was stirred for 20 min at r.t. It was cooled to 0°C and CH₃I 0.16ml), 2.59mmol) was added drop wise. After 30 mins at 0°C, it was warmed up to r.t. and stirred further for 2 hr. Then it was diluted with EtOAc (50ml) and washed with water (2 x 10ml) and brine (1 x 15ml). Organic extracts were dried over MgSO₄, filtered and concentrated. The residue was purified on silica gel eluting with 1/2 EtOAc/Hexane to give the desired product 4-lodo-2-methoxymethyl-pyridine **7AN** (0.39, 72%).

### Step 3

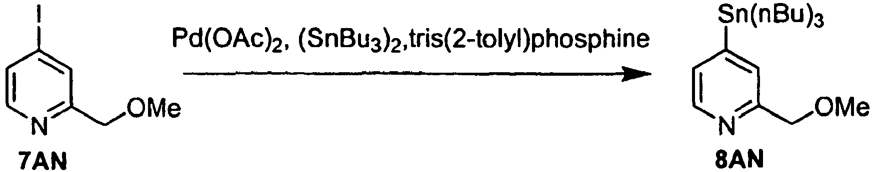

To a mixture of 4-iodo-2-methoxymethyl-pyridine **7AN** (0.22g, 0.89mmol), Pd(OAc)₂ (9mg, 0.04mmol) and tris (2-tolyl)phosphine (72mg, 0.23mmol) in ACN (7ml) was added TEA (0.27ml, 1.78mmol) and bis(tributyltin) (0.53ml, 1.07mmol). It was degassed for 5 minutes. Then it was heated at 85°C for 2hr. Cooled to room temperature and diluted with EtOAc (50ml). The organic layer was washed with water (15ml), dried over MgSO₄, filtered on a celite pad and concentrated. The residue was purified on silica gel eluting with 1/2 EtOAc/Hexane to give the desired product 2-methoxymethyl-4-tributylstannanyl-pyridine **8AN** (0.36g, 80%).

### Step 4

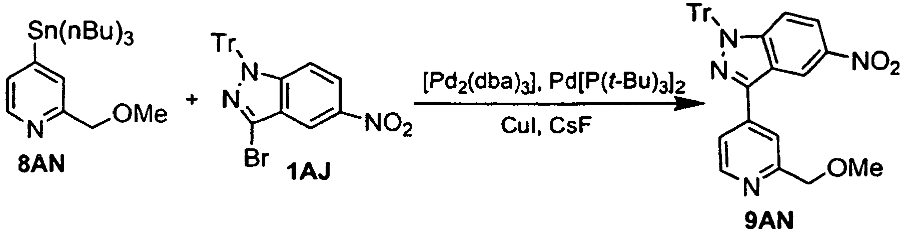

A mixture of 2-methoxymethyl-4-tributylstannanyl-pyridine **8AN** (0.1g, 0.24mmol), 3-bromo-5-nitro-1-trityl-1*H*-indazole **1AJ** (0.12g, 0.24mmol), CsF (0.08g, 0.53mmol), [Pd₂(dba)₃] (5mg, 0.005mmol), Pd[P(t-Bu)₃)₂, and Cul (4mg, 0.024mmol) in DMF (2ml) was degassed for 5 minutes. Then it was heated at 120°C for two hours. Cooled to room temperature and diluted with EtOAc (10ml). It was then filtered on a celite pad. Filtrate was washed with water (3ml) and the organic extracts were dried over MgSO₄. It was filtered again on a celite pad and concentrated. The residue was purified on silica gel eluting with 2/1 EtOAc/Hexane to give the desired product 3-(2-methoxymethyl-pyridin-4-yl)-5-nitro-1-trityl-1*H*-indazole **9AN** (0.095g, 76%).

### Step 5

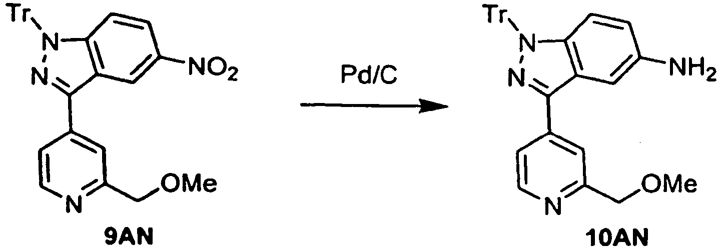

A solution of 3-(2-methoxymethyl-pyridin-4-yl)-5-nitro-1-trityl-1*H*-indazole **9AN** (0.095g 0.18mmol) in EtOAc (5ml) was degassed for 5 minutes. To this solution 10% Pd/C (20mg) was added. After stirring for overnight at r.t, it was cooled and filtered on a celite pad. The filtrate was concentrated to give the desired product 3-(2-methoxymethyl-pyridin-4-yl)-1-trityl-1*H*-indazol-5-ylamine **10AN** (0.086g, 96%).

### Preparation 64

### Step 1

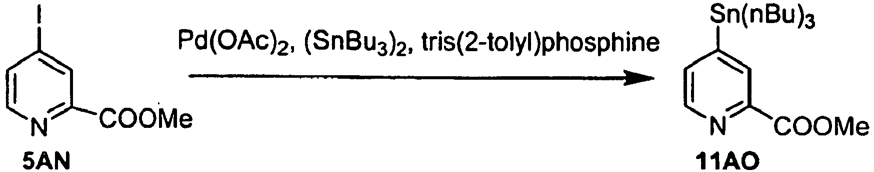

To a mixture of methyl 4-iodopicolinate **5AN** (2.6g, 9.89mmol), Pd(OAc)₂ (0.1g, 0.45mmol) and tris (2-tolyl)phosphine (0.79g, 2.58mmol) in ACN (20ml) was added TEA (2.8ml, 19.89mmol) and bis(tributyltin) (6ml, 11.93mmol). It was degassed for 5 minutes. Then it was heated at 85°C for 2hr. Cooled to room temperature and diluted with EtOAc (200ml). The organic layer was washed with water (50ml), dried over MgSO₄, filtered on a celite pad and concentrated. The residue was purified on silica gel eluting with 1/2 EtOAc/Hexane to give the desired product 4-tributylstannanyl-pyridine-2-carboxylic acid methyl ester **11AO** (3.48g, 82%).

### Step 2

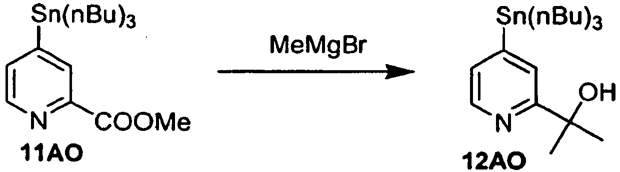

In a solution of 4-tributylstannanyl-pyridine-2-carboxylic acid methyl ester **11AO** (0.34g, 0.8mmol) in THF (7ml) at -78°C was added MeMgBr (1.15ml, 1.6mmol, 1.4M in totuene/THF) drop wise. It was stirred at -78 °C for 30 mins. Reaction mixture was warmed up to r.t. After 1.5hr, it was cooled again to 0°C and quenched with 5ml of saturated. NH₄Cl. Organic extracts were washed with water (3ml) and brine (3ml) and dried over MgSO₄. The residue was purified on silica gel eluting with 1/2 EtOAc/Hexane to give the desired product 2-(4-tributylstannanyl-pyridin-2-yl)-propan-2-ol **12AO** (012g, 77%).

### Step 3

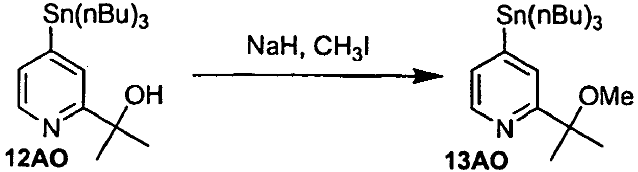

A solution of 2-(4-tributylstannanyl-pyridin-2-yl)-propan-2-ol **12AO** (0.12g, 0.27mmol) in THF (2ml) was added in a dry NaH (0.02g, 0.54mmol). Reaction mixture was stirred for 20 min at r.t. It was cooled to 0 °C and CH₃I (0.02ml, 0.32mmol) was added drop wise. After 30 mins at 0°C, reaction mixture was warmed up to r.t. It was diluted with EtOAc (15ml) after 2 hr and washed with water (2 x 3ml) and brine (5ml). Organic extracts were dried over MgSO₄, filtered and concentrated. The residue was purified on silica gel eluting with 1/4 EtOAc/Hexane to give the desired product 2-(1-methoxy-1-methyl-ethyl)-4-tributylstannanyl-pyridine.**13AO** (0.1g, 88%).

### Step 4

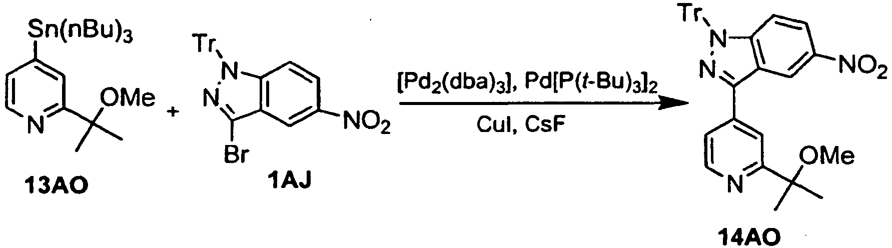

2-(1-methoxy-1-methyl-ethyl)-4-tributylstannanyl-pyridine **13AO** (0.1g,, 0.24mmol), 3-bromo-5-nitro-1-trityl-1*H*-indazole **1AJ** (0.12g, 0.24mmol), CsF (0.08g, 0.53mmol), [Pd₂(dba)₃] (5mg, 0.005mmol), Pd[P(*t*-Bu)₃)₂, and CuI (4mg, 0.024mmol) in DMF (2ml) was degassed for 5 minutes. Then it was heated at 120°C for two hours. Cooled to room temperature and diluted with EtOAc (10ml). It was then filtered on a celite pad. Filtrate was washed with water (3ml) and the organic extracts were dried over MgSO₄. It was filtered again on a celite pad and concentrated. The residue was purified on silica gel eluting with 1/3 EtOAc/Hexane to give the desired product 3-[2-(1-methoxy-1-methyl-ethyl)-pyridin-4-yl]-5-nitro-1-trityl-1*H*-indazole **14AO** (0.05g, 39%).

### Step 5

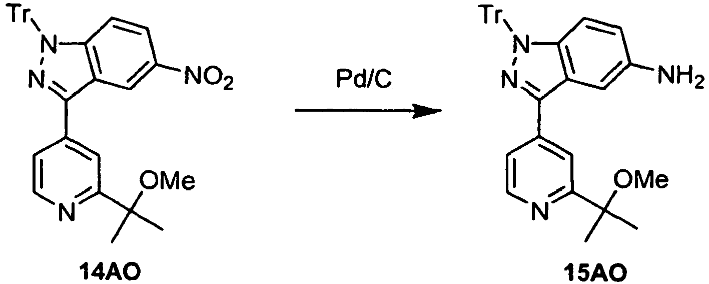

A solution of 33-[2-(1-methoxy-1-methyl-ethyl)-pyridin-4-yl]-5-nitro-1-trityl-1*H-*indazole **14AO** (0.05g, 0.09mmol) in EtOAc (5ml) was degassed for 5 minutes. To this solution 10% Pd/C (10mg) was added. After stirring for overnight at r.t, it was cooled and filtered on a celite pad. The filtrate was concentrated to give the desired product 3-[2-(1-methoxy-1-methyl-ethyl)-pyridin-4-yl]-1-trityl-1*H*-indazole-5-ylamine **15AO** (0.046g, 97%).

### Procedure 65

### Step 1

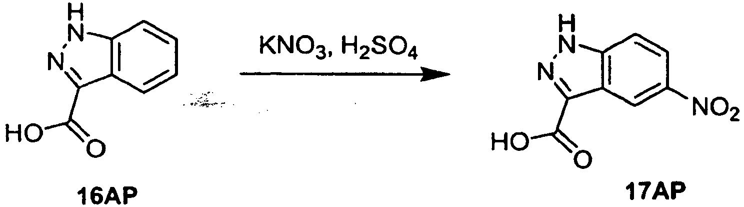

To a suspension of indazole-3-carboxylic acid **16AP** (10.5g, 64.8mmol) in concentrated sulfuric acid (125ml) at 0°C was added KNO₃ (64.8mmol, 6.55g). The reaction mixture was warmed up to r.t. After stirring for 4hr at r.t, it was poured into a 525ml of ice/water. Solid was collected by filtration and washed with water to give desired 5-nitro-1*H*-indazole-3-carboxylic acid **17AP** (10.74g, 80%).

### Step 2

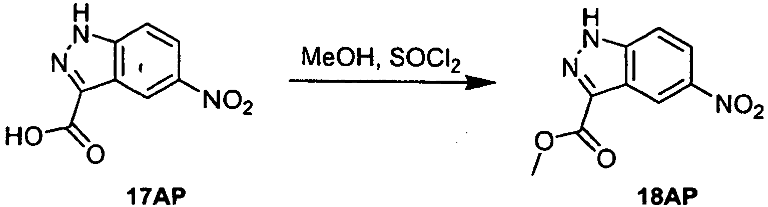

To a suspension of 5-nitro-1*H*-indazole-3-carboxylic acid **17AP** (10.74g, 51.88mmol) in MeOH (145ml) at 0°C was added SOCl₂ (35ml) dropwise. After stirring for 10 min at 0°C, the reaction mixture was refluxed overnight. NCl gas was evolved (Condenser was equipped with empty balloon to trap HCl). It was then cooled to room temperature, solid was collected by filtration and washed with MeOH to give desired 5-nitro-1*H*-indazole-3-carboxylic acid methyl ester **18AP** (7g, 61%).

### Step 3

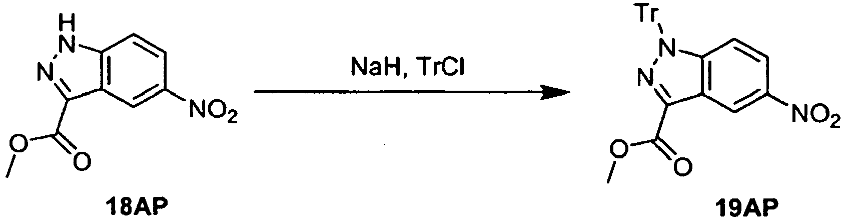

Suspension of 5-nitro-1*H*-indazole-3-carboxylic acid methyl ester **18AP** (7g, 31.67mmol) in THF (130ml) and DCM (15ml) was added to a NaH (60% in Mineral Oil, 1.61g, 41.17mmol) at 0°C. H₂ gas was evolved. Chlorotriphenyl methane (8.83g, 31.67mmol) was added after stirring for 20 min at 0°C. Reaction mixture was subjected to heat at 60°C for overnight. It was then cooled to room temperature and concentrated under reduced pressure. Solid was redissolved in DCM and filtered. Filtrate was concentrated under reduced pressure to give desired 5-nitro-1-trityl-1*H-*indazole-3-carboxylic acid methyl ester **19AP** (12.95g, 88%).

### Step 4

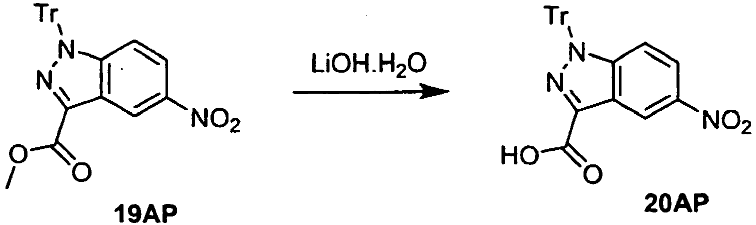

To a solution of 5-nitro-1-trityl-1*H*-indazole-3-carboxylic acid methyl ester **19AP** (12.95g, 27.96mmol) in THF (270ml) and H₂O (135ml) was added LiOH·H₂O (2.34g, 55.92mmol) at room temperature. After stirring for overnight at room temperature, it was cooled to 0°C and acidified with slow addition of 1 N HCl (55.9ml, 55.92mmol). Reaction mixture was concentrated under reduced pressure. Solid was collected by filtration and washed with water to give desired 5-nitro-1-trityl-1*H*-indazole-3-carboxylic acid **20AP** (12.06g, 96%).

### Step 5

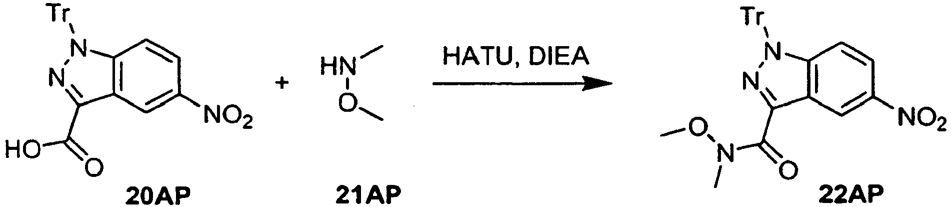

5-nitro-1-trityl-1*H*-indazole-3-carboxylic acid **20AP** (12.06g, 26.85mmol) and HATU (10.2g, 26.85mmol) was dissolved in DMF (100ml). After stirring for 10 min at room temperature, *N,O*-dimethylhydroxyl amine hydrochloride **21AP** (2.62g, 26.85mmol) and DIEA (14.1 ml, 80.55mmol) was added. It was stirred further for 1 hr at room temperature. Reaction mixture was poured into 300ml of water. Solid was collected by filtration. This solid was redissolved in EtOAc (300ml) and 75ml of sat. NaHCO₃ was added. Organic fraction was washed with water (2 X 75ml), brine (75ml) and dried over MgSO₄. Solvent was removed under reduced pressure to give desired 5-nitro-1-trityl-1*H*-indazole-3-carboxylic acid methoxy-methyl-amide **22AP** (12.56g, 95%).

### Step 6

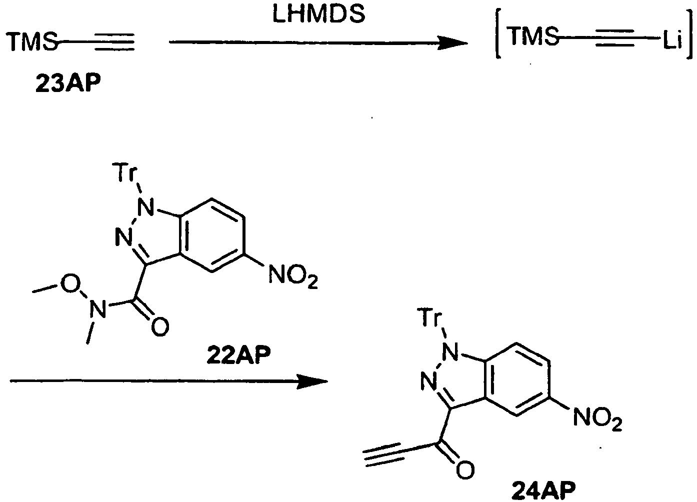

To a cold (-20°C) solution of TMS-acetylene **23AP** (5.5ml, 38.28mmol) in toluene (23ml) was added LHMDS (33.2ml, 1 M in THF, 33.2mmol) dropwise over 5 min keeping T < -5°C. The mixture was stirred at 0°C for 30 min, then transferred to the cold (-10°C) reaction mixture that contains 5-nitro-1-trityl-1*H*-indazole-3-carboxylic acid methoxy-methyl-amide **22AP** (12.56g, 25.52mmol) in THF (120ml) at a rate that kept T < -5°C. The resulting mixture was stirred for 20 min at -5°C then 1.5hr at room temperature. Reaction mixture was cooled to 0°C and quenched by a slow addition of 1 N HCl (40ml) under N₂. It was stirred further for 5 min. Reaction mixture was diluted with EtOAc (500ml). The organic fraction was separated and washed with water (2 x 100ml) and brine (1 x 125ml). It was dried over MgSO₄, filtered and concentrated. The residue was purified on silica gel eluting with 2/1 EtOAc/Hexane to give the desired 1-(5-nitro-1-trityl-1*H*-indazol-3-yl-)-propynone **24AP** (6.99g, 60%).

### Step 7

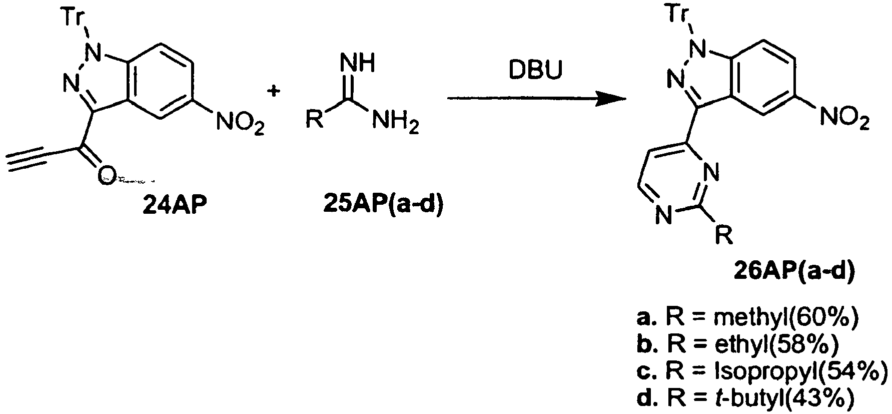

A mixture of 1-(5-nitro-1-trityl-1*H*-indazol-3-yl-)-propynone **24AP** (0.5g, 1.09mmol), amidine hydrochloride **25AP** (1.75mmol) and DBU (1.6ml), 10.9mmol) in acetonitrile (10ml) was heated at 120°C for 50 minutes under microwave. It was then cooled to room temperature and diluted with DCM (60ml). The organic layer was washed with water (10ml), dried over MgSO₄, filtered and concentrated. The residue was purified on silica gel eluting with 1/1 EtOAc/Hexane to give the desired product **26AP.**

### Step 8

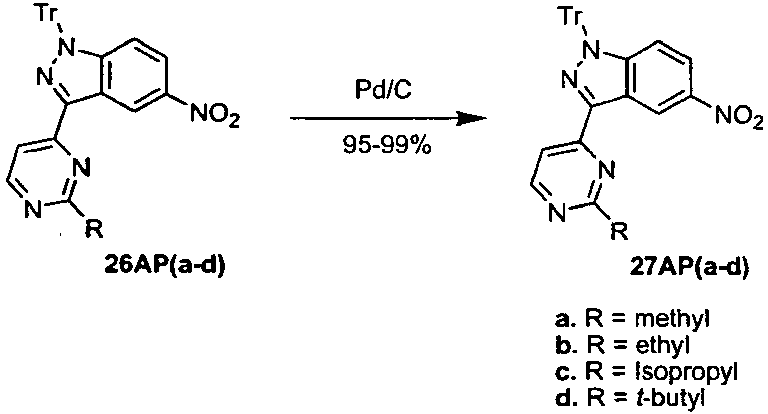

A solution of **26AP** (0.96mmol) in EtOAc (20ml) and MeOH (5ml) was degassed for 5 minutes. To this solution 10% Pd/C (0.08g) was added. After stirring for overnight at 40°C, it was cooled and filtered on a celite pad. The filtrate was concentrated to give the desired **27AP.**

### Preparation 66

### Preparation of 4-(2,5-Difluoro-4-pyrimidin-2-yl-phenyl)-3,6-dihydro-2H-pyridine-1-carboxylic acid tert-butyl ester (Compound 12AQ)

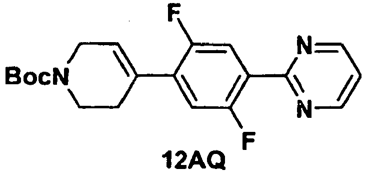

The Compound **12AQ** was prepared from 4-(4,4,5,5-tetramethyl-[1,3,2]dioxaborolan-2-yl)-3,6-dihydro-2*H*-pyridine-1-carboxylic acid *tert*-butyl ester using the procedure as described for the preparation of Compound 4-(2-fluoro-4-pyrimidi-2-yl-phenyl)-3,6-dihydro-2*H*-pyridine-1-carboxylic acid *tert*-butyl ester but using 1,4-dibromo-2,5-difluoro-benzene in place of 4-bromo-2-fluoro-1-iodobenzene.

### Example 622

### Preparation of 1-(2-{4-[4-(5-Ethylamino-[1,3,4]oxadiazol-2-yl)-2-fluoro-phenyl]-3,6-dihydro-2H-pyridin-1-yl}-2-oxo-ethyl)-3-methoxy-pyrrolidine-3-carboxylic acid [3-(4-fluoro-phenyl)-1H-indazol-5-yl]-amide

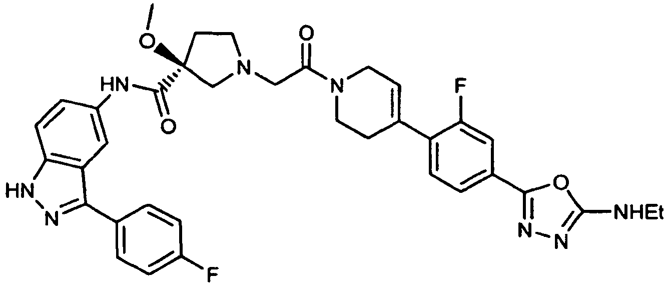

### Example 622

### Step 1: Preparation of 4-Bromo-3-fluoro-benzoic acid hydrazide

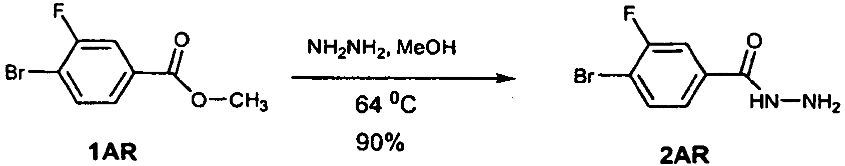

A mixture of compound 4-bromo-3-fluoro-benzoic acid methyl ester (1 g, 4.29 mmol), hydrazine hydrate (2.2 mL, 42.9 mmol) and MeOH (20 mL) was heated at 70 °C for overnight. Concentrated, diluted with EtOAc (300 mL) and washed with water (100 mL). The organic layer was dried over Na₂SO₄, filtered and concentrated give the desired product **2AR** (0.9 g, 90%).

### Step 2: Preparation of [5-(4-Bromo-3-fluoro-phenyl)-[1,3,4]oxadiazol-2-yl]-ethyl-amine

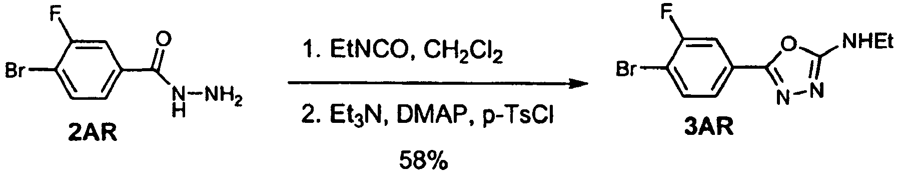

A mixture of Compound **2AR** (0.9 g, 2.53 mmol), CH₂Cl₂ (5 mL) and ethyl isocyanate (0.34 mL, 4.35 mmol) was stirred at room temperature for 3 hours. To the reaction mixture was added triethylamine (0.94 mL, 6.7 mmol), DMAP (0.205 g, 1.675 mmol) and a solution of p-toluenesulfonyl chloride (0.83 g, 4.36 mmol) in CH₂Cl₂ (10 mL). Reaction mixture was stirred at room temperature for 18 hours. Diluted with CH₂Cl₂ (200 mL) and washed with water (100 mL). The organic layer was dried over Na₂SO₄, filtered and concentrated. The residue was purified on silica gel eluting with 3% MeOH/ CH₂Cl₂ to give the desired product **3AR** (0.56 g, 58%).

### Step 3: Preparation of 4-[4-(5-Ethylamino-[1,3,4]oxadiazol-2-yl)-2-fluoro-phenyl]-3,6-dihydro-2H-pyridine-1-carboxylic acid tert-butyl ester

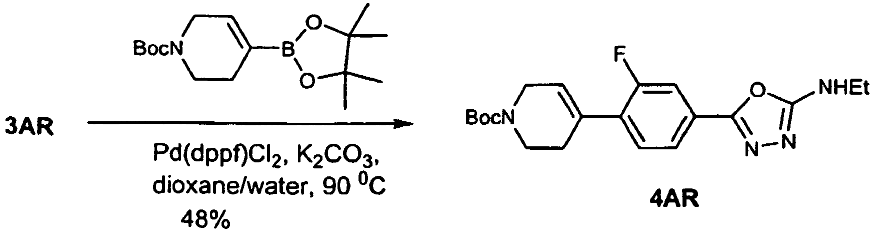

A mixture of Compound **3AR** (0.56 g, 1.96 mmol), 4-(4,4,5,5-tetramethyl-[1,3,2]dioxaborolan-2-yl)-3,6-dihydro-2*H*-pyridine-1-carboxylic acid *tert*-butyl ester (0.73 g, 2.35 mmol), potassium carbonate (0.81 g, 5.88 mmol), Pd(dppf)Cl₂ (0.192 g, 0.235 mmol) and 4/1 /dioxane/water (10 ml) was degassed for 15 minutes. Then it was heated at 80 °C for overnight. Cooled to room temperature and diluted with EtOAc (200 ml). The organic layer was washed with water (100 ml), dried over Na₂SO₄, filtered and concentrated. The residue was purified on silica gel eluting with 5% MeOH/CH₂Cl₂ to give the desired product **4AR** (0.44 g, 48%).

### Step 4: Preparation of Ethyl-{5-[3-fluoro-4-(1,2,3,6-tetrahydro-pyridin-4-yl]-[1,3,4]oxadiazol-2yl}-amine

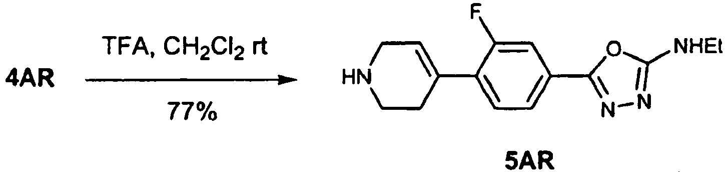

A mixture of Compound **4AR** (0.44 g, 1.13 mmol), CH₂Cl₂ (20 mL) and TFA (2 mL) was stirred at room temperature for 18 hours. Concentrated and purified on silica gel eluting with 5% MeOH (NH₃)/ CH₂Cl₂ to give the desired product **5AR** (0.25 g, 77%).

### Step 5: Preparation of 2-Chloro-1-{4-[4-(5-ethylamino-[1,3,4]oxadiazol-2-yl)-2-fluoro-phenyl]-3,6-dihydro-2H-pyridin-1-yl}-ethanone

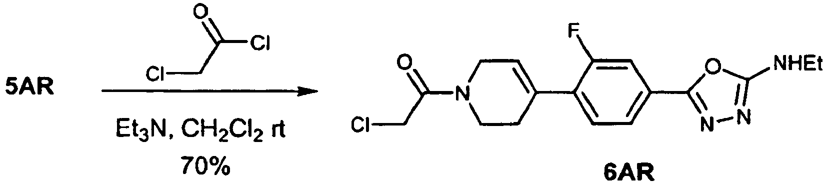

To a mixture of Compound **5AR** (0.1 g, 0.35 mmol), CH₂Cl₂ (5 mL), MeOH (1 mL) and triethyl amine (0.041 mL, 0.29 mmol) at -78 °C was added chloroacetyl chloride (0.021 mL, 0.264 mmol). Reaction mixture was stirred at -78 °C for 10 minutes then warm to 0 °C and stirred for 1 hour. Diluted with CH₂Cl₂ (100 mL) and washed with saturated aq. NaHCO₃ (100 mL). The organic layer was dried over Na₂SO₄, filtered and concentrated. The residue was purified on silica gel eluting with 2% MeOH/ CH₂Cl₂ to give the desired product **6AR** (0.09 g, 70%).

### Step 6

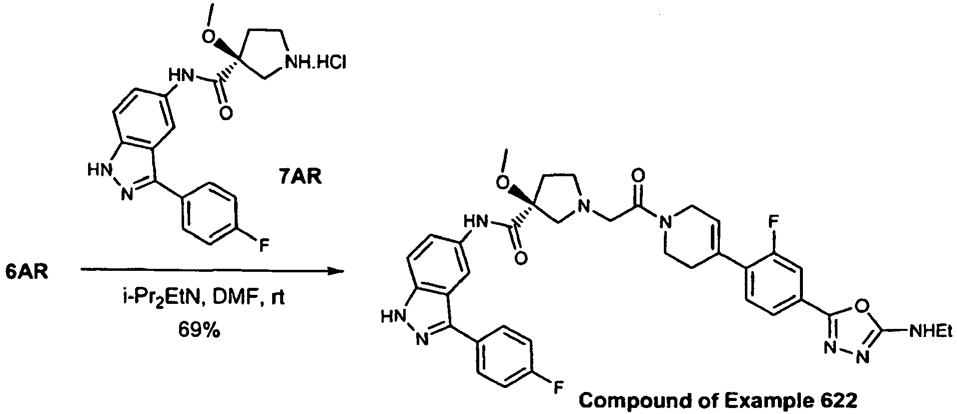

A mixture of Compound **6AR** (0.09 g, 0.247 mmol), compound **7AR** (0.073 g, 0.2 mmol), DMF (2 mL) and N,N-diisopropylethylamine (0.131 mL, 0.74 mmol) was stirred at room temperature for 18 hours. Diluted with EtOAc (100 mL) and washed with water (2 x 100 mL). The organic layer was dried over Na₂SO₄, filtered and concentrated. The residue was purified on silica gel eluting with 5% MeOH (NH₃)/ CH₂Cl₂ to give the desired compound of **Example 622** (0.11 g, 69%).

### Preparation 67

### Step 1

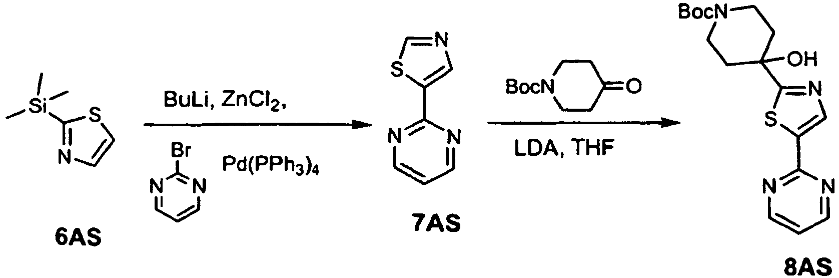

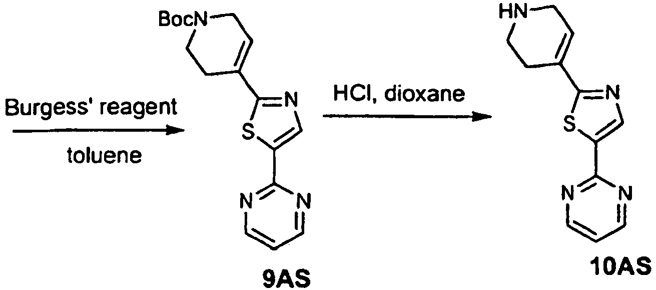

In a 250 round bottom flask was placed butyl lithium (6.1 mL, 2.5 M in hexanes, 15.2 mmol) in THF at -78 °C under Ar. To this was added 6 (2.0 g, 12.7 mmol), stirred for 15 min and added Zinc chloride (38.1 mL, 0.5 M in THF, 19.1 mmol). The mixture was warmed up to room temperature and stirred for 1 hr. To this was added 2-bromopyrimidine (2.4 g, 15.2 mmol) and Pd(PPh₃)₄ (293 mg, 0.252 mmol). The reaction was heated to reflux overnight, cooled to room temperature and filtered. The filtrate was partitioned between brine and ethyl acetate. The organic layer was dried over sodium sulfate and concentrated. The resulting mixture was purified by biotage column chromatography to afford **7AS** (936 mg, 54.2%)

To a solution of **7AS** (710 mg, 4.35 mmol) in THF at -78 °C was added LDA (2.61 mL, 2.0 M, 5.22 mmol), and then Boc-4-piperidone (1.04 g, 5.22 mmol). The reaction was stirred at -78 °C for 1 hr, warmed up to room temperature and quenched with ammonium chloride solution. The mixture was extracted with ethyl acetate. The combined organic layer was dried over sodium sulfate and concentrated. The resulting oil was purified by biotage column chromatography to afford **8AS** (1.08 g, 68%)

To a solution of **8AS** (800 mg, 2.21 mmol) in toluene was added Burgess reagent (1.09 g, 4.58 mmol). The mixture was heated to 100 °C, stirred for 2 hrs, cooled to room temperature and concentrated. The residue was purified biotage column chromatography to afford **9AS** (562 mg, 74%)

### Step 2

To **9AS** (560 mg, 1.63 mmol) in a 20 mL vial was added 4 mL of HCl in dioxane (4 M). The reaction was stirred at room temperature for 4 hrs and the precipitate was filtered. The resulting solid was pump dried to afford **10** (350 mg, 88%)

### Step 3

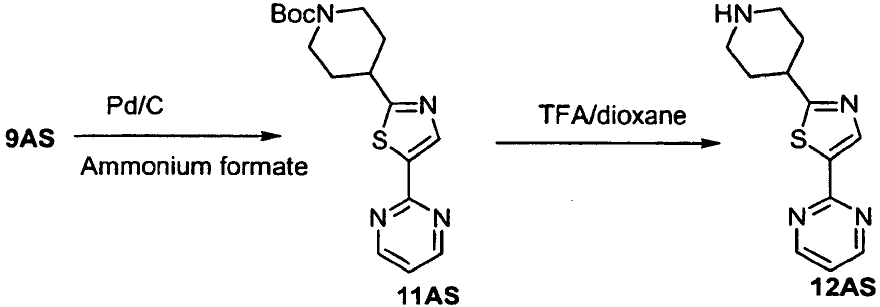

To a solution of **9AS** (100 mg, 0.291 mmol) and ammonium formate (183 mg, 2.91 mmol) in methanol was added catalytic amount of 10 % Palladium on carbon. The mixture was heated to reflux overnight, cooled to room temperature and filtrated. The filtrate was concentrated and the residue was purified biotage column chromatography to afford **11AS** (52 mg, 52%) and recovered 9 (18 mg, 18%)

To **11 AS** (52 mg, 0.15 mmol) in 1 mL of DCM was added 1 mL of TFA. The reaction was stirred at room temperature for 2 hrs and concentrated to afford crude **12AS**.

### Preparation 68

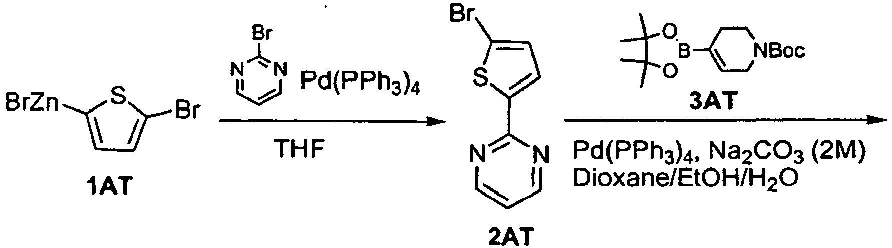

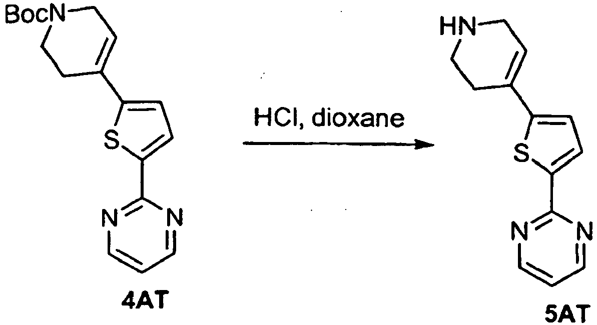

In a 250 mL of round bottom flask was placed **1AT** (0.5 M in THF, 20.0 mL, 10.0 mol). To this was added 2-bromopyrimidine (2.00 g, 12.6 mol) and Pd(PPh₃)₄ (346 mg, 0.3 mmol). The mixture was heated to reflux under Ar overnight and cooled down to room temperature. The reaction was quenched with ammonium chloride solution and extracted with ethyl acetate. The combined organic layer was dried over sodium sulfate and concentrated. The resulting oil was purified by biotage column chromatography to afford **2AT** (1.62 g, 69%)

In a 5 mL of biotage microwave vessel was placed **2AT** (136 mg, 0.568 mmol), pinacol ester **3AT** (193 mg, 0.625 mmol), Pd(PPh₃)₄ (32.8 mg, 0.0284 mmol) and sodium carbonate solution (0.85 mL, 2 M) in dioxane/EtOH/H2O (7:3:2, 2.5 mL) under Ar. The vessel was sealed and heated in microwave reactor at 150 °C for 10 minutes. The reaction was partitioned between ethyl acetate and water. The organic layer was dried over sodium sulfate and concentrated. The resulting oil was purified by biotage column chromatography to afford **4AT** (149 mg, 76%).

To **4AT** (144 mg, 0.420 mmol) in a 20 mL vial was added 2 mL of HCl in dioxane (4 M). The reaction was stirred at room temperature for 4 hrs and the precipitate was filtered. The resulting solid was pump dried to afford **5AT** (83 mg, 82%)

### Preparation 69

### Step 1: Preparation of 3-(4-Fluoro-3-hydroxyphenyl)-5-nitro-1-trityl-1H-indazole

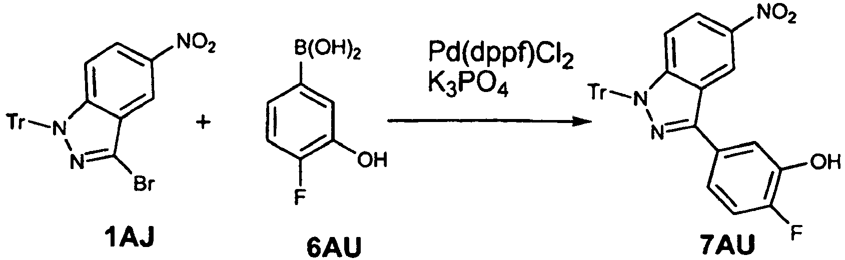

A suspension of 3-bromo-5-nitro-1-trityl-1H-indazole **1AJ** (10.0 g, 20.68 mmol), 4-fluoro-3-hydroxyphenylboronic acid **6AU** (3.55 g, 22.78 mmol), Pd(dppf)Cl₂ (1.6 mg, 1.96 mmol) and K₃PO₄ (10.6 g, 4.99 mmol) in 200 mL of dioxane/H₂O (4/1) was heated to 75°C for overnight. After removal of most of the solvent, the black residue was diluted with EtOAc (250 mL) and H2O (60 mL). The resulting mixture was filtered through a pad of Celite. Additional 150 mL of EtOAc was used to wash the Celite cake. The filtrates were combined. The aqueous layer was separated and the organic layer was washed with brine, dried (MgSO₄) and concentrated to give a green solid, which was recrystallized from CH₂Cl₂/hexane to give 3-(4-fluoro-3-hydroxyphenyl)-5-nitro-1-trityl-1H-indazole **7AU** (8.1 g).

### Step 2: Preparation of 3-[4-Fluoro-3-(2-methoxy-ethoxy)-phenyl]-1-trityl-5-nitro-1H-indazole

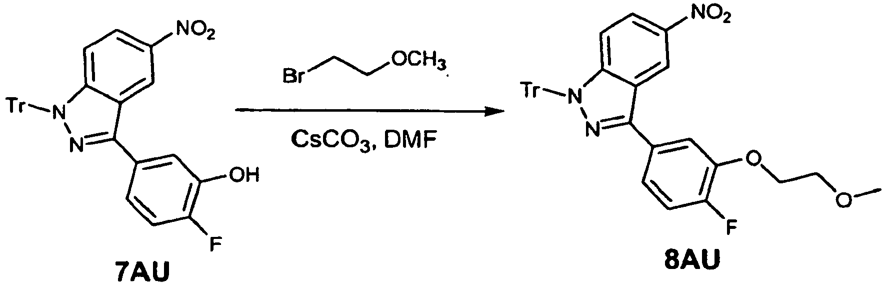

To a stirred mixture of 3-(4-fluoro-3-hydroxyphenyl)-5-nitro-1-trityl-1H-indazole **7AU** (2 g, 3.88 mmol) in DMF (25 mL) was added CsCO₃ 1.9 g, 5.82 mmol) and bromoethylmethyl ether (474 µL, 5.04 mmol). The resulting mixture was stirred at rt for overnight then diluted with EtOAc (300 mL), which was washed with brine, dried (MgSO₄) and concentrated. Chromatograph on silica gel (2:1, hexanes/EtOAc) gave 3-[4-fluoro-3-(2-methoxy-ethoxy)-phenyl]-1-trityl-5-nitro-1H-indazole **8AU** (1.8 g) as a yellow solid.

### Step 3: Preparation of 3-[4-Fluoro-3-(2-methoxy-ethoxy)-phenyl]-1-trityl-1H-indazol-5-ylamine

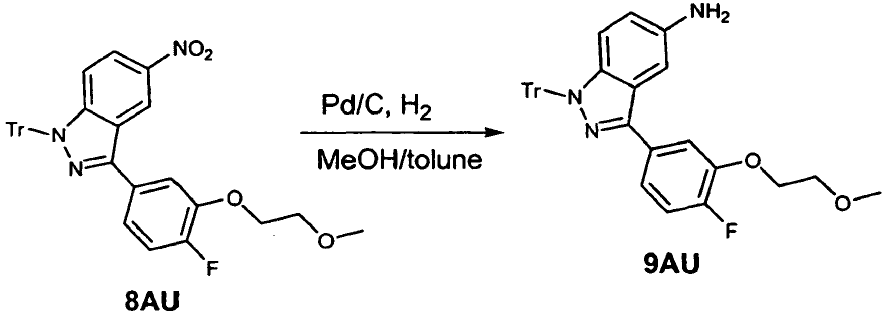

A suspension of **8AU** (890 mg, 1.55 mmol) and catalytic amount of Pd on carbon (5 wt%, 180 mg) in 45 mL of methanol/tolune/CH₂Cl₂ (4:4:1) was stirred under a hydrogen atmosphere for 2 d and filtered through Celite. The filtrate was concentrated and purified on silica gel column (1:1, hexanes/EtOAc) provide 3-[4-fluoro-3-(2-methoxy-ethoxy)-phenyl]-1-trityl-1H-indazol-5-ylamine 9AU (680 mg) as a white solid.

### Preparation 70

### Step 1: Preparation of 3-(4-Fluoro-3-hydroxymethylphenyl)-5-nitro-1-trityl-1H-indazole

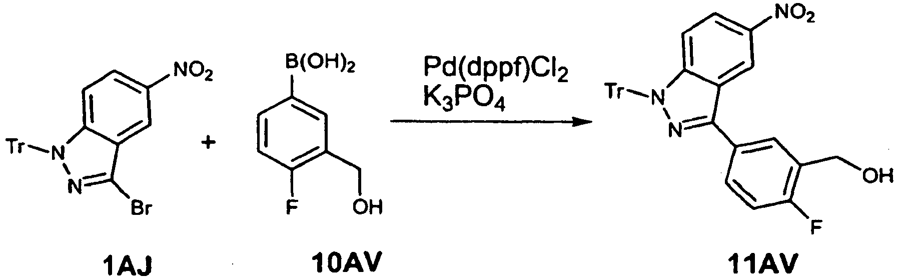

A suspension of 3-bromo-5-nitro-1-trityl-1 H-indazole 1 (6 g, 12.41 mmol), 4-fluoro-3-hydroxymethylphenylboronic acid **10AV** (2 g, 12.87 mmol), Pd(dppf)Cl₂ (800 mg, 0.98 mmol) and K₃PO₄ (6 g, 28.30 mmol) in 124 mL of dioxane/H₂O (25:6) was heated to 80°C for overnight. After removal of most of the solvent, the black residue was diluted with EtOAc (250 mL) and H₂O (60 mL). The resulting mixture was filtered through a pad of Celite. Additional 150 mL of EtOAc was used to wash the Celite cake. The filtrates were combined. The aqueous layer was separated and the organic layer was washed with brine, dried (MgSO₄) and concentrated to give crude 3-(4-fluoro-3-hydroxymethylphenyl)-5-nitro-1-trityl-1 H-indazole **11AV** (8.2 g) as a greenish yellow solid.

### Step 2: Preparation of 3-[4-Fluoro-3-(2-methoxymethyl)-phenyl]-1-trityl-5-nitro-1H-indazole

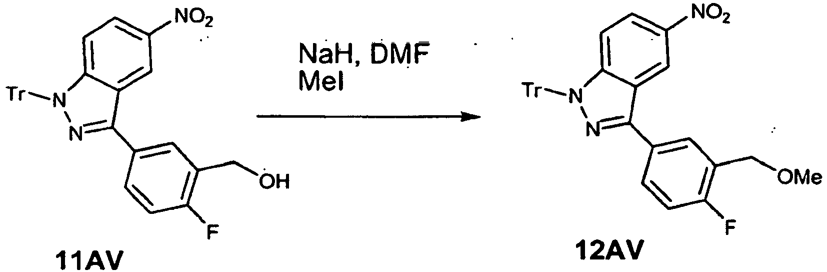

To a stirred solution of 3-(4-fluoro-3-hydroxymethylphenyl)-5-nitro-1-trityl-1H-indazole **11AV** (5.5 g, 10.4 mmol) in DMF (120 mL) at 0°C under N₂ was added NaH (60% in mineral oil, 500 mg, 12.5 mmol) in portions over 10 min. After 15 min, Mel (1 mL, 16.06 mmol) was added dropwise. The reaction mixture was stirred at 0°C to rt for overnight and quenched with H₂O. The resulting mixture was extracted with EtOAc, washed with brine, dried (MgSO₄) and concentrated to give the crude 3-[4-fluoro-3-(2-methoxymethyl)-phenyl]-1-trityl-5-nitro-1 H-indazole **12AV** (7.2 g) as a yellow solid.

### Step 3: Preparation of 3-[4-Fluoro-3-(2-methoxymethyl)-phenyl]-1-trityl-1H-indazol-5-ylamine

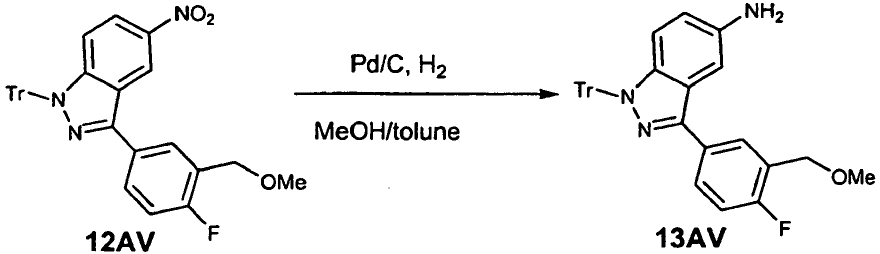

A suspension of the crude 3-[4-fluoro-3-(2-methoxymethyl)-phenyl]-1-trityl-5-nitro-1H-indazole **12AV** (7.2 g) from step 2 and catalytic amount of Pd on carbon (5 wt%, 1.3 g) in 150 mL of methanol/toluene (1:1) was stirred under a hydrogen atmosphere for overnight and filtered through Celite. The filtrate was concentrated and purified on silica gel column (4:1, hexanes/EtOAc) provide 3-[4-fluoro-3-(2-methoxymethyl)-phenyl]-1-trityl-1H-indazol-5-ylamine **13AV** (5 g) as a white solid.

### Preparation 71

### Step 1: Preparation of 3-(3-Fluoro-4-hydroxyphenyl)-5-nitro-1-trityl-1H-indazole

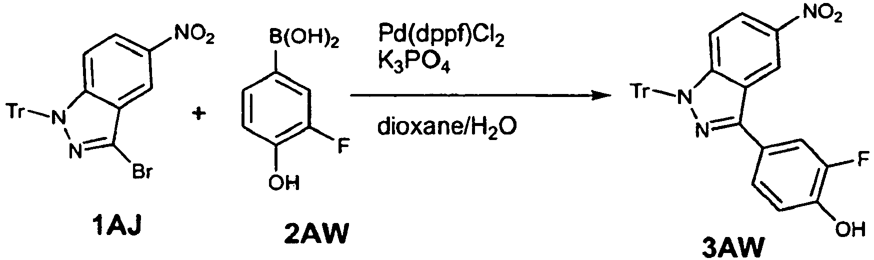

A suspension of 3-bromo-5-nitro-1-trityl-1 H-indazole **1AJ** (10.0 g, 20.68 mmol), 3-fluoro-4-hydroxyphenylboronic acid **2AW** (3.55 g, 22.78 mmol), Pd(dppf)Cl₂ (1.6 g, 1.96 mmol) and K₃PO₄ (10.6 g, 4.99 mmol) in 200 mL of dioxane/H₂O (4/1) was heated to 75°C for overnight. After removal of most of the solvent, the black residue was diluted with EtOAc (250 mL) and H₂O (60 mL). The resulting mixture was filtered through a pad of Celite. Additional 150 mL of EtOAc was used to wash the Celite cake. The filtrates were combined. The aqueous layer was separated and the organic layer was washed with brine, dried (MgSO₄) and concentrated to give a green solid, which was recrystalized from CH₂Cl₂/hexane to give 3-(3-fluoro-4-hydroxyphenyl)-5-nitro-1-trityl-1H-indazole **3AW** (8.1 g).

### Step 2: Preparation of 3-[3-Fluoro-4-(2-methoxy-ethoxy)-phenyl]-1-trityl-5-nitro-1H-indazole

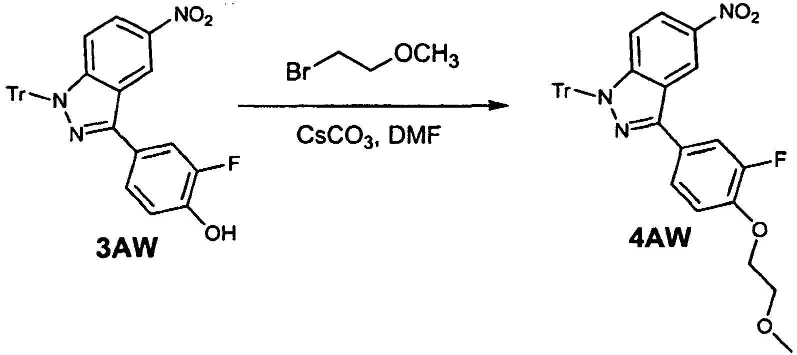

To a stirred mixture of 3-(3-fluoro-4-hydroxyphenyl)-5-nitro-1-trityl-1H-indazole 3AW (3 g, 5.82 mmol) in DMF (40 mL) was added CsCO₃ (2.85 g, 8.75 mmol) and bromoethylmethyl ether (711 µL, 7.57 mmol). The resulting mixture was stirred at rt for overnight then diluted with EtOAc (300 mL), which was washed with brine, dried (MgSO₄) and concentrated. Chromatograph on silica gel (2:1, hexanes/EtOAc) gave 3-[3-fluoro-4-(2-methoxy-ethoxy)-phenyl]-1-trityl-5-nitro-1 H-indazole **4AW** (2.8 g).

### Step 3: Preparation of 3-[3-Fluoro-4-(2-methoxy-ethoxy)-phenyl]-1-trityl-1H-indazol-5-ylamine

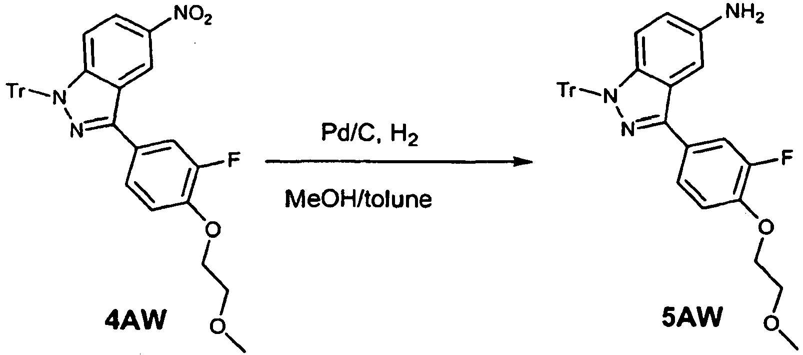

A suspension of 3-[3-fluoro-4-(2-methoxy-ethoxy)-phenyl]-1-trityl-5-nitro-1 H-indazole 4 (2.8 g, 4.88 mmol) and catalytic amount of Pd on carbon (5 wt%, 0.5 g) in 120 mL of methanol/toluene (1:1) was stirred under a hydrogen atmosphere for 6 h and filtered through Celite. The filtrate was concentrated to provide 3-[3-fluoro-4-(2-methoxy-ethoxy)-phenyl]-1-trityl-1H-indazol-5-ylamine 5 (2.6 g) as an off-white solid.

### Preparation 72

### Step 1: Preparation of -3-[4-Fluoro-3-(2-hydroxy-ethoxy)-phenyl]-1-trityl-5-nitro 1H-indazole

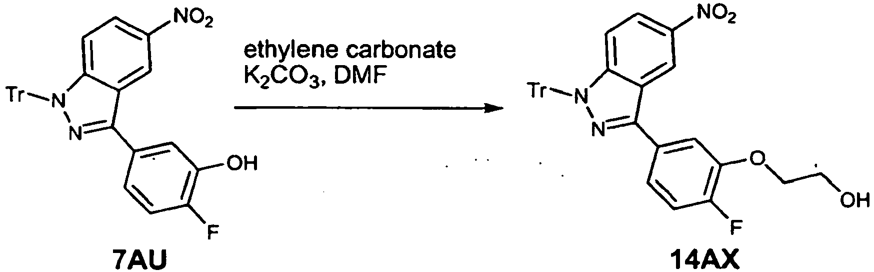

A mixture of 3-(4-fluoro-3-hydroxyphenyl)-5-nitro-1-trityl-1 H-indazole **7AU** (1.25 g, 2.42 mmol), K₂CO₃ (402 mg, 2.91 mmol) and ethylene carbonate (2.6 mL, 24.00 mmol) in DMF (10 mL) was stirred in a sealed tube at 160°C for 4 h and cooled to rt. H₂O was added to quench the reaction. The resulting mixture was extracted with EtOAc, which was washed with H₂O, brine, dried (MgSO₄) and concentrated to give a yellow oil. Chromatograph on silica gel (4:1, hexanes/EtOAc) gave 3-[4-fluoro-3-(2-hydroxy-ethoxy)-phenyl]-1-trityl-5-nitro-1 H-indazole **14AX** (1.4 g) as a yellow oil.

### Step 2: Preparation of 3-[4-Fluoro-3-(2-hydroxy-ethoxy)-phenyl]-1-trityl-1H-indazol-5-ylamine

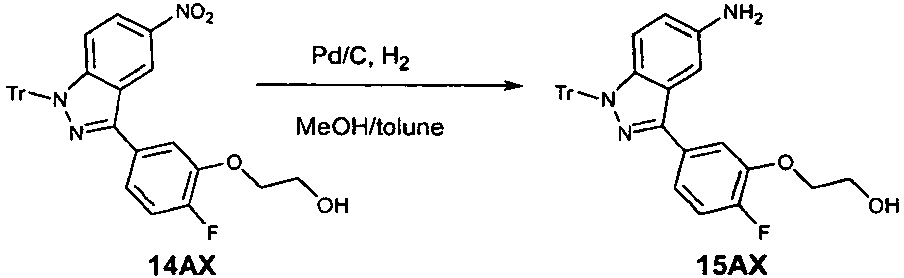

A suspension of 3-[4-fluoro-3-(2-hydroxy-ethoxy)-phenyl]-1-trityl-5-nitro-1 H-indazole **14AX** (1.5 g) and catalytic amount of Pd on carbon (5 wt%, 300 mg) in 40 mL of methanol/toluene (1:1) was stirred under a hydrogen atmosphere for 1 d and filtered through Celite. The filtrate was concentrated to provide 3-[4-fluoro-3-(2-hydroxy-ethoxy)-phenyl]-1-trityl-1 H-indazol-5-ylamine 15AX (1.4 g) as an off-white solid.

### Preparation 73

### Synthesis of 6-Pyrimidin-2-yl-1,2,3,4-tetrahydro-isoquinoline

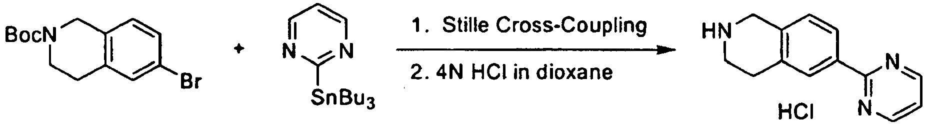

To a Schlenk tube were charged Pd₂(dba)₃ (10 mg, 0.01 mmol), bis(tri-*tert-*butylphosphine)palladium (20 mg, 0.04 mmol), CuI (16 mg, 0.08 mmol) and CsF (334 mg, 2.2 mmol). The tube was evacuated under high vacuum and back-filled with nitrogen for three cycles. DMF (2 ml) was introduced, followed by 2-tributylstannylpyrimidine (537 mg, 1.4 mmol). The tube was sealed with a Teflon cap and the reaction mixture was heated with stirring at 120°C for 2 hours. After cooling, the mixture was filtered through Celite, washed with ethyl acetate. Filtrate was washed with water three times, brine and dried (MgSO₄). After concentration the residue was purified on silica gel eluting with ethyl acetate in hexanes (0-100%) to give 6-pyrimidin-2-yl-3, 4-dihydro-1*H-*isoquinoline-2-carboxylic acid *tert*-butyl ester (28 mg). The compound was treated with 4N HCl in dioxane for 30 minutes. After concentration title compound was obtained as hydrochloride salt.

### Preparation 74

### Preparation of 2-[5-(5-amino-1-trityl-1H-indazol-3-yl)-2-fluoro-phenylamino]-ethanol

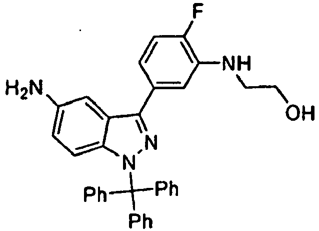

### Step 1: Preparation of 2-fluoro-5-(5-nitro-1-trityl-1H-indazol-3-yl)-phenylamine

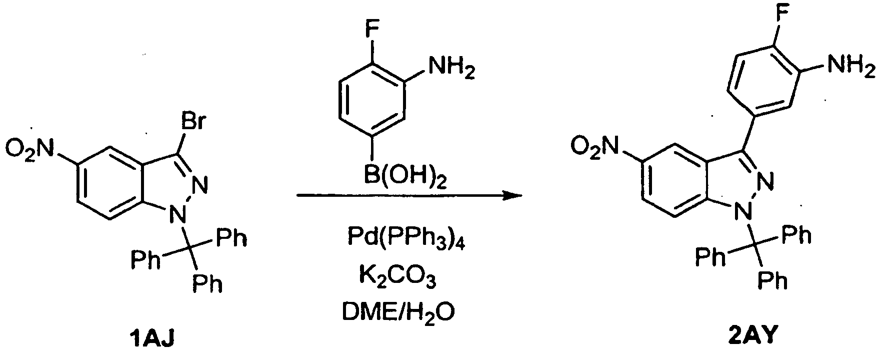

To a pressure tube were charged compound **1AJ** (1g, 2.1 mmol), 3-amino-4-fluoro-phenylboronic acid (448 mg, 2.89 mmol), Pd(PPh₃)₄ (120 mg, 0.1 mmol), K₂CO₃ (1.4 g, 10.1 mmol), DME (8 ml) and water (2 ml). The resulting mixture was degassed with nitrogen for 20 seconds and the tube was sealed with a Teflon cap, and heated at 100 C with stirring overnight. After cooling the reaction mixture was diluted with ethyl acetate, organic layer was isolated, washed with brine. After concentration, the residue was purified on silica gel. Elution with ethyl acetate in hexanes (0-70%) gave compound **2AJ** (1g).

### Step 2: Synthesis of 3-[2-fluoro-5-(5-nitro-1-trityl-1H-indazol-3-yl)-phenyl]-oxazolidin-2-one

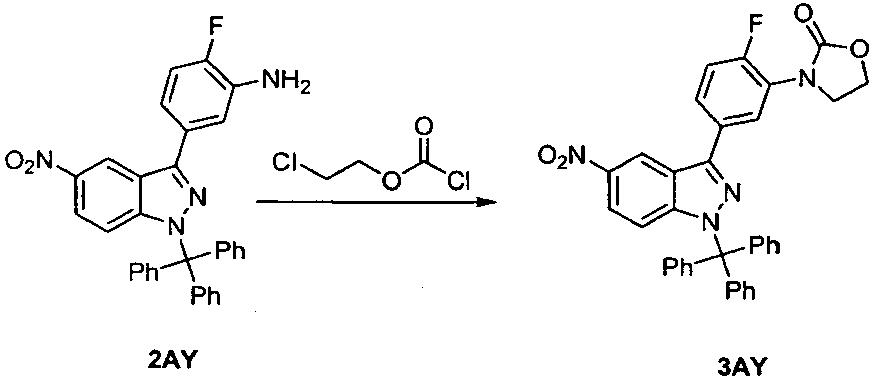

To a mixture of compound **2AY** (1g, 1.95 mmol), Cs₂CO₃ (1.2 g, 3.68 mmol) in acetonitrile (20 ml) was added 2-chloroethyl chloroformate (200 µl, 1.94 mmol) and the resulting mixture was refluxed for 20 minutes. After addition of 2-chloroethyl chloroformate (150 µl) the reaction mixture was allowed to reflux overnight. A solution of 40% KOH was added and the mixture was further refluxed for 2 hours. After cooling the reaction mixture was diluted with ethyl acetate and water, and aqueous layer was separated and extracted with ethyl acetate twice. Combined organic layers were washed with brine and dried (MgSO₄). After concentration the residue was purified on silica gel eluting with ethyl acetate in hexanes (0-40%) to give compound **3AY** (1g).

### Step 3: Preparation of 2-[2-fluoro-5-(5-nitro-1-trityl-1H-indazol-3-yl)-phenylamino]-ethanol

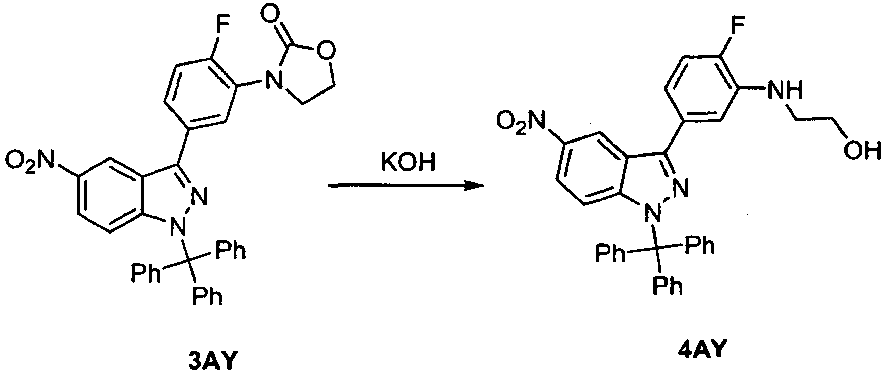

A mixture of compound **3AY** (658 mg) in DME (15 ml), MeOH (6 ml) and 40% KOH (4 ml) was refluxed overnight. After cooling, the mixture was diluted with ethyl acetate, organic layer was separated, washed with brine and dried (MgSO₄). After concentration the residue was purified on silica gel eluting with ethyl acetate in hexanes (0-100%) to give compound **4AY** (559 mg).

### Step 4: Preparation of 2-[5-(5-amino-1-trityl-1H-indazol-3-yl)-2-fluoro-phenylamino]-ethanol

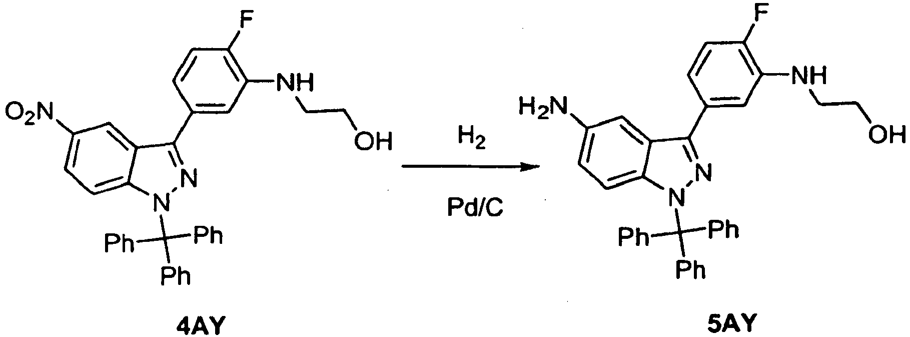

A mixture of compound **4AY** (173 mg), 10% Pd/C (50 mg) in ethyl acetate (10 ml) was stirred under hydrogen (balloon pressure) overnight. After filtration and concentration, the residue was purified on silica eluting with ethyl acetate to provide compound **5AY** (81 mg).

### Preparation 75

### Synthesis of 2-[4-(5-amino-1-trityl-1H-indazol-3-yl)-phenylamino]-ethanol

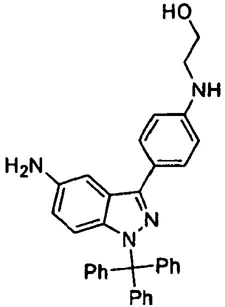

### Step 1: Preparation of 4-(5-nitro-1-trityl-1H-indazol-3-yl)-phenylamine

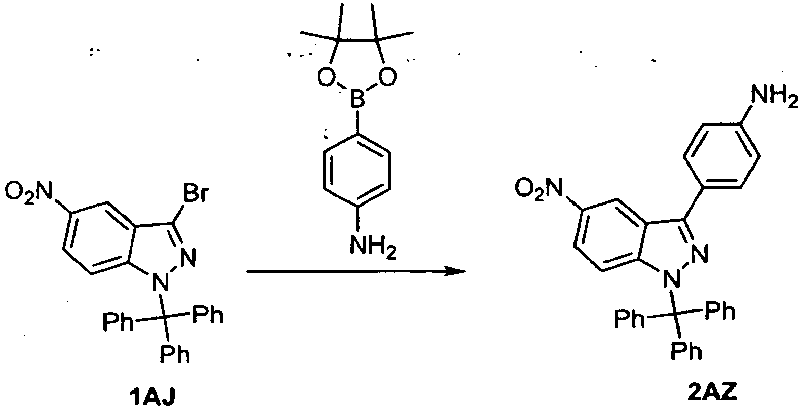

To a pressure tube were charged with compound **1AJ** (1g, 2.1 mmol), 4-(4,4,5,5-tetramethyl-[1,3,2]dioxaborolan-2-yl)-phenylamine (0.635 g, 2.9 mmol), Pd(PPh₃)₄ (0.12 g, 0.1 mmol), K₂CO₃ (1.4 g, 10.1 mmol), DME (8 ml) and water (2 ml). The resulting mixture was degassed with nitrogen briefly and the tube was sealed with a Teflon cap, and heated at 100 C with stirring overnight. After cooling, the reaction mixture was diluted with ethyl acetate, organic layer was isolated, washed with brine. After concentration, the residue was purified on silica gel. Elution with ethyl acetate in hexanes (0-70%) gave compound **2AZ** (0.956 g).

### Step 2: Preparation of 3-[4-(5-nitro-1-trityl-1H-indazol-3-yl)-phenyl]-oxazolidin-2-one

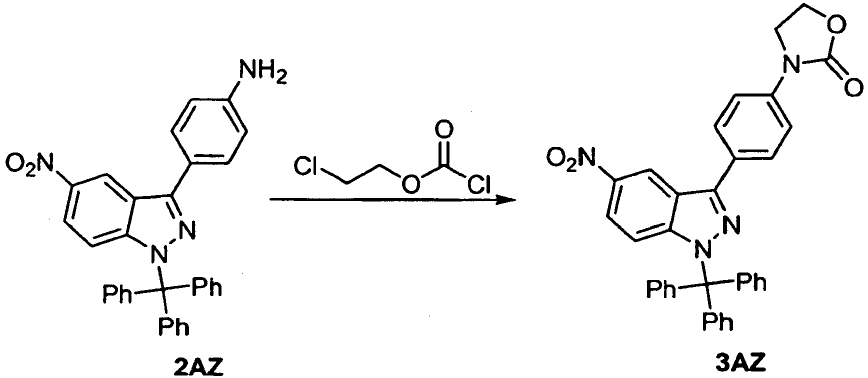

To a mixture of compound **2AZ** (409 mg, 0.82 mmol), 10% NaOH (2 ml) in DME (8 ml) was added 2-chloroethyl chloroformate (102 µl, 1 mmol) and the resulting mixture was stirred at rt for overnight. The mixture was diluted with ethyl acetate and water, and aqueous layer was separated and extracted with ethyl acetate twice. Combined organic layers were washed with brine and dried (MgSO₄). After concentration the residue was purified on silica gel eluting with ethyl acetate in hexanes (0-80%) to give compound **3AZ** (340 mg).

### Step 3: Preparation of 2-[4-(5-nitro-1-trityl-1H-indazol-3-yl)-phenylamino]-ethanol

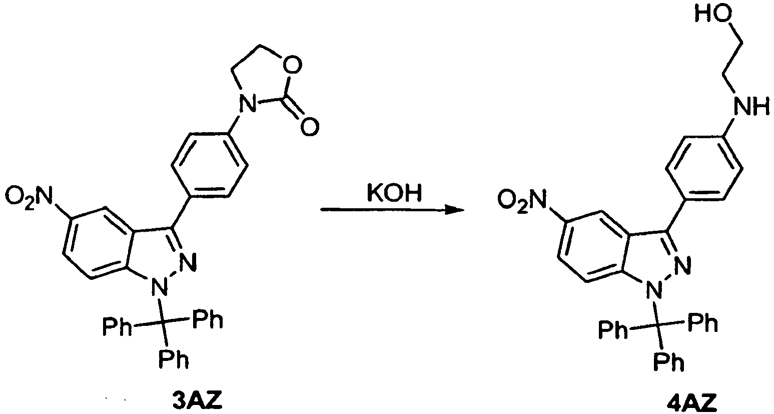

A mixture of compound **3AZ** (340 mg) in DME (6 ml), MeOH (4 ml) and 40% KOH (2 ml) was refluxed for two hours. After cooling, solvents were removed, diluted with water, extracted with dichloromethane three times, combined organic layers were washed with brine and dried (MgSO₄). After concentration the residue was purified on silica gel eluting with ethyl acetate in hexanes (0-100%) to give compound 4AZ (280 mg).

### Step 4: Preparation of 2-[4-(5-amino-1-trityl-1H-indazol-3-yl)-phenylamino]-ethanol

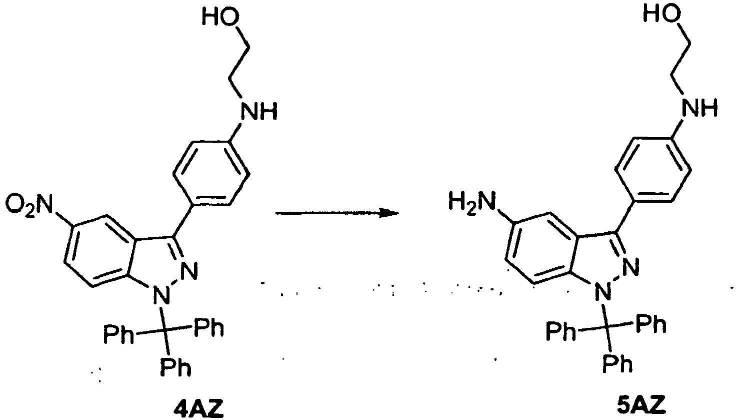

A mixture of compound **4AZ** (280 mg), 10% Pd/C (70 mg) in ethanol (10 ml) was stirred at 45°C under hydrogen (balloon pressure) overnight. After filtration and concentration, the residue was purified on silica eluting with ethyl acetate to provide compound 5AZ (224 mg).

### Preparation 76

### Synthesis of 3-(2-Isobutyl-thiazol-5-yl)-1-trityl-1H-indazol-5-ylamine

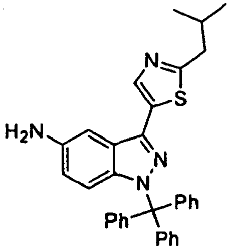

### Step 1: Preparation of 3-(2-Isobutyl-thiazol-5-yl)-5-nitro-1-trityl-1H-indazole

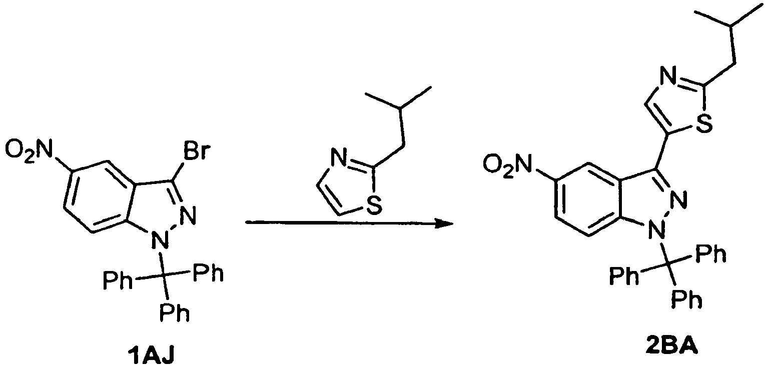

To a pressure tube were charged compound **1AJ** (194 mg, 0.4 mmol), Pd(OAc)₂ (90 mg, 0.4 mmol), (2-biphenyl)-di-*tert*-butylphospine (12 mg, 0.1 mmol), C₂CO₃ (313 mg, 0.96 mmol) and the tube was evacuated under vacuum and back-filled with nitrogen. Dioxane (4 ml) was introduced, followed by 2-isobutylthiazole (114 µl, 0.8 mmol). The tube was sealed and heated at 100 °C with stirring overnight. After cooling the reaction mixture was filtered through Celite, washed with ethyl acetate several times. Filtrate was concentrated and the residue was purified on silica gel. Elution with ethyl acetate in hexanes (0-30%) gave compound **2BA** (124 mg).

### Step 2: Preparation of 3-(2-Isobutyl-thiazol-5-yl)-1-trityl-1H-indazol-5-ylamine

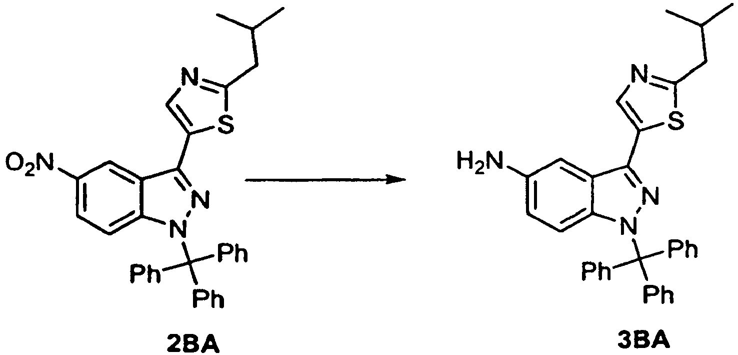

A mixture of compound **2BA** (180 mg), 10% Pd/C (50 mg) in ethyl acetate (8 ml) was stirred at 45°C under hydrogen (balloon pressure) overnight. After filtration and concentration, the residue was purified on silica eluting with ethyl acetate to provide compound **3BA** (75 mg).

### Preparation 77

### Synthesis of 3-(2-methyl-thiazol-5-yl)-1-trityl-1H-indazol-5-ylamine

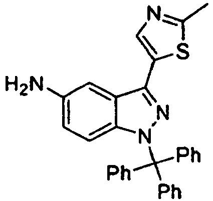

Preparation of 3-(2-methyl-thiazol-5-yl)-1-trityl-1*H-*indazol-5-ylamine was similar to that of 3-(2-isobutyl-thiazol-5-yl)-1-trityl-1*H-*indazol-5-ylamine as illustrated above except using 2-methylthiazole in the cross-coupling reaction.

### Preparation 78

### Synthesis of 3-[5-(5-amino-1-trityl-1H-indazol-3-yl)-2-fluoro-phenyl]-oxazolidin-2-one

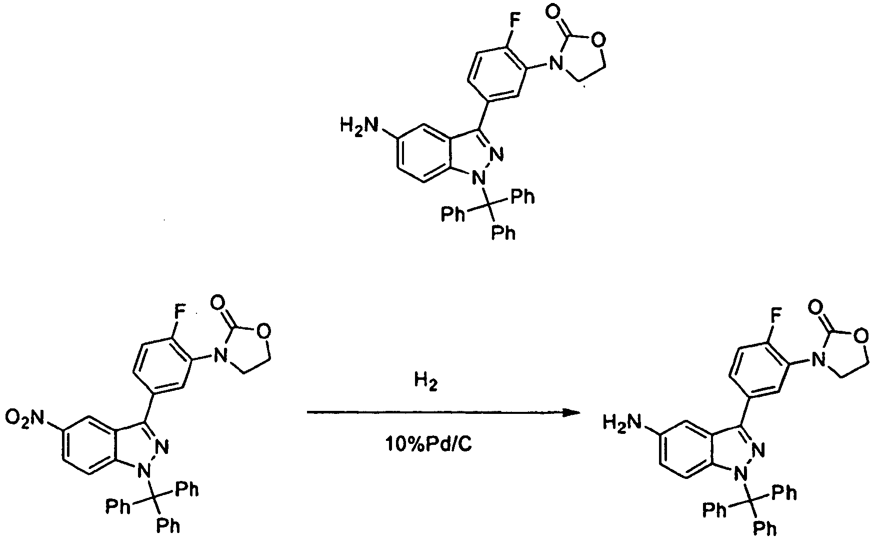

The hydrogenation of 3-[2-fluoro-5-(5-nitro-1-trityl-1*H-*indazol-3-yl)-phenyl]-oxazolidin-2-one was conducted in similar way to the other examples as illustrated above.

### Preparation 79

### Synthesis of 3-[4-(5-amino-1-trityl-1H-indazol-3-yl)-phenyl]-oxazolidin-2-one

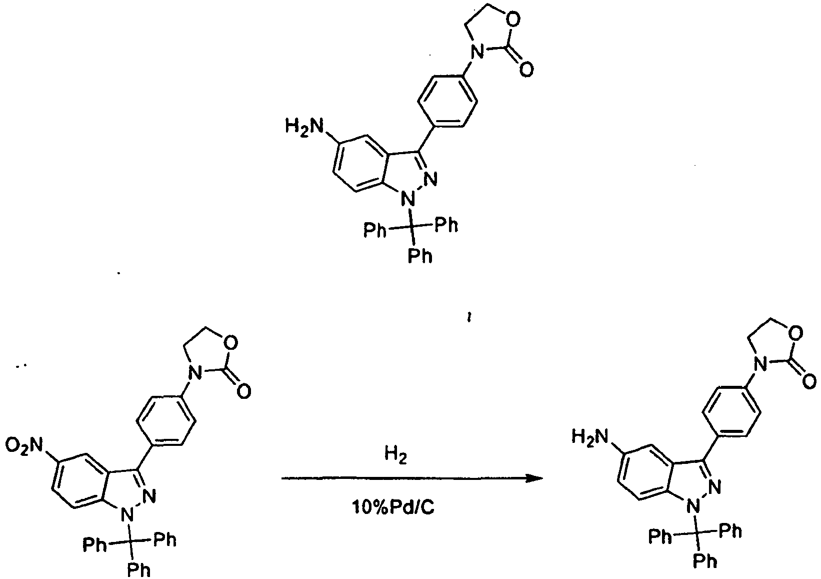

The hydrogenation of 3-[4-(5-nitro-1-trityl-1*H-*indazol-3-yl)-phenyl]-oxazolidin-2-one was conducted in similar way to the other examples as illustrated above.

### Preparation 80

### Synthesis of 6-Pyrimidin-2-yl-1,2,3,4-tetrahydro-isoquinoline

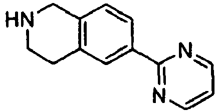

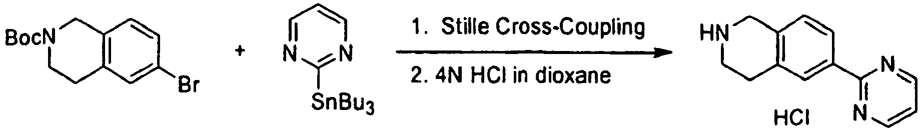

To a Schlenk tube were charged Pd₂(dba)₃ (10 mg, 0.01 mmol), bis(tri-*tert-*butylphosphine)palladium (20 mg, 0.04 mmol), Cul (16 mg, 0.08 mmol) and CsF (334 mg, 2.2 mmol). The tube was evacuated under high vacuum and back-filled with nitrogen for three cycles. DMF (2 ml) was introduced, followed by 2-tributylstannylpyrimidine (537 mg, 1.4 mmol). The tube was sealed with a Teflon cap and the reaction mixture was heated with stirring at 120°C for 2 hours. After cooling, the mixture was filtered through Celite, washed with ethyl acetate. Filtrate was washed with water three times, brine and dried (MgSO₄). After concentration the residue was purified on silica gel eluting with ethyl acetate in hexanes (0-100%) to give 6-pyrimidin-2-yl-3, 4-dihydro-1*H-*isoquinoline-2-carboxylic acid *tert*-butyl ester (28 mg). The compound was treated with 4N HCl in dioxane for 30 minutes. After concentration title compound was obtained as hydrochloride salt.

### Preparation 81

### Preparation of 4-Benzothiazol-2-yl-3,6-dihydro-2H-pyridine-1-carboxylic acid tert-butyl ester (Compound 21BB)

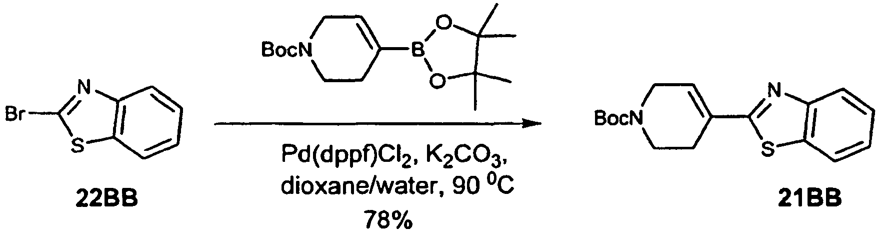

A mixture of 2-bromo-benzothiazole (0.38 g, 1.1 mmol), 4-(4,4,5,5-tetramethyl-[1,3,2]dioxaborolan-2-yl)-3,6-dihydro-2*H-*pyridine-1-carboxylic acid *tert*-butyl ester (0.5 g, 1.62 mmol), potassium carbonate (0.67 g, 4.85 mmol), Pd(dppf)Cl₂ (0.132 g, 0.16 mmol) and 4/1 /dioxane/water (10 ml) was degassed for 15 minutes. Then it was heated at 90°C for overnight. Cooled to room temperature and diluted with EtOAc (200 mL). The organic layer was washed with water (100 ml), dried over Na₂SO₄, filtered and concentrated. The residue was purified on silica gel to give the desired product **21BB** (0.4 g, 78%).

### Preparation 82

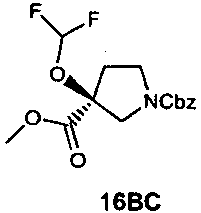

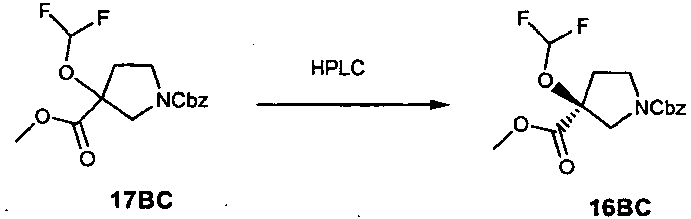

The Compound **17BC** is prepared following a procedure similar to that of Preparation 37 Steps 1 to 4 by substituting 3-oxo-pyrrolidine-1-carboxylic acid benzyl ester for 3-oxo-pyrrolidine-1-carboxylic acid tert-butyl ester in Step 1 of Ppreparation 37. Compound **17B** was separated on HPLC using ChiralCel AS column eluting with 25% isopropanol/ 75% hexane. The first peak (retention time 46 minutes) was the desired product **16BC**.

### Preparation 83

### Preparation of 3-(4,4-Difluoro-piperidin-1-yl)-1-trityl-1H-indazol-5-ylamine (Compound 13BD)

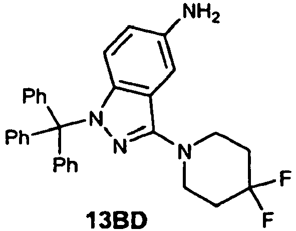

### Step 1: Preparation of 3-(4,4-Difluoro-piperidin-1-yl)-5-nitro-1-trityl-1H-indazole

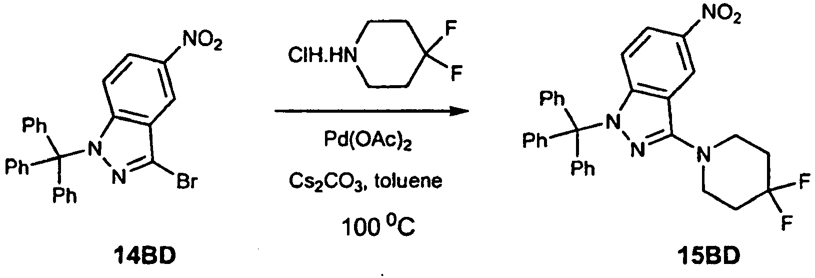

The Compound 14BD was converted to Compound 15BD using the procedure as described for the preparation of Compound 4AD (Preparation 49 Step 2) from Compound 2AD (Preparation 49 Step 2).

### Step 2

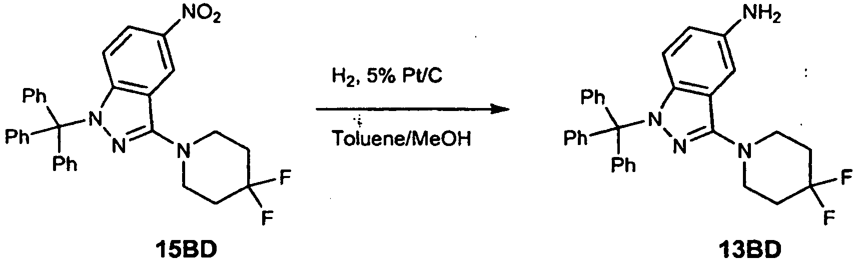

A mixture of Compound **15BD** (1.6 g, 3.1 mmol), 1/1 toluene/MeOH (25 mL) and 5% Pt/C (0.726 mg) was stirred under hydrogen at room temperature for 18 hours. Reaction mixture was filtered over celite and the filtrate was concentrated. The residue was purified on silica gel eluting with 5% MeOH/CH₂Cl₂ to give the desired product **13BD** (1.38 g, 91%).

### Example 623

### Preparation of 1-(2-{4-[4-(5-Ethylamino-[1,3,4]oxadiazol-2-yl)-phenyl]-piperidin-1-yl}-2-oxo-ethyl)-3-methoxy-pyrrolidine-3-carboxylic acid [3-(4-fluoro-phenyl)-1H-indazol-5-yl]-amide (Compound of Example 623)

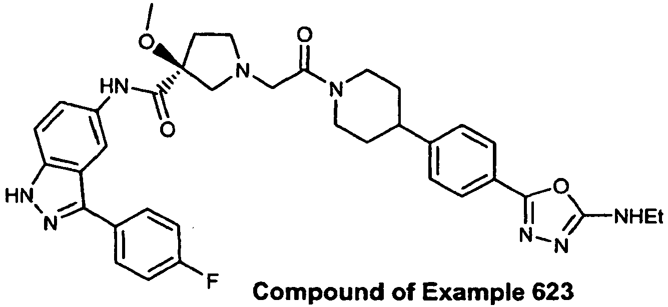

### Step 1: Preparation of 4-(4-Methoxycarbonyl-phenyl)-piperidine-1-carboxylic acid tert-butyl ester

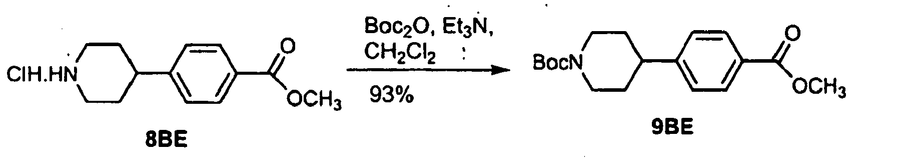

A mixture of Compound **8BE** (3 g, 11.73 mmol), CH₂Cl₂ (30 mL), triethyl amine (4.9 mL, 35.19 mmol) and di-*tert*-butyl dicarbonate (3.83 g, 17.55 mmol) was stirred at room temperature for 3 hours. Diluted with CH₂Cl₂ (100 mL) and washed with water (100 mL). The organic layer was dried over Na₂SO₄, filtered and concentrated. The residue was purified on silica gel eluting with 100% EtOAc to give the desired product **9BE** (3.5 g, 93%).

### Step 2: Preparation of 4-(4-Hydrazinocarbonyl-phenyl)-piperidine-1-carboxylic acid tert-butyl ester

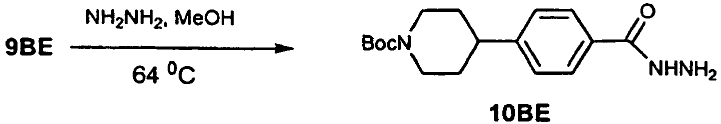

The Compound **9BE** was converted to Compound **10BE** using the procedure as described for the preparation of Compound **2AR** from Compound **1AR** (Example 622 Step 1).

### Step 3: Preparation of 4-[4-(5-Ethylamino-[1,3,4]oxadiazol-2-yl)-phenyl]-piperidine-1-carboxylic acid tert-butyl ester (Compound 11BE)

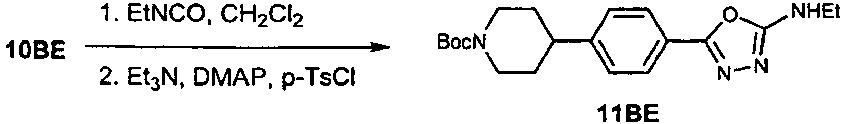

The Compound **10BE** was converted to Compound **11BE** using the procedure as described for the preparation of Compound **3AR** from Compound **2AR** (Example 622 Step 2).

### Step 4:

The Compound **11BE** was converted to the compound of Example **623** using the procedure as described for the preparation of the compound of Example **622** from Compound **4AR**.

### Example 624

### Preparation of 1-(2-{4-[4-(5-methylamino-[1,3,4]oxadiazol-2-yl)-phenyl]-piperidin-1-yl}-2-oxo-ethyl)-3-methoxy-pyrrolidine-3-carboxylic acid [3-(4-fluoro-phenyl)-1H-indazol-5-yl]-amide (Compound of Example 624)

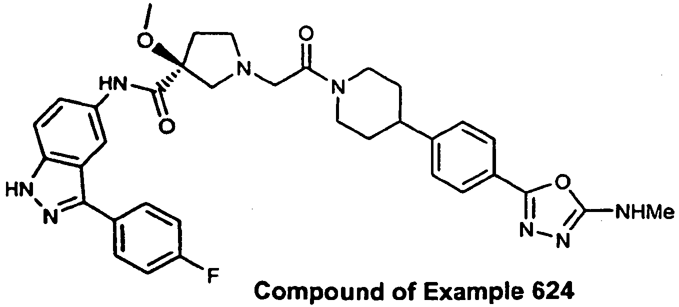

The compound of Example **624** was prepared from Compound **10BE** (Example 623 Step 3) using the procedure as described for the preparation of the compound of Example **623** from Compound **10BE** but using methyl isocyanate in place of ethyl isocyanate.

### Preparation 84

### Preparation of of 2-Chloro-1-{4-[2-Fluoro-4-(5-methyl-[1,3,4]oxadiazol-2-yl)-phenyl]-3,6-dihydro-2H-pyridin-1-yl}-ethanone (Compound 20BF)

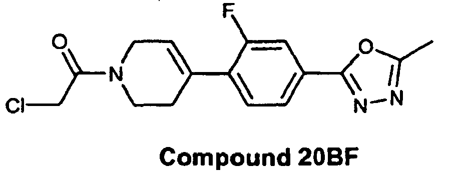

### Step 1: Preparation of of 2-(4-Bromo-3-fluoro-phenyl)-5-methyl-[1,3,4]oxadiazole

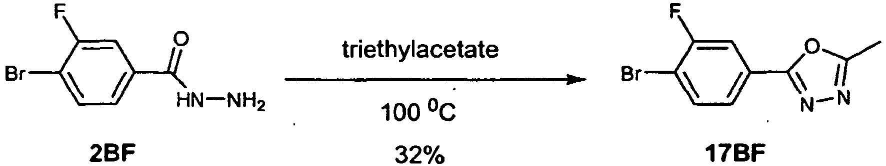

A mixture of Compound **2BF** (0.9 g, 2.53 mmol) and triethylacetate (5 mL) was heated at 100 °C for 18 hours. Cooled to room temperature and poured into water (100 mL). Extracted with EtOAc (100 mL). The organic layer was dried (Na₂SO₄), filtered and concentrated. The residue was purified on silica gel eluting with 20% EtOAc/ hexane to give the desired product **17BF** (0.36 g, 32%).

### Step 2: Preparation of of 4-[2-Fluoro-4-(5-methyl-[1,3,4]oxadiazol-2-yl)-phenyl]-3,6-dihydro-2H-pyridine-1-carboxylic acid tert-butyl

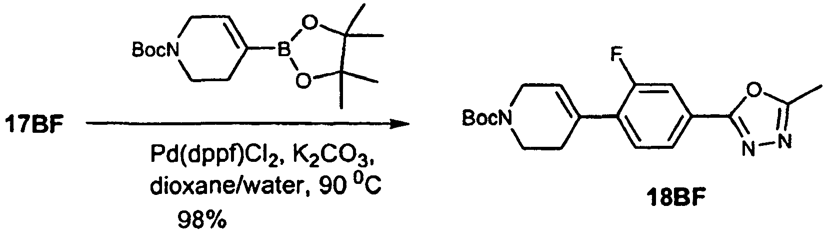

A mixture of Compound **17BF** (0.34 g, 0.99 mmol), 4-(4,4,5,5-tetramethyl-[1,3,2]dioxaborolan-2-yl)-3,6-dihydro-2*H-*pyridine-1-carboxylic acid *tert*-butyl ester (0.37 g, 1.19 mmol), potassium carbonate (0.41 g, 2.97 mmol), Pd(dppf)Cl₂ (0.081 g, 0.099 mmol) and 4/1 /dioxane/water (10 ml) was degassed for 15 minutes. Then it was heated at 90 °C for overnight. Cooled to room temperature and diluted with EtOAc (200 ml). The organic layer was washed with water (100 ml), dried over Na₂SO₄, filtered and concentrated. The residue was purified on silica gel to give the desired product 18BF (0.35 g, 98%).

### Step 3: Preparation of 4-[2-Fluoro-4-(5-methyl-[1,3,4]oxadiazol-2-yl)-phenyl]-1,2,3,6-tetrahydro-pyridine

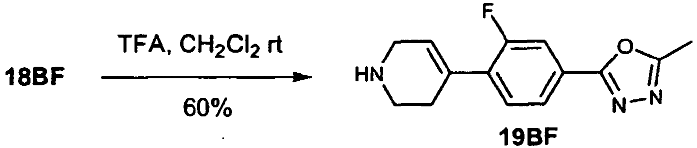

The Compound **18BF** (0.44 g, 1.13 mmol) was converted to Compound **19BF** using the procedure as described for the preparation of Compound **5AR** from Compound **4AR** (Example 622 Step 4).

### Step 4:

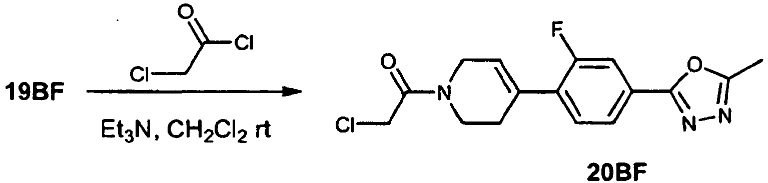

The Compound **19BF** (0.44 g, 1.13 mmol) was converted to Compound **20BF** using the procedure as described for the preparation of Compound **6AR** from Compound **5AR** (Example 622 Step 5).

### Preparation 85

### 3-(1-Methyl-1H-pyrazol-3-yl)-5-nitro-1-trityl-1H-indazole

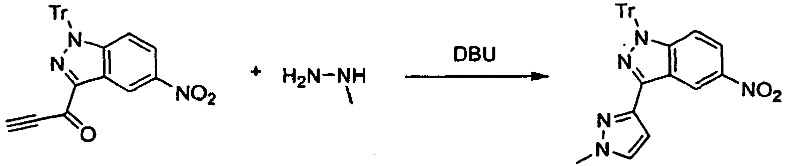

A mixture of 1-(5-nitro-1-trityl-1*H-*indazol-3-yl-)-propynone (0.5g, 1.09mmol), methyl hydrazine hydrochloride (1.75mmol) and DBU **(1.6ml,** 10.9mmol) in acetonitrile (10ml) was heated at 120°C for 50 minutes under microwave. It was then cooled to room temperature and diluted with DCM (60ml). The organic layer was washed with water (10ml), dried over MgSO₄. filtered and concentrated. The residue was purified on silica gel eluting with 1/1 EtOAc/Hexane to give the desired product (0.42 g, 79%).
MS (ESMS, M+H 486)

Reduction of the 5-nitro compound was achieved by hydrogenation as stated previously

### Preparation 63

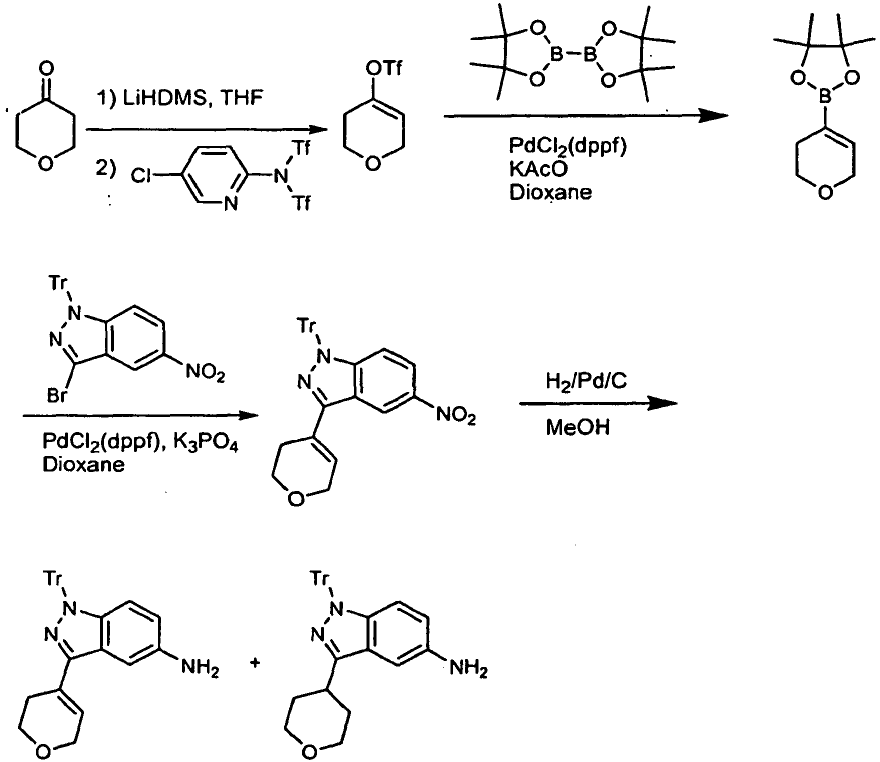

### Step 1: Preparation of Trifluoro-methanesulfonic acid 3,6-dihydro-2H-pyran-4-yl ester

To a solution of 4-tetrahydropyranone (2g, 0.02 mol) in THF (10 mL) at -78 °C, LiHMDS (1 M, 11 ml) was added dropwise through a syringe. The formed solution was then stirred at -78 °C for about 1 hour. The reaction was warmed up to room temperature briefly and was cooled back to -78 °C. Then 2-[N, N-bis(trifluoromethylsulfonyl)amino]-5-chloropyrine (7.85 g, 0.02 mol) was added in four portions. The resulted reaction mixture was gradually warmed up to room temperature and was stirred for overnight. After removal of solvent, the residue was purified using chromatography (eluted with DCM/Hexane = 80/20) to give product as a colorless oil (3g).

### Step 2: Preparation of 4-(4,4,5,5-Tetramethyl-[1,3,2]dioxaborolan-2-yl)-3,6-dihydro-2H-pyran

A 250 mL round bottom flask containing trifluoro-methanesulfonic acid 3, 6-dihydro-2H-pyran-4-yl ester (2.7 g, 11.6 mmol), PdCl₂(dppf) (440 mg, 0.6 mmol) and potassium acetate (3g, 36 mmol) in dioxane (20 mL) was flushed with Ar three times. The reaction mixture was heated at 80 °C for overnight. After the reaction was cooled to room temperature, the reaction mixture was filter through a column packed with celite and the filtrate was concentrated. The residue was purified using column chromatography (10 % hexane in dichloromethane) and product was obtained as white solid (2g, 81% yield).

### Step 3: Preparation of 3-(3,6-Dihydro-2H-pyran-4-yl)-5-nitro-1-trityl-1H-indazole

A mixture containing 4-(4,4,5,5-Tetramethyl-[1,3,2]dioxaborolan-2-yl)-3,6-dihydro-2H-pyran (0.8 g, 3.8 mmol), 3-Bromo-5-nitro-1-trityl-1H-indazote (2g, 4.0 mmol), PdCl₂(dppf) (150 mg, 0.2 mmol) and potassium phosphate (2.6 g, 12 mmol) in dioxane (10 mL) was flushed with Ar and was heated at 80°C for overnight under Ar. LC-MS indicated the completion of reaction and the mixture was filter through celite. The filtrate was concentrated and was purified on a column (10 hexane in dichloromethane) to give 1.2 g of product (64 % yield).

### Step 4: Preparation of 3-(3,6-Dihydro-2H-pyran-4-yl)-1-trityl-1H-indazol-5-ylamine and 3-(Tetrahydro-pyran-4-yl)-1-trityl-1H-indazol-5-ylamine

To a solution of 3-(3,6-Dihydro-2H-pyran-4-yl)-5-nitro-1-trityl-1H-indazole (100 mg, 0.2 mmol) in methanol was added and Pd/C (10%, 20 mg). The reaction mixture was evacuated under vacuum and hydrogen gas was filled. After repeated three times the reaction was kept under hydrogen atmosphere overnight at room temperature. LCMS showed the starting material was consumed completely and both 3-(3,6-dihydro-2H-pyran-4-yl)-1-trityl-1 H-indazol-5-ylamine and 3-(tetrahydro-pyran-4-yl)-1-trityl-1 H-indazol-5-ylamine were obtained. The two products were separated using prep-HPLC to give both product in 60 % and 40 % yield respectively.

### Preparation 87 4-Quinoxalin-6-yl-3,6-dihydro-2H-pyridine-1-carboxylic acid tert-butyl ester

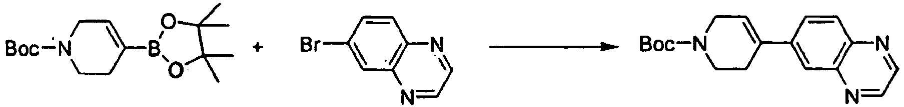

A solution containing 6-bromo-quinoxaline (417 mg, 2.0 mmol), 4-(4,4,5,5-tetramethyl-[1,3,2]dioxaborolan-2-yl)-3,6-dihydro-2H-pyridine-1-carboxylic acid tert-butyl ester (600 mg, 1.94 mmol), tetrakis [triphenylphosphine] palladium (108 mg, 0.1 mmol) and sodium carbonate (2 M solution, 3 mL) in 5 mL of dioxane/ethanol/water (7:3:1) was heated at 160 °C using microwave reactor for 15 minutes. After the reaction, ethylacetate was added and the mixture was filtered, washed with water. After concentration under vacuum, the product was purified using column chromatography (5% methanol in dichloromethane)

### Preparation 88

### 1-N Trityl-4-(5-Amino-1-methyl-1 H-indazol-3-yl)-pyrazole-1-carboxylic acid tert-butyl ester

### Step 1

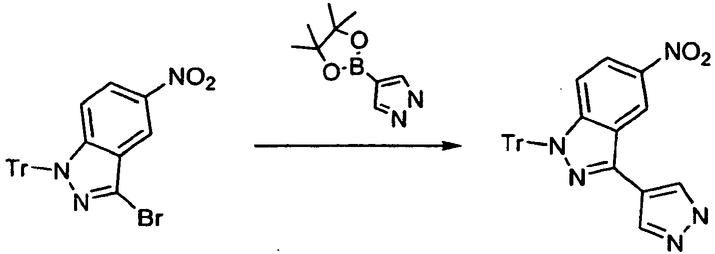

To a vial containing 3-bromo-5-nitro-1-trityl-1H-indazole (1.45 g, 3.00 mmol), 4-(4,4,5,5-TETRAMETHYL-1,3,2,-DIOXABOROLAN-2-YL)-1H-PYRAZOLE (0.873 g, 4.50 mmol) [1,1'-Bis(diphenylphosphino)ferrocene]dichloropalladium(II),complex with dichloromethane (1:1) (0.245 g, 0.300 mmol) and Potassium phosphate (1.59 g. 7.50 mmol) in a mixture of 1,4-Dioxane (25 mL, 320 mmol) Water (12 mL, 670 mmol) was degassed under an atmosphere of Nitrogen. This rxn mixture was was heated at 80 °C overnight under an atmosphere of Nitrogen. TLC (20%EtOAc/hexane) showed starting material. MS showed (M+H) at 472. The rxn was diluted with EtOAc and Water (50/50 mL) and filtered through Celite. The org. layer was washed with water, dried over Magnesium sulfate (2 g, 20 mmol) and rotavap to give a crude, 2.2g. The crude was chrom. (30%EtOAc/Hexane) on Biotage (40L) to obtain the product (fraction 2), M+H at 472, 5-nitro-3-(1H-pyrazol-4-yl)-1-trityl-1H-indazole (0.54 g; Yield = 38%)

### Step 2

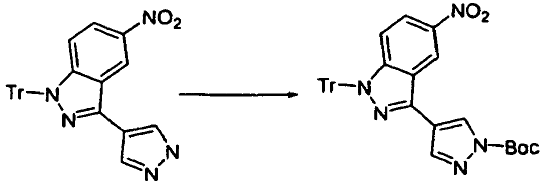

Into a Round bottom flask was added 5-nitro-3-(1H-pyrazol-4-yl)-1-trityl-1H-indazole (0.390 g, 0.827 mmol, recovered unreacted starting material, Methylene chloride (5.00 mL), 78.0 mmol), Di-tert-Butyldicarbonate (0.228 g, 1.04 mmol) 4-Dimethylaminopyridine (0.127 g, 1.04 mmol) The rxn was stirred for 2 days under an atmosphere of Nitrogen. TLC (20%EtOAc/Hexane) showed rxn complete. The rxn was loaded onto Biotage 40S and eluted with 20%EtOAc/hexane to give tert-butyl 4-(5-nitro-1-trityl-1H-indazol-3-yl)-1H-pyrazole-1-carboxylate (0.44 g; Yield = 93%)

### Step 3

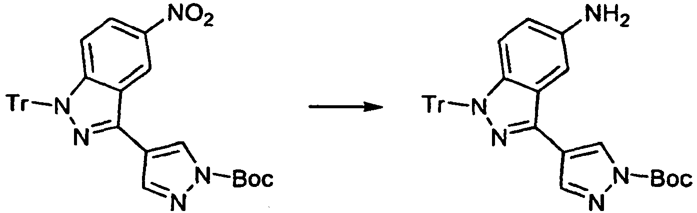

Into a Round bottom flask was added tert-butyl 4-(5-nitro-1-trityl-1H-indazol-3-yl)-1H-pyrazole-1-carboxylate (0.440 g, 0.770 mmol) and 10.mL of MeOH and 5 mL of EtOAc followed by Palladium (0.100 g, 0.000470 mmol). The rxn was degassed under Hydrogen (2000 mL), 90 mmol) in ballon, was stirred overnight. TLC (20%EtOAc/Hexane) and MS showed rxn complete. The rxn was filtered through Celite and was rotovaped to dryness to give tert-butyl 4-(5-amino-1-trityl-1 H-indazol-3-yl)-1H-pyrazole-1-carboxylate (0.400 g; Yield = 95.9%)

### Preparation 89

### Preparation of 1-N-Trityl-3-Benzo[1,3]dioxol-5-yl-1-methyl-1H-indazol-5-ylamine

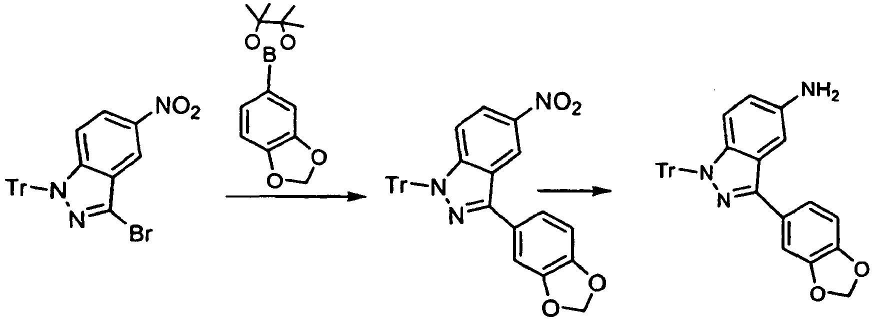

1-N-Trityl-3-Benzo[1,3]dioxol-5-yl-1-methyl-1H-indazol-5-ylamine was prepared by substituting 5-(4,4,5,5-Tetramethyl-[1,3,2]dioxaborolan-2-yl)-benzo[1,3]dioxole for 4-(4,4,5,5-Tetramethyl-[1,3,2]dioxaborolan-2-yl)-1 H-pyrazole in Preparation 88 in Steps 1 & 3.

Table 27 provides data for the compounds of Examples 613 to 623.

**Table 27**

| Ex No. | COMPOUND | M+1 | Retention Time (min) |
|---|---|---|---|
| 613 (16AA) | 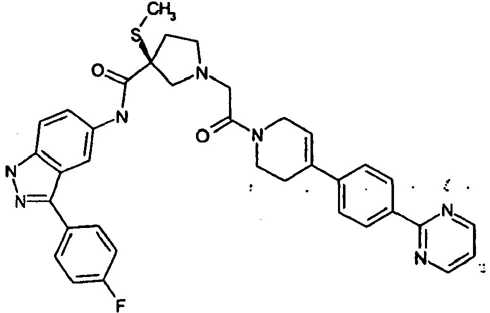 | 648.4 | 2.8 |
| 614 (9AB) | 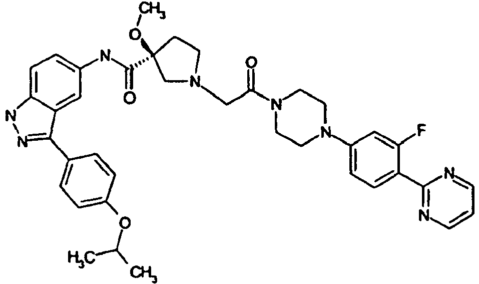 | 693.4 | 2.84 |
| 615 11AB | 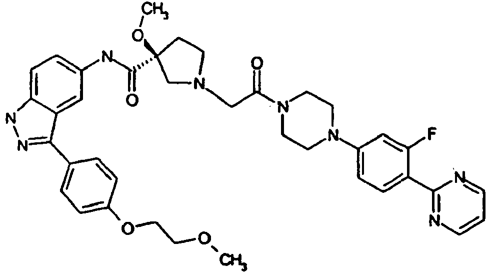 | 709.4 | 2.54 |
| 616 (13AB) | 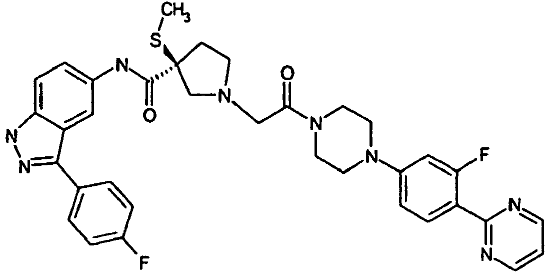 | 669.4 | 2.75 |
| 617 (25AB) | 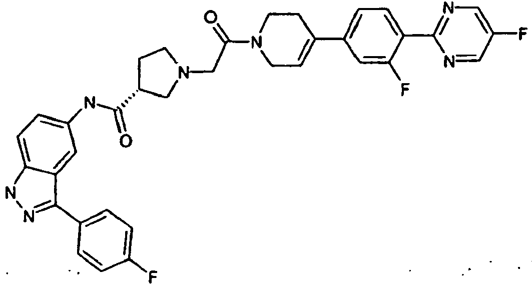 | 638.4 | 3.33 |
| 619 (33AB) | 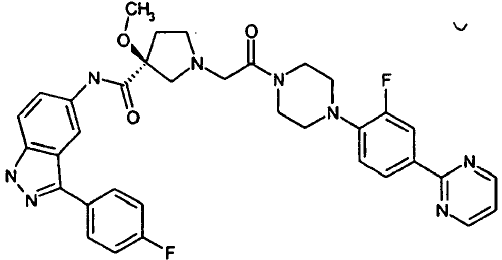 | 653.4 | 2.89 |
| 620 | 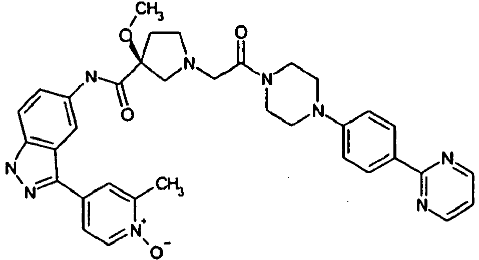 | 648.4 | 2.12 |
| 621 | 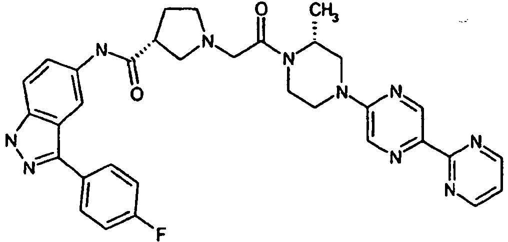 | 623 | 2.42 |
| 622 | 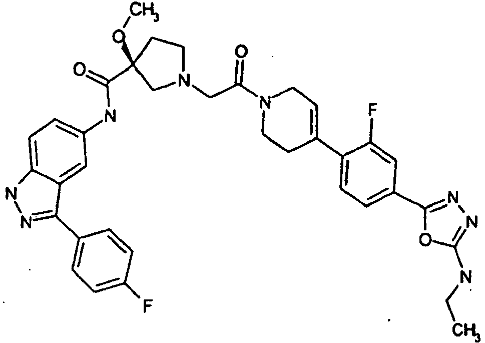 | 683.4 | 2.98 |
| 623 | 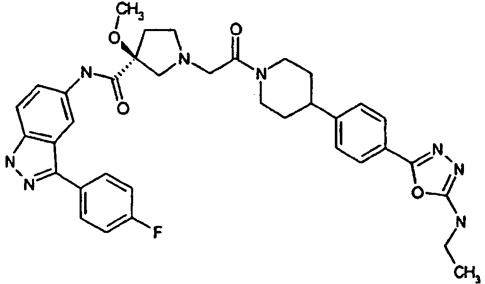 | 667.4 | 2.69 |

### Examples 625 to 825

Following the procedures described above, as well as those indicated in Table 28, the compounds in Table 28 were prepared.

**Table 28**

| Ex No. | COMPOUND | M+1 | Retention Time (min) |
|---|---|---|---|
| 625 | 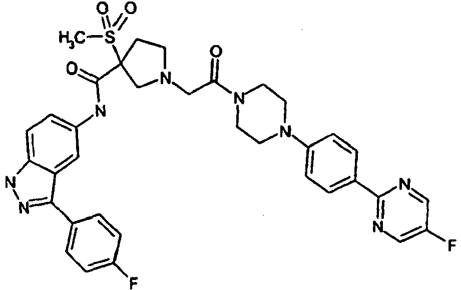 | 701.4 | 3.05 |
| 626 | 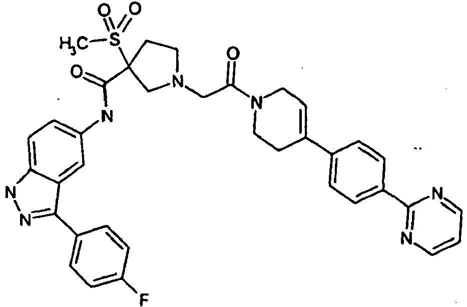 | 680.4 | 2.99 |
| 627 | 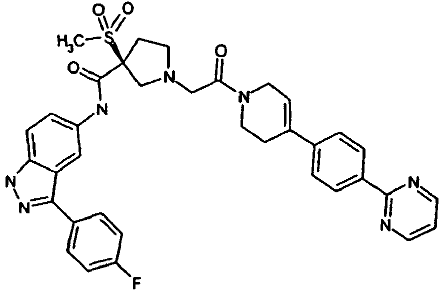 | | |
| | (see also Example 619) | 680.4 | 2.8 |
| 628 | 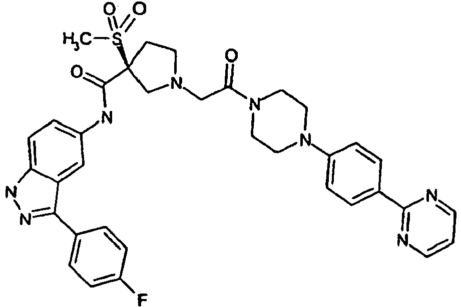 | 683.4 | 2.93 |
| 629 | 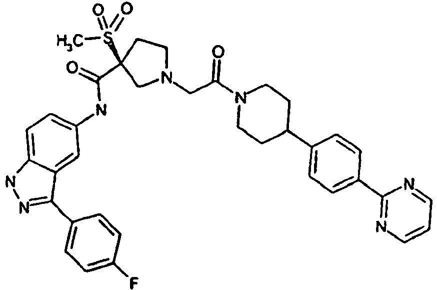 | 682.4 | 2.83 |
| 630 | 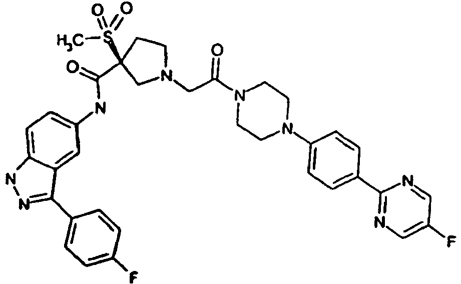 | 701.4 | 3.04 |
| 631 | 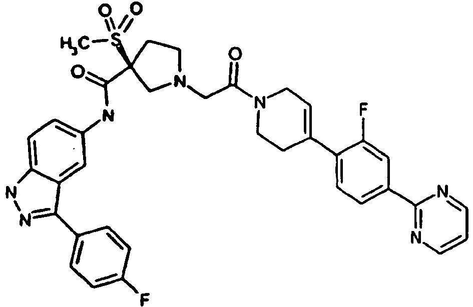 | 698.4 | 3.1 |
| 632 | 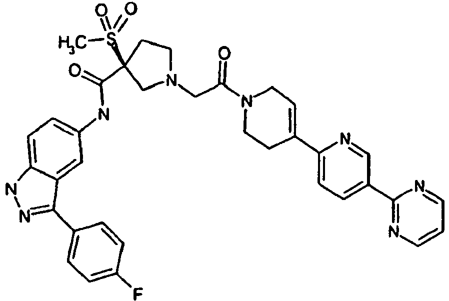 | 681.4 | 2.5 |
| 633 | 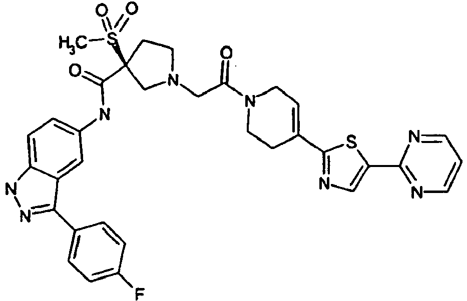 | 687.4 | 2.54 |
| 634 | 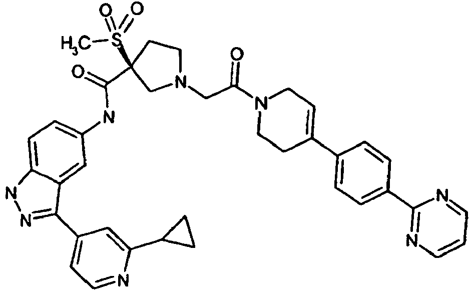 | 703.4 | 2.33 |
| 635 | 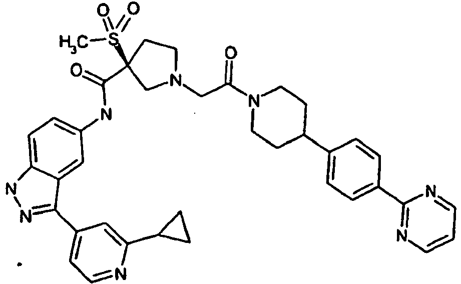 | 705.4 | 2.25 |
| 636 | 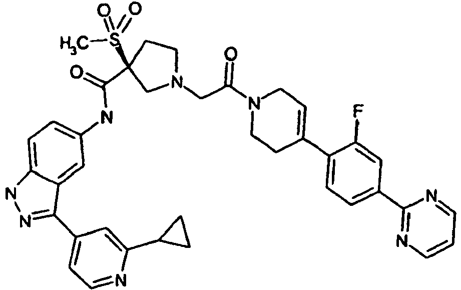 | 721.4 | 2.38 |
| 637 | 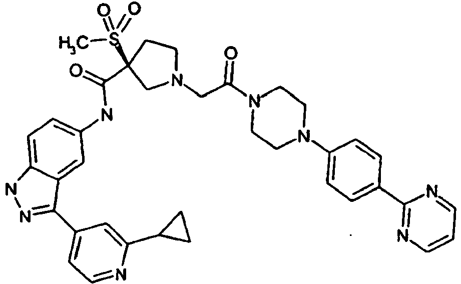 | 706.4 | 2.14 |
| 638 | 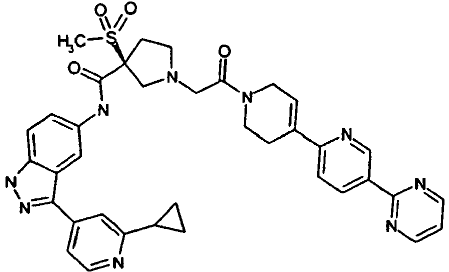 | 704.4 | 1.93 |
| 639 | 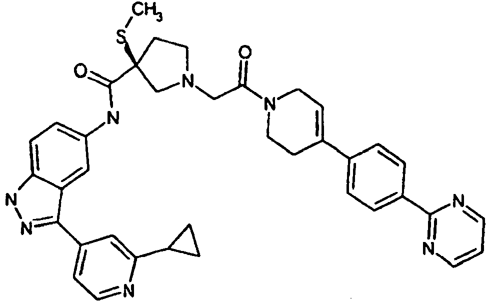 | 671.4 | 2.31 |
| 640 | 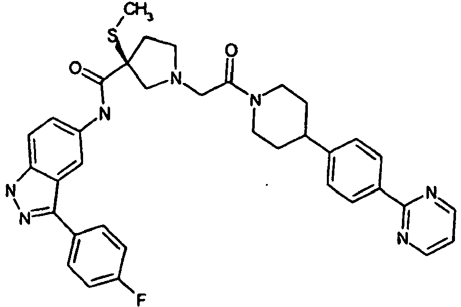 | 650.4 | 2.8 |
| 641 | 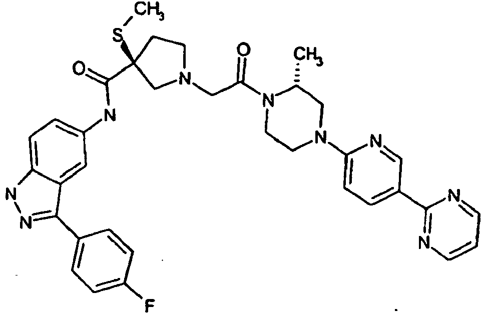 | 666.4 | 2.37 |
| 642 | 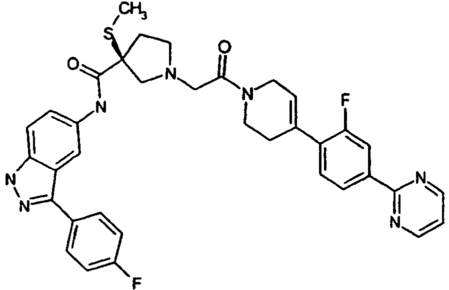 | 666.4 | 3.14 |
| 643 | 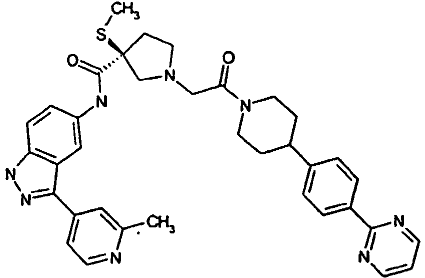 | 647.4 | 2.16 |
| 644 | 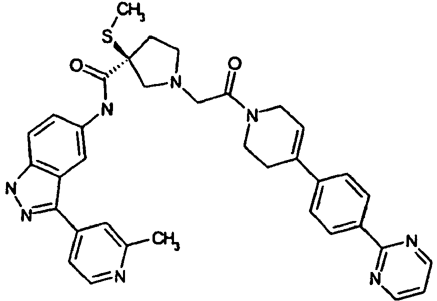 | 645.4 | 2.03 |
| 645 | 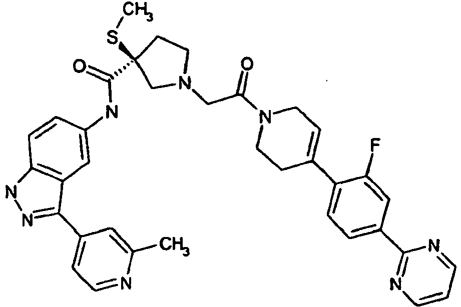 | 663.4 | 2.14 |
| 646 | 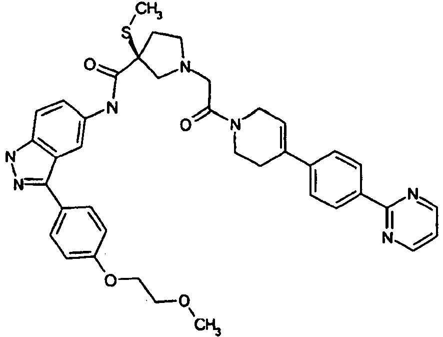 | 704.4 | 2.72 |
| 647 | 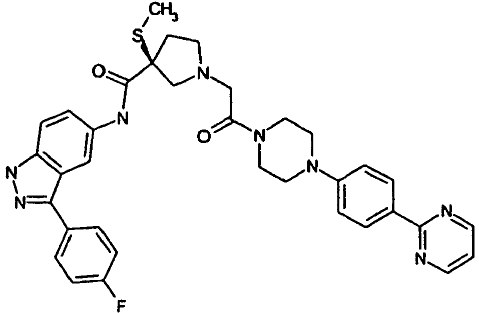 | 651.4 | 2.74 |
| 648 | 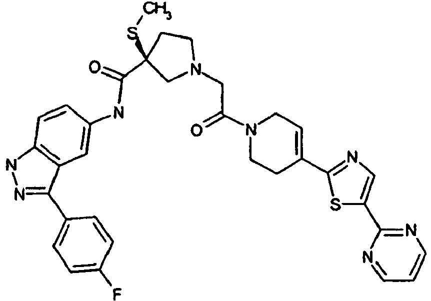 | 655.4 | 2.72 |
| 649 | 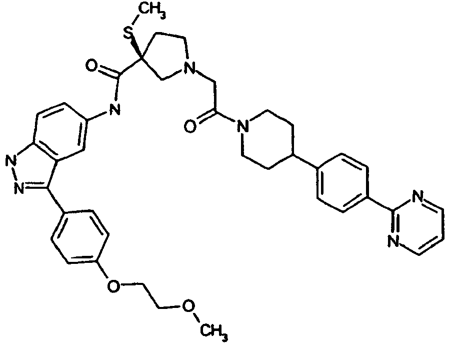 | 706.4 | 2.8 |
| 650 | 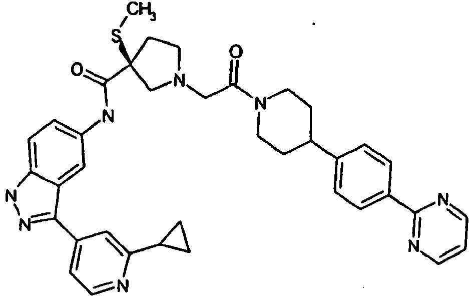 | 673.4 | 2.29 |
| 651 | 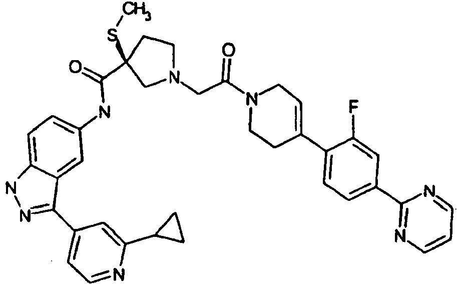 | 689.4 | 2.4 |
| 652 | 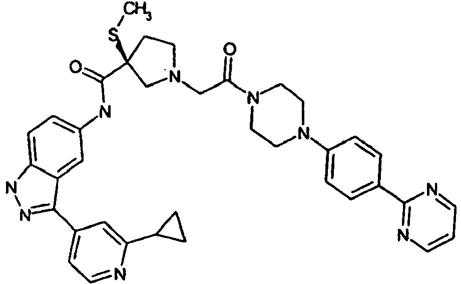 | 674.4 | 2.17 |
| 653 | 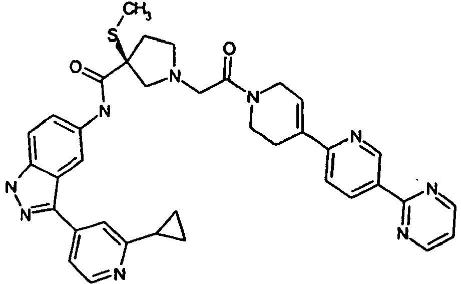 | 672.4 | 2.01 |
| 654 | 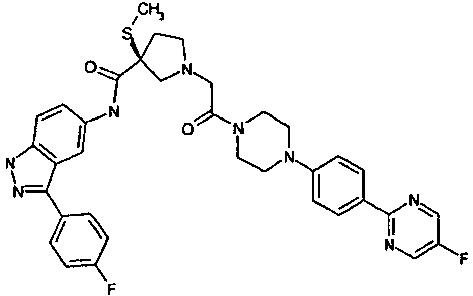 | 669.4 | 3.16 |
| 655 | 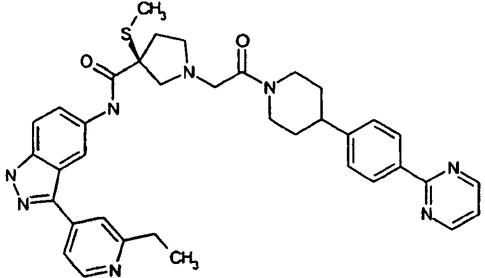 | 661.4 | 2.82 |
| 656 | 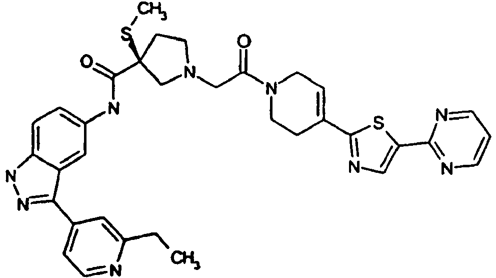 | 666.3 | 2.66 |
| 657 | 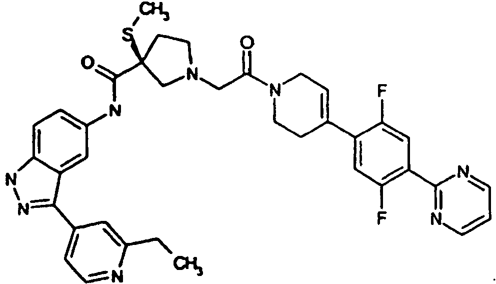 | 695.3 | 2.83 |
| 658 | 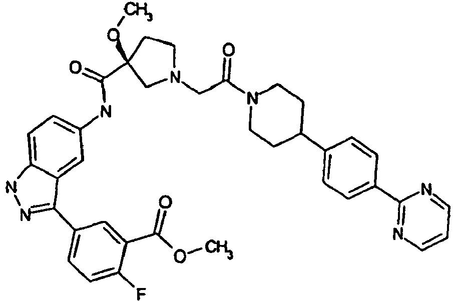 | | |
| | (see also Preparation 46) | 692.4 | 2.92 |
| 659 | 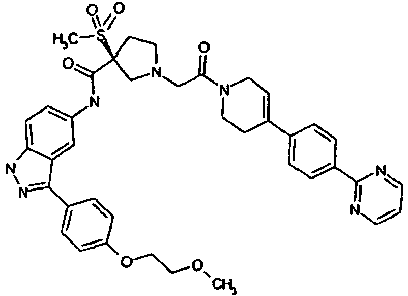 | 736.4 | 2.8 |
| 660 | 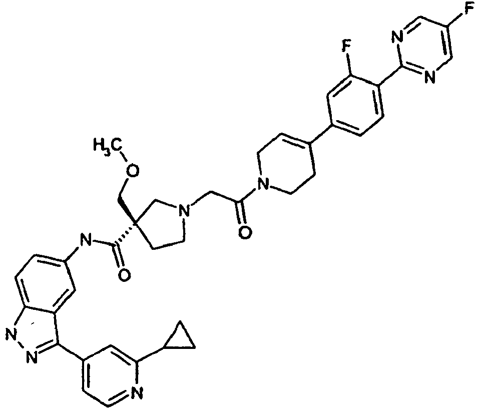 | | |
| | (see also Example 618) | 705.4 | 2.51 |
| 661 | 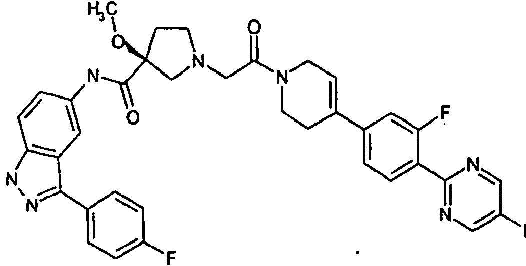 | 668.4 | 3.06 |
| 662 | 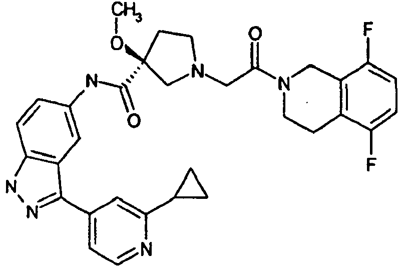 | | |
| | (see also Preparation 47) | 587.3 | 2.46 |
| 663 | 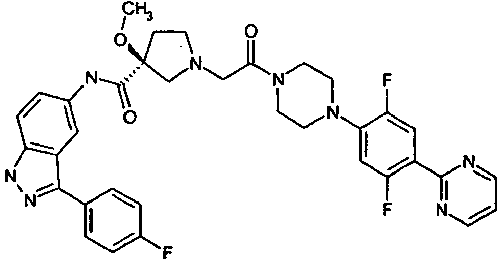 | | |
| | (see also Preparation 49) | 671.4 | 2.8 |
| 664 | 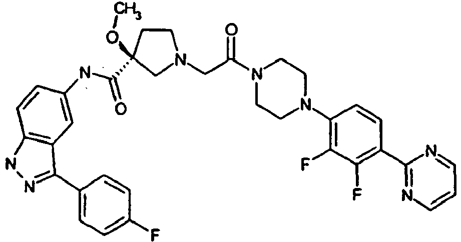 | | |
| | (see also Preparation 48) | 671.4 | 2.78 |
| 665 | 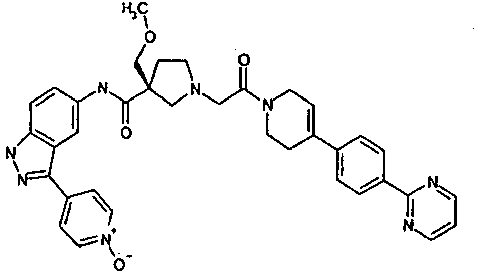 | 645.4 | 2.48 |
| 666 | 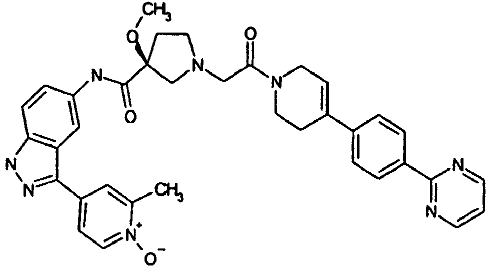 | | |
| | (see also Example 620) | 645.4 | 2.31 |
| 667 | 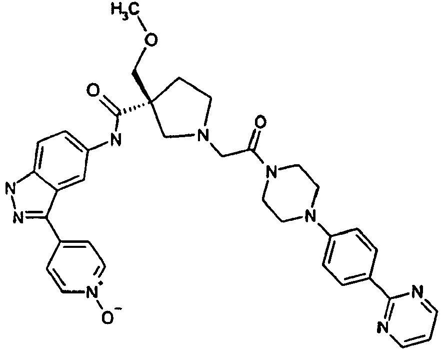 | 648.4 | 2.15 |
| 668 | 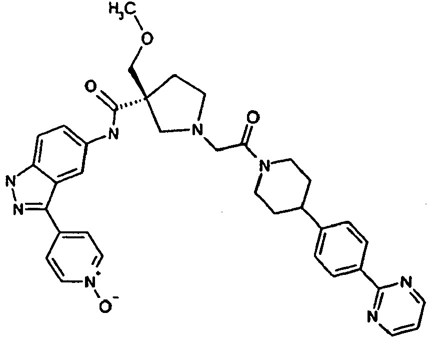 | 647.4 | 2.28 |
| 669 | 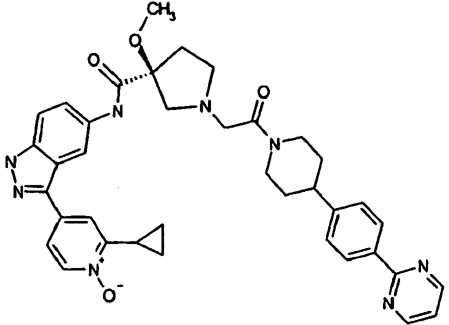 | 673.4 | 2.56 |
| 670 | 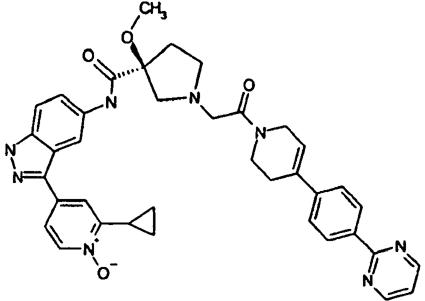 | 671.4 | 2.58 |
| 671 | 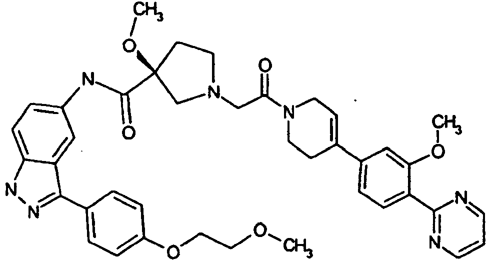 | | |
| | (see also Preparation 50) | 718.4 | 2.67 |
| 672 | 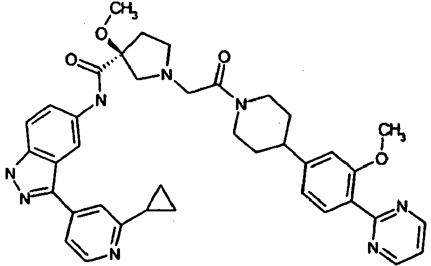 | 687.4 | 2.18 |
| 673 | 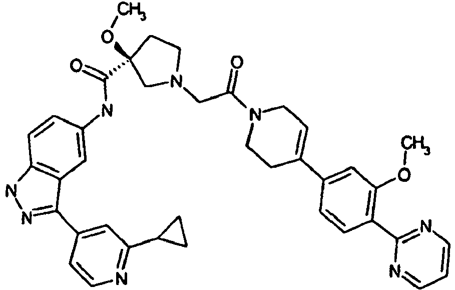 | 685.4 | 2.13 |
| 674 | 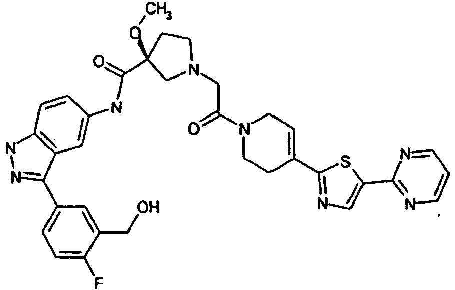 | | |
| | (see also Preparation 51) | 669.4 | 2.45 |
| 675 | 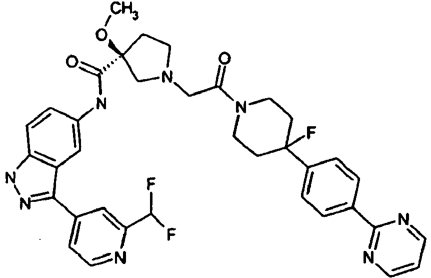 | | |
| | (see also Preparation 52) | 685.4 | 2.98 |
| 676 | 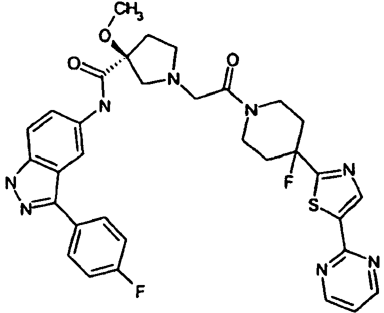 | | |
| | (see also Preparation 54) | 659.4 | 2.86 |
| 677 | 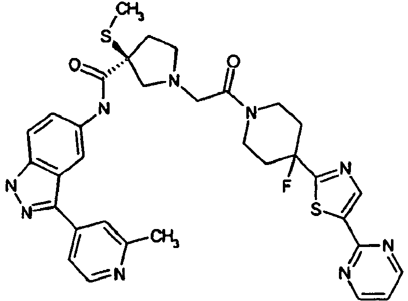 | 672.4 | 2.01 |
| 678 | 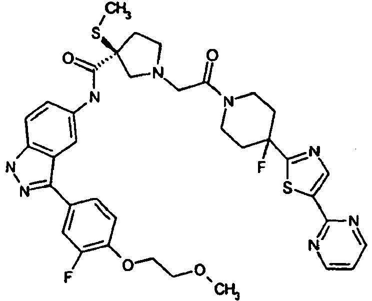 | 749.4 | 2.9 |
| 679 | 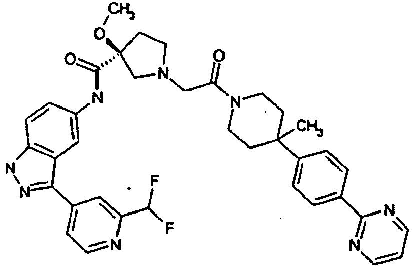 | 681.4 | 2.85 |
| 680 | 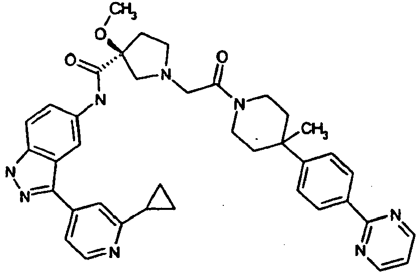 | | |
| | (see also Preparation 55) | 671.4 | 2.47 |
| 681 | 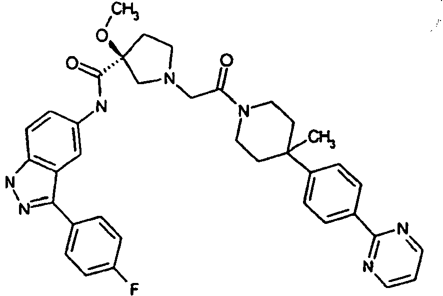 | 648.4 | 2.87 |
| 682 | 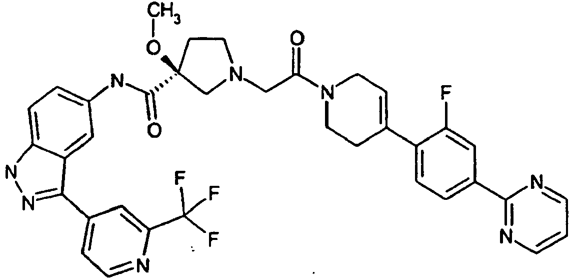 | 701.4 | 3.36 |
| 683 | 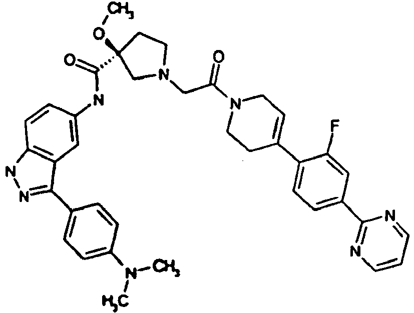 | 675.4 | 2.33 |
| 684 | 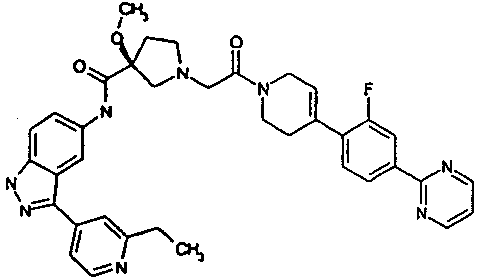 | 661.7 | 3.21 |
| 685 | 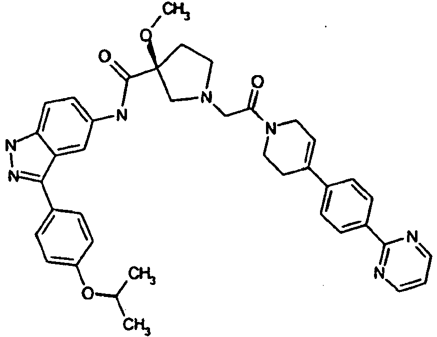 | | |
| | (see also Preparation 56) | 623 | 2.41 |
| 686 | 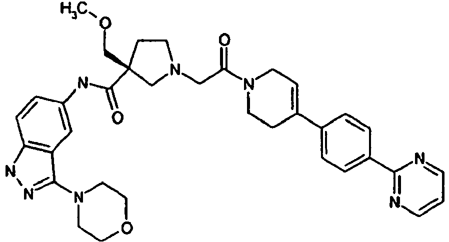 | 637 | 2.65 |
| 687 | 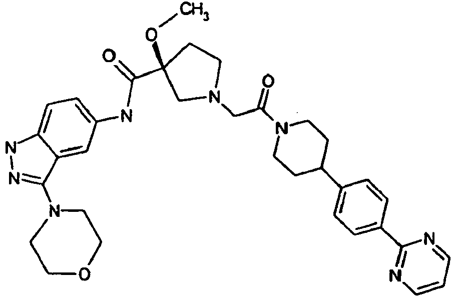 | 625 | 2.47 |
| 688 | 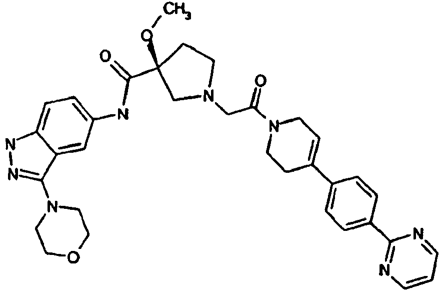 | 672 | 3.08 |
| 689 | 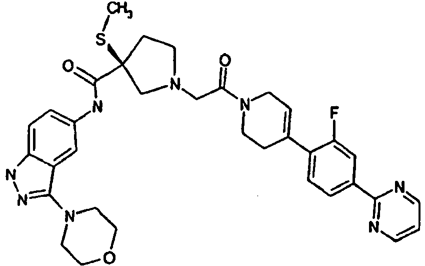 | 657 | 2.63 |
| 690 | 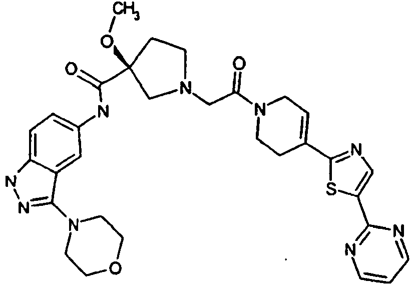 | 630 | 2.18 |
| 691 | 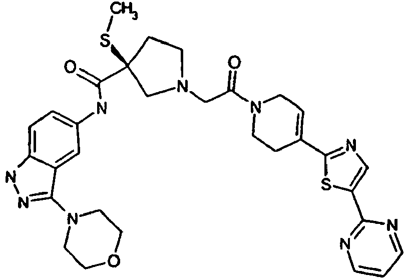 | 646 | 2.29 |
| 692 | 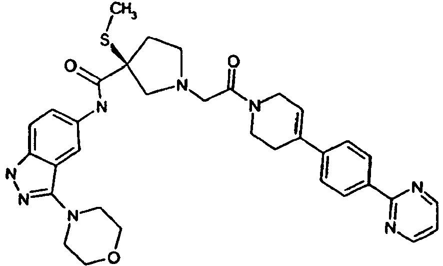 | 639 | 2.46 |
| 693 | 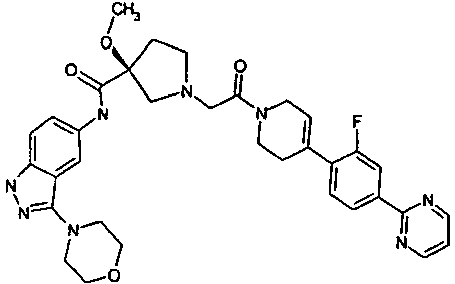 | 641 | 2.51 |
| 694 | 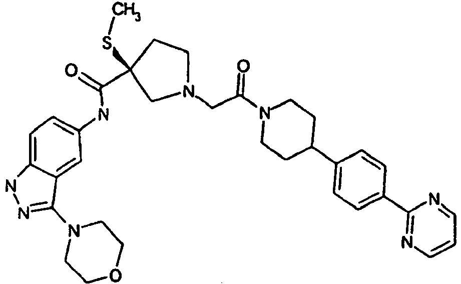 | 641 | 2.49 |
| 695 | 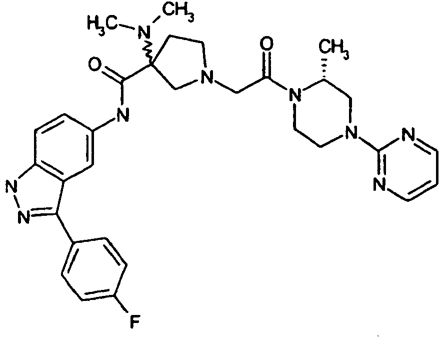 | 586 | 2.62 |
| 696 | 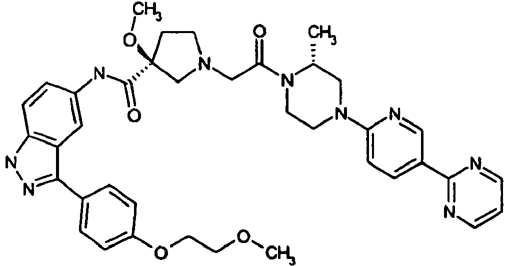 | | |
| | (see also Preparation 57) | 706 | 2.46 |
| 697 | 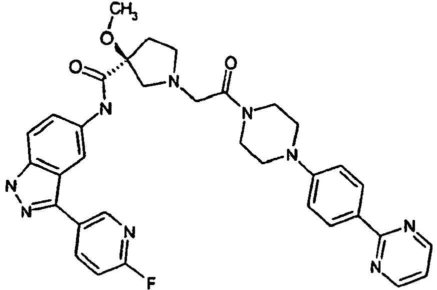 | 636.7 | 3.12 |
| 698 | 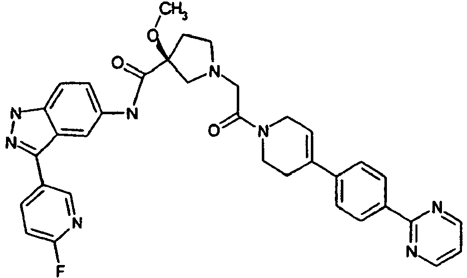 | 633.7 | 3.46 |
| 699 | 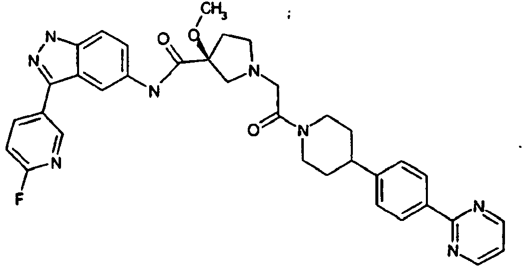 | 635.7 | 3.33 |
| 700 | 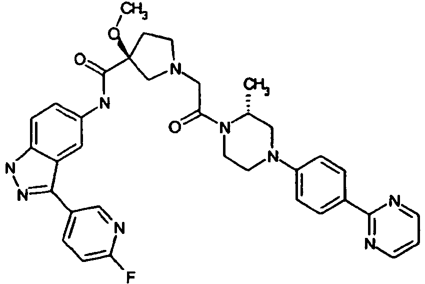 | | |
| | (see also Preparation 58) | 650.7 | 3.24 |
| 701 | 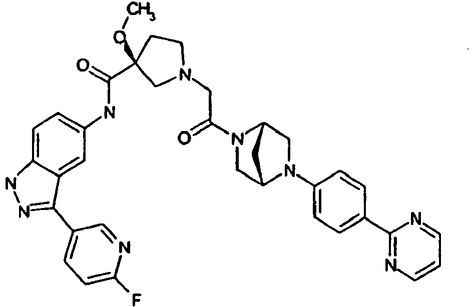 | 648.7 | 3.05 |
| 702 | 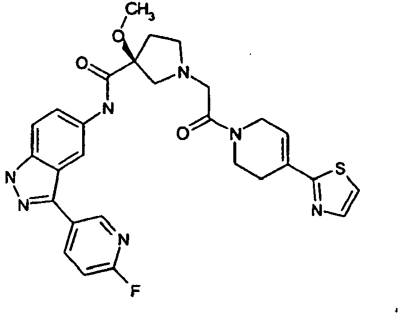 | 562.6 | 2.83 |
| 703 | 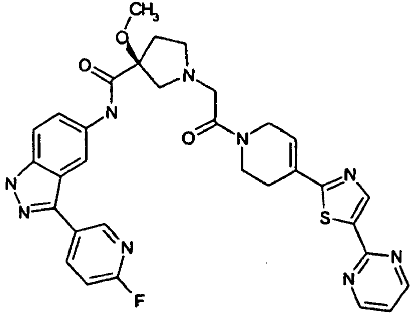 | 640.7 | 3.12 |
| 704 | 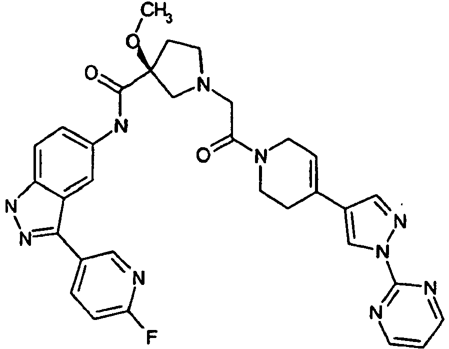 | | |
| | (see also Preparation 59) | 623.7 | 2.88 |
| 705 | 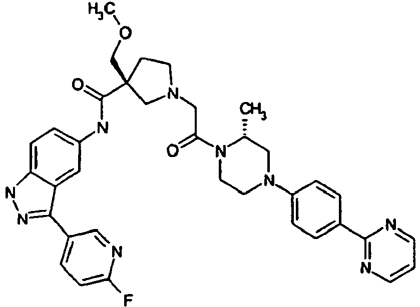 | 664.7 | 3.4 |
| 706 | 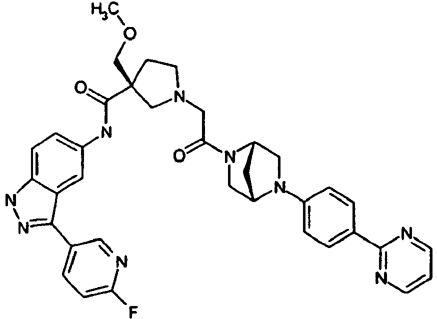 | 662.7 | 3.16 |
| 707 | 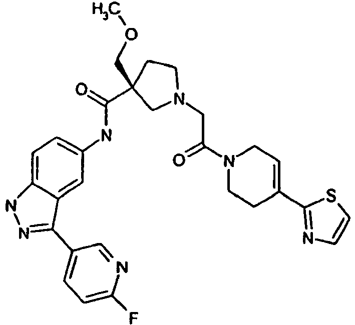 | 576.7 | 2.97 |
| 708 | 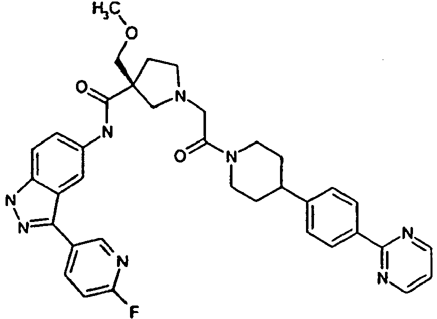 | 649.7 | 3.45 |
| 709 | 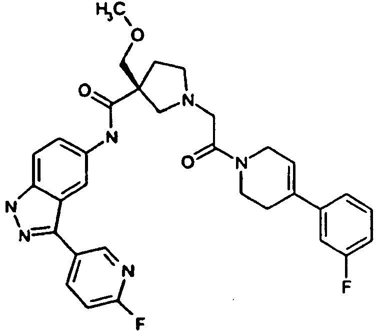 | 587.6 | 3.58 |
| 710 | 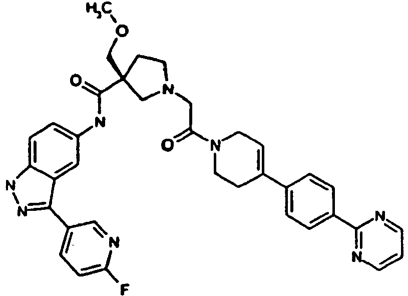 | 647.7 | 3.49 |
| 711 | 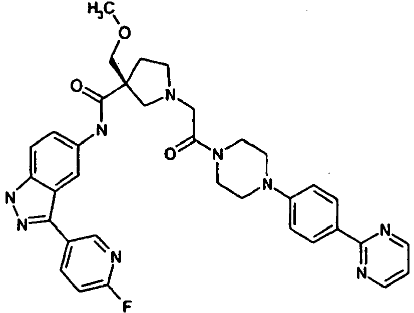 | 650.7 | 3.23 |
| 712 | 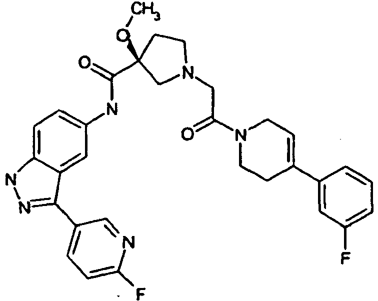 | 573.6 | 3.45 |
| 713 | 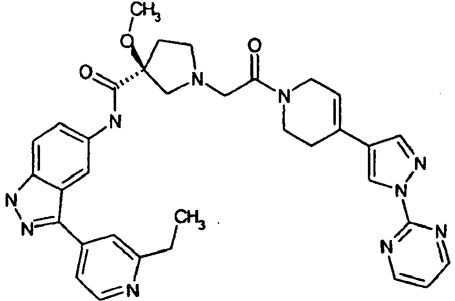 | 633.7 | 2.34 |
| 714 | 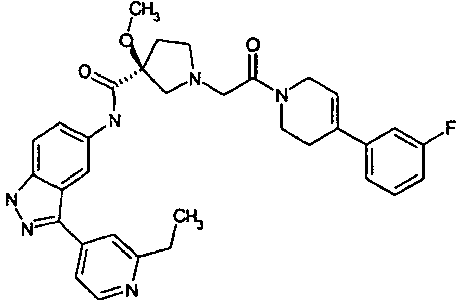 | | |
| | (see also Preparation 60) | 583.7 | 2.81 |
| 715 | 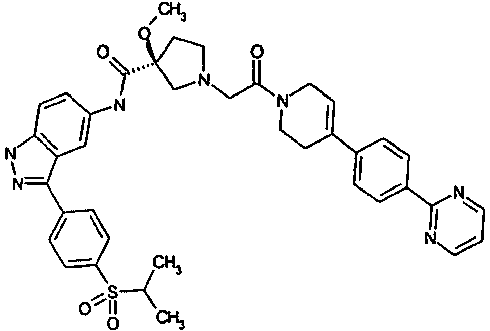 | | |
| | (see also Preparation 62) | 602.8 | 3.65 |
| 716 | 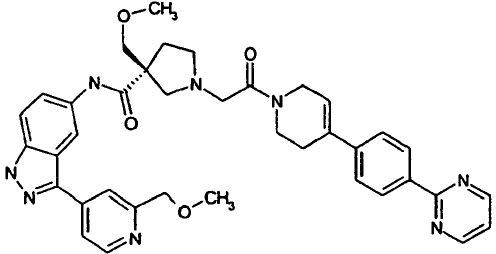 | | |
| | (see also Preparation 63) | 709.8 | 2.62 |
| 717 | 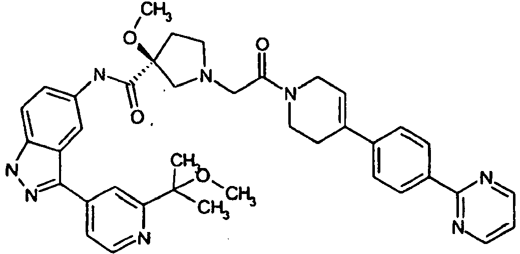 | | |
| | (see also Preparation 64) | 683.9 | 3 |
| 718 | 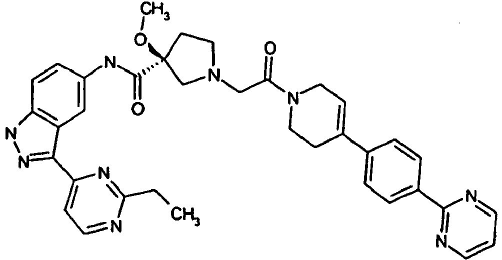 | | |
| | (see also Preparation 65) | 676.8 | 3.09 |
| 719 | 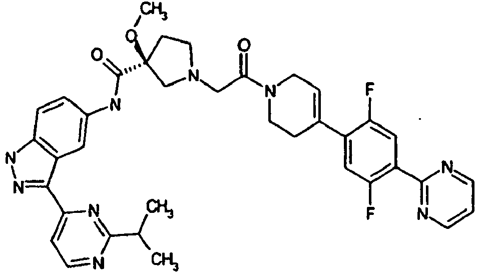 | | |
| | (see also Preparation 65) | 694.3 | 3.71 |
| 720 | 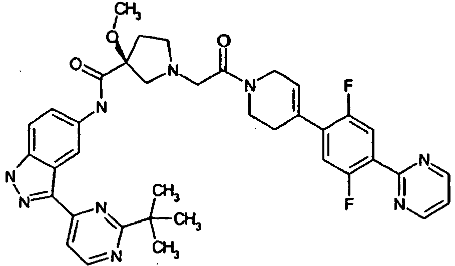 | | |
| | (see also Preparation 65) | 708.4 | 4.08 |
| 721 | 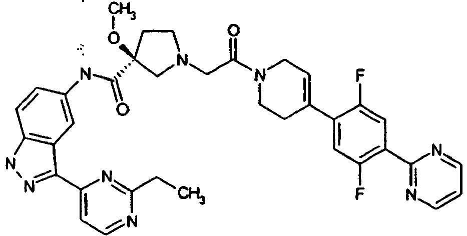 | | |
| | (see also Preparation 65) | 680.3 | 3.09 |
| 722 | 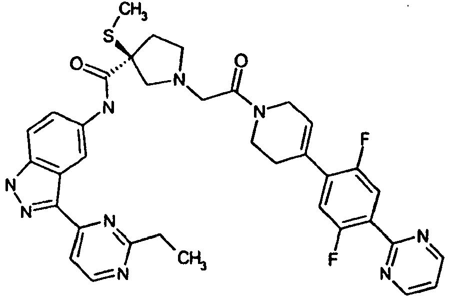 | 696.2 | 3.52 |
| 723 | 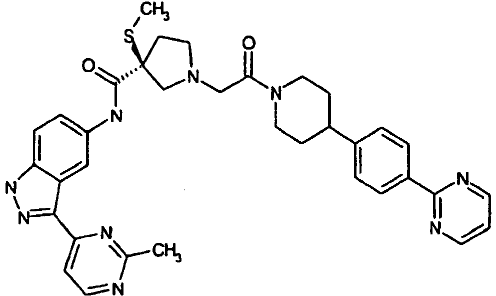 | 648.3 | 2.98 |
| 724 | 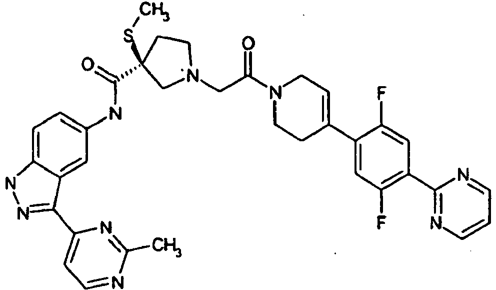 | 682.2 | 3.01 |
| 725 | 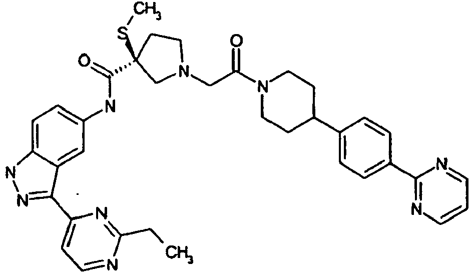 | 667.3 | 3.12 |
| 726 | 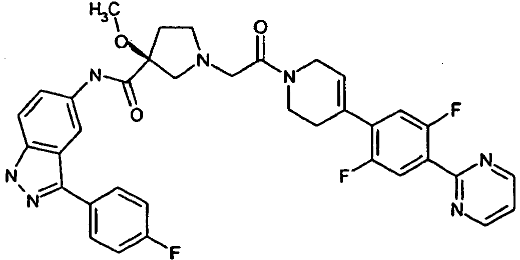 | | |
| | (see also Preparation 66) | 668.4 | 3.21 |
| 727 | 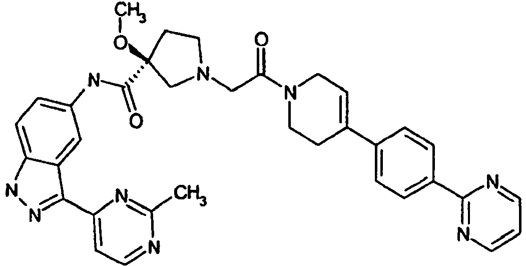 | 630.3 | 3.29 |
| 728 | 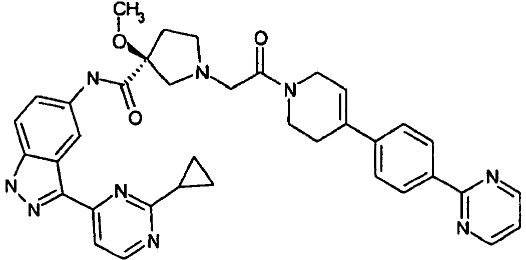 | 656 | 3.22 |
| 729 | 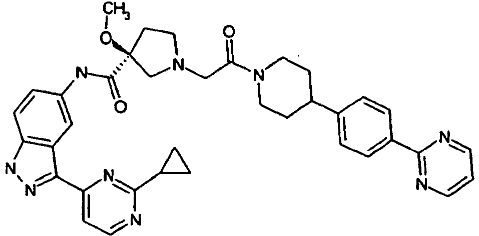 | 658.3 | 3.38 |
| 730 | 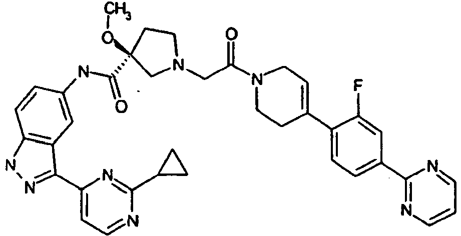 | 674 | 3.84 |
| 731 | 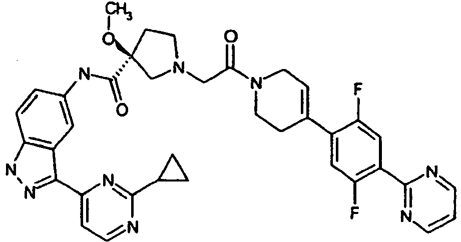 | 692 | 3.41 |
| 732 | 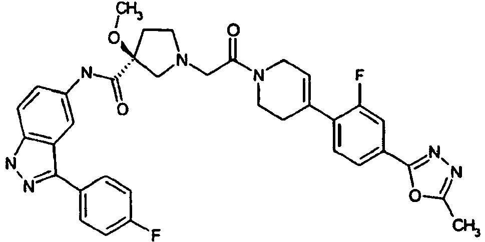 | | |
| | (see also Preparation 84) | 654.4 | 3.02 |
| 733 | 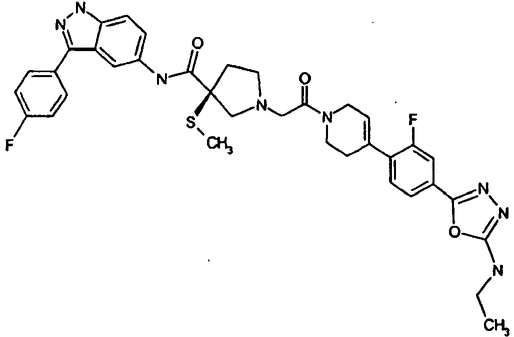 | 699.4 | 2.9 |
| 734 | 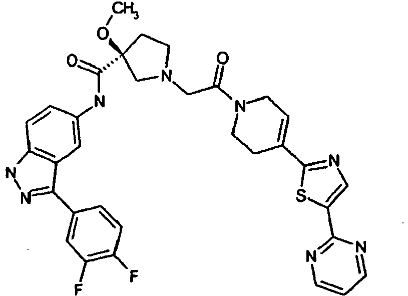 | | |
| | (see also Preparation 67) | 657.4 | 2.73 |
| 735 | 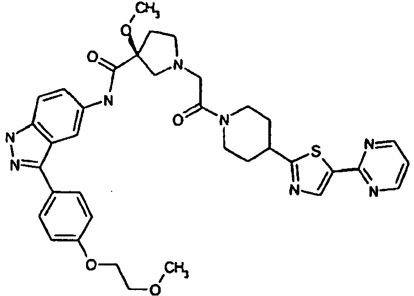 | | |
| | (see also Preparation 67) | 697.3 | 3.31 |
| 736 | 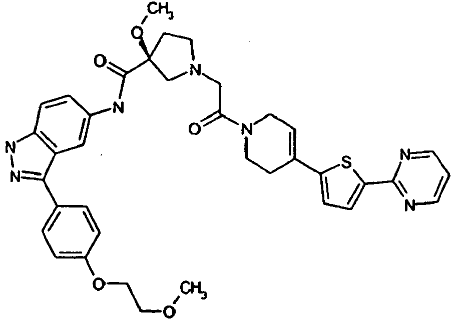 | | |
| | (see also Preparation 68) | 694.2 | 4.07 |
| 737 | 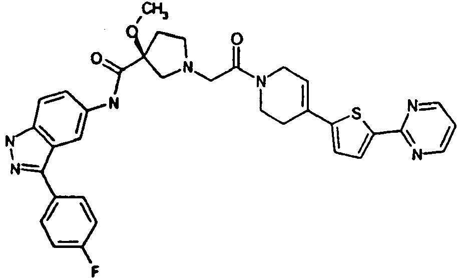 | 638.3 | 3.56 |
| 738 | 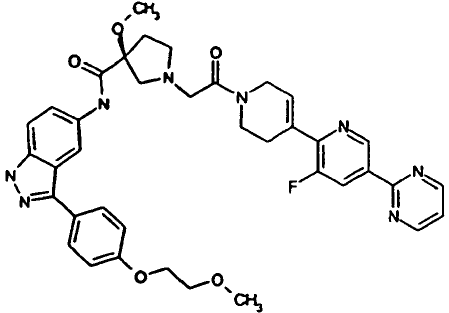 | 707.3 | 3.57 |
| 739 | 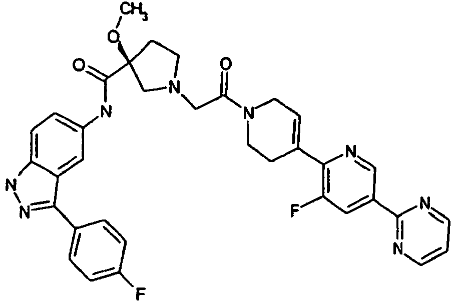 | 651.3 | 3.69 |
| 740 | 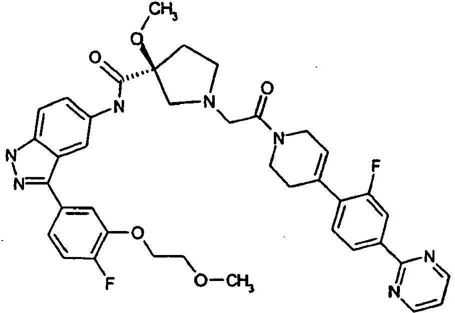 | | |
| | (see also Preparation 69) | 724.4 | 3.16 |
| 741 | 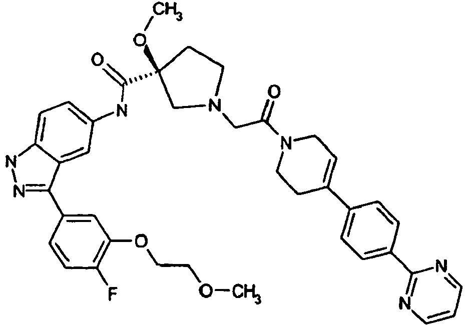 | 706.4 | 3.24 |
| 742 | 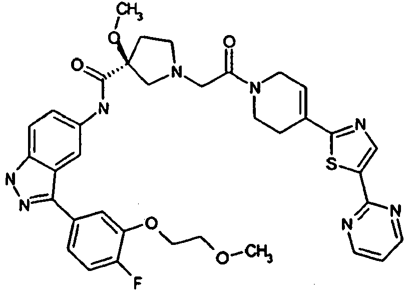 | 713.4 | 2.7 |
| 743 | 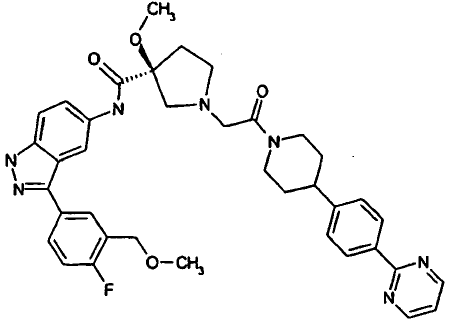 | | |
| | (see also Preparation 70) | 678.4 | 3.2 |
| 744 | 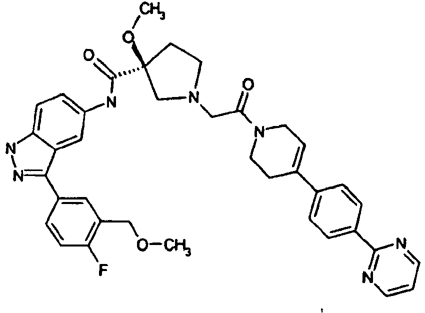 | 676.4 | 3.18 |
| 745 | 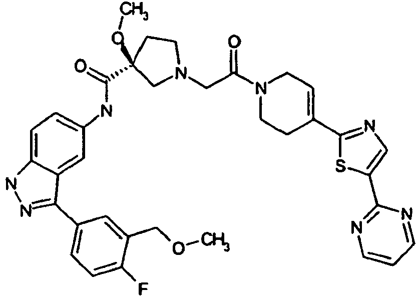 | 683.4 | 2.71 |
| 746 | 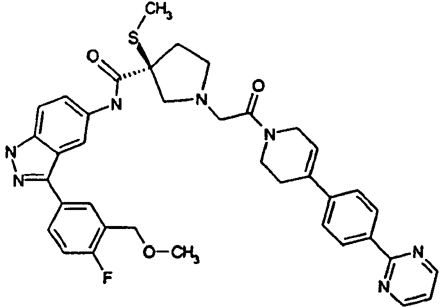 | 692.4 | 2.91 |
| 747 | 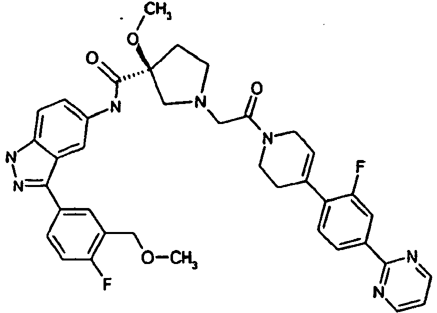 | 694.4 | 2.91 |
| 748 | 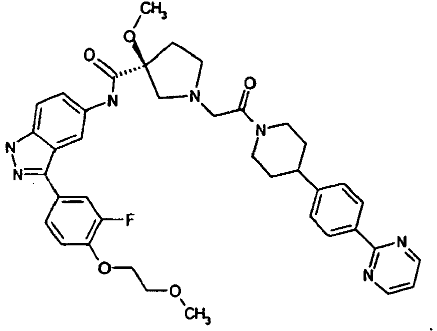 | 708.4 | 2.81 |
| 749 | 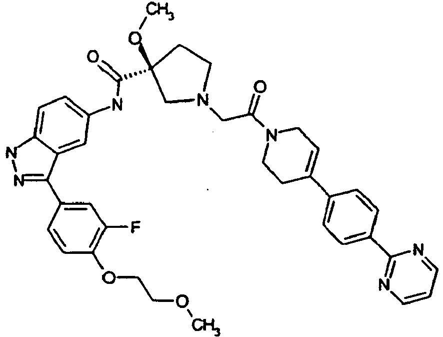 | | |
| | (see also Preparation 71) | 706.4 | 2.85 |
| 750 | 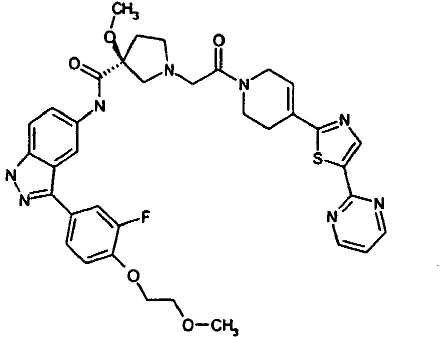 | 713.4 | 2.68 |
| 751 | 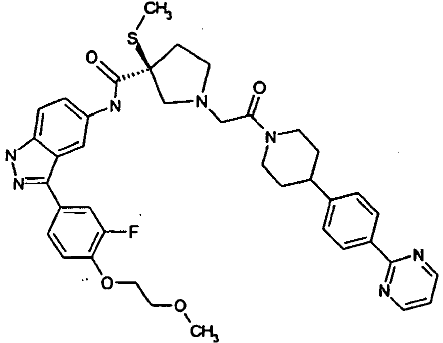 | 724.4 | 2.94 |
| 752 | 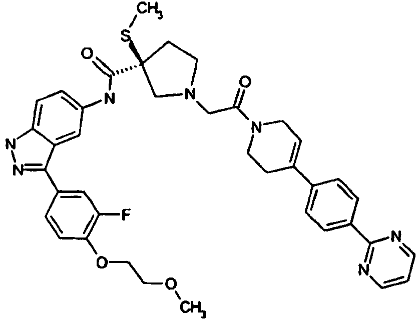 | 722.4 | 2.97 |
| 753 | 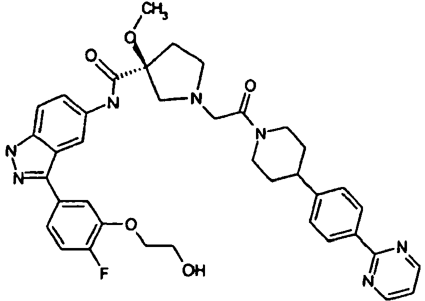 | | |
| | (see also Preparation 72) | 694.4 | 2.62 |
| 754 | 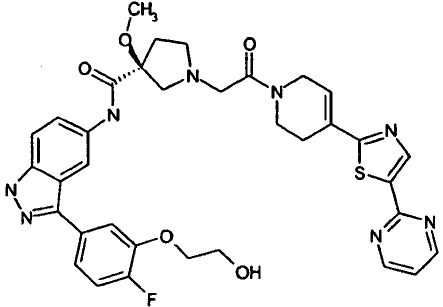 | 699.4 | 2.44 |
| 755 | 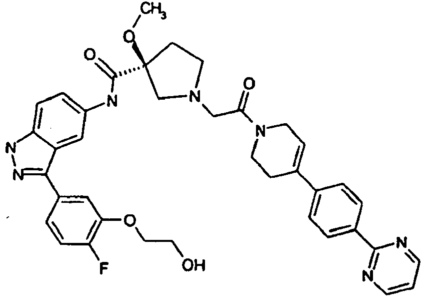 | 692.4 | 2.65 |
| 756 | 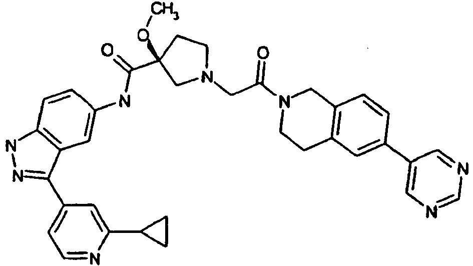 | | |
| | (see also Preparation 73) | 629.7 | 2.57 |
| 757 | 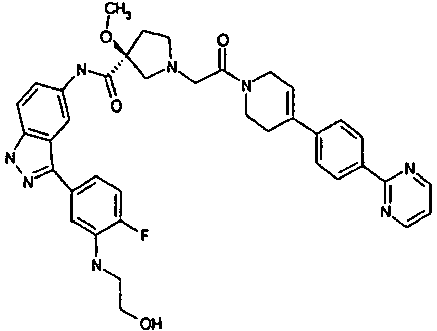 | | |
| | (see also Preparation 74) | 691.2 | 3.54 |
| 758 | 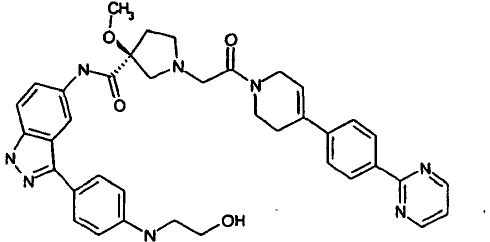 | 673.2 | 2.92 |
| 759 | 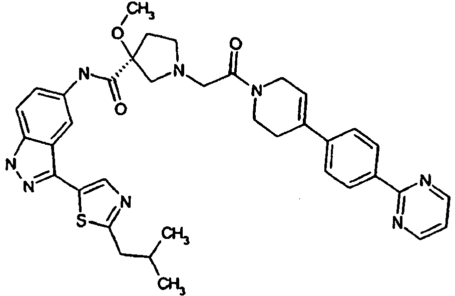 | | |
| | (see also Preparation 76) | 677.2 | 3.93 |
| 760 | 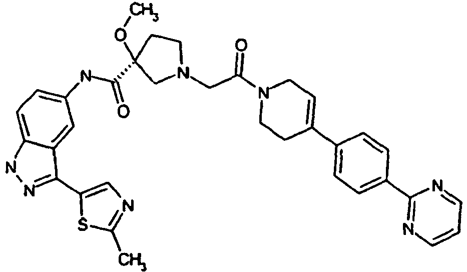 | | |
| | (see also Preparation 77) | 635.2 | 3.33 |
| 761 | 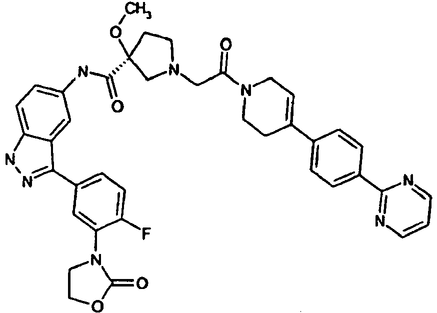 | | |
| | (see also Preparation 78) | 717.2 | 3.8 |
| 762 | 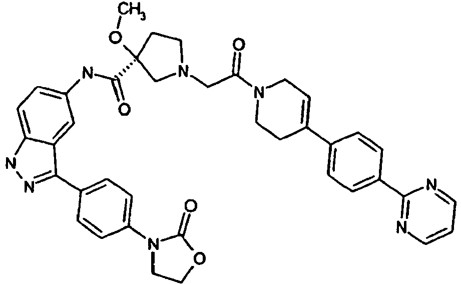 | | |
| | (see also Preparation 79) | 699.7 | 3.31 |
| 763 | 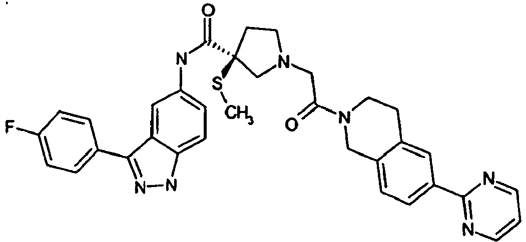 | | |
| | (see also Preparation 80) | 622.3 | 3.52 |
| 764 | 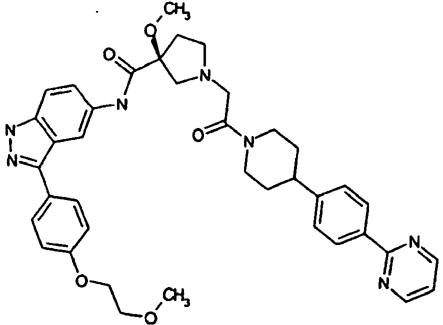 | 690.3 | 3.8 |
| 765 | 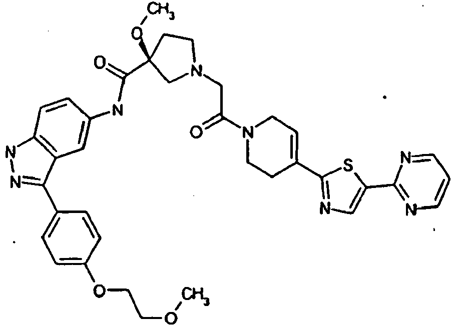 | 695.3 | 3.56 |
| 766 | 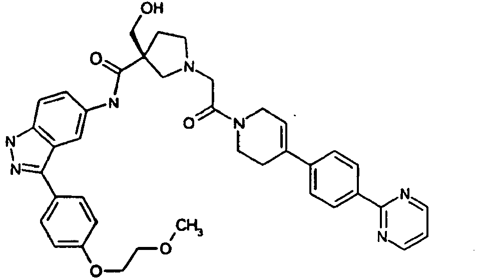 | 688.4 | 3.43 |
| 767 | 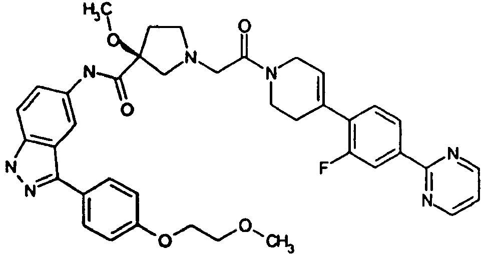 | 706.4 | 3.05 |
| 768 | 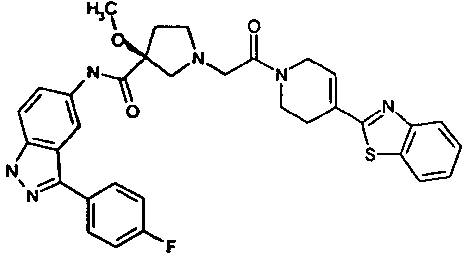 | | |
| | (see also Preparation 81) | 611.3 | 3.17 |
| 769 | 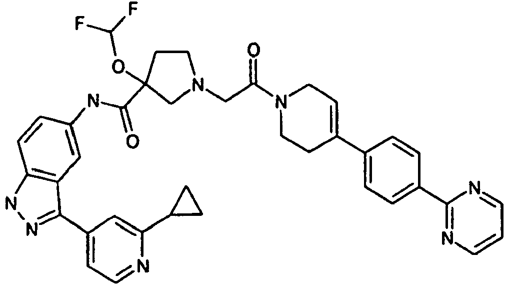 | 691.4 | 2.44 |
| 770 | 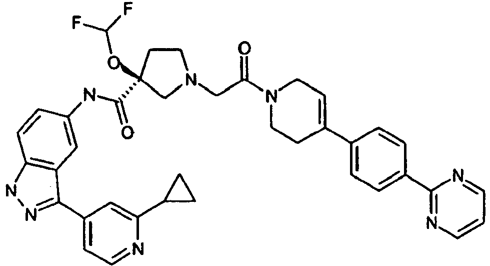 | | |
| | (see also Preparation 82) | 691.4 | 2.31 |
| 771 | 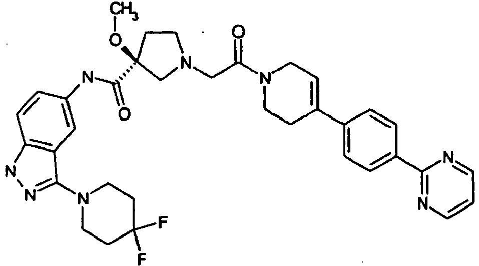 | | |
| | (see also Preparation 83) | 657.4 | 2.78 |
| 772 | 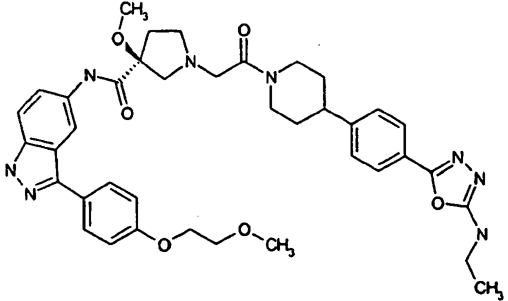 | 723.4 | 2.64 |
| 773 | 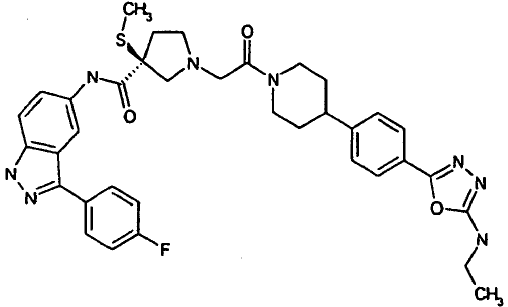 | 683.4 | 2.67 |
| 774 | 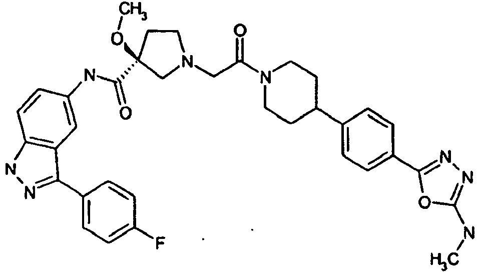 | | |
| | (see also Example 624) | 653.4 | 2.54 |
| 775 | 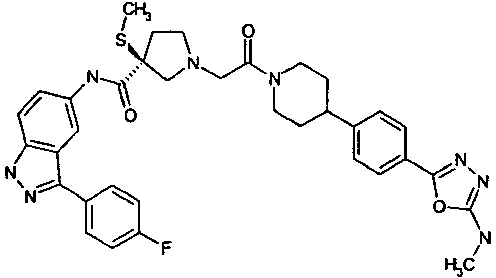 | 669.4 | 2.58 |
| 776 | 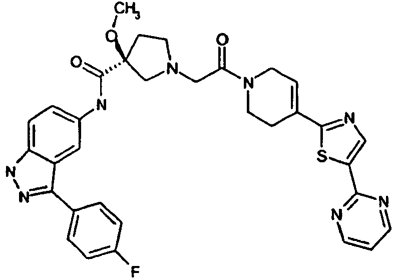 | 639.7 | 3.41 |
| 777 | 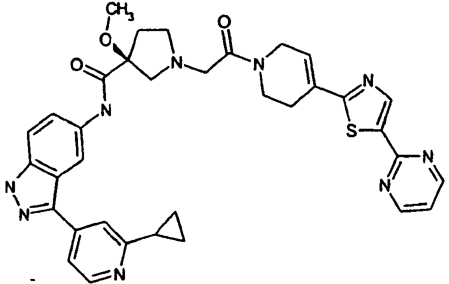 | 662 | 2.72 |
| | (see also Preparation 67) | | |
| 778 | 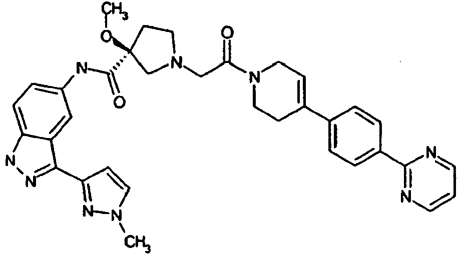 | 618 | 3.19 |
| | (see also Preparation 85) | | |
| 779 | 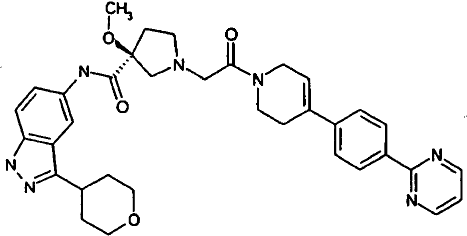 | 622 | 3.16 |
| | (see also Preparation 86) | | |
| 780 | 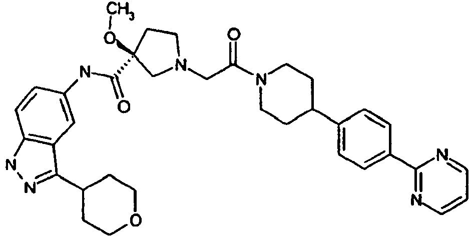 | 624 | 3.13 |
| 781 | 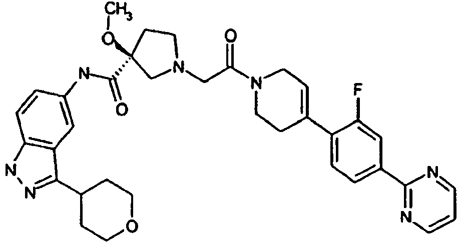 | 640 | 3.37 |
| 782 | 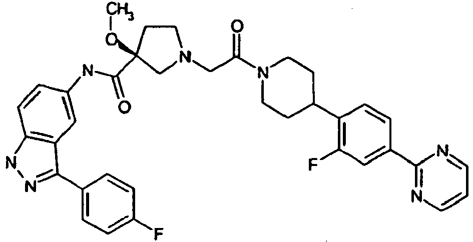 | 652.4 | 3.35 |
| 783 | 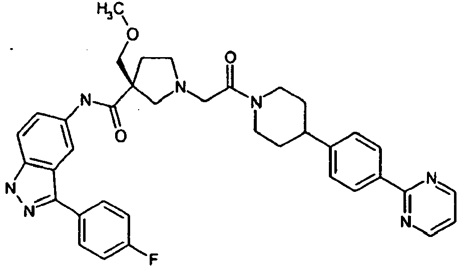 | 648.4 | 3.11 |
| 784 | 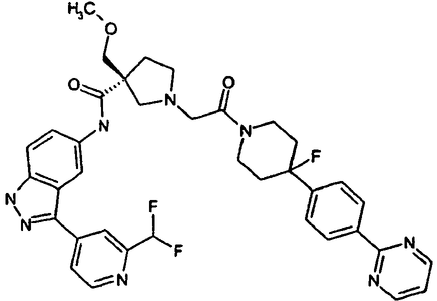 | 699.4 | 3 |
| 785 | 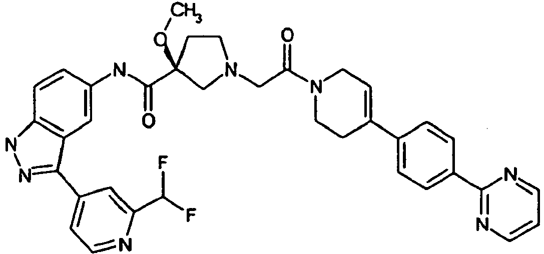 | 665.4 | 2.97 |
| 786 | 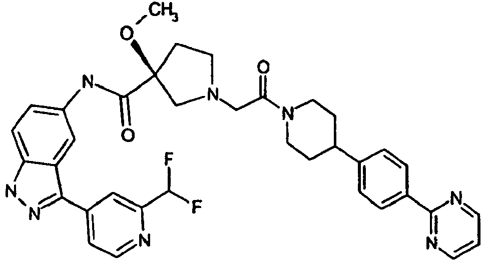 | 667.4 | 2.89 |
| 787 | 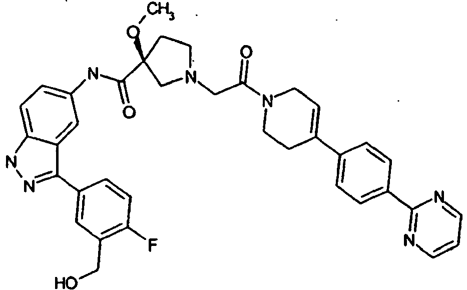 | 662.4 | 2.84 |
| 788 | 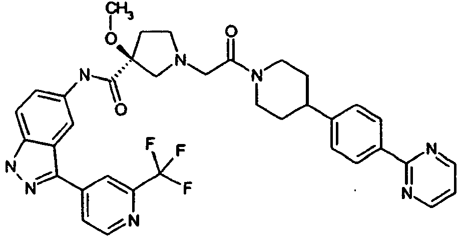 | 685.4 | 2.92 |
| 789 | 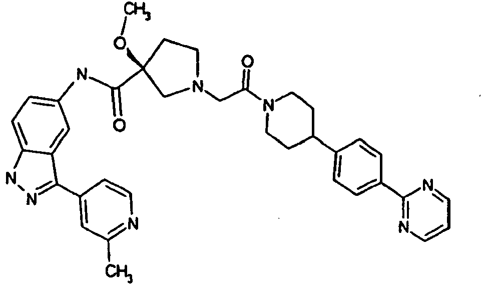 | 631.3 | 2.07 |
| 790 | 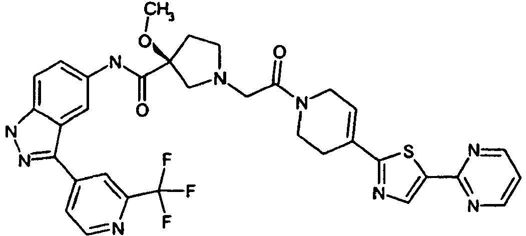 | 690.4 | 2.75 |
| 791 | 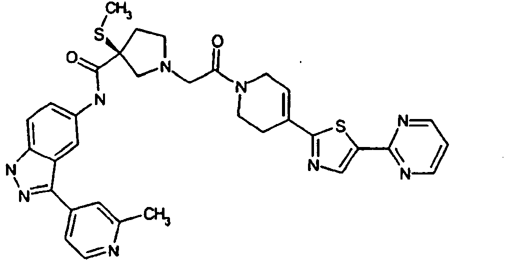 | 652.4 | 1.83 |
| 792 | 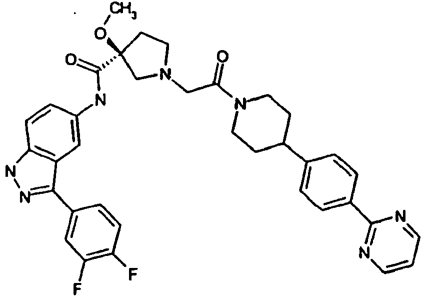 | 652.4 | 2.94 |
| 793 | 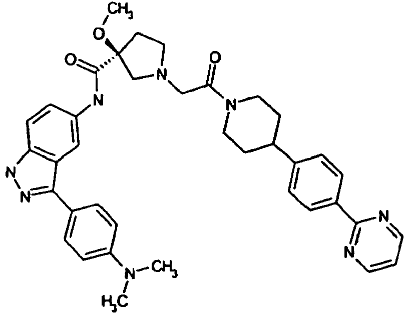 | 659.4 | 2.41 |
| 794 | 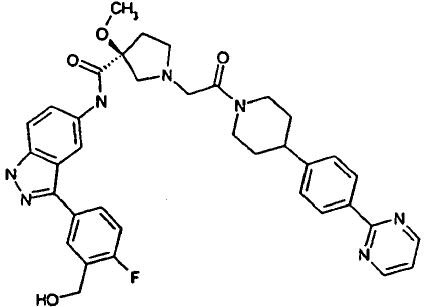 | 664.4 | 2.76 |
| 795 | 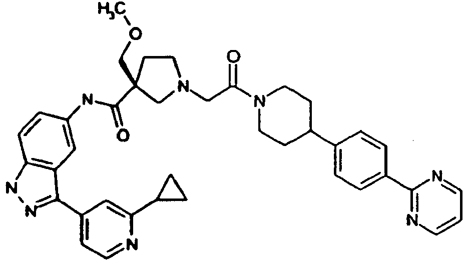 | 671 | 2.59 |
| 796 | 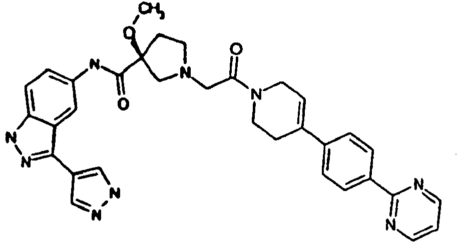 | 604 | 2.33 |
| 797 | 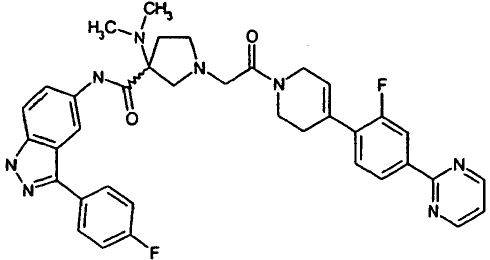 | 663 | 3.26 |
| 798 | 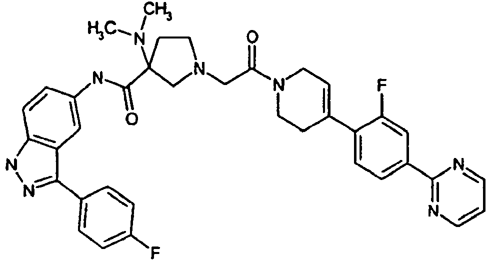 | 663 | 3.26 |
| 799 | 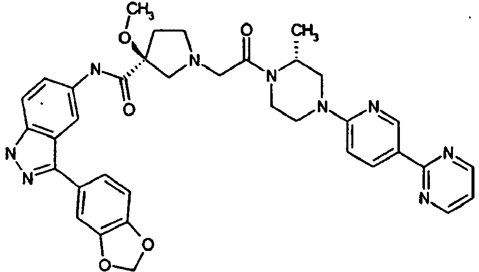 | 676 | 2.49 |
| 800 | 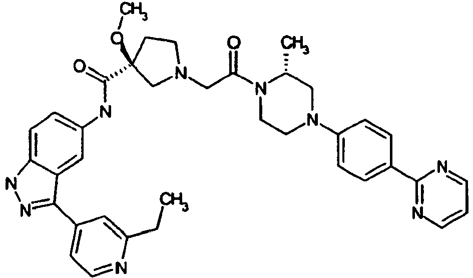 | 660.8 | 2.71 |
| 801 | 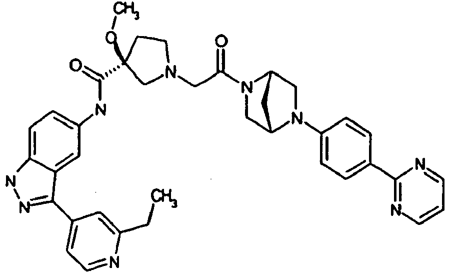 | 658.8 | 2.5 |
| 802 | 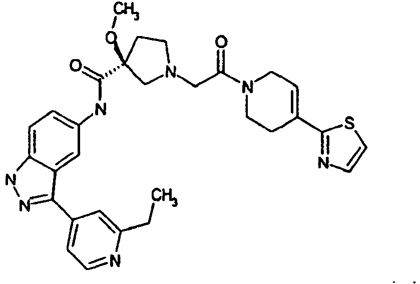 | 572.7 | 2.27 |
| 803 | 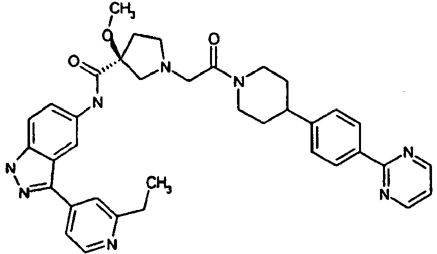 | 645.8 | 2.73 |
| 804 | 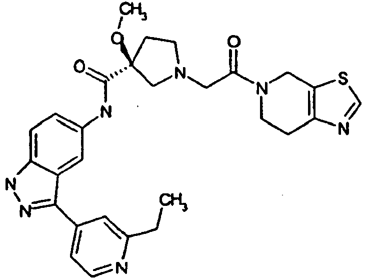 | 546.7 | 2.02 |
| 805 | 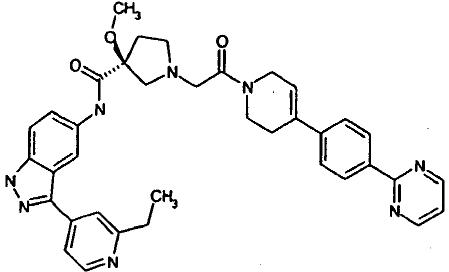 | 643.7 | 2.76 |
| 806 | 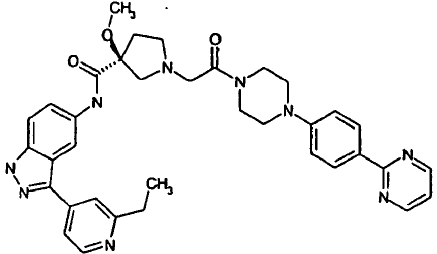 | 646.8 | 2.55 |
| 807 | 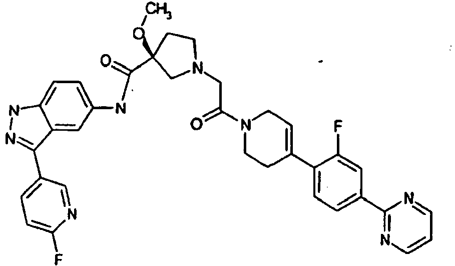 | 651.7 | 3.99 |
| 808 | 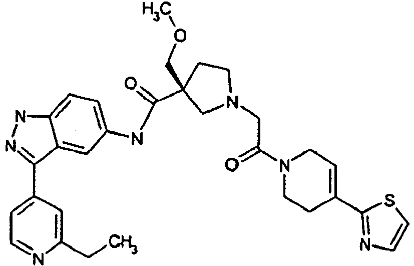 | 586.7 | 2.28 |
| 809 | 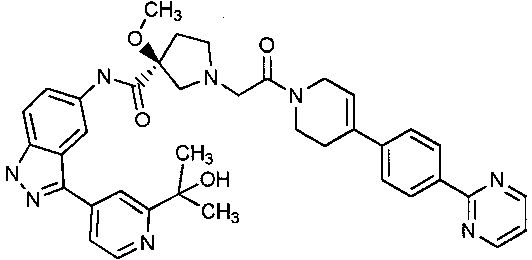 | 576.7 | 2.75 |
| 810 | 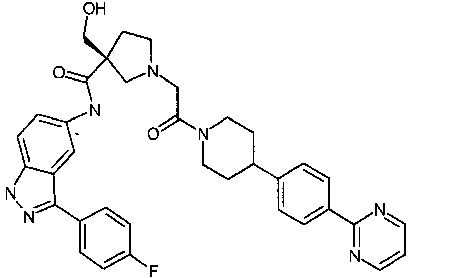 | 634.3 | 3.52 |
| 811 | 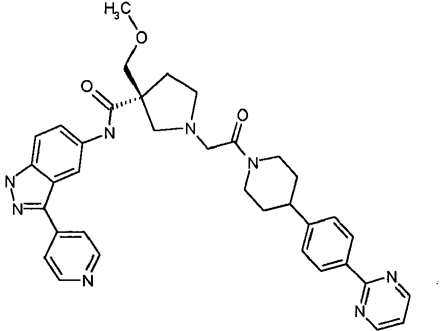 | 631.3 | 2.25 |
| 812 | 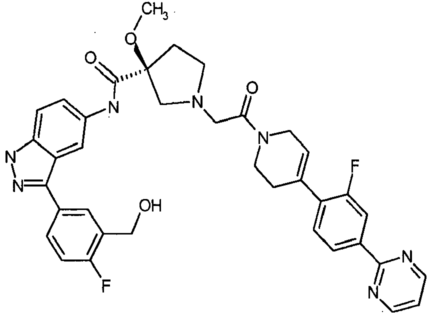 | 680.4 | 2.91 |
| 813 | 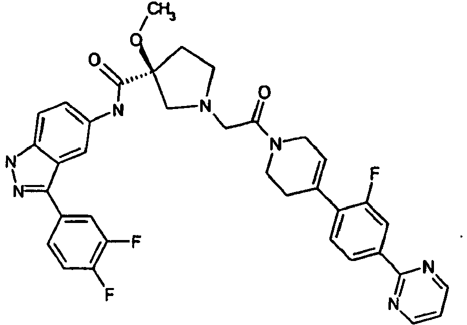 | 668.4 | 3.31 |
| 814 | 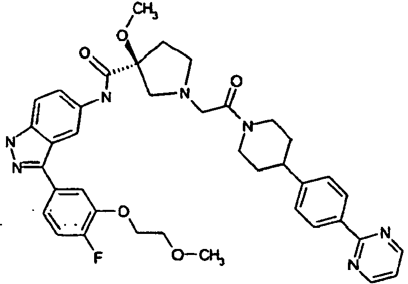 | 708.4 | 3.07 |
| 815 | 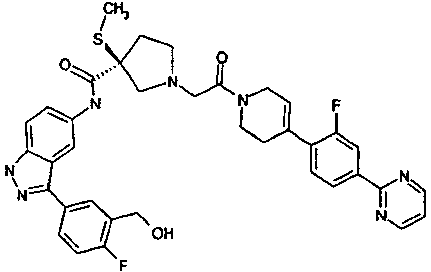 | 696.4 | 2.84 |
| 816 | 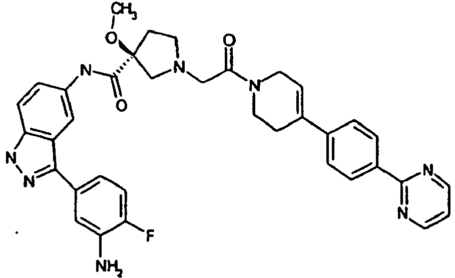 | 647.2 | 3.34 |
| 817 | 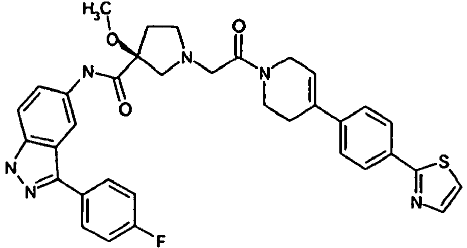 | 637.4 | 3.34 |
| 818 | 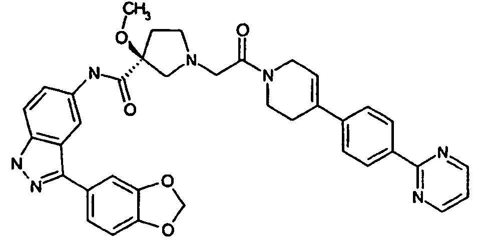 | | |
| | (see also Preparation 89) | 658.4 | 3.13 |
| 819 | 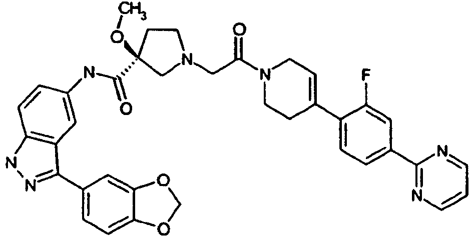 | 676.4 | 3.11 |
| 820 | 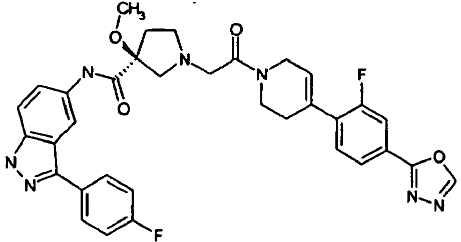 | 640.4 | 2.78 |
| 821 | 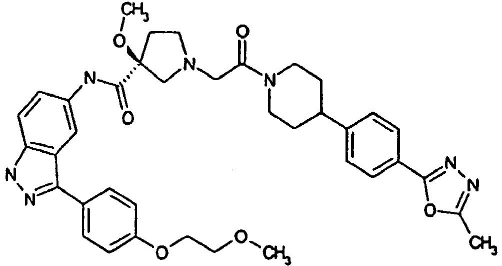 | 694.4 | 2.64 |
| 822 | 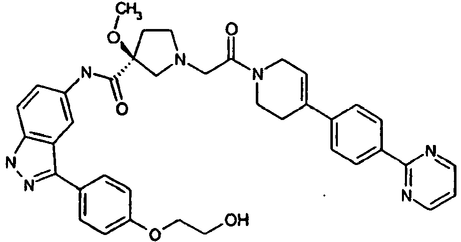 | 674.4 | 2.53 |
| 823 | 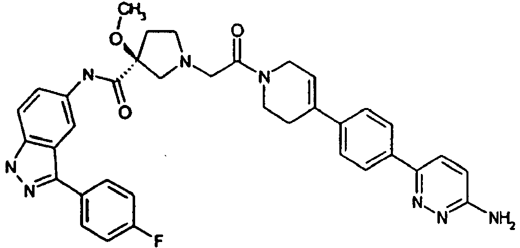 | 647.4 | 2.38 |
| 824 | 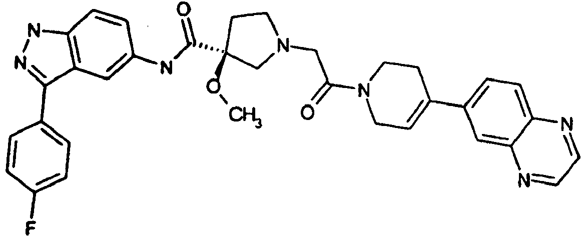 | 606 | 3.32 |
| | (see also Preparation 87) | | |

### Examples 826 and 827

### Synthesis of 5-Nitro-3-trimethylsilanylethynyl-1-trityl-1H-indazole:

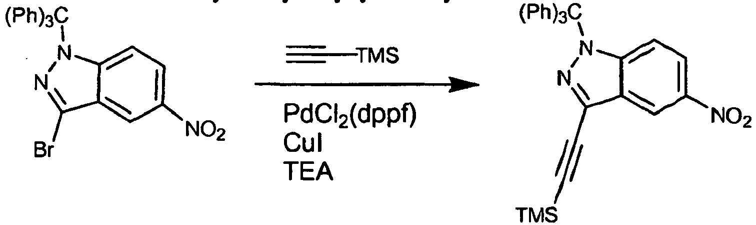

A mixture of 3-bromo-5-nitro-1-trityl-1H-indazole (4.83 g, 10 mmol), ethynyl-trimethylsilane (1g, 10 mmol), copper (I) iodide (91 mg, 0.5 mmol), PdCl₂(dppf) (337 mg, 0.5 mmol) and triethylamine (1.2 g, 12 mmol) in DMF (50 mL) was stirred overnight at 80 °C under argon. Then ethyl acetate (100 mL) was added, followed by water. The organic layer was collected and aqueous layer was extracted three times with ethyl acetate. The combined organic fraction was washed with water and then brine. The solvent was removed under vacuum and the product was purified using chromatography (20% hexane in dichloromethane) to give desired product (3.4 g) in 67% yield.

### Synthesis of 3-ethynyl-5-nitro-1-trityl-1H-indazole:

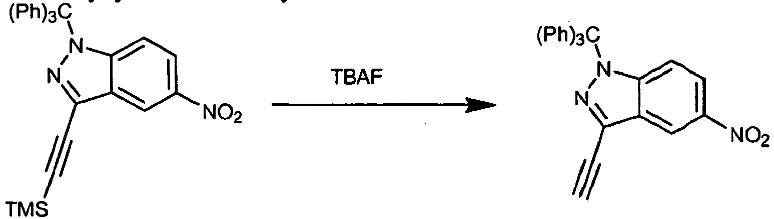

To a solution of 5-nitro-3-trimethylsilanylethynyl-1-trityl-1H-indazole (700 mg, 1.4 mmol) in THF (5 mL) was added tributylammonium fluoride (1M, 2.8 mL, 2.8 mmol). The resulted solution was stirred at room temperature for 2 hours. TLC showed the completion of reaction. After removal the solvent under vacuum, the product was purified using silica (20 hexane in dichloromethane) to give 560 mg of titled product (93% yield).

### Synthesis of 2-(5-Nitro-1-trityl-1H-indazol-3-yl)-furo[3,2-c]pyridine

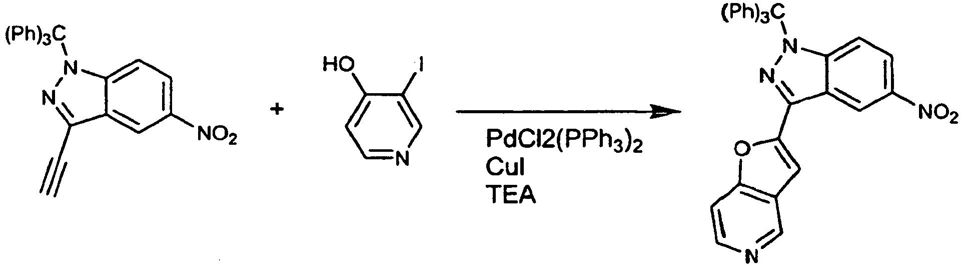

A reaction mixture containing 3-ethynyl-5-nitro-1-trityl-1H-indazole (215 mg, 0.5 mmol), 3-iodo-pyridin-4-ol (132 mg, 0.6 mmol), copper (I) iodide (4.5 mg, 0.025 mmol), PdCl₂(PPh₃)₂ and triethylamine (120 mg, 1.2 mmol) in DMF (3 mL) was heated at 100 °C overnight under argon. After the completion of reaction (shown by TLC), ethyl acetate (50 mL)was added and the reaction mixture was added to water. The organic layer was collected, washed with water, brine and concentrated under vacuum. After purification using silica (7% methanol in dichloromethane), the desired product was obtained (210 mg, 0.4 mmol) in 80% yield.

### Synthesis of 2-(5-amino-1-trityl-1H-indazol-3-yl)-furo[3,2-c]pyridine

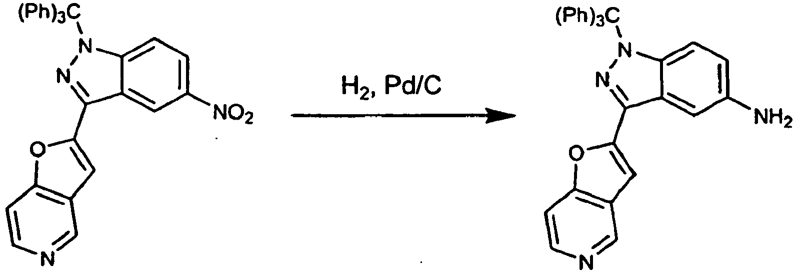

In a round-bottom flask equipped with a balloon, a mixture 2-(5-Nitro-1-trityl-1 H-indazol-3-yl)-furo[3,2-c]pyridine (210 mg, 0.4 mmol) and 5% Pd on carbon (21 mg) in ethyl acetate and methanol (10 mL, 1:1) was hydrogenated overnight at room temperature to give 157 mg desired product (80% yield).

Following procedures described above the compounds in Table 29 were prepared.

**Table 29**

| Compound | Retention Time (min) | M+1 |
|---|---|---|
| 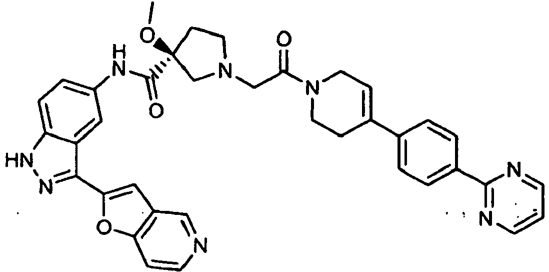 | 2.67 | 655 |
| (Example 826) | | |
| 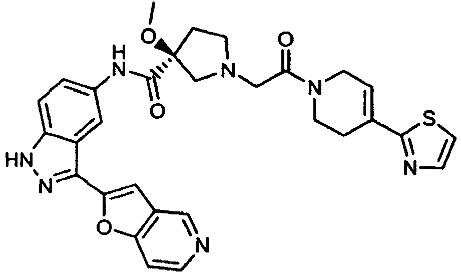 | 2.18 | 584 |
| (Example 827) | | |

### ASSAYS

### Coupled ERK2 Assay:

Activity of compounds against inactive ERK2 was tested in a coupled MEK1/ERK2 IMAP assay as follows: Compounds were diluted to 25x final test concentration in 100% DMSO. 14µl of kinase buffer (10mM Tris.HCl pH 7.2, 10mM MgCl₂, 0.01% Tween-20, 1 mM DTT) containing 0.4ng unphosphorylated Mouse ERK2 protein was added to each well of a black 384-well assay plate. 1 µl of 25x compound was added to each well and incubated at room temperature for 30 minutes to allow an opportunity for the compound to bind to the inactive enzyme. DMSO concentration during initial incubation is 6.7%. ERK2 activity was determined to be insensitive to DMSO concentrations up to 20%. ERK2 was then activated and it's kinase activity measured by the addition of 10µl kinase buffer with the following components (final concentration per reaction): 2ng active (phosphorylated) human MEK1 protein and 4µM (total) ERK2 IMAP substrate peptides (3.9µM unlabeled IPTTPITTTYFFFK-CONH₂ and 100nM IPTTPITTTYFFFK(5-carboxyfluorescein)-CONH₂) and 30µM ATP. DMSO concentration during ERK activation was 4%. After one hour, reactions were terminated by addition of 60µl IMAP detections beads in binding buffer (Molecular Devices). Binding was allowed to equilibrate for 30 minutes before reading the plate on an LJL Analyst Fluorescence Polarization plate reader. Compound inhibition was calculated relative to DMSO and fully inhibited standards. Active compounds were reconfirmed in an independent assay.

### Active ERK2 Assay:

**Activated ERK2** activity was also determined in the IMAP assay format using the procedure outlined above. 1 µl of 25x compound was added to 14µl of kinase buffer containing 0.25ng fully phosphorylated, active Mouse ERK2 protein. Following a 30 minute incubation, the reactions were initiated by addition of 10µl of kinase buffer containing 1µM ERK2 IMAP substrate peptide (0.9µM unlabeled IPTTPITTTYFFFK-CONH₂ and 100nM IPTTPITTTYFFFK(5-carboxyfluorescein)-CONH₂) and 30µM ATP. Reactions proceeded for 30 minutes before termination by addition of 60µl IMAP detection beads in binding buffer. Plates were read as above after 30 minute binding equilibration. Active compounds were reconfirmed in an independent assay.

### Soft Agar Assay:

Anchorage-independent growth is a characteristic of tumorigenic cell lines. Human tumor cells can be suspended in growth medium containing 0.3% agarose and an indicated concentration of a farnesyl transferase inhibitor. The solution can be overlayed onto growth medium solidified with 0.6% agarose containing the same concentration of ERK1 and ERK2 inhibitor as the top layer. After the top layer is solidified, plates can be incubated for 10-16 days at 37°C under 5% CO₂ to allow colony outgrowth. After incubation, the colonies can be stained by overlaying the agar with a solution of MTT (3-[4,5-dimethyl-thiazol-2-yll-2.5-diphenyltetrazolium bromide, Thiazolyl blue) (1 mg/mL in PBS). Colonies can be counted and the IC₅₀'s can be determined.

### The AUC (area under the concentration-time curve during the first 6 hours (AUC₆ₕᵣ) in Table 30 below was determined using the Protocol of Cassette Accelerating Rapid Rat screen (CARRS)

### Animal dosing and sample collection

Male Sprague-Dawley rats (Charles River, Co.) were pre-cannulated (femoral artery) in order to facilitate precise blood sampling times, and to reduce the stress on the animals caused by serial bleedings. Following an overnight fast, two rats were dosed orally with one compound at a dose of 10 mg/kg in a 5-mUkg dose volume. Blood was collected into heparin-containing tubes serially from each animal at 0.5, 1, 2, 3, 4 and 6 h post-dosing and centrifuged to generate plasma. Approximately 100 µL of plasma were collected at the individual time points. The plasma samples were stored at -20 °C until analysis.

### Plasma sample and standard curve preparation

A set of 12 rat plasma samples was generated for each NCE (i.e. 6 timepoints and n = 2 rats). These 12 samples were pooled across the two rats at each timepoint to provide 6 pooled samples (one sample per time point) for each NCE. The pooled samples were assayed as cassettes of six (36 samples total) to provide data on the six compounds. The50-µL aliquots of the 36 plasma samples were placed into individual wells of a 96-well plate. An additional compound (often a structural analog of the test compounds) was selected as the internal standard. A mini-calibration curve was prepared (three points plus a zero) for each compound assayed. Drug-free rat plasma was measured into 1-mL aliquots and each aliquot was spiked with known concentrations of the compounds to generate standards of the desired concentrations. The concentrations of the standards were chosen to bracket the expected concentration of the pooled samples based on historical data from previous studies on other compounds. For this work, the standards were set to contain concentrations of 25, 250 and 2500 ng NCE/mL plasma. The plasma standards were precipitated in duplicate along with the samples. Protein precipitation occurred after addition of 150 µL of acetonitrile containing the internal standard at a concentration of 1 ng/mL into each sample well using the Tomtec Quadra 96 system. The precipitated samples and standards were vortexed and centrifuged in the 96-well plate. Approximately 50-100 µL of the supernatant were removed and placed into a fresh 96-well plate using the Tomtec Quadra 96 system. A volume of 5-10 µL of the supernatant was used for analysis by HPLC-MS/MS. The mini-standard curve was run in duplicate, once before and once after the samples. Thus, a total of 14 study samples plus standards were analyzed per compound. In addition, solvent blanks were injected before and after each set of 14 and after the highest calibration standard for each compound; therefore, a total of 103 injections were made into each HPLC system for each set of six compounds. Multiple solvent blank injections could be made from a single well. Twelve solvent blank wells were designated in each 96-well plate. Thus, one batch (cassette) of six NCEs was prepared and assayed using one 96-well plate format.

### HPLC-MS/MS analysis

All the compounds were analyzed using selected reaction monitoring (SRM) methods with LC/MS/MS instruments. Once the method development had been completed, the assay was quickly set up using a standard injection sequence template for the CARRS assay.

The final compounds of Examples 1, 2, 4-61, 65-73, 77-84, 86, 88-98, 100, 102-114, 116-118, 120, 121, 124, 125, 151-155, 159-179, 183, 184, 186, 188-193, 196-199, 202-205, 250, 253-259, 260-299, 301-318, 320-323, 332-347, 356 (Isomer Z), 356 (Isomer E), 357-360, 362, 362 (compound 9P), 364, 368-436, 440-509, 511-602, 606, 607, 609-623, and 625 to 824 had an AERK2 IC50 in the range of 0.16 to 20,000 nM. The final compounds of Examples 1 to 611 are disclosed in WO2007/070398 (published on June 21, 2007).

The final compounds of Examples 1, 2, 4-28, 61, 86, 88, 89, 92, 95, 98, 100, 120, 125, 132, 142, 152, 154, 168, 176, 183, 184, 186, 188-193, 196-199, 202-205, 250, 251, 253-259, 261, 264, 269, 271, 274-276, 282, 286, 289, 290, 292, 294, 302, 303, 304, 306, 314-316, 332, 333, 335, 338, 343, 358, 362, 368-370, 384-390, 440-493, 510-558, 606, 611, 613-617, 620, 622, 623, 627-630, 634, 635, 639, 641, 643-645, 647, 651, 654-658, 661, 663, 666, 669, 670, 672, 673, 675-677, 679, 680, 682, 685, 688-694, 696-703, 705-707, 710-735, 737, 738, 740, 741, 743-745, 747, 749, 751-753, 755, 757, 758, 760-762, 764, 765, 767, 770-772, 776, 777, 779, 785, 786, 788, 789, 792, 793, 794, 796, 799, 802, 803-805, 807-809, 811-812, 814-816, 818-820, 822, and 823 had an AERK2 IC₅₀ in the range of 0.16 to 18 nM. The final compounds of Examples 1 to 611 are disclosed in WO2007/070398 (published on June 21, 2007).

The final compounds of Examples 6, 100, 183, 184, 186, 188-192, 261, 440-450, 510-517 and 787 had an AERK2 IC₅₀ in the range of 0.16 to 1.5 nM. The final compounds of 6, 100, 183, 184, 186, 188-192, 261, 440-450, 510-517 are disclosed in WO2007/070398 (published on June 21, 2007).

Table 30 provides AERK2 IC₅₀ data and Rat Auc data for compounds of this invention.

**Table 30**

| **Ex.** | **Compound** | **AERK2 IC50 (nM)** | **Rat AUC PO (nM.h)** |
|---|---|---|---|
| 622 | 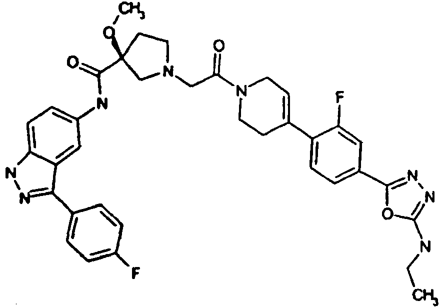 | 5 | 1128 |
| 613 | 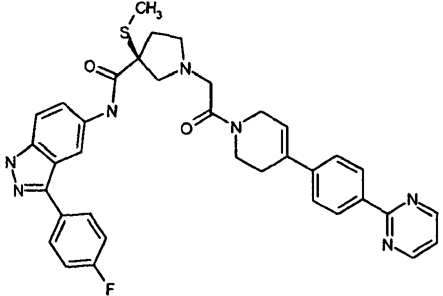 | 10 | 25914 |

For preparing pharmaceutical compositions from the compounds described by this invention, inert, pharmaceutically acceptable carriers can be either solid or liquid. Solid form preparations include powders, tablets, dispersible granules, capsules, cachets and suppositories. The powders and tablets may be comprised of from about 5 to about 95 percent active ingredient. Suitable solid carriers are known in the art, e.g. magnesium carbonate, magnesium stearate, talc, sugar or lactose. Tablets, powders, cachets and capsules can be used as solid dosage forms suitable for oral administration. Examples of pharmaceutically acceptable carriers and methods of manufacture for various compositions may be found in A. Gennaro (ed.), Remington: The Science and Practice of Pharmacy, 20th Edition, (2000), Lippincott Williams & Wilkins, Baltimore, MD.

Liquid form preparations include solutions, suspensions and emulsions. As an example may be mentioned water or water-propylene glycol solutions for parenteral injection or addition of sweeteners and opacifiers for oral solutions, suspensions and emulsions. Liquid form preparations may also include solutions for intranasal administration.

Aerosol preparations suitable for inhalation may include solutions and solids in powder form, which may be in combination with a pharmaceutically acceptable carrier, such as an inert compressed gas, e.g. nitrogen.

Also included are solid form preparations which are intended to be converted, shortly before use, to liquid form preparations for either oral or parenteral administration. Such liquid forms include solutions, suspensions and emulsions.

The compounds of the invention may also be deliverable transdermally. The transdermal compositions can take the form of creams, lotions, aerosols and/or emulsions and can be included in a transdermal patch of the matrix or reservoir type as are conventional in the art for this purpose.

Preferably the compound is administered orally.

Preferably, the pharmaceutical preparation is in a unit dosage form. In such form, the preparations subdivided into suitably sized unit doses containing appropriate quantities of the active component, e.g., an effective amount to achieve the desired purpose.

The quantity of active compound in a unit dose of preparation may be varied or adjusted from about 0.01 mg to about 1000 mg, preferably from about 0.01 mg to about 750 mg, more preferably from about 0.01 mg to about 500 mg, and most preferably from about 0.01 mg to about 250 mg according to the particular application.

The actual dosage employed may be varied depending upon the requirements of the patient and the severity of the condition being treated. Determination of the proper dosage regimen for a particular situation is within the skill in the art. For convenience, the total daily dosage may be divided and administered in portions during the day as required.

The amount and frequency of administration of the compounds of the invention and/or the pharmaceutically acceptable salts thereof will be regulated according to the judgment of the attending clinician considering such factors as age, condition and size of the patient as well as severity of the symptoms being treated. A typical recommended daily dosage regimen for oral administration can range from about 0.04 mg/day to about 4000 mg/day, in two to four divided doses.

## Claims

1. An ERK Inhibitor selected from the group consisting of the compounds having the following structures:
| Example No. | COMPOUND |
|---|---|
| 612 | 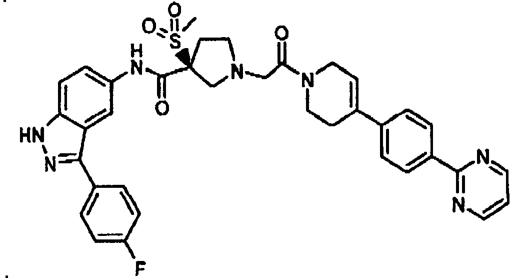 |
| 613 | 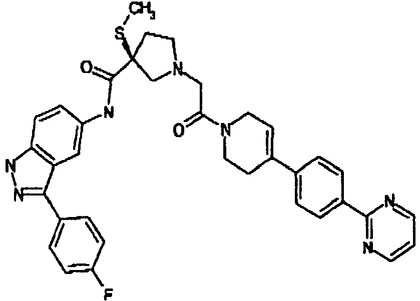 |
| 614 | 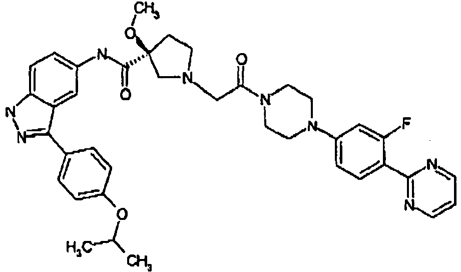 |
| 615 | 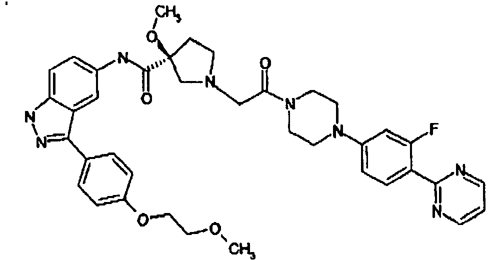 |
| 616 | 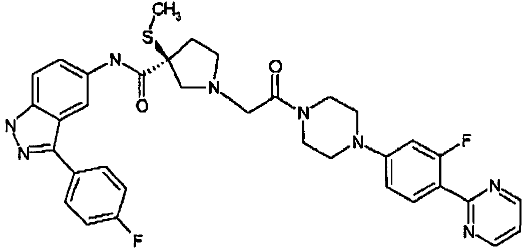 |
| 617 | 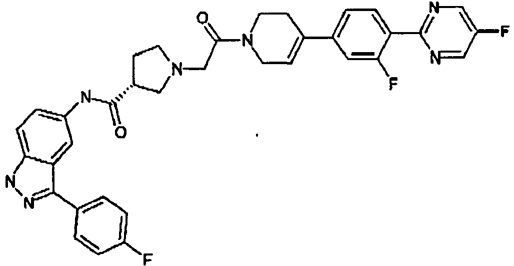 |
| 619 | 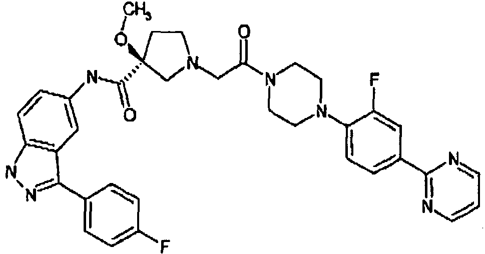 |
| 620 | 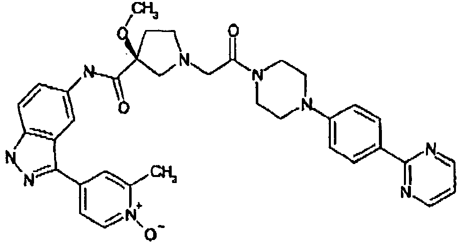 |
| 621 | 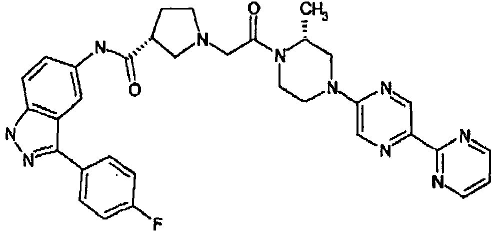 |
| 622 | 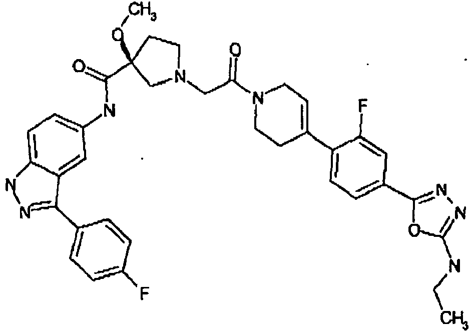 |
| 623 | 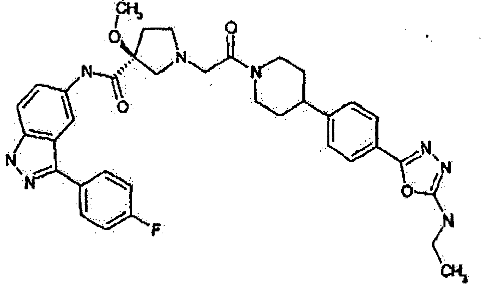 |
| 624 | 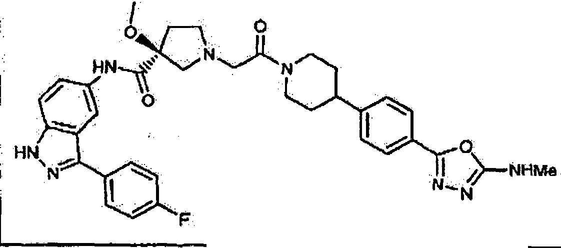 |
| | |
| 625 | 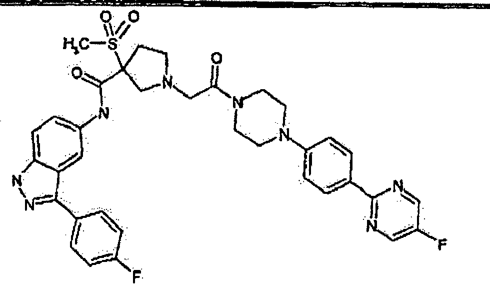 |
| 626 | 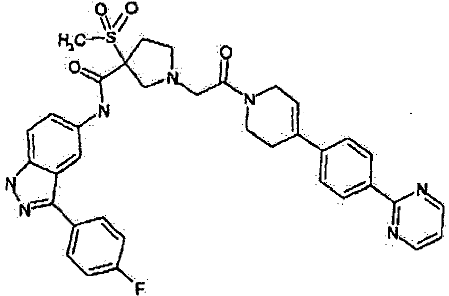 |
| 627 | 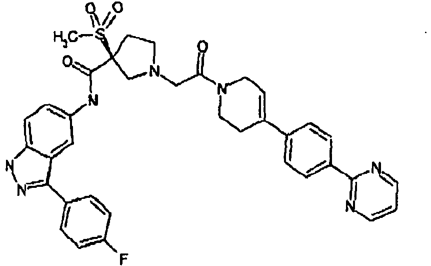 |
| 628 | 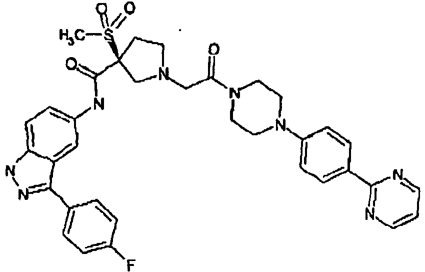 |
| 629 | 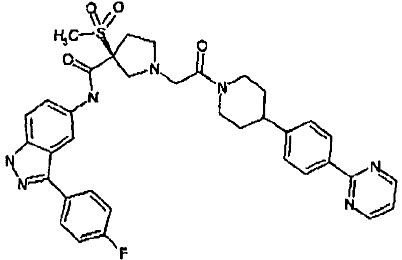 |
| 630 | 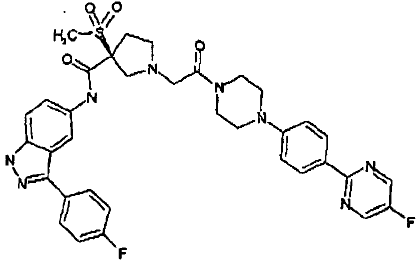 |
| 631 | 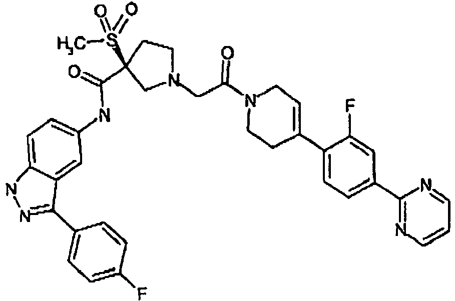 |
| 632 | 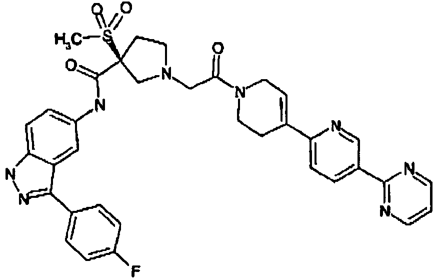 |
| 633 | 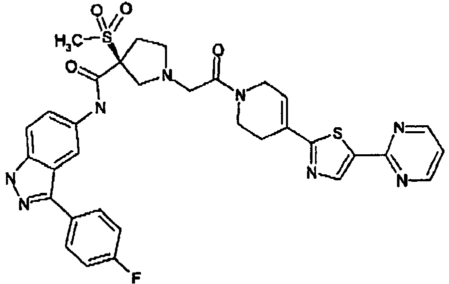 |
| 634 | 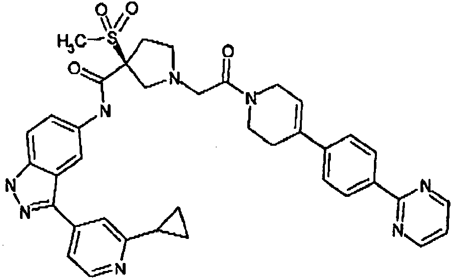 |
| 635 | 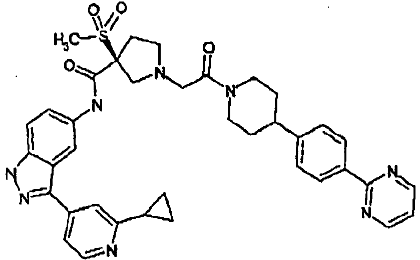 |
| 636 | 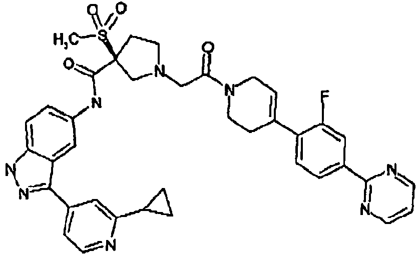 |
| 637 | 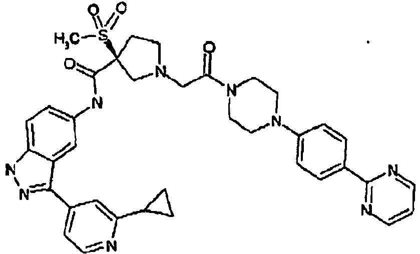 |
| 638 | 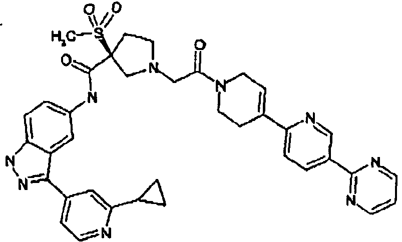 |
| 639 | 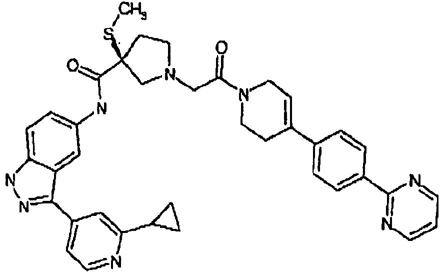 |
| 640 | 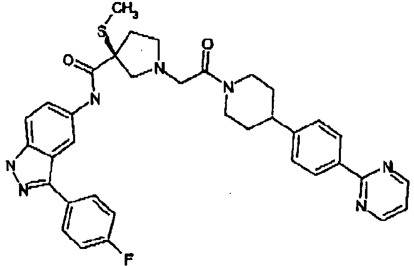 |
| 641 | 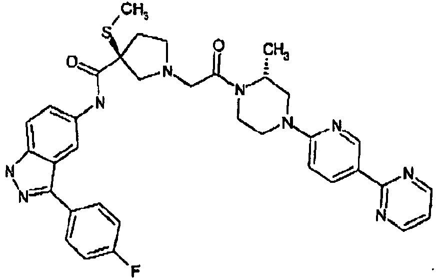 |
| 642 | 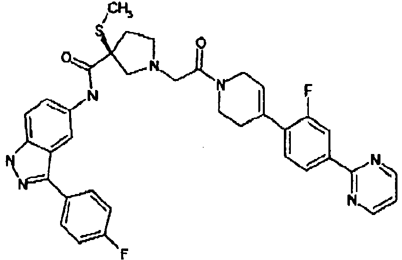 |
| 643 | 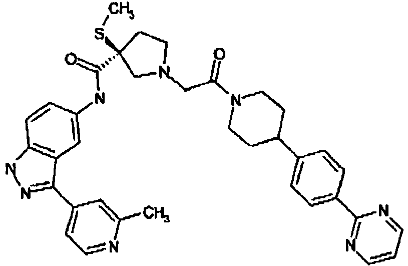 |
| 644 | 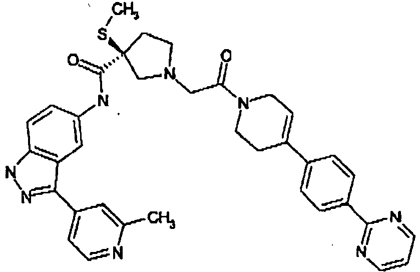 |
| 645 | 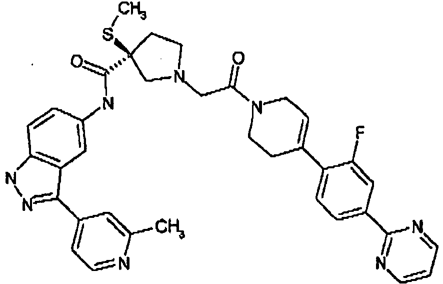 |
| 646 | 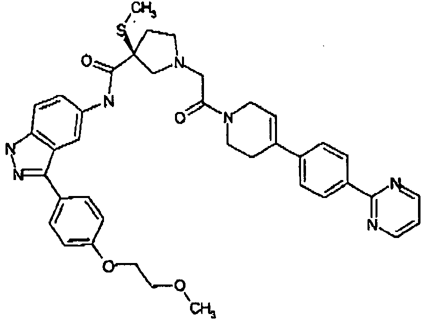 |
| 647 | 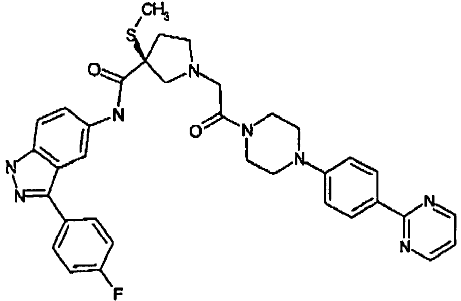 |
| 648 | 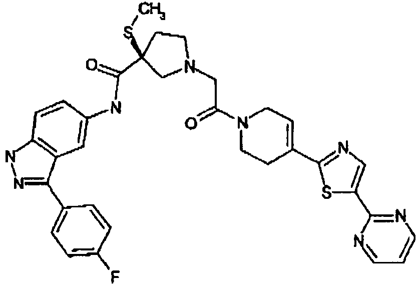 |
| 649 | 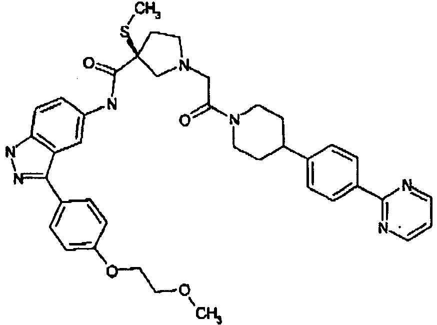 |
| 650 | 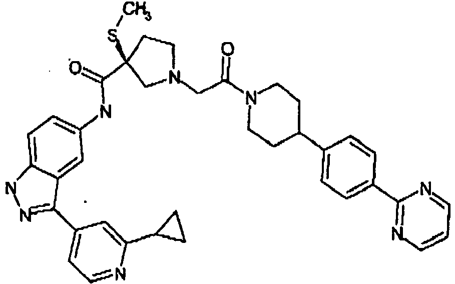 |
| 651 | 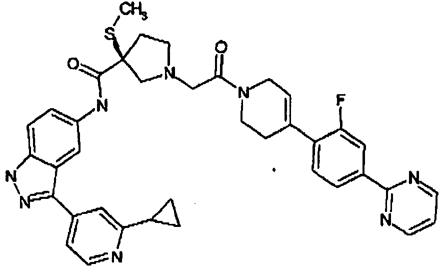 |
| 652 | 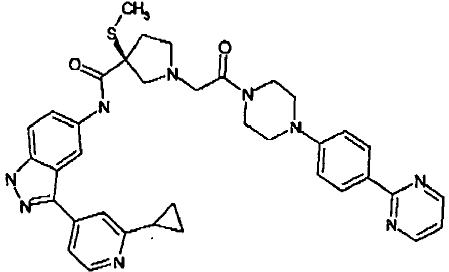 |
| 653 | 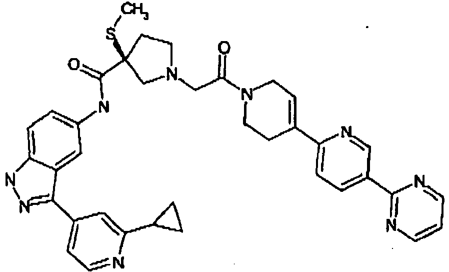 |
| 654 | 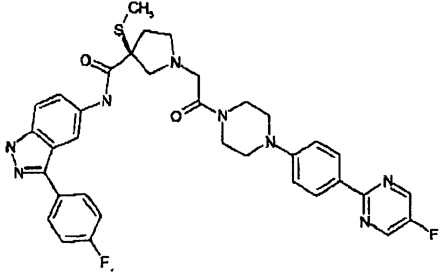 |
| 655 | 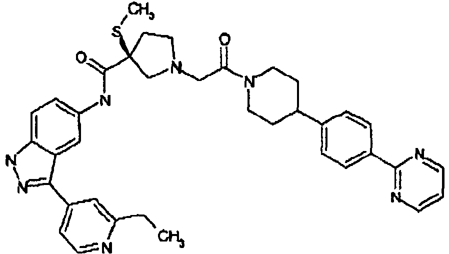 |
| 656 | 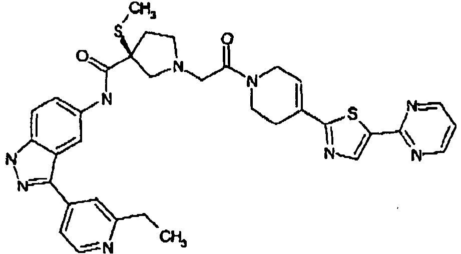 |
| 657 | 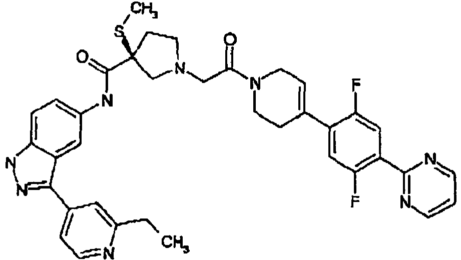 |
| 658 | 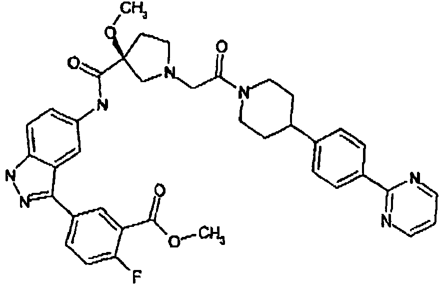 |
| 659 | 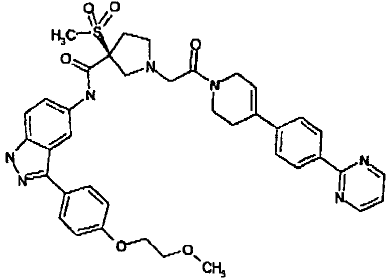 |
| 660 | 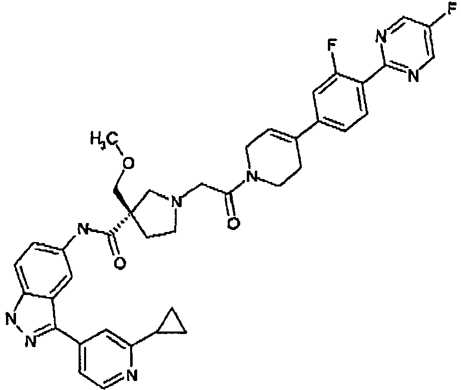 |
| 861 | 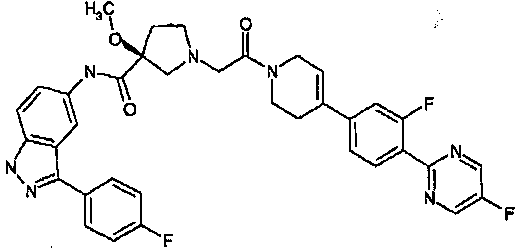 |
| 662 | 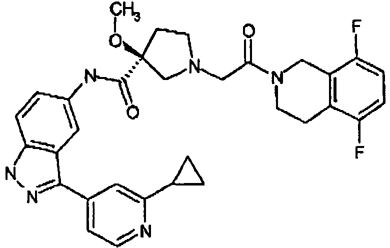 |
| 663 | 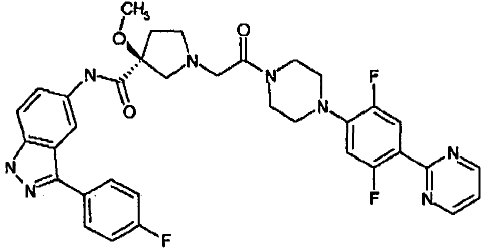 |
| 664 | 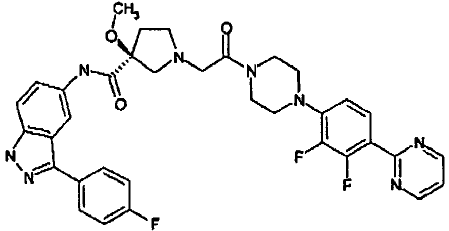 |
| 665 | 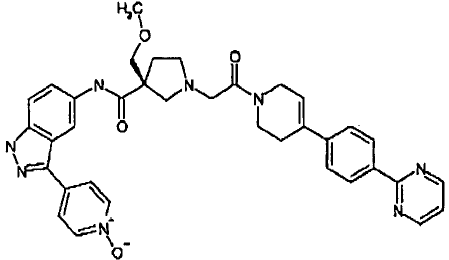 |
| 666 | 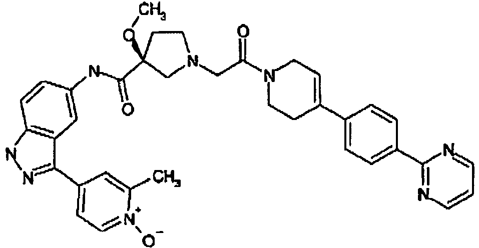 |
| 667 | 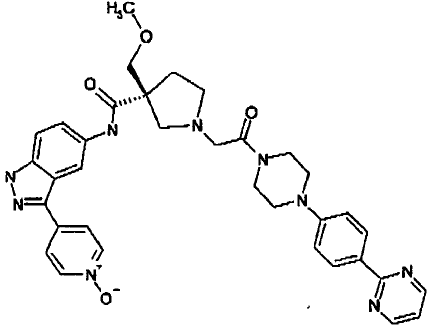 |
| 668 | 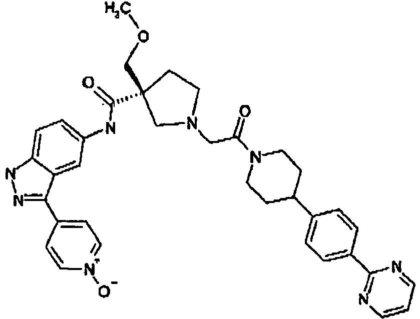 |
| 669 | 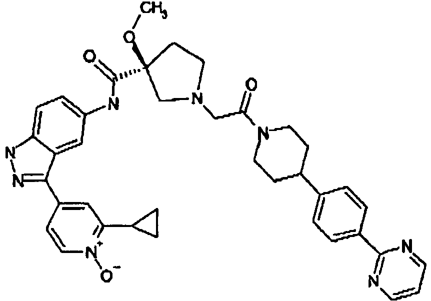 |
| 670 | 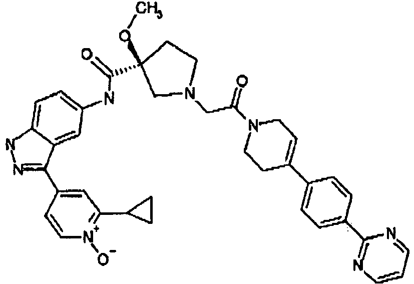 |
| 671 | 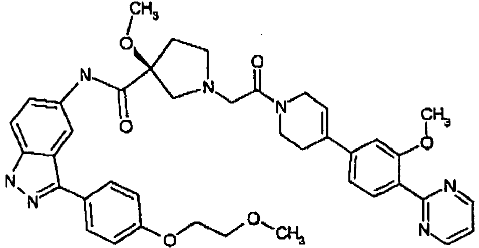 |
| 672 | 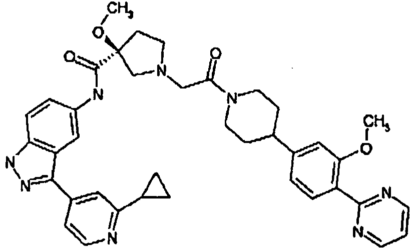 |
| 673 | 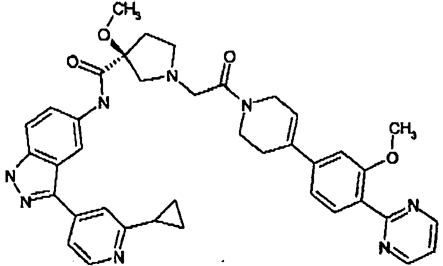 |
| 674 | 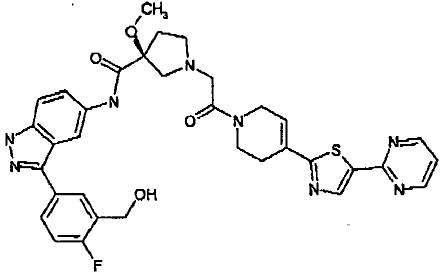 |
| 675 | 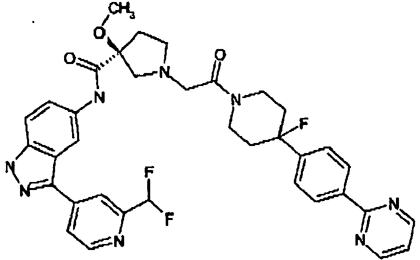 |
| 676 | 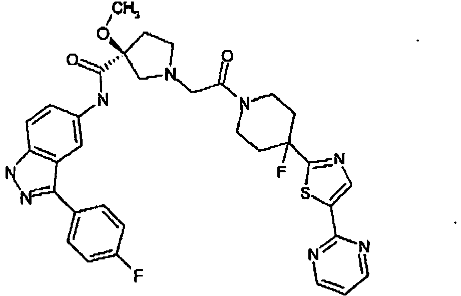 |
| 677 | 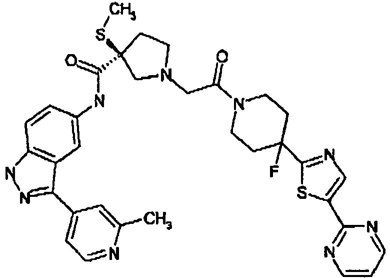 |
| 678 | 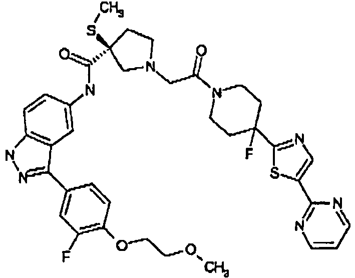 |
| 679 | 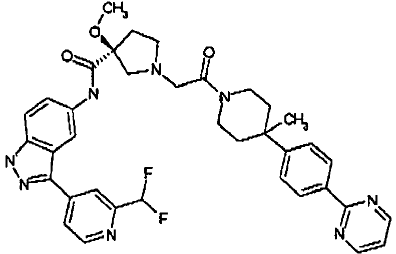 |
| 680 | 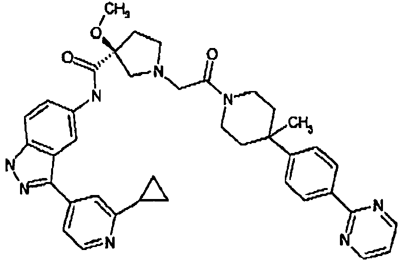 |
| 681 | 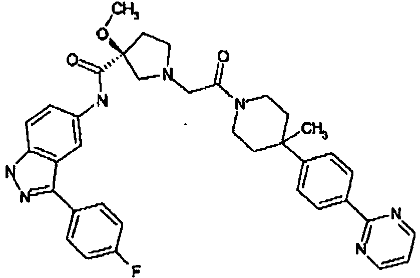 |
| 682 | 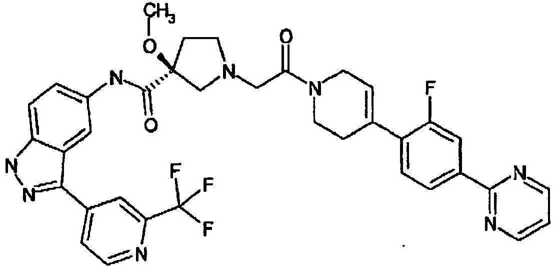 |
| 683 | 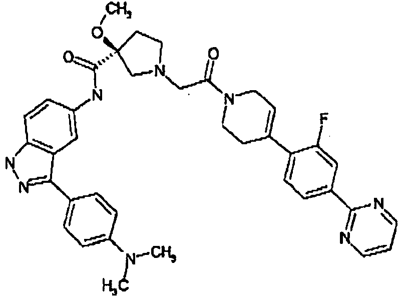 |
| 684 | 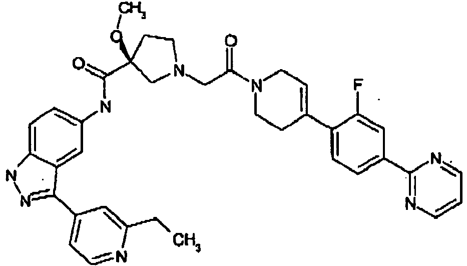 |
| 685 | 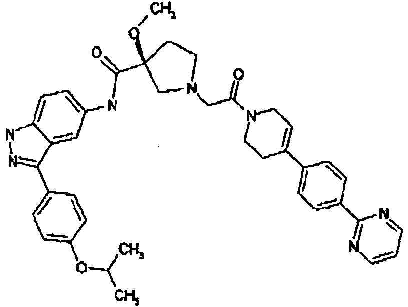 |
| 686 | 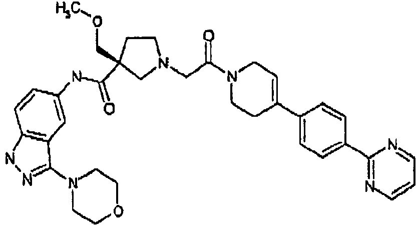 |
| 687 | 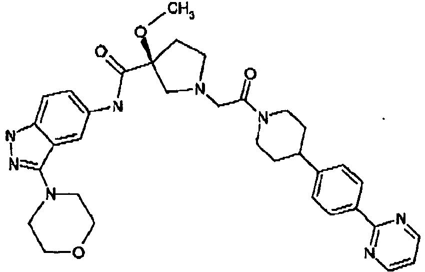 |
| 688 | 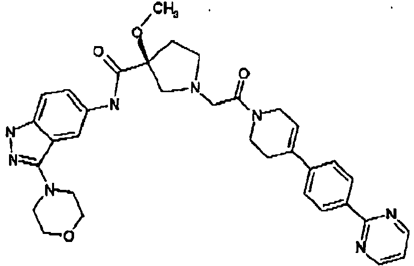 |
| 689 | 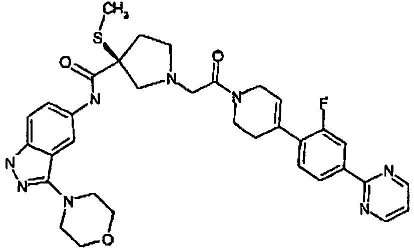 |
| 690 | 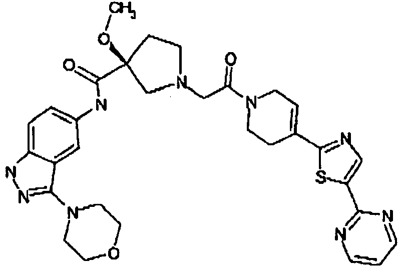 |
| 691 | 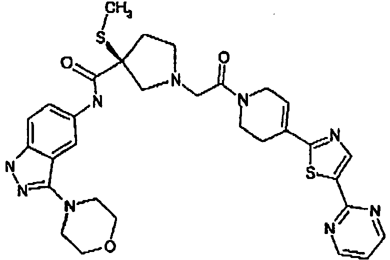 |
| 692 | 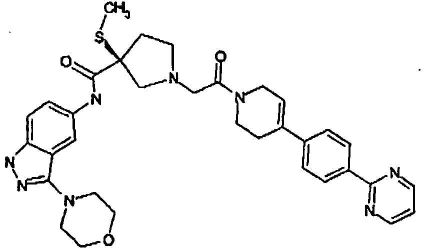 |
| 693 | 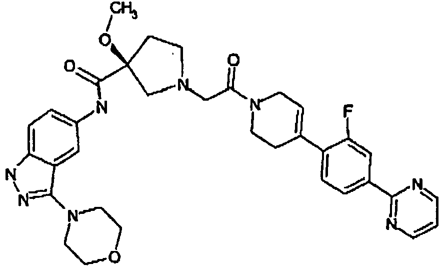 |
| 694 | 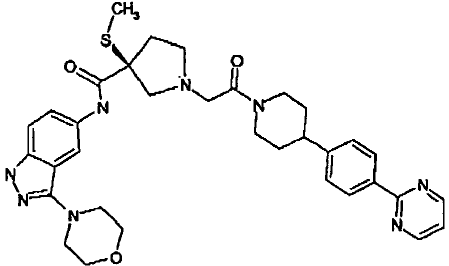 |
| 695 | 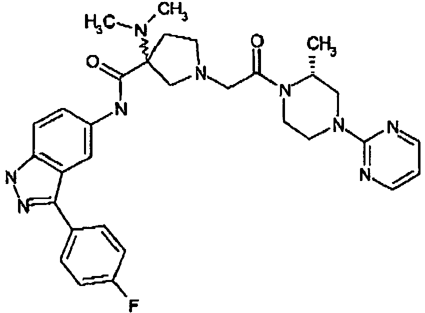 |
| 696 | 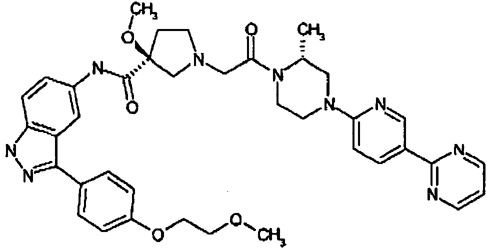 |
| 697 | 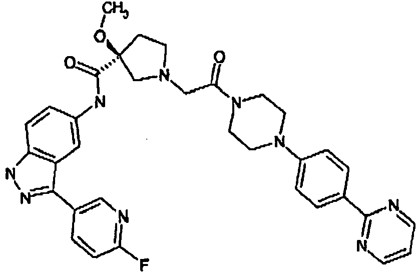 |
| 698 | 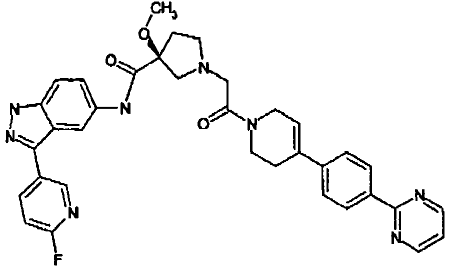 |
| 699 | 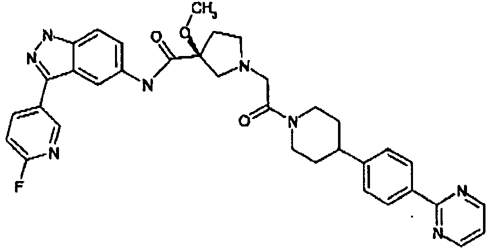 |
| 700 | 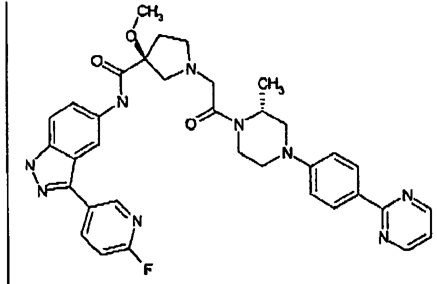 |
| 701 | 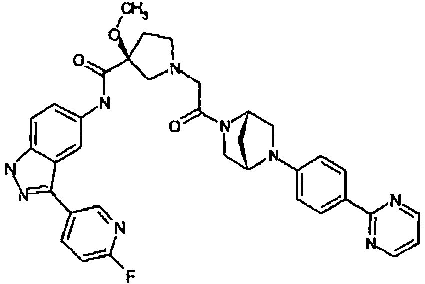 |
| 702 | 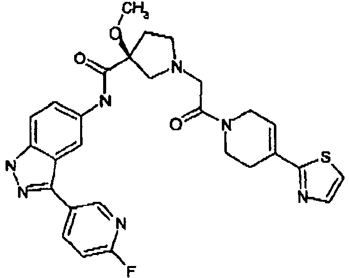 |
| 703 | 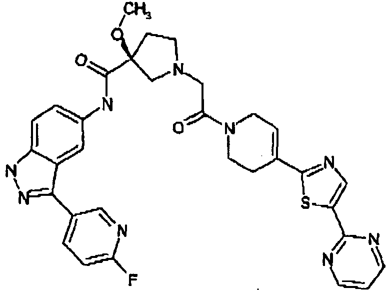 |
| 704 | 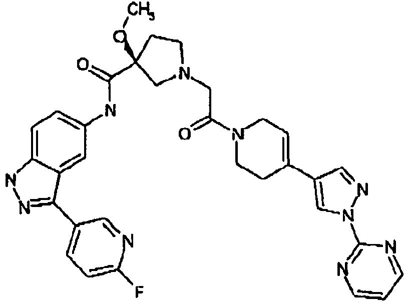 |
| 705 | 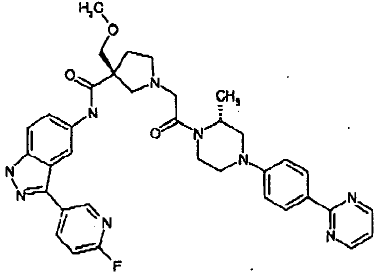 |
| 706 | 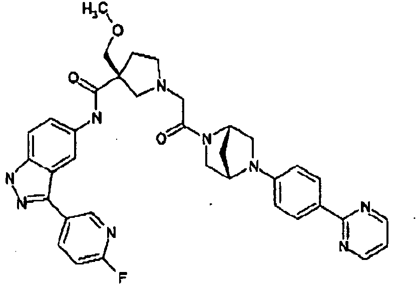 |
| 707 | 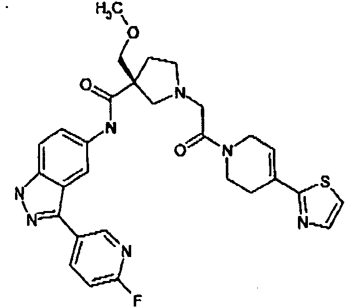 |
| 708 | 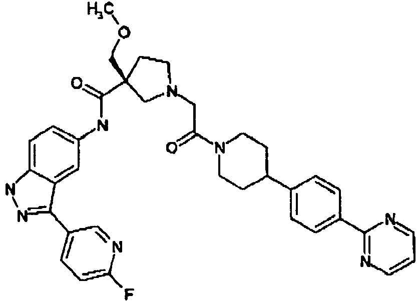 |
| 709 | 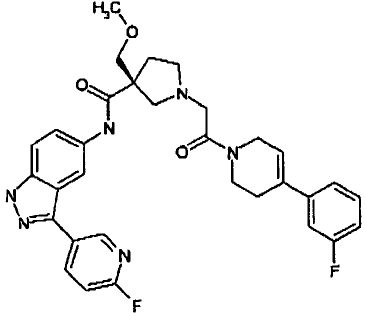 |
| 710 | 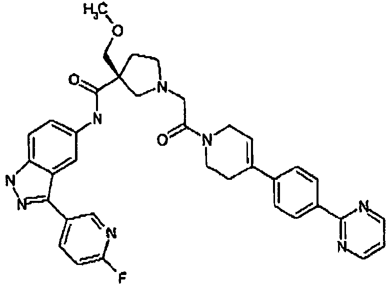 |
| 711 | 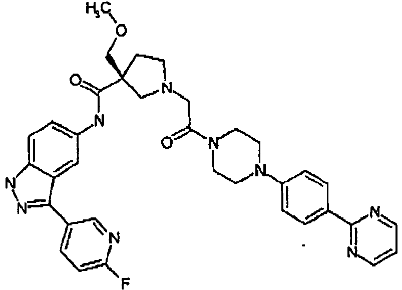 |
| 712 | 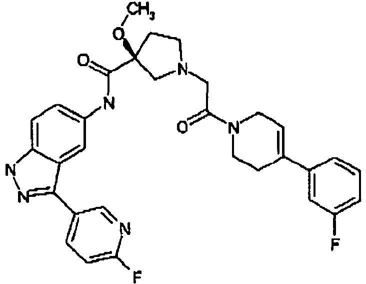 |
| 713 | 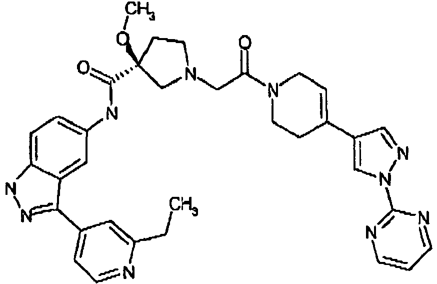 |
| 714 | 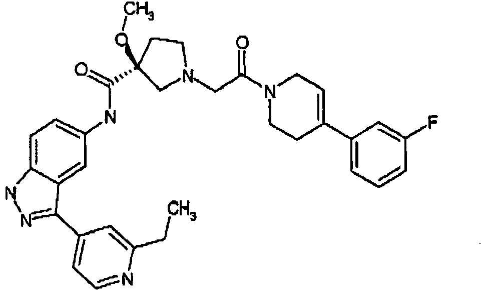 |
| 715 | 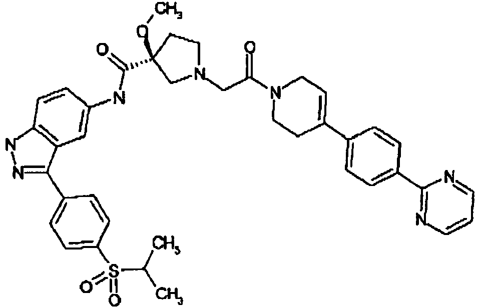 |
| 716 | 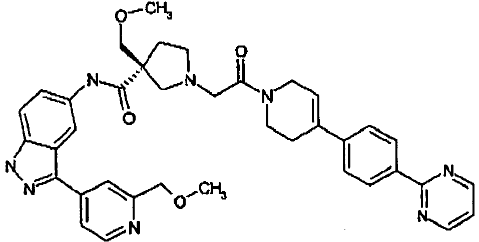 |
| 717 | 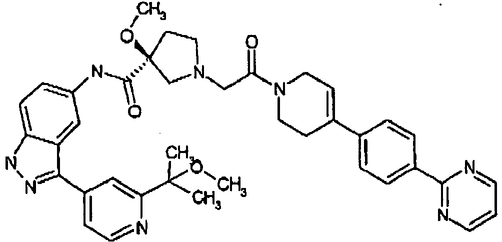 |
| 718 | 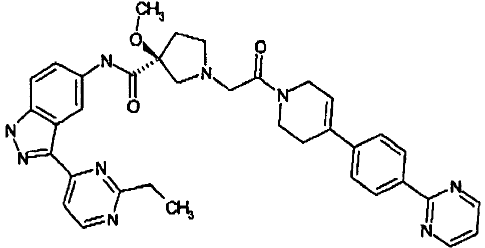 |
| 719 | 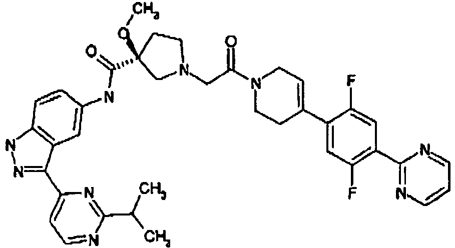 |
| 720 | 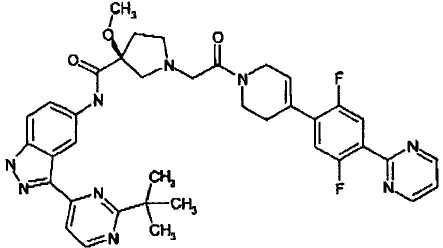 |
| 721 | 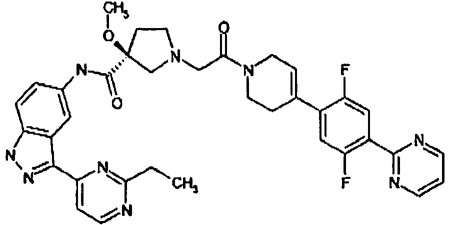 |
| 722 | 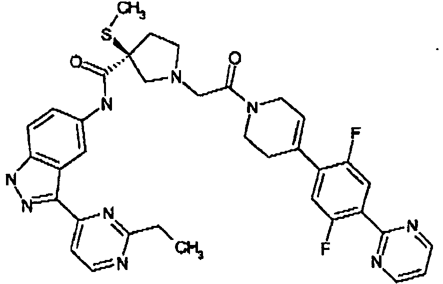 |
| 723 | 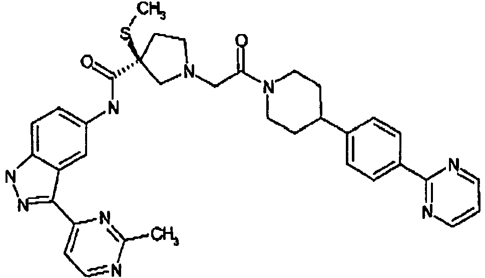 |
| 724 | 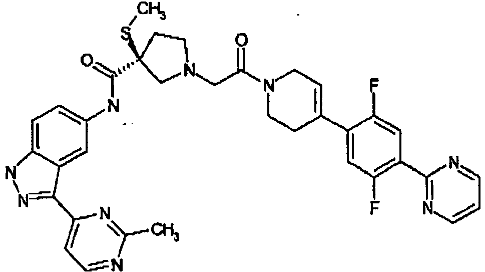 |
| 725 | 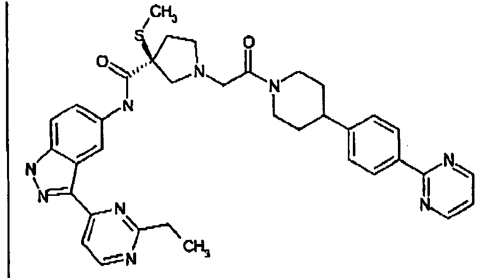 |
| 726 | 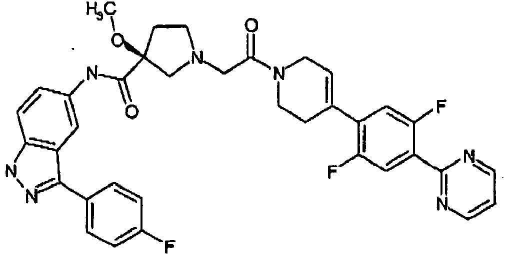 |
| 727 | 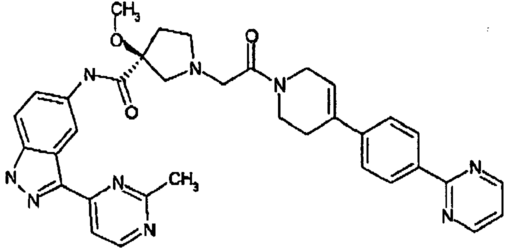 |
| 728 | 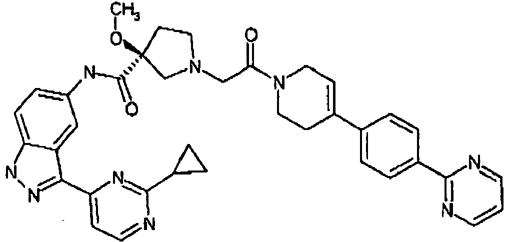 |
| 729 | 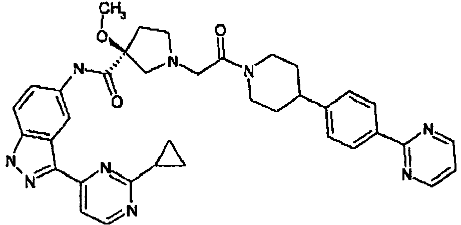 |
| 730 | 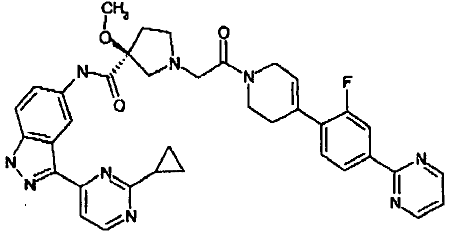 |
| 731 | 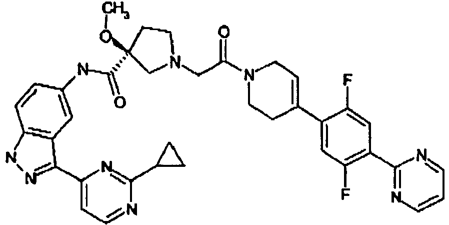 |
| 732 | 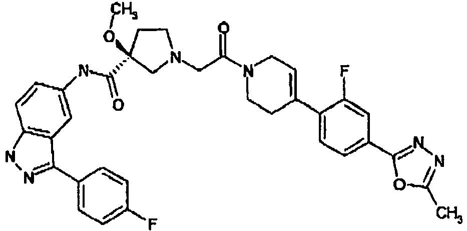 |
| 733 | 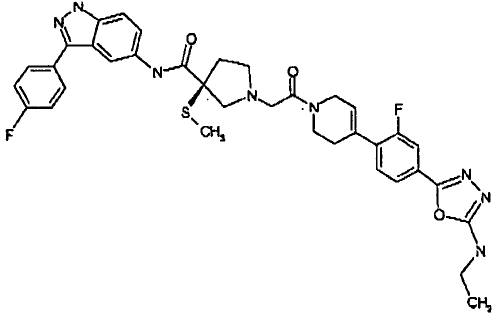 |
| 734 | 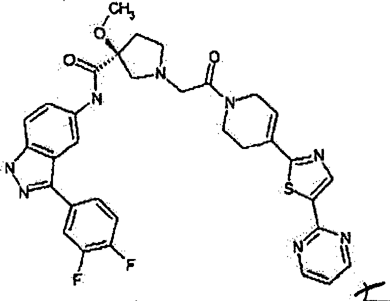 |
| 735 | 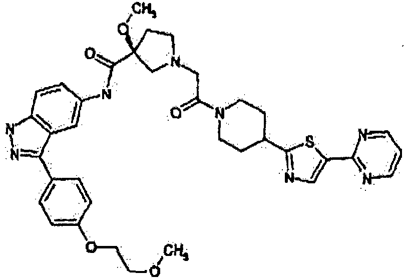 |
| 736 | 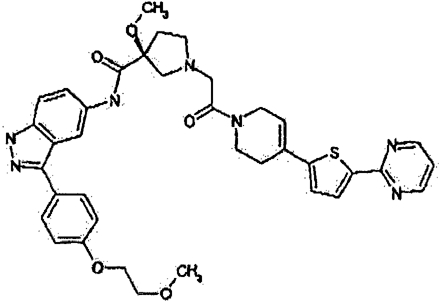 |
| 737 | 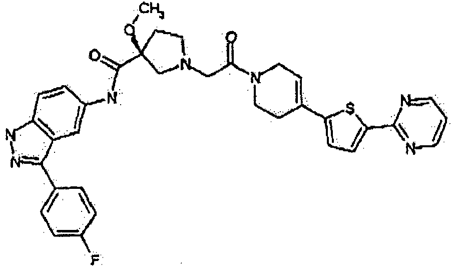 |
| 738 | 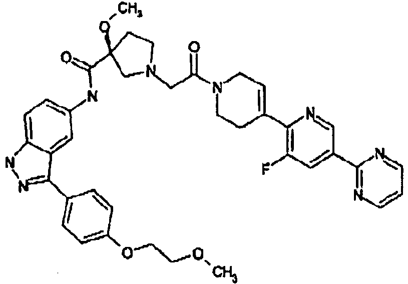 |
| 739 | 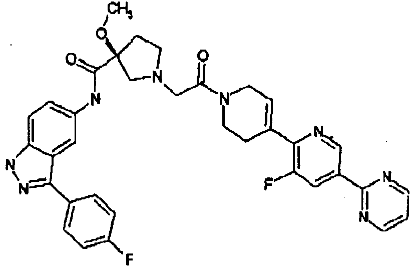 |
| 740 | 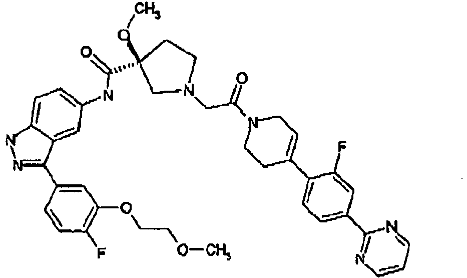 |
| 741 | 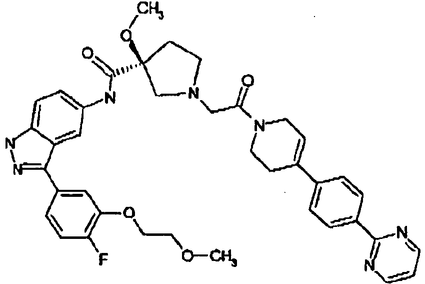 |
| 742 | 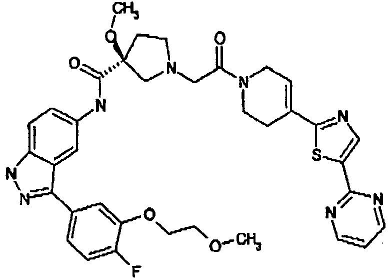 |
| 743 | 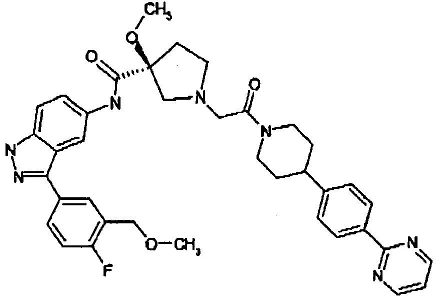 |
| 744 | 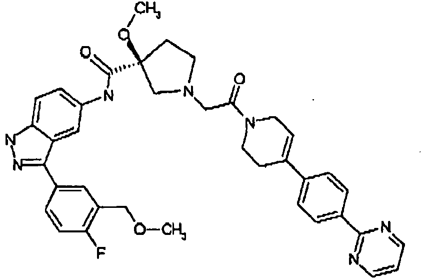 |
| 745 | 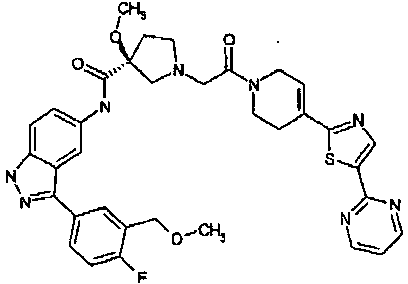 |
| 746 | 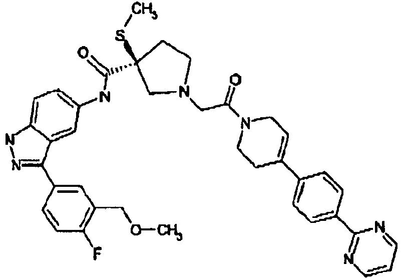 |
| 747 | 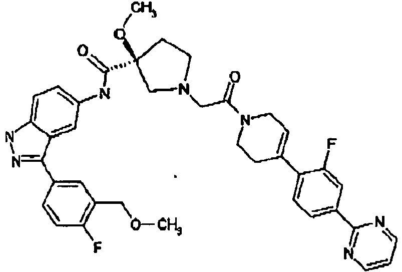 |
| 748 | 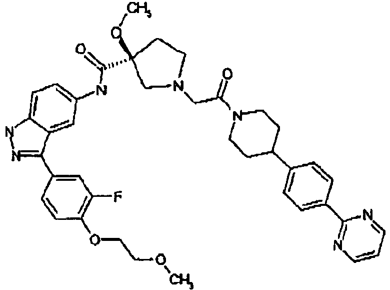 |
| 749 | 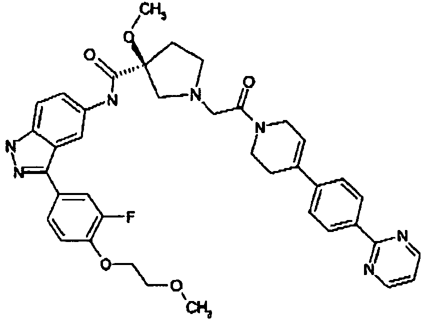 |
| 750 | 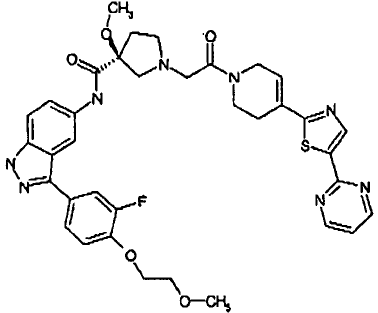 |
| 751 | 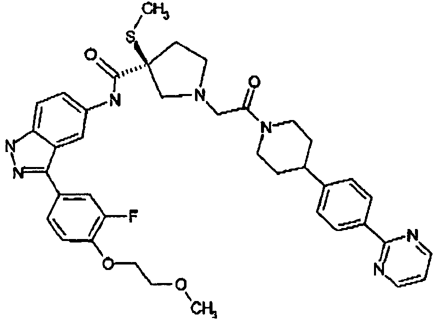 |
| 752 | 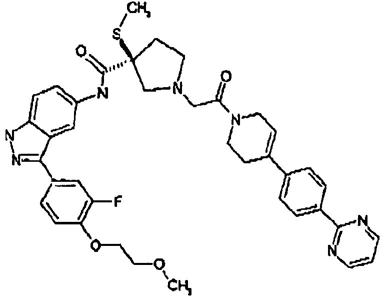 |
| 753 | 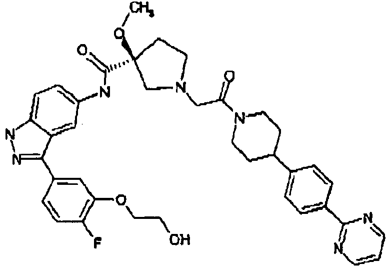 |
| 754 | 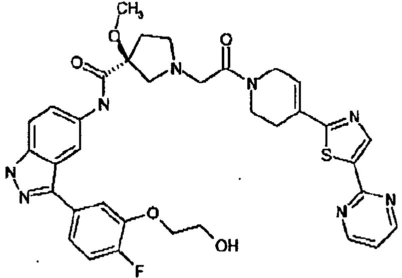 |
| 755 | 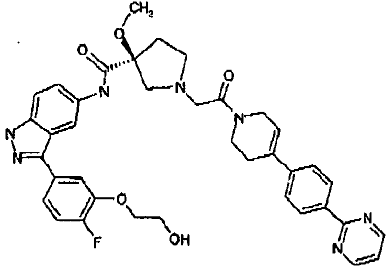 |
| 756 | 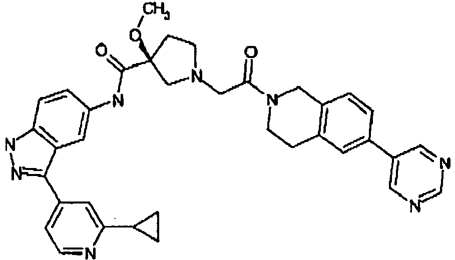 |
| 757 | 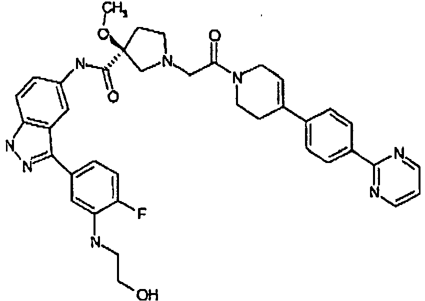 |
| 758 | 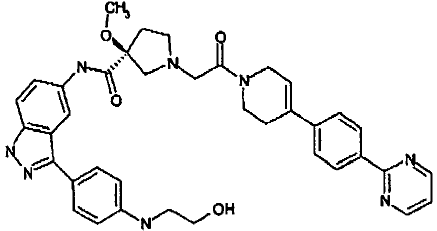 |
| 759 | 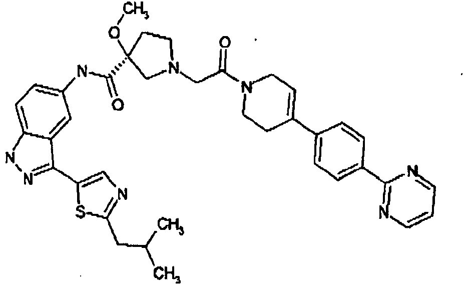 |
| 760 | 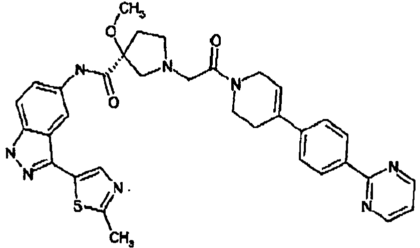 |
| 761 | 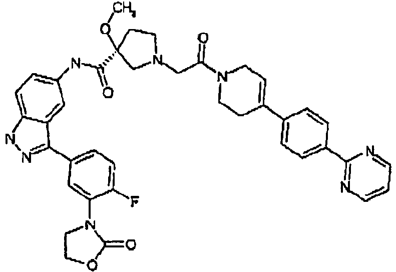 |
| 762 | 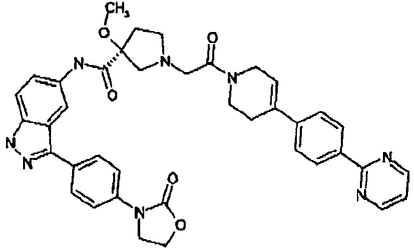 |
| 763 | 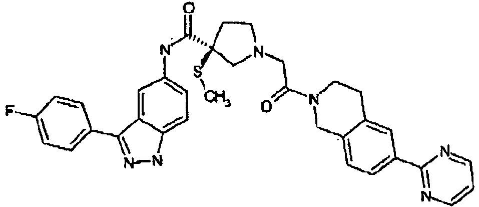 |
| 764 | 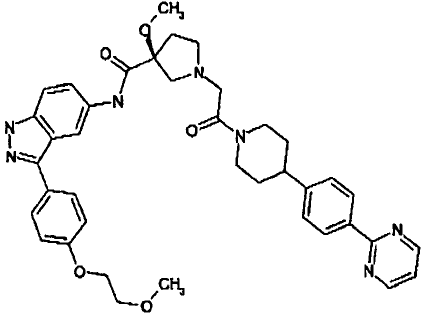 |
| 765 | 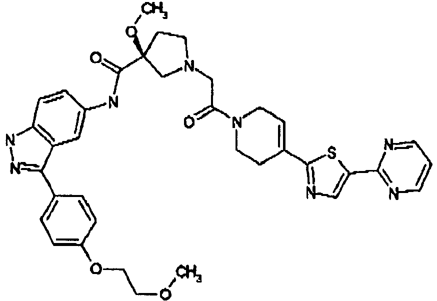 |
| 766 | 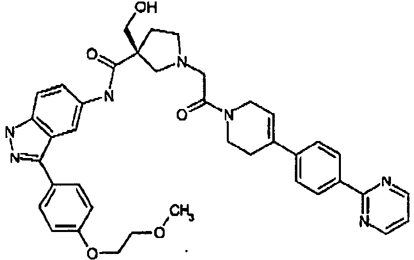 |
| 767 | 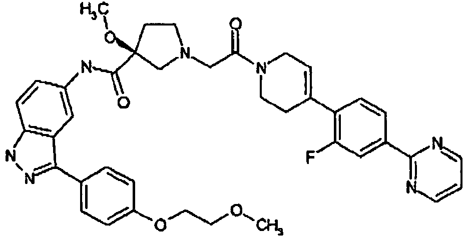 |
| 768 | 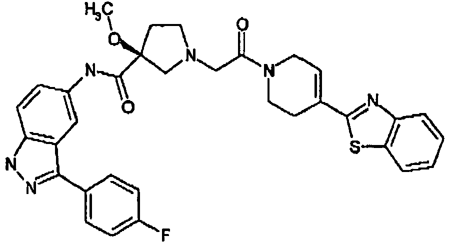 |
| 769 | 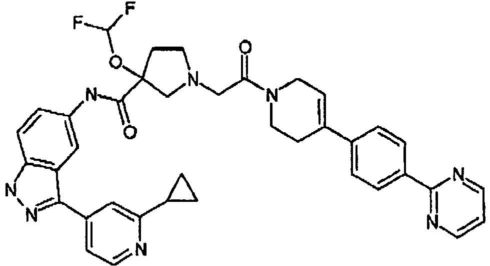 |
| 770 | 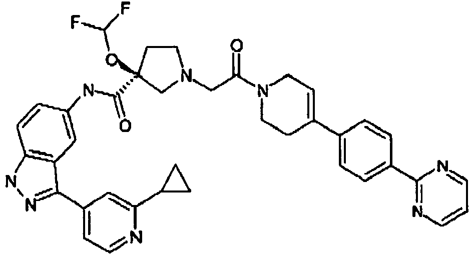 |
| 771 | 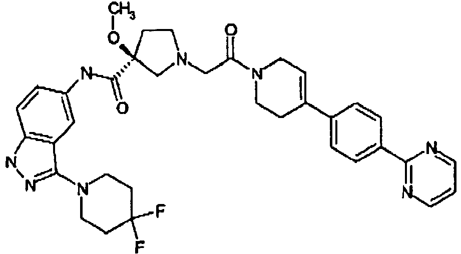 |
| 772 | 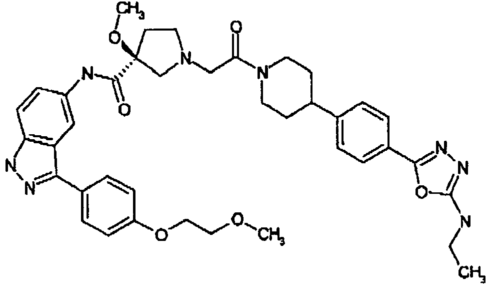 |
| 773 | 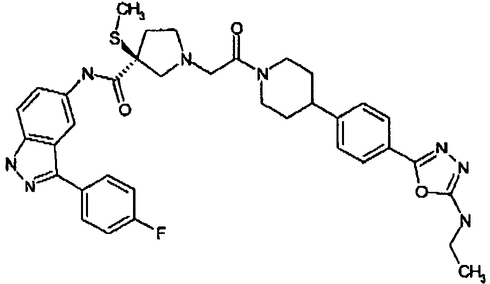 |
| 774 | 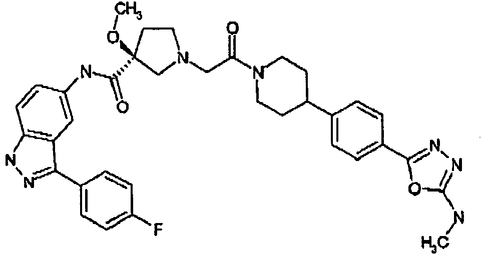 |
| 775 | 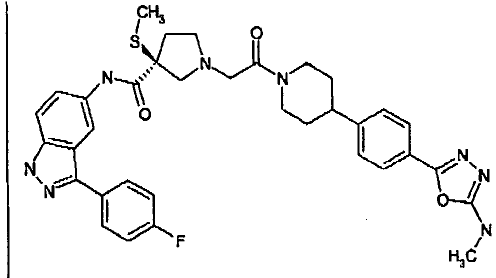 |
| 776 | 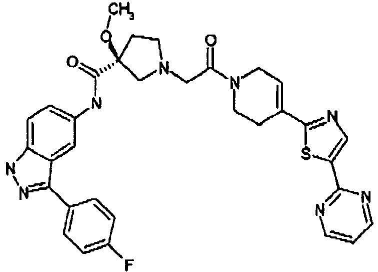 |
| 777 | 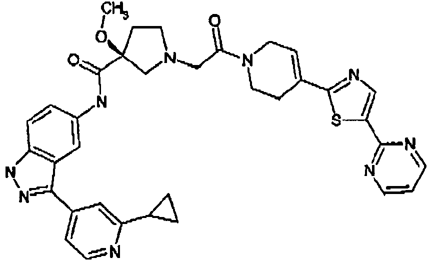 |
| 778 | 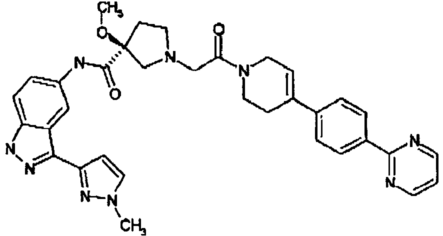 |
| 779 | 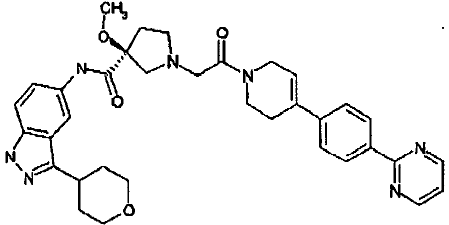 |
| 780 | 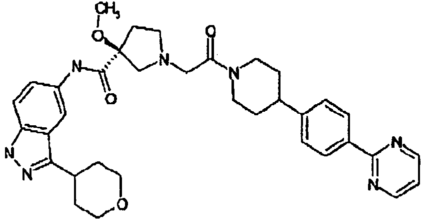 |
| 781 | 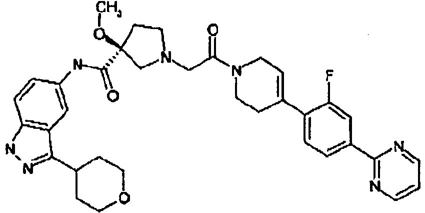 |
| 782 | 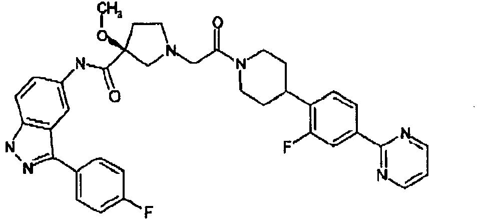 |
| 783 | 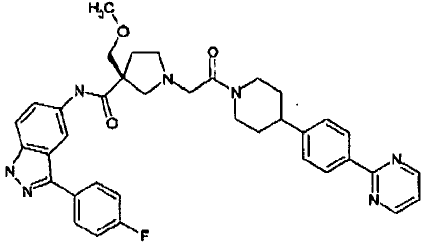 |
| 784 | 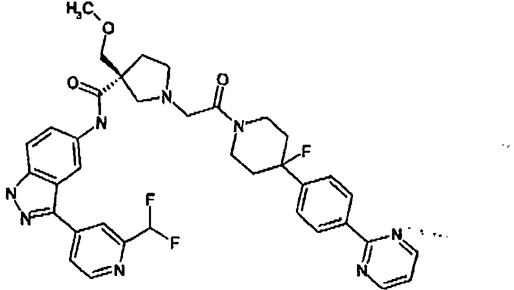 |
| 785 | 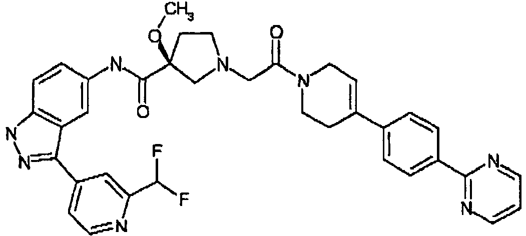 |
| 786 | 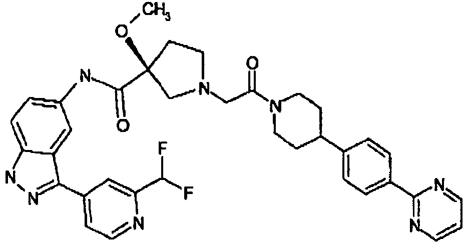 |
| 787 | 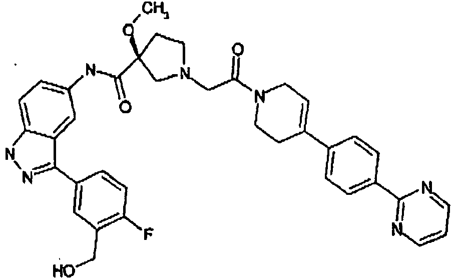 |
| 788 | 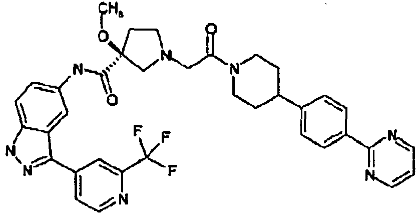 |
| 789 | 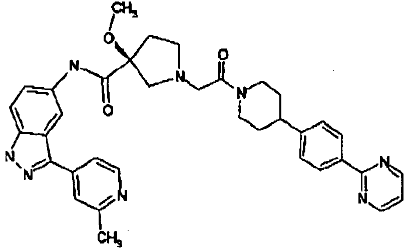 |
| 790 | 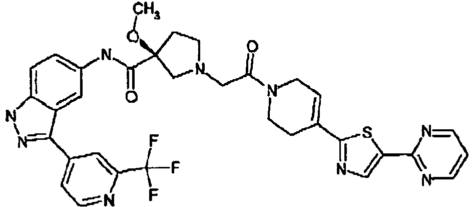 |
| 791 | 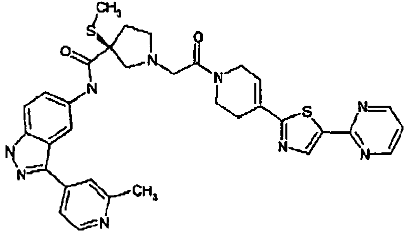 |
| 792 | 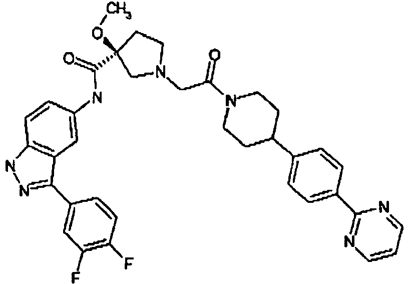 |
| 793 | 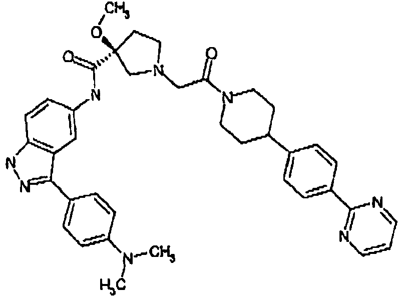 |
| 794 | 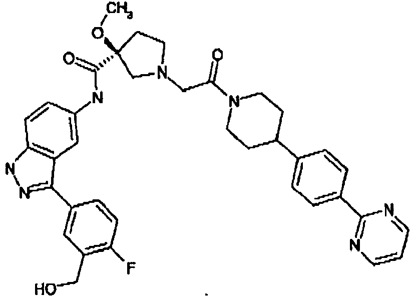 |
| 795 | 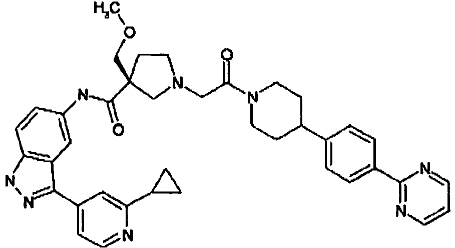 |
| 796 | 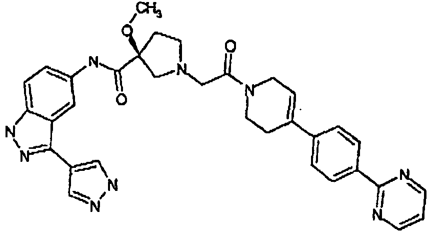 |
| 797 | 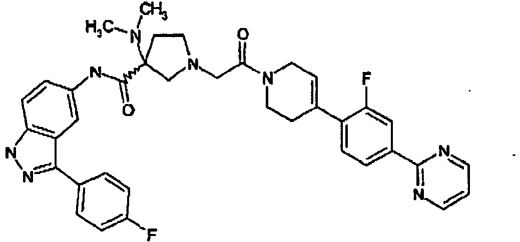 |
| 798 | 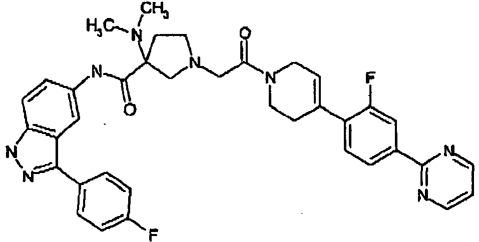 |
| 799 | 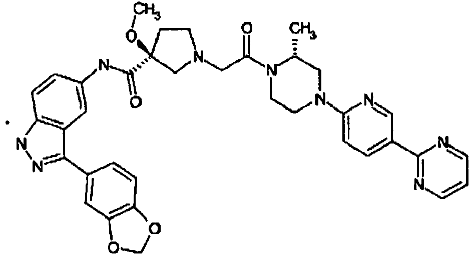 |
| 800 | 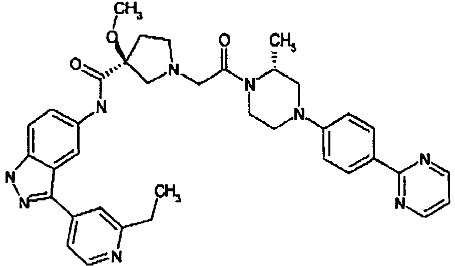 |
| 801 | 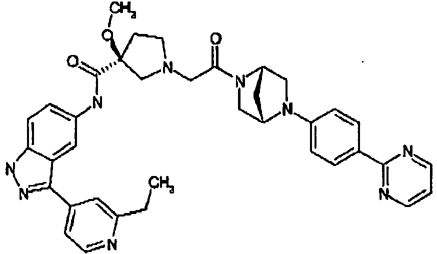 |
| 802 | 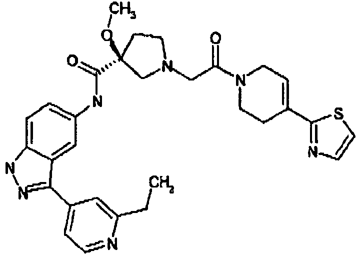 |
| 803 | 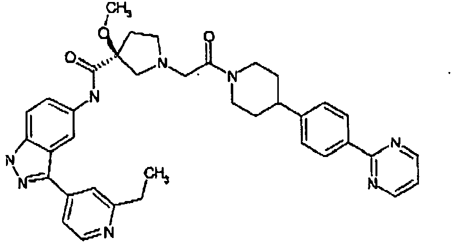 |
| 804 | 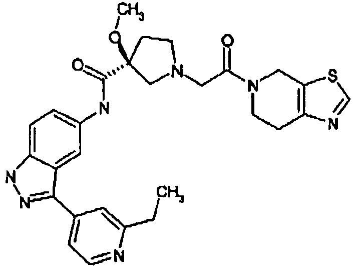 |
| 805 | 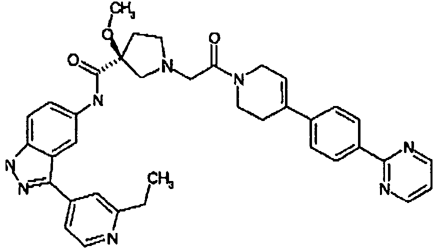 |
| 806 | 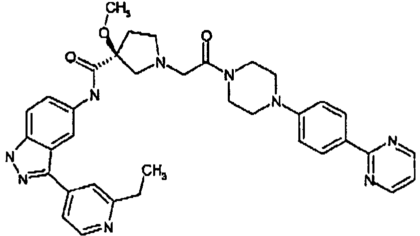 |
| 807 | 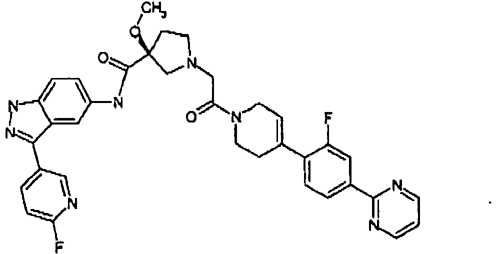 |
| 808 | 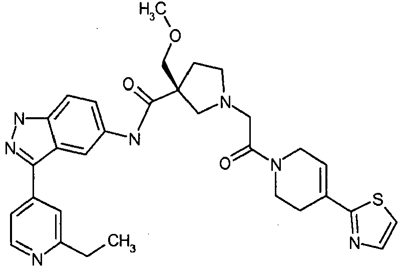 |
| 809 | 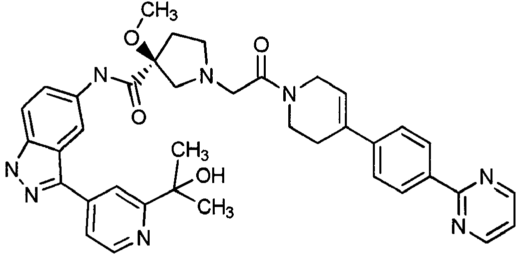 |
| 810 | 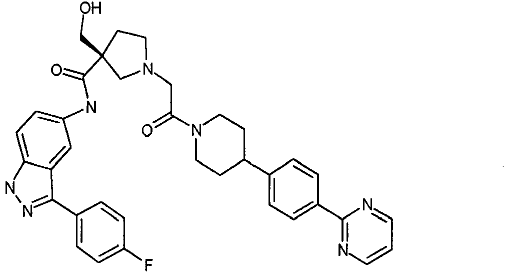 |
| 811 | 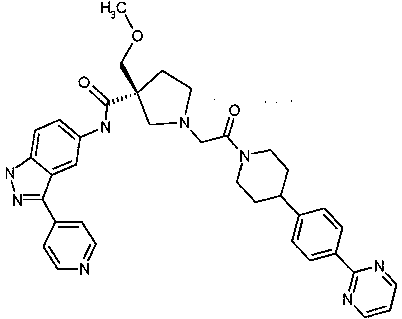 |
| 812 | 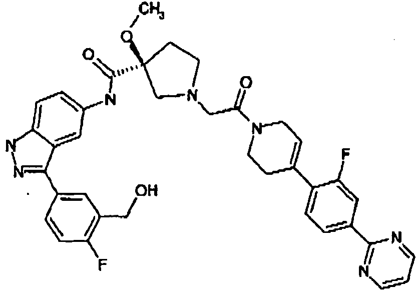 |
| 813 | 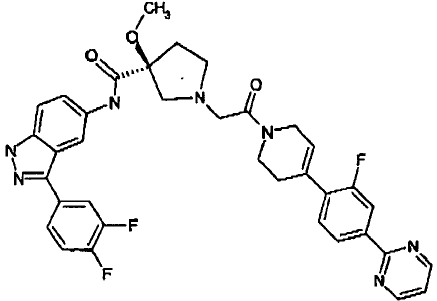 |
| 814 | 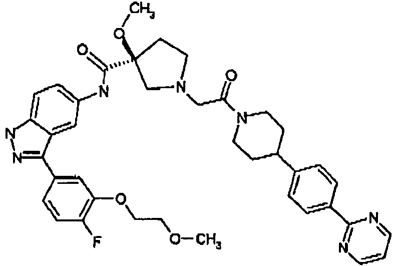 |
| 815 | 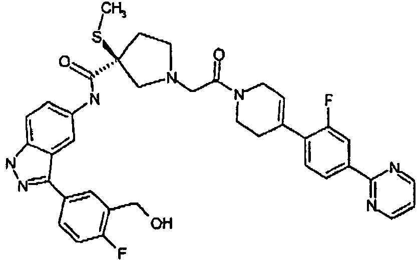 |
| 816 | 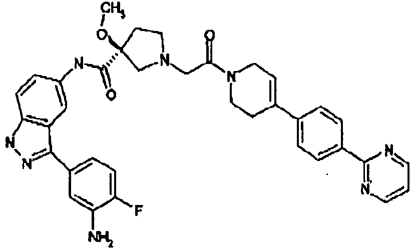 |
| 817 | 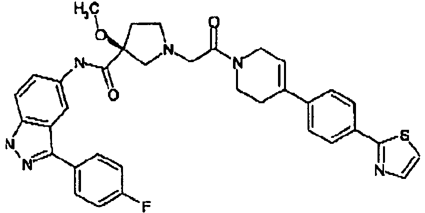 |
| 818 | 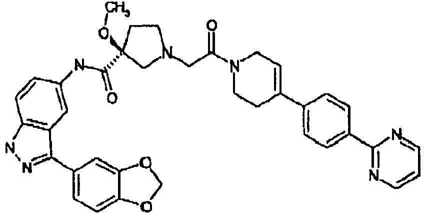 |
| 819 | 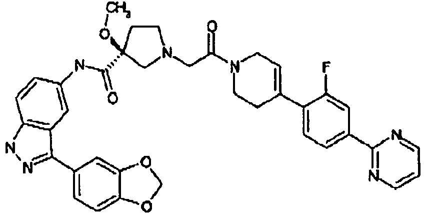 |
| 820 | 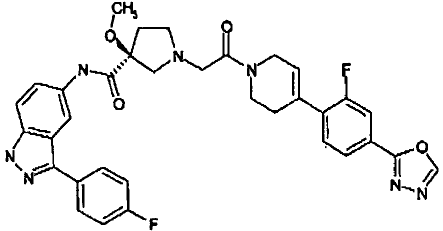 |
| 821 | 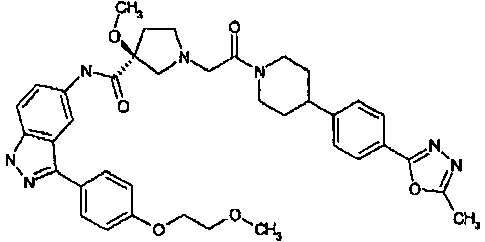 |
| 822 | 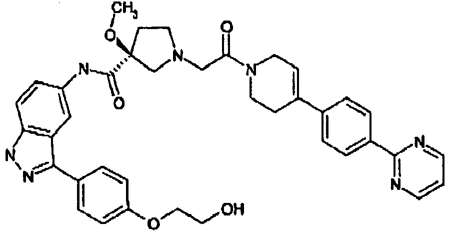 |
| 823 | 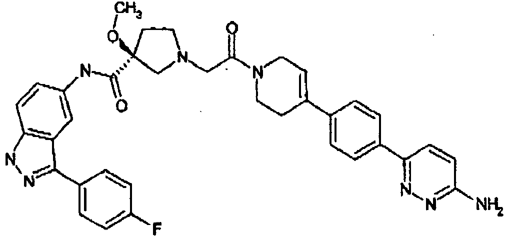 |
| 824 | 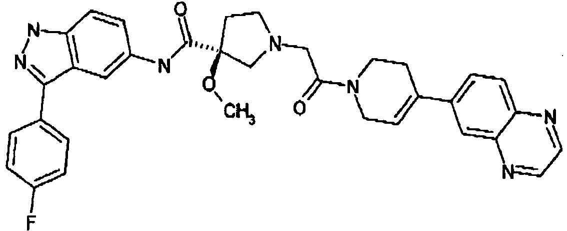 |
| 826 | 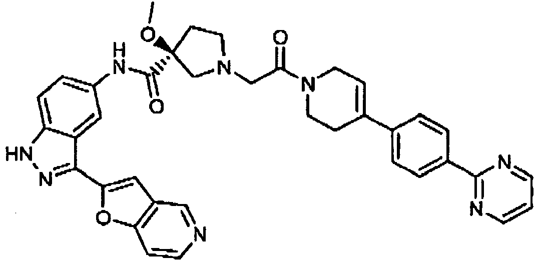 |
| 827 | 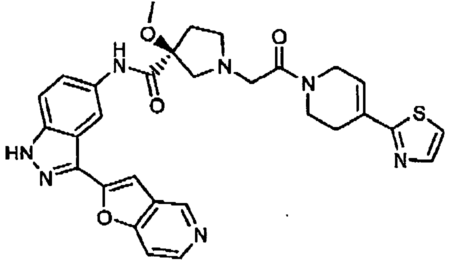 |
or a pharmaceutically acceptable salt thereof.

2. The ERK inhibitor of claim 1:
(a) having the formula: 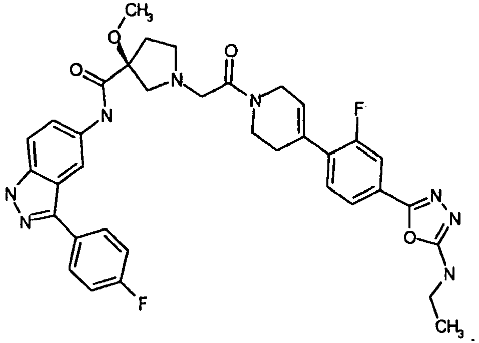 or
(b) having the formula: 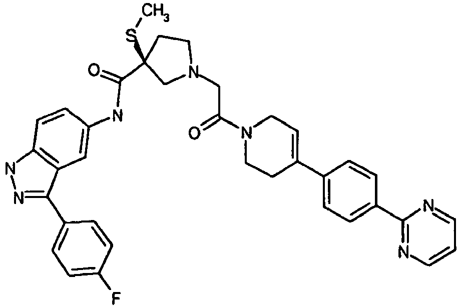 or a pharmaceutically acceptable salt thereof.

3. A pharmaceutical composition comprising at least one compound of claims 1 or 2 or a pharmaceutically acceptable salt thereof and a pharmaceutically acceptable carrier.

4. A use of at least one compound of claims 1 or 2 or a pharmaceutically acceptable salt thereof:
(1) for the manufacture of a medicament for treating cancer; or
(2) for the manufacture of a medicament for treating cancer, said medicament being used in combination with at least one chemotherapeutic agent; or
(3) for the manufacture of a medicament for treating cancer, said medicament being used in combination with at least one chemotherapeutic agent, and radiation therapy; or
(4) for the manufacture of a medicament for treating cancer, said medicament being used in combination with at least one chemotherapeutic agent selected from the group consisting of (1) taxanes, (2) platinum coordinator compounds, (3) epidermal growth factor (EGF) inhibitors that are antibodies, (4) EGF inhibitors that are small molecules, (5) vascular endolithial growth factor (VEGF) inhibitors that are antibodies, (6) VEGF kinase inhibitors that are small molecules, (7) estrogen receptor antagonists or selective estrogen receptor modulators (SERMs), (8) anti-turnor nucleoside derivatives, (9) epothilones, (10) topoisomerase inhibitors, (11) vinca alkaloids, (12) antibodies that are inhibitors of αVβ3 integrins, (13) folate antagonists, (14) ribonucleotide reductase inhibitors, (15) anthracyclines, (16) biologics; (17) inhibitors of angiogenesis and/or suppressors of tumor necrosis factor alpha (TNF-alpha) such as thalidomide (or related imid), (18) Bcr/abl kinase inhibitors, (19) MEK1 and/or MEK 2, inhibitors that are small molecules, (20) IGF-1 and IGF-2 inhibitors that are small molecules, (21) small molecule inhibitors of RAF and BRAF kinases, (22) small molecule inhibitors of cell cycle dependent kinases such as CDK1, CDK2, CDK4 and CDK6, (23) alkylating agents, and (24) farnesyl protein transferase inhibitors; or
(5) for the manufacture of a medicament for treating treating cancer, said medicament being administered with at least one signal transduction inhibitor; or
(6) for the manufacture of a medicament for treating cancer, said cancer being selected from the group consisting of: lung cancer, pancreatic cancer, colon cancer, myeloid leukemias, thyroid cancer, myelodysplastic syndrome, bladder carcinoma, epidermal carcinoma, melanoma, breast cancer, prostate cancer, head and neck cancers, ovarian cancer, brain cancers, cancers of mesenchymal origin, sarcomas, tetracarcinomas, nuroblastomas, kidney carcinomas, hepatomas, non-Hodgkin's lymphoma, multiple myeloma, and anaplastic thyroid carcinoma; or
(7) for the manufacture of a medicament for treating cancer wherein said cancer is selected from the group consisting of: melanoma, pancreatic cancer, thryroid cancer, colorectal cancer, lung cancer, breast cancer, and ovarian cancer; or
(8) for the manufacture of a medicament for treating cancer, said medicament being used in combination with an effective amout of at least one chemotherapeutic agent, wherein said cancer is selected from the group consisting of: melanoma, pancreatic cancer, thryroid cancer, colorectal canc er, lung cancer, breast cancer, and ovarian cancer; or
(9) for the manufacture of a medicament for treating melanoma; or
(10) for the manufacture of a medicament for treating melanoma, said medicament being used in combination with at least one chemotherapeutic agent; or
(11) for the manufacture of a medicament for treating pancreatic cancer, or
(12) for the manufacture of a medicament for treating pancreatic cancer, said medicament being used in combination with at least one chemotherapeutic agent; or
(13) for the manufacture of a medicament for treating thyroid cancer; or
(14) for the manufacture of a medicament for treating thyroid cancer, said medicament being used, in combination with at least one chemotherapeutic agent; or
(15) for the manufacture of a medicament for treating colorectal cancer; or
(16) for the manufacture of a medicament for treating colorectal cancer, said medicament being used in combination with at least one chemotherapeutic agent; or
(17) for the manufacture of a medicament for treating lung cancer; or
(18) for the manufacture of a medicament for treating lung cancer, said medicament being used in combination with at least one chemotherapeutic agent; or
(19) for the manufacture of a medicament for treating breast cancer; or
(20) for the manufacture of a medicament for treating breast cancer, said medicament being used in combination with at least one chemotherapeutic agent; or
(21) for the manufacture of a medicament for treating ovarian cancer; or
(22) for the manufacture of a medicament for treating ovarian cancer, said medicament being used in combination with at least one chemotherapeutic agent; or
(23) for the manufacture of a medicament for treating hormone-dependent breast cancer, said medicament being used in combination with antihormonal agents; or
(24) for the manufacture of a medicament for treating hormone-dependent breast cancer, said medicament being used in combination with antihormonal agents, and at least one chemotherapeutic agent; or
(25) for the manufacture of a medicament for preventing hormone-dependent breast cancer, said medicament being used in combination with antihormonal agents; or
(26) for the manufacture of a medicament for preventing hormone-dependent breast cancer, said medicament being used in combination with antihormonal agents, and at least one chemotherapeutic agent; or
(27) for the manufacture of a medicament for treating brain cancer; or
(28) for the manufacture of a medicament for treating brain cancer, said medicament being used in combination with at least one chemotherapeutic agent; or
(29) for the manufacture of a medicament for treating brain cancer, said medicament being used in combination with a chemotherapeutic agent wherein said chemotherapeutic agent is temozolomide; or
(30) for the manufacture of a medicament for treating prostate cancer; or
(31) for the manufacture of a medicament for treating prostate cancer, said medicament being used in combination with at least one chemotherapeutic agent; or
(32) for the manufacture of a medicament for treating myelodysplastic syndrome; or
(33) for the manufacture of a medicament for treating myelodysplastic syndrome, said medicament being used in combination with at least one chemotherapeutic agent; or
(34) for the manufacture of a medicament for treating myeloid leukemias; or
(35) for the manufacture of a medicament for treating myeloid leukemias, said medicament being used in combination with at least one chemotherapeutic agent; or
(36) for the manufacture of a medicament for treating acute myelogenous leukemia; or
(37) for the manufacture of a medicament for treating acute myelogenous leukemia, said medicament being used in combination with at least one chemotherapeutic agent; or
(38) for the manufacture of a medicament for treating chronic myelomonocytic leukemia; or
(39) for the manufacture of a medicament for treating chronic myelomonocytic leukemia, said medicament being used in combination with at least one chemotherapeutic agent; or
(40) for the manufacture of a medicament for treating chronic myelogenous leukemia; or
(41) for the manufacture of a medicament for treating chronic myelogenous leukemia, said medicament being used in combination with at least one chemotherapeutic agent; or
(42) for the manufacture of a medicament for treating bladder cancer; or
(43) for the manufacture of a medicament for treating bladder cancer, said medicament being used in combination with at least one chemotherapeutic agent; or
(44) for the manufacture of a medicament for treating non-Hodgkin's lymphoma; or
(45) for the manufacture of a medicament for treating non-Hodgkin's lymphoma, said medicament being used in combination with at least one chemotherapeutic agent; or
(46) for the manufacture of a medicament for treating multiple myeloma; or
(47) for the manufacture of a medicament for treating multiple myeloma, said medicament being used in combination with at least one chemotherapeutic agent.

5. A compound of claim 1 or 2, or a pharmaceutically acceptable salt thereof, for use in a method of treatment of the human body by therapy.

6. A compound of claim 5 wherein the therapy is as defined in claim 4.

7. A combination of a compound of claim 1 or 2, or a pharmaceutically acceptable salt thereof, and at least one chemotherapeutic agent selected from:
(1) taxanes,
(2) platinum coordinator compounds,
(3) epidermal growth factor (EGF) inhibitors that are antibodies,
(4) EGF inhibitors that are small molecules,
(5) vascular endolithial growth factor (VEGF) inhibitors that are antibodies,
(6) VEGF kinase inhibitors that are small molecules,
(7) estrogen receptor antagonists or selective estrogen receptor modulators (SERMs),
(8) anti-tumor nucleoside derivatives,
(9) epothilones,
(10) topoisomerase inhibitors,
(11) vinca alkaloids,
(12) antibodies that are inhibitors of αVβ3 integrins,
(13) folate antagonists,
(14) ribonucleotide reductase inhibitors,
(15) anthracyclines,
(16) biologics;
(17) inhibitors of angiogenesis and/or suppressors of tumor necrosis factor alpha (TNF-alpha) such as thalidomide (or related imid),
(18) Bcr/abl kinase inhibitors,
(19) MEK1 and/or MEK 2 inhibitors that are small molecules,
(20) IGF-1 and IGF-2 inhibitors that are small molecules,
(21) small molecule inhibitors of RAF and BRAF kinases,
(22) small molecule inhibitors of cell cycle dependent kinases such as CDK1, CDK2, CDK4 and CDK6,
(23) alkylating agents, and
(24) farnesyl protein transferase inhibitors (also know as FPT inhibitors or FTI (i.e., farnesyl transfer inhibitors)).

## Patentansprüche

1. Ein ERK-Inhibitor, ausgewählt aus der Gruppe, bestehend aus den Verbindungen der folgenden Strukturen:
| Beispiel Nr. | VERBINDUNG |
|---|---|
| 612 | 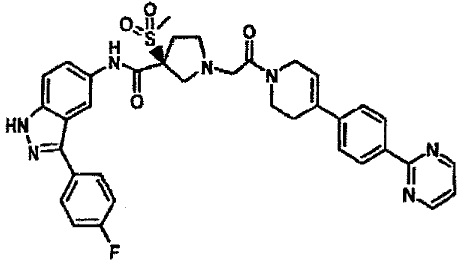 |
| 613 | 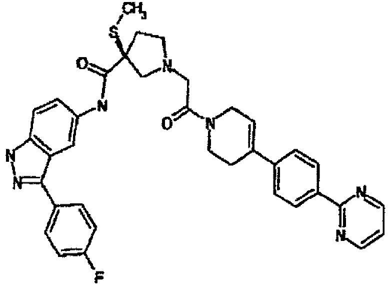 |
| 614 | 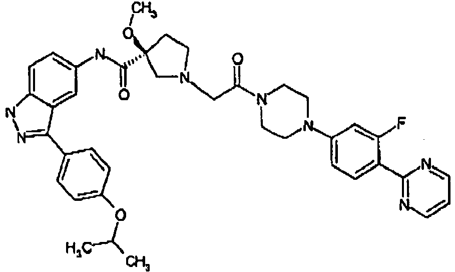 |
| 615 | 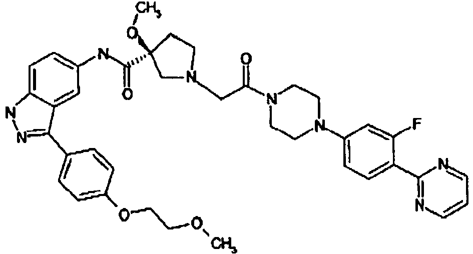 |
| 616 | 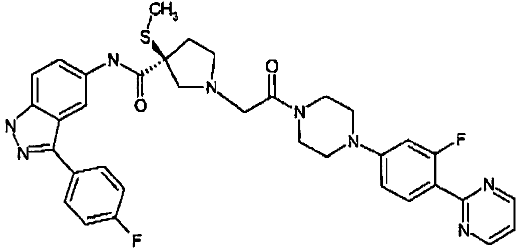 |
| 617 | 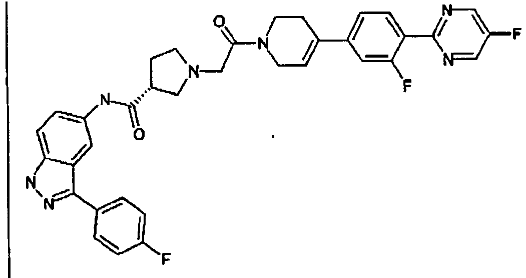 |
| 619 | 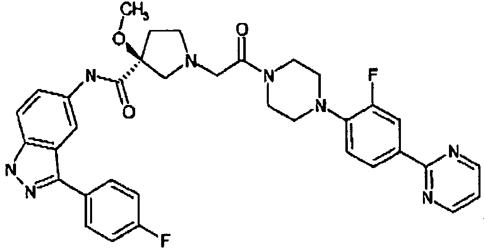 |
| 620 | 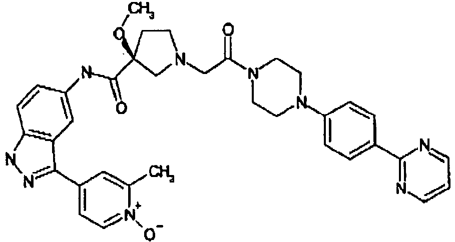 |
| 621 | 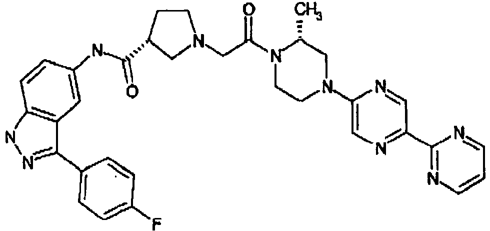 |
| 622 | 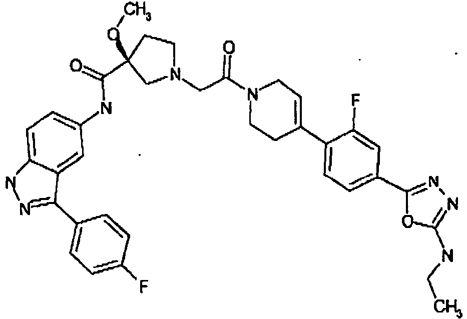 |
| 623 | 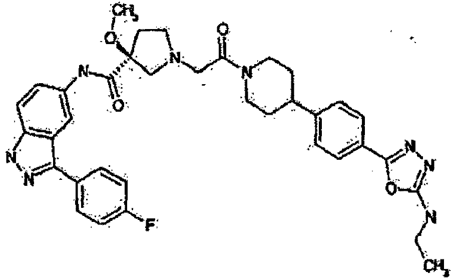 |
| 624 | 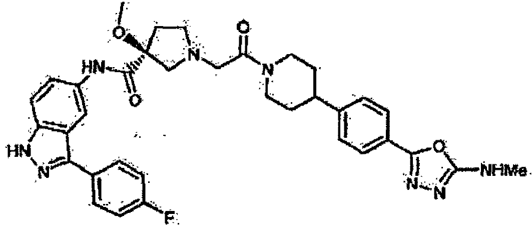 |
| | |
| 625 | 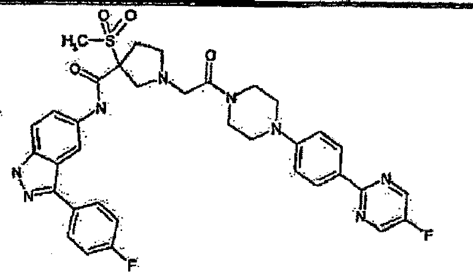 |
| 626 | 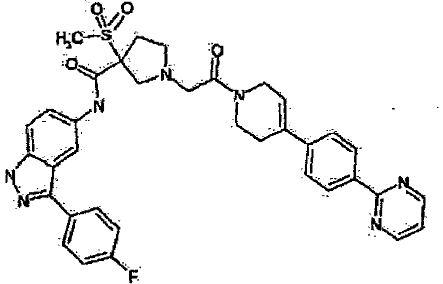 |
| 627 | 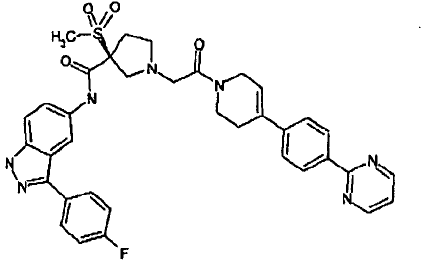 |
| 628 | 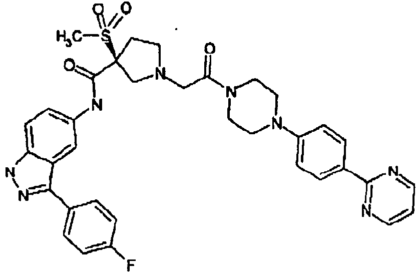 |
| 629 | 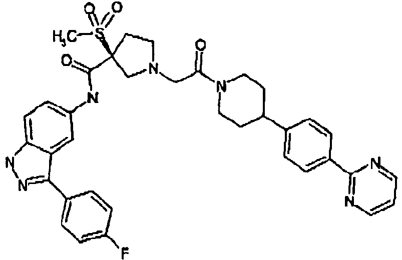 |
| 630 | 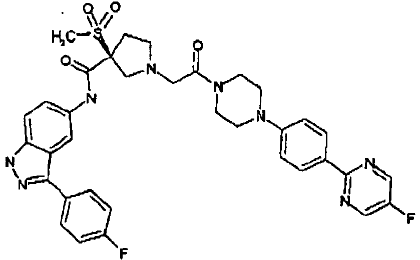 |
| 631 | 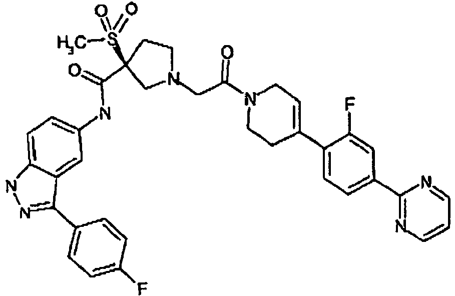 |
| 632 | 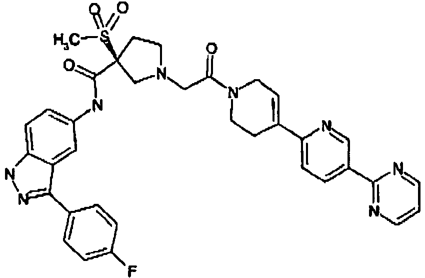 |
| 633 | 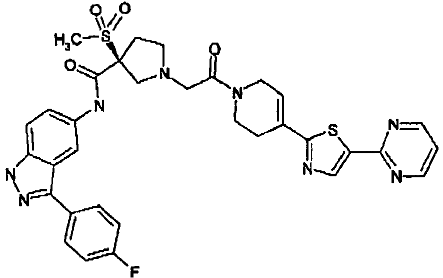 |
| 634 | 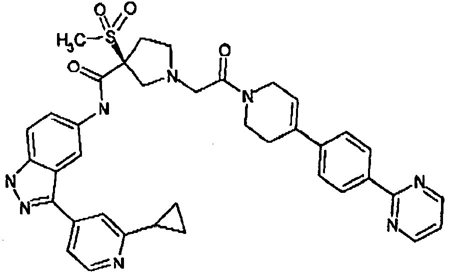 |
| 635 | 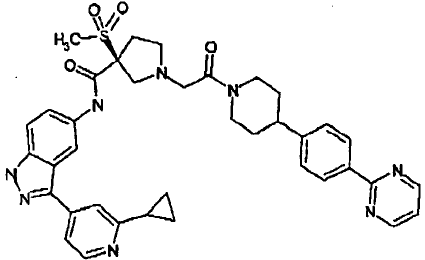 |
| 636 | 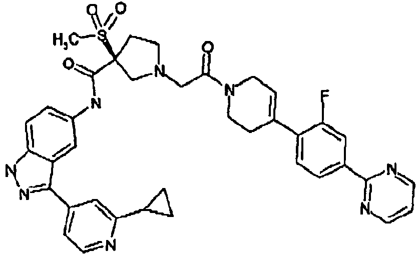 |
| 637 | 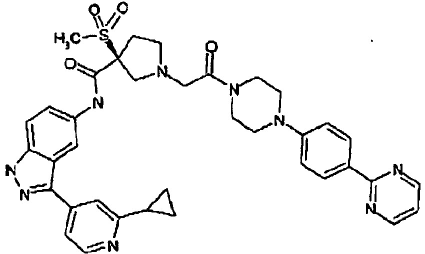 |
| 638 | 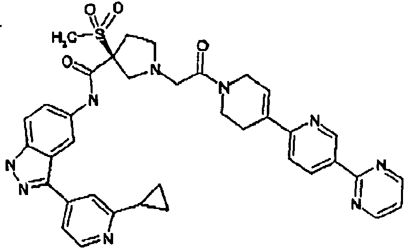 |
| 639 | 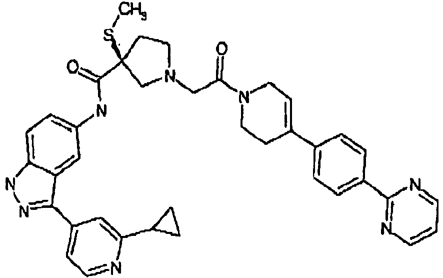 |
| 640 | 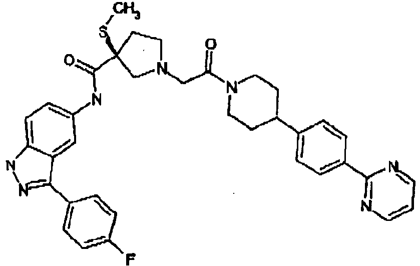 |
| 641 | 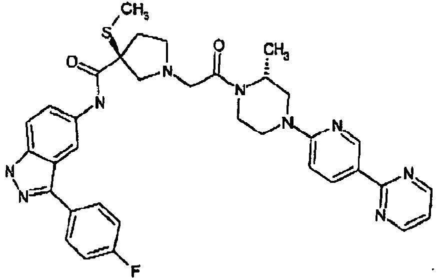 |
| 642 | 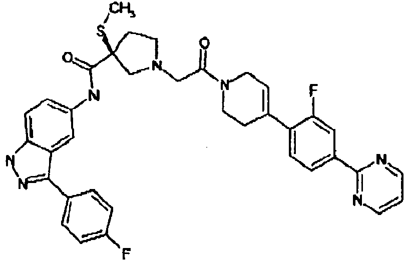 |
| 643 | 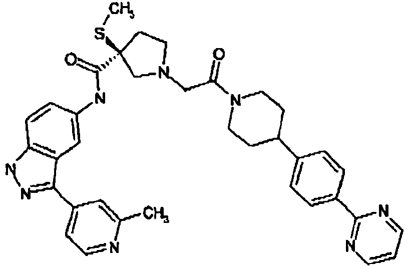 |
| 644 | 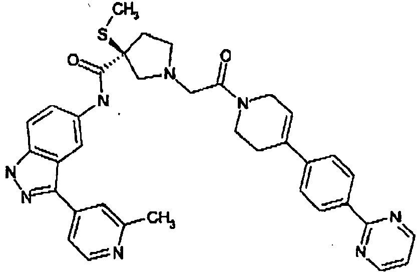 |
| 645 | 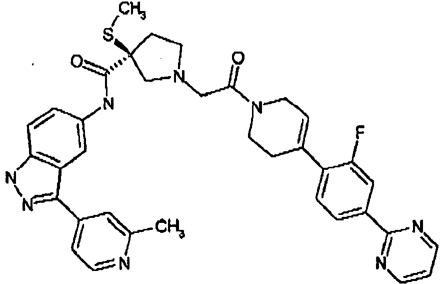 |
| 646 | 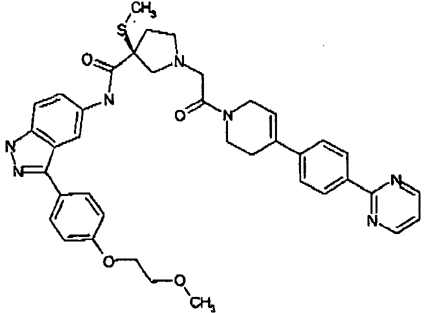 |
| 647 | 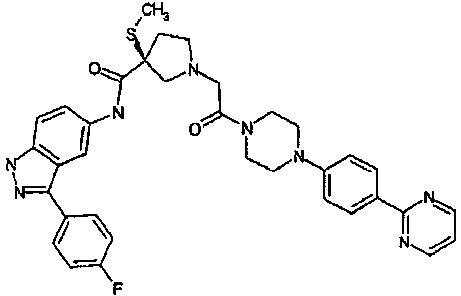 |
| 648 | 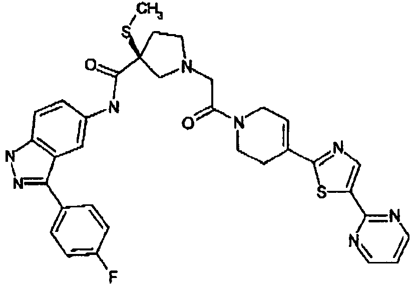 |
| 649 | 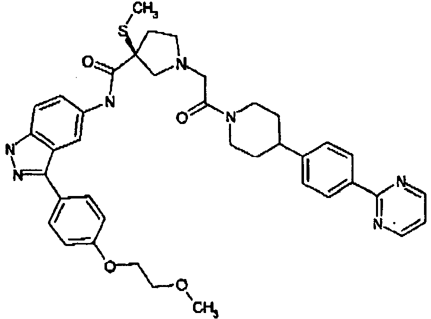 |
| 650 | 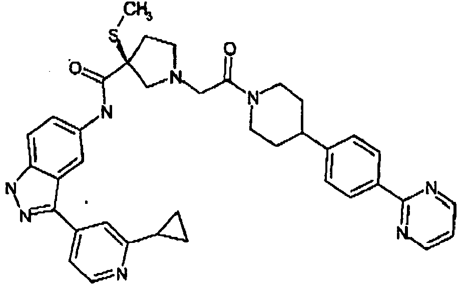 |
| 651 | 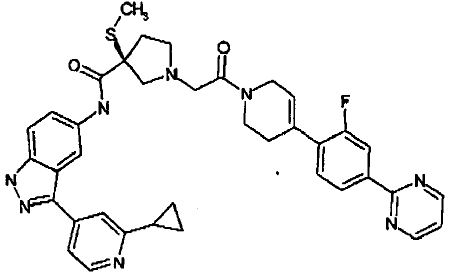 |
| 652 | 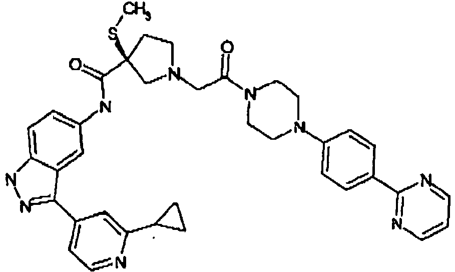 |
| 653 | 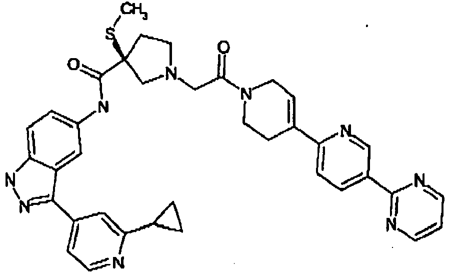 |
| 654 | 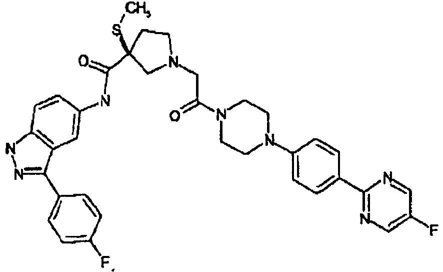 |
| 655 | 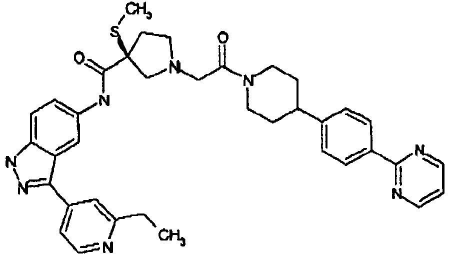 |
| 656 | 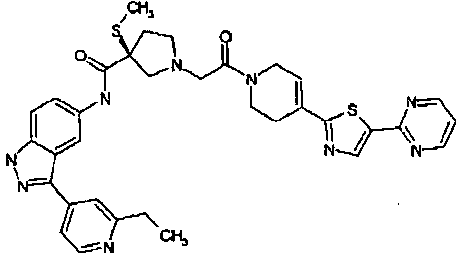 |
| 657 | 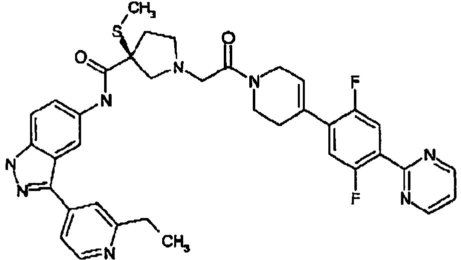 |
| 658 | 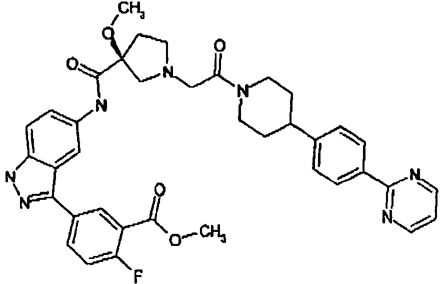 |
| 659 | 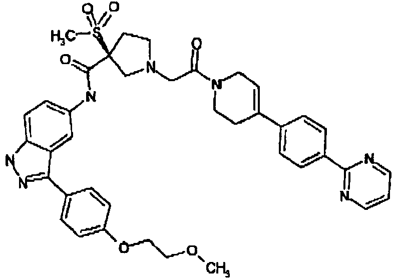 |
| 660 | 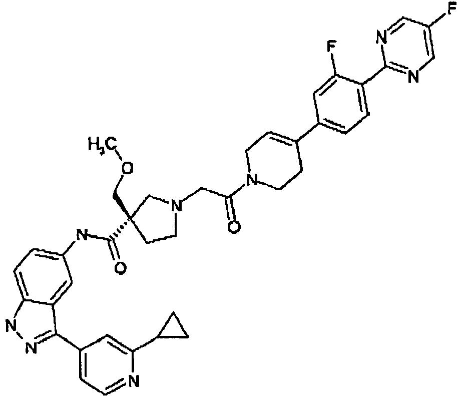 |
| 661 | 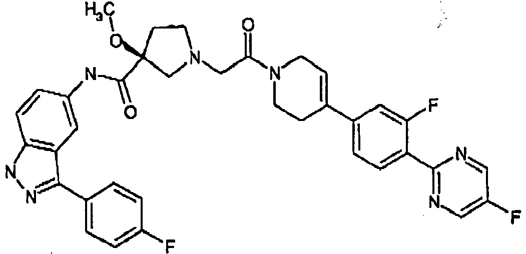 |
| 662 | 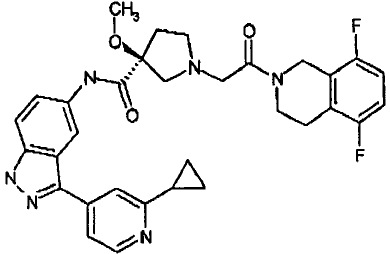 |
| 663 | 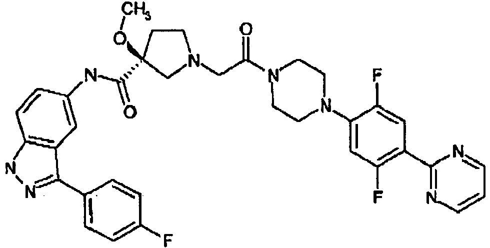 |
| 664 | 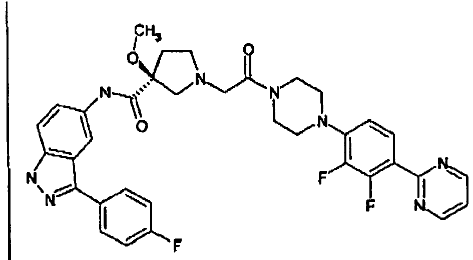 |
| 665 | 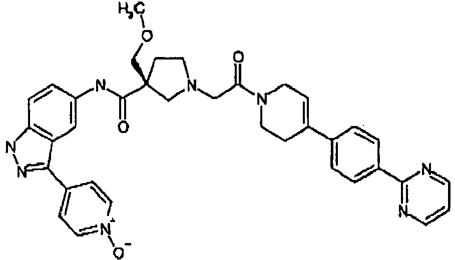 |
| 666 | 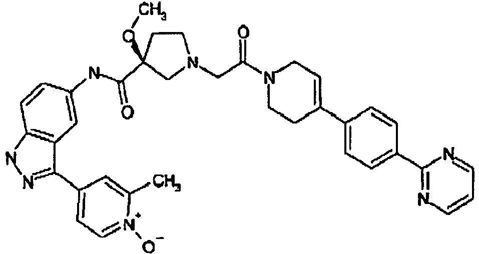 |
| 667 | 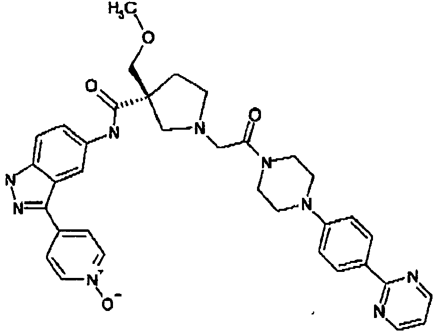 |
| 668 | 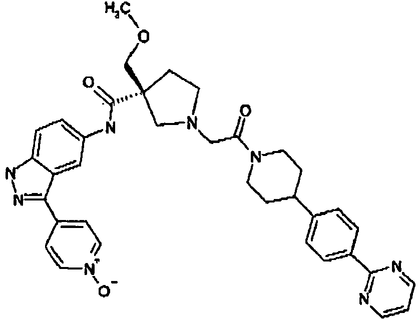 |
| 669 | 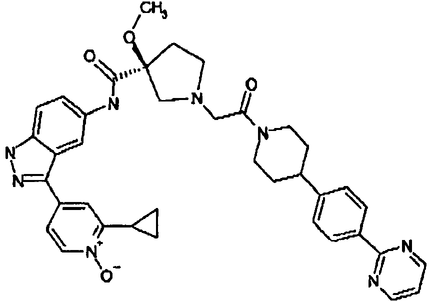 |
| 670 | 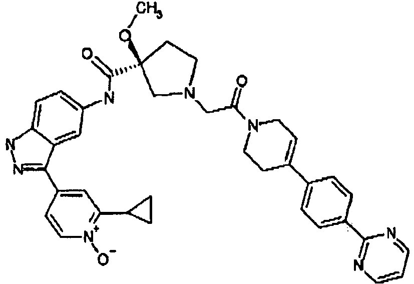 |
| 671 | 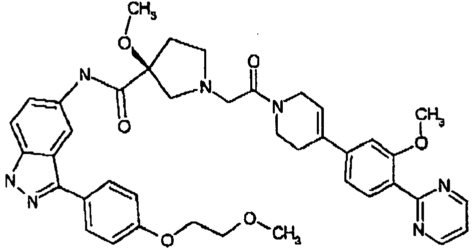 |
| 672 | 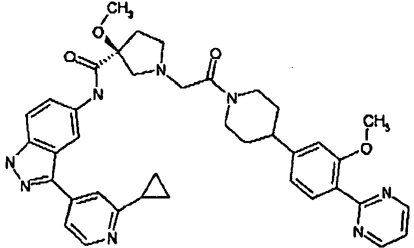 |
| 673 | 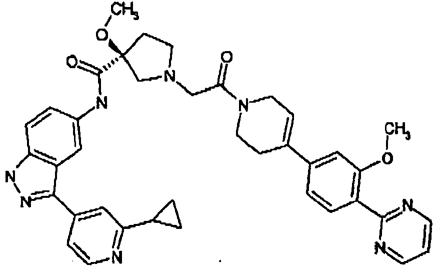 |
| 674 | 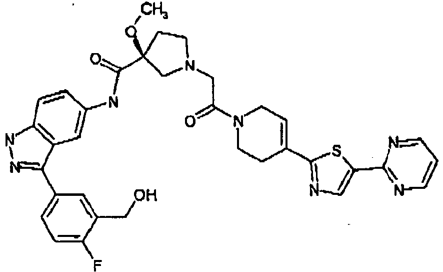 |
| 675 | 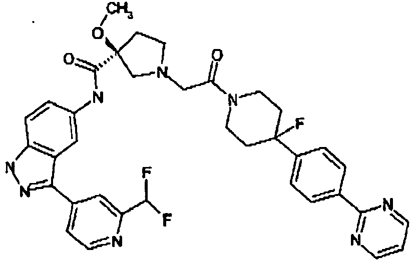 |
| 676 | 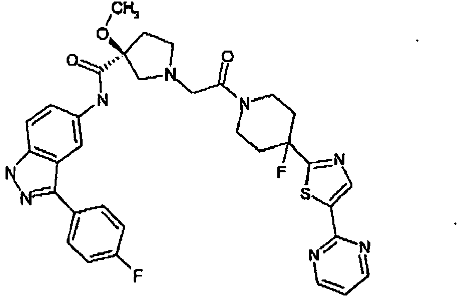 |
| 677 | 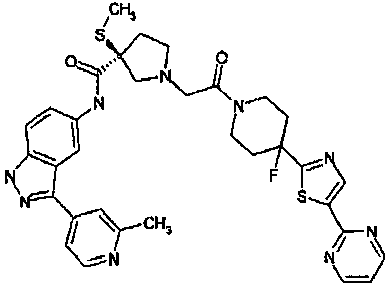 |
| 678 | 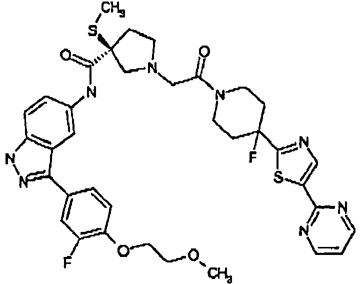 |
| 679 | 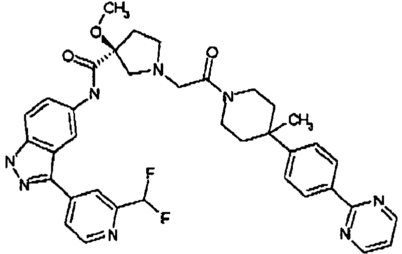 |
| 680 | 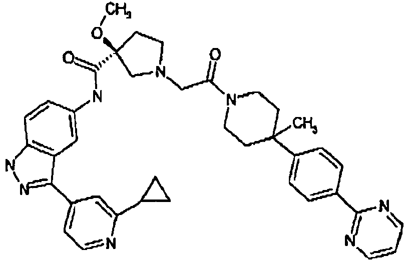 |
| 681 | 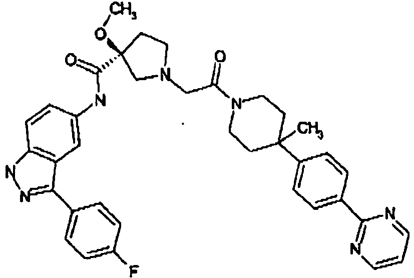 |
| 682 | 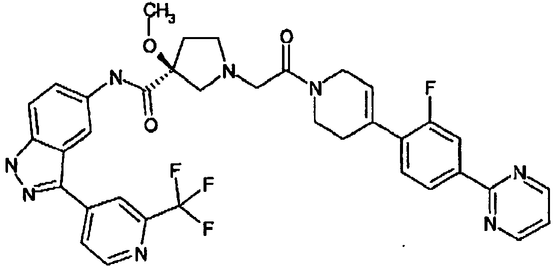 |
| 683 | 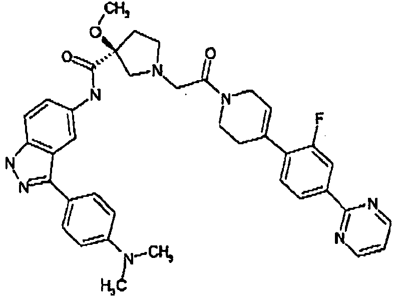 |
| 684 | 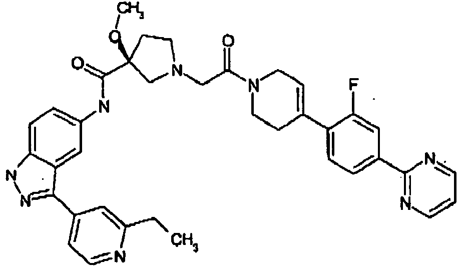 |
| 685 | 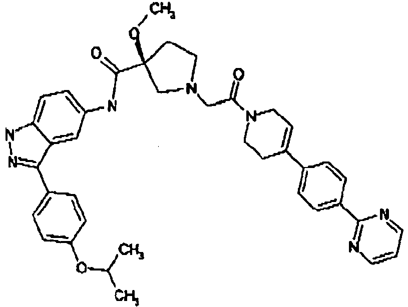 |
| 686 | 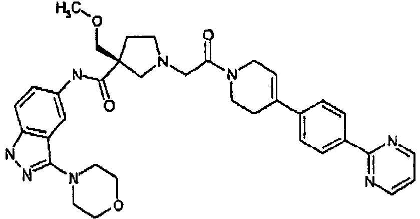 |
| 687 | 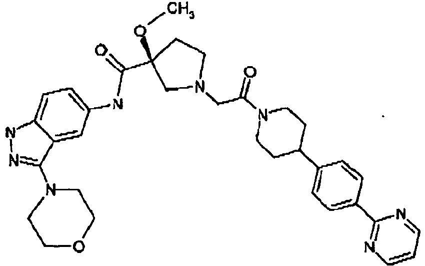 |
| 688 | 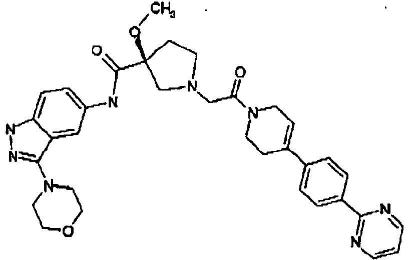 |
| 689 | 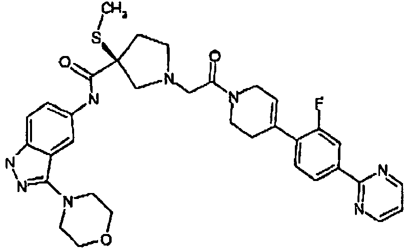 |
| 690 | 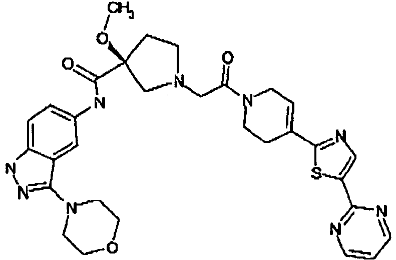 |
| 691 | 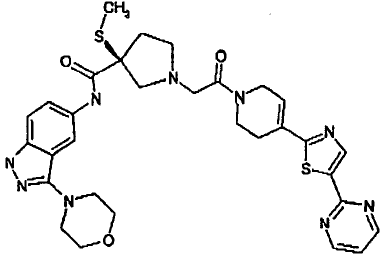 |
| 692 | 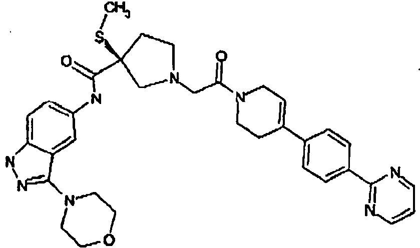 |
| 693 | 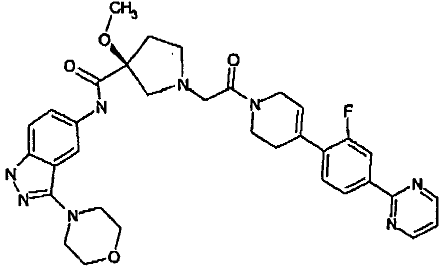 |
| 694 | 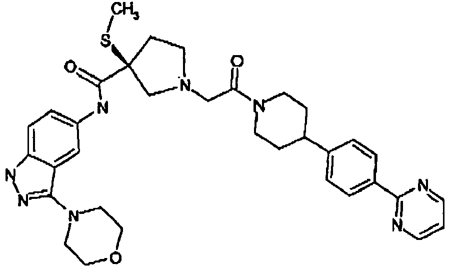 |
| 695 | 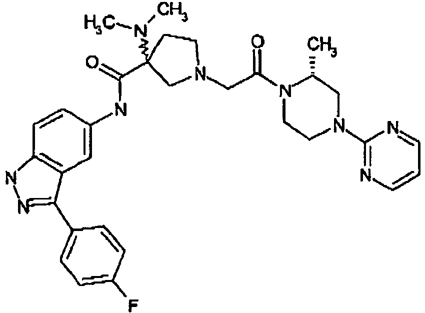 |
| 696 | 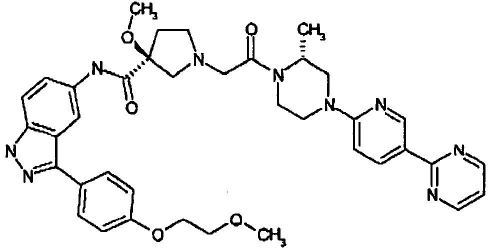 |
| 697 | 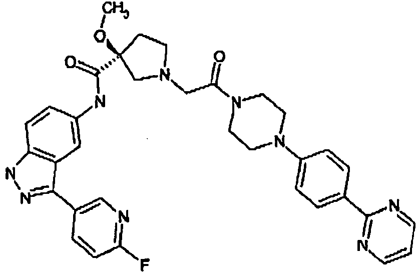 |
| 698 | 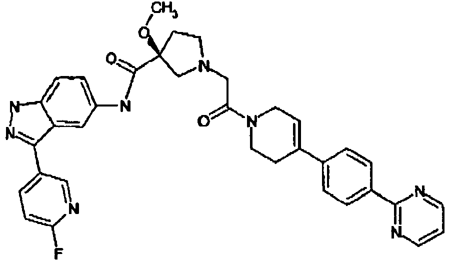 |
| 699 | 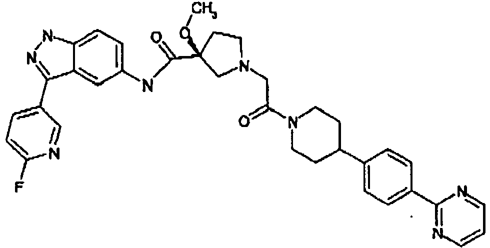 |
| 700 | 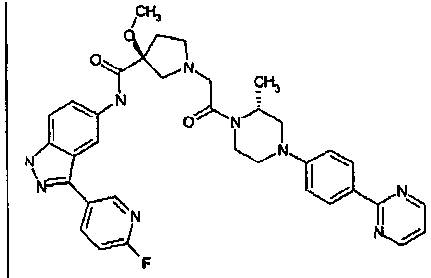 |
| 701 | 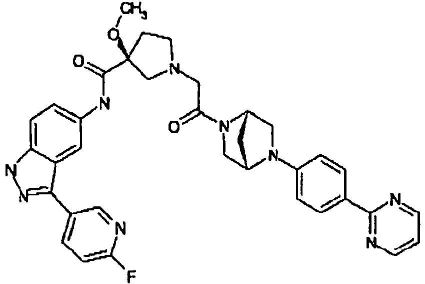 |
| 702 | 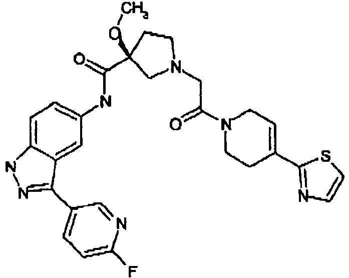 |
| 703 | 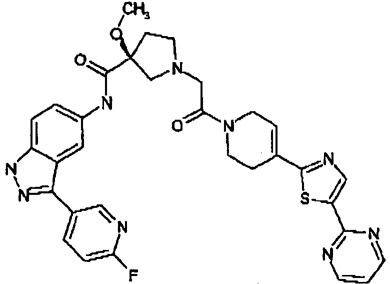 |
| 704 | 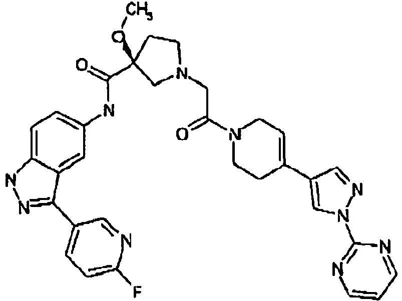 |
| 705 | 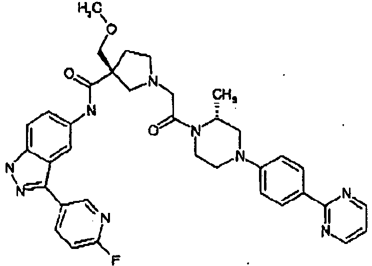 |
| 706 | 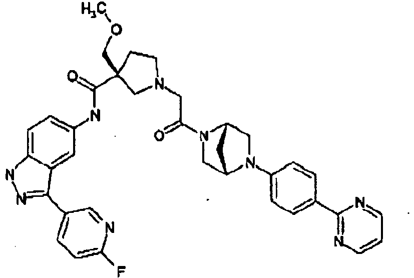 |
| 707 | 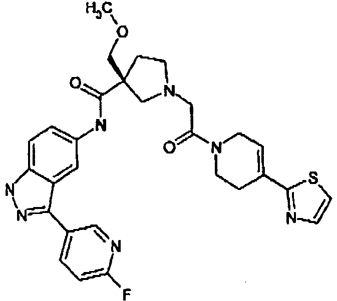 |
| 708 | 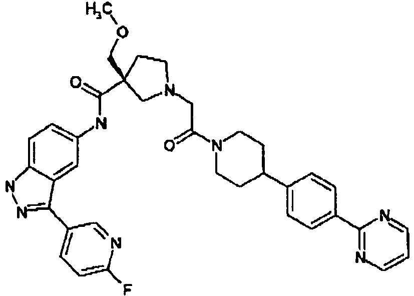 |
| 709 | 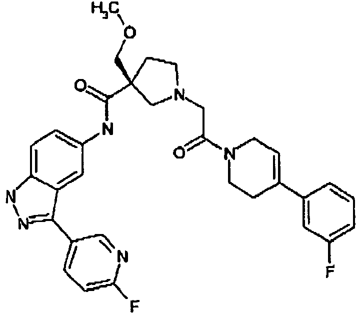 |
| 710 | 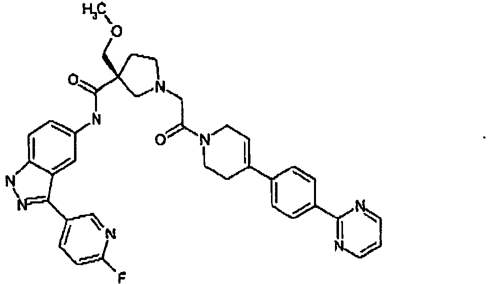 |
| 711 | 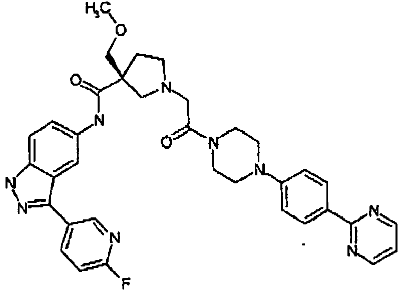 |
| 712 | 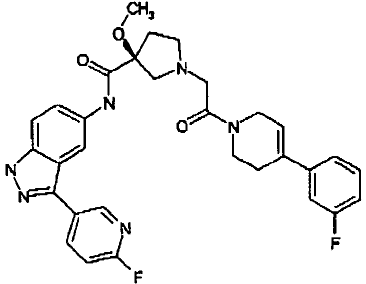 |
| 713 | 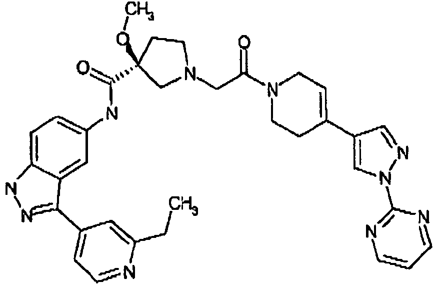 |
| 714 | 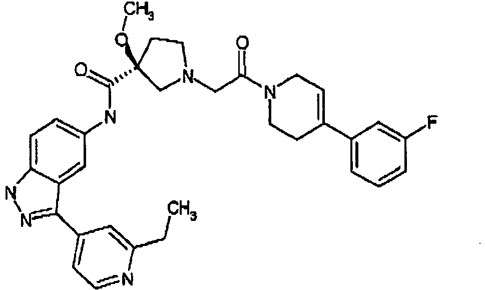 |
| 715 | 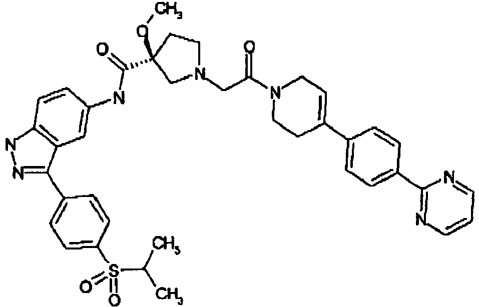 |
| 716 | 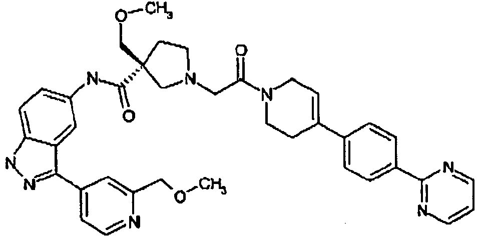 |
| 717 | 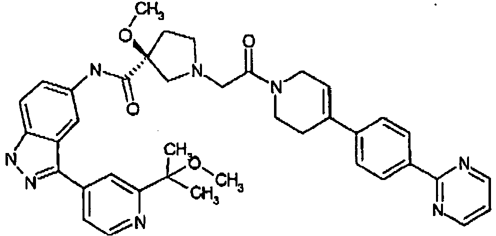 |
| 718 | 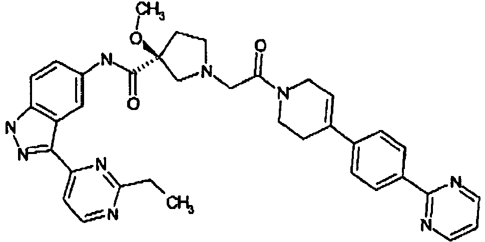 |
| 719 | 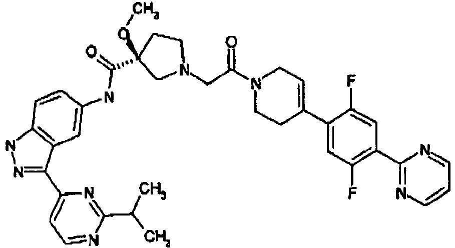 |
| 720 | 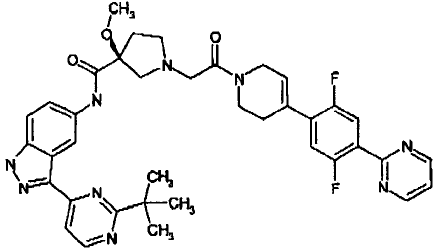 |
| 721 | 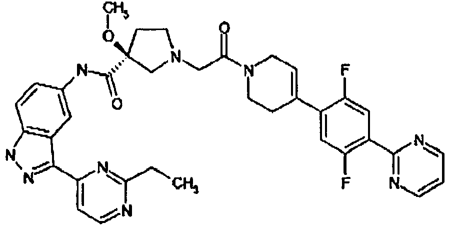 |
| 722 | 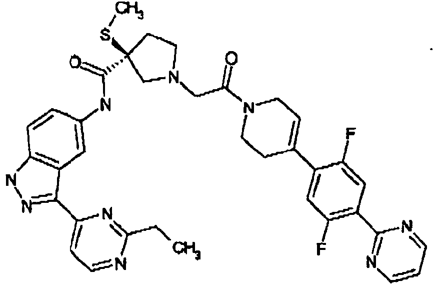 |
| 723 | 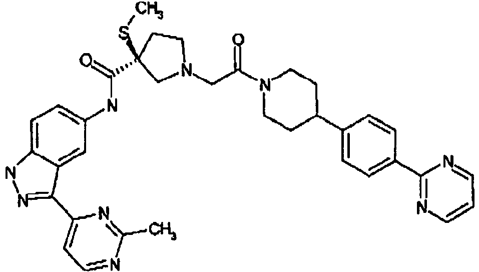 |
| 724 | 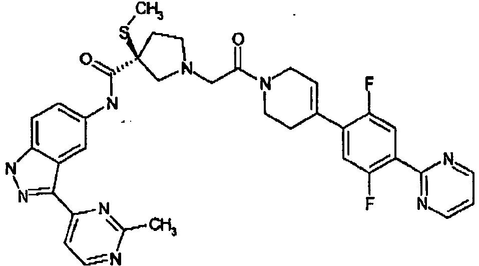 |
| 725 | 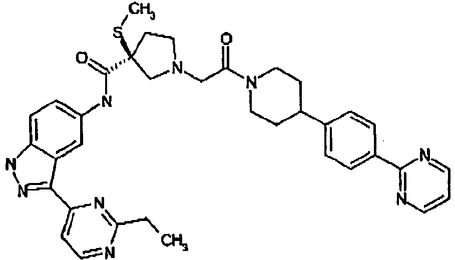 |
| 726 | 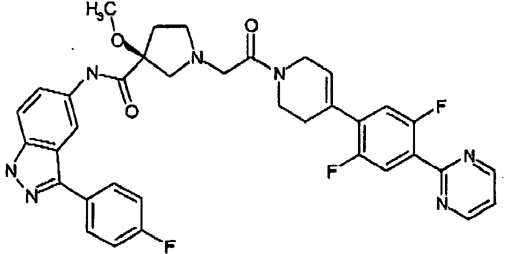 |
| 727 | 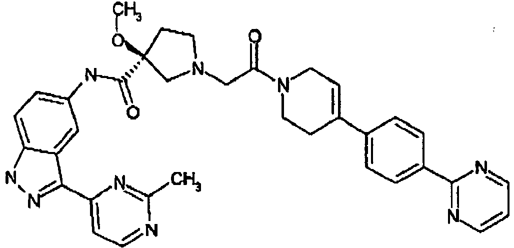 |
| 728 | 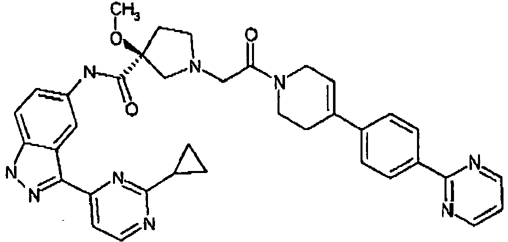 |
| 729 | 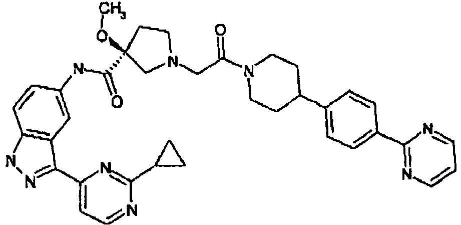 |
| 730 | 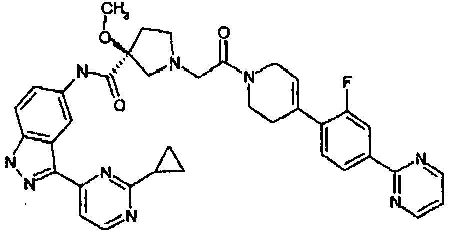 |
| 731 | 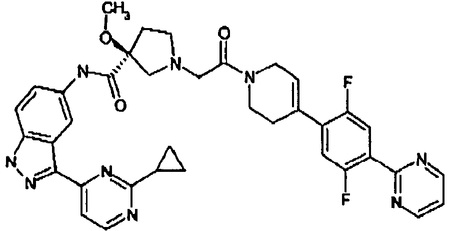 |
| 732 | 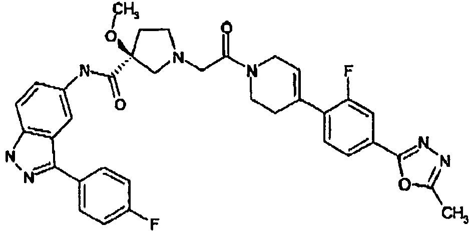 |
| 733 | 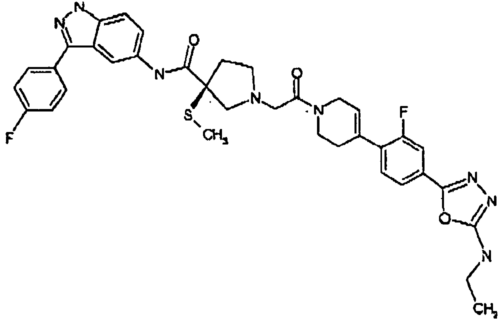 |
| 734 | 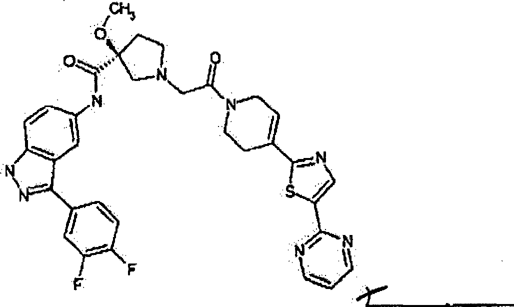 |
| 735 | 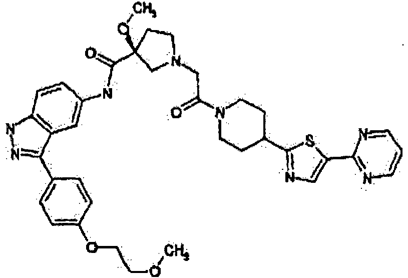 |
| 736 | 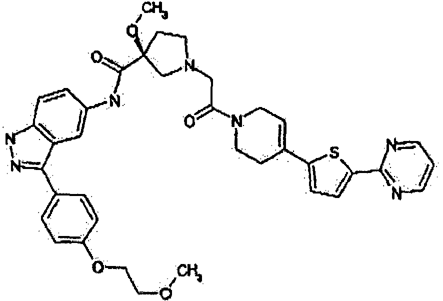 |
| 737 | 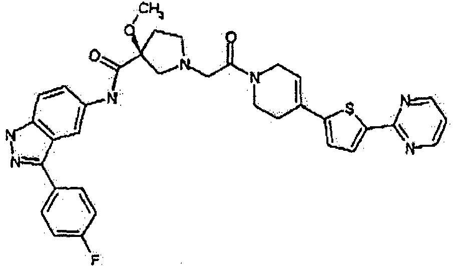 |
| 738 | 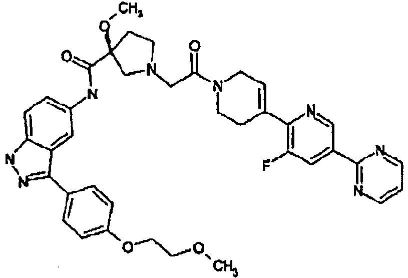 |
| 739 | 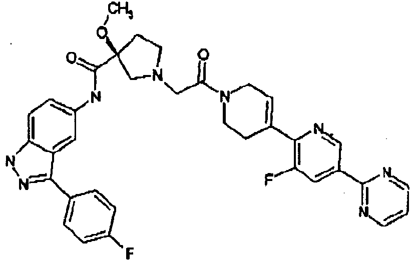 |
| 740 | 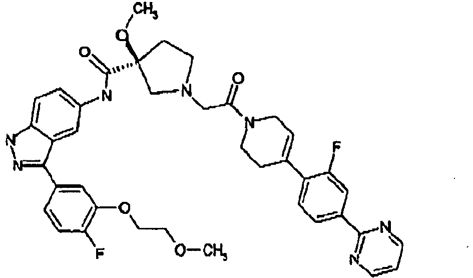 |
| 741 | 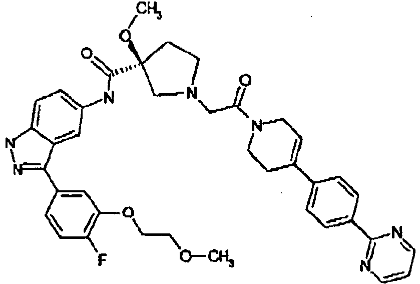 |
| 742 | 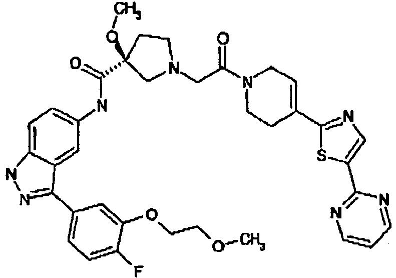 |
| 743 | 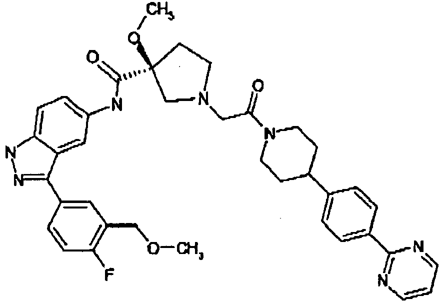 |
| 744 | 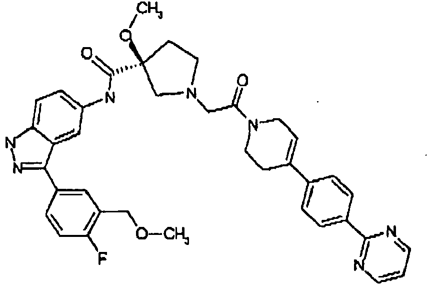 |
| 745 | 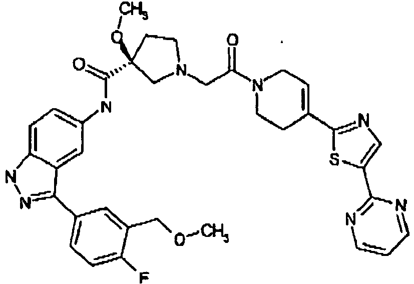 |
| 746 | 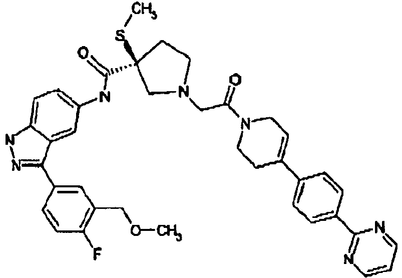 |
| 747 | 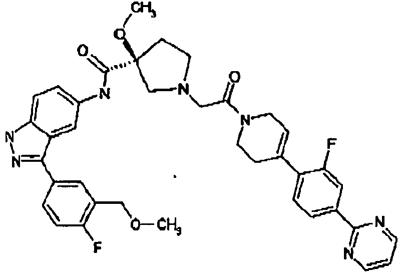 |
| 748 | 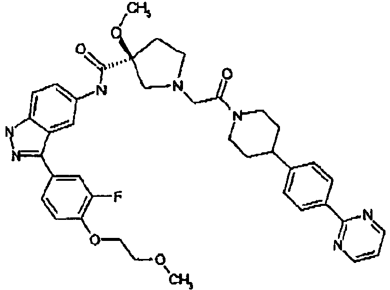 |
| 749 | 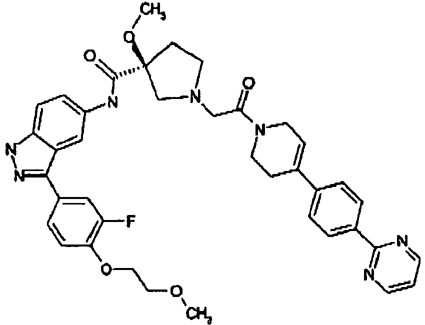 |
| 750 | 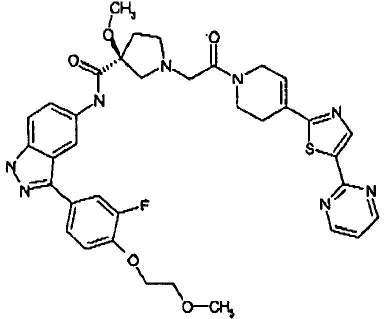 |
| 751 | 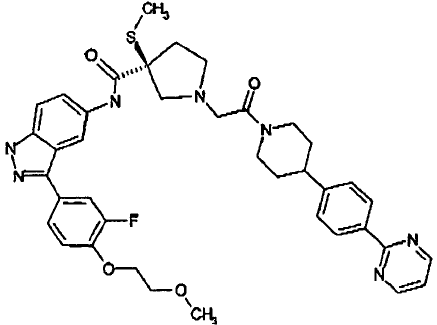 |
| 752 | 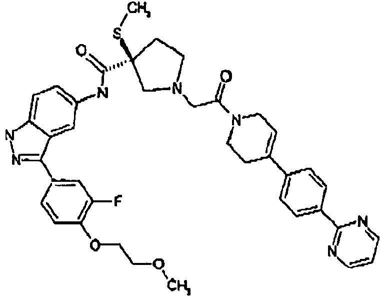 |
| 753 | 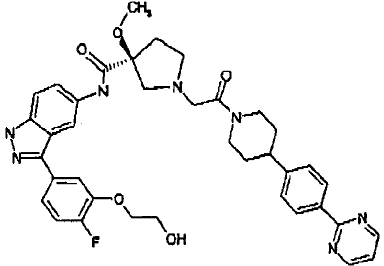 |
| 754 | 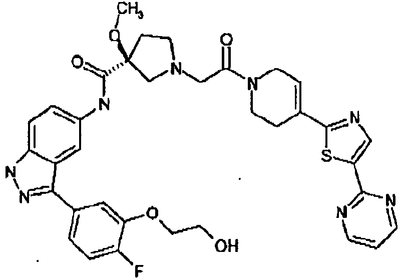 |
| 755 | 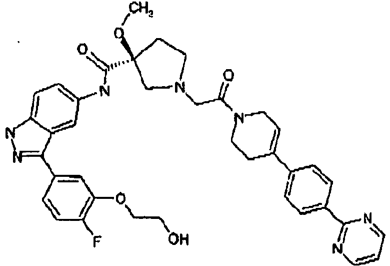 |
| 756 | 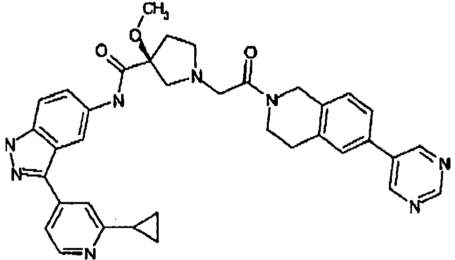 |
| 757 | 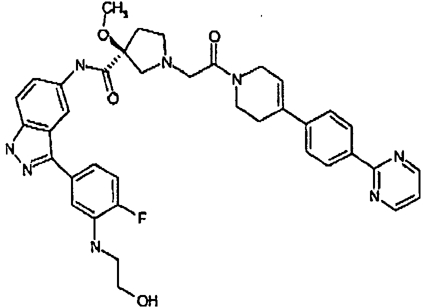 |
| 758 | 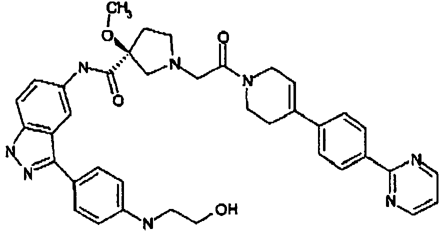 |
| 759 | 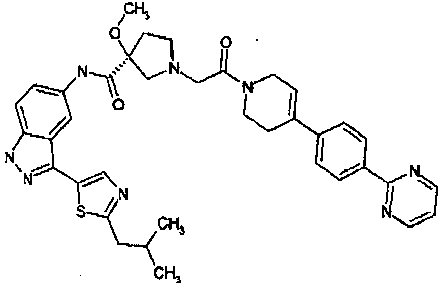 |
| 760 | 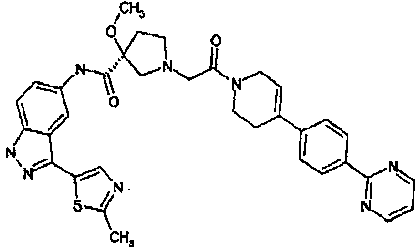 |
| 761 | 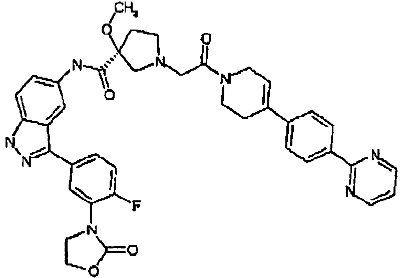 |
| 762 | 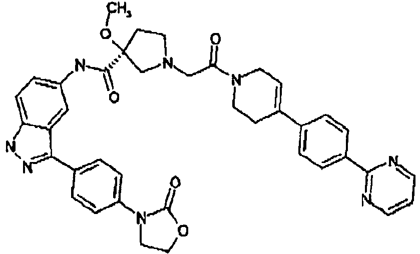 |
| 763 | 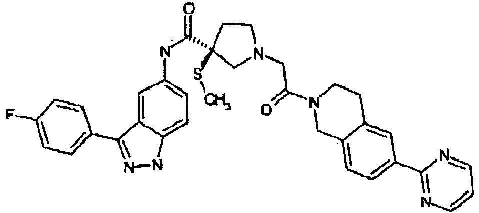 |
| 764 | 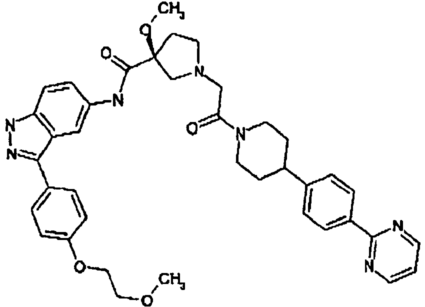 |
| 765 | 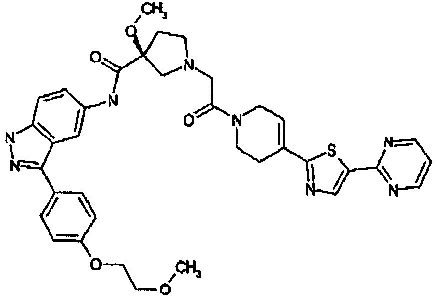 |
| 766 | 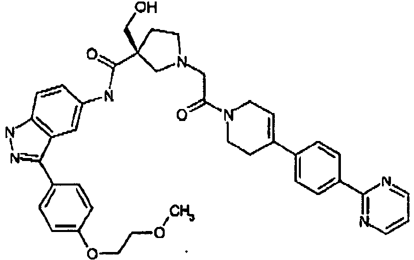 |
| 767 | 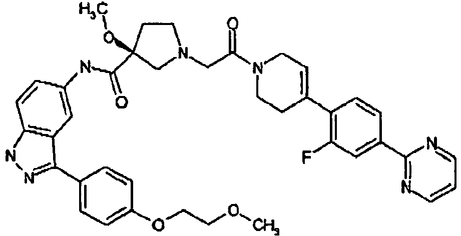 |
| 768 | 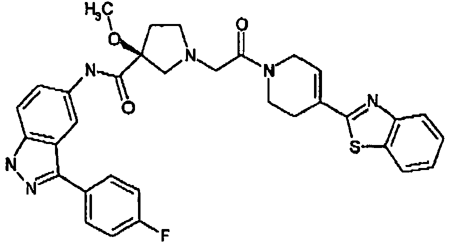 |
| 769 | 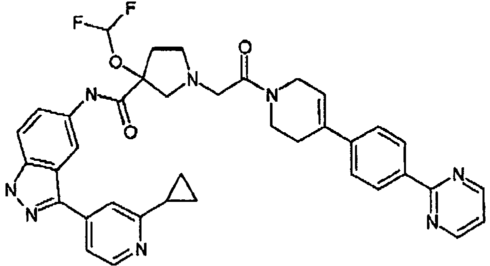 |
| 770 | 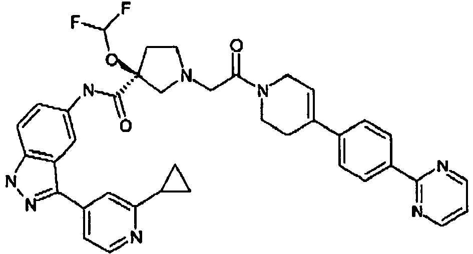 |
| 771 | 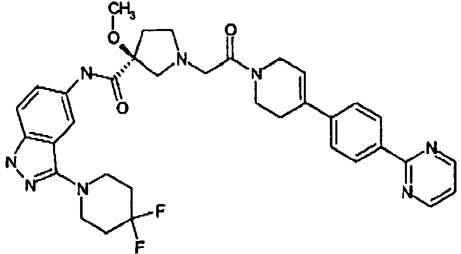 |
| 772 | 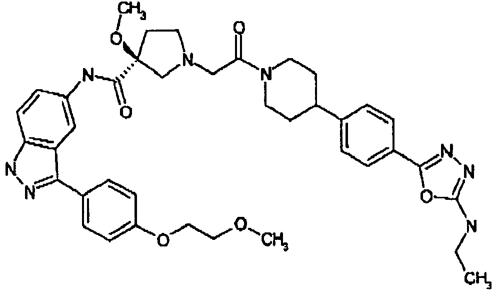 |
| 773 | 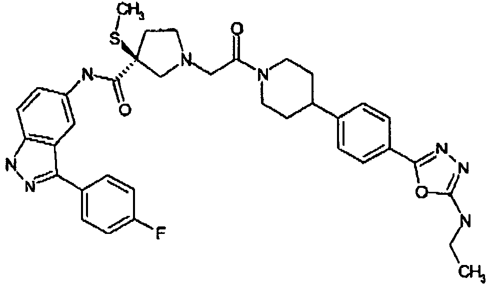 |
| 774 | 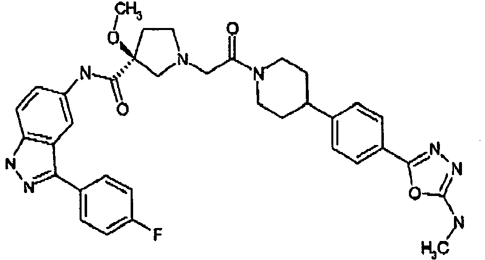 |
| 775 | 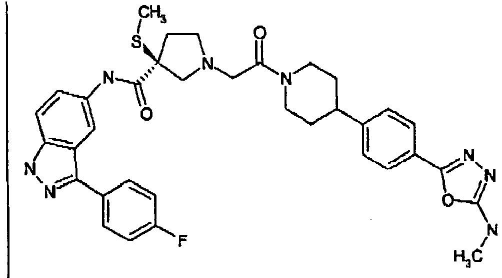 |
| 776 | 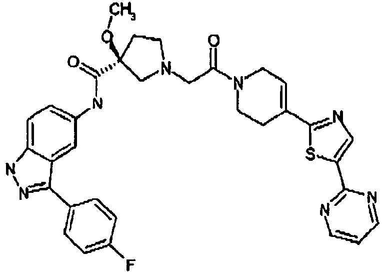 |
| 777 | 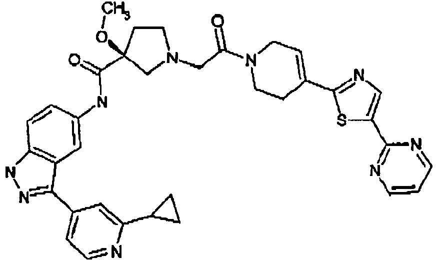 |
| 778 | 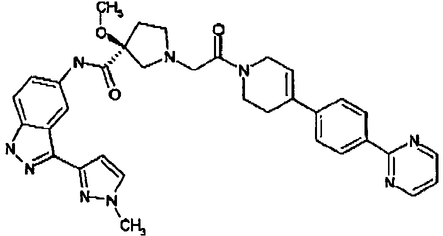 |
| 779 | 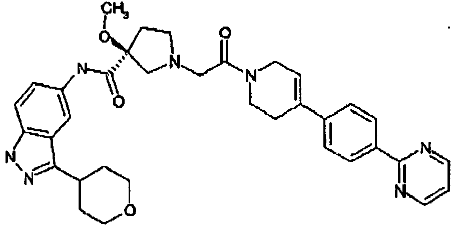 |
| 780 | 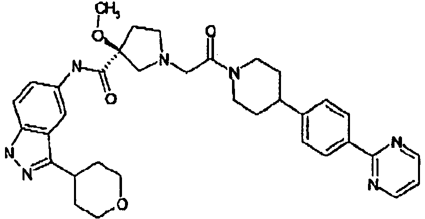 |
| 781 | 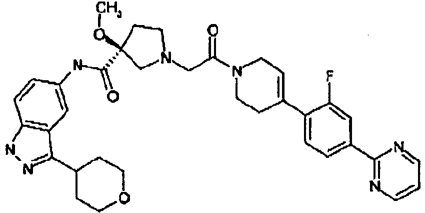 |
| 782 | 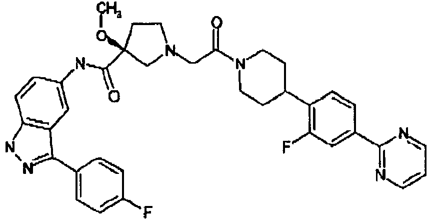 |
| 783 | 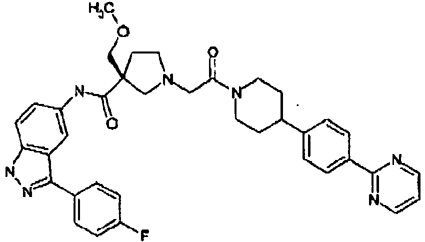 |
| 784 | 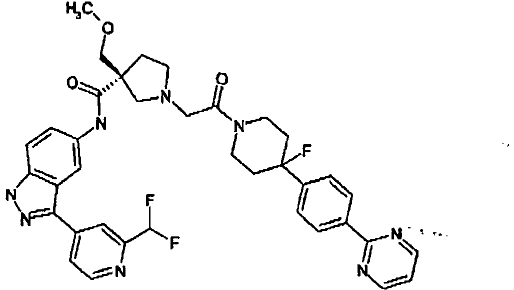 |
| 785 | 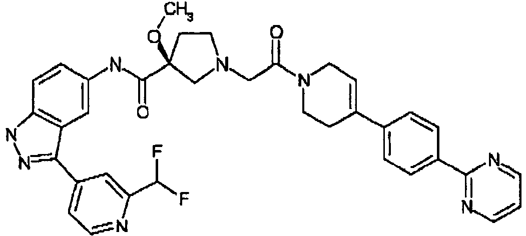 |
| 786 | 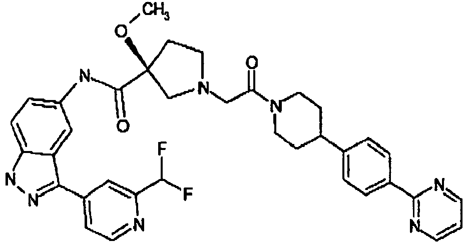 |
| 787 | 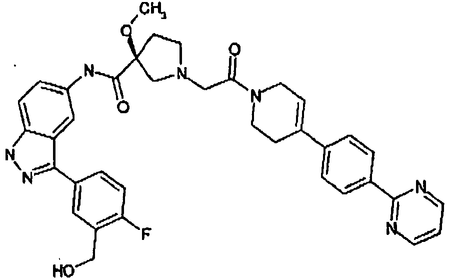 |
| 788 | 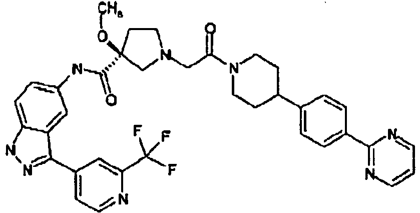 |
| 789 | 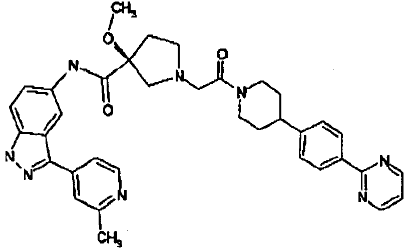 |
| 790 | 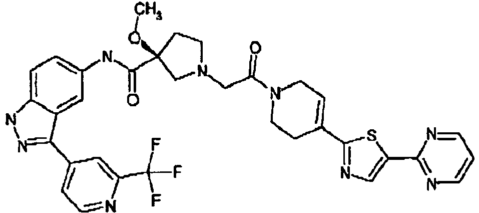 |
| 791 | 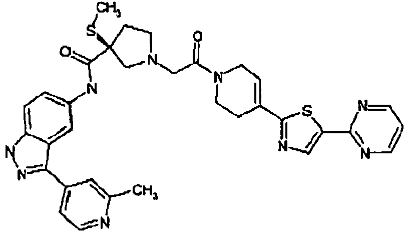 |
| 792 | 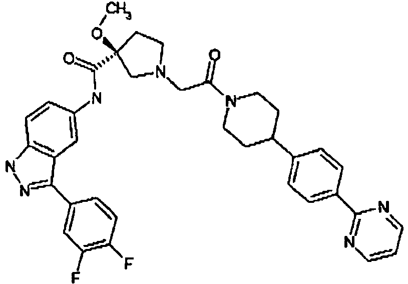 |
| 793 | 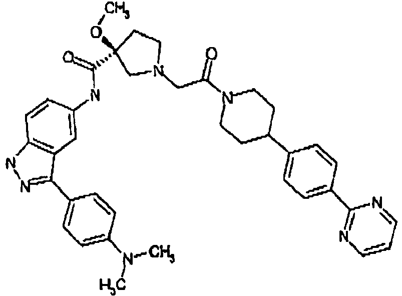 |
| 794 | 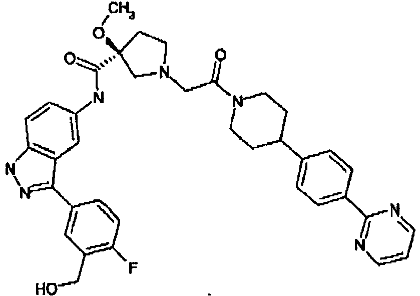 |
| 795 | 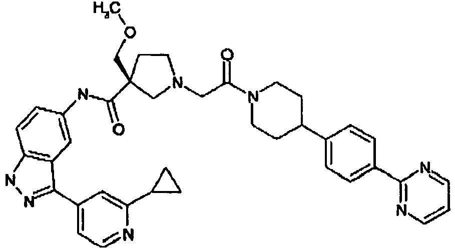 |
| 796 | 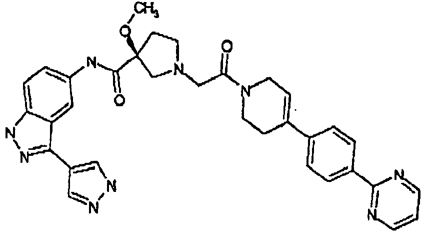 |
| 797 | 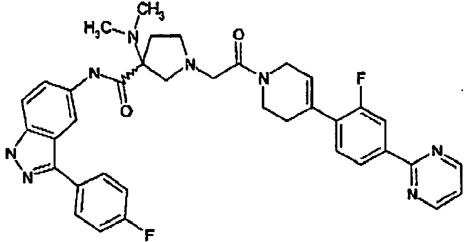 |
| 798 | 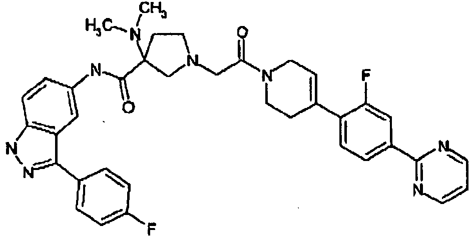 |
| 799 | 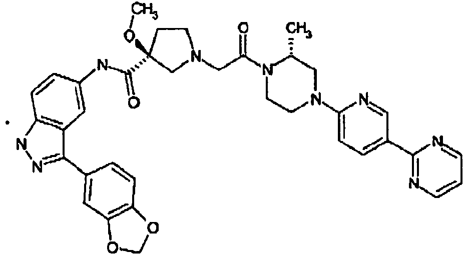 |
| 800 | 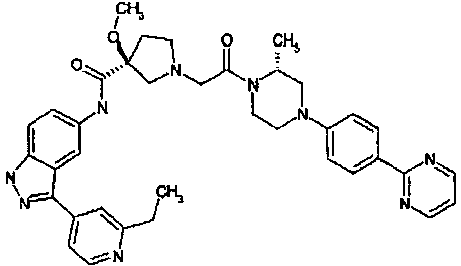 |
| 801 | 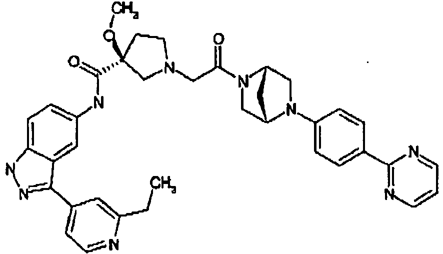 |
| 802 | 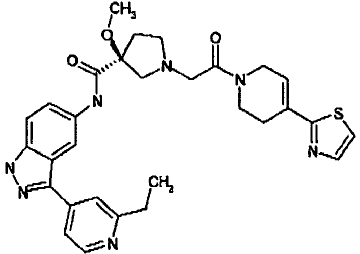 |
| 803 | 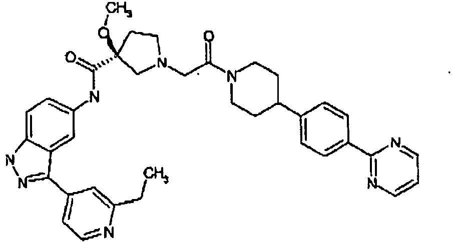 |
| 804 | 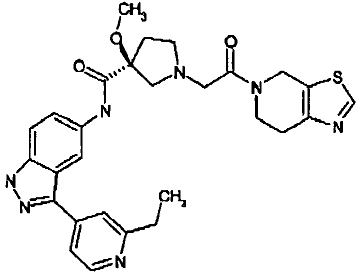 |
| 805 | 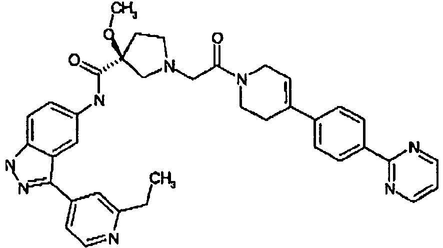 |
| 806 | 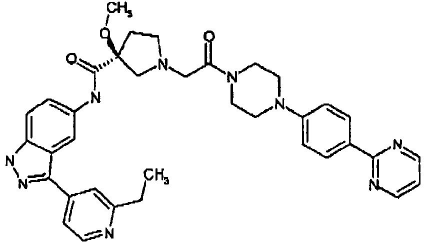 |
| 807 | 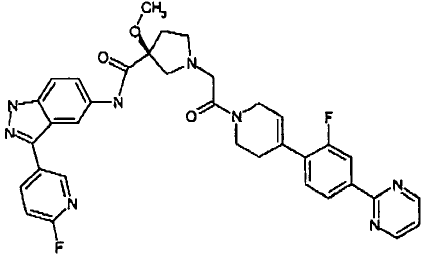 |
| 808 | 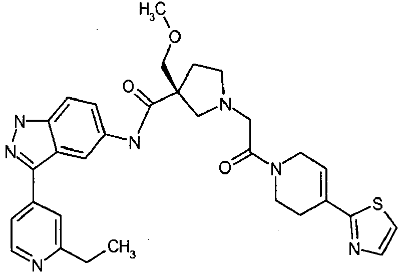 |
| 809 | 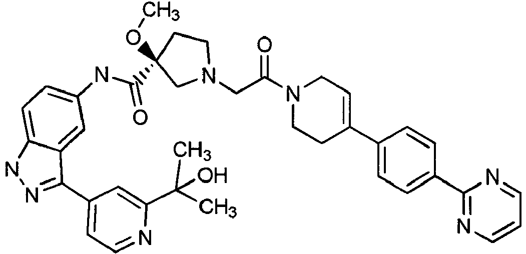 |
| 810 | 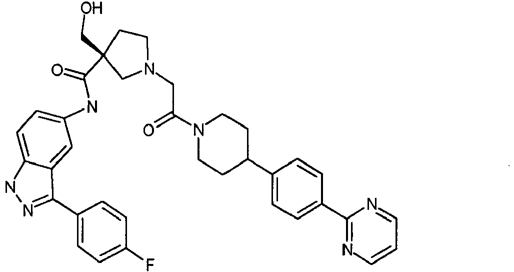 |
| 811 | 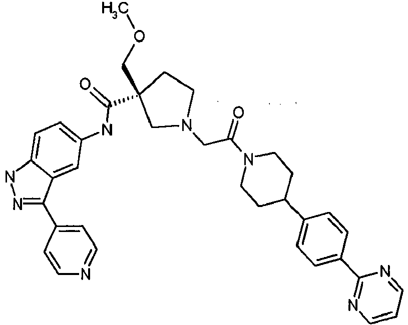 |
| 812 | 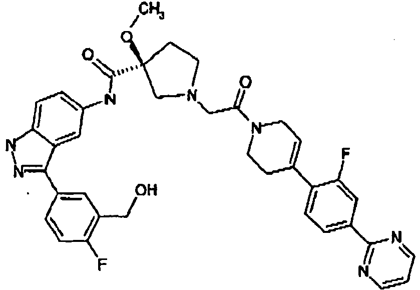 |
| 813 | 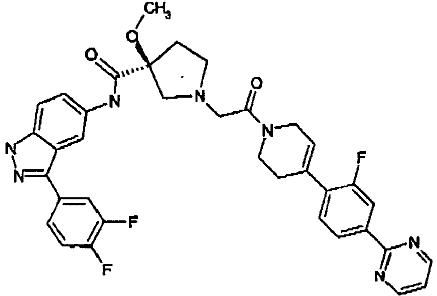 |
| 814 | 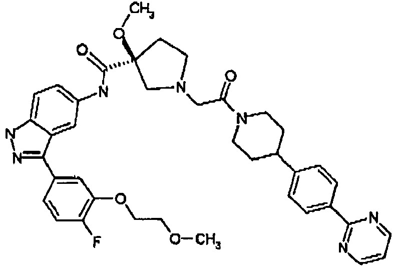 |
| 815 | 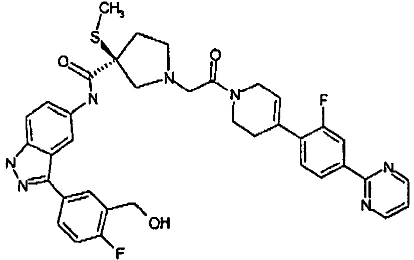 |
| 816 | 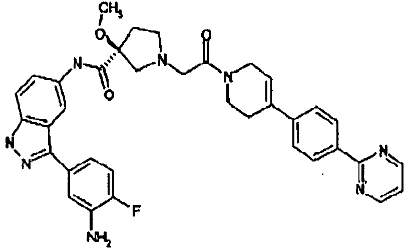 |
| 817 | 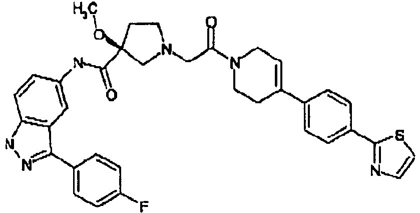 |
| 818 | 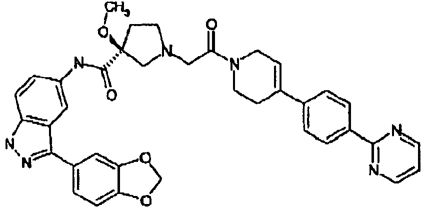 |
| 819 | 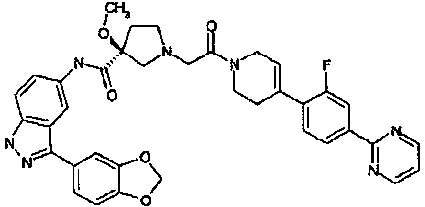 |
| 820 | 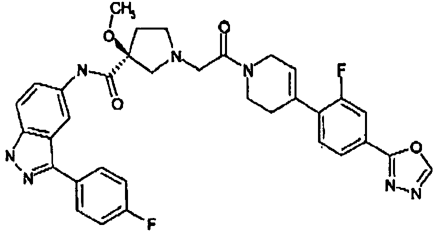 |
| 821 | 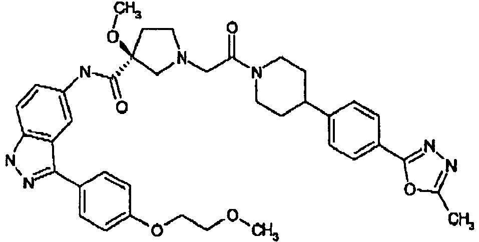 |
| 822 | 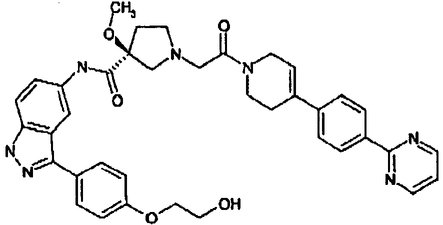 |
| 823 | 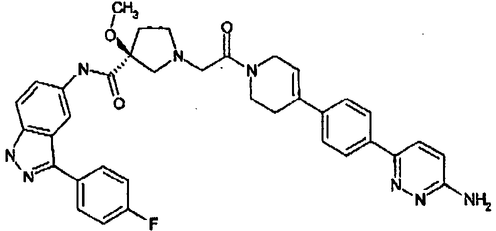 |
| 824 | 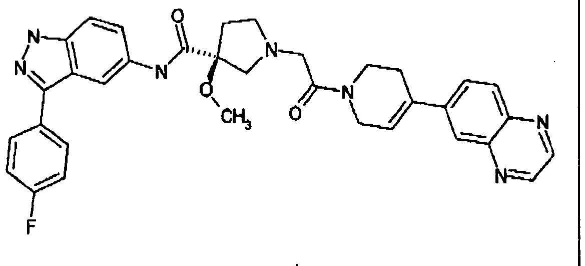 |
| 826 | 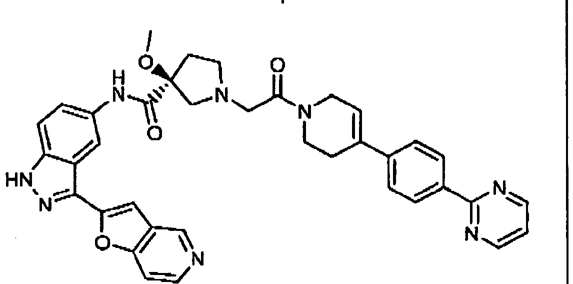 |
| 827 | 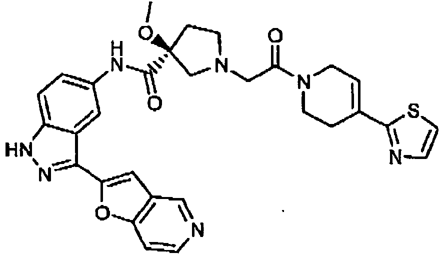 |
oder einem pharmazeutisch annehmbaren Salz davon.

2. Der ERK-Inhibitor nach Anspruch 1:
(a) der Formel: 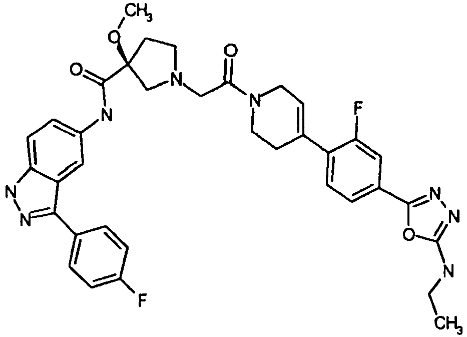 oder (b) der Formel: 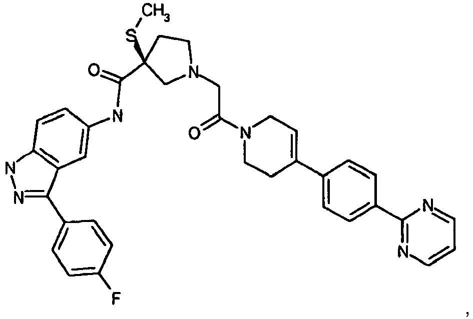 oder ein pharmazeutisch annehmbares Salz davon.

3. Eine pharmazeutische Zusammensetzung, die wenigstens eine Verbindung nach Anspruch 1 oder 2 oder ein pharmazeutisch annehmbares Salz davon und einen pharmazeutisch annehmbaren Träger umfasst.

4. Eine Verwendung von wenigstens einer Verbindung nach Anspruch 1 oder 2 oder eines pharmazeutisch annehmbaren Salzes davon:
(1) zur Herstellung eines Medikaments zur Behandlung von Krebs oder
(2) zur Herstellung eines Medikaments zur Behandlung von Krebs, wobei das Medikament in Kombination mit wenigstens einem chemotherapeutischen Mittel verwendet wird, oder
(3) zur Herstellung eines Medikaments zur Behandlung von Krebs, wobei das Medikament in Kombination mit wenigstens einem chemotherapeutischen Mittel und Strahlentherapie verwendet wird, oder
(4) zur Herstellung eines Medikaments zur Behandlung von Krebs, wobei das Medikament in Kombination mit wenigstens einem chemotherapeutischen Mittel, ausgewählt aus der Gruppe, bestehend aus: (1) Taxanen, (2) Platin-Koordinationsverbindungen, (3) Inhibitoren des epidermalen Wachstumsfaktors (EGF), die Antikörper sind, (4) EGF-Inhibitoren, die kleine Moleküle sind, (5) Inhibitoren des vaskulo-endothelialen Wachstumsfaktors (VEGF), die Antikörper sind, (6) VEGF-Kinase-Inhibitoren, die kleine Moleküle sind, (7) Östrogenrezeptorantagonisten oder selektive Östrogenrezeptormodulatoren (SERMs), (8) Antitumor-Nukleosid-Derivate, (9) Epothilone, (10) Topoisomerase-Inhibitoren (11) Vincaalkaloide, (12) Antikörper, die Inhibitoren der αVβ3-Integrine sind, (13) Folat-Antagonisten, (14) Ribonukleotidreduktase-Inhibitoren, (15) Anthracycline, (16) biologische Präparate, (17) Inhibitoren der Angiogenese und/oder Suppressoren von Tumornekrosefaktor alpha (TNF-alpha), wie z.B. Thalidomid (oder verwandtes Imid), (18) Bcr/abl-Kinase-Inhibitoren, (19) MEK1- und/oder MEK2-Inhibitoren, die kleine Moleküle sind, (20) IGF-1- und IGF-2-Inhibitoren, die kleine Moleküle sind, (21) RAF- und BRAF-Kinasen-Inhibitoren, die kleine Moleküle sind, (22) Inhibitoren von zellzyklusabhängigen Kinasen, wie z.B. CDK1, CDK2, CDK4 und CDK6, die kleine Moleküle sind, (23) Alkylierungsmittel und (24) Farnesylproteintransferase-Inhibitoren, oder
(5) zur Herstellung eines Medikaments zur Behandlung von Krebs, wobei das Medikament mit wenigstens einem Signaltransduktionsinhibitor verabreicht wird, oder
(6) zur Herstellung eines Medikaments zur Behandlung von Krebs, wobei der Krebs ausgewählt ist aus der Gruppe, bestehend aus: Lungenkrebs, Bauchspeicheldrüsenkrebs, Kolonkrebs, myeloische Leukämien, Schilddrüsenkrebs, myelodysplastisches Syndrom, Blasenkrebs, Hautkrebs, Melanom, Brustkrebs, Prostatakrebs, Kopf- und Halskarzinomen, Ovarialkarzinom, Hirntumore, Karzinome mesenchymalen Ursprungs, Sarkome, Tetrakarzinome, Nuroblastome, Nierenkarzinome, Hepatome, Nicht-Hodgkins-Lmyphom, multiples Myelom und anaplastisches Schilddrüsenkarzinom, oder
(7) zur Herstellung eines Medikaments zur Behandlung von Krebs, wobei der Krebs ausgewählt ist aus der Gruppe, bestehend aus: Melanom, Bauchspeicheldrüsenkrebs, Schilddrüsenkrebs, Kolorektalkarzinom, Lungenkrebs, Brustkrebs und Ovarialkarzinom, oder
(8) zur Herstellung eines Medikaments zur Behandlung von Krebs, wobei das Medikament in Kombination mit einer wirksamen Menge von wenigstens einem chemotherapeutischen Mittel verwendet wird, wobei der Krebs ausgewählt ist aus der Gruppe, bestehend aus: Melanom, Bauchspeicheldrüsenkrebs, Schilddrüsenkrebs, Kolorektalkarzinom, Lungenkrebs, Brustkrebs und Ovarialkarzinom, oder
(9) zur Herstellung eines Medikaments zur Behandlung eines Melanoms, oder
(10) zur Herstellung eines Medikaments zur Behandlung eines Melanoms, wobei das Medikament in Kombination mit wenigstens einem chemotherapeutischen Mittel verwendet wird, oder
(11) zur Herstellung eines Medikaments zur Behandlung von Bauchspeicheldrüsenkrebs, oder
(12) zur Herstellung eines Medikaments zur Behandlung von Bauchspeicheldrüsenkrebs, wobei das Medikament in Kombination mit wenigstens einem chemotherapeutischen Mittel verwendet wird, oder
(13) zur Herstellung eines Medikaments zur Behandlung von Schilddrüsenkrebs, oder
(14) zur Herstellung eines Medikaments zur Behandlung von Schilddrüsenkrebs, wobei das Medikament in Kombination mit wenigstens einem chemotherapeutischen Mittel verwendet wird, oder
(15) zur Herstellung eines Medikaments zur Behandlung von Kolorektalkarzinom, oder
(16) zur Herstellung eines Medikaments zur Behandlung von Kolorektalkarzinom, wobei das Medikament in Kombination mit wenigstens einem chemotherapeutischen Mittel verwendet wird, oder
(17) zur Herstellung eines Medikaments zur Behandlung von Lungenkrebs, oder
(18) zur Herstellung eines Medikaments zur Behandlung von Lungenkrebs, wobei das Medikament in Kombination mit wenigstens einem chemotherapeutischen Mittel verwendet wird, oder
(19) zur Herstellung eines Medikaments zur Behandlung von Brustkrebs, oder
(20) zur Herstellung eines Medikaments zur Behandlung von Brustkrebs, wobei das Medikament in Kombination mit wenigstens einem chemotherapeutischen Mittel verwendet wird, oder
(21) zur Herstellung eines Medikaments zur Behandlung von Ovarialkarzinom, oder
(22) zur Herstellung eines Medikaments zur Behandlung von Ovarialkarzinom, wobei das Medikament in Kombination mit wenigstens einem chemotherapeutischen Mittel verwendet wird, oder
(23) zur Herstellung eines Medikaments zur Behandlung von hormonabhängigem Brustkrebs, wobei das Medikament in Kombination mit antihormonellen Mitteln verwendet wird, oder
(24) zur Herstellung eines Medikaments zur Behandlung von hormonabhängigem Brustkrebs, wobei das Medikament in Kombination mit antihormonellen Mitteln und wenigstens einem chemotherapeutischen Mittel verwendet wird, oder
(25) zur Herstellung eines Medikaments zur Prävention von hormonabhängigem Brustkrebs, wobei das Medikament in Kombination mit antihormonellen Mitteln verwendet wird, oder
(26) zur Herstellung eines Medikaments zur Prävention von hormonabhängigem Brustkrebs, wobei das Medikament in Kombination mit antihormonellen Mitteln und wenigstens einem chemotherapeutischen Mittel verwendet wird, oder
(27) zur Herstellung eines Medikaments zur Behandlung von Gehirntumor, oder
(28) zur Herstellung eines Medikaments zur Behandlung von Gehirntumor, wobei das Medikament in Kombination mit wenigstens einem chemotherapeutischen Mittel verwendet wird, oder
(29) zur Herstellung eines Medikaments zur Behandlung von Gehirntumor, wobei das Medikament in Kombination mit wenigstens einem chemotherapeutischen Mittel verwendet wird, wobei das chemotherapeutische Mittel Temozolomid ist, oder
(30) zur Herstellung eines Medikaments zur Behandlung von Prostatakrebs, oder
(31) zur Herstellung eines Medikaments zur Behandlung von Prostatakrebs, wobei das Medikament in Kombination mit wenigstens einem chemotherapeutischen Mittel verwendet wird, oder
(32) zur Herstellung eines Medikaments zur Behandlung von myelodysplastischem Syndrom, oder
(33) zur Herstellung eines Medikaments zur Behandlung von myelodysplastischem Syndrom, wobei das Medikament in Kombination mit wenigstens einem chemotherapeutischen Mittel verwendet wird, oder
(34) zur Herstellung eines Medikaments zur Behandlung von myeloischen Leukämien, oder
(35) zur Herstellung eines Medikaments zur Behandlung von myeloischen Leukämien, wobei das Medikament in Kombination mit wenigstens einem chemotherapeutischen Mittel verwendet wird, oder
(36) zur Herstellung eines Medikaments zur Behandlung von akuter myeloischer Leukämie, oder
(37) zur Herstellung eines Medikaments zur Behandlung von akuter myeloischer Leukämie, wobei das Medikament in Kombination mit wenigstens einem chemotherapeutischen Mittel verwendet wird, oder
(38) zur Herstellung eines Medikaments zur Behandlung von chronischer myelomonozytischer Leukämie, oder
(39) zur Herstellung eines Medikaments zur Behandlung von chronischer myelomonozytischer Leukämie, wobei das Medikament in Kombination mit wenigstens einem chemotherapeutischen Mittel verwendet wird, oder
(40) zur Herstellung eines Medikaments zur Behandlung von chronischer myeloischer Leukämie, oder
(41) zur Herstellung eines Medikaments zur Behandlung von chronischer myeloischer Leukämie, wobei das Medikament in Kombination mit wenigstens einem chemotherapeutischen Mittel verwendet wird, oder
(42) zur Herstellung eines Medikaments zur Behandlung von Blasenkrebs, oder
(43) zur Herstellung eines Medikaments zur Behandlung von Blasenkrebs, wobei das Medikament in Kombination mit wenigstens einem chemotherapeutischen Mittel verwendet wird, oder
(44) zur Herstellung eines Medikaments zur Behandlung von Nicht-Hodgkins-Lymphom, oder
(45) zur Herstellung eines Medikaments zur Behandlung von Nicht-Hodgkins-Lymphom, wobei das Medikament in Kombination mit wenigstens einem chemotherapeutischen Mittel verwendet wird, oder
(46) zur Herstellung eines Medikaments zur Behandlung von multiplem Myelom oder
(47) zur Herstellung eines Medikaments zur Behandlung von multiplem Myelom, wobei das Medikament in Kombination mit wenigstens einem chemotherapeutischen Mittel verwendet wird.

5. Eine Verbindung nach Anspruch 1 oder 2 oder ein pharmazeutisch annehmbares Salz davon zur Verwendung bei einem Verfahren zur Behandlung des menschlichen Körpers durch Therapie.

6. Eine Verbindung nach Anspruch 5, wobei die Therapie wie in Anspruch 4 definiert ist.

7. Eine Kombination aus einer Verbindung nach Anspruch 1 oder 2 oder einem pharmazeutisch annehmbaren Salz davon und wenigstens einem chemotherapeutischen Mittel, ausgewählt aus:
(1) Taxanen,
(2) Platin-Koordinationsverbindungen,
(3) Inhibitoren des epidermalen Wachstumsfaktors (EGF), die Antikörper sind,
(4) EGF-Inhibitoren, die kleine Moleküle sind,
(5) Inhibitoren des vaskulo-endothelialen Wachstumsfaktors (VEGF), die Antikörper sind,
(6) VEGF-Kinase-Inhibitoren, die kleine Moleküle sind,
(7) Östrogenrezeptorantagonisten oder selektive Östrogenrezeptormodulatoren (SERMs),
(8) Antitumor-Nukleosid-Derivate,
(9) Epothilone,
(10) Topoisomerase-Inhibitoren
(11) Vincaalkaloide,
(12) Antikörper, die Inhibitoren der αVβ3-Integrine sind,
(13) Folat-Antagonisten,
(14) Ribonukleotidreduktase-Inhibitoren,
(15) Anthracycline,
(16) biologische Präparate,
(17) Inhibitoren der Angiogenese und/oder Suppressoren von Tumornekrosefaktor alpha (TNF-alpha), wie z.B. Thalidomid (oder verwandtes Imid),
(18) Bcr/abl-Kinase-Inhibitoren,
(19) MEK1- und/oder MEK2-Inhibitoren, die kleine Moleküle sind,
(20) IGF-1- und IGF-2-Inhibitoren, die kleine Moleküle sind,
(21) RAF- und BRAF-Kinasen-Inhibitoren, die kleine Moleküle sind,
(22) Inhibitoren von zellzyklusabhängigen Kinasen, wie z.B. CDK1, CDK2, CDK4 und CDK6, die kleine Moleküle sind,
(23) Alkylierungsmittel und
(24) Farnesylproteintransferase-Inhibitoren (auch bekannt als FPT-Inhibitoren oder FTI (d.h. Farnesyltransferinhibitoren)).

## Revendications

1. Inhibiteur ERK choisi dans le groupe constitué des composés présentant les structures suivantes:
| N° exemple | COMPOSÉ |
|---|---|
| 612 | 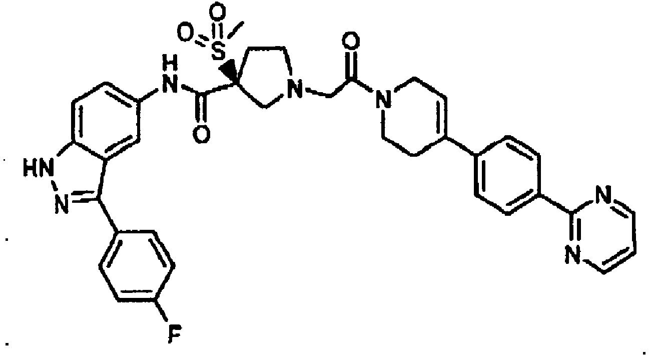 |
| 613 | 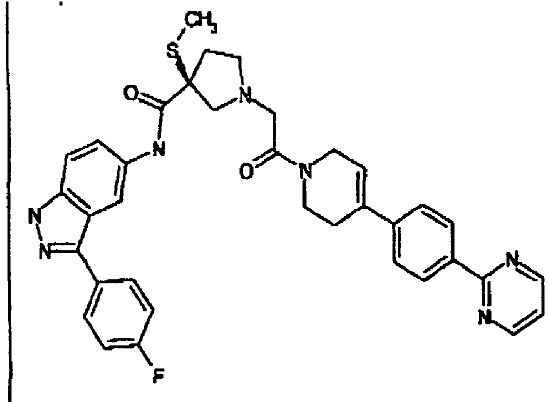 |
| 614 | 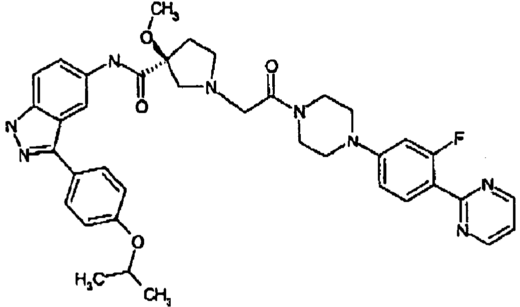 |
| 615 | 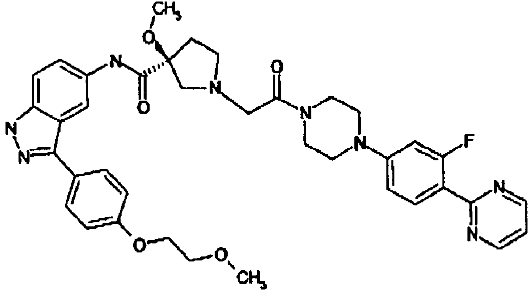 |
| 616 | 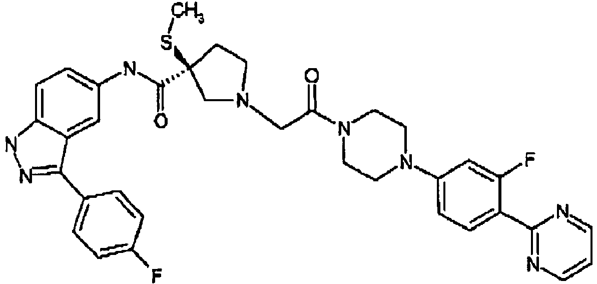 |
| 617 | 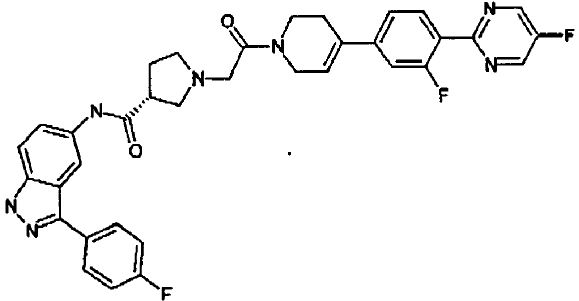 |
| 619 | 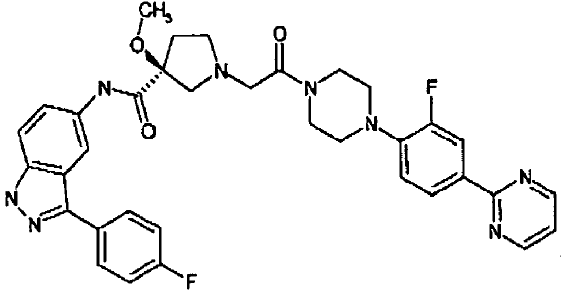 |
| 620 | 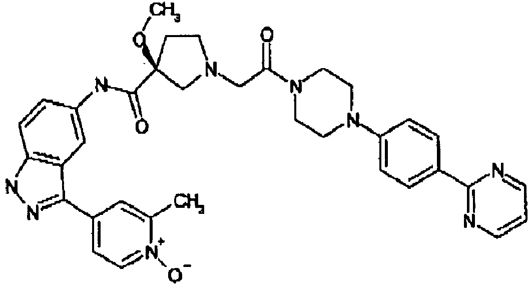 |
| 621 | 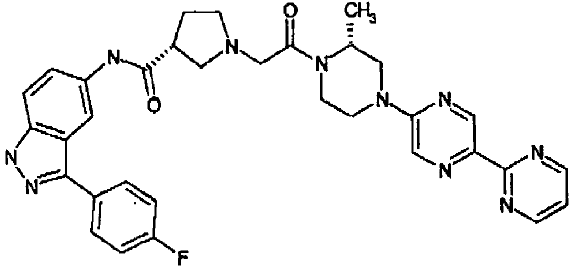 |
| 622 | 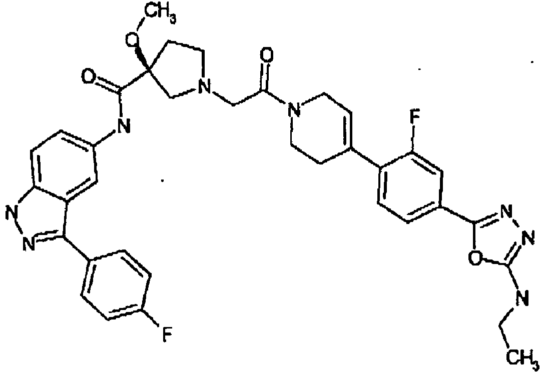 |
| 623 | 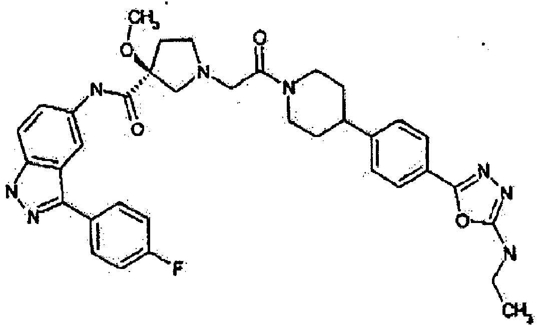 |
| 624 | 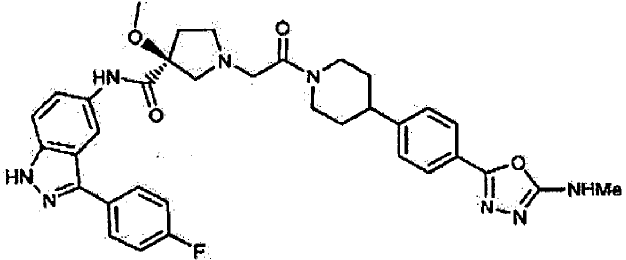 |
| 625 | 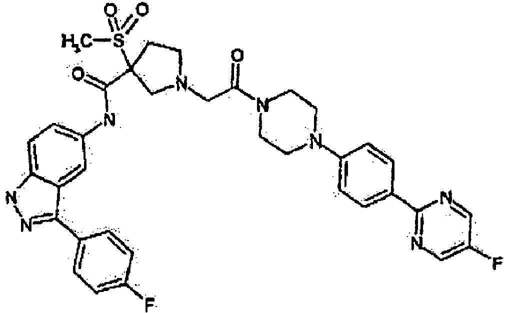 |
| 626 | 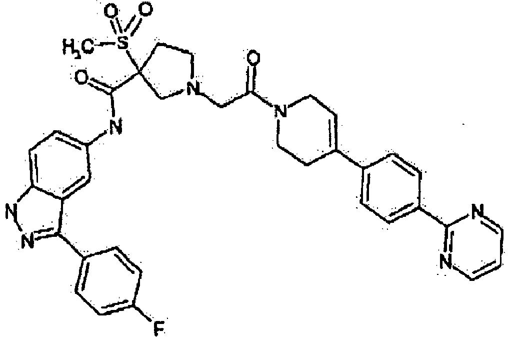 |
| 627 | 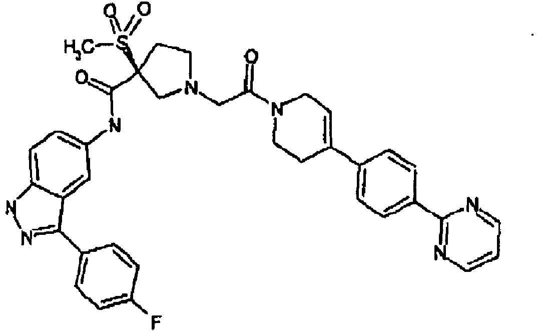 |
| 628 | 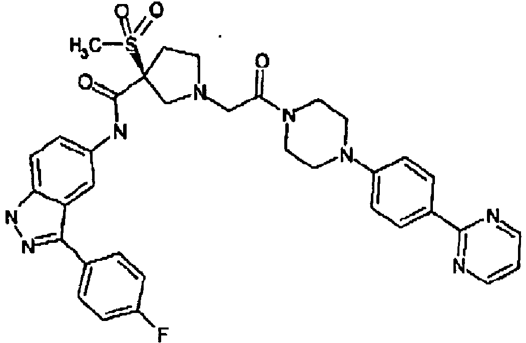 |
| 629 | 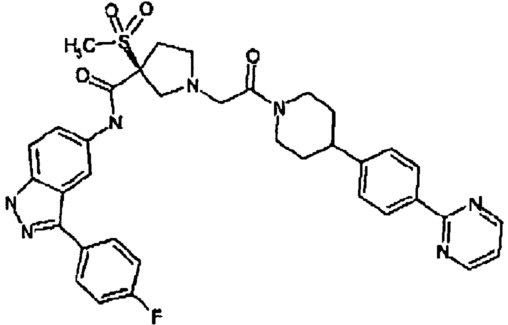 |
| 630 | 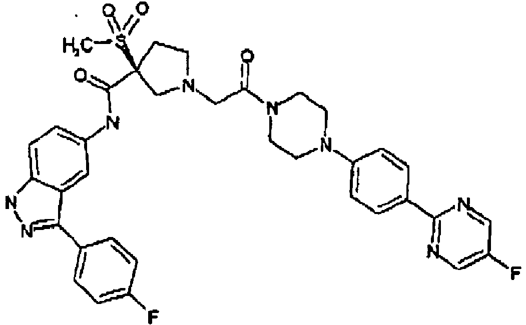 |
| 631 | 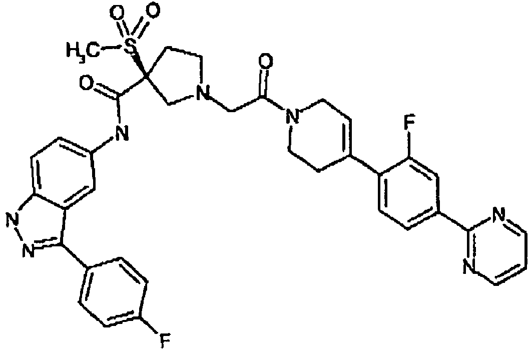 |
| 632 | 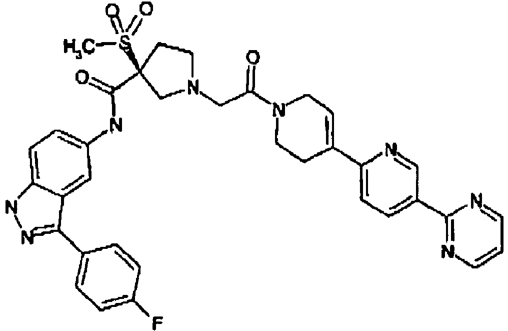 |
| 633 | 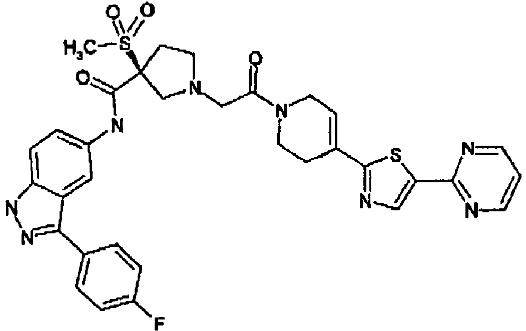 |
| 634 | 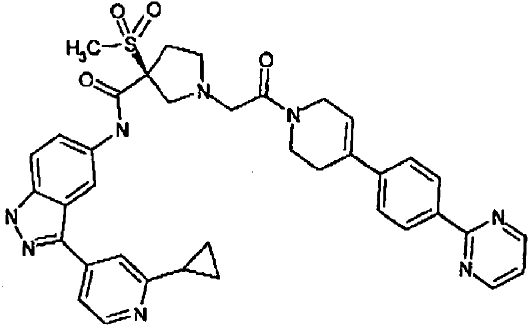 |
| 635 | 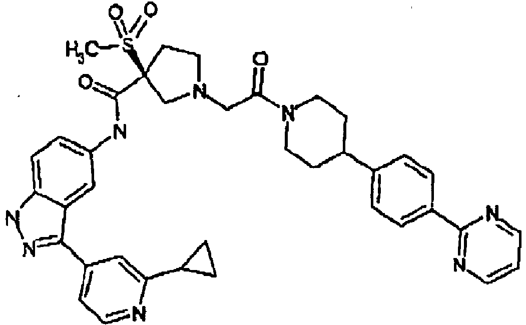 |
| 636 | 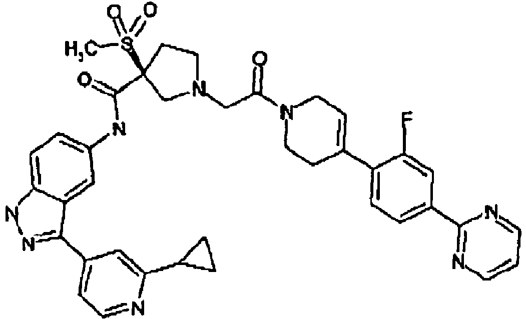 |
| 637 | 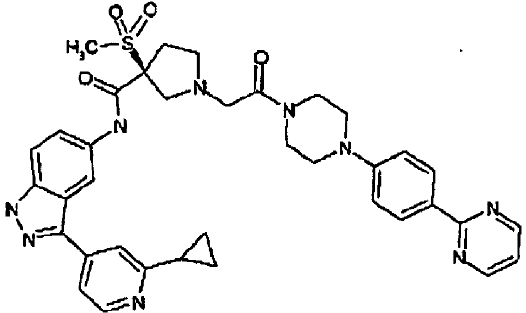 |
| 638 | 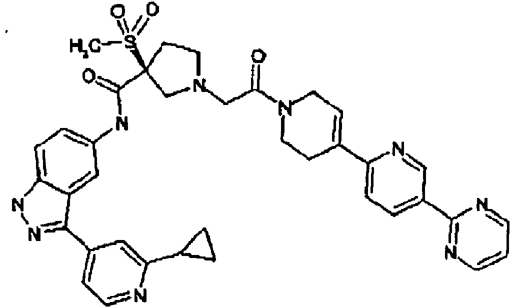 |
| 639 | 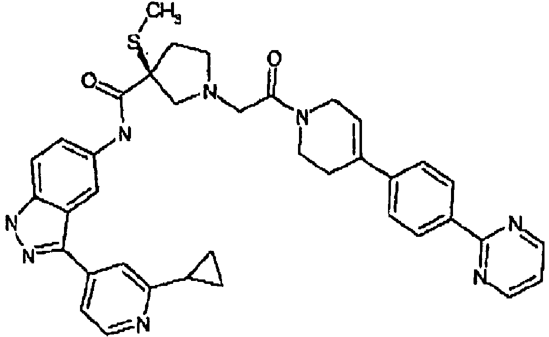 |
| 640 | 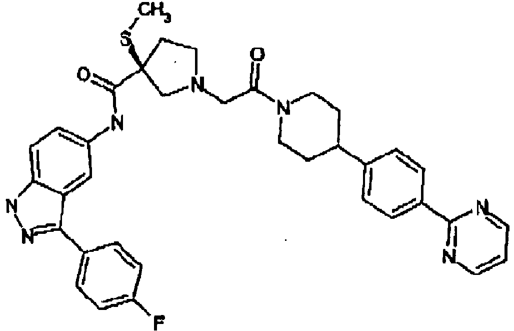 |
| 641 | 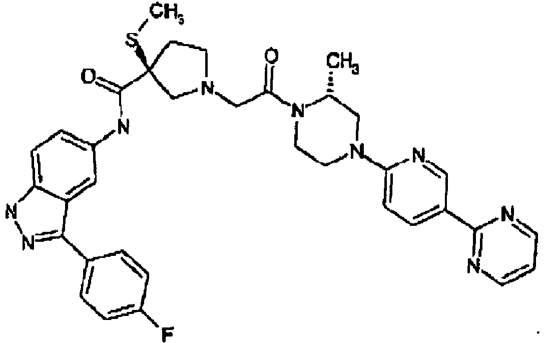 |
| 642 | 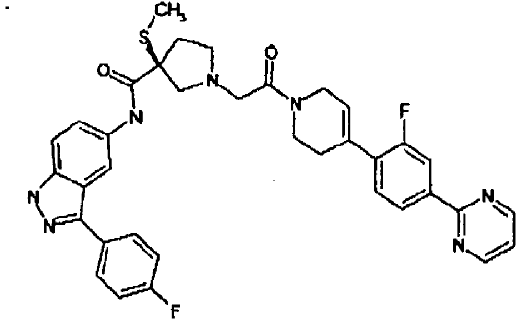 |
| 643 | 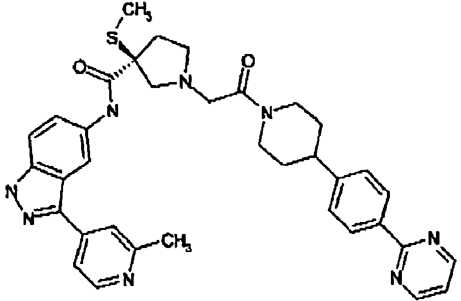 |
| 644 | 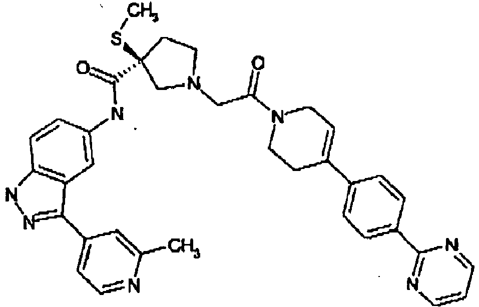 |
| 645 | 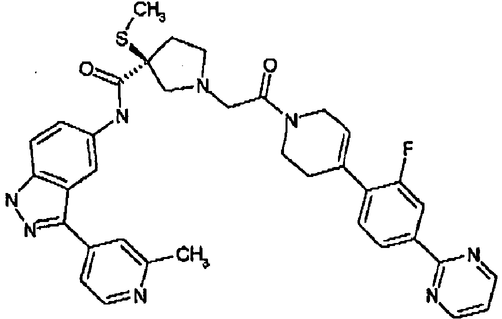 |
| 646 | 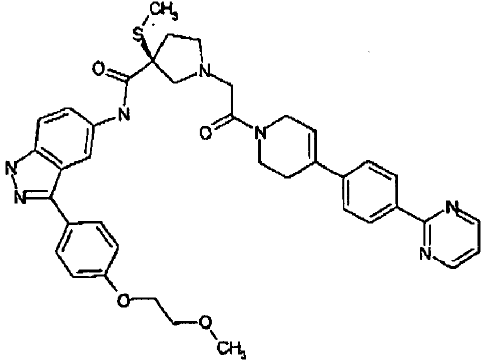 |
| 647 | 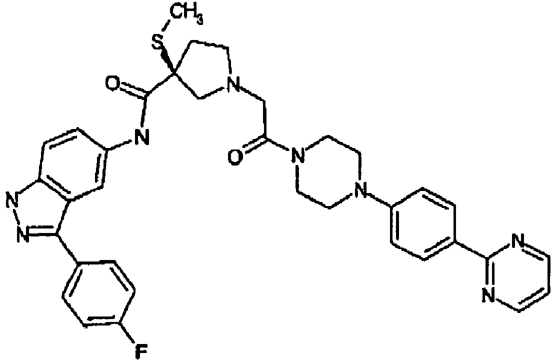 |
| 648 | 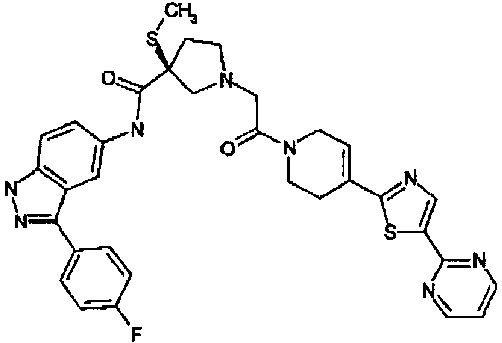 |
| 649 | 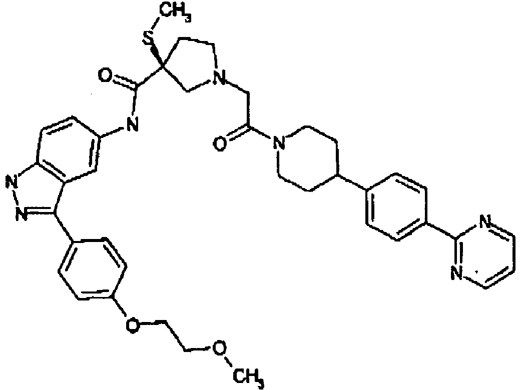 |
| 650 | 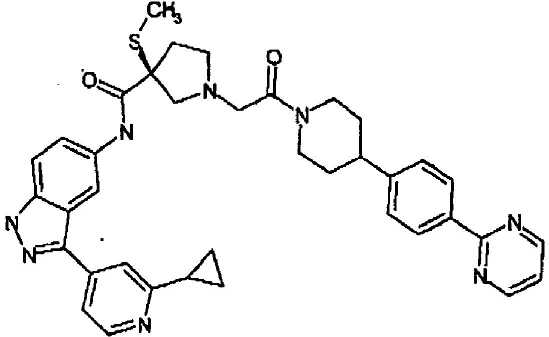 |
| 651 | 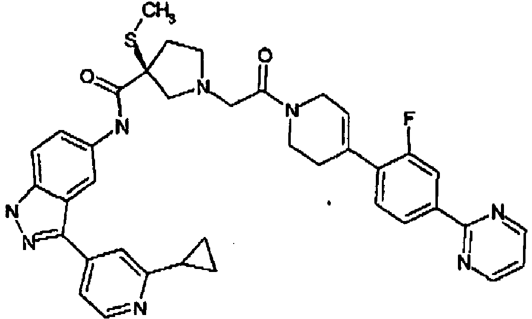 |
| 652 | 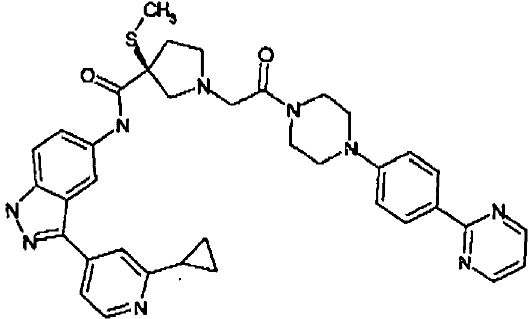 |
| 653 | 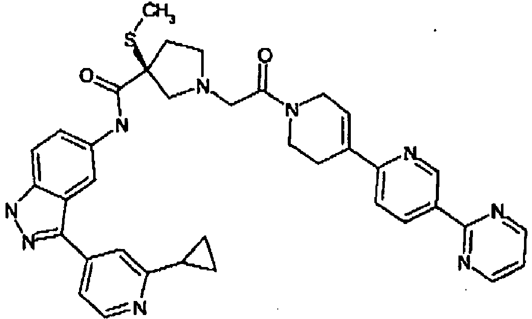 |
| 654 | 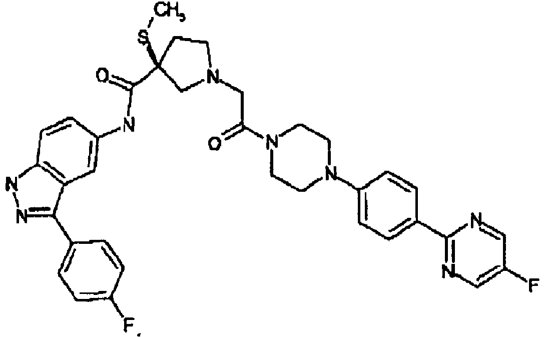 |
| 655 | 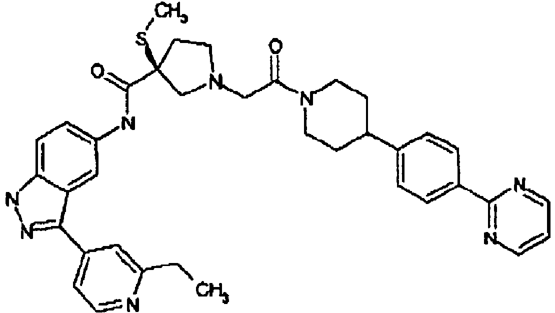 |
| 656 | 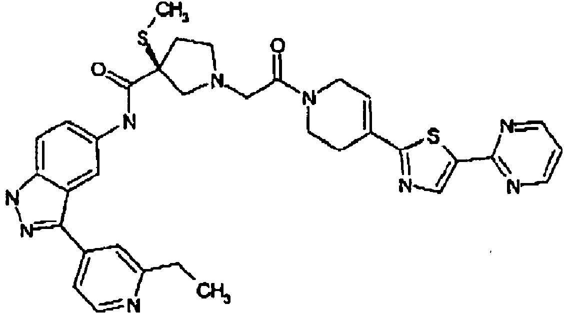 |
| 657 | 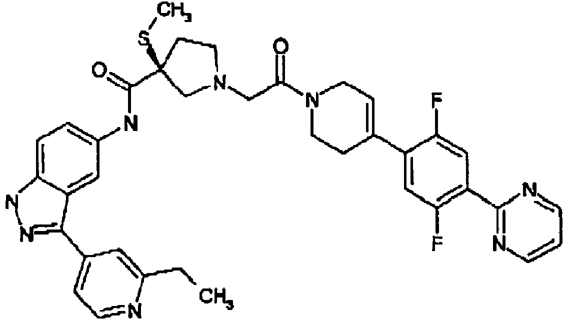 |
| 658 | 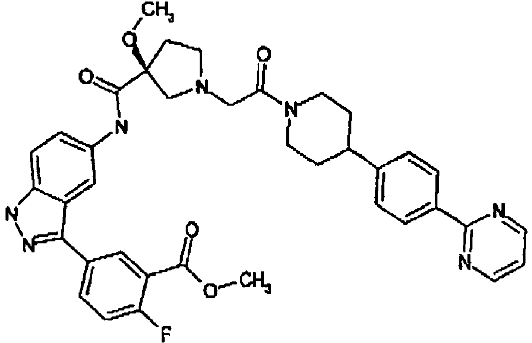 |
| 659 | 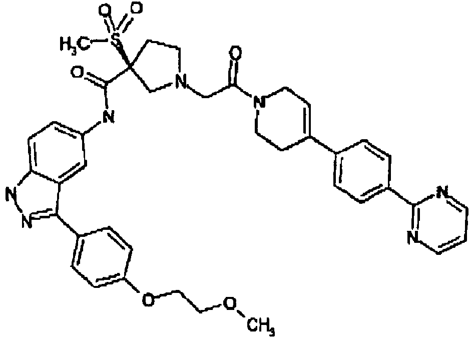 |
| 660 | 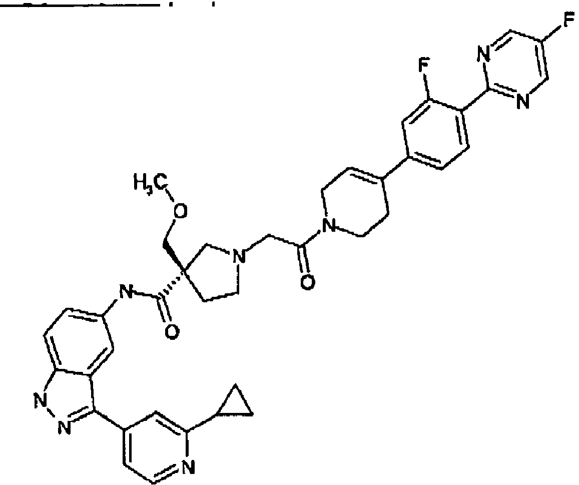 |
| 661 | 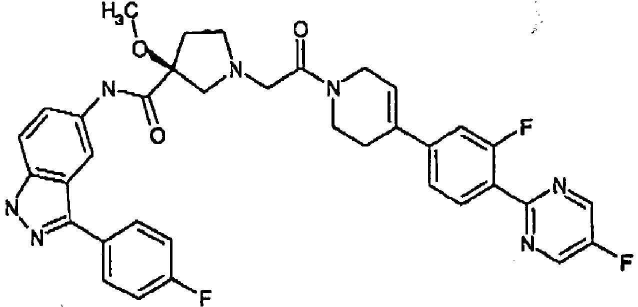 |
| 662 | 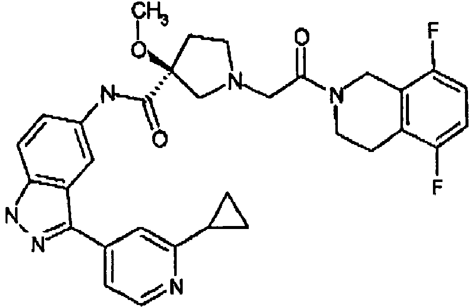 |
| 663 | 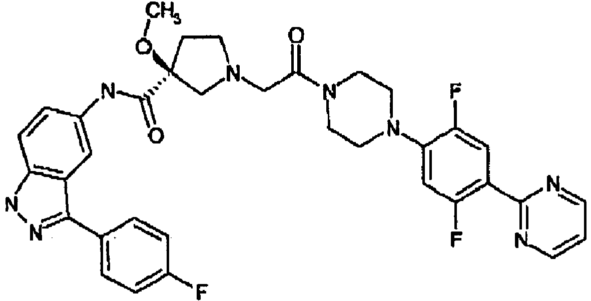 |
| 664 | 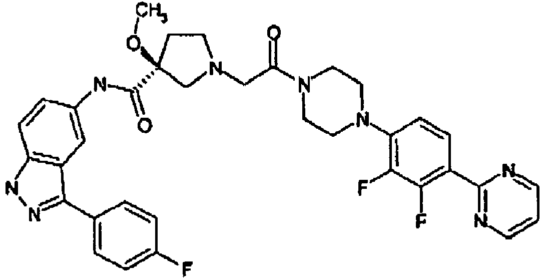 |
| 665 | 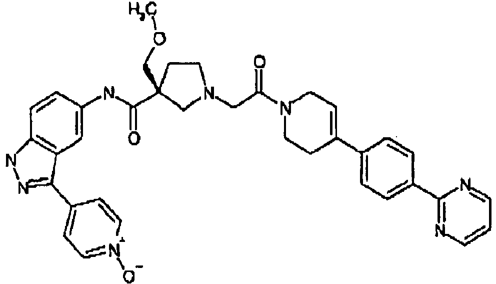 |
| 666 | 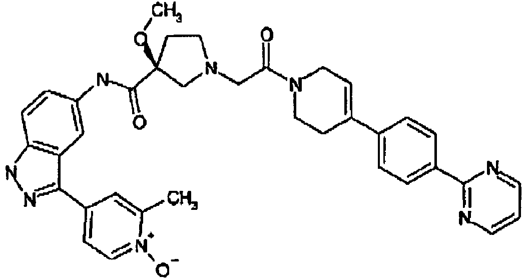 |
| 667 | 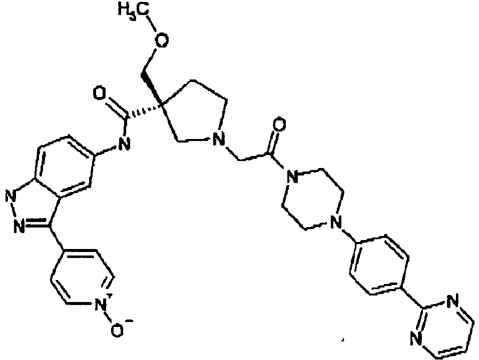 |
| 668 | 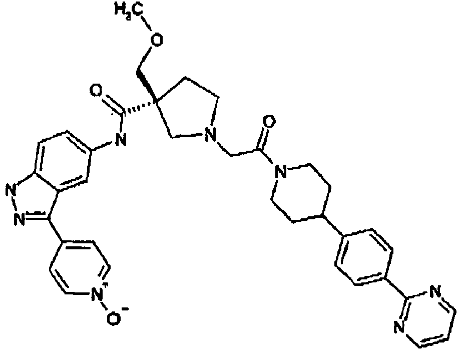 |
| 669 | 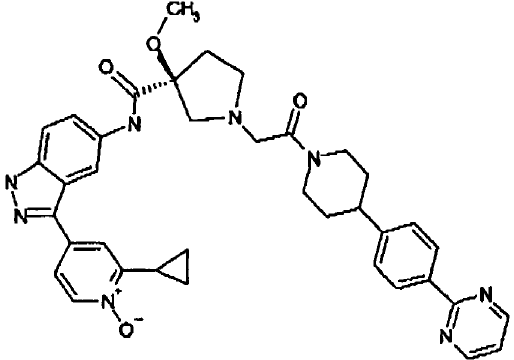 |
| 670 | 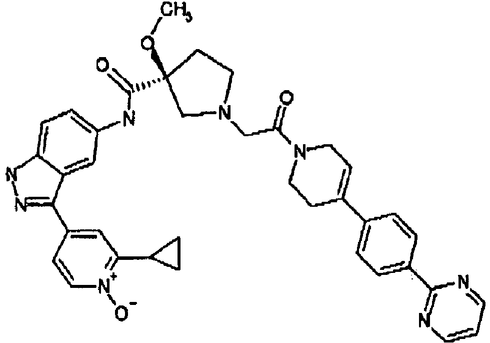 |
| 671 | 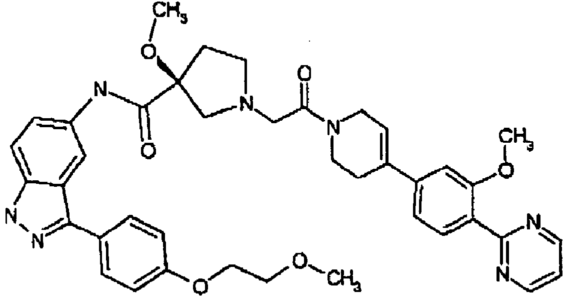 |
| 672 | 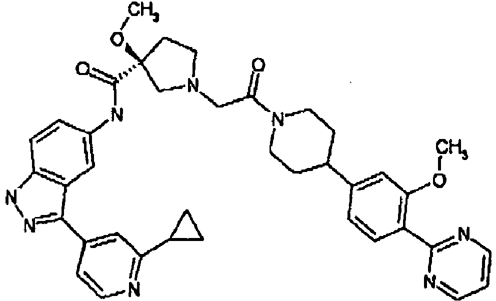 |
| 673 | 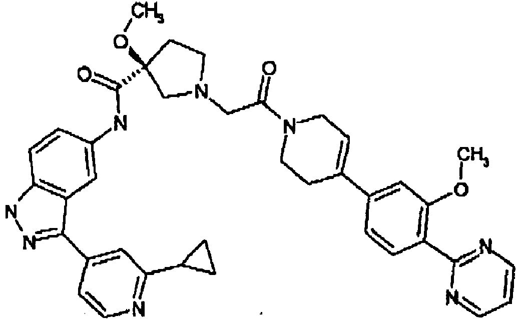 |
| 674 | 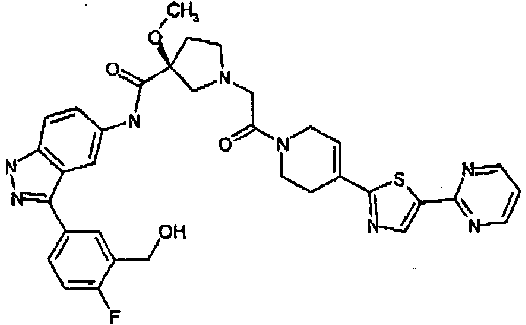 |
| 675 | 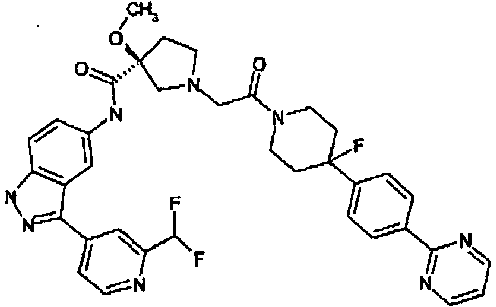 |
| 676 | 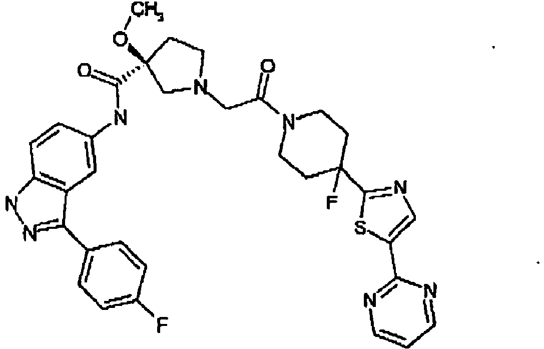 |
| 677 | 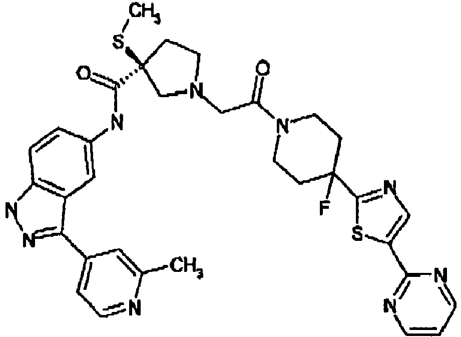 |
| 678 | 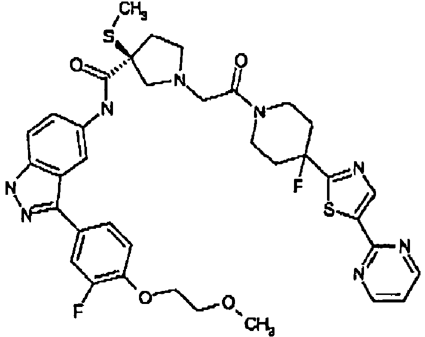 |
| 679 | 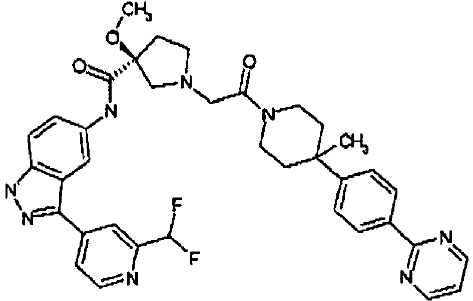 |
| 680 | 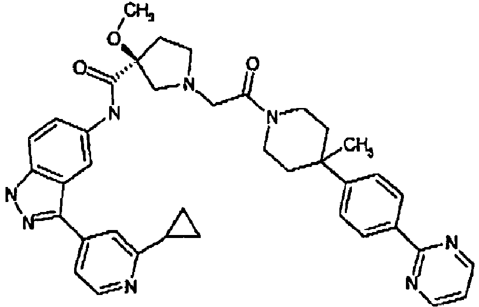 |
| 681 | 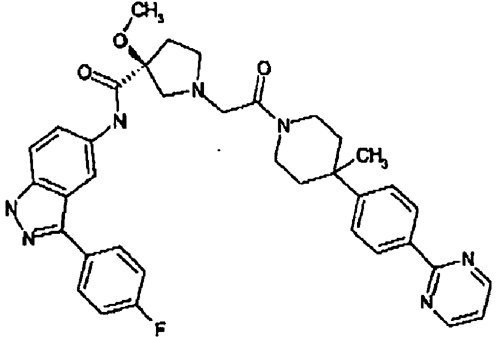 |
| 682 | 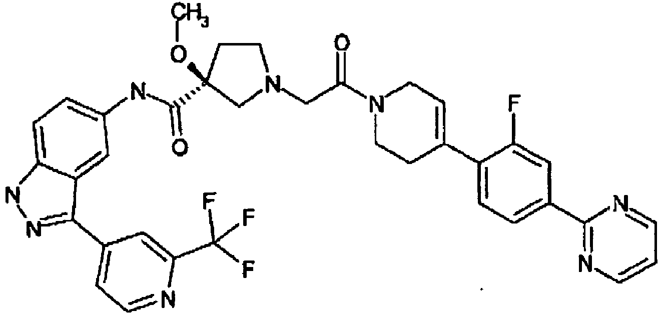 |
| 683 | 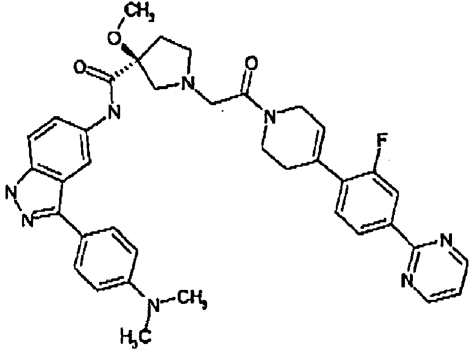 |
| 684 | 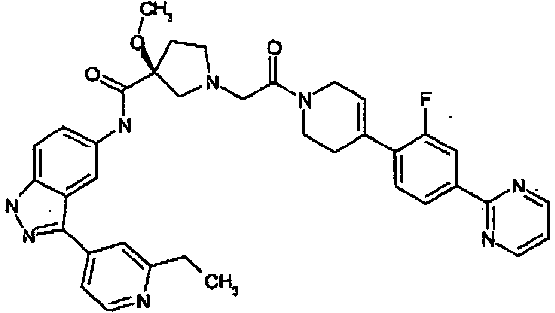 |
| 685 | 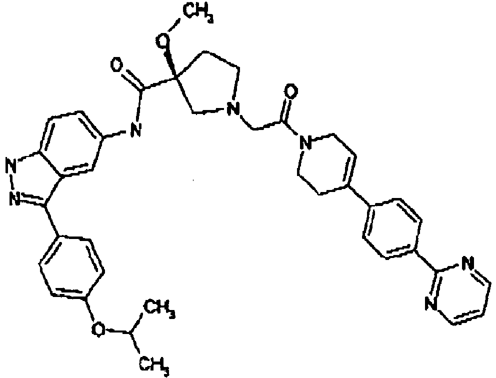 |
| 686 | 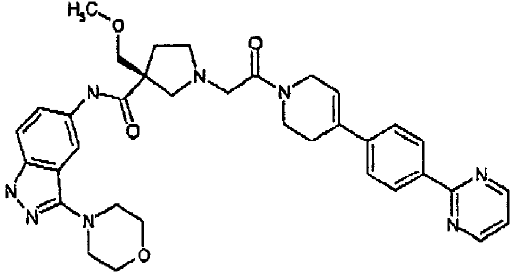 |
| 687 | 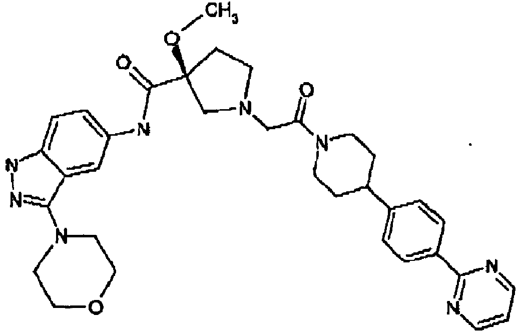 |
| 688 | 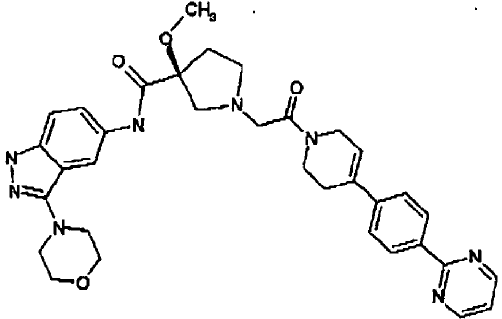 |
| 689 | 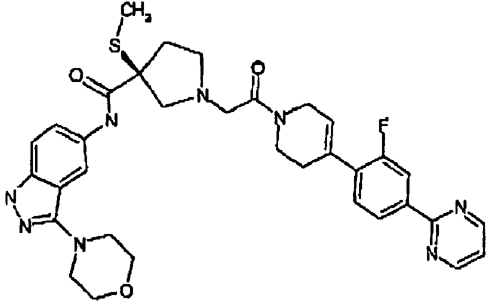 |
| 690 | 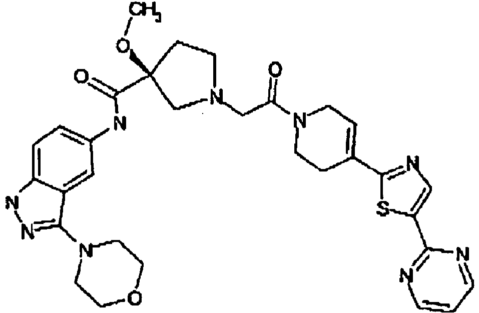 |
| 691 | 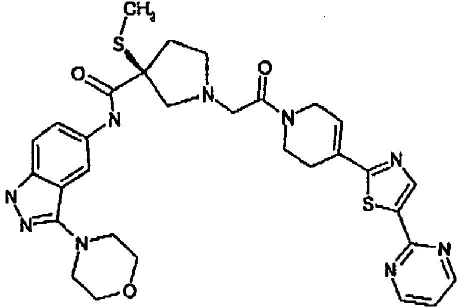 |
| 692 | 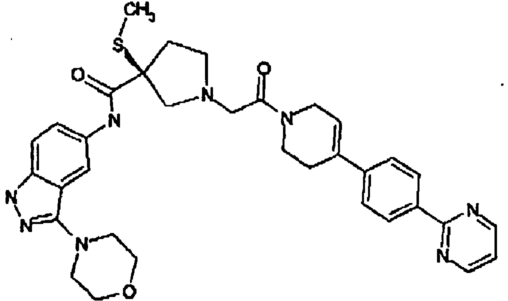 |
| 693 | 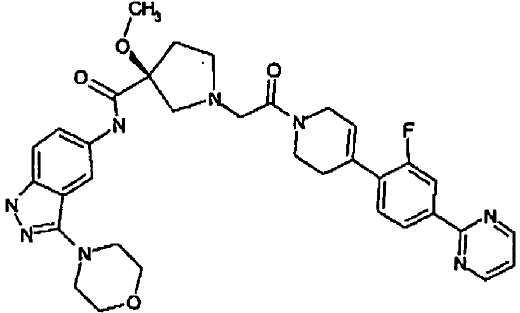 |
| 694 | 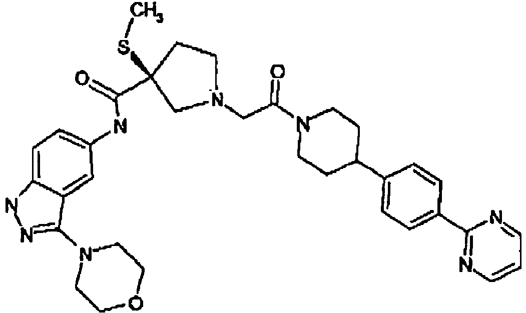 |
| 695 | 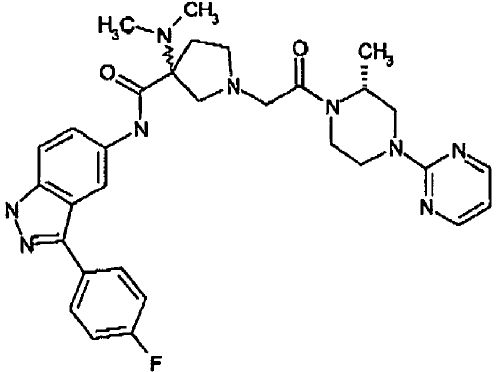 |
| 696 | 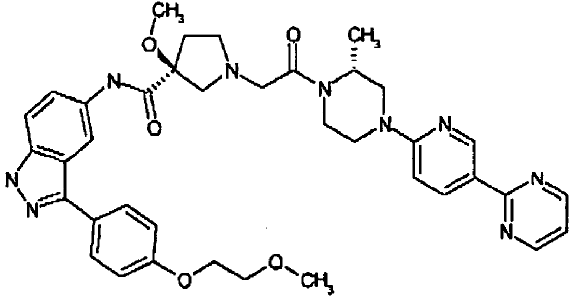 |
| 697 | 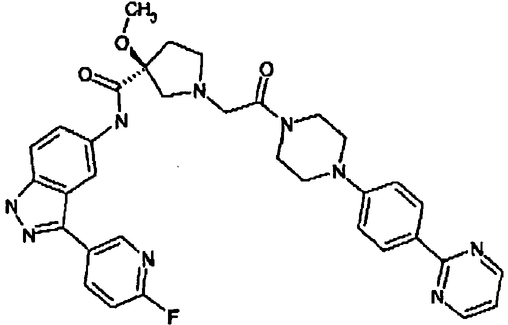 |
| 698 | 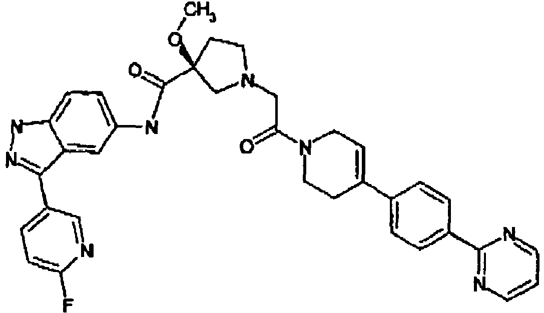 |
| 699 | 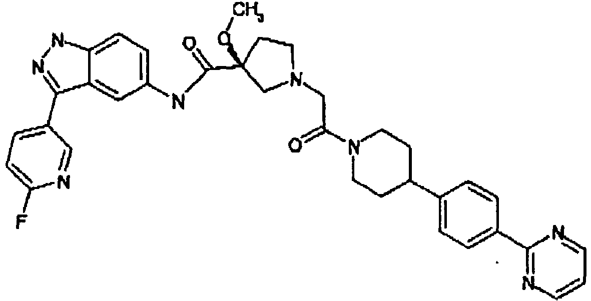 |
| 700 | 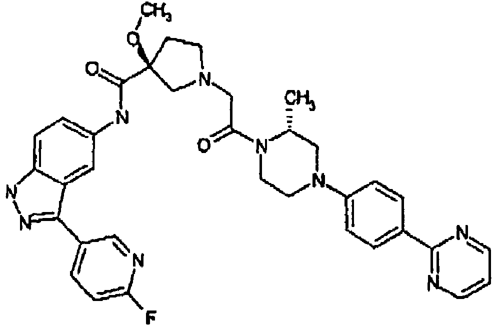 |
| 701 | 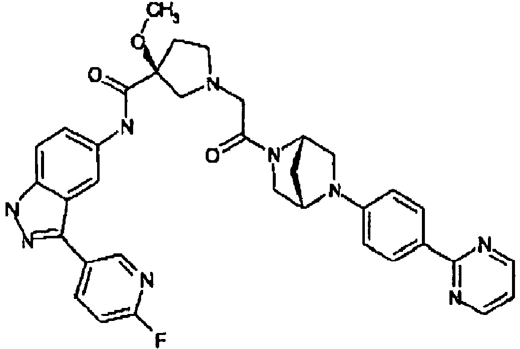 |
| 702 | 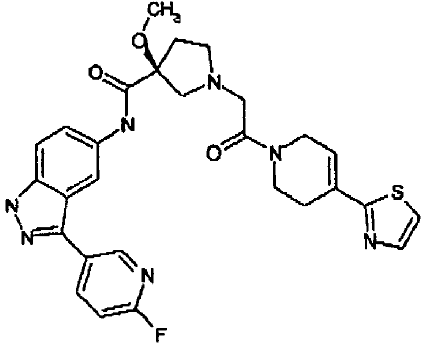 |
| 703 | 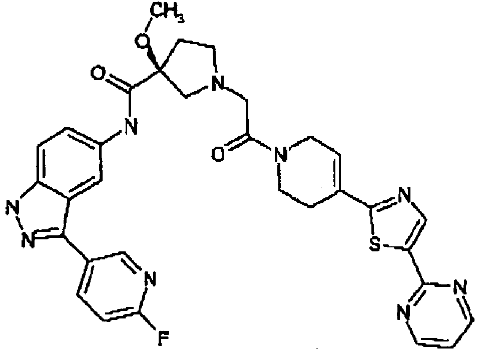 |
| 704 | 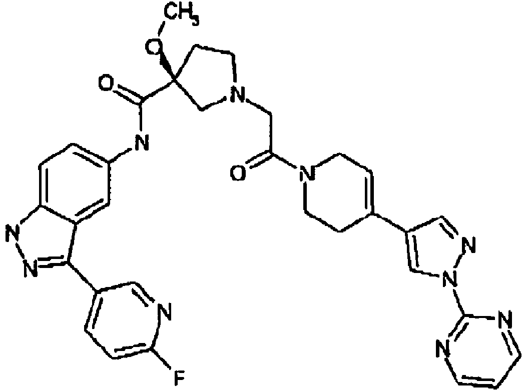 |
| 705 | 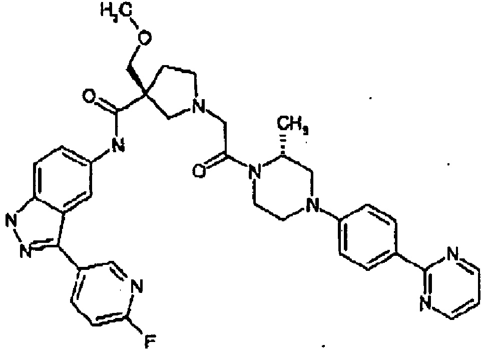 |
| 706 | 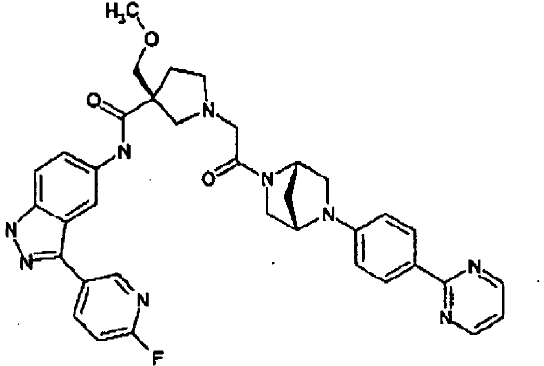 |
| 707 | 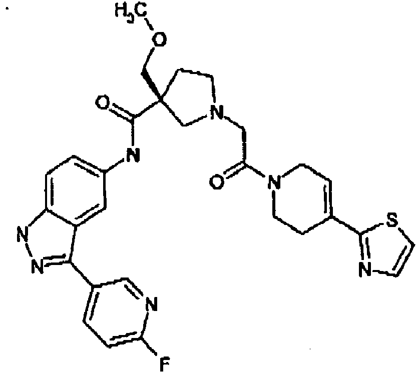 |
| 708 | 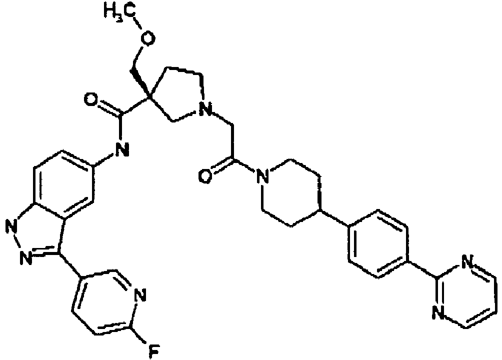 |
| 709 | 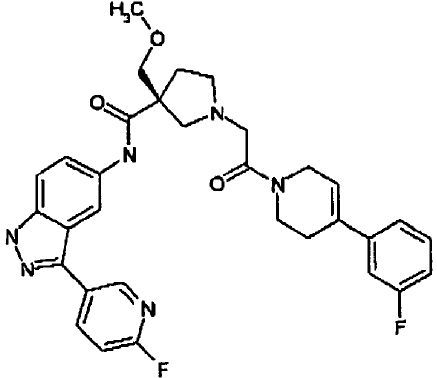 |
| 710 | 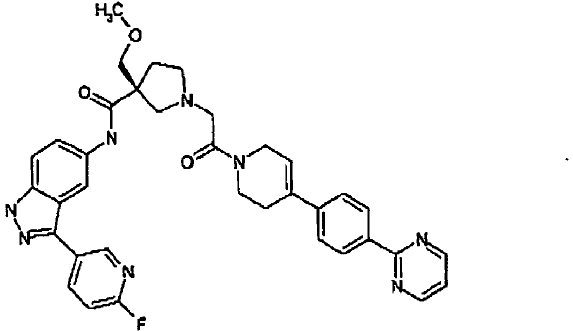 |
| 711 | 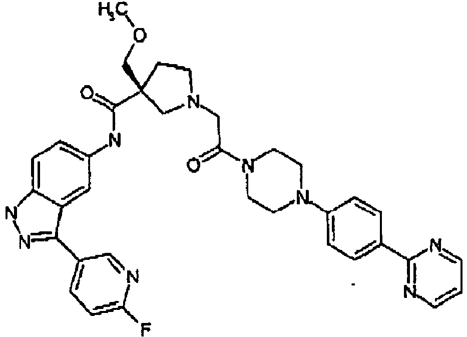 |
| 712 | 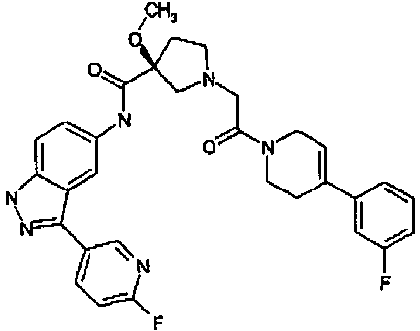 |
| 713 | 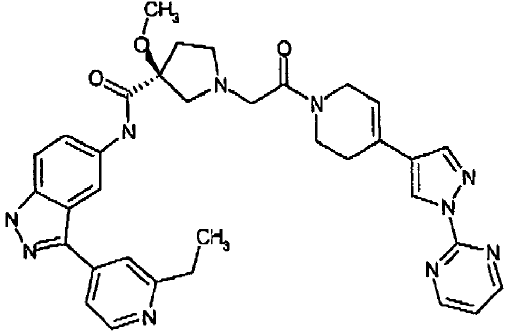 |
| 714 | 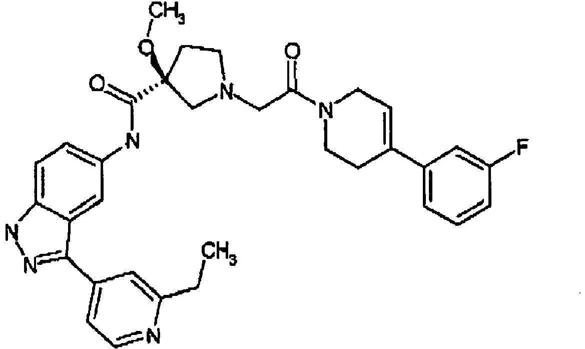 |
| 715 | 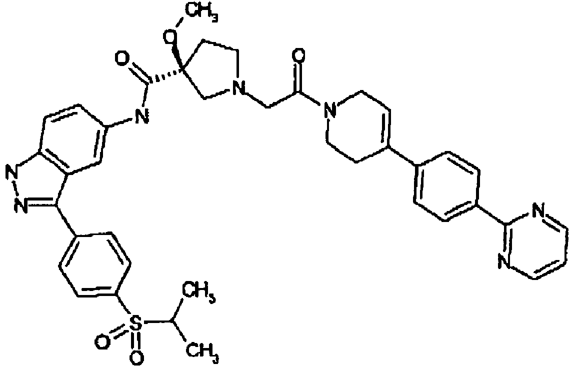 |
| 716 | 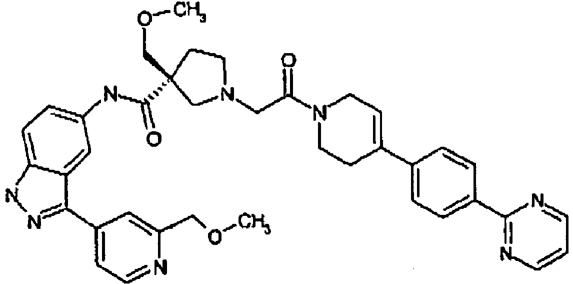 |
| 717 | 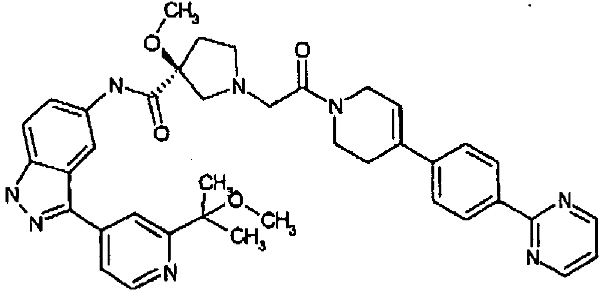 |
| 718 | 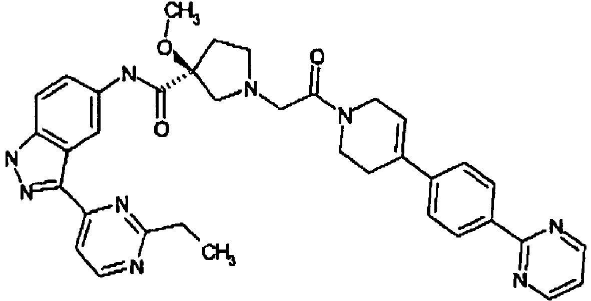 |
| 719 | 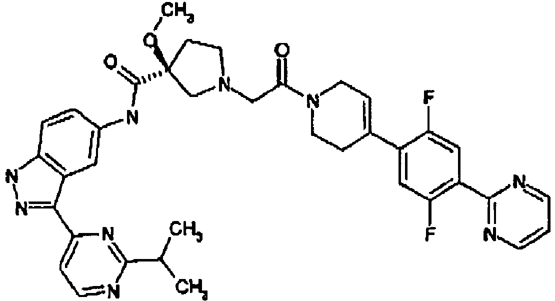 |
| 720 | 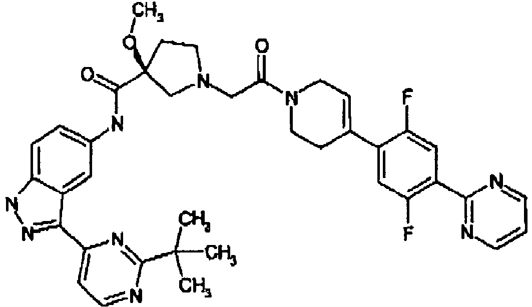 |
| 721 | 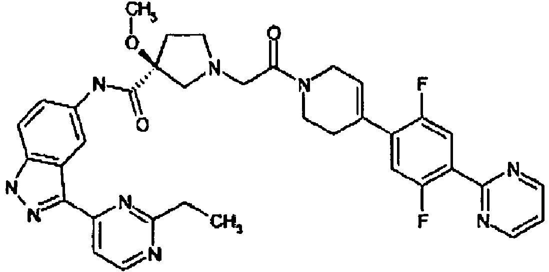 |
| 722 | 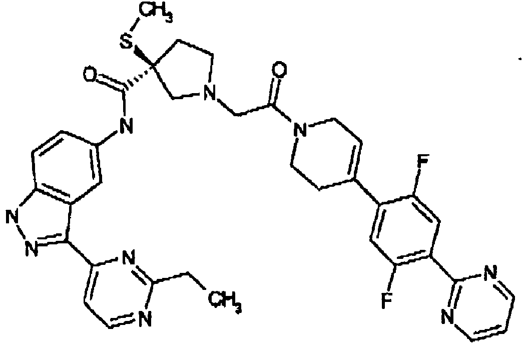 |
| 723 | 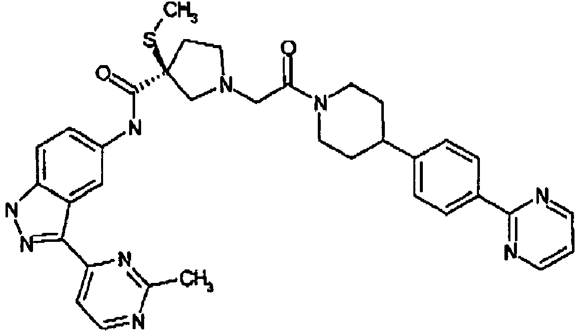 |
| 724 | 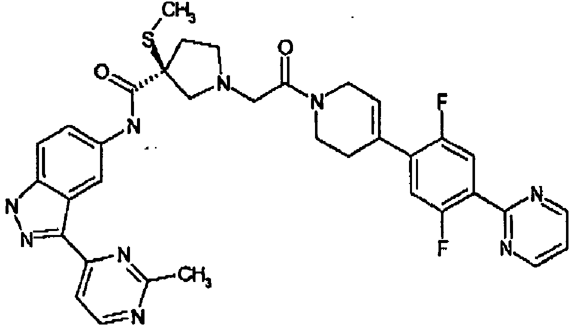 |
| 725 | 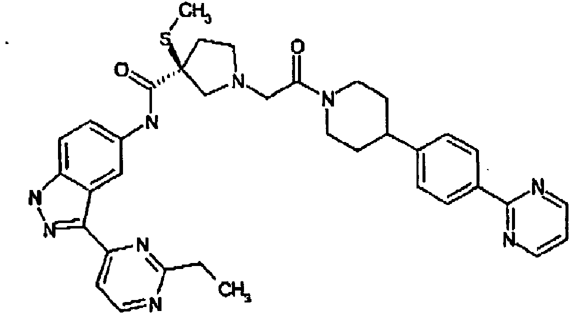 |
| 726 | 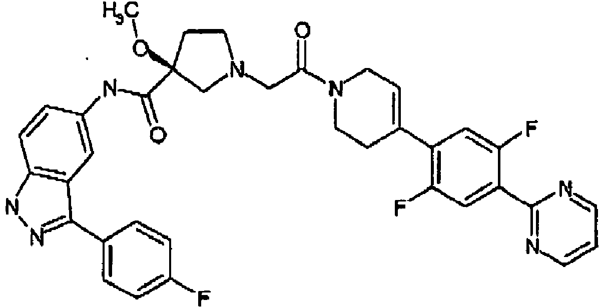 |
| 727 | 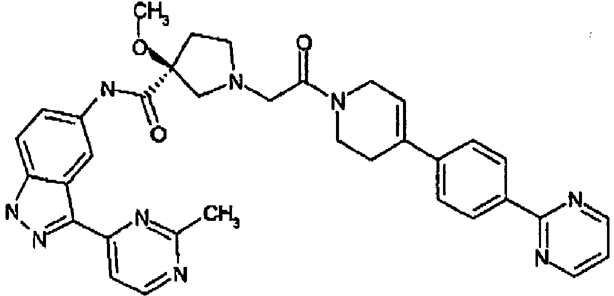 |
| 728 | 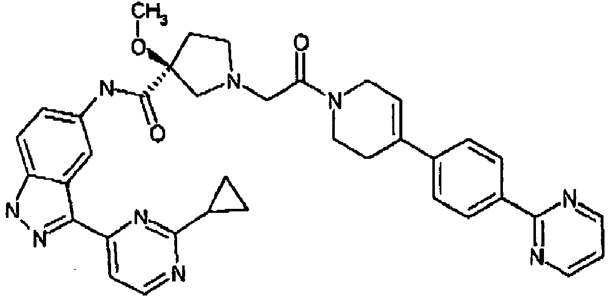 |
| 729 | 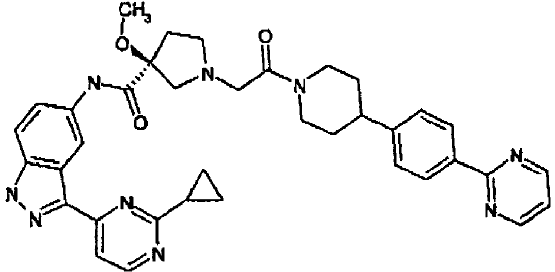 |
| 730 | 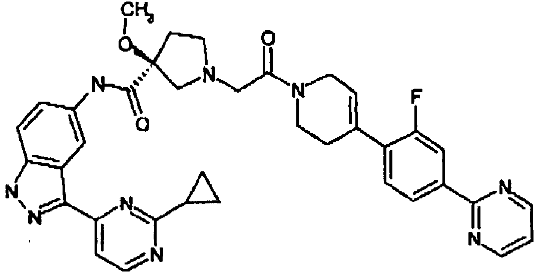 |
| 731 | 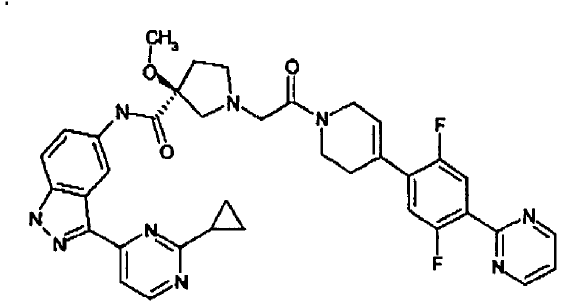 |
| 732 | 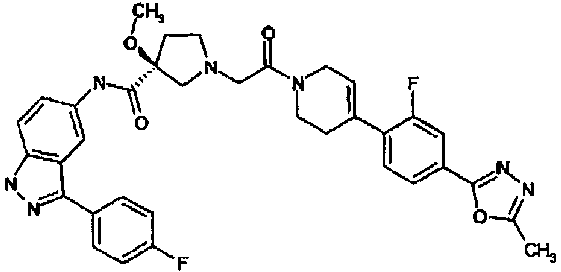 |
| 733 | 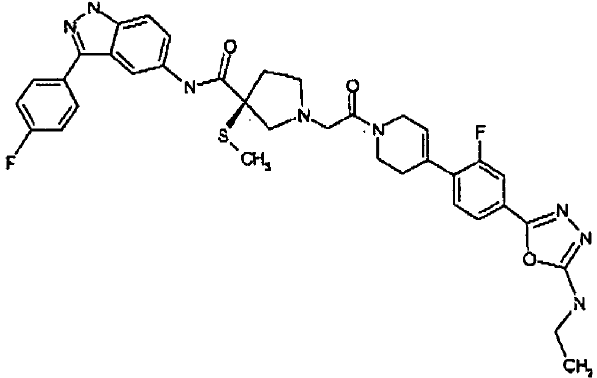 |
| 734 | 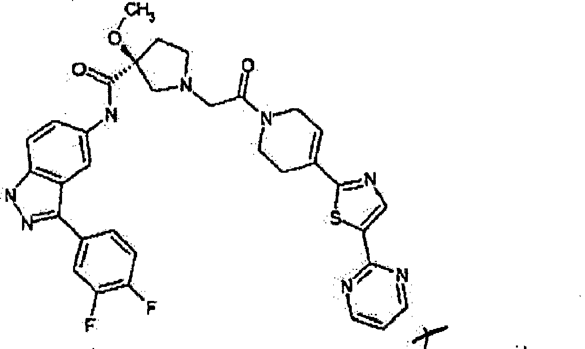 |
| 735 | 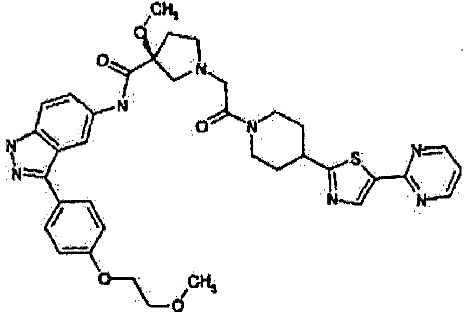 |
| 736 | 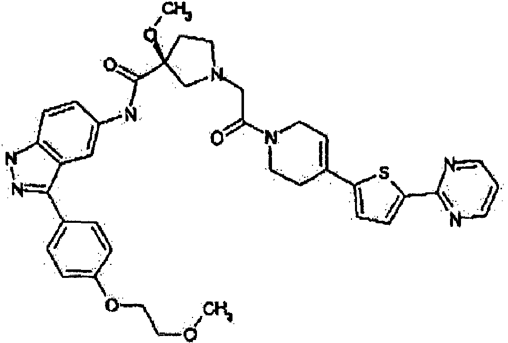 |
| 737 | 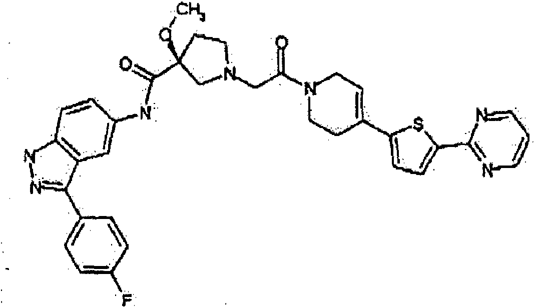 |
| 738 | 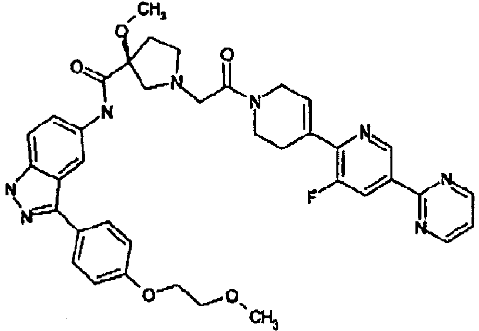 |
| 739 | 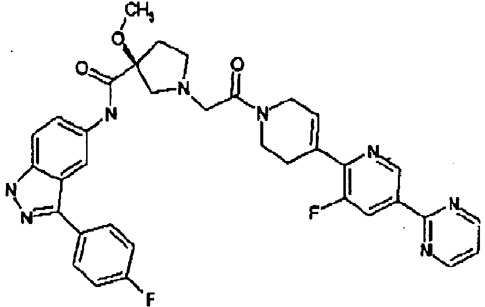 |
| 740 | 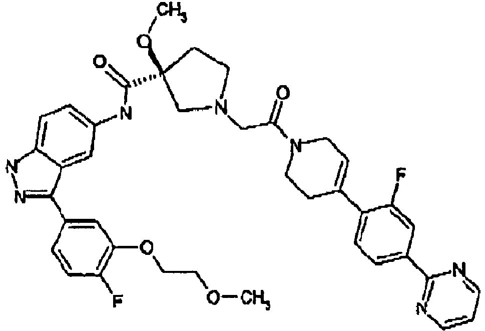 |
| 741 | 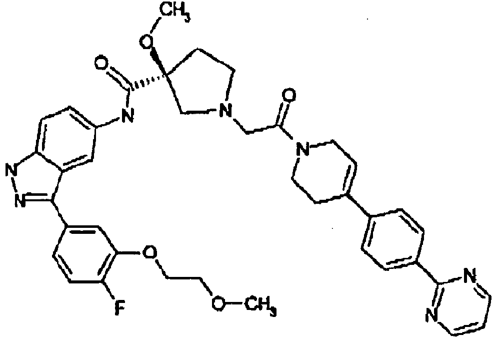 |
| 742 | 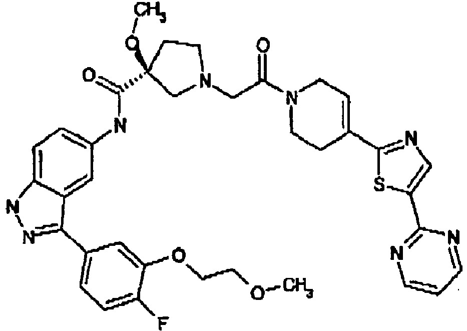 |
| 743 | 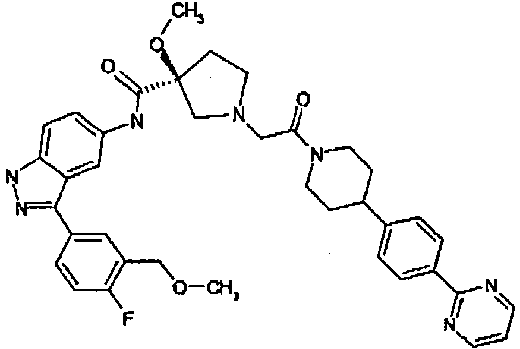 |
| 744 | 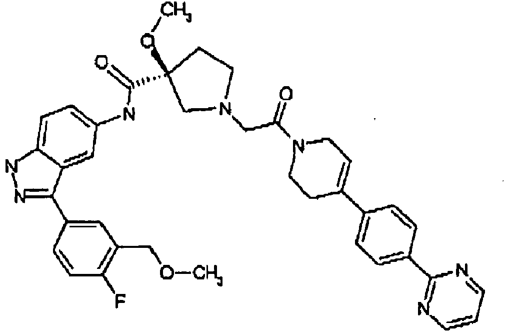 |
| 745 | 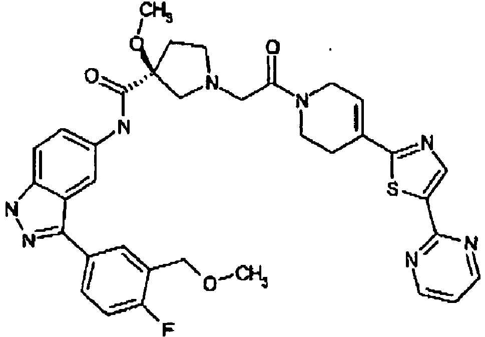 |
| 746 | 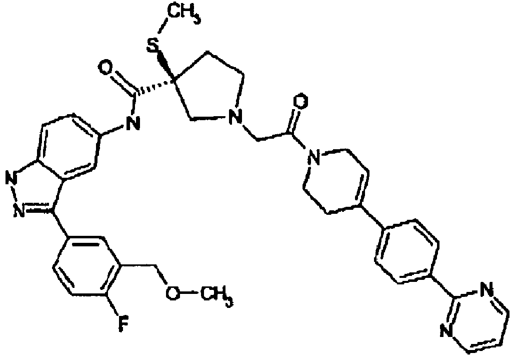 |
| 747 | 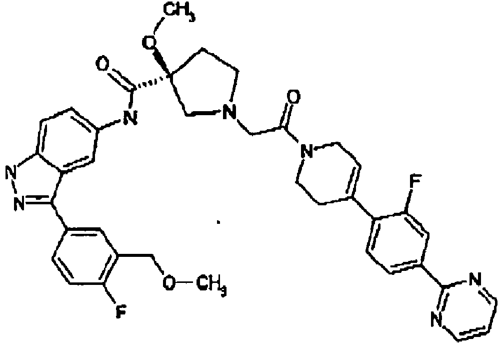 |
| 748 | 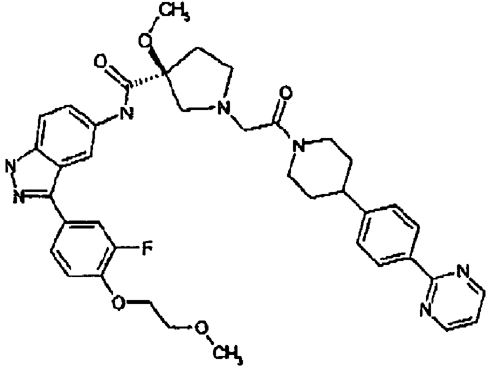 |
| 749 | 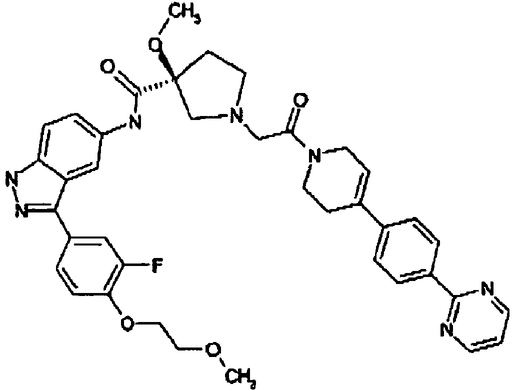 |
| 750 | 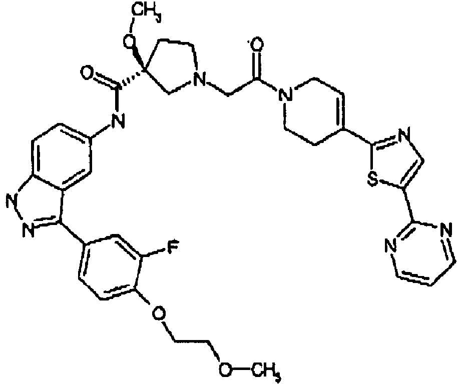 |
| 751 | 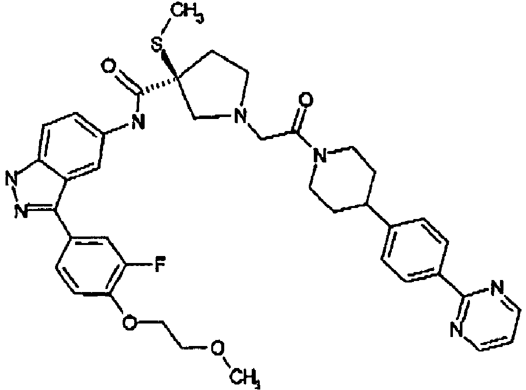 |
| 752 | 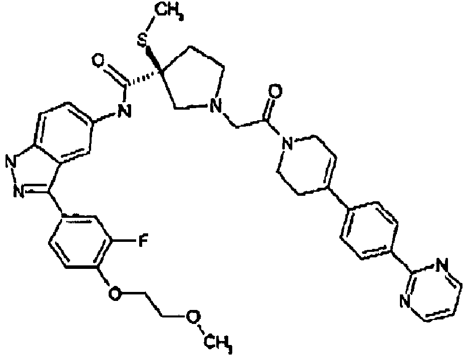 |
| 753 | 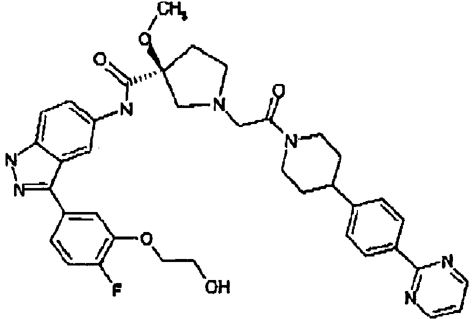 |
| 754 | 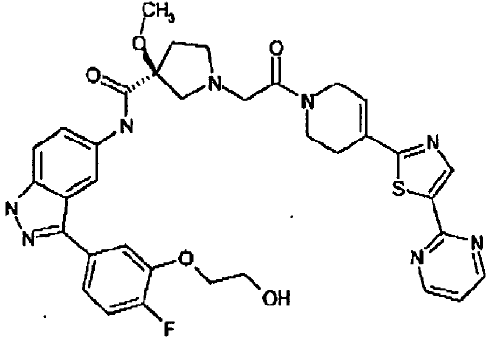 |
| 755 | 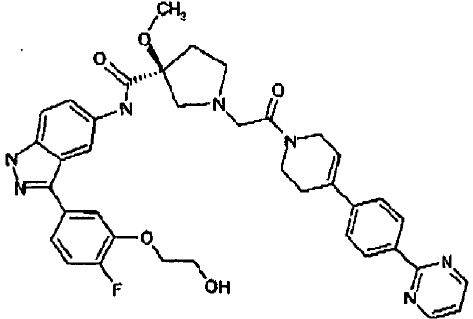 |
| 756 | 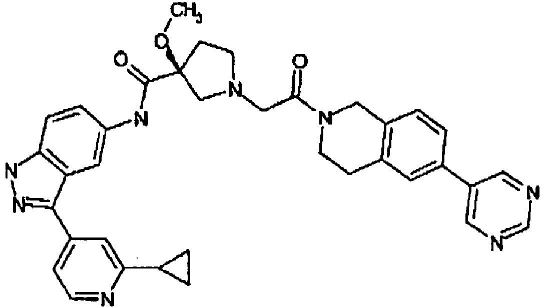 |
| 757 | 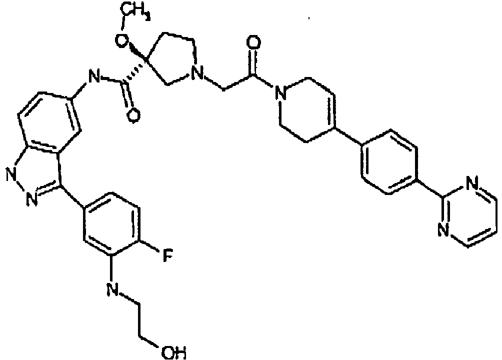 |
| 758 | 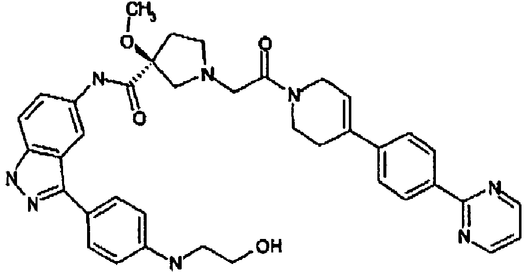 |
| 759 | 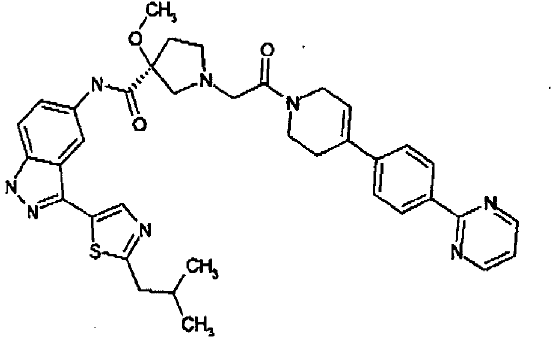 |
| 760 | 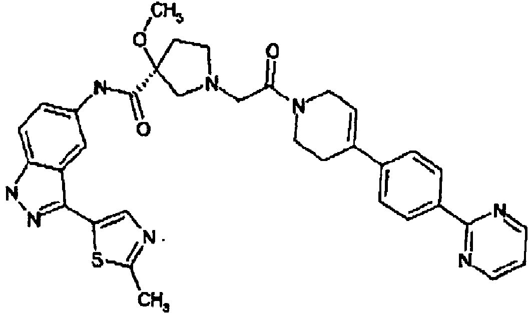 |
| 761 | 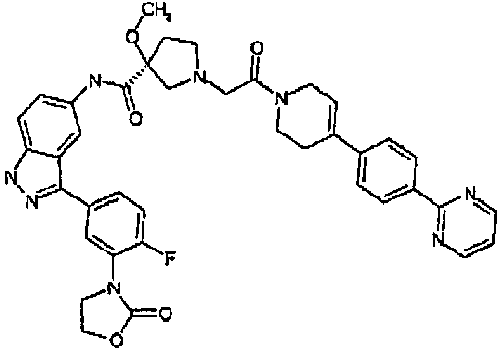 |
| 762 | 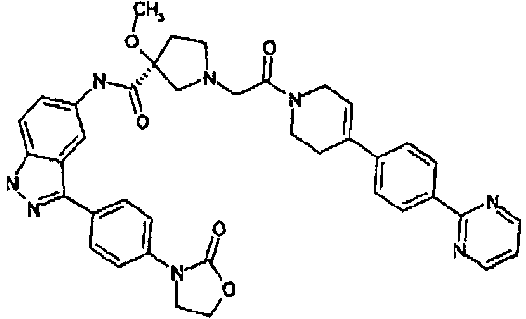 |
| 763 | 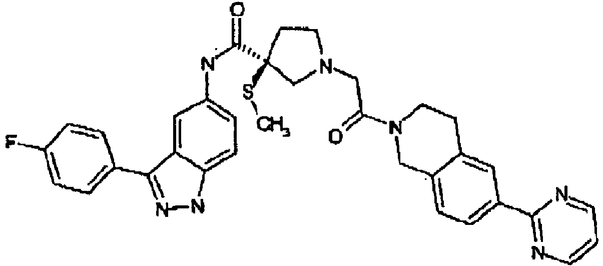 |
| 764 | 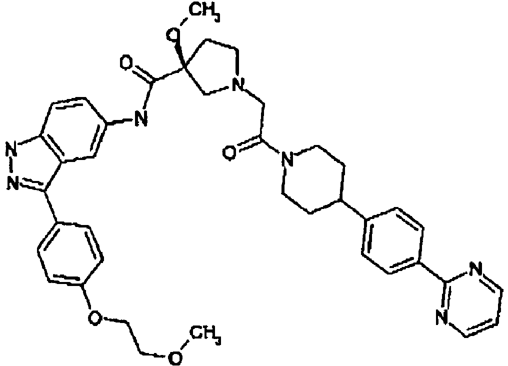 |
| 765 | 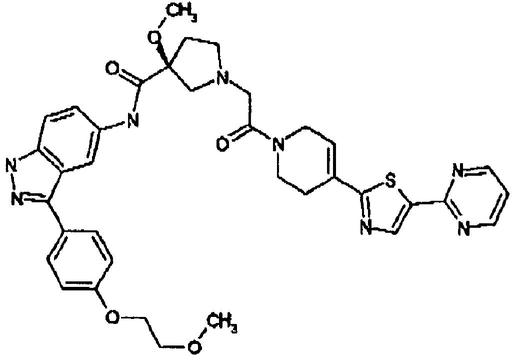 |
| 766 | 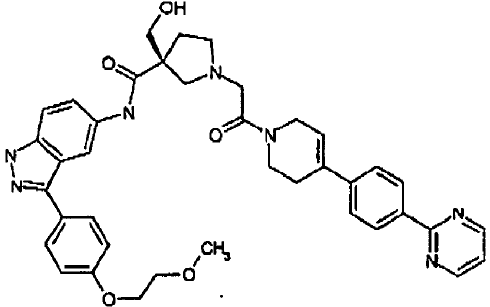 |
| 767 | 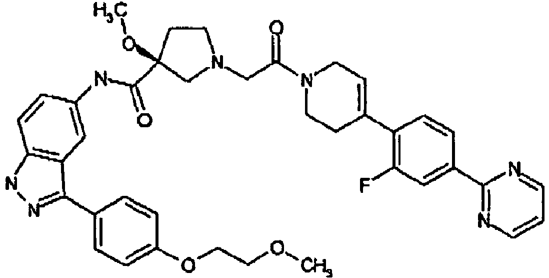 |
| 768 | 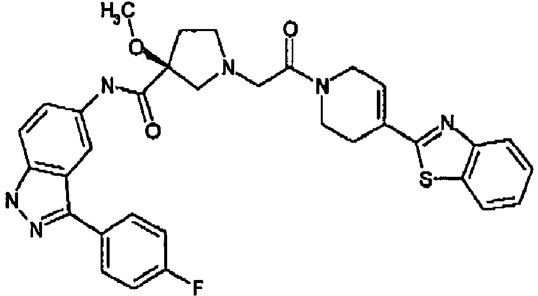 |
| 769 | 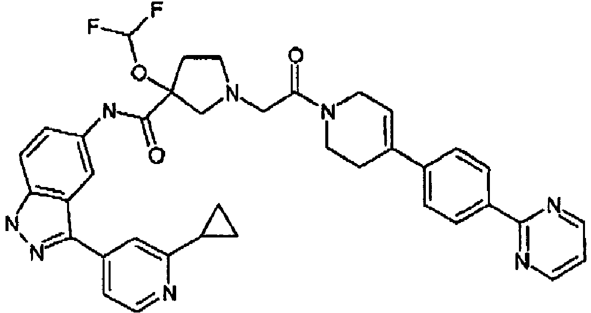 |
| 770 | 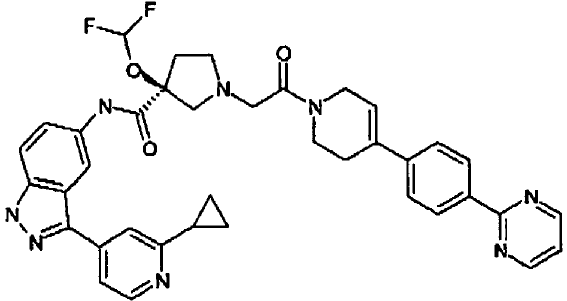 |
| 771 | 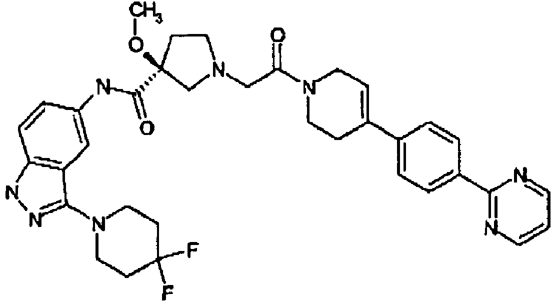 |
| 772 | 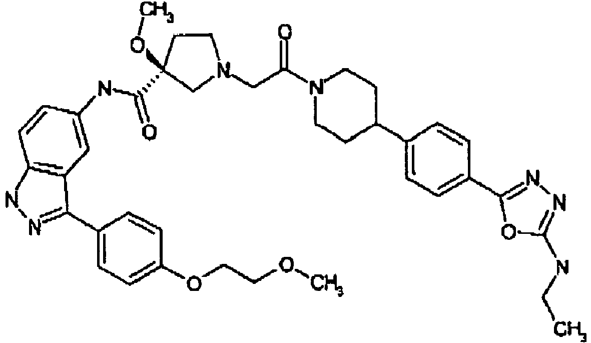 |
| 773 | 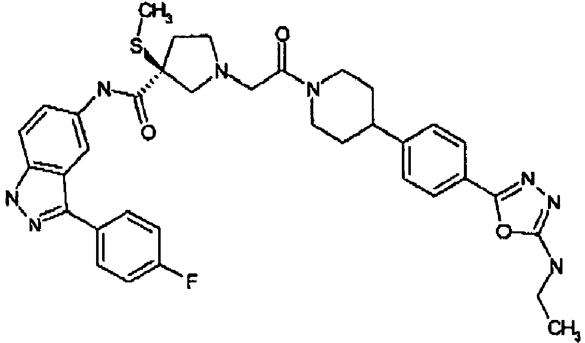 |
| 774 | 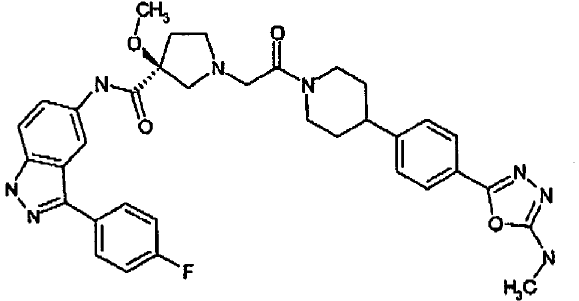 |
| 775 | 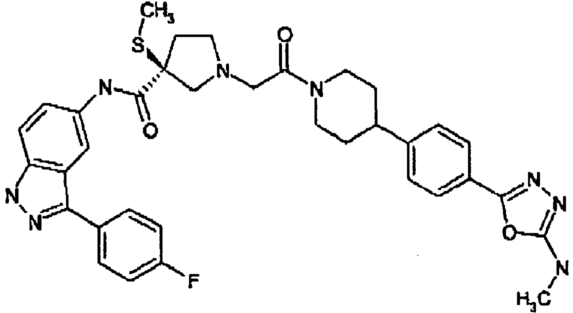 |
| 776 | 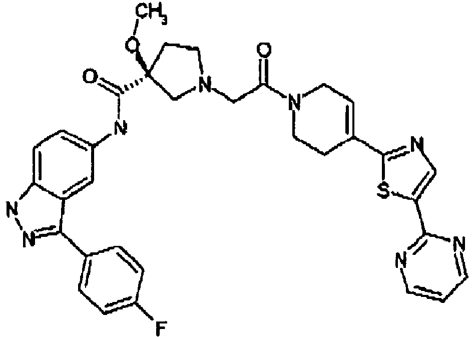 |
| 777 | 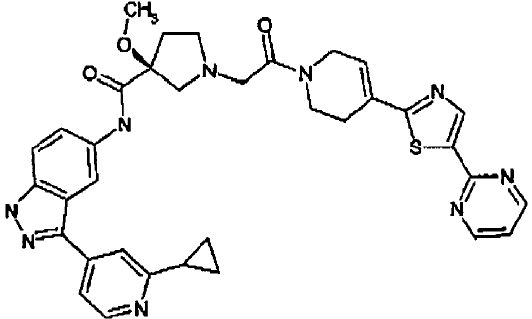 |
| 778 | 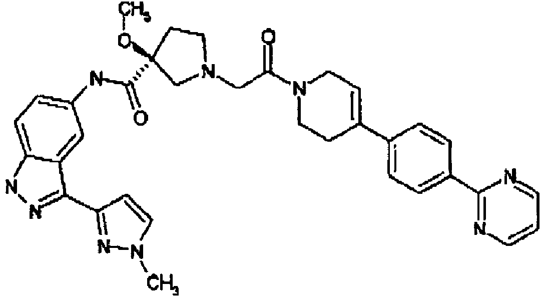 |
| 779 | 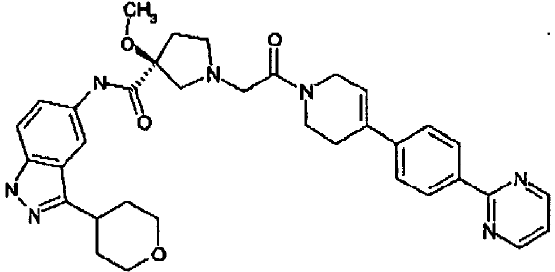 |
| 780 | 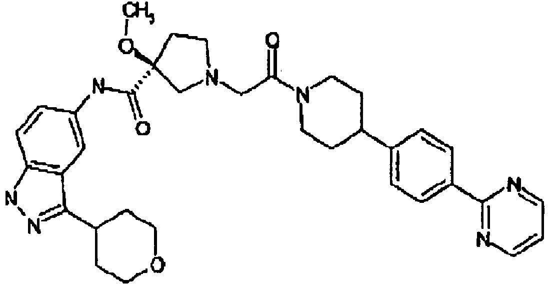 |
| 781 | 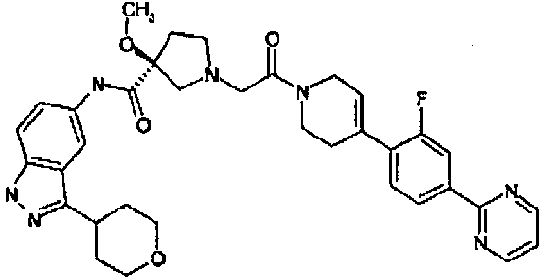 |
| 782 | 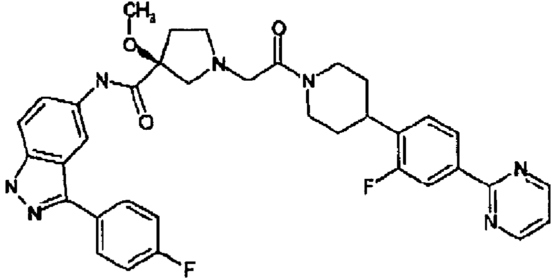 |
| 783 | 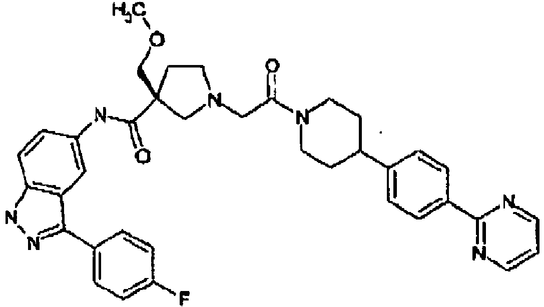 |
| 784 | 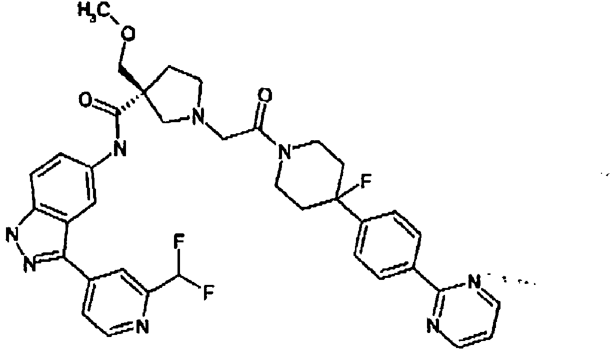 |
| 785 | 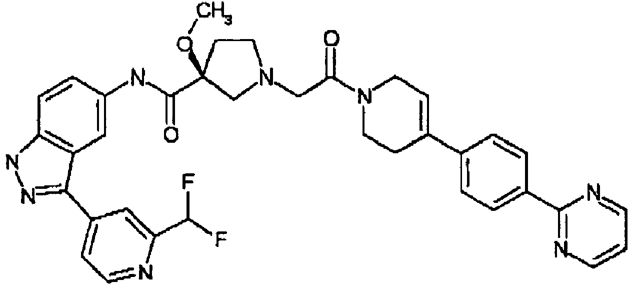 |
| 786 | 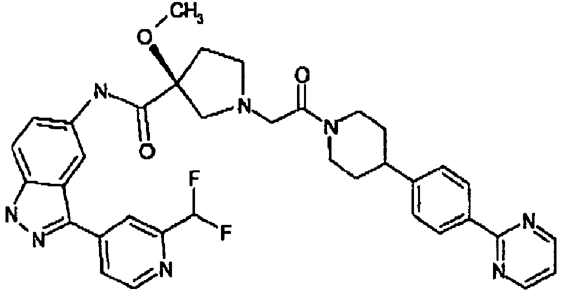 |
| 787 | 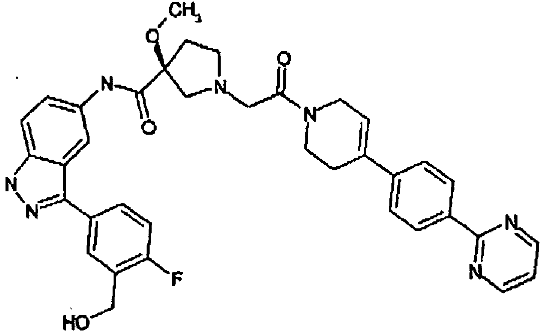 |
| 788 | 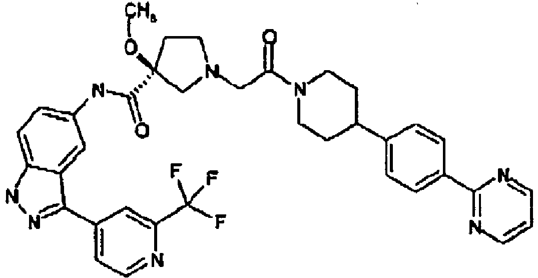 |
| 789 | 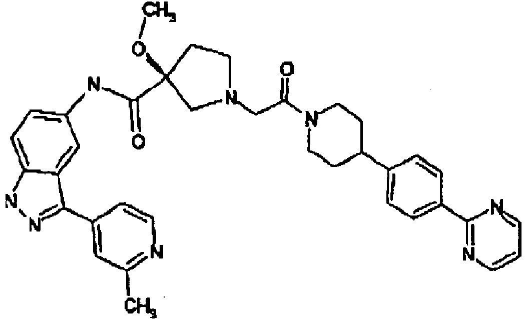 |
| 790 | 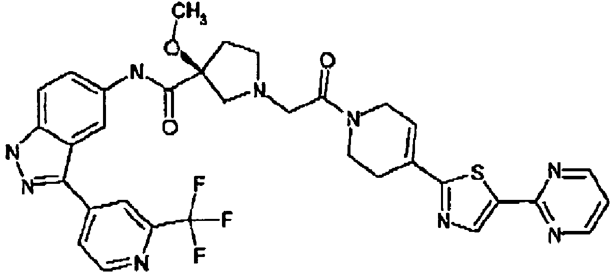 |
| 791 | 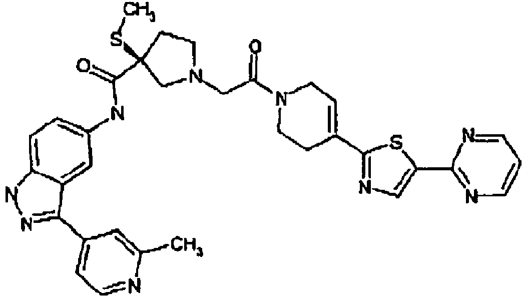 |
| 792 | 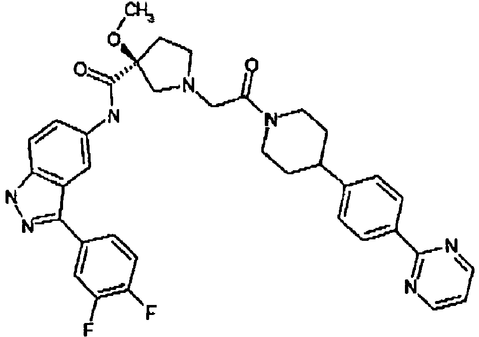 |
| 793 | 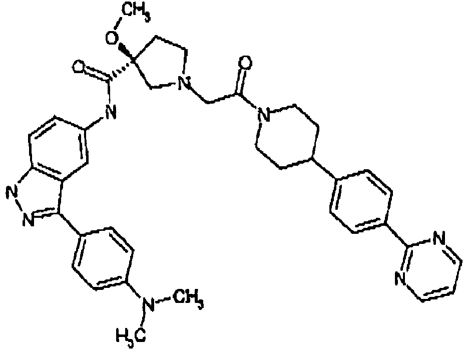 |
| 794 | 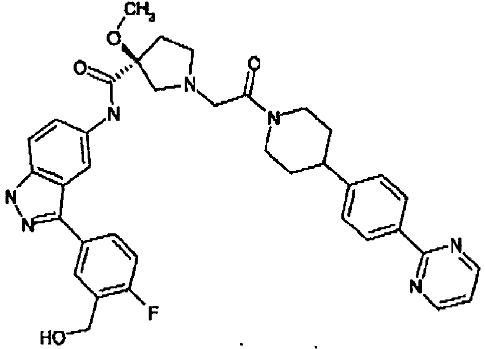 |
| 795 | 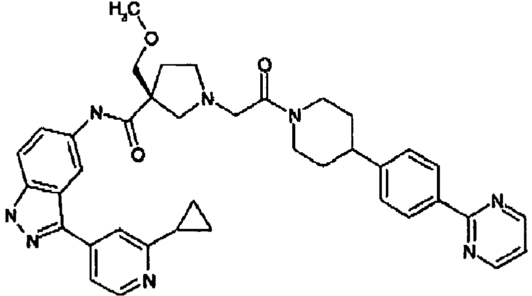 |
| 796 | 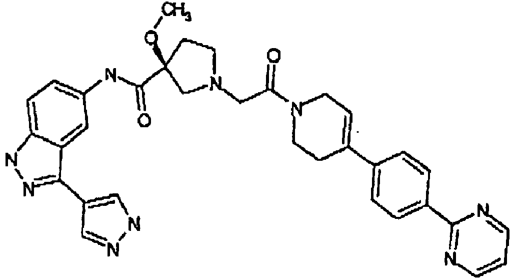 |
| 797 | 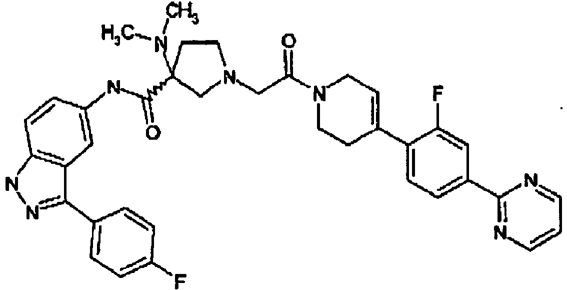 |
| 798 | 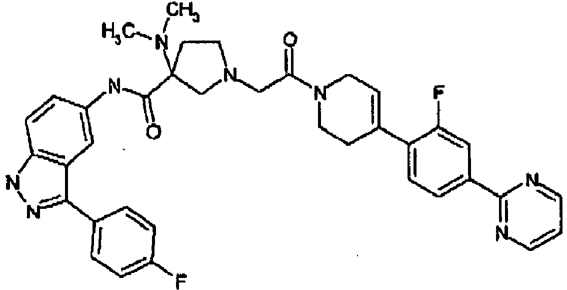 |
| 799 | 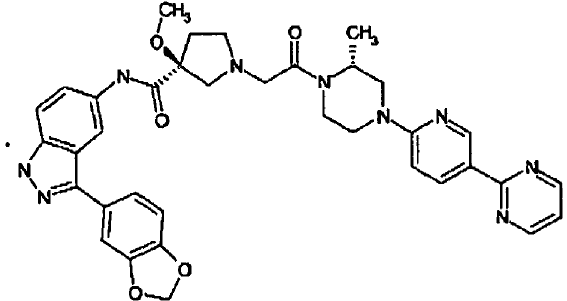 |
| 800 | 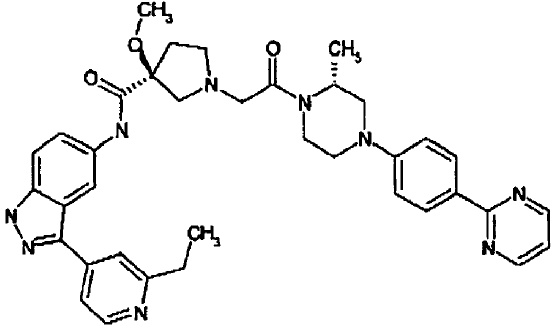 |
| 801 | 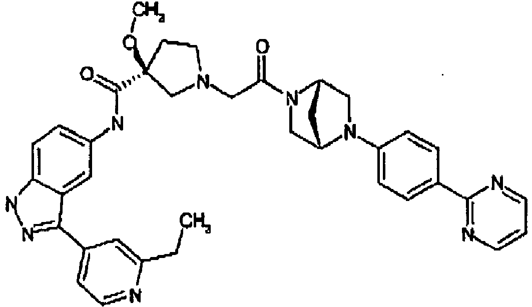 |
| 802 | 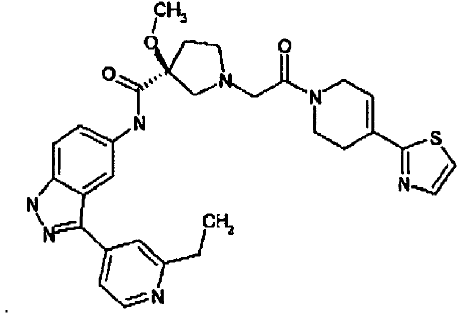 |
| 803 | 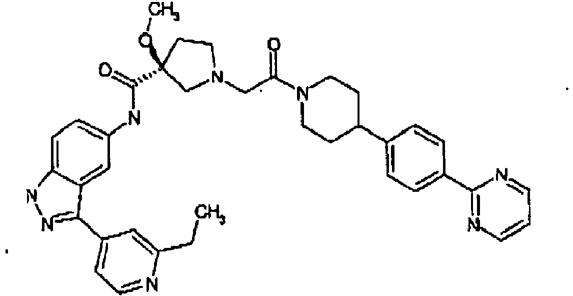 |
| 804 | 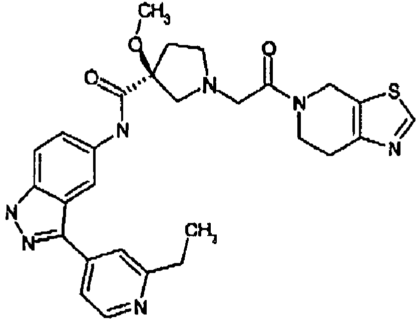 |
| 805 | 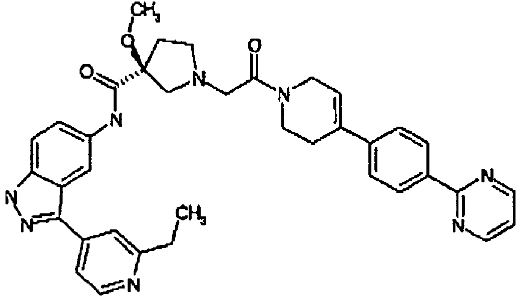 |
| 806 | 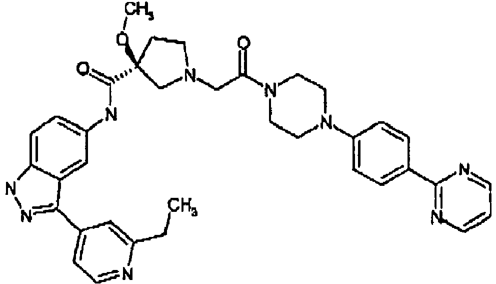 |
| 807 | 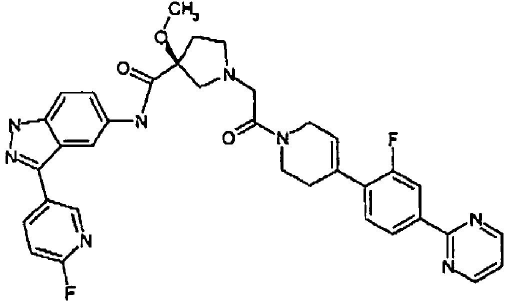 |
| 808 | 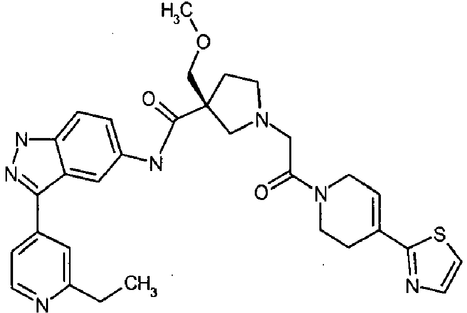 |
| 809 | 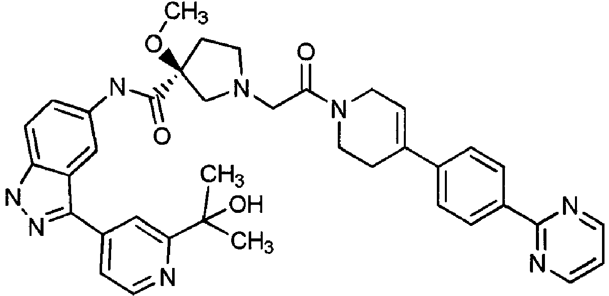 |
| 810 | 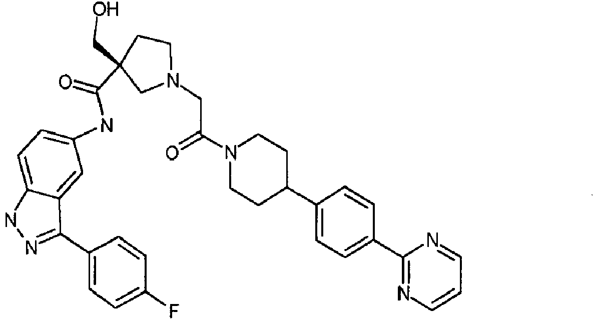 |
| 811 | 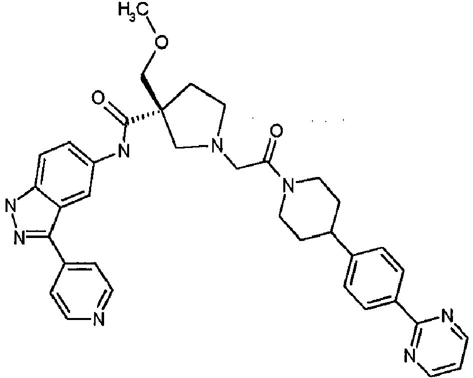 |
| 812 | 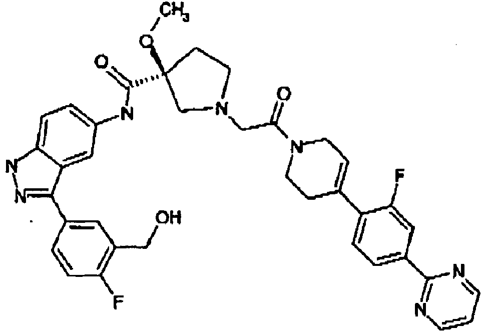 |
| 813 | 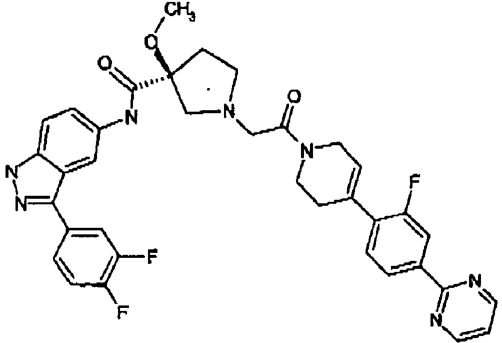 |
| 814 | 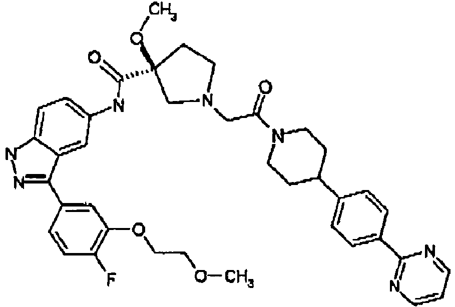 |
| 815 | 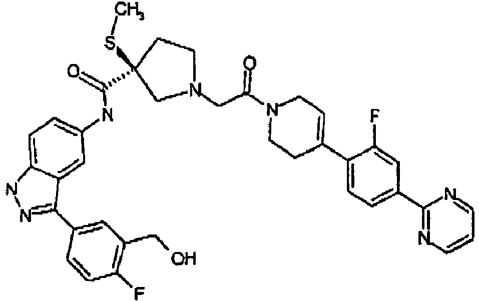 |
| 816 | 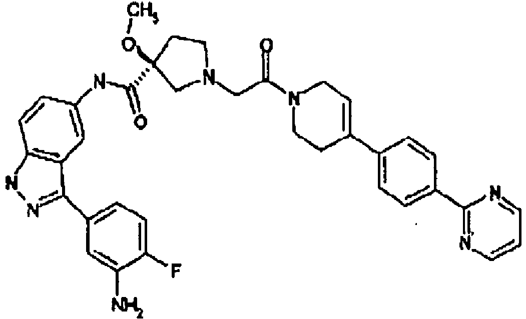 |
| 817 | 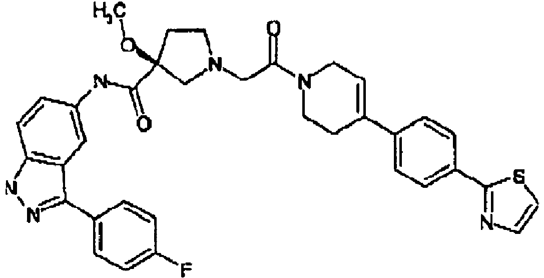 |
| 818 | 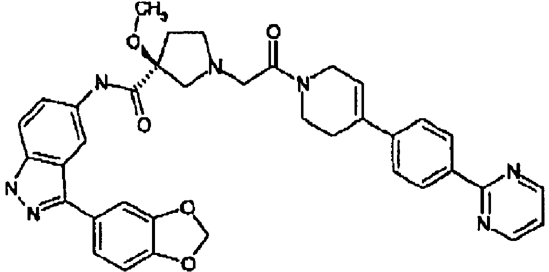 |
| 819 | 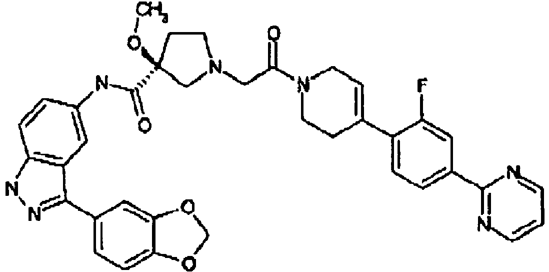 |
| 820 | 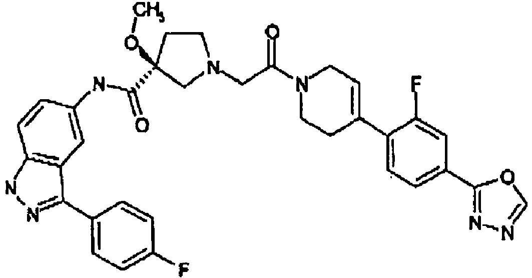 |
| 821 | 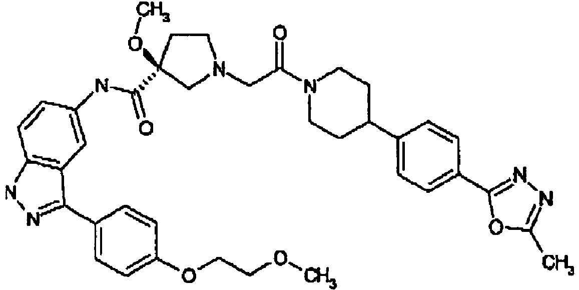 |
| 822 | 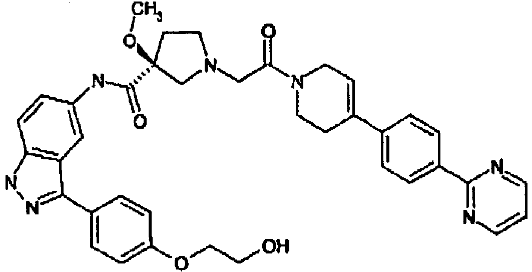 |
| 823 | 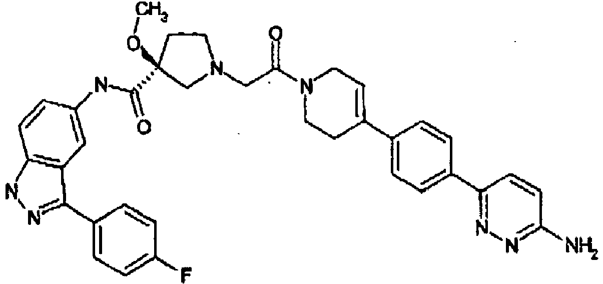 |
| 824 | 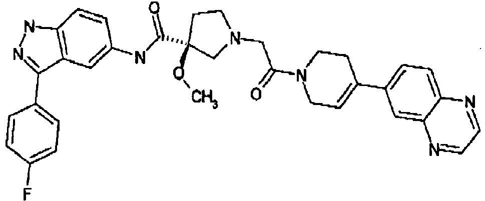 |
| 826 | 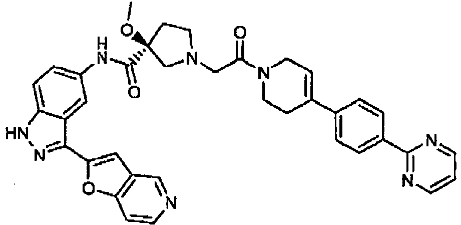 |
| 827 | 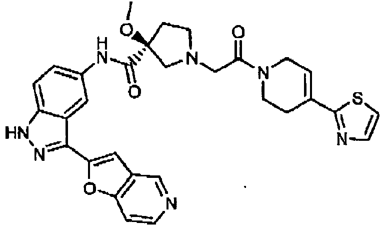 |
ou un sel pharmaceutiquement acceptable de ceux-ci.

2. Inhibiteur ERK selon la revendication 1:
(a) présentant la formule: 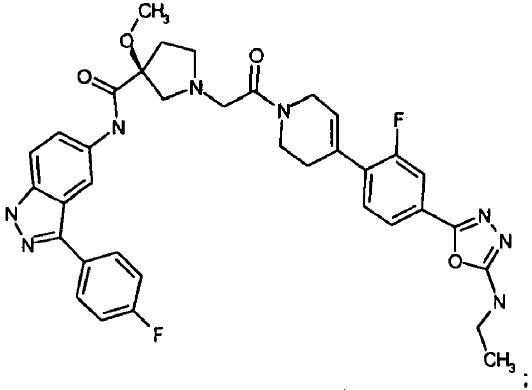 ou
(b) présentant la formule: 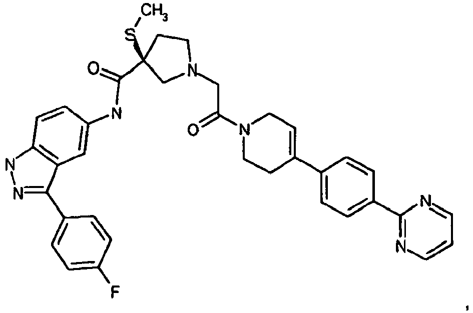 ou un sel pharmaceutiquement acceptable de ceux-ci.

3. Composition pharmaceutique comprenant au moins un composé selon la revendication 1 ou 2 ou un sel pharmaceutiquement acceptable de celui-ci et un support pharmaceutiquement acceptable.

4. Utilisation d'au moins un composé selon la revendication 1 ou 2 ou d'un sel pharmaceutiquement acceptable de celui-ci:
(1) pour la fabrication d'un médicament pour le traitement d'un cancer; ou
(2) pour la fabrication d'un médicament pour le traitement d'un cancer, ledit médicament étant utilisé en combinaison avec au moins un agent chimiothérapeutique; ou
(3) pour la fabrication d'un médicament pour le traitement d'un cancer, ledit médicament étant utilisé en combinaison avec au moins un agent chimiothérapeutique et une radiothérapie; ou
(4) pour la fabrication d'un médicament pour le traitement d'un cancer, ledit médicament étant utilisé en combinaison avec au moins un agent chimiothérapeutique choisi dans le groupe constitué de: (1) taxanes, (2) composés de coordination du platine, (3) inhibiteurs de facteur de croissance épidermique (EGF) qui sont des anticorps, (4) inhibiteurs de EGF qui sont de petites molécules, (5) inhibiteurs de facteur de croissance endothéliale vasculaire (VEGF) qui sont des anticorps, (6) inhibiteurs de VEGF qui sont de petites molécules, (7) antagonistes de récepteur d'oestrogènes ou modulateurs de récepteur d'oestrogènes sélectifs (SERMs), (8) dérivés nucléosidiques anti-tumoraux, (9) épothilones, (10) inhibiteurs de topoisomérase, (11) alcaloïdes de la pervenche, (12) anticorps qui sont des inhibiteurs d'intégrines αVβ3, (13) antagonistes de folate, (14) inhibiteurs de ribonucléotide réductase, (15) anthracyclines, (16) composés biologiques, (17) inhibiteurs d'angiogénèse et/ou suppresseurs de facteur de nécrose tumorale alpha (TNF-alpha) tels que le thalidomide (ou un imide apparenté), (18) inhibiteurs de Bcr/abl kinase, (19) inhibiteurs de MEK1 et/ou de MEK2 qui sont de petites molécules, (20) inhibiteurs de IGF-1 et de IGF-2 qui sont de petites molécules, (21) inhibiteurs à petites molécules de RAF et BRAF kinases, (22) inhibiteurs à petites molécules de kinases dépendant du cycle cellulaire tels que CDK1, CDK2, CDK4 et CDK6, (23) agents d'alkylation et (24) inhibiteurs de famésyle protéine transférase; ou
(5) pour la fabrication d'un médicament pour le traitement d'un cancer, ledit médicament étant administré avec au moins un inhibiteur de transduction de signal; ou
(6) pour la fabrication d'un médicament pour le traitement d'un cancer, ledit cancer étant choisi dans le groupe constitué de: cancer du poumon, cancer pancréatique, cancer du côlon, leucémies myéloïdes, cancer de la thyroïde, syndrome myélodysplasique, carcinome de la vessie, carcinome épidermique, mélanome, cancer du sein, cancer de la prostate, cancer de la tête et du cou, cancer ovarien, cancers du cerveau, cancers d'origine mésenchymateuse, sarcomes, tétracarcinomes, neuroblastomes, carcinomes du rein, hépatomes, lymphome non hodgkinien, myélome multiple et carcinome de la thyroïde anaplasique; ou
(7) pour la fabrication d'un médicament pour le traitement d'un cancer, ledit cancer étant choisi dans le groupe constitué de: mélanome, cancer pancréatique, cancer de la thyroïde, cancer colorectal, cancer du poumon, cancer du sein et cancer ovarien; ou
(8) pour la fabrication d'un médicament pour le traitement d'un cancer, ledit médicament étant utilisé en combinaison avec une quantité efficace d'au moins un agent chimiothérapeutique, dans lequel ledit cancer étant choisi dans le groupe constitué de: mélanome, cancer pancréatique, cancer de la thyroïde, cancer colorectal, cancer du poumon, cancer du sein et cancer ovarien; ou
(9) pour la fabrication d'un médicament pour le traitement d'un mélanome; ou
(10) pour la fabrication d'un médicament pour le traitement d'un mélanome, ledit médicament étant utilisé en combinaison avec au moins un agent chimiothérapeutique; ou
(11) pour la fabrication d'un médicament pour le traitement d'un cancer pancréatique; ou
(12) pour la fabrication d'un médicament pour le traitement d'un cancer pancréatique, ledit médicament étant utilisé en combinaison avec au moins un agent chimiothérapeutique; ou
(13) pour la fabrication d'un médicament pour le traitement d'un cancer de la thyroïde; ou
(14) pour la fabrication d'un médicament pour le traitement d'un cancer de la thyroïde, ledit médicament étant utilisé en combinaison avec au moins un agent chimiothérapeutique; ou
(15) pour la fabrication d'un médicament pour le traitement d'un cancer colorectal; ou
(16) pour la fabrication d'un médicament pour le traitement d'un cancer colorectal, ledit médicament étant utilisé en combinaison avec au moins un agent chimiothérapeutique; ou
(17) pour la fabrication d'un médicament pour le traitement d'un cancer du poumon; ou
(18) pour la fabrication d'un médicament pour le traitement d'un cancer du poumon, ledit médicament étant utilisé en combinaison avec au moins un agent chimiothérapeutique; ou
(19) pour la fabrication d'un médicament pour le traitement d'un cancer du sein; ou
(20) pour la fabrication d'un médicament pour le traitement d'un cancer du sein, ledit médicament étant utilisé en combinaison avec au moins un agent chimiothérapeutique; ou
(21) pour la fabrication d'un médicament pour le traitement d'un cancer ovarien; ou
(22) pour la fabrication d'un médicament pour le traitement d'un cancer ovarien, ledit médicament étant utilisé en combinaison avec au moins un agent chimiothérapeutique; ou
(23) pour la fabrication d'un médicament pour le traitement d'un cancer du sein hormono-dépendant, ledit médicament étant utilisé en combinaison avec des agents antihormonaux; ou
(24) pour la fabrication d'un médicament pour le traitement d'un cancer du sein hormono-dépendant, ledit médicament étant utilisé en combinaison avec des agents antihormonaux et au moins un agent chimiothérapeutique; ou
(25) pour la fabrication d'un médicament pour la prévention d'un cancer du sein hormono-dépendant, ledit médicament étant utilisé en combinaison avec des agents antihormonaux; ou
(26) pour la fabrication d'un médicament pour la prévention d'un cancer du sein hormono-dépendant, ledit médicament étant utilisé en combinaison avec des agents antihormonaux et au moins un agent chimiothérapeutique; ou
(27) pour la fabrication d'un médicament pour le traitement d'un cancer du cerveau; ou
(28) pour la fabrication d'un médicament pour le traitement d'un cancer du cerveau, ledit médicament étant utilisé en combinaison avec au moins un agent chimiothérapeutique; ou
(29) pour la fabrication d'un médicament pour le traitement d'un cancer du cerveau, ledit médicament étant utilisé en combinaison avec un agent chimiothérapeutique, dans lequel ledit agent chimiothérapeutique est le témozolomide; ou
(30) pour la fabrication d'un médicament pour le traitement d'un cancer de la prostate; ou
(31) pour la fabrication d'un médicament pour le traitement d'un cancer de la prostate, ledit médicament étant utilisé en combinaison avec au moins un agent chimiothérapeutique; ou
(32) pour la fabrication d'un médicament pour le traitement d'un syndrome myélodysplasique; ou
(33) pour la fabrication d'un médicament pour le traitement d'un syndrome myélodysplasique, ledit médicament étant utilisé en combinaison avec au moins un agent chimiothérapeutique; ou
(34) pour la fabrication d'un médicament pour le traitement de leucémies myéloïdes; ou
(35) pour la fabrication d'un médicament pour le traitement de leucémies myéloïdes, ledit médicament étant utilisé en combinaison avec au moins un agent chimiothérapeutique; ou
(36) pour la fabrication d'un médicament pour le traitement d'une leucémie myélogène aiguë; ou
(37) pour la fabrication d'un médicament pour le traitement d'une leucémie myélogène aiguë, ledit médicament étant utilisé en combinaison avec au moins un agent chimiothérapeutique; ou
(38) pour la fabrication d'un médicament pour le traitement d'une leucémie myélomonocytique chronique; ou
(39) pour la fabrication d'un médicament pour le traitement d'une leucémie myélomonocytique chronique, ledit médicament étant utilisé en combinaison avec au moins un agent chimiothérapeutique; ou
(40) pour la fabrication d'un médicament pour le traitement d'une leucémie myélogène chronique; ou
(41) pour la fabrication d'un médicament pour le traitement d'une leucémie myélogène chronique, ledit médicament étant utilisé en combinaison avec au moins un agent chimiothérapeutique; ou
(42) pour la fabrication d'un médicament pour le traitement d'un cancer de la vessie; ou
(43) pour la fabrication d'un médicament pour le traitement d'un cancer de la vessie, ledit médicament étant utilisé en combinaison avec au moins un agent chimiothérapeutique; ou
(44) pour la fabrication d'un médicament pour le traitement d'un lymphome non hodgkinien; ou
(45) pour la fabrication d'un médicament pour le traitement d'un lymphome non hodgkinien, ledit médicament étant utilisé en combinaison avec au moins un agent chimiothérapeutique; ou
(46) pour la fabrication d'un médicament pour le traitement d'un myélome multiple; ou
(47) pour la fabrication d'un médicament pour le traitement d'un myélome multiple, ledit médicament étant utilisé en combinaison avec au moins un agent chimiothérapeutique.

5. Composé selon la revendication 1 ou 2 ou un sel pharmaceutiquement acceptable de celui-ci pour une utilisation dans une méthode de traitement du corps humain par une thérapie.

6. Composé selon la revendication 5, dans lequel la thérapie est telle que définie dans la revendication 4.

7. Combinaison d'un composé selon la revendication 1 ou 2 ou d'un sel pharmaceutiquement acceptable de celui-ci et d'au moins un agent chimiothérapeutique choisi parmi:
(1) des taxanes,
(2) des composés de coordination du platine,
(3) des inhibiteurs de facteur de croissance épidermique (EGF) qui sont des anticorps,
(4) des inhibiteurs de EGF qui sont de petites molécules,
(5) des inhibiteurs de facteur de croissance endothéliale vasculaire (VEGF) qui sont des anticorps,
(6) des inhibiteurs de VEGF qui sont de petites molécules,
(7) des antagonistes de récepteur d'oestrogènes ou des modulateurs de récepteur d'oestrogènes sélectifs (SERMs),
(8) des dérivés nucléosidiques anti-tumoraux,
(9) des épothilones,
(10) des inhibiteurs de topoisomérase,
(11) des alcaloïdes de la pervenche,
(12) des anticorps qui sont des inhibiteurs d'intégrines αVβ3,
(13) des antagonistes de folate,
(14) des inhibiteurs de ribonucléotide réductase,
(15) des anthracyclines,
(16) des composés biologiques,
(17) des inhibiteurs d'angiogénèse et/ou des suppresseurs de facteur de nécrose tumorale alpha (TNF-alpha) tels que le thalidomide (ou un imide apparenté),
(18) des inhibiteurs de Bcr/abl kinase,
(19) des inhibiteurs de MEK1 et/ou de MEK2 qui sont de petites molécules,
(20) des inhibiteurs de IGF-1 et de IGF-2 qui sont de petites molécules,
(21) des inhibiteurs à petites molécules de RAF et BRAF kinases,
(22) des inhibiteurs à petites molécules de kinases dépendant du cycle cellulaire tels que CDK1, CDK2, CDK4 et CDK6,
(23) des agents d'alkylation, et
(24) des inhibiteurs de famésyle protéine transférase (également connus sous le nom d'inhibiteurs de FPT ou FTI (c'est-à-dire inhibiteurs de transfert de famésyle)).
